(19) Europäisches Patentamt
European Patent Office
Office européen des brevets

(11) EP 2 834 221 B1

(12) # EUROPÄISCHE PATENTSCHRIFT

(45) Veröffentlichungstag und Bekanntmachung des Hinweises auf die Patenterteilung:
**25.05.2016 Patentblatt 2016/21**

(21) Anmeldenummer: **13711291.8**

(22) Anmeldetag: **21.03.2013**

(51) Int Cl.:
*C07D 223/08* (2006.01) *C07D 223/10* (2006.01)
*C07D 401/04* (2006.01) *C07D 401/06* (2006.01)
*C07D 401/10* (2006.01) *C07D 403/04* (2006.01)
*C07D 403/06* (2006.01) *C07D 403/10* (2006.01)
*C07D 403/14* (2006.01) *C07D 207/24* (2006.01)
*C07D 207/26* (2006.01) *C07D 211/74* (2006.01)
*C07D 211/76* (2006.01) *A61K 31/402* (2006.01)
*A61K 31/4025* (2006.01)

(86) Internationale Anmeldenummer:
**PCT/EP2013/000867**

(87) Internationale Veröffentlichungsnummer:
**WO 2013/149704 (10.10.2013 Gazette 2013/41)**

(54) **CYCLISCHE AMIDE ALS METAP-2 INHIBITOREN**

CYCLIC AMIDES AS METAP-2 INHIBITORS

AMIDES CYCLIQUES EN TANT QUE INHIBITEURS METAP-2

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.04.2012 DE 102012006884**

(43) Veröffentlichungstag der Anmeldung:
**11.02.2015 Patentblatt 2015/07**

(73) Patentinhaber: **Merck Patent GmbH**
**64293 Darmstadt (DE)**

(72) Erfinder:
- **HEINRICH, Timo**
  **64823 Gross-Umstadt (DE)**
- **ZENKE, Frank**
  **64291 Darmstadt (DE)**
- **KRIER, Mireille**
  **64285 Darmstadt (DE)**
- **FRIESE-HAMIM, Manja**
  **64546 Moerfelden-Walldorf (DE)**
- **SEENISAMY, Jeyaprakashnarayanan**
  **Bangalore 560100 (IN)**

(56) Entgegenhaltungen:
**WO-A1-2012/033956 WO-A2-2010/003475**

- **MOSKALENKO, A. I.; BELOPUKHOV, S. L.; IVLEV, A. A.; BOEV, V. I.: "General procedure for the synthesis of spirocyclic 3-hydroxy- and 3-oxotetrahydrofurans containing carbo- and heterocyclic fragments", RUSSIAN JOURNAL OF ORGANIC CHEMISTRY, Bd. 47, Nr. 7, 2011, Seiten 1091-1096, XP002697587, ISSN: 1070-4280, DOI: 10.1134/S1070428011070207**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Verbindungen der Formel I

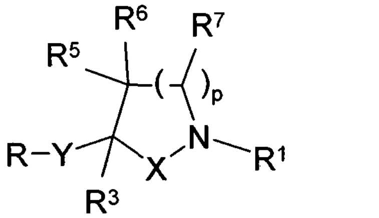

I

worin

R $NR^2R^4$, Alk, $C(=CH_2)[C(R^4)_2]_nAr^2$, $Het^2$, $O[C(R^4)_2]_nAr^2$ oder OA,

X CO oder $CH_2$,

Y CO oder $CH_2$,

$R^1$ H, $[C(R^4)_2]_nAr^1$, $(CH_2)_nHet$, $(CH_2)_nCyc$, $[C(R^4)_2]_nCOOH$, $[C(R^4)_2]_nCONHAr^1$, $[C(R^4)_2]_nCONH_2$, $[C(R^4)_2]_nNHA$, $[C(R^4)_2]_nNA_2$, $O[C(R^4)_2]_nAr^1$, $[C(R^4)_2]_nOR^7$, $[C(R^4)_2]_nCOO(CH_2)_nAr^1$, $[C(R^4)_2]_nCOOA$, $[C(R^4)_2]_nCONH[C(R^4)_2]_pCON(R^4)_2$ oder $[C(R^4)_{2]n}CONHCR^4[(CH_2)_nN(R^4)_2]CON(R^4)_2$,

$R^2$ H, $[C(R^4)_2]_nAr^2$, $(CH_2)_nCOHet^1$, $(CH_2)_nCOAr^2$, $(CH_2)_mNA_2$ oder $(CH_2)_nHet$,

$R^3$ OH oder OCOA,

$R^4$ H oder Alkyl mit 1, 2, 3 oder 4 C-Atomen,

$R^2$ und $R^4$ zusammen auch Alkylen mit 2, 3, 4 oder 5 C-Atomen, wobei eine $CH_2$-Gruppe auch durch $N(CH_2)_mOH$ oder $SO_2$ ersetzt sein kann,

$R^5$, $R^6$ jeweils unabhängig voneinander H, F oder A,

$R^5$ und $R^6$ zusammen auch Alkylen mit 2, 3, 4 oder 5 C-Atomen, wobei eine $CH_2$-Gruppe auch durch NCOA oder O ersetzt sein kann,

$R^7$ H oder A,

$Ar^1$ unsubstituiertes oder ein-, zwei-, drei-, vier- oder fünffach durch Hal, OH, OA, $CONH_2$, CONHA, $CONA_2$, $NHSO_2A$, CONHCyc, $NHSO_2Cyc$, $CONHAr^2$, $COHet^1$ und/oder $NASO_2A$ substituiertes Phenyl,

$Ar^2$ unsubstituiertes oder ein-, zwei-, drei-, vier- oder fünffach durch Hal, A, $CONH_2$, und/oder $OAr^3$ substituiertes Phenyl,

$Ar^3$ unsubstituiertes oder einfach durch $NH_2$ substituiertes Phenyl,

Het einen unsubstituierten oder ein-, zwei- oder dreifach durch Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O und/oder $Ar^3$ substituierten ein- oder zweikernigen gesättigten, ungesättigten oder aromatischen Heterocyclus mit 1 bis 4 N-, und/oder O-und/oder S-Atomen,

$Het^1$ einen unsubstituierten oder ein-, zwei- oder dreifach durch =O und/oder COOA substituierten einkernigen gesättigten Heterocyclus mit 1 bis 4 N-, und/oder O- und/oder S-Atomen,

$Het^2$ Isoindolyl,

A unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können, und/oder worin eine oder zwei nicht-benachbarte CH- und/oder $CH_2$-Gruppen durch O ersetzt sein können, oder Cyc,

Alk Alkenyl mit 2, 3, 4, 5 oder 6 C-Atomen

Cyc unsubstituiertes oder ein-, zwei- oder dreifach durch NHCOA, $NHSO_2$, OH, OA, A, $NH_2$, NHA, $NA_2$, COOA, COOH und/oder CONHA substituiertes cyclisches Alkyl mit 3-7 C-Atomen,

Hal F, Cl, Br oder I,

m 1, 2, 3 oder 4,

n 0, 1, 2, 3 oder 4,

p 1, 2 oder 3,

bedeuten,
sowie ihre pharmazeutisch verwendbaren Salze, Tautomere und Stereoisomere, einschließlich deren Mischungen in allen Verhältnissen,
wobei die folgenden Verbindungen ausgenommen sind:

3-Hydroxypiperidin-3-carbonsäure-methylester,
3-Hydroxypiperidin-1-carbonsäure-tert.-butylester-3-carbonsäure-methylester,
3-Allyl-3-hydroxypyrrolidin-1-carbonsäure-tert.-butylester,
3-Allyl-3-hydroxypiperidin-1-carbonsäure-tert.-butylester.

Der Erfindung lag die Aufgabe zugrunde, neue Verbindungen mit wertvollen Eigenschaften aufzufinden, insbesondere solche, die zur Herstellung von Arzneimitteln verwendet werden können.
Es wurde gefunden, daß die Verbindungen der Formel I und ihre Salze bei guter Verträglichkeit sehr wertvolle pharmakologische Eigenschaften besitzen.
Insbesondere zeigen sie eine regulatorische, modulatorische und/oder inhibierende Wirkung auf Metallproteasen, vorzugsweise auf die Methionin-Amino-Peptidase (MetAP), besonders auf den Subtyp MetAP-2.
Sie können als Arzneimittel gegen Krebs aber auch als Arzneimittel, die den Fett-Stoffwechsel positiv beeinflussen, aber auch als Arzneimittel gegen Entzündungen verwendet werden.
Es wurde gefunden, dass das S-Enantiomer der erfindungsgemäßen Verbindungen deutlich aktiver gegen MetAP-2 ist, als das Spiegelbild (R-Enantiomer).
Andere hydroxy-substituierte Pyrrolidinone kennt man aus:

Zeitschrift für Naturforschung, B: Chemical Sciences (1994), 49(11), 1586-95;
Analytica Chimica Acta (1987), 202, 167-74;
Journal of Electroanalytical Chemistry and Interfacial Electrochemistry (1988), 239(1-2), 161-73;
Zeitschrift fuer Naturfor.Teil B: Anorg. Chem. Org. Chem (1978), 33B(12), 1540-6;
J. Chem. Soc. (1965), (Oct.), 5556-62;
J. Chem. Soc. (1965), (Oct.), 5551-6.

Andere Pyrrolidinderivate sind als METAP-2 Inhibitoren in der WO 2010/003475 A2 beschrieben.
In WO 2012/033956 A1 sind Hydroxypiperidin-derivate beschrieben, die als Zwischenverbindungen zur Herstellung von Muscarin-Agonisten verwendet werden.
A.I. Moskalenko et al., Russian Journal of Organic Chemistry, 2011, Vol. 47, No. 7, pp. 1091-1096, beschreibt 3-Hydroxypyrrolidin-derivate, die als Zwischenverbindungen zur Herstellung spirocyclischer Tetrahydrofuranderivate verwendet werden.
Die Entwicklung von MetAP-2 Inhibitoren bei der Behandlung von Krebs ist von S.-Q. Yin et al. in Current Medicinal Chemistry, 2012, 19, 1021-1035, beschrieben.
In der WO 01/79157 sind substituierte Hydrazide und N-Alkoxyamide beschrieben, die MetAP-2 inhibitorische Aktivität aufweisen und zur Inhibierung von Angiogenese verwendet werden können, insbesondere zur Behandlung von Krankheiten, wie z.B. Krebs, deren Entwicklung von Angiogenese abhängt.
In der WO 02/081415 sind MetAP-2 Inhibitoren beschrieben, die zur Behandlung von Krebs, Hämangiom, proliferativer Retinopathie, rheumatoider Arthritis, atherosklerotischer Neovaskularisation, Psoriasis, okularer Neovaskularisation und Fettleibigkeit verwendet werden können.
In der WO 2008/011114 sind Verbindungen als Angiogenese-Inhibitoren und MetAP-2-Inhibitoren beschrieben, die zur Behandlung von lymphoider Leukämie und Lymphom verwendet werden können.
Die Wirkung der erfindungsgemäßen Verbindungen gegen Krebs liegt besonders in ihrer Wirkung gegen Angiogenese. Angiogenese-Hemmung hat sich bei über 70 Krankheiten als hilfreich erwiesen, wie z. B. Eierstockkrebs (F. Spinella et al. J. Cardiovasc. Pharmacol. 2004, 44, S140), Brustkrebs (A. Morabito et al. Crit. Rev. Oncol./Hematol. 2004, 49, 91), Prostatakrebs (B. Nicholson et al. Cancer Metastas. Rev. 2001, 20, 297), diabetische Erblindung, Schuppenflechte und Makuladegeneration (E. Ng et al. Can. J. Ophthalmol. 2005, 23, 3706).
Proteasen regulieren viele unterschiedliche Zell-Prozesse, besonders die Modulation von Peptiden und Proteinen, besonders den Protein-Umsatz, die Protein-Reifung und Signalpeptid-Prozessierung, den Abbau von anormalen Proteinen sowie die In-/Aktivierung von regulatorischen Proteinen. Besonders die Amino-terminale Modifikation von naszierenden Polypeptiden stellt die häufigste Modulation dar. Aminoproteasen sind Metalloproteasen, die Aminosäuren vom ungeschützten N-Terminus von Peptiden oder Proteinen abspalten, was sowohl co- als auch posttranslatorisch erfolgen kann. Methionin Aminopeptidase (MetAP) spaltet terminales Methionin naszierender Peptide besonders, wenn die vorletzte Aminosäure klein und ungeladen ist (z. B. Gly, Ala, Ser, Thr, Val, Pro oder Cys).
Bei vielen Krankheitsprozessen steht die Angiogenese entweder ursächlich im Mittelpunkt der Erkrankung oder wirkt sich verschlimmernd auf die Progression der Erkrankung aus. Beispielsweise im Krebsgeschehen führt die Angiogenese dazu, dass der Tumor sich vergrößern und in andere Organe übertreten kann. Weitere Erkrankungen, bei denen Angiogenese eine wichtige Rolle spielt sind Psoriasis, Arthrose, Arteriosklerose sowie Augenerkrankungen wie diabetische Retinopathie, altersbedingte makulare Degeneration, Rubeosis iridis oder neovasculäres Glaukom, ferner bei Entzündungen. Die dieser Erfindung zugrunde liegenden Verbindungen der Formel I, Zusammensetzungen, die

diese Verbindungen enthalten, sowie die beschriebenen Verfahren können somit zur Behandlung dieser Krankheiten eingesetzt werden.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome der Lungen, des Pankreas, der Schilddrüse, der Harnblase oder des Kolons, myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen.

Es kann gezeigt werden, dass die erfindungsgemäßen Verbindungen anticancerogene Wirkung aufweisen. Die erfindungsgemäßen Verbindungen werden an einen Patienten mit einer Erkrankung verabreicht, z. B. zur Inhibierung des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt. Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es wurde gefunden, dass die erfindungsgemäßen Verbindungen eine spezifische Inhibierung der MetAP-2 bewirken. Die erfindungsgemäßen Verbindungen zeigen bevorzugt eine vorteilhafte biologische Aktivität, die in den, zum Beispiel hierin beschriebenen Tests nachweisbar ist. In derartigen Tests zeigen und bewirken die erfindungsgemäßen Verbindungen einen inhibierenden Effekt, der gewöhnlich durch $IC_{50}$-Werte in einem geeigneten Bereich, bevorzugt im mikromolaren Bereich und bevorzugter im nanomolaren Bereich dokumentiert wird.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Die erfindungsgemäßen Verbindungen können auch zur Behandlung von Fettleibigkeit (Obesitas) verwendet werden. Henri R. Lijnen et al. beschreibt in Obesity, Vol.18 no.12, 2241-2246 (2010) die Verwendung von Fumagillin, einem Met-AP2-Inhibitor, bei der Reduzierung von adipösem Gewebe.
Die Verwendung von Met-AP2-Inhibitoren (Verbindungen vom Fumagillin-Typ) zur Behandlung von Obesitas ist auch in der WO 2011/085201 A1 beschrieben.

Die erfindungsgemäßen Verbindungen können auch zur Behandlung von Malaria verwendet werden. X. Chem et al. beschreibt in Chemistry & Biology, Vol.16, 193-202 (2009) die Verwendung von Fumagillin, einem Met-AP2-Inhibitor, zur Behandlung von Malaria.

Die erfindungsgemäßen Verbindungen können auch zur Behandlung von gutartiger Prostatahypertrophie verwendet werden.
Die Verwendung von Met-AP2-Inhibitoren (Verbindungen vom Fumagillin-Typ) zur Behandlung von gutartiger Prostatahypertrophie ist in der WO 2011/085198 A1 beschrieben.

Unter Verbindungen der Formel I versteht man auch die Hydrate und Solvate dieser Verbindungen.
Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Die Erfindung umfaßt selbstverständlich auch die Solvate der Salze der Verbindungen der Formel I, wie z.B. das Hydrochlorid Hydrat.
Unter pharmazeutisch verwendbaren Derivaten versteht man z.B. die Salze der erfindungsgemäßen Verbindungen.
Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.
Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.
Gegenstand der Erfindung sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man

a) zur Herstellung von Verbindungen der Formel I, worin Y CO und R $NR^2R^4$ bedeuten,
eine Verbindung der Formel II

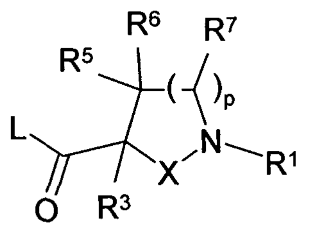

II
I

worin X, $R^1$, $R^3$, $R^5$, $R^6$, $R^7$ und p die in Anspruch 1 angegebenen Bedeutungen haben,
und L Cl, Br, I oder eine freie oder reaktionsfähig funktionell abgewandelte OH-Gruppe bedeutet,
mit einer Verbindung der Formel III

$R^2$-$NHR^4$ III

worin $R^2$ und $R^4$ die in Anspruch 1 angegebenen Bedeutungen haben, umsetzt,
oder

b) eine Verbindung der Formel IV

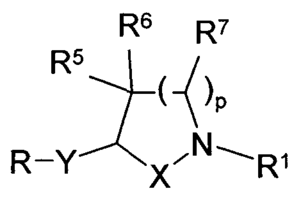

IV

worin $R^1$, $R^5$, $R^6$, $R^7$, R, X, Y und p die in Anspruch 1 angegebenen Bedeutungen haben,

oxidiert,
oder

c) zur Herstellung von Verbindungen der Formel I, worin
X und Y $CH_2$ bedeuten,
eine Verbindung der Formel I, worin X und Y CO bedeuten,
reduziert,
und/oder eine Base oder Säure der Formel I in eines ihrer Salze umwandelt.

Vor- und nachstehend haben die Reste $R^1$, $R^3$, $R^5$, $R^6$ , $R^7$, R, X, Y und p die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
Vorzugsweise bedeutet A unverzweigtes oder verzweigtes Alkyl mit 1-6 C-Atomen, worin 1-7 H-Atome durch F und/oder Cl ersetzt sein können, und/oder worin eine oder zwei nicht-benachbarte CH- und/oder $CH_2$-Gruppen durch O ersetzt sein können
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cyclisches Alkyl bedeutet vorzugsweise Cyclopropyl, Cyclobutyl, Cylopentyl, Cyclohexyl oder Cycloheptyl.

R bedeutet vorzugsweise $NR^2R^4$, ferner Alk, $C(=CH_2)[C(R^4)_2]_nAr^2$ oder $Het^2$. R bedeutet besonders bevorzugt $NR^2R^4$, ganz besonders bevorzugt $NHCH_2Ar^2$.

X bedeutet vorzugsweise CO, ferner $CH_2$.
Y bedeutet vorzugsweise CO, ferner $CH_2$.
$R^1$ bedeutet vorzugsweise $[C(R^4)_2]_nAr^1$, $(CH_2)_nHet$ oder $(CH_2)_nCyc$, ferner $[C(R^4)_2]_nCOOH$, $[C(R^4)_2]_nCONHAr^1$, $[C(R^4)_2]_nCONH_2$, $[C(R^4)_2]_nNHA$ oder $[C(R^4)_2]_nNA_2$.

$R^4$ bedeutet vorzugsweise H, Methyl, Ethyl oder Propyl, ganz besonders bevorzugt H oder Methyl.

$Ar^1$ bedeutet z.B. Phenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Aminocarbonylphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl oder 2,5-Difluor-4-bromphenyl.

$Ar^2$ bedeutet z.B. Phenyl, o-, m- oder p-Tolyl, o-, m- oder p-Ethylphenyl, o-, m-oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Aminocarbonylphenyl,
weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl.
$Ar^2$ bedeutet weiterhin besonders bevorzugt ein- oder zweifach durch Hal substituiertes Phenyl.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.

Unsubstituiertes Het kann also z. B. auch bedeuten 2,3-Dihydro-2-, -3-, -4-oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4-oder -5-pyrazolyl, Tetrahydro-1-, -3- oder -4-pyrazolyl, 1,4-Dihydro-1-, -2-, -3-oder -4-pyridyl,

1,2,3,4-Tetrahydro-1-, -2-, -3-, -4-, -5- oder -6-pyridyl, 1-, 2-, 3-oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet weiterhin vorzugsweise unsubstituiertes oder ein-, zwei- oder dreifach durch Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O und/oder $Ar^3$ substituiertes Pyrazinyl, Pyrazolyl, Benzimidazolyl, Pyridyl, Indolyl, Dihydro-indolyl, Benzofuranyl, Tetrahydropyranyl, Dihydrochinolinyl, Dihydroisochinolinyl, Tetrahydrochinolinyl, Tetrahydroisochinolinyl, Indazolyl, Imidazolyl, Pyrrolyl, Oxazolyl, Oxadiazolyl, Isoxazolyl, Benzothiazolyl, Piperidin-1-yl, Pyrrolidin-1-yl, 3,4-Dihydro-2H-pyrido[3,2-b][1,4]oxazinyl, 3,4-Dihydro-2H-benzo[1,4]oxazinyl, Benzofuranyl, Azetidinyl, 3-Azabicylo[3.2.0]hexyl, Pyrrolo[2,3-b]pyridinyl, Tetrahydrofuranyl, Tetrahydro-[1,8]naphthyridinyl, 2,3-Dihydro-benzo-isothiazolyl, 1,2,3,4-Tetrahydro-benzothiazinyl oder Hexahydro-benzo[1,3]dioxolyl.

$Het^1$ bedeutet vorzugsweise unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind. Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ic ausgedrückt werden, die der Formel I entsprechen und worin die nicht näher bezeichneten Reste die bei der Formel I angegebene Bedeutung haben, worin jedoch

| | |
|---|---|
| in Ia Het | unsubstituiertes oder ein-, zwei- oder dreifach durch Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O und/oder $Ar^3$ substituiertes Pyrazinyl, Pyrazolyl, Benzimidazolyl, Pyridyl, Indolyl, Dihydro-indolyl, Benzofuranyl, Tetrahydropyranyl, Dihydrochinolinyl, Dihydroisochinolinyl, Tetrahydro-chinolinyl, Tetrahydroisochinolinyl, Indazolyl, Imidazolyl, Pyrrolyl, Oxazolyl, Oxadiazolyl, Isoxazolyl, Benzothiazolyl, Piperidin-1-yl, Pyrrolidin-1-yl, 3,4-Dihydro-2H-pyrido[3,2-b][1,4]oxazinyl, 3,4-Dihydro-2H-benzo[1,4]oxazinyl, Benzofuranyl, Azetidinyl, 3-Aza-bicylo[3.2.0]hexyl, Pyrrolo[2,3-b]pyridinyl, Tetrahydrofuranyl, Tetrahydro-[1,8]naphthyridinyl, 2,3-Dihydro-benzo-isothiazolyl, 1,2,3,4-Tetrahydro-benzo-thiazinyl oder Hexahydro-benzo[1,3]dioxolyl bedeutet; |
| in Ib $Het^1$ | unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinylbedeutet; |
| in Ic R | $NR^2R^4$, Alk, $C(=CH_2)[C(R^4)_2]_nAr^2$, $Het^2$, $O[C(R^4)_2]_nAr^2$ oder OA, |
| X | CO oder $CH_2$, |
| Y | CO oder $CH_2$, |
| $R^1$ | H, $[C(R^4)_2]_nAr^1$, $(CH_2)_nHet$, $(CH_2)_nCyc$, $[C(R^4)_2]_nCOOH$, $[C(R^4)_2]_nCONHAr^1$, $[C(R^4)_2]_nCONH_2$, $[C(R^4)_2]_nNHA$, $[C(R^4)_2]_nNA_2$, $O[C(R^4)_2]_nAr^1$, $[C(R^4)_2]_nOR^7$, $[C(R^4)_2]_nCOO(CH_2)_nAr^1$, $[C(R^4)_2]_nCOOA$, $[C(R^4)_2]_nCONH[C(R^4)_2]_pCON(R^4)_2$ oder $[C(R^4)_2]_nCONHCR^4[(CH^2)_nN(R^4)_2]CON(R^4)_2$, |
| $R^2$ | H, $[C(R^4)_2]_nAr^2$, $(CH_2)_nCOHet^1$, $(CH_2)_nCOAr^2$, $(CH_2)_mNA_2$ oder $(CH_2)_nHet$, |
| $R^3$ | OH oder OCOA, |
| $R^4$ | H oder Alkyl mit 1, 2, 3 oder 4 C-Atomen, |
| $R^2$ und $R^4$ | zusammen auch Alkylen mit 2, 3, 4 oder 5 C-Atomen, wobei eine $CH_2$-Gruppe auch durch $N(CH_2)_mOH$ oder $SO_2$ ersetzt sein kann, |
| $R^5$, $R^6$ | jeweils unabhängig voneinander H oder A, |
| $R^5$ und $R^6$ | zusammen auch Alkylen mit 2, 3, 4 oder 5 C-Atomen, wobei eine $CH_2$-Gruppe auch durch NCOA oder O ersetzt sein kann, |

| | |
|---|---|
| $R^7$ | H oder A, |
| $Ar^1$ | unsubstituiertes oder ein-, zwei-, drei-, vier- oder fünffach durch Hal, OH, OA, $CONH_2$, CONHA, $CONA_2$, $NHSO_2A$, CONHCyc, $NHSO_2Cyc$, $CONHAr^2$, $COHet^1$ und/oder $NASO_2A$ substituiertes Phenyl, |
| $Ar^2$ | unsubstituiertes oder ein-, zwei-, drei-, vier- oder fünffach durch Hal, A, $CONH_2$, und/oder $OAr^3$ substituiertes Phenyl, |
| $Ar^3$ | unsubstituiertes oder einfach durch $NH_2$ substituiertes Phenyl, |
| Het | unsubstituiertes oder ein-, zwei- oder dreifach durch Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O und/oder $Ar^3$ substituiertes Pyrazinyl, Pyrazolyl, Benzimidazolyl, Pyridyl, Indolyl, Dihydro-indolyl, Benzofuranyl, Tetrahydropyranyl, Dihydrochinolinyl, Dihydroisochinolinyl, Tetrahydro-chinolinyl, Tetrahydroisochinolinyl, Indazolyl, Imidazolyl, Pyrrolyl, Oxazolyl, Oxadiazolyl, Isoxazolyl, Benzothiazolyl, Piperidin-1-yl, Pyrrolidin-1-yl, 3,4-Dihydro-2H-pyrido[3,2-b][1,4]oxazinyl, 3,4-Dihydro-2H-benzo[1,4]oxazinyl, Benzofuranyl, Azetidinyl, 3-Aza-bicylo[3.2.0]hexyl, Pyrrolo[2,3-b]pyridinyl, Tetrahydrofuranyl, Tetrahydro-[1,8]naphthyridinyl, 2,3-Dihydro-benzo-isothiazolyl, 1,2,3,4-Tetrahydro-benzo-thiazinyl oder Hexahydro-benzo[1,3]dioxolyl, |
| $Het^1$ | unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl, |
| $Het^2$ | Isoindolyl, |
| A | unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können, und/oder worin eine oder zwei nicht-benachbarte CH- und/oder $CH_2$-Gruppen durch O ersetzt sein können, oder Cyc, |
| Alk | Alkenyl mit 2, 3, 4, 5 oder 6 C-Atomen |
| Cyc | unsubstituiertes oder ein-, zwei- oder dreifach durch NHCOA, $NHSO_2$, OH, OA, A, $NH_2$, NHA, $NA_2$, COOA, COOH und/oder CONHA substituiertes cyclisches Alkyl mit 3-7 C-Atomen, |
| Hal | F, Cl, Br oder I, |
| m | 1, 2, 3 oder 4, |
| n | 0, 1, 2, 3 oder 4, |
| p | 1, 2 oder 3, |

bedeuten;
sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Her- stellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man Verbindungen der Formel II mit einer Verbindung der Formel III umsetzt. Die Verbindungen der Formel II und der Formel III sind in der Regel bekannt. Sind sie neu, so können sie aber nach an sich bekannten Methoden hergestellt werden.

In den Verbindungen der Formel II bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Die Reaktion gelingt vorzugsweise in Gegenwart eines Dehydratisierungsmittels, wie z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid T3P (vgl. Angew. Chem. 92, 129 (1980)), Diphenylphosphorylazid oder 2-Ethoxy-N-ethoxycarbonyl-1,2-dihydrochinolin, gegebenenfalls in Gegenwart von N-Hydroxybenzotriaol;

T3P = 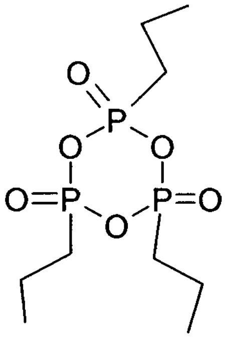 .

Die Umsetzung erfolgt in einem inerten Lösungsmittel und erfolgt in der Regel in Gegenwart eines säurebindenden Mittels vorzugsweise einer organischen Base wie DIPEA, Triethylamin, Dimethylanilin, Pyridin oder Chinolin. Auch der Zusatz eines Alkali- oder Erdalkalimetall-hydroxids, -carbonats oder - bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -15° und 150°, normalerweise zwischen 40° und 130°, besonders bevorzugt zwischen 60° und 110°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt sind Glykolether, wie Ethylenglycolmonomethylether, THF, Dichlormethan und/oder DMF.

Verbindungen der Formel I können weiterhin vorzugsweise erhalten werden, indem man Verbindungen der Formel IV oxidiert.
Die Oxidation erfolgt vorzugsweise mit tert.-Butylhydroperoxid.
Die Reaktionszeit liegt je nach den angewendeten Bedingungen zwischen einigen Minuten und 14 Tagen, die Reaktionstemperatur zwischen etwa -15° und 150°, normalerweise zwischen 40° und 130°, besonders bevorzugt zwischen 60° und 110°C.
Als Lösungsmittel ist Wasser bevorzugt, wobei der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums, bevorzugt ist.

Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat,

Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$-$C_4$) Alkylhalogeniden, z.B. Methyl-, Ethyl-, Isopropyl- und tert.-Butylchlorid, -bromid und -iodid; Di($C_1$-$C_4$)Alkylsulfaten, z.B. Dimethyl-, Diethyl- und Diamylsulfat; ($C_{10}$-$C_{18}$)Alkylhalogeniden, z.B. Decyl-, Dodecyl-, Lauryl-, Myristyl- und Stearylchlorid, -bromid und -iodid; sowie Aryl-($C_1$-$C_4$)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I

und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im

Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von

(a) einer wirksamen Menge an einer Verbindung der Formel I und/oder ihrer pharmazeutisch verwendbaren Salze und Stereoisomere, einschließlich deren Mischungen in allen Verhältnissen,
und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen, und einer wirksamen Menge eines weiteren Arzneimittelwirkstoffs gelöst oder in lyophilisierter Form vorliegt.

Gegenstand der Erfindung sind die Verbindungen der Formel I gemäß Anspruch 1-5, sowie ihrer pharmazeutisch verwendbaren Salze, Tautomeren und Stereoisomeren, einschließlich deren Mischungen in allen Verhältnissen, zur Verwendung zur Behandlung von Tumoren, Tumormetastasen, proliferativer Erkrankungen der Mesangiumzellen, Hämangiom, proliferativer Retinopathie, rheumatoider Arthritis, atherosklerotischer Neovaskularisation, Psoriasis, okularer Neovaskularisation, Osteoporose, Diabetes und Fettleibigkeit, lymphoider Leukämie, Lymphom, Malaria und Prostatahypertrophie.

<u>Isotope</u>

Es ist weiterhin vorgesehen, daß eine Verbindung der Formel I isotopenmarkierte Formen davon umfaßt. Eine isotopenmarkierte Form einer Verbindung der Formel I ist mit dieser Verbindung bis auf die Tatsache, daß eines oder mehrere Atome der Verbindung durch ein Atom bzw. Atome mit einer Atommasse oder Massenzahl ersetzt wurden, die sich von der Atommasse oder Massenzahl des Atoms, das üblicherweise natürlich vorkommt, unterscheidet, identisch. Zu den Isotopen, die leicht im Handel erhältlich sind und in eine Verbindung der Formel I nach gut bekannten Verfahren eingebaut werden können, zählen zum Beispiel Isotope von Wasserstoff, Kohlenstoff, Stickstoff, Sauerstoff, Phosphor, Fluor und Chlor, z.B. $^{2}H$, $^{3}H$, $^{13}C$, $^{14}C$, $^{15}N$, 180, $^{17}O$, $^{31}P$, $^{32}P$, $^{35}S$, $^{18}F$ bzw. $^{36}Cl$. Eine Verbindung der Formel I oder eines ihrer pharmazeutisch unbedenklichen Salze davon, die eines oder mehrere der oben genannten Isotope und/oder andere Isotope von anderen Atomen enthält, ist als Bestandteil der vorliegenden Erfindung vorgesehen. Eine isotopenmarkierte Verbindung der Formel I läßt sich auf vielerlei nützliche Art verwenden. Zum Beispiel eignet sich eine isotopenmarkierte Verbindung der Formel I, in die z.B. ein Radioisotop wie $^{3}H$ oder $^{14}C$ eingebaut worden ist, für Assays zur Verteilung des Arzneistoffs und/oder Substratgewebes. Diese Radioisotope, d.h. Tritium ($^{3}H$) und Kohlenstoff-14 ($^{14}C$), sind aufgrund ihrer einfachen Herstellung und ausgezeichneten Nachweisbarkeit besonders bevorzugt. Der Einbau schwererer Isotope, z.B. Deuterium ($^{2}H$), in eine Verbindung der Formel I weist therapeutische Vorteile aufgrund der höheren Stabilität dieser isotopenmarkierten Verbindung im Metabolismus auf. Höhere Stabilität in Metabolismus bedeutet unmittelbar eine erhöhte Halbwertszeit in vivo oder niedrigere Dosierungen, was unter den meisten Umständen eine bevorzugte Ausführungsform der vorliegenden Erfindung darstellen würde. Eine isotopenmarkierte Verbindung der Formel I läßt sich üblicherweise durch Durchführung der in den Syntheseschemata und der damit in Zusammenhang stehenden Beschreibung, im Beispielteil und im Herstellungsteil im vorliegenden Text offenbarten Vorgehensweisen herstellen, wobei ein nicht isotopenmarkierter Reaktionspartner durch einen leicht verfügbaren isotopenmarkierten Reaktionspartner ersetzt wird.

Zur Manipulation des oxidativen Metabolismus der Verbindung über den primären kinetischen Isotopeneffekt kann auch Deuterium ($^2H$) in eine Verbindung der Formel I eingebaut werden. Beim primären kinetischen Isotopeneffekt handelt es sich um eine Veränderung der Geschwindigkeit einer chemischen Reaktion aufgrund des Austausches isotopischer Kerne, was wiederum durch die Änderung der für die Bildung kovalenter Bindungen im Anschluß an diesen isotopischen Austausch erforderlichen Grundzustandsenergien verursacht wird. Der Austausch eines schwereren Isotops führt üblicherweise zu einer Erniedrigung der Grundzustandsenergie für eine chemische Bindung und verursacht so eine Verringerung der Geschwindigkeit bei einem geschwindigkeitslimitierenden Bindungsbruch. Findet der Bindungsbruch an bzw. in der Nähe einer Sattelpunktregion entlang der Koordinate einer Reaktion mit mehreren Produkten statt, so können sich die Produktverteilungsverhältnisse stark ändern. Zur Erläuterung: Wird Deuterium an ein Kohlenstoffatom in einer nichtaustauschbaren Position gebunden, so sind Geschwindigkeitsunterschiede von $k_M/k_D$ = 2-7 typisch. Wird dieser Geschwindigkeitsunterschied erfolgreich auf eine oxidationsanfällige Verbindung der Formel I angewandt, so kann sich dadurch das Profil dieser Verbindung in vivo drastisch ändern und zu verbesserten pharmakokinetischen Eigenschaften führen.

Bei der Entdeckung und Entwicklung von Therapeutika versucht der Fachmann, pharmakokinetische Parameter zu optimieren und gleichzeitig wünschenswerte In-vitro-Eigenschaften beizubehalten. Man kann vernünftig annehmen, daß viele Verbindungen mit schlechten pharmakokinetischen Profilen gegenüber dem oxidativen Metabolismus anfällig sind. Aus derzeitig verfügbaren In-vitro-Assays mit Lebermikrosomen erhält man wertvolle Informationen über den Verlauf dieses oxidativen Metabolismus, aufgrund dessen wiederum deuterierte Verbindungen der Formel I mit einer verbesserten Stabilität durch Resistenz gegenüber einem derartigen oxidativen Metabolismus rational gestaltet werden können. So gelangt man zu wesentlichen Verbesserungen der pharmakokinetischen Profile der Verbindungen der Formel I, die sich quantitativ als erhöhte In-vivo-Halbwertszeit (T/2), Konzentration bei maximaler therapeutischer Wirkung ($C_{max}$), Fläche unter der Dosis-Wirkungskurve (AUC) sowie F und als verringerte Clearance, Dosis und Materialkosten ausdrücken lassen.

Zur Veranschaulichung des Obigen soll folgendes dienen: eine Verbindung der Formel I mit mehrfachen potentiellen Angriffsstellen für den oxidativen Metabolismus, z.B. Wasserstoffatome an einem Benzylrest und Wasserstoffatome, die an ein Stickstoffatom gebunden sind, wird als Reihe von Analogen hergestellt, in denen verschiedene Kombinationen von Wasserstoffatomen durch Deuteriumatome ersetzt werden, so daß einige, die meisten oder alle dieser Wasserstoffatome durch Deuteriumatome ersetzt sind. Durch Bestimmungen der Halbwertszeit gelangt man zu einer günstigen und genauen Bestimmung, wie sehr sich die Verbesserung der Widerstandsfähigkeit gegenüber oxidativen Metabolismen verbessert hat. Auf diese Weise wird bestimmt, daß aufgrund eines derartigen Austausches von Wasserstoff gegen Deuterium die Halbwertszeit der Ausgangsverbindung um bis zu 100% verlängert werden kann.

Der Austausch von Wasserstoff gegen Deuterium in einer Verbindung der Formel I läßt sich auch dazu verwenden, um zu einer günstigen Änderung des Stoffwechselproduktspektrums der Ausgangsverbindung zwecks Verringerung oder Ausschluß von unerwünschten toxischen Stoffwechselprodukten zu gelangen. Entsteht zum Beispiel ein toxisches Stoffwechselprodukt aufgrund der Spaltung einer oxidativen Kohlenstoff-Wasserstoff (C-H)-Bindung kann vernünftigerweise angenommen werden, daß das deuterierte Analog die Produktion des unerwünschten Stoffwechselprodukts wesentlich verringert oder ausschließt, sogar dann, wenn es sich bei der jeweiligen Oxidation nicht um einen geschwindigkeitsbestimmenden Schritt handelt. Weitere Informationen zum Stand der Technik in bezug auf den Austausch von Wasserstoff gegen Deuterium finden sich z.B. bei Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al., Biochemistry 33(10), 2927-2937, 1994, und Jarman et al., Carcinogenesis 16(4), 683-688, 1993.

## VERWENDUNG

Die vorliegenden Verbindungen eignen sich als pharmazeutische Wirkstoffe für Säugetiere, insbesondere für den Menschen, bei der Behandlung und Bekämpfung von Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen), sowie proliferative Erkrankungen der Mesangiumzellen.

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Tumoren, Tumorerkrankungen und/oder Tumormetastasen.

Die Tumorerkrankung ist vorzugsweise ausgewählt aus der Gruppe Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung von Osteoporose, Diabetes und Fettleibigkeit.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.

Die angiogene Erkrankung ist vorzugsweise ausgewählt aus der Gruppe diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

Die proliferative Erkrankung der Mesangiumzellen ist vorzugsweise ausgewählt aus der Gruppe Glomerulonephritis, diabetische Nephropathie, maligne Nephrosclerose, thrombotisches Mikroangiopathie-Syndrom, Transplantatabstossung, Glomerulopathie.

Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Die inflammatorische Erkrankung ist vorzugsweise ausgewählt aus der Gruppe entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis, durch T-Zellen - vermittelte Immunerkrankung.

Die entzündliche Darmerkrankung ist vorzugsweise ausgewählt aus der Gruppe ulcerative Colitis, Morbus Crohn, unbestimmte Colitis.

Die durch T-Zellen - vermittelte Immunerkrankung ist vorzugsweise ausgewählt aus der Gruppe allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

Die Arthritis-Erkrankung ist vorzugsweise ausgewählt aus der Gruppe rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

Die entzündliche Nierenerkrankung ist vorzugsweise ausgewählt aus der Gruppe Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

Die entzündliche Hauterkrankung ist vorzugsweise ausgewählt aus der Gruppe Psoriasis, atopische Dermatitis, Kontaktempfindlichkeit, Akne.

Ebenfalls umfasst ist die Verwendung der Verbindungen der Formel I und/oder ihre physiologisch unbedenklichen Salze und Solvate zur Herstellung eines Arzneimittels zur Behandlung oder Vorbeugung einer Krankheit bzw. eines Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.

Ebenfalls umfasst ist die Verwendung der Verbindungen der Formel I und/oder ihre physiologisch unbedenklichen Salze zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die Verbindungen der Formel I können auch gemeinsam mit anderen gut bekannten Therapeutika, die aufgrund ihrer jeweiligen Eignung für das behandelte Leiden ausgewählt werden, verabreicht werden.

Die vorliegenden Verbindungen eignen sich auch zur Kombination mit bekannten Antikrebsmitteln. Zu diesen bekannten

Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll. "Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5$\alpha$-Reduktase-Hemmer, Nilutamid, Flutamid, Bicalutamid, Liarozol und Abirateron-acetat. "Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, $\alpha$-Difluormethylornithin, ILX23-7553, trans-N-(4'-Hydroxyphenyl)retinamid und N-4-Carboxyphenylretinamid.

"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkalierende Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.

Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797. Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virus-vermittelten Gentransfer abgegeben werden können (siehe z.B. US-Patent Nr. 6,069,134).

**Wirkungsnachweis von pharmakologischen Inhibitoren auf die Proliferation/Vitalität von Tumorzellen *in vitro***

## 1.0 Hintergrund

In der vorliegenden Versuchsbeschreibung wird die Hemmung der Tumorzellproliferation/ Tumorzellvitalität durch Wirkstoffe beschrieben.
Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

## 2.0 Versuchsdurchführung

### 2.1 Zellkultur

Beispielsweise käuflich erhältliche Colon-Carcinom-Zelllinien, Zelllinien des Eierstocks, Zellinien der Prostata oder Zelllinien der Brust etc.
Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% $CO_2$ kultiviert.

### 2.2. Aussaat der Zellen

Eine definierte Zellzahl (z.B. 2000 Zellen) werden pro Kultur/ well in einem Volumen von 180µl Kulturmedium in Mikrotiterplatten (96 weil Zellkulturplatten) mit einer Mehrkanalpipette ausgesät. Die Zellen werden anschließend in einem CO2-Brutschrank (37°C und 10% CO2) kultiviert.

### 2.3. Zugabe der Testsubstanzen

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungs-stufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20µl Substanzlösung in die Kulturen/wells gegeben. Die Zellen werden für weitere 4 Tage bei 37°Celsius und 10% $CO_2$ kultiviert.

### 2.4. Messung der Farbreaktion

Pro weil werden jeweils 20µl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

## 3. Auswertung

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt:
Rechnung:

$$\frac{100 * (\text{Wert mit Zellen und Testsubstanz - Wert der Mediumkontrolle})}{(\text{Wert mit Zellen - Wert der Mediumkontrolle})}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

| Material | Best. Nr. | Hersteller |
|---|---|---|
| Mikrotiterplatten für die Zellkultur (Nunclon Surface 96well Plate) | 167008 | Nunc |
| DMEM | P04-03550 | Pan Biotech |
| PBS (10x) Dulbecco | 14200-067 | Gibco |
| 96well Platten (Polypropylen) | 267334 | Nunc |
| AlamarBlue™ | BUF012B | Serotec |
| FCS | 1302 | Pan Biotech GmbH |
| Trypsin/EDTA Solution 10x | L 2153 | Biochrom AG |
| 75cm$^2$ Kulturflaschen | 353136 | BD Falcon |
| A2780 | 93112519 | ECACC |
| Colo205 | CCL222 | ATCC |
| MCF7 | HTB22 | ATCC |
| PC3 | CRL-1435 | ATCC |

**Bestimmung der Proliferationshemmung durch Inhibitoren der Methioninaminopeptidase 2 im BrdU Proliferationstest (zellulärer Assay)**

Die Hemmung der Proliferation wird durch Inkorporation von Bromodesoxyuridin (BrdU) in humanen Endothelzellen aus der Nabelschnur (HUVEC, PromoCell, C-12200) bestimmt. Die HUVEC werden bei 37°C und 5% $CO_2$ in Basalmedium (PromoCell, C-22200) mit Supplementmix (PromoCell, C-39225) kultiviert. Nach Ablösung der Zellen mittels Trypsin/EDTA wird die Lebendzellzahl bestimmt und die Zellen in einer Dichte von 1000 Zellen pro Kavität in einem Gesamtvolumen von 175 µl ausgesät (Kavitäten werden zuvor entweder mit supplementiertem Kulturmedium für 1-2 Stunden bei 37°C oder mit 1,5% Gelatine für 0,5 - 2 Stunden bei 37°C beschichtet). Nach 24 stündiger Kultivierung werden die Testsubstanzen in verschiedenen Konzentrationen (z.B. finale Konzentrationen 30 µM bis 0,03 nM in 10-fach Verdünnungsschritten) und einem Volumen von 25 µl hinzugegeben. Die DMSO Konzentration wird mit 0,3% konstant gehalten. Nach insgesamt 48 oder 72 stündiger Kultivierung werden 20 µl Bromdesoxyuridin (Roche, # 11647229001 1:1000 verdünnt in Kulturmedium, Endkonzentration 10µM) hinzugegeben und für weitere 20 bis 24 Stunden kultiviert. Nach insgesamt 72 bzw. 96 Stunden Inkubation mit Testsubstanzen wird das Kulturmedium entfernt und ein immunhistochemischer Nachweis zur Detektion der BrdU-Inkorporation durchgeführt (BrdU ELISA, Roche, # 11647229001). Hierzu werden die Zellen für 30 min bei Raumtemperatur mit einem Fixativ behandelt und anschließend mit einem Peroxidase-markiertem anti-BrdU Antikörper (1:100 verdünnt in Antikörperverdünnungspuffer) für 60 min bei Raumtemperatur inkubiert. Nach dreimaligem Waschen mit 1-fach konzentriertem DPBS-Puffer (Gibco, # 14200) wird die enzymatische Umsetzung in TMB-Substratlösung initiiert. Die Farbentwicklung wird nach 15 min durch Zugabe von 25 µl einer 1 M Schwefelsäurelösung abgestoppt. Eine Bestimmung der optischen Dichte erfolgt innerhalb von 5 min durch Messung bei einer Wellenlänge von 450 nM. Als Kontrollen dienen Kavitäten mit DMSO-behandelten Zellen (100% Kontrolle) oder leere Kavitäten (Leerwert). Die Sensitivität dieses Tests gegenüber Inhibitoren der Methioninaminopeptidase wird durch Verwendung des Inhibitors Fumagillin überprüft und bestätigt.

**MetAP-2 Aktivitätsmessung**

Die MetAP-2 Aktivität wird durch eine Kopplung von enzymatischen Reaktionen nachgewiesen. Das Tripeptid Met-Arg-Ser (MAS) wird als Substrat eingesetzt. Das freigesetzte Methionin wird zunächst durch die L-Aminooxidase (AAO) zu $Met_{ox}$ und $H_2O_2$ umgesetzt. Im zweiten Schritt katalysiert die Peroxidase (POD) mt Hilfe des $H_2O_2$ die Oxidation des Leukofarbstoffs Dianisidin zu $Dianisidin_{ox}$, dessen Zunahme photometrisch bei 450 nm detektiert wird.
MetAP-2 Aktivität kann als Kinetik kontinuierlich aufgezeichnet werden. Das Reaktionsschema verdeutlicht, dass pro mol Methionen ein mol $Dianisidin_{ox}$ gebildet wird. Die MetAP-2 Enzymaktivität lässt sich deshalb direkt als $\Delta$ Absorption pro Zeiteinheit berechnen. Eine Qualifizierung der MetAP-2 Aktivität (mol Met/Zeiteinheit) ist mit Hilfe des $Dianisidin_{ox}$-Extinktionskoeffizienten möglich.

Die Extinktionsänderung pro Zeiteinheit wird graphisch dargestellt und eine Steigungsberechnung im visuell linearen Bereich der Reaktion durchgeführt. Die Aktivitäten der verbindungen sind in Tabelle 1 zusammengefasst.

Löslichkeitsmessung

Bestimmung nach "Shake flask solubility measurement"

| Eluentenherstellung: | |
|---|---|
| Eluent A: | 2 ml Diethylamin, zur Synthese + 1000 ml Methanol, LiChrosolv |
| Eluent B: | 5 g Ammoniumacetat, zur Analyse + 5 ml Methanol, LiChrosolv + 995 ml Reinstwasser |

Probenlösungsmittel:

Puffer: 3,954 g Natriumdihydrogenphosphat-Monohydrat + 6,024 g Natriumchlorid + 950 ml Reinstwasser mit 0,1 M NaOH oder 0,1 M HCl wird der pH-Wert eingestellt.

Probenvorbereitung:

Die Proben werden bei 37°C und 450 rpm 24 h lang geschüttelt.
Nach ca. 7h wird der pH Wert der Proben überprüft und gegebenenfalls nachgestellt.
Es wird auch kontrolliert, ob die Probe noch im Überschuss vorhanden ist.

Kurz vor Ende der 24h-Schüttelzeit werden die Proben nochmals auf pH-Wert und auf einen Niederschlag überprüft.

Reinstwasser Anlage: MilliQ Gradient, Millipore, Gerät: F3PN37462D

Schüttler: TiMix control, Bühler
Inkubationshaube: TH 15 Bühler
pH Meter: 766 Calimatic Knick Gerät: pH 1
pH Elektrode: InLab 423 Mettler
APCI-MS (atmospheric pressure chemical ionization - mass spectrometry) $(M+H)^+$.

Die racemischen Endprodukte der erfindungsgemäßen Verbindungen oder die racemischen Intermediate lassen sich über eine chirale HPLC oder SFC-Säule einfach und sowohl im analytischen als auch im präparativen Maßstab trennen.

LCMS:

Methode: A- 0.1% TFA in $H_2O$, B- 0.1% TFA in ACN: Flow - 2.0 ml/min.
Column: X Bridge C8 (50 x 4.6 mm.3.5 u) + ve mode

| Time | %B |
|---|---|
| 0 | 05 |
| 8.0 | 100 |
| 8.1 | 100 |
| 8.5 | 05 |
| 10 | 05 |

$

LC-MS-Methode: (Gerät: Agilent 1100 Series)
Säule: Chromolith Speed Rod RP18e-50-4.6
Flussrate: 2.4ml/min
Solvent A: Wasser + 0,05% HCOOH
Solvent B: Acetonitril + 0,04% HCOOH
WL: 220 nm
Gradient: 0-2.8 min: 4%B auf 100% B, 2.8-3.3 min: 100% B.

$$

Methode: A- 10 mM $NH_4HCO_3$, B- ACN: Flow -1.0 ml/min.
Column: X Bridge C8 (50 x 4.6 mm.3.5u) - ve mode

| Time | %B |
|---|---|
| 0 | 05 |
| 8.0 | 100 |
| 8.1 | 100 |
| 8.5 | 05 |
| 10 | 05 |

HPLC:

Methode: A- 0.1% TFA in $H_2O$, B- 0.1% TFA in ACN: Flow - 2.0 ml/min.
Column: X Bridge C8 (50 x 4.6 mm.3.5u) + ve mode

| Time | %B |
|---|---|
| 0 | 5 |
| 8.0 | 100 |
| 8.1 | 100 |
| 8.5 | 5 |
| 10 | 5 |

$$$

Methode: Isopropanol: Flow - 0.8 ml/min.
Run time: 20 min
Column: Chiralpak AD

1) Enantiomerentrennung:

Trennung auf Chiralcel OD-H mit n-Heptan/Ethanol = 70/30.
Die Substanz wird in 10 ml n-Heptan/EtOH = 1/1 gelöst und über 5x25cm Chiralcel OD- Säule mit 20 μm Material bei einem Fluß von 100mL/min n-Heptan/Ethanol = 70/30 getrennt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

F.: Schmelzpunkt
Massenspektrometrie (MS): EI (Elektronenstoß-Ionisation) $M^+$
FAB (Fast Atom Bombardment) $(M+H)^+$
ESI (Electrospray Ionization) $(M+H)^+$

APCI-MS (atmospheric pressure chemical ionization - mass spectrometry) $(M+H)^+$.

Syntheseschemata zur Herstellung von Verbindungen der Formel I:

Beispiel 1

Herstellung von 3-Hydroxy-5-methyl-2-oxo-1-phenyl-pyrrolidin-3-carbonsäure-3-chlor-5-fluor-benzylamid ("A119")

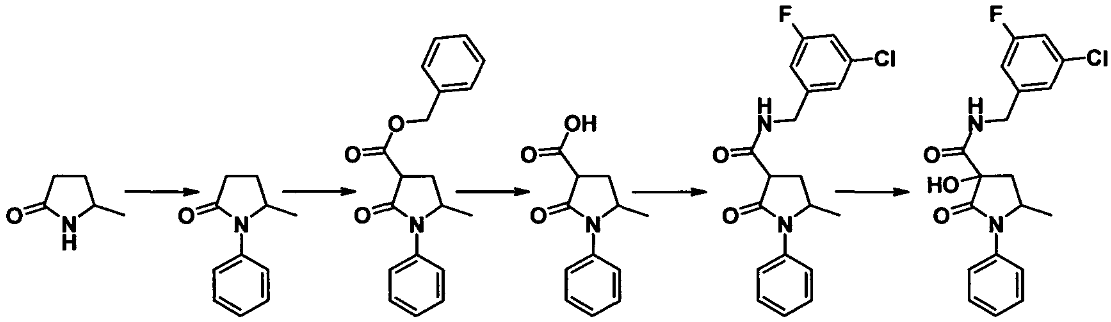

Beispiel 2

Herstellung von 3-[(3-Chlor-5-fluor-benzylamino)-methyl]-1-phenyl-pyrrolidin-3-ol ("A120")

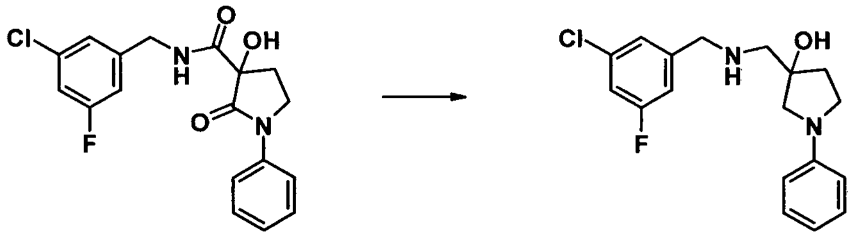

Man löst 3-Hydroxy-2-oxo-1-phenyl-pyrrolidin-3-carbonsäure-3-chlor-5-fluorbenzylamid (100 mg) in trockenem THF (2 ml) und gibt bei -78°C Borantetrahydrofuran-Komplex (1,0 M in THF; 1 ml) tropfenweise hinzu.
Man rührt fünf Stunden bei 60°C nach und arbeitet dann bei 0°C durch Zugabe von 3 ml Methanol auf. Nach dem Eindampfen wird der Rückstand chromatographisch aufgereinigt und man erhält 3-[(3-Chlor-5-fluorbenzylamino)-methyl]-1-phenyl-pyrrolidin-3-ol (22 mg) also amorphen Feststoff.

Beispiel 3

Herstellung von 1-Benzyl-3-hydroxy-pyrrolidin-3-carbonsäure-3-chlor-5-fluorbenzylamid ("A121")

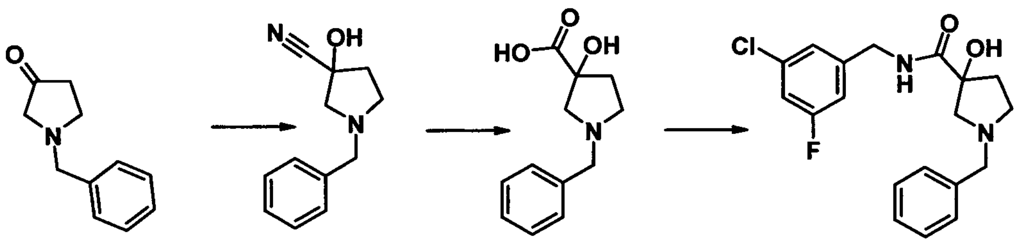

3.1 Man löst 1 ml kommerziell erhältliches 1-Benzyl-pyrrolidin-3-on in 30 ml Wasser und 10 ml 1 N HCl. Dazu gibt man tropfenweise eine Lösung von 460 mg Natriumcyanid in 10 ml Wasser und rührt bei RT für eine Stunde. Das Produkt wird nicht isoliert und direkt in der nächsten Stufe umgesetzt.
Man löst das Rohprodukt aus der Vorstufe in 50 ml 25%iger HCl und erhitzt für 2 h zum Rückfluß. Flüchtige Bestandteile werde im Vakuum entfernt und der Rückstand chromatographisch aufgereinigt. Man erhält 400 mg 1-Benzyl-3-hydroxy-pyrrolidin-3-carbonsäure als amorphen Feststoff;
$^1H$ (400 MHz, DMSO-$d_6$) $\delta$ [ppm] 7.35 (5 H, m), 3.90 (2 H, s), 3.06 (1 H, d, J 10.5), 2.99 (1 H, m), 2.87 (1 H, m), 2.75 (1 H, d, J 10.4), 2.25 (1 H, dt, J 13.0, 7.7), 1.83 (1 H, m).
3.2 1-Benzyl-3-hydroxy-pyrrolidine-3-carbosäure (100 mg) und 3-Chlor-5-fluorobenzylamin (79 mg) werden in einem Milliliter getrocknetem DMSO gelöst und auf 0°C gekühlt. Dann gibt man o-(7-Azabenzotrial-1-yl)-N,N,N',N'-tetramethyluronium hexafluorophosphate (206 mg) und 4-Methylmorpholin (0,124 ml) hinzu. Der Ansatz wird für 2 Stunden bei 25 °C gerührt und dann sofort chromatographisch aufgereinigt.
Man erhält 1-Benzyl-3-hydroxy-pyrrolidin-3-carbonsäure 3-chloro-5-fluoro-benzylamid (37 mg) als farblosen amorphen Feststoff.

Beispiel 4

Herstellung von N-[(3-chlor-5-fluor-phenyl)methyl]-4-fluor-3-hydroxy-1-phenyl-pyrrolidin-3-carboxamid ("B1")

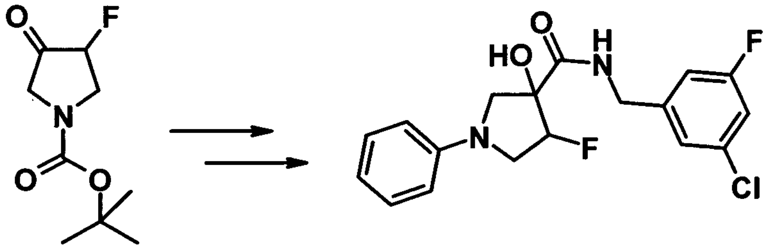

Nach bekannten Methoden und in analoger Weise läßt sich kommerziell erhältliches t-Butyl-3-fluoro-4-oxopyrrolidin-1-carboxylat (Shanghai AQBioPharma) umsetzen und man erhält "B1".

Beispiel 5

Herstellung von 3-(1,3-Dihydro-isoindol-2-carbonyl)-3-hydroxy-1-phenyl-pyrrolidin-2-on ("A267")

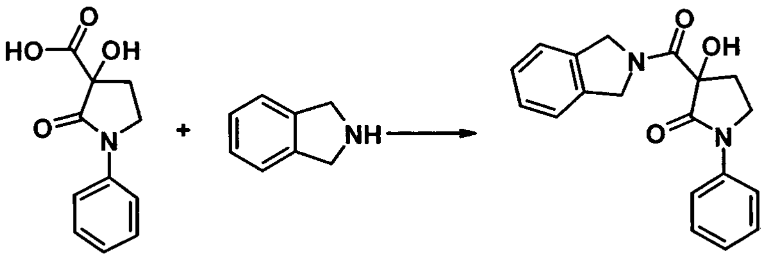

Eine Lösung von 3-Hydroxy-2-oxo-1-phenyl-pyrrolidin-3-carbonsäure (100 mg) und 2,3-Dihydro-1 H-isoindol (66 mg) in N,N-Dimethyl'-formamide (1 ml) wird im Eisbad gekühlt. o-(7-Azabenzotrial-1-yl)-N,N,N',N'-tetramethyluronium hexafluorophosphat (206 mg) und 4-Methylmorpholin (0,1 ml) weden zugegeben und die Mischung wird 20 h be 25 °C gerührt.
Man erhält 3-(1,3-Dihydro-isoindol-2-carbonyl)-3-hydroxy-1-phenyl-pyrrolidin-2-on (63 mg).

Beispiel 6

Herstellung von 1-Benzyl-3-(1,3-dihydro-isoindol-2-carbonyl)-3-hydroxy-pyrrolidin-2-on ("A268")

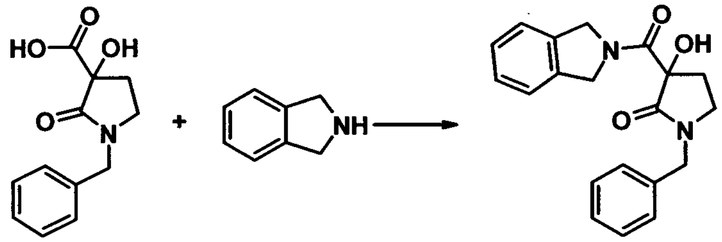

Man löst 1-Benzyl-3-hydroxy-2-oxo-pyrrolidin-3-carbosäure (100 mg) und 2,3-Dihydro-1H-isoindol (62 mg) in N,N-Dimethyl-formamid (1 ml) und kühlt die Lösung auf 0°C ab. o-(7-Azabenzotrial-1-yl)-N,N,N',N'-tetramethyluronium hexafluorophosphat (194 mg) und 4-Methylmorpholin (0,1 ml) werden zugetropft und der Ansatz für 20 Stunden bei 25°C gerührt.

Man erhält 1-Benzyl-3-(1,3-dihydro-isoindol-2-carbonyl)-3-hydroxy-pyrrolidin-2-on (57 mg) als farblosen amorphen Feststoff.

Beispiel 7

Herstellung von 1-(1H-Benzimidazol-2-ylmethyl)-3-hydroxy-2-oxo-pyrrolidin-3-carbonsäure-3-chlor-5-fluor-benzylamid ("A269")

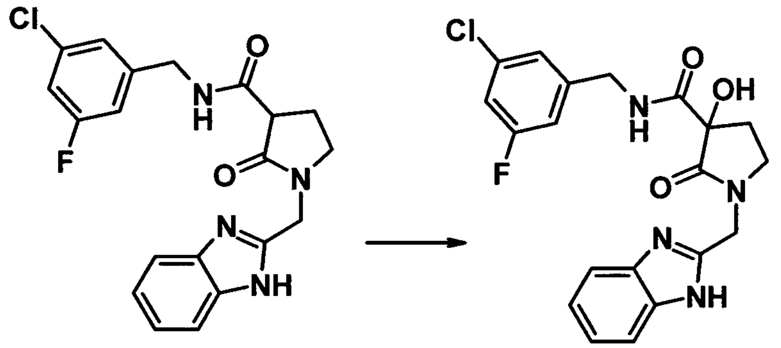

Man lost 1-(1 H-Benzimidazol-2-ylmethyl)-2-oxo-pyrrolidin-3-carbonsäure-3-chlor-5-fluor-benzylamid (396 mg) in tert-Butanol (5 ml), Natriumethylat (20%ige Lösung in Ethanol, 0,6 ml) und tert-Butylhydroperoxid (70%ige Lösung in Wasser, 0,2 ml. Die Mischung wird für eine Stunde bei 80°C gerührt und dann eingedampft. Nach dem Versetzen mit gleichen Teilen Wasser und Ethylacetat fällt das Produkt als farbloser Festsoff aus. Man erhält 1-(1 H-Benzimidazol-2-ylmethyl)-3-hydroxy-2-oxo-pyrrolidin-3-carbobonsäure 3-chloro-5-fluoro-benzylamid (237 mg).

Beispiel 7a

Herstellung von 3-Hydroxy-2-oxo-1-phenyl-piperidin-3-carbonsäure-3-chlor-5-fluor-benzylamid ("A65")

7.1 1-Phenyl-piperidin-2-on:

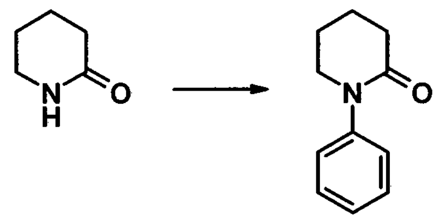

Man löst δ-Valerolactam (2 g) in 1,4-Dioxan (10 mL), gibt Brombenzol (3.32 g) und Cesiumcarbonat (13.2 g) hinzu und verschließt das Reaktionsgefäß. Anschließend leitet man für 15 min Stickstoff durch die Suspension. Dann wird Xantphos (1.16 g) und tris-(Dibenzyliden acetone)dipalladium (0) (1.84 g) hinzugegeben und die Mischung für 12 h auf 100°C erwärmt. Nach beendeter Reaktion werden die bei RT unlöslichen Bestandteile abfiltriert und die Reaktionslösung eingedampft. Das so erhaltene Rohprodukt wird an Kieselgel chromatographiert und man erhält 1.3 g (37%) des Produkts als hellgelben Feststoff;
$^1$H NMR (400 MHz, DMSO-$d_6$) δ [ppm] 7.38-7.34 (m, 2H), 7.26-7.20 (m, 3H), 3.58 (t, J = 6.08 Hz, 2H), 2.37 (t, J = 6.12 Hz, 2H), 1.87-1.80 (m, 4H);

LCMS: Mass found (M+1, 176.2);
Method: A- 0.1% TFA in $H_2O$, B- 0.1% TFA in ACN: Flow - 2.0 ml/min.
Column: X Bridge C8 (50x4.6mm.3.5 μ) + ve mode
Rt (min): 3.91 Area % 76.24 (max), 74.74(220nm)

7.2 2-Oxo-1-phenyl-piperidin-3-carbonsäure ethylester:

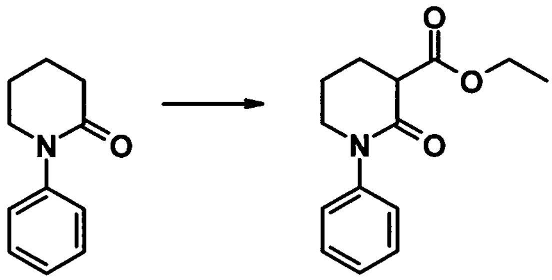

Eine Lösung von 1-Phenyl-piperidin-2-on (1.3 g) in THF (20 mL) wird mit Lithium bis-trimethylsilylamid (1M in THF) (15 mL) bei -78°C unter Stickstoff deprotoniert. Nach einer Stunde wird bei der angegebenen Temperatur Ethylchloroformiat (0.806 g) zugetropft und das Kältebad entfernt. Nach beendeter Reaktion wird mit Eiswasser aufgearbeitet und mit Ethylacetat extrahiert. Die organische Phase wird mit 10% Natriumbicarbonat-Lösung und gesättigter Natriumchlorid-Lösung gewaschen. Nach dem Trocknen und Eindampfen wird der Rückstand an Kieselgel chromatographiert und man erhält 600 mg (32%) einer hellbraunen Flüssigkeit;
$^1$H NMR (400 MHz, DMSO-$d_6$) δ [ppm] 7.40-7.37 (m, 2H), 7.27-7.23 (m, 3H), 4.14-4.08 (m, 2H), 3.67-3.60 (m, 2H), 3.56-3.52 (m, 1 H), 2.12-2.11 (m, 1 H), 2.06-2.03 (m, 1H), 1.94-1.90 (m, 1H), 1.19 (t, J = 7.08 Hz, 3H);

LCMS: Mass found (M+1, 248.2)
Methode: A- 0.1% TFA in $H_2O$, B- 0.1% TFA in ACN: Flow - 2.0 ml/min.
Column: X Bridge C8 (50x4.6mm.3.5 μ) + ve mode
Rt (min) : 2.61 Area % 96.52 ( max), 96.55 (254 nm)

7.3 3-Hydroxy-2-oxo-1-phenyl-piperidin-3-carbonsäure-ethylester:

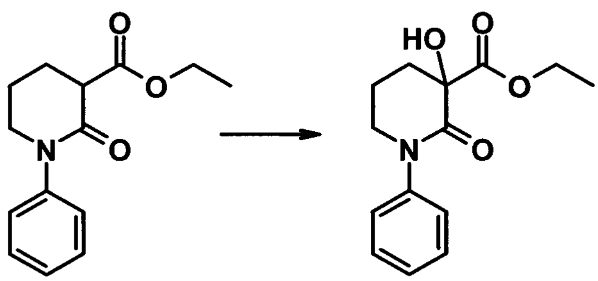

Eine Lösung von 2-Oxo-1-phenyl-piperidin-3-carbonsäure-ethylester (280 mg) in IPA (10 mL) wird mit Cerchlorid heptahydrat (85 mg) versetzt und für 15 min mit Sauerstoff begast. Nachfolgend wird für 12 h unter $O_2$ Atmosphäre

gerührt. Nach beendeter Reaktion wird im Vakuum eingedampft und chromatographisch aufgereinigt. Neben dem gezeigten Produkt 100 mg (34%) wird auch das Chloranaloge erhalten;

LCMS: Mass found (M+1, 264)
Methode: A- 0.1% TFA in $H_2O$, B- 0.1% TFA in ACN: Flow - 2.0ml/min.
Column: X Bridge C8 (50x4.6mm.3.5u) + ve mode

$^1H$ NMR (400 MHz, DMSO-$d_6$) $\delta$ [ppm]7.40-7.38 (m, 2H), 7.28-7.24 (m, 3H), 6.25 (s, 1 H), 4.18-4.12 (m, 2H), 3.69-3.66 (m, 2H), 2.22 (m, 1 H), 1.99 (m, 2H), 1.94-1.89 (m, 1H), 1.21 (t, J = 7.08 Hz, 3H).

7.4 3-Hydroxy-2-oxo-1-phenyl-piperidin-3-carbonsäure:

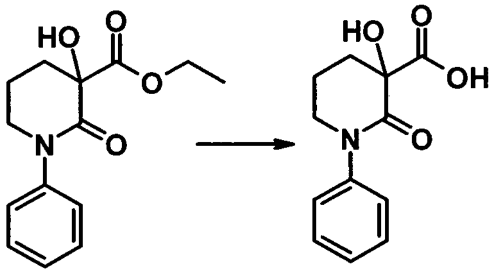

Zu einer Lösung von 3-Hydroxy-2-oxo-1-phenyl-piperidin-3-carbonsäure-ethylester (100 mg) in THF/$H_2O$ = 8:2 (10 mL) wird LiOH.$H_2O$ (32 mg) gegeben und für 1 h gerührt. Nach beendeter Reaktion wird mit 1.5 N HCl Lösung neutralisiert, über Natriumsulfat getrocknet, im Vakuum eingeengt und das Podukt als farbloser Feststoff mit 89%iger Ausbeute (80 mg) erhalten.

7.5 3-Hydroxy-2-oxo-1-phenyl-piperidin-3-carbonsäure-3-chlor-5-fluor-benzylamid ("A65"):

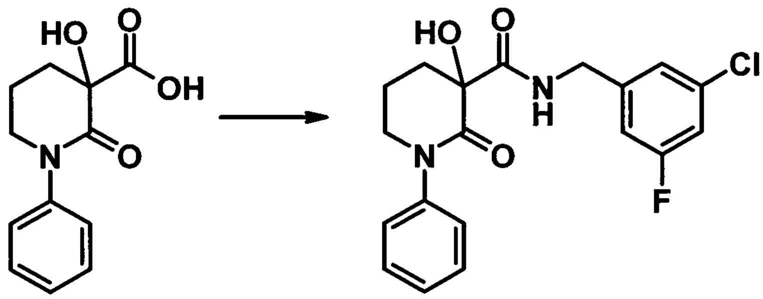

Eine Lösung von 3-Hydroxy-2-oxo-1-phenyl-piperidin-3-carbonsäure (80 mg) und 3-Chlor-5-fluor-benzylamin (65 mg) in Dichlormethan (15 mL) wird mit Triethylamine (0.14 mL) und Propanphosphorsäureanhydrid (T3P; 0.33 g) erst bei 0°C, dann bei RT für 1 h unter Stickstoff gerührt. Nach beendeter Reaktion wird mit Dichlormethan verdünnt und mit 10%iger Natriumbicarbonat-Lösung sowie gesättiger NaCl-Lösung gewaschen. Nach Filtration, Eindampfen und Chromatographie erhält man das Produkt mit 12 % Ausbeute (15 mg) als farblosen Feststoff;

LCMS: Mass found (M+1,377.0)
Methode: A- 0.1% TFA in $H_2O$, B- 0.1% TFA in ACN: Flow - 2.0 ml/min.
Column: X Bridge C8 (50 x 4.6mm.3.5$\mu$) + ve mode
Rt (min): 4.03 Area % 92.24 (max), 91.79 (220nm)
HPLC:
Method: A: 0.1%TFA in $H_2O$, B: 0.1%TFA in ACN, Flow Rate:2.0ml/min
COLUMN: XBridge C8 (50X4.6)mm, 3.5 $\mu$m
Rt (min): 4.01 Area % 94.30 (max), 94.44 (220 nm);

Beispiel 8

Herstellung von (S)-1-Cyclohexylmethyl-3-hydroxy-2-oxo-piperidin-3-carbonsäure-3-fluor-benzylamid ("A256") und (R)-1-Cyclohexylmethyl-3-hydroxy-2-oxo-piperidin-3-carbonsäure-3-fluor-benzylamid ("A263")

8.1 1-Cyclohexylmethyl-piperidin-2-on:

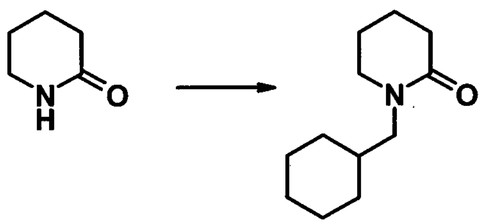

Man löst ö-Valerolactam (5 g) in trockenem N,N-Dimethylformamid (25 mL) und gibt diese Lösung bei 0°C zu einer Suspension von Natriumhydrid (2.42 g) in N,N-Dimethylformamid (25 mL). Nach 30 Minuten Rühren bei der angegebenen Temperatur gibt man Brommethylcyclohexan (11.60 g, 65.57 mmol) tropfenweise hinzu. Nachfolgend wird der Ansatz für 8 h bei RT gerührt und zur Aufarbeitung am Rotavapor vollständig eingedampft. Der Rückstand wird mit Wasser aufgenommen und mit Ethylacetat erschöpfend extrahiert. Die über Natriumsulfat getrockneten organischen Phasen werden eingedampft. Man erhält die Titelverbindung als hellbraune Flüssigkeit;
Ausbeute: 6.6 g (67%);
$^1H$ NMR (400 MHz, DMSO-$d_6$) $\delta$ [ppm] 3.21-3.18 (m, 2H), 3.10-3.08 (m, 2H), 2.18 (t, J = 6.00 Hz, 2H), 1.72-1.54 (m, 11 H), 1.20-1.08 (m, 4H), 0.89-0.80 (m, 2H), .

LCMS: Mass found (M+1, 196.2)
Methode: A- 0.1% TFA in $H_2O$, B- 0.1% TFA in ACN: Flow - 2.0 ml/min.
Column: X Bridge C8 (50x4.6 mm.3.5u) + ve mode
Rt (min): 3.91 Area % 92.59 (ELSD).

1-Cyclohexylmethyl-2-oxo-piperidin-3-carbonsäure ethyl ester:

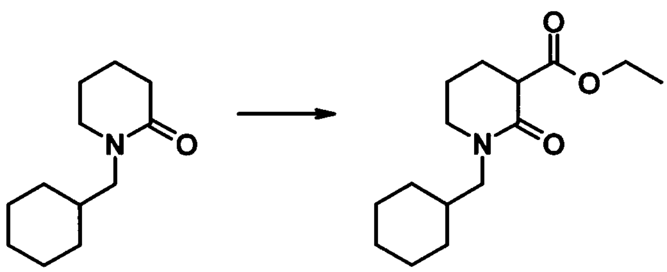

Eine Lösung des zuvor hergestellten 1-Cyclohexylmethyl-piperidin-2-on (6.6 g) in THF (70 mL) wird tropfenweise mit Lithium-bis-trimethylsilylamid (1 M in THF; 68 mL) bei -78°C unter Stickstoff versetzt. Nach einer Stunde wird bei der angegebenen Temperatur Ethylchloroformiat (3.67 g) zugetropft und nach beendeter Zugabe das Kältbad entfernt. Zur Aufarbeitung wird der Ansatz mit Eiswasser versetzt und mit Ethylacetat extrahiert. Nach dem Waschen der organischen Phase mit 10%iger Natriumbicarbonat-Lösung und gesättigter NaCl-Lösung wird über Natriumsulfat getrocknet, abfiltriert und eingeengt. Der Rückstand wird über Kieselgel chromatographiert und das Produkt als braune Flüssigkeit erhalten.

Ausbeute: 5 g (55%);
$^1H$ NMR (400 MHz, DMSO-$d_6$) $\delta$ [ppm] 4.08-4.03 (m, 2H), 3.32-3.18 (m, 3H), 3.04-3.00 (m, 1 H), 1.98-1.87 (m, 1 H), 1.68-1.56 (m, 9H), 1.20-1.12 (m, 7H), 0.87-0.84 (m, 2H);

LCMS: Mass found (M+1, 268.2)
Methode: A- 0.1% TFA in $H_2O$, B- 0.1% TFA in ACN: Flow - 2.0 ml/min.
Column: X Bridge C8 (50 x 4.6 mm.3.5u) + ve mode
Rt (min) : 4.41 Area % 95.21 ( max), 93.63 (220 nm)

1-Cyclohexyfmethyl-3-hydroxy-2-oxo-piperidin-3-carbonsäure ethyl ester:

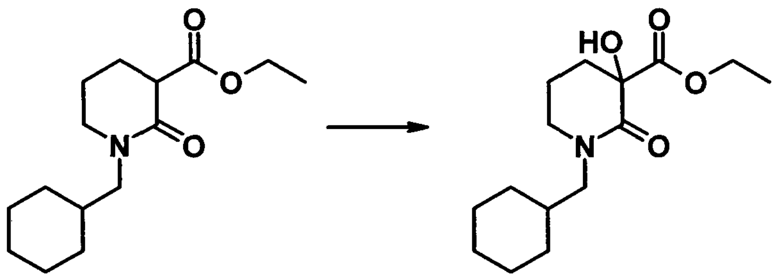

Eine Lösung von 1-Cyclohexylmethyl-2-oxo-piperidin-3-carbonsäure-ethylester (2.5 g) in IPA (20 mL) wird mit Cerchlorid heptahydrat (0.697 g) versetzt und mit $O_2$ für 15 min begast. Nach 12 h unter $O_2$ Atmosphäre werden alle flüchtigen Bestandteile entfernt und der Rückstand an Kieselgel aufgereinigt.

1-Cyclohexylmethyl-3-hydroxy-2-oxo-piperidin-3-carbonsäure

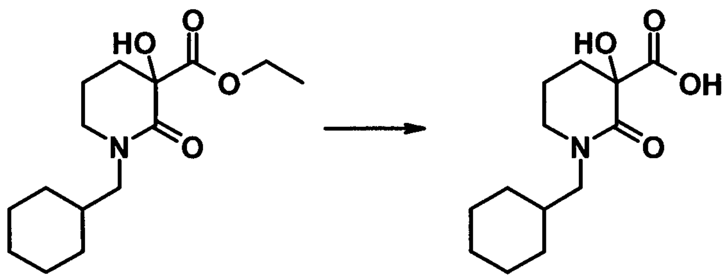

Der zuvor hergestellte Ester (700 mg) wird in THF/$H_2O$ = 16:4 (20 mL) gelöst und mit LiOH x $H_2O$ (207 mg) versetzt. Nach einer Stunde werden alle flüchtigen Bestandteile im Vakuum entfernt und mit 1.5 N HCl angesäuert. Die wässrige Phase wird mit Ethylacetat extrahiert und die organische Phase wie beschrieben getrocknet. Ohne weitere Aufreinigung erhält man die Titelverbindung als gelbe Flüssigkeit (600 mg, 95%);

LCMS: Mass found (M+1, 256)
Methode: A- 0.1% TFA in $H_2O$, B- 0.1% TFA in ACN: Flow - 2.0ml/min.
Column: X Bridge C8 (50x4.6mm.3.5u) + ve mode
Rt (min) : 6.00 Area % 96.63 (ELSD).

1-Cyclohexylmethyl-3-hydroxy-2-oxo-piperidin-3-carbonsäure-3-fluor-benzylamid

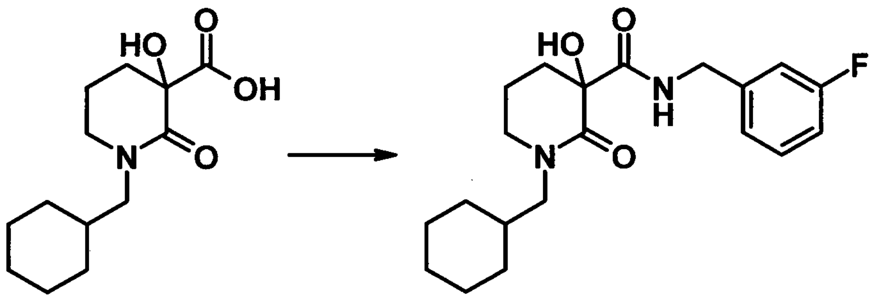

Eine Lösung von 1-Cyclohexylmethyl-3-hydroxy-2-oxo-piperidin-3-carbonsäure (200 mg) und 3-Fluorbenzylamin (116 mg) in Dichlormethan (20 mL) wird mit Triethylamin (0.33 mL) und Propanphosphorsäureanhydrid (T3P - 748 g) bei 0°C versetzt. Nach einer Stunde bei RT wird wie beschrieben aufgearbeitet. Da man teilweise eine Reduktion der Hydroxygruppe beobacht, wird der Ansatz erneut mit tert-Butylhydroperoxid in tert-Butanol oxidiert.

Nach der üblichen Aufarbeitung wird an der chiralen HPLC aufgereinigt; Mobile phase: 0.1% DEA in HEXANE/IPA = 60:40

Column: CHIRALPAK AD-H (250x4.6)mm, 5□m
FLOW: 1.0mL\min
Rt (min): 5.1 & 10.3 Area % 53.43 & 46.56
Man erhält das S-Enantiomer in 3% Ausbeute ("A256");
LCMS: Mass found (M+1,363.3)
Methode: A- 0.1% TFA in $H_2O$, B- 0.1% TFA in ACN: Flow - 2.0 ml/min.
Column: X Bridge C8 (50 x 4.6 mm.3.5μ) + ve mode
Rt (min) : 4.42 Area % 95.85 (max), 95.34 (220 nm)
HPLC:
Methode: A: 0.1%TFA in $H_2O$, B: 0.1%TFA in ACN, Flow Rate: 2.0 ml/min
COLUMN: XBridge C8 (50 x 4.6) mm, 3.5 μm
Rt (min): 4.44 Area % 95.49 (max), 95.20 (220 nm);
$^1H$ NMR (400 MHz, DMSO-$d_6$) δ [ppm] 8.40-8.39 (m, 1 H), 7.31 (t, J = 7.48 Hz, 1 H), 7.11 (m, 2H), 7.05-7.00 (m, 1 H), 6.08 (s, 1 H), 4.39-4.33 (m, 1 H), 4.26-4.20 (m, 1H), 3.28-3.22 (m, 3H), 3.04-3.01 (m, 1H), 2.17 (m, 1H), 1.85-1.77 (m, 3H), 1.63-1.62 (m, 6H), 1.23-1.13 (m, 3H), 0.89-0.83 (m, 2H).

Man erhält das R-Enantiomer in 13% Ausbeute ("A263" aus Tabelle)

LCMS: Mass found (M+1,363.3)
Methode: A- 0.1% TFA in $H_2O$, B-0.1% TFA in ACN: Flow - 2.0 ml/min.
Column: X Bridge C8 (50 x 4.6 mm.3.5μ) + ve mode
Rt (min): 4.45 Area % 96.21 (max), 96.17 (220 nm)
HPLC:
Methode: A: 0.1%TFA in $H_2O$, B: 0.1%TFA in ACN, Flow Rate: 2.0 ml/min
COLUMN: XBridge C8 (50 xX 4.6) mm, 3.5 μm
Rt (min) : 4.44 Area % 98.19 (max), 97.90 (220 nm)
$^1H$ NMR (400 MHz, DMSO-$d_6$) δ [ppm] 8.40-8.39 (m, 1 H), 7.31 (t, J = 7.48 Hz, 1 H), 7.11 (m, 2H), 7.05-7.00 (m, 1 H), 6.08 (s, 1 H), 4.39-4.33 (m, 1 H), 4.26-4.20 (m, 1 H), 3.28-3.22 (m, 3H), 3.04-3.01 (m, 1 H), 2.17 (m, 1 H), 1.85-1.77 (m, 3H), 1.63-1.62 (m, 6H), 1.23-1.13 (m, 3H), 0.89-0.83 (m, 2H).

Beispiel 9

Herstellung von (S)-3-((E)-But-2-enoyl)-3-hydroxy-1-phenyl-pyrrolidin-2-on ("A70")

9.1 (E)-3-(1-hydroxybut-2-en-1-yl)-1-phenylpyrrolidin-2-on

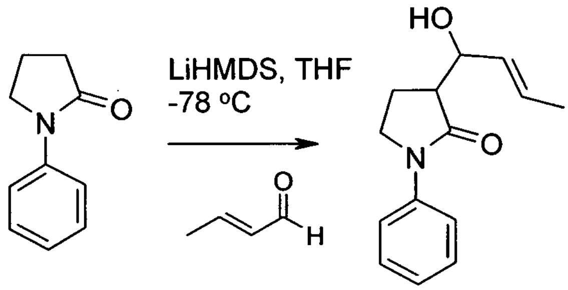


Kommerziell erhältliches 1-Phenylpyrrolidin-2-on (483 mg) wird in THF (10 mL) gelöst und bei -78 °C mit LiHMDS (3.6 mL, 1 M in THF) tropfenweise versetzt. Nach 30 Minuten wird Crotonaldehyd (252 mg) in THF (5 mL) zugetropft und anschließend das Kältebad entfernt. Der Ansatz wird mit $NH_4Cl$ Lösung (5 mL) aufgearbeitet und mit Ethylacetat extrahiert. Nach dem Trocknen über Natriumsulfat wird an Kieselgel aufgereinigt. Man erhält einen farblosen Feststoff. (66 %; 455 mg).
$^1H$ NMR: 400 MHz, DMSO-d6: δ 7.61-7.68 (m, 2H), 7.33-7.37 (m, 2H), 7.11-7.14 (m, 1 H), 5.63-5.65 (m, 1 H), 5.54-5.54 (m, 1 H), 5.03 (d, *J* = 3.88 Hz) & 4.96 (d, *J* = 4.88 Hz, 1 H), 4.41 (t, *J* = 72.00 Hz, 1H), 3.70-3.75 (m, 2H), 2.60-2.80 (m, 1H), 1.98-2.09 (m, 2H), 1.61-1.67 (m, 3H).

9.2 (E)-3-(but-2-enoyl)-3-hydroxy-1-phenylpyrrolidin-2-on

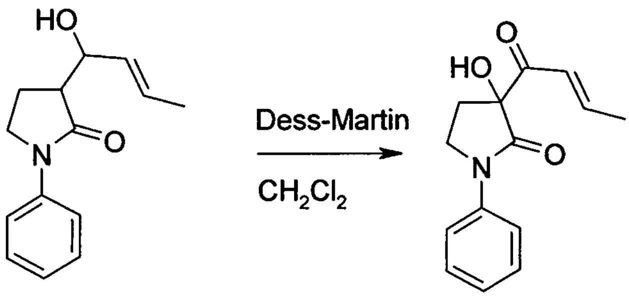


Eine Lösung von (E)-3-(1-Hydroxybut-2-en-1-yl)-1-phenylpyrrolidin-2-on (226 mg) in Dichlormethan (10 mL) wird bei O°C mit Dess-Martin Periodinan (850 mg) umgesetzt. Nach beendeter Reaktion wird üblich aufgearbeitet und aufgereinigt. Man erhält das Produkt als farblosen Feststoff (75 %, 185 mg); $^1H$ NMR: 400 MHz, DMSO-$d_6$: δ [ppm] 7.66-7.69 (m, 2H), 7.38-7.42 (m, 2H), 7.16-7.19 (m, 1H), 6.90-6.95 (m, 1 H), 6.79 (d, *J* = 15.56 Hz, 1 H), 6.65 (s, 1H), 3.82-3.84 (m, 1H), 3.71-3.74 (m, 1H), 2.49-2.53 (m, 1H), 2.06-2.09 (m, 1H), 1.90 (d, *J* = 6.72 Hz, 3H);

LCMS: (Methode A) 246.0 (M+H), RT. 3.16 min, 98.5 % (Max), 96.8 % (254 nm).
HPLC: (Methode A) RT 3.3 min, 98.1 % (Max), 95.9 % (254 nm).

Nach Enantiomerentrennung mittel chiraler HPLC erhält man (S)-3-((E)-But-2-enoyl)-3-hydroxy-1-phenyl-pyrrolidin-2-on:

$^1H$ NMR (400 MHz, DMSO-$d_6$) δ [ppm] 7.70 - 7.65 (m, 2H), 7.43 - 7.37 (m, 2H), 7.21-7.15 (m, 1 H), 6.83-6.76 (m, 1H), 6.65(s, 1 H), 3.84 (td, *J* = 9.2, 3.0 Hz, 1 H), 3.73 (dt, *J* = 9.6, 7.5 Hz, 1 H), 2.57-2.50 (m, 1 H), 2.13-2.03 (m, 1 H), 1.90 (dd, *J* = 6.7,1.5 Hz, 3H).

Beispiel 10

Herstellung von 3-(2-Benzyl-acryloyl)-3-hydroxy-1-phenyl-pyrrolidin-2-on ("A89")

10.1 3-(2-Benzyl-1-hydroxyallyl)-1-phenylpyrrolidin-2-on

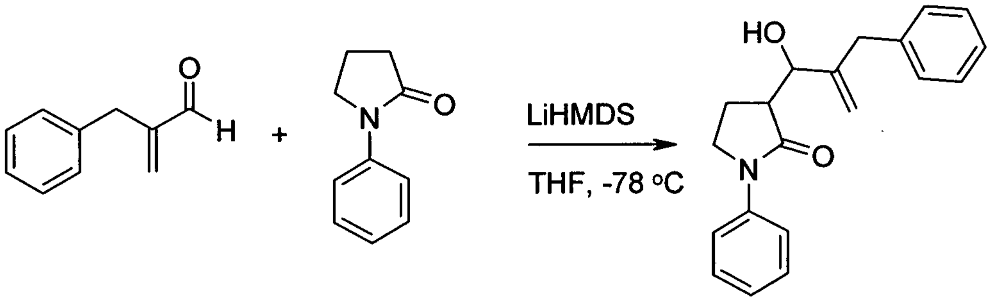


Die Reaktion erfolgt wie oben für den Crotonaldehyd beschrieben.
$^1$H NMR: 400 MHz, DMSO-$d_6$: δ [ppm] 7.64-7.67 (m, 2H), 7.12-7.39 (m, 10H), 5.23 (d, *J* = 3.56 Hz, 1 H), 5.13 (d, *J* = 4.32 Hz, 1 H), 4.65 (d, *J* = 1.20 Hz, 1 H), 4.30-4.45 (m, 1 H), 3.72-7.75 (m, 2H), 3.30-3.50 (m, 3H), 2.85-3.00 (m, 1 H), 1.80-2.20 (m, 2H);

LCMS: (Methode A) 308.2 (M+H), RT. 4.71 min, 30.39 % (Max) and 308.2 (M+H), RT. 4.96 min, 42.73 % (Max).

10.2 3-(2-Benzylacryloyl)-1-phenylpyrrolidin-2-on

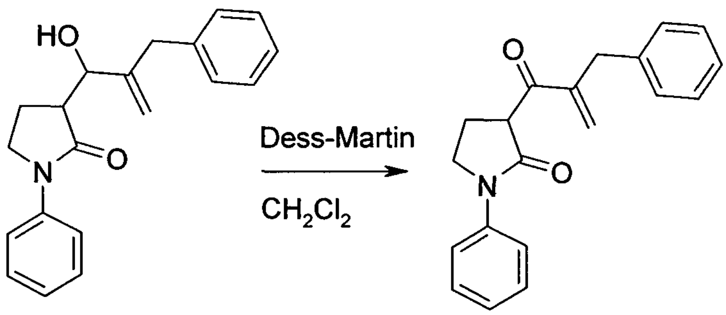


Die Umsetzung erfolgt analog zu der oben beschriebenen Reaktion, wobei hier nur die schon vorhandene Hydroxy-Gruppe zum Keton oxidiert und nicht, wie oben, auch gleichzeitig die zweite OH-Funktion eingeführt wird.

LCMS: (Methode A) 306.2 (M+H), RT. 4.98 min, 80.6 % (Max), 91.59 % (254 nm);
$^1$H NMR: 400 MHz, DMSO-$d_6$: δ [ppm] 7.60-7.62 (m, 2H), 7.35-7.39 (m, 2H), 7.25-7.29 (m, 2H), 7.13-7.19 (m, 5H), 6.46 (s, 1 H), 6.02 (s, 1H), 4.67-4.71 (m, 1 H), 3.83 (t, J = 6.96 Hz, 2H), 3.58 (s, 2H).

10.3 3-(2-Benzylacryloyl)-3-hydroxy-1-phenylpyrrolidin-2-on

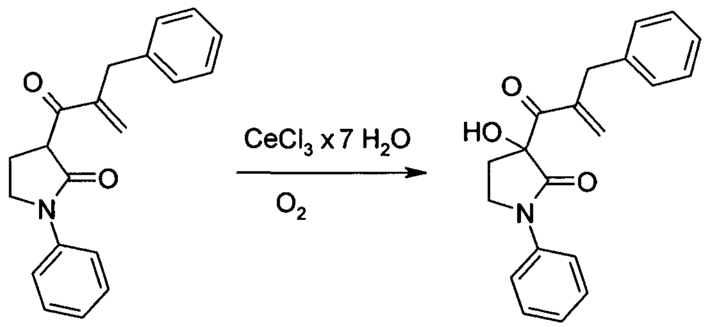


Zu einer Lösung von 3-(2-Benzylacryloyl)-1-phenylpyrrolidin-2-on (400 mg) und $CeCl_3$ x 7 $H_2O$ (37 mg) in 2-Propanol (15 mL) wird für 30 min Sauerstoff gegeben und dann 14 h gerührt. Nachfolgend wird wie üblich aufgearbeitet

und aufgereinigt.

LCMS: (Methode A) 322.0 (M+H), RT. 4.63 min, 98.2 % (Max), 98.9 % (254 nm);
HPLC: (Methode A) RT 4.6 min, 99.1 % (Max), 99.6 % (254 nm);
$^1H$ NMR: 400 MHz, DMSO-$d_6$: δ [ppm] 7.65 (t, J = 0.88 Hz, 2H), 7.37-7.41 (m, 2H), 7.24-7.28 (m, 2H), 7.15-7.19 (m, 4H), 6.79 (s, 1H), 6.66 (s, 1H), 5.91 (d, J = 1.00 Hz, 1H), 3.81-3.82 (m, 1H), 3.57-3.59 (m, 1H), 3.54 (s, 2H), 3.34-3.35 (m, 1 H), 2.52-2.55 (m, 1 H), 2.13-2.16 (m, 1 H).

Beispiel 11

Herstellung von 1-Benzyl-N-(3-chlor-5-fluorbenzyl)-3-hydroxypiperidin-3-carboxamid ("A301")

11.1 tert-Butyl 3-cyan-3-hydroxypiperidin-1-carboxylat

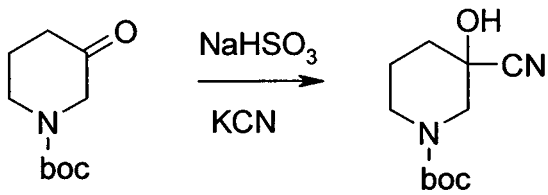


Zu einer Lösung von tert-Butyl-3-oxopiperidin-1-carboxylat (5 g) in Wasser / Diethylether (50 : 25 mL) wird $NaHSO_3$ (3.8 g) gegeben und für 15 min gerührt. KCN (2.4 g) wird anschließend hinzugefügt. Nach beendeter Reaktion werden die Phasen getrennt und das Produkt durch Extraktion und Chromatographie isoliert. Man erhält 4.2 g (74%) eines orangen Feststoffs;

LCMS: (Methode A) 100.2 (M+H), RT. 3.32 min, 92.85 % (Max);
$^1H$ NMR: 400 MHz, DMSO-$d_6$: δ [ppm] 6.86 (s, 1H), 4.01-4.09 (m, 1H), 3.75 (s, 1 H), 2.76-2.90 (m, 2H), 2.08 (d, *J* = 12.24 Hz, 1 H), 1.30-1.50 (m, 2H).

11.2 3-Hydroxypiperidin-3-carbonsäuremethylester

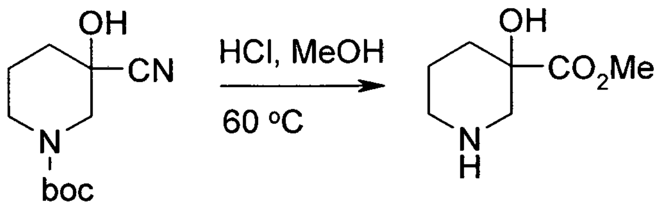


Zu einer Lösung von tert-Butyl 3-cyan-3-hydroxypiperidin-1-carboxylat (4.2 g) in MeOH (40 mL) gibt man konzentrierte HCl (20 mL) und erwärmt die Mischung zum Rückfluß. Nachfolgend wird das Wasser im Vakuum entfernt, mit gesättigter $NaHCO_3$ Lösung neutralisiert, mit Ethylacetat extrahiert, über $Na_2SO_4$ getrocknet und nach dem Eindampfen der Rückstand ohne weitere Aufreinigung umgesetzt.

LCMS: (Methode A) 160.2 (M+H), RT. 0.52 min, 14.78 % (Max).

11.3 1-Benzyl-3-hydroxypiperidin-3-carbonsäuremethylester

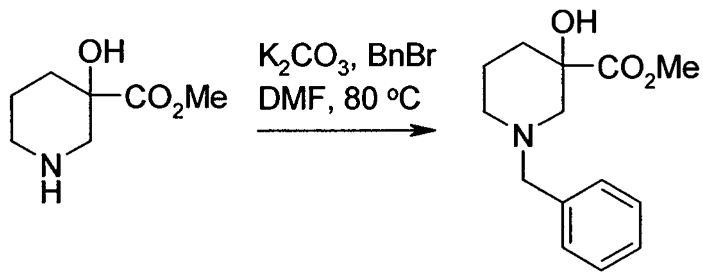


Das Rohprodukt aus der vorherigen Stufe wird in 40 mL DMF suspendiert und mit stöchiometrischen Mengen $t_2CO_3$ und Benzylbromid versetzt. Die Mischung wird für 14 h auf 80°C erwärmt und anschließend wird das Lösungsmittel im Vakuum entfernt. Der Rückstand wird in Wasser aufgenommen und mit Essigester extrahiert. Die vereinigten organischen Phasen werde mit gesättigter NaCl-Lösung gewaschen, über $Na_2SO_4$ getrocknet und nach dem Aufkonzentrieren über Kieselgel gereinigt. Man erhält 400 mg eines gelben Öls;

LCMS: (Methode A) 150.0 (M+H), RT. 1.84 min, 26.66 % (Max).

11.4 1-Benzyl-3-hydroxypiperidin-3-carbonsäure

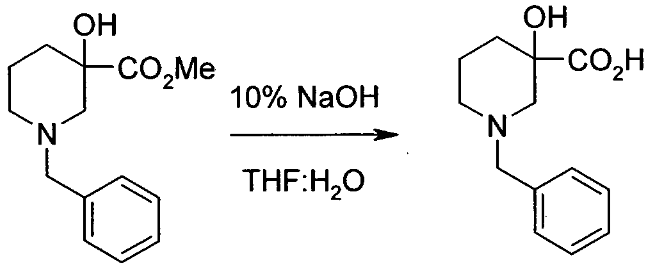


Eine Lösung von 1-Benzyl-3-hydroxypiperidin-3-carbonsäuremethylester (400 mg) in THF : $H_2O$ (10 mL, 1:1) wird mit 10 %iger NaOH-Lösung (2 mL) versetzt und bei RT für 3 h gerührt. Nachfolgend wird im Vakuum aufkonzentriert und mit 1.5 N HCl-Lösung neutralisiert. Der Rückstand wird in $CH_3OH$ : $CH_2Cl_2$ (1:1, 25 mL) suspendiert und der anorganische Rückstand abfiltriert. Nach dem erneuten Eindampfen erhält man 200 mg eines farblosen Feststoffs; $^1H$ NMR: 400 MHz, DMSO-$d_6$: δ [ppm] 13.31 (s, 1H), 9.58 (s, 1H), 7.45-7.57 (m, 5H), 6.16 (s, 1H), 4.38 (d, J = 10.80 Hz, 1H), 4.17-4.22 (m, 1H), 3.18-3.23 (m, 1 H), 2.98 (d, J = 11.56 Hz, 2H), 2.04-2.08 (m, 1 H), 1.60-1.90 (m, 3H).

11.5 1-Benzyl-N-(3-chlor-5-fluorbenzyl)-3-hydroxypiperidin-3-carboxamid

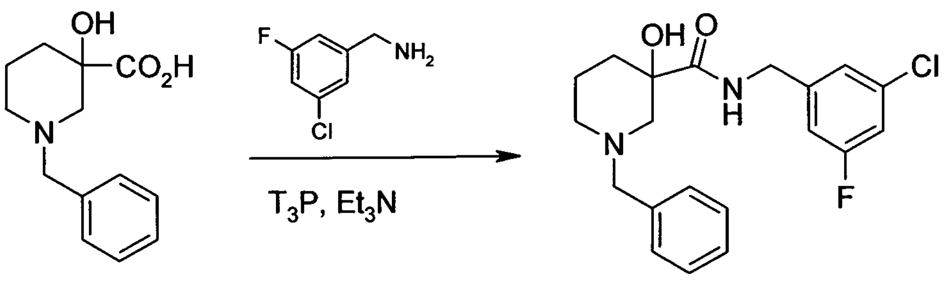


Die zuvor hergestellte Säure wird mit 3-Chlor-5-fluorbenzylamin unter den beschriebenen Bedingungen zur Amidkupplung mit T3P umgesetzt und wie üblich aufgearbeitet und aufgereinigt;

LCMS: (Methode A) 378.0 (M+H), RT. 3.56 min, 95.42 % (Max).

Beispiel 12

Herstellung von (S)-3-Hydroxy-2-oxo-1-phenyl-azepan-3-carbonsäure-3-chlor-5-fluor-benzylamid ("A66") und von (R)-3-Hydroxy-2-oxo-1-phenyl-azepan-3-carbonsäure-3-chlor-5-fluor-benzylamide ("A67")

12.1 1-Phenyl-azepan-2-on:

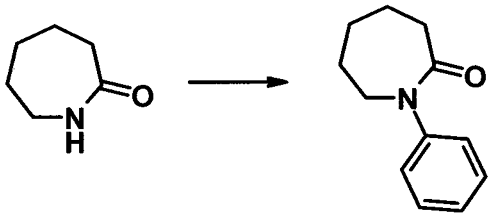

Eine Lösung von ε-Caprolactam (1 g) in 1,4-Dioxan (10 mL) wird mit Brombenzol (1.66 g) und $Cs_2CO_3$ (4.3 g) in einem Reaktionsgefäß verschlossen. Die Mischung wird mit $N_2$ für 15 min entgast. Dann gibt man Xanthphos (0.307 g) und Tris(dibenzylidenaceton)-di-palladium(0) (0.243 g) hinzu und erwärmt auf 100 °C für 12 h. Nach beendeter Reaktion werden unlösliche Bestandteile abfiltriert und das Lösungsmittel abgedampft. Der Rückstand wird durch Chromatographie aufgereinigt. Man erhält 1.2 g (72%) des Produkts als hellgelben Feststoff;

LCMS: Mass found (M+1, 190.0)
Methode: A-0.1% TFA in $H_2O$, B-0.1% TFA in ACN: Flow-2.0 mL/min.
Column: X Bridge C8 (50 x 4.6 mm.3.5 μ) + ve mode
Rt (min) : 3.09 Area % 95.94 (Max), 97.13 (254 nm)

12.2 2-Oxo-1-phenyl-azepan-3-carbonsäureethylester:

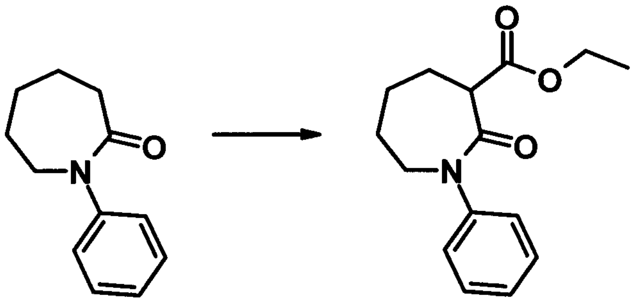

Zu einer Lösung von 1-Phenyl-azepan-2-on (1.2 g) in THF (20 mL) gibt man bei -78°C Lithium-bis-trimethylsilylamid (1M in THF; 13 mL). Nach einer Stunde bei der angegebenen Temperatur wird tropfenweise Ethylchloroformiat (0.65 g) zugegeben. Nachfolgend läßt man bis zur Vervollständigung der Reaktion bei RT nachrühren. Nach beendeter Reaktion wird mit Eiswasser aufgearbeitet und mit Ethylacetat extrahiert. Die organische Phase wird mit 10%iger Natriumbicarbonat- und gesättigter NaCl-Lösung gewaschen. Anschließend wird die organische Phase über Natriumsulfat getrocknet, filtriert und eingedampft. Nach chromatographischer Reinigung erhält man 300 mg (19%) des Produkts;

LCMS: Mass found (M+1, 262.2)
Methode: A- 0.1 % TFA in $H_2O$, B-0.1% TFA in ACN: Flow-2.0 mL/min.
Column: X Bridge C8 (50 x 4.6 mm.3.5 μ) + ve mode
Rt (min): 3.84 Area % 90.35 (Max), 86.73 (220 nm).

12.3 2-Oxo-1-phenyl-azepan-3-carbonsäure

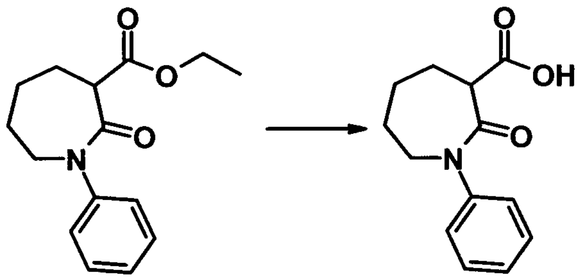

Zu einer Lösung von 2-Oxo-1-phenyl-azepan-3-carbonsäureethylester (300 mg) in THF/$H_2O$ = 8:2 (15 mL) gibt man LiOH x $H_2O$ (111 mg) und rührt für 1 h. Nach beendeter Reaktion wird das Lösungsmittel im Vakuum entfernt und mit 1.5 N HCl Lösung neutralisiert. Die wässrige Lösung wird mit Ethylacetat extrahiert und die vereinigten organischen Phasen über Natriumsulfat getrocknet, filtriert und eingedampft. Man erhält das Produkt mit 97% Ausbeute (250 mg);

LCMS: Mass found (M+1, 234.0)
Methode A: 0.1% TFA in $H_2O$, B: 0.1% TFA in ACN: Flow-2.0 mL/min.
Column: X Bridge C8 (50 x 4.6 mm, 3.5 μm) + ve mode
Rt (min) : 2.95 Area % 97.57 (Max), 97.38 (220 nm);
HPLC:
Methode A: 0.1%TFA in $H_2O$, B: 0.1%TFA in ACN, Flow Rate: 2.0mL/min Column: X Bridge C8 (50 x 4.6) mm, 3.5 μm
Rt (min): 2.90 Area % 99.67 (Max), 99.24 (254 nm)
$^1H$ NMR (400 MHz, DMSO-$d_6$): δ [ppm]12.45 (s, 1H), 7.39-7.35 (m, 2H), 7.25-7.19 (m, 3H), 3.98-3.87 (m, 2H), 3.53-3.47 (m, 1H), 2.00 (m, 1H), 1.88 (m, 1H), 1.77-1.60 (m, 4H).

12.4 2-Oxo-1-phenyl-azepan-3-carbonsäure 3-chlor-5-fluor-benzylamid:

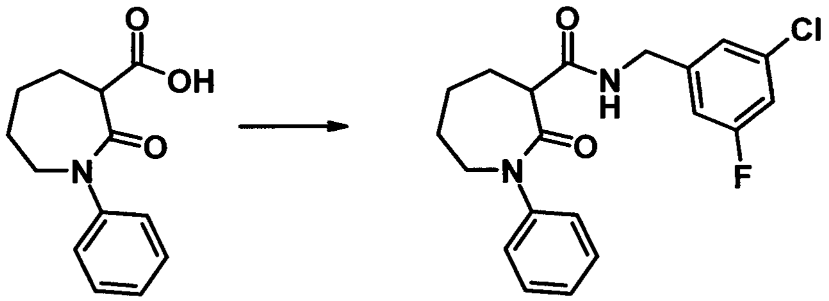

Zu einer Lösung von 2-Oxo-1-phenyl-azepan-3-carbonsäure (250 mg) und 3-Chlor-5-fluorbenzylamin (205 mg) in Dichlormethan (15 mL) gibt man bei 0°C Triethylamin (0.74 mL) und Propanphosphorsäureanhydrid (T3P; 1.02 g). Nachdem die Reaktion bei RT zur Vollständigkeit gebracht ist, verdünnt man mit weiterem Dichlormethan und wäscht mit 10%iger Natriumbicarbonat- und gesättigter NaCl-Lösung. Die organische Phase wird wie üblich behandelt und der Rückstand aufgereinigt. Man erhält das Produkt in 87% (350 mg) Ausbeute als farblosen Feststoff;

LCMS: Mass found (M+1,375.2)
Methode A: 0.1 % TFA in $H_2O$, B: 0.1 % TFA in ACN: Flow-2.0 mL/min.
Column: X Bridge C8 (50 x 4.6 mm, 3.5μm) + ve mode
Rt (min): 4.57 Area % 98.66 (Max), 97.22 (220 nm)
HPLC:
Methode A: 0.1%TFA in $H_2O$, B: 0.1%TFA in ACN, Flow Rate: 2.0 mL/min
Column: XBridge C8 (50 x 4.6) mm, 3.5 μm
Rt (min): 4.63 Area % 97.55 (Max), 97.87 (220 nm);
$^1H$ NMR (400 MHz, DMSO-$d_6$): ö [ppm] 8.37-8.36 (m, 1H), 7.40-7.36 (m, 2H), 7.27-7.20 (m, 6H), 4.44-4.38 (m, 1H), 4.25-4.20 (m, 1H), 3.92-3.83 (m, 2H), 3.60-3.59 (m, 1 H), 2.03-2.01 (m, 1 H), 1.98-1.95 (m, 1 H), 1.78-1.63 (m, 4H).

12.5 3-Hydroxy-2-oxo-1-phenyl-azepan-3-carbonsäure 3-chlor-5-fluor-benzylamid

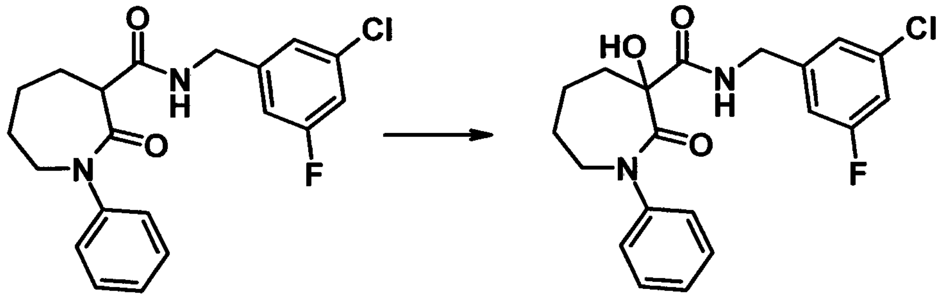

Zu einer Lösung von 2-Oxo-1-phenyl-azepan-3-carbonsäure-3-chlor-5-fluor-benzylamid (350 mg) in tert-Butanol (10 mL) gibt man langsam bei 0°C Natriumethoxid (20% in Ethanol) (0.91 mL) und tert-Butylhydroperoxid (70%ige wässrige Lösung; 0.37 mL). Nach beendeter Zugabe wird die Mischung für 1 h bei 75°C gerührt und nach Vervollständigung flüchtige Bestandteile im Vakuum entfernt. Der Rückstand wird mit Wasser und Ethylacetat aufgenommen, die organische Phase wird wie beschrieben behandelt und der Rückstand an der chiralen HPLC aufgereinigt.

"A66":
LCMS: Mass found (M+1, 391.0)
Methode A: 0.1% TFA in $H_2O$, B: 0.1% TFA in ACN: Flow - 2.0 mL/min.
Column: X Bridge C8 (50 x 4.6 mm, 3.5μm) + ve mode
Rt (min): 4.86 Area % 98.24 (Max), 94.59 (254nm)
HPLC:
Methode A: 0.1%TFA in $H_2O$, B: 0.1%TFA in ACN, Flow Rate: 2.0 mL/min
Column: X Bridge C8 (50 x 4.6) mm, 3.5 μm
Rt (min) : 5.01 Area % 99.39 (Max), 96.44 (254 nm);
$^1H$ NMR (400 MHz, DMSO-$d_6$) δ [ppm] 8.62 (t, *J* = 6.2 Hz, 1H), 7.39 (m, 2H), 7.26 (m, 2H), 7.22 (s, 1H), 7.17 (m, 2H), 7.13 (dd, J= 9.3, 1.8 Hz, 1H), 5.85 (s, 1 H), 4.37 (dd, 1 H), 4.27 (dd, 1 H), 3.95 (dd, J = 14.5, 9.0 Hz, 1 H), 3.77 (dd, *J* = 14.8, 5.4 Hz, 1 H), 2.26 (m, 2H), 1.82 (m, 4H), 1.66 (m, 1 H).
"A67":
LCMS: Mass found (M+1, 391.0)
Methode A: 0.1% TFA in $H_2O$, B: 0.1% TFA in ACN: Flow- 2.0 mL/min.
Column: X Bridge C8 (50 x 4.6 mm.3.5μm) + ve mode
Rt (min): 4.86 Area % 98.09 (Max), 95.22 (254nm)
HPLC:
Methode A: 0.1%TFA in $H_2O$, B: 0.1%TFA in ACN, Flow Rate -2.0 mL/min
Column: XBridge C8 (50 x 4.6 mm, 3.5 μm)
Rt (min): 5.01 Area % 99.15 (Max), 96.19 (254 nm);
$^1H$ NMR (400 MHz, DMSO-$d_6$) δ [ppm] 8.62 (t, *J* = 6.2 Hz, 1H), 7.43 - 7.35 (m, 2H), 7.30 - 7.23 (m, 2H), 7.22 (s, 1H), 7.19 - 7.15 (m, 2H), 7.13 (d, *J* = 9.7 Hz, 1 H), 5.85 (s, 1 H), 4.37 (dd, *J* = 15.9, 6.4 Hz, 1 H), 4.27 (dd, *J* = 15.8, 6.0 Hz, 1 H), 4.01 - 3.88 (m, 1 H), 3.78 (dd, *J* = 14.7, 5.6 Hz, 1 H), 2.25 (m, 1 H), 1.90 - 1.72 (m, 4H), 1.66 (m, 1 H).

Analog werden die nachstehenden Verbindungen erhalten

| Nr. | Struktur / Name | $^1$H NMR (400 MHz, DMSO-$d_6$) δ [ppm] * (DMSO-$d_6$ + TFA-$d_1$) ** 500 MHz | LC-MS; rt; [M+H$^+$] |
|---|---|---|---|
| "A56" | 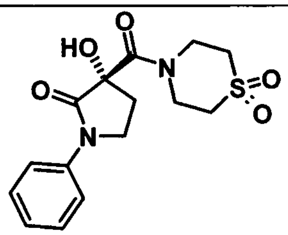 | | 3.73 [475.3] |

| | | | |
|---|---|---|---|
| | (S)-3-(1,1-Dioxo-1l6-thiomorpholin-4-carbonyl)-3-hydroxy-1-phenyl-pyrrolidin-2-on | | |
| "A57" | 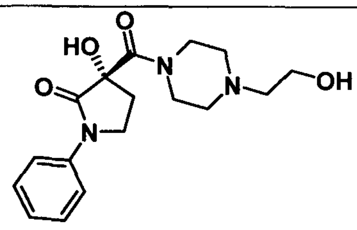 (S)-3-Hydroxy-3-[4-(2-hydroxy-ethyl)-piperazin-1-carbonyl]-1-phenyl-pyrrolidin-2-on | | 1.87 [334.3] |
| "A58" | 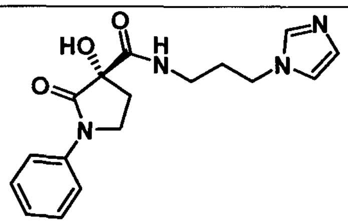 (S)-3-Hydroxy-2-oxo-1-phenyl-pyrrolidin-3-carbonsäure-(3-imidazol-1-yl-propyl)-amid | | 1.87 [329.3] |
| "A59" | 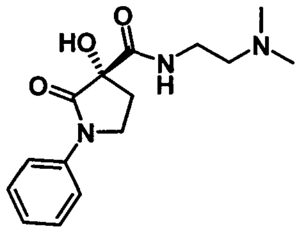 (S)-3-Hydroxy-2-oxo-1-phenyl-pyrrolidin-3-carbonsäure-(2-dimethylamino-ethyl)-amid | | 1.82 [292.3] |
| "A60" | 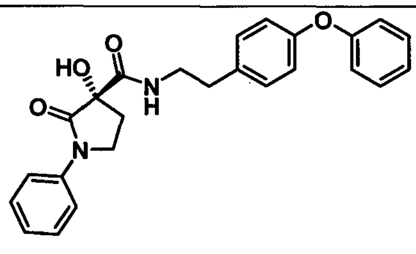 | | 4.75 [417.3] |

| | | | |
|---|---|---|---|
| | (S)-3-Hydroxy-2-oxo-1-phenyl-pyrrolidin-3-carbonsäure-[2-(4-phenoxy-phenyl)-ethyl]-amid | | |
| "A61" | 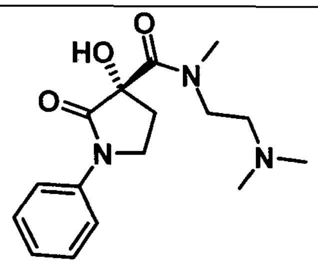 (S)-3-Hydroxy-2-oxo-1-phenyl-pyrrolidin-3-carbonsäure-(2-dimethylamino-ethyl)-methyl-amid | | 1.94 [306.3] |
| "A62" | 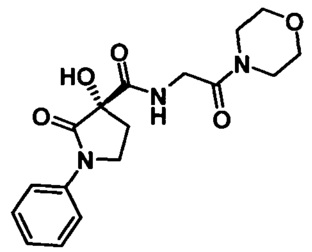 (S)-3-Hydroxy-2-oxo-1-phenyl-pyrrolidin-3-carbonsäure-(2-morpholin-4-yl-2-oxo-ethyl)-amid | | 2.18 [348.3] |
| "A63" | 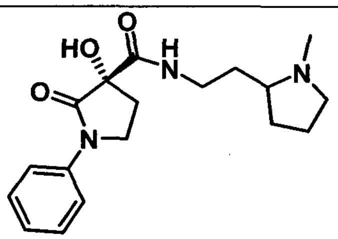 (S)-3-Hydroxy-2-oxo-1-phenyl-pyrrolidin-3-carbonsäure-[2-(1-methyl-pyrrolidin-2-yl)-ethyl]-amid | | 1.99 [332.3] |
| "A64" | 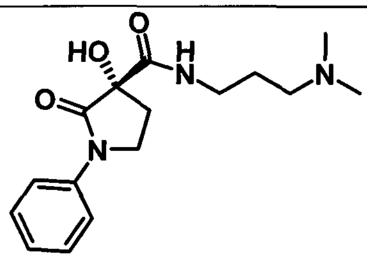 | | 1.86 [306.3] |

| | | | |
|---|---|---|---|
| | (S)-3-Hydroxy-2-oxo-1-phenyl-pyrrolidin-3-carbonsäure-(3-dimethylamino-propyl)-amid | | |
| "A65" | 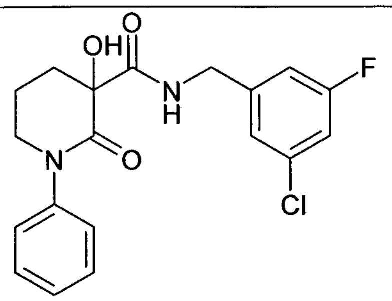 3-Hydroxy-2-oxo-1-phenyl-piperidin-3-carbonsäure-3-chlor-5-fluor-benzylamid | 8.62 (t, $J$ = 6.2 Hz, 1H), 7.39 (m, 2H), 7.26 (m, 2H), 7.22 (s, 1H), 7.17 (m, 2H), 7.13 (dd, $J$ = 9.3, 1.8 Hz, 1H), 5.85 (s, 1H), 4.37 (dd, 1H), 4.27 (dd, 1H), 3.95 (dd, $J$ = 14.5, 9.0 Hz, 1H), 3.77 (dd, $J$ = 14.8, 5.4 Hz, 1H), 2.26 (m, 2H), 1.82 (m, 4H), 1.66 (m, 1H) | 4.01 [377] |
| "A66" | 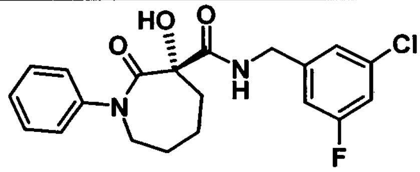 (S)-3-Hydroxy-2-oxo-1-phenyl-azepan-3-carbonsäure-3-chlor-5-fluor-benzylamid | 8.62 (t, $J$ = 6.2 Hz, 1H), 7.43 – 7.35 (m, 2H), 7.30 – 7.23 (m, 2H), 7.22 (s, 1H), 7.19 – 7.15 (m, 2H), 7.13 (d, $J$ = 9.7 Hz, 1H), 5.85 (s, 1H), 4.37 (dd, $J$ = 15.9, 6.4 Hz, 1H), 4.27 (dd, $J$ = 15.8, 6.0 Hz, 1H), 4.01 – 3.88 (m, 1H), 3.78 (dd, $J$ = 14.7, 5.6 Hz, 1H), 2.25 (m, 1H), 1.90 – 1.72 (m, 4H), 1.66 (m, 1H). | 5.01 [391] |
| "A67" | 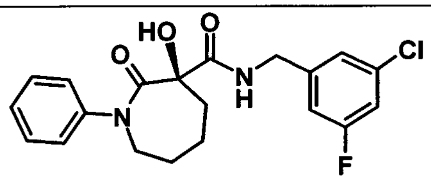 (R)-3-Hydroxy-2-oxo-1-phenyl-azepan-3-carbonsäure-3-chlor-5-fluor-benzylamid | 11.11 (s, 1H), 8.71 (t, $J$ = 6.4 Hz, 1H), 7.84 – 7.74 (m, 1H), 7.53 (d, $J$ = 8.6 Hz, 1H), 7.38 – 7.29 (m, 1H), 7.29 – 7.20 (m, 3H), 7.12 (d, $J$ = 9.7 Hz, 1H), 6.71 (s, 1H), 6.40 (ddd, $J$ = 2.9, 1.9, 0.8 Hz, 1H), 4.40 (dd, $J$ = 15.8, 6.7 Hz, 1H), 4.25 (dd, $J$ = 15.8, 6.0 Hz, 1H), 3.89 (t, $J$ = 6.8 Hz, 2H), 2.64 – 2.56 (m, 1H), 2.13 (m, 1H). | 5.01 [391] |

| | | | |
|---|---|---|---|
| "A68" | 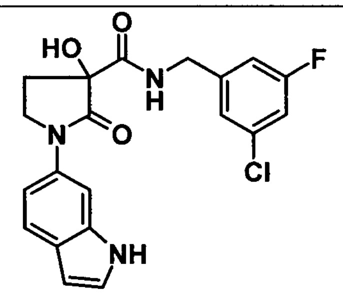 3-Hydroxy-1-(1H-indol-6-yl)-2-oxo-pyrrolidin-3-carbonsäure-3-chlor-5-fluor-benzylamid | 10.44 (s, 1H), 8.77 (s, 1H), 8.20 (d, *J* = 20.2 Hz, 2H), 7.96 (dd, *J* = 22.7, 8.6 Hz, 3H), 7.79 (d, *J* = 7.7 Hz, 1H), 7.58 (dd, *J* = 14.7, 7.5 Hz, 2H), 7.42 (t, *J* = 7.9 Hz, 1H), 7.35 (s, 1H), 7.27 (d, *J* = 9.0 Hz, 1H), 7.21 (s, 1H), 7.11 (d, *J* = 9.7 Hz, 1H), 6.83 (s, 1H), 4.39 (d, *J* = 14.9 Hz, 1H), 4.26 (d, *J* = 15.6 Hz, 1H), 3.95 (t, *J* = 6.7 Hz, 2H), 2.69 – 2.57 (m, 1H), 2.22 – 2.10 (m, 1H). | 4.13 [402] |
| "A69" | 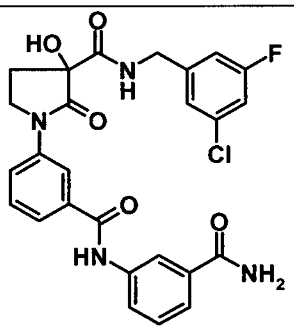 1-[3-(3-Carbamoyl-phenylcarbamoyl)-phenyl]-3-hydroxy-2-oxo-pyrrolidin-3-carbonsäure-3-chlor-5-fluor-benzylamid | 7.70 – 7.65 (m, 2H), 7.43 – 7.37 (m, 2H), 7.21 – 7.15 (m, 1H), 6.83 – 6.76 (m, 1H), 6.65 (s, 1H), 3.84 (td, *J* = 9.2, 3.0 Hz, 1H), 3.73 (dt, *J* = 9.6, 7.5 Hz, 1H), 2.57 – 2.50 (m, 1H), 2.13 – 2.03 (m, 1H), 1.90 (dd, *J* = 6.7, 1.5 Hz, 3H). | 3.63 [525.2] |
| "A70" | 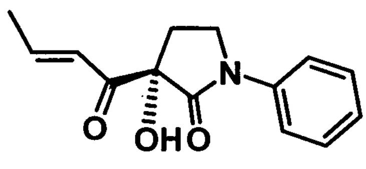 (S)-3-((E)-But-2-enoyl)-3-hydroxy-1-phenyl-pyrrolidin-2-on | 8.62 (t, *J* = 6.2 Hz, 1H), 7.39 (m, 2H), 7.26 (m, 2H), 7.22 (s, 1H), 7.17 (m, 2H), 7.13 (dd, *J* = 9.3, 1.8 Hz, 1H), 5.85 (s, 1H), 4.37 (dd, 1H), 4.27 (dd, 1H), 3.95 (dd, *J* = 14.5, 9.0 Hz, 1H), 3.77 (dd, *J* = 14.8, 5.4 Hz, 1H), 2.26 (m, 2H), 1.82 (m, 4H), 1.66 (m, 1H) | 3.26 [246] |

| | | | |
|---|---|---|---|
| "A71" | 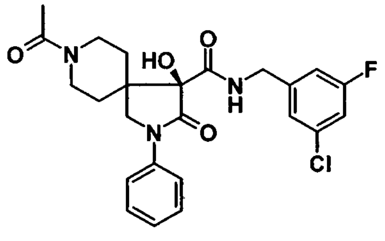 (S)-8-Acetyl-4-hydroxy-3-oxo-2-phenyl-2,8-diaza-spiro[4.5]decan-4-carbonsäure-3-chlor-5-fluor-benzylamid | 8.78 (dd, *J* = 13.6, 6.5 Hz, 1H), 7.70 (d, *J* = 8.5 Hz, 2H), 7.40 (t, *J* = 8.0 Hz, 2H), 7.27 (d, *J* = 8.7 Hz, 1H), 7.18 (dd, *J* = 13.1, 5.8 Hz, 2H), 7.09 (d, *J* = 9.7 Hz, 1H), 6.87 (d, *J* = 3.9 Hz, 1H), 4.39 – 4.27 (m, 1H), 4.25 – 4.13 (m, 2H), 4.08 (m, 1H), 3.86 (dd, *J* = 9.5, 5.1 Hz, 1H), 3.74 (m, 2H), 3.67 (m, 1H), 3.41 – 3.33 (m, 1H), 3.12 (m, 1H), 2.93 (m, 1H), 2.75 (s, 1H), 1.86 – 1.74 (m, 1H), 1.66 (m, 1H), 1.57 – 1.18 (m, 5H). | 4.04 [474] |
| "A72" | 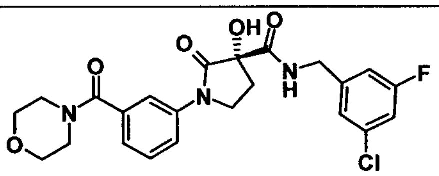 (S)-3-Hydroxy-1-[3-(morpholin-4-carbonyl)-phenyl]-2-oxo-pyrrolidin-3-carbonsäure-3-chlor-5-fluor-benzylamid | 8.74 (t, *J* = 6.3 Hz, 1H), 7.80 (s, 1H), 7.74 (d, *J* = 8.1 Hz, 1H), 7.48 (t, *J* = 7.9 Hz, 1H), 7.27 (d, *J* = 8.7 Hz, 1H), 7.21 (d, *J* = 4.1 Hz, 2H), 7.10 (d, *J* = 9.7 Hz, 1H), 6.79 (s, 1H), 4.38 (dd, *J* = 15.6, 6.7 Hz, 1H), 4.24 (dd, *J* = 15.6, 5.9 Hz, 1H), 3.88 (t, *J* = 7.0 Hz, 2H), 3.61 (m, 6H), 3.38 (m, 2H), 2.65 – 2.55 (m, 1H), 2.22 – 2.08 (m, 1H). | 3.67 [476] |
| "A73" | 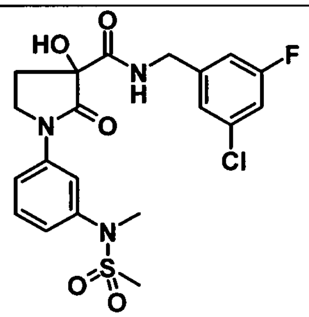 3-Hydroxy-1-[3-(methansulfonyl-methyl-amino)-phenyl]-2-oxo- | 8.73 (t, *J* = 6.4 Hz, 1H), 7.77 (t, *J* = 2.1 Hz, 1H), 7.63 (dd, *J* = 8.3, 1.3 Hz, 1H), 7.44 (t, *J* = 8.1 Hz, 1H), 7.29 – 7.25 (m, 1H), 7.24 (dd, *J* = 8.5, 1.8 Hz, 1H), 7.20 (s, 1H), 7.10 (d, *J* = 9.7 Hz, 1H), 6.79 (s, 1H), 4.37 (dd, *J* = 15.7, 6.7 Hz, 1H), 4.25 (dd, *J* = 15.7, 6.0 Hz, 1H), 3.87 | 3.96 [470] |

| | | | |
|---|---|---|---|
| | pyrrolidin-3-carbonsäure-3-chlor-5-fluor-benzylamid | (dd, J = 8.8, 5.7 Hz, 2H), 3.24 (s, 3H), 2.59 (dt, J = 11.8, 5.6 Hz, 1H), 2.13 (dt, J = 12.9, 7.6 Hz, 1H). | |
| "A74" | 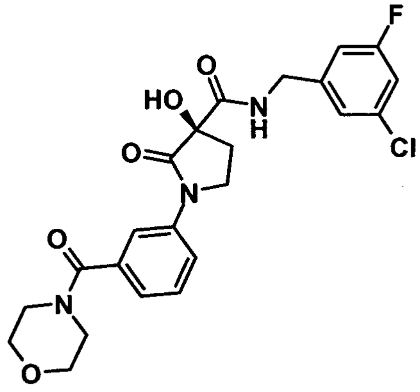 (R)-3-Hydroxy-1-[3-(morpholin-4-carbonyl)-phenyl]-2-oxo-pyrrolidin-3-carbonsäure-3-chlor-5-fluor-benzylamid | 8.74 (t, J = 6.4 Hz, 1H), 7.82 – 7.78 (m, 1H), 7.73 (ddd, J = 8.3, 2.3, 0.9 Hz, 1H), 7.48 (t, J = 7.9 Hz, 1H), 7.27 (dt, J = 8.8, 2.2 Hz, 1H), 7.23 – 7.19 (m, 2H), 7.10 (d, J = 8.8 Hz, 1H), 6.80 (s, 1H), 4.38 (dd, J = 15.7, 6.7 Hz, 1H), 4.24 (dd, J = 15.8, 6.0 Hz, 1H), 3.88 (dd, J = 8.4, 5.7 Hz, 2H), 3.61 (m, 7H), 3.43 – 3.35 (m, 1H), 2.64 – 2.55 (m, 1H), 2.13 (dt, J = 12.9, 7.6 Hz, 1H). | 3.67 [476] |
| "A75" | 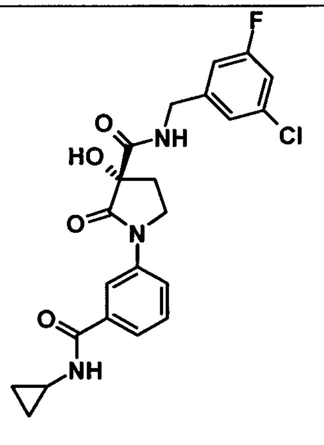 (S)-1-(3-Cyclopropylcarbamoyl-phenyl)-3-hydroxy-2-oxo-pyrrolidin-3-carbonsäure-3-chlor-5-fluor-benzylamid | 8.75 (t, J = 6.4 Hz, 1H), 8.49 (d, J = 4.0 Hz, 1H), 7.98 (t, J = 1.8 Hz, 1H), 7.91 (dd, J = 8.1, 1.4 Hz, 1H), 7.60 (d, J = 7.8 Hz, 1H), 7.47 (t, J = 7.9 Hz, 1H), 7.27 (dt, J = 8.7, 2.1 Hz, 1H), 7.20 (s, 1H), 7.10 (d, J = 9.5 Hz, 1H), 6.80 (s, 1H), 4.38 (dd, J = 15.8, 6.7 Hz, 1H), 4.25 (dd, J = 15.7, 6.1 Hz, 1H), 3.89 (t, J = 6.9 Hz, 2H), 2.91 – 2.77 (m, 1H), 2.64 – 2.54 (m, 1H), 2.14 (dt, J = 13.0, 7.7 Hz, 1H), 0.69 (td, J = 7.1, 4.7 Hz, 2H), 0.59 – 0.53 (m, 2H). | 3.84 [446] |

| | | | |
|---|---|---|---|
| "A76" | 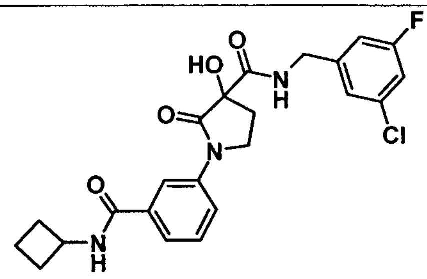 1-(3-Cyclobutylcarbamoyl-phenyl)-3-hydroxy-2-oxo-pyrrolidin-3-carbnsäure-3-chlor-5-fluor-benzylamid | 8.75 (t, *J* = 6.3 Hz, 1H), 8.66 (d, *J* = 7.5 Hz, 1H), 8.00 (s, 1H), 7.91 (d, *J* = 8.1 Hz, 1H), 7.64 (d, *J* = 7.8 Hz, 1H), 7.48 (t, *J* = 7.9 Hz, 1H), 7.27 (dd, *J* = 8.7, 2.1 Hz, 1H), 7.20 (s, 1H), 7.10 (d, *J* = 9.5 Hz, 1H), 6.80 (s, 1H), 4.48 – 4.33 (m, 2H), 4.25 (dd, *J* = 15.7, 6.0 Hz, 1H), 3.91 (t, *J* = 6.9 Hz, 2H), 2.65 – 2.55 (m, 1H), 2.27 – 1.98 (m, 5H), 1.74 – 1.61 (m, 2H). | 4.07 [460] |
| "A77" | 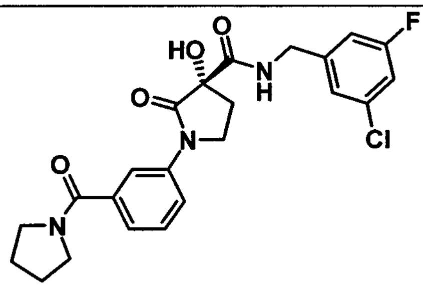 (S)-3-Hydroxy-2-oxo-1-[3-(pyrrolidin-1-carbonyl)-phenyl]-pyrrolidin-3-carbonsäure-3-chlor-5-fluor-benzylamid | 8.75 (t, *J* = 6.4 Hz, 1H), 7.88 (s, 1H), 7.72 (dd, *J* = 8.2, 1.4 Hz, 1H), 7.46 (t, *J* = 7.9 Hz, 1H), 7.32 – 7.25 (m, 2H), 7.20 (s, 1H), 7.10 (d, *J* = 9.6 Hz, 1H), 6.79 (s, 1H), 4.38 (dd, *J* = 15.8, 6.7 Hz, 1H), 4.24 (dd, *J* = 15.8, 5.9 Hz, 1H), 3.92 – 3.84 (m, 2H), 3.46 (t, *J* = 6.8 Hz, 2H), 3.37 (t, *J* = 6.5 Hz, 2H), 2.59 (dt, *J* = 6.9, 5.7 Hz, 1H), 2.13 (dt, *J* = 13.0, 7.6 Hz, 1H), 1.91 – 1.74 (m, 4H). | 3.97 [460.2] |
| "A78" | 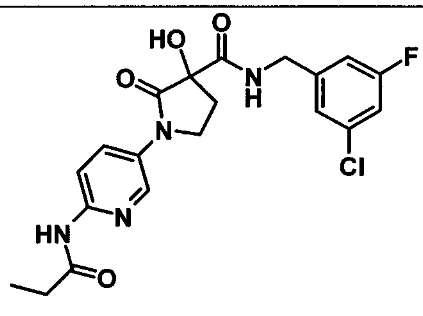 3-Hydroxy-2-oxo-1-(6-propionylamino-pyridin-3-yl)- | 10.48 (s, 1H), 8.75 (t, *J* = 6.4 Hz, 1H), 8.65 (s, 1H), 8.09 (dt, *J* = 9.1, 5.8 Hz, 2H), 7.27 (d, *J* = 8.7 Hz, 1H), 7.20 (s, 1H), 7.10 (d, *J* = 9.4 Hz, 1H), 6.80 (s, 1H), 4.38 (dd, *J* = 15.7, 6.7 Hz, 1H), 4.24 (dd, *J* = 15.7, 6.0 Hz, 1H), 3.86 (t, *J* = 6.1 Hz, | 3.39 [435.2] |

| | | | |
|---|---|---|---|
| | pyrrolidin-3-carbonsäure-3-chlor-5-fluor-benzylamid | 2H), 2.65 – 2.56 (m, 1H), 2.38 (q, *J* = 7.5 Hz, 2H), 2.21 – 2.07 (m, 1H), 1.06 (t, *J* = 7.5 Hz, 3H).. | |
| "A79" | 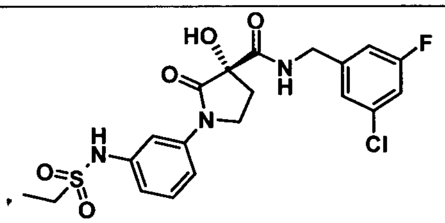 (S)-1-(3-Ethansulfonylamino-phenyl)-3-hydroxy-2-oxo-pyrrolidin-3-carbonsäure-3-chlor-5-fluor-benzylamid | 9.88 (s, 1H), 8.73 (t, *J* = 6.4 Hz, 1H), 7.70 (d, *J* = 1.7 Hz, 1H), 7.37 – 7.30 (m, 2H), 7.27 (dt, *J* = 8.8, 2.2 Hz, 1H), 7.20 (s, 1H), 7.09 (d, *J* = 8.9 Hz, 1H), 7.05 – 7.00 (m, 1H), 6.79 (s, 1H), 4.37 (dd, *J* = 16.0, 6.8 Hz, 1H), 4.24 (dd, *J* = 15.9, 6.0 Hz, 1H), 3.81 (dd, *J* = 13.0, 5.7 Hz, 2H), 3.10 (q, *J* = 7.4 Hz, 2H), 2.63 – 2.53 (m, 1H), 2.18 – 2.05 (m, 1H), 1.18 (t, *J* = 7.3 Hz, 3H). | 3.94 [470] |
| "A80" | 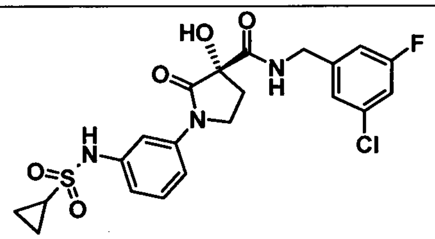 (S)-1-(3-Cyclopropan-sulfonylamino-phenyl)-3-hydroxy-2-oxo-pyrrolidin-3-carbonsäure-3-chlor-5-fluor-benzylamid | 9.82 (s, 1H), 8.73 (t, *J* = 6.4 Hz, 1H), 7.73 (s, 1H), 7.37 – 7.30 (m, 2H), 7.29 – 7.25 (m, 1H), 7.20 (s, 1H), 7.10 (d, *J* = 9.6 Hz, 1H), 7.06 – 7.02 (m, 1H), 6.79 (s, 1H), 4.38 (dd, *J* = 15.8, 6.6 Hz, 1H), 4.24 (dd, *J* = 15.7, 6.0 Hz, 1H), 3.82 (m, 2H), 3.29 (m, 1H), 2.59 (m, 2H), 2.19 – 2.05 (m, 1H), 1.00 – 0.89 (m, 4H). | 4.05 [482] |
| "A81" | 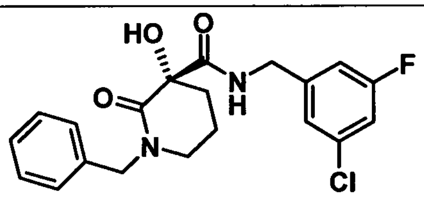 | 8.60 (t, *J* = 6.3 Hz, 1H), 7.36 – 7.29 (m, 2H), 7.26 (d, *J* = 7.6 Hz, 5H), 7.16 (d, *J* = 9.9 Hz, 1H), 6.38 (s, 1H), 4.59 (d, *J* = 15.1 Hz, 1H), 4.47 (d, *J* = 15.1 Hz, 1H), 4.40 (dd, *J* = 15.9, 7.0 | 4.55 [391.2] |

| | | | |
|---|---|---|---|
| | (S)-1-Benzyl-3-hydroxy-2-oxo-piperidin-3-carbonsäure-3-chlor-5-fluor-benzylamid | Hz, 1H), 4.24 (dd, *J* = 15.9, 5.7 Hz, 1H), 3.28 – 3.15 (m, 2H), 2.25 – 2.11 (m, 1H), 1.83 (m, 3H), 1.21 (m, 2H). | |
| "A82" | 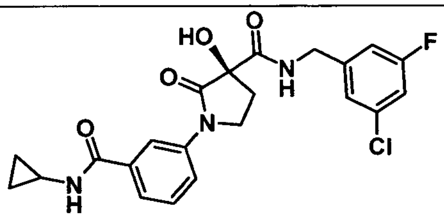 (R)-1-(3-Cyclopropylcarbamoyl-phenyl)-3-hydroxy-2-oxo-pyrrolidin-3-carbonsäure-3-chlor-5-fluor-benzylamid | 8.75 (t, *J* = 6.4 Hz, 1H), 8.49 (d, *J* = 4.0 Hz, 1H), 7.98 (s, 1H), 7.94 – 7.87 (m, 1H), 7.60 (d, *J* = 7.8 Hz, 1H), 7.47 (t, *J* = 7.9 Hz, 1H), 7.27 (dt, *J* = 8.8, 2.1 Hz, 1H), 7.20 (s, 1H), 7.10 (d, *J* = 9.4 Hz, 1H), 6.80 (s, 1H), 4.38 (dd, *J* = 15.7, 6.7 Hz, 1H), 4.25 (dd, *J* = 15.7, 6.0 Hz, 1H), 3.89 (t, *J* = 6.8 Hz, 2H), 2.89 – 2.78 (m, 1H), 2.64 – 2.55 (m, 1H), 2.14 (dt, *J* = 13.0, 7.6 Hz, 1H), 0.69 (td, *J* = 7.1, 4.7 Hz, 2H), 0.60 – 0.50 (m, 2H). | 3.84 [446] |
| "A83" | 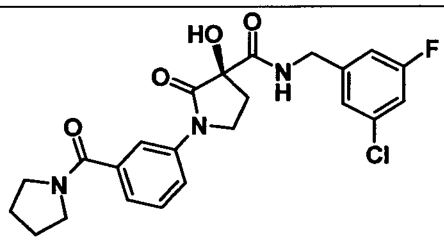 (R)-3-Hydroxy-2-oxo-1-[3-(pyrrolidin-1-carbonyl)-phenyl]-pyrrolidin-3-carbonsäure-3-chlor-5-fluor-benzylamid | 8.75 (t, *J* = 6.4 Hz, 1H), 7.88 (s, 1H), 7.76 – 7.69 (m, 1H), 7.46 (t, *J* = 7.9 Hz, 1H), 7.29 (m, 2H), 7.24 – 7.15 (m, 1H), 7.10 (d, *J* = 9.2 Hz, 1H), 6.79 (s, 1H), 4.38 (dd, *J* = 15.7, 6.7 Hz, 1H), 4.24 (dd, *J* = 15.8, 5.9 Hz, 1H), 3.92 – 3.85 (m, 1H), 3.46 (t, *J* = 6.8 Hz, 1H), 3.37 (t, *J* = 6.5 Hz, 2H), 2.64 – 2.55 (m, 1H), 2.13 (dt, *J* = 13.0, 7.8 Hz, 1H), 1.92 – 1.74 (m, 3H). | 3.97 [460.2] |

| | | | |
|---|---|---|---|
| "A84" | 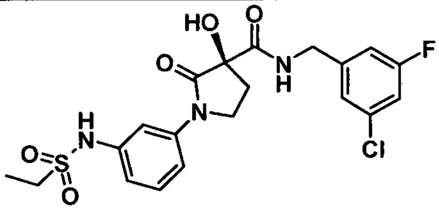 (R)-1-(3-Ethansulfonylamino-phenyl)-3-hydroxy-2-oxo-pyrrolidin-3-carbonsäure-3-chlor-5-fluor-benzylamid | 9.88 (s, 1H), 8.73 (t, *J* = 6.4 Hz, 1H), 7.70 (s, 1H), 7.34 (m, 2H), 7.27 (dt, *J* = 8.8, 2.2 Hz, 1H), 7.20 (s, 1H), 7.09 (m, 1H), 7.03 (dt, *J* = 5.0, 2.2 Hz, 1H), 6.79 (s, 1H), 4.37 (dd, *J* = 15.8, 6.7 Hz, 1H), 4.24 (dd, *J* = 15.8, 6.0 Hz, 1H), 3.82 (dd, *J* = 13.0, 5.6 Hz, 2H), 3.10 (q, *J* = 7.4 Hz, 2H), 2.56 (dd, *J* = 13.0, 7.4 Hz, 1H), 2.16 – 2.06 (m, 1H), 1.18 (t, *J* = 7.4 Hz, 3H). | 3.94 [470] |
| "A85" | 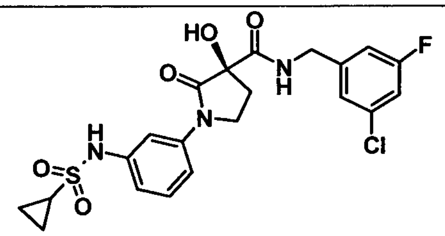 (R)-1-(3-Cyclopropan-sulfonylamino-phenyl)-3-hydroxy-2-oxo-pyrrolidin-3-carbonsäure-3-chlor-5-fluor-benzylamide | 9.82 (s, 1H), 8.73 (t, *J* = 6.4 Hz, 1H), 7.73 (s, 1H), 7.33 (d, *J* = 5.1 Hz, 1H), 7.27 (d, *J* = 8.7 Hz, 1H), 7.20 (s, 1H), 7.10 (d, *J* = 9.9 Hz, 1H), 7.04 (dd, *J* = 7.1, 4.0 Hz, 1H), 6.79 (s, 1H), 4.38 (dd, *J* = 15.6, 6.7 Hz, 1H), 4.24 (dd, *J* = 15.8, 5.9 Hz, 1H), 3.82 (m, 2H), 2.59 (m, 2H), 2.13 (m, 1H), 1.01 – 0.79 (m, 4H). | 4.05 [482] |
| "A86" | 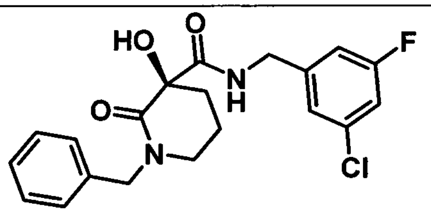 (R)-1-Benzyl-3-hydroxy-2-oxo-piperidin-3-carbonsäure-3-chlor-5-fluor-benzylamid | 8.60 (t, *J* = 6.1 Hz, 1H), 7.36 – 7.29 (m, 2H), 7.26 (d, *J* = 7.7 Hz, 5H), 7.16 (d, *J* = 9.8 Hz, 1H), 6.38 (s, 1H), 4.59 (d, *J* = 15.0 Hz, 1H), 4.47 (d, *J* = 15.3 Hz, 1H), 4.40 (dd, *J* = 15.9, 6.6 Hz, 1H), 4.24 (dd, *J* = 15.9, 5.6 Hz, 1H), 3.28 – 3.14 (m, 2H), 2.17 (dd, *J* = 15.1, 10.1 Hz, 1H), 1.83 (s, 3H). | 4.55 [391.2] |

| | | | |
|---|---|---|---|
| "A87" | 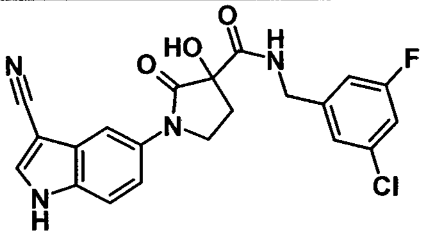 1-(3-Cyan-1H-indol-5-yl)-3-hydroxy-2-oxo-pyrrolidin-3-carbonsäure-3-chlor-5-fluor-benzylamid | 8.74 (t, *J* = 6.3 Hz, 1H), 8.27 (s, 1H), 7.90 (d, *J* = 1.8 Hz, 1H), 7.61 (dd, *J* = 8.9, 1.9 Hz, 1H), 7.57 (d, *J* = 8.9 Hz, 1H), 7.27 (d, *J* = 8.8 Hz, 1H), 7.22 (s, 1H), 7.11 (d, *J* = 9.9 Hz, 1H), 6.76 (s, 1H), 4.39 (dd, *J* = 15.7, 6.6 Hz, 1H), 4.25 (dd, *J* = 15.6, 6.0 Hz, 1H), 3.93 (t, *J* = 7.2 Hz, 2H), 2.65 – 2.57 (m, 1H), 2.20 – 2.08 (m, 1H). | 3.82 [427] |
| "A88" | 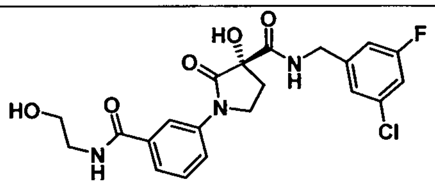 (S)-3-Hydroxy-1-[3-(2-hydroxy-ethylcarbamoyl)-phenyl]-2-oxo-pyrrolidin-3-carbonsäure-3-chlor-5-fluor-benzylamid | 8.75 (t, *J* = 6.3 Hz, 1H), 8.50 (t, *J* = 5.5 Hz, 1H), 8.02 (s, 1H), 7.93 (d, *J* = 8.1 Hz, 1H), 7.65 (d, *J* = 7.8 Hz, 1H), 7.48 (t, *J* = 8.0 Hz, 1H), 7.27 (d, *J* = 8.7 Hz, 1H), 7.20 (s, 1H), 7.10 (d, *J* = 9.1 Hz, 1H), 6.80 (s, 1H), 4.73 (t, *J* = 5.6 Hz, 1H), 4.38 (dd, *J* = 15.6, 6.7 Hz, 1H), 4.25 (dd, *J* = 15.7, 6.0 Hz, 1H), 3.90 (t, *J* = 6.8 Hz, 2H), 3.50 (q, *J* = 6.1 Hz, 2H), 2.60 (dt, *J* = 11.8, 5.8 Hz, 1H), 2.21 – 2.08 (m, 1H). | 3.16 [450] |
| "A89" | 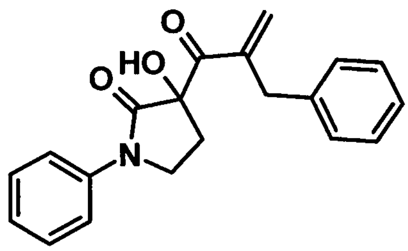 3-(2-Benzyl-acryloyl)-3-hydroxy-1-phenyl-pyrrolidin-2-on | | 4.63 [322] |

| | | | |
|---|---|---|---|
| "A90" | 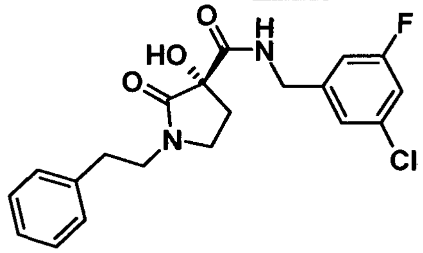 (S)-3-Hydroxy-2-oxo-1-phenethyl-pyrrolidin-3-carbonsäure-3-chlor-5-fluor-benzylamid | 8.61 (d, *J* = 6.4 Hz, 1H), 7.40 – 7.23 (m, 5H), 7.20 (s, 1H), 7.10 (d, *J* = 8.4 Hz, 1H), 6.53 (d, *J* = 15.8 Hz, 1H), 5.21 (dd, *J* = 10.1, 7.1 Hz, 1H), 4.37 (dd, *J* = 15.8, 6.8 Hz, 1H), 4.24 (dd, *J* = 15.8, 6.0 Hz, 1H), 3.04 – 2.93 (m, 1H), 2.45 – 2.36 (m, 1H), 2.00 – 1.84 (m, 1H), 1.50 (d, *J* = 7.2 Hz, 1H), 1.45 (d, *J* = 7.2 Hz, 1H). | 4.36 [391] |
| "A91" | 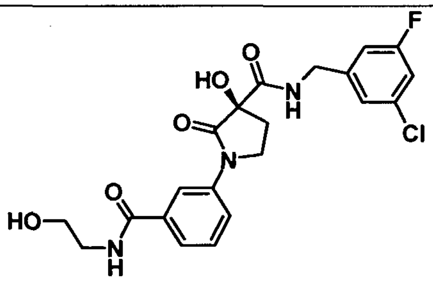 (R)-3-Hydroxy-1-[3-(2-hydroxy-ethylcarbamoyl)-phenyl]-2-oxo-pyrrolidin-3-carbonsäure-3-chlor-5-fluor-benzylamid | 8.75 (t, *J* = 6.4 Hz, 1H), 8.50 (t, *J* = 5.6 Hz, 1H), 8.02 (s, 1H), 7.96 – 7.88 (m, 1H), 7.65 (d, *J* = 7.8 Hz, 1H), 7.48 (t, *J* = 7.9 Hz, 1H), 7.27 (dd, *J* = 8.8, 2.1 Hz, 1H), 7.20 (s, 1H), 7.10 (d, *J* = 9.5 Hz, 1H), 6.80 (s, 1H), 4.73 (t, *J* = 5.6 Hz, 1H), 4.38 (dd, *J* = 15.7, 6.8 Hz, 1H), 4.25 (dd, *J* = 15.7, 6.0 Hz, 1H), 3.90 (t, *J* = 6.8 Hz, 2H), 3.50 (q, *J* = 6.1 Hz, 2H), 2.65 – 2.56 (m, 1H), 2.20 – 2.09 (m, 1H). | 3.16 [450] |
| "A92" | 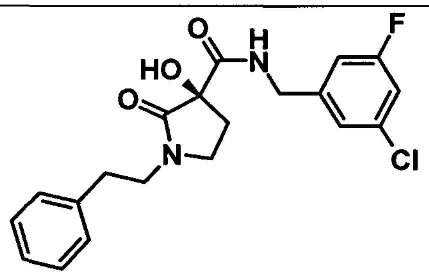 (R)-3-Hydroxy-2-oxo-1-phenethyl-pyrrolidin-3-carbonsäure-3-chlor-5-fluor-benzylamid | 8.61 (t, *J* = 6.3 Hz, 1H), 7.40 – 7.23 (m, 5H), 7.20 (s, 1H), 7.10 (d, *J* = 9.8 Hz, 1H), 6.51 (s, 1H), 5.20 (q, *J* = 7.4 Hz, 1H), 4.37 (dd, *J* = 15.8, 6.7 Hz, 1H), 4.24 (dd, *J* = 15.8, 6.0 Hz, 1H), 3.40 – 3.33 (m, 1H), 2.98 (td, *J* = 8.8, 3.4 Hz, 1H), 2.45 – 2.35 (m, 1H), 1.96 – 1.83 (m, 1H), 1.45 (d, *J* = 7.2 Hz, 2H). | 4.36 [391] |

| | | | |
|---|---|---|---|
| "A93" | 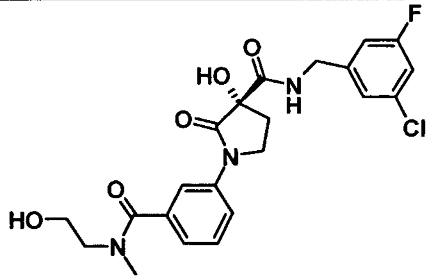 (S)-3-Hydroxy-1-{3-[(2-hydroxy-ethyl)-methyl-carbamoyl]-phenyl}-2-oxo-pyrrolidin-3-carbonsäure-3-chlor-5-fluor-benzylamid | 8.74 (t, *J* = 6.4 Hz, 1H), 7.73 (s, 2H), 7.45 (d, *J* = 7.3 Hz, 1H), 7.31 – 7.25 (m, 1H), 7.19 (d, *J* = 7.4 Hz, 2H), 7.10 (d, *J* = 9.6 Hz, 1H), 6.79 (s, 1H), 4.79 (t, *J* = 5.5 Hz, 1H), 4.38 (dd, *J* = 15.8, 6.7 Hz, 1H), 4.24 (dd, *J* = 15.8, 6.0 Hz, 1H), 3.87 (m, 2H), 3.61 (m, 1H), 3.48 (m, 2H), 3.26 (m, 1H), 2.95 (s, 3H), 2.58 (m, 1H), 2.20 – 2.07 (m, 1H). | 3.21 [464] |
| "A94" | 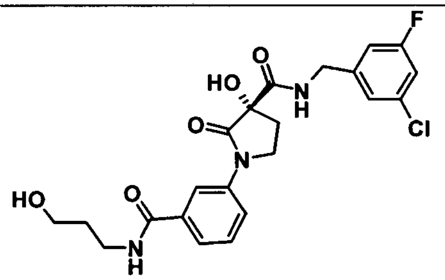 (S)-3-Hydroxy-1-[3-(3-hydroxy-propylcarbamoyl)-phenyl]-2-oxo-pyrrolidin-3-carbonsäure-3-chlor-5-fluor-benzylamid | 8.75 (t, *J* = 6.4 Hz, 1H), 8.50 (t, *J* = 5.6 Hz, 1H), 8.01 (d, *J* = 1.7 Hz, 1H), 7.95 – 7.88 (m, 1H), 7.63 (d, *J* = 7.8 Hz, 1H), 7.48 (t, *J* = 7.9 Hz, 1H), 7.27 (dt, *J* = 8.7, 2.1 Hz, 1H), 7.20 (s, 1H), 7.10 (d, *J* = 9.5 Hz, 1H), 6.80 (s, 1H), 4.47 (t, *J* = 5.2 Hz, 1H), 4.38 (dd, *J* = 15.8, 6.7 Hz, 1H), 4.25 (dd, *J* = 15.8, 6.1 Hz, 1H), 3.90 (t, *J* = 6.8 Hz, 2H), 3.45 (dd, *J* = 11.6, 6.2 Hz, 2H), 3.30 – 3.26 (m, 1H), 2.65 – 2.55 (m, 1H), 2.22 – 2.09 (m, 1H), 1.72 – 1.62 (m, 2H). | 3.24 [464.2] |
| "A95" | 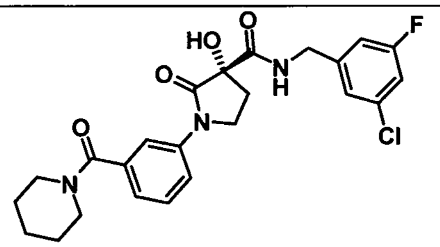 (S)-3-Hydroxy-2-oxo-1-[3-(piperidin-1-carbonyl)-phenyl]- | 8.75 (t, *J* = 6.4 Hz, 1H), 7.81 – 7.77 (m, 1H), 7.69 (dd, *J* = 8.3, 1.4 Hz, 1H), 7.47 (t, *J* = 7.9 Hz, 1H), 7.27 (dt, *J* = 8.8, 2.1 Hz, 1H), 7.20 (s, 1H), 7.16 (d, *J* = 7.7 Hz, 1H), 7.10 (d, *J* = 9.7 Hz, 1H), 6.79 (s, 1H), 4.38 (dd, | 4.15 [474] |

| | | | |
|---|---|---|---|
| | pyrrolidin-3-carbonsäure-3-chlor-5-fluor-benzylamid | J = 15.8, 6.6 Hz, 1H), 4.24 (dd, J = 15.8, 6.0 Hz, 1H), 3.94 – 3.83 (m, 2H), 3.57 (m, 2H), 3.26 (m, 2H), 2.64 – 2.55 (m, 1H), 2.13 (dt, J = 13.0, 7.6 Hz, 1H), 1.69 – 1.34 (m, 6H). | |
| "A96" | 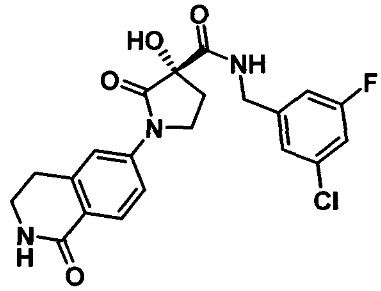 (S)-3-Hydroxy-2-oxo-1-(1-oxo-1,2,3,4-tetrahydro-isochinolin-6-yl)-pyrrolidin-3-carbonsäure-3-chlor-5-fluor-benzylamid | 8.75 (t, J = 6.4 Hz, 1H), 7.91 – 7.81 (m, 2H), 7.71 (dd, J = 8.6, 2.1 Hz, 1H), 7.63 (s, 1H), 7.27 (d, J = 8.7 Hz, 1H), 7.20 (s, 1H), 7.09 (d, J = 9.5 Hz, 1H), 6.82 (s, 1H), 4.38 (dd, J = 15.8, 6.7 Hz, 1H), 4.24 (dd, J = 15.8, 6.0 Hz, 1H), 3.89 (m, 2H), 3.40 – 3.33 (m, 2H), 2.91 (t, J = 6.6 Hz, 2H), 2.64 – 2.55 (m, 1H), 2.14 (dt, J = 13.0, 7.7 Hz, 1H). | 3.38 [432] |
| "A97" | 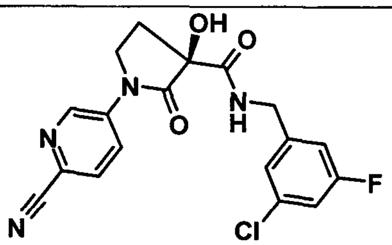 (S)-1-(6-Cyan-pyridin-3-yl)-3-hydroxy-2-oxo-pyrrolidin-3-carbonsäure-3-chlor-5-fluor-benzylamid | 9.12 (d, J = 2.4 Hz, 1H), 8.82 (t, J = 6.4 Hz, 1H), 8.38 (dd, J = 8.7, 2.6 Hz, 1H), 8.10 (d, J = 8.7 Hz, 1H), 7.28 (d, J = 8.8 Hz, 1H), 7.20 (s, 1H), 7.09 (d, J = 9.1 Hz, 1H), 6.96 (s, 1H), 4.37 (dd, J = 15.7, 6.8 Hz, 1H), 4.25 (dd, J = 15.7, 6.1 Hz, 1H), 4.02 – 3.85 (m, 2H), 2.66 – 2.58 (m, 1H), 2.19 (dt, J = 13.1, 7.7 Hz, 1H). | 3.84 [389] |

| | | | |
|---|---|---|---|
| "A98" | 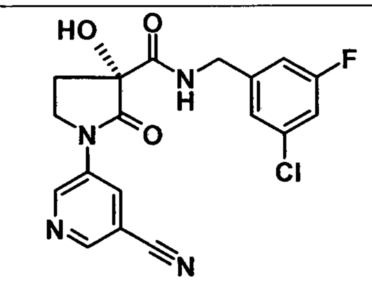 (R)-1-(5-Cyan-pyridin-3-yl)-3-hydroxy-2-oxo-pyrrolidin-3-carbonsäure-3-chlor-5-fluor-benzylamid | 9.28 (d, *J* = 2.6 Hz, 1H), 8.83 (d, *J* = 1.8 Hz, 1H), 8.80 (d, *J* = 6.4 Hz, 1H), 8.61 – 8.55 (m, 1H), 7.28 (dt, *J* = 8.8, 2.1 Hz, 1H), 7.20 (s, 1H), 7.09 (d, *J* = 9.5 Hz, 1H), 6.93 (s, 1H), 4.37 (dd, *J* = 15.7, 6.9 Hz, 1H), 4.24 (dd, *J* = 15.7, 6.0 Hz, 1H), 3.94 (m, 2H), 3.40 – 3.34 (m, 1H) 2.62 (m, 1H), 2.27 – 2.12 (m, 1H). | 3.63 [389] |
| "A99" | 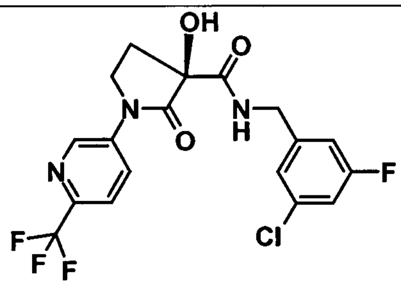 (S)-3-Hydroxy-2-oxo-1-(6-trifluormethyl-pyridin-3-yl)-pyrrolidin-3-carbonsäure-3-chlor-5-fluor-benzylamid | 9.10 (d, *J* = 2.4 Hz, 1H), 8.81 (t, *J* = 6.3 Hz, 1H), 8.44 (dd, *J* = 8.6, 2.2 Hz, 1H), 7.97 (d, *J* = 8.7 Hz, 1H), 7.28 (dt, *J* = 8.7, 2.1 Hz, 1H), 7.20 (s, 1H), 7.10 (d, *J* = 9.6 Hz, 1H), 6.94 (s, 1H), 4.38 (dd, *J* = 15.7, 6.8 Hz, 1H), 4.25 (dd, *J* = 15.7, 6.0 Hz, 1H), 4.02 – 3.91 (m, 2H), 3.40 – 3.34 (m, 1H), 2.65 – 2.55 (m, 1H), 2.20 (dt, *J* = 13.2, 7.8 Hz, 2H). | 4.44 [432] |
| "A100" | 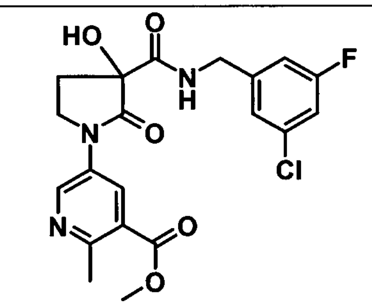 5-[3-(3-Chlor-5-fluor-benzylcarbamoyl)-3-hydroxy-2-oxo-pyrrolidin-1-yl]-2-methyl-nicotinsäure-methylester | 8.88 (d, *J* = 2.7 Hz, 1H), 8.79 (t, *J* = 6.4 Hz, 1H), 8.59 (d, *J* = 2.7 Hz, 1H), 7.28 (dt, *J* = 8.8, 2.1 Hz, 1H), 7.20 (s, 1H), 7.10 (d, *J* = 9.0 Hz, 1H), 6.87 (s, 1H), 4.38 (dd, *J* = 15.7, 6.7 Hz, 1H), 4.25 (dd, *J* = 15.7, 6.1 Hz, 1H), 3.94 (m, 2H), 3.87 (s, 3H), 2.69 (s, 3H), 2.61 (m, 1H), 2.21 – 2.11 (m, 1H). | 3.35 [436] |

| | | | |
|---|---|---|---|
| "A101" | 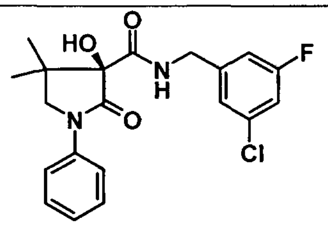 (S)-3-Hydroxy-4,4-dimethyl-2-oxo-1-phenyl-pyrrolidin-3-carbonsäure-3-chlor-5-fluor-benzylamid | 8.72 (t, *J* = 6.3 Hz, 1H), 7.65 (d, *J* = 7.7 Hz, 2H), 7.44 – 7.35 (m, 2H), 7.27 (dt, *J* = 8.8, 2.1 Hz, 1H), 7.20 (s, 1H), 7.16 (t, *J* = 7.4 Hz, 1H), 7.09 (d, *J* = 9.0 Hz, 1H), 6.68 (s, 1H), 4.31 (dd, *J* = 15.5, 6.5 Hz, 1H), 4.22 (dd, *J* = 15.5, 6.2 Hz, 1H), 3.77 (d, *J* = 8.9 Hz, 1H), 3.41 (d, *J* = 8.9 Hz, 1H), 1.06 (s, 3H), 1.01 (s, 3H). | 4.92 [391] |
| "A102" | 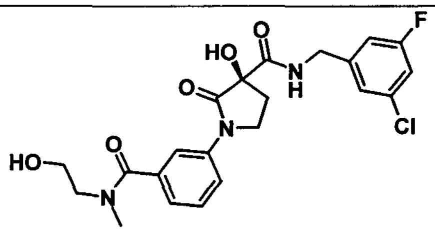 (R)-3-Hydroxy-1-{3-[(2-hydroxy-ethyl)-methyl-carbamoyl]-phenyl}-2-oxo-pyrrolidin-3-carbonsäure-3-chlor-5-fluor-benzylamid | 8.74 (t, *J* = 6.4 Hz, 1H), 7.73 (s, 2H), 7.45 (d, *J* = 7.2 Hz, 1H), 7.31 – 7.25 (m, 1H), 7.19 (d, *J* = 7.4 Hz, 2H), 7.10 (d, *J* = 9.7 Hz, 1H), 6.79 (s, 1H), 4.79 (t, *J* = 5.4 Hz, 1H), 4.38 (dd, *J* = 15.7, 6.7 Hz, 1H), 4.24 (dd, *J* = 15.7, 6.0 Hz, 1H), 3.87 (m, 2H), 3.62 (m, 1H), 3.49 (m, 2H), 3.27 (m, 1H), 2.95 (s, 3H), 2.64 – 2.53 (m, 1H), 2.21 – 2.05 (m, 1H). | 3.21 [464] |
| "A103" | 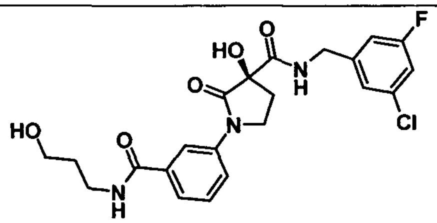 (R)-3-Hydroxy-1-[3-(3-hydroxy-propylcarbamoyl)-phenyl]-2-oxo-pyrrolidin-3-carbonsäure-3-chlor-5-fluor-benzylamid | 8.75 (t, *J* = 6.4 Hz, 1H), 8.50 (t, *J* = 5.6 Hz, 1H), 8.02 (s, 1H), 7.91 (d, *J* = 8.1 Hz, 1H), 7.63 (d, *J* = 7.8 Hz, 1H), 7.48 (t, *J* = 8.0 Hz, 1H), 7.27 (d, *J* = 8.8 Hz, 1H), 7.20 (s, 1H), 7.10 (d, *J* = 9.2 Hz, 1H), 6.80 (s, 1H), 4.47 (t, *J* = 5.2 Hz, 1H), 4.38 (dd, *J* = 15.7, 6.7 Hz, 1H), 4.25 (dd, *J* = 15.7, 6.0 Hz, 1H), 3.90 (t, *J* = 6.8 Hz, 2H), 3.45 (dd, *J* | 3.24 [464] |

| | | | |
|---|---|---|---|
| | | = 11.6, 6.2 Hz, 2H), 3.30 – 3.26 (m, 1H), 2.64 – 2.55 (m, 1H), 2.24 – 2.09 (m, 1H), 1.75 – 1.59 (m, 2H). | |
| "A104" | 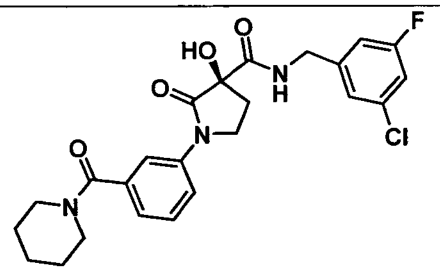 (R)-3-Hydroxy-2-oxo-1-[3-(piperidin-1-carbonyl)-phenyl]-pyrrolidin-3-carbonsäure-3-chlor-5-fluor-benzylamid | 8.75 (t, *J* = 6.3 Hz, 1H), 7.79 (s, 1H), 7.69 (d, *J* = 8.1 Hz, 1H), 7.47 (t, *J* = 7.9 Hz, 1H), 7.27 (d, *J* = 8.7 Hz, 1H), 7.20 (s, 1H), 7.16 (d, *J* = 7.6 Hz, 1H), 7.10 (d, *J* = 9.6 Hz, 1H), 6.79 (s, 1H), 4.38 (dd, *J* = 15.8, 6.8 Hz, 1H), 4.24 (dd, *J* = 15.8, 6.0 Hz, 1H), 3.88 (m, 2H), 3.57 (m, 2H), 3.26 (m, 2H), 2.64 – 2.53 (m, 1H), 2.20 – 2.08 (m, 1H), 1.70 – 1.37 (m, 6H). | 4.15 [474] |
| "A105" | 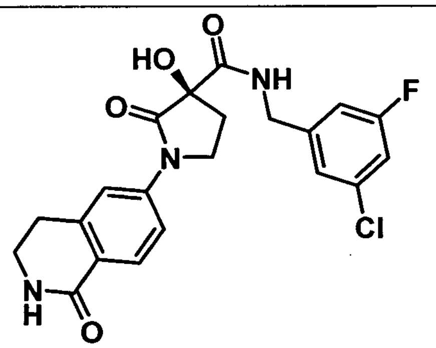 (R)-3-Hydroxy-2-oxo-1-(1-oxo-1,2,3,4-tetrahydro-isochinolin-6-yl)-pyrrolidin-3-carbonsäure-3-chlor-5-fluor-benzylamid | 8.75 (t, *J* = 6.4 Hz, 1H), 7.91 – 7.81 (m, 2H), 7.71 (dd, *J* = 8.6, 2.1 Hz, 1H), 7.63 (d, *J* = 1.9 Hz, 1H), 7.30 – 7.24 (m, 1H), 7.20 (s, 1H), 7.09 (d, *J* = 9.9 Hz, 1H), 6.82 (s, 1H), 4.37 (dt, *J* = 12.6, 6.3 Hz, 1H), 4.24 (dd, *J* = 15.7, 6.1 Hz, 1H), 3.89 (m, 2H), 3.36 (td, *J* = 6.8, 2.7 Hz, 3H), 2.91 (t, *J* = 6.5 Hz, 2H), 2.63 – 2.53 (m, 1H), 2.21 – 2.07 (m, 1H). | 3.38 [432] |

| | | | |
|---|---|---|---|
| "A106" | 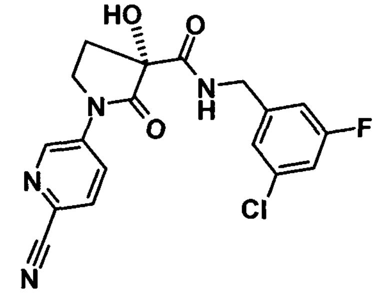 (R)-1-(6-Cyan-pyridin-3-yl)-3-hydroxy-2-oxo-pyrrolidin-3-carbonsäure-3-chlor-5-fluor-benzylamid | 9.13 (s, 1H), 8.83 (d, *J* = 6.3 Hz, 1H), 8.38 (d, *J* = 8.6 Hz, 1H), 8.10 (d, *J* = 8.6 Hz, 1H), 7.28 (d, *J* = 8.7 Hz, 1H), 7.20 (s, 1H), 7.09 (d, *J* = 9.9 Hz, 1H), 6.96 (s, 1H), 4.37 (dd, *J* = 15.6, 6.5 Hz, 1H), 4.25 (dd, *J* = 15.6, 6.1 Hz, 1H), 3.94 (m, 2H), 2.71 – 2.59 (m, 1H), 2.27 – 2.10 (m, 1H). | 3.84 [389] |
| "A107" | 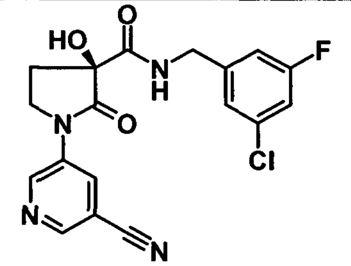 (S)-1-(5-Cyan-pyridin-3-yl)-3-hydroxy-2-oxo-pyrrolidin-3-carbonsäure-3-chlor-5-fluor-benzylamid | 9.28 (d, *J* = 2.6 Hz, 1H), 8.82 (dd, *J* = 11.5, 4.1 Hz, 2H), 8.58 (dd, *J* = 2.5, 1.8 Hz, 1H), 7.28 (dt, *J* = 8.7, 2.1 Hz, 1H), 7.20 (s, 1H), 7.09 (d, *J* = 8.8 Hz, 1H), 6.94 (s, 1H), 4.37 (dd, *J* = 15.7, 6.7 Hz, 1H), 4.24 (dd, *J* = 15.7, 5.9 Hz, 1H), 3.94 (m, 2H), 2.65 – 2.58 (m, 1H), 2.25 – 2.09 (m, 1H). | 3.79 [389] |
| "A108" | 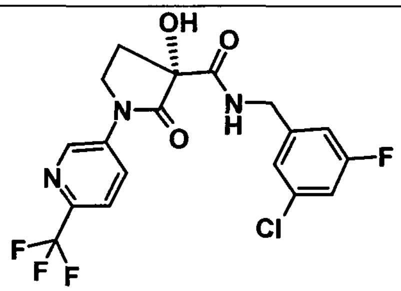 (R)-3-Hydroxy-2-oxo-1-(6-trifluoromethyl-pyridin-3-yl)-pyrrolidin-3-carbonsäure-3-chlor-5-fluor-benzylamid | 9.10 (d, *J* = 2.5 Hz, 1H), 8.82 (t, *J* = 6.4 Hz, 1H), 8.44 (dd, *J* = 8.6, 2.4 Hz, 1H), 7.97 (d, *J* = 8.7 Hz, 1H), 7.28 (dt, *J* = 8.8, 2.2 Hz, 1H), 7.20 (s, 1H), 7.10 (d, *J* = 9.7 Hz, 1H), 6.95 (s, 1H), 4.38 (dd, *J* = 15.8, 6.8 Hz, 1H), 4.25 (dd, *J* = 15.9, 6.1 Hz, 1H), 4.04 – 3.91 (m, 2H), 2.67 – 2.58 (m, 1H), 2.20 (dt, *J* = 13.1, 7.7 Hz, 1H). | 4.43 [432] |

| | | | |
|---|---|---|---|
| "A109" | 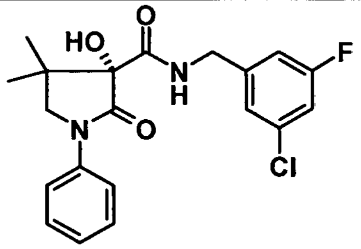 (R)-3-Hydroxy-4,4-dimethyl-2-oxo-1-phenyl-pyrrolidin-3-carbonsäure-3-chlor-5-fluor-benzylamid | 8.72 (t, $J$ = 6.3 Hz, 1H), 7.65 (d, $J$ = 7.8 Hz, 2H), 7.39 (t, $J$ = 8.0 Hz, 2H), 7.31 – 7.23 (m, 1H), 7.20 (s, 1H), 7.16 (t, $J$ = 7.4 Hz, 1H), 7.09 (d, $J$ = 9.5 Hz, 1H), 6.68 (s, 1H), 4.31 (dd, $J$ = 15.5, 6.5 Hz, 1H), 4.22 (dd, $J$ = 15.5, 6.2 Hz, 1H), 3.77 (d, $J$ = 8.9 Hz, 1H), 3.41 (d, $J$ = 8.9 Hz, 1H), 1.06 (s, 3H), 1.01 (s, 3H). | 4.92 [391] |
| "A110" | 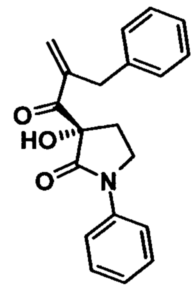 (S)-3-(2-Benzyl-acryloyl)-3-hydroxy-1-phenyl-pyrrolidin-2-on | 7.69 – 7.62 (m, 2H), 7.43 – 7.36 (m, 2H), 7.26 (t, $J$ = 7.3 Hz, 2H), 7.22 – 7.14 (m, 4H), 6.79 (s, 1H), 6.66 (s, 1H), 5.91 (d, $J$ = 1.0 Hz, 1H), 3.81 (td, $J$ = 9.2, 2.7 Hz, 1H), 3.63 – 3.55 (m, 1H), 3.54 (s, 2H), 2.54 (m, 1H), 2.14 (dt, $J$ = 13.0, 8.5 Hz, 1H). | 4.61 [322.3] |
| "A111" | 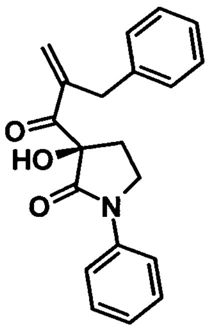 (R)-3-(2-Benzyl-acryloyl)-3-hydroxy-1-phenyl-pyrrolidin-2-on | 7.69 – 7.62 (m, 2H), 7.43 – 7.36 (m, 2H), 7.26 (t, $J$ = 7.3 Hz, 2H), 7.22 – 7.12 (m, 4H), 6.79 (s, 1H), 6.66 (s, 1H), 5.91 (d, $J$ = 0.9 Hz, 1H), 3.81 (td, $J$ = 9.3, 2.6 Hz, 1H), 3.58 (m, 1H), 3.54 (s, 2H), 2.57 – 2.51 (m, 1H), 2.14 (dt, $J$ = 13.0, 8.4 Hz, 1H). | 4.61 [322.3] |

| | | | |
|---|---|---|---|
| "A112" | 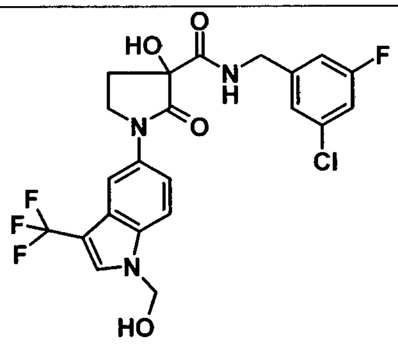 3-Hydroxy-1-(1-hydroxymethyl-3-trifluormethyl-1H-indol-5-yl)-2-oxo-pyrrolidin-3-carbonsäure-3-chlor-5-fluor-benzylamid | 8.73 (t, *J* = 6.4 Hz, 1H), 8.08 (s, 1H), 7.92 (s, 1H), 7.73 (d, *J* = 9.0 Hz, 1H), 7.57 (dd, *J* = 9.0, 2.0 Hz, 1H), 7.27 (d, *J* = 8.7 Hz, 1H), 7.22 (s, 1H), 7.11 (d, *J* = 9.4 Hz, 1H), 6.75 (s, 1H), 6.70 (t, *J* = 7.4 Hz, 1H), 5.58 (d, *J* = 7.4 Hz, 2H), 4.39 (dd, *J* = 15.7, 6.6 Hz, 1H), 4.26 (dd, *J* = 15.7, 5.9 Hz, 1H), 3.92 (t, *J* = 6.7 Hz, 2H), 3.36 (m, 1H), 2.66 – 2.56 (m, 1H), 2.20 – 2.08 (m, 1H). | 4.4 [500] |
| "A113" | 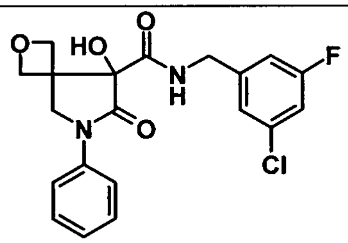 8-Hydroxy-7-oxo-6-phenyl-2-oxa-6-aza-spiro[3.4]octan-8-carbonsäure-3-chlor-5-fluor-benzylamid | 8.97 (t, *J* = 6.3 Hz, 1H), 7.69 (d, *J* = 7.9 Hz, 2H), 7.41 (t, *J* = 7.9 Hz, 2H), 7.29 – 7.25 (m, 1H), 7.25 (s, 1H), 7.22 – 7.15 (m, 2H), 7.06 (d, *J* = 9.4 Hz, 1H), 4.95 (d, *J* = 6.1 Hz, 1H), 4.48 (d, *J* = 6.8 Hz, 1H), 4.44 (d, *J* = 6.8 Hz, 1H), 4.38 (d, *J* = 6.1 Hz, 1H), 4.28 (m, 4H). | 4.19 [405] |
| "A114" | 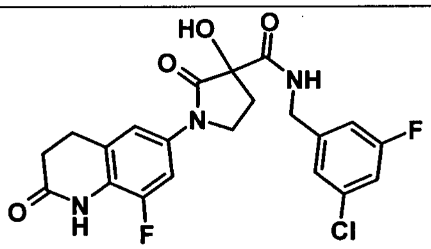 1-(8-Fluor-2-oxo-1,2,3,4-tetrahydro-chinolin-6-yl)-3-hydroxy-2-oxo-pyrrolidin-3-carbonsäure-3-chlor-5-fluor-benzylamid | 10.14 (s, 1H), 8.74 (s, 1H), 7.59 (dd, *J* = 12.9, 1.9 Hz, 1H), 7.34 – 7.24 (m, 2H), 7.20 (s, 1H), 7.09 (d, *J* = 9.4 Hz, 1H), 6.78 (s, 1H), 4.37 (d, *J* = 15.6 Hz, 1H), 4.23 (d, *J* = 15.6 Hz, 1H), 3.81 (m, 2H), 2.93 (t, *J* = 7.4 Hz, 2H), 2.63 – 2.52 (m, 1H), 2.18 – 2.04 (m, 1H). | 3.68 [450] |

| | | | |
|---|---|---|---|
| "A115" | 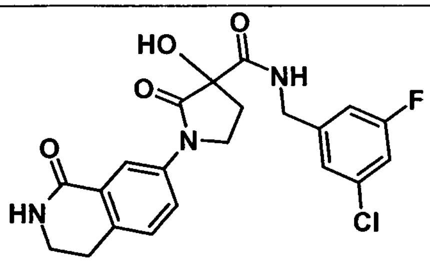<br>3-Hydroxy-2-oxo-1-(1-oxo-1,2,3,4-tetrahydro-isochinolin-7-yl)-pyrrolidin-3-carbonsäure-3-chlor-5-fluor-benzylamid | 10.12 (s, 1H), 8.72 (t, *J* = 6.4 Hz, 1H), 7.31 (s, 1H), 7.27 (d, *J* = 8.7 Hz, 1H), 7.20 (s, 1H), 7.17 (s, 1H), 7.15 – 7.11 (m, 1H), 7.09 (d, *J* = 9.7 Hz, 1H), 6.75 (s, 1H), 4.37 (dd, *J* = 15.7, 6.7 Hz, 1H), 4.24 (dd, *J* = 15.7, 5.9 Hz, 1H), 3.78 m, 2H), 2.84 (t, *J* = 7.5 Hz, 2H), 2.60 – 2.53 (m, 1H), 2.46 – 2.38 (m, 2H), 2.16 – 2.05 (m, 1H). | 3.57 [432] |
| "A116" | 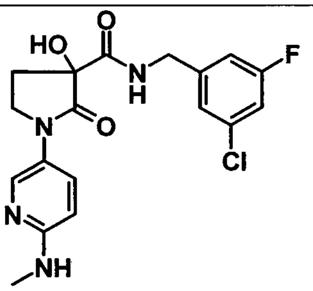<br>3-Hydroxy-1-(6-methylamino-pyridin-3-yl)-2-oxo-pyrrolidin-3-carbonsäure-3-chlor-5-fluor-benzylamid | 8.69 (t, *J* = 6.3 Hz, 1H), 8.17 (d, *J* = 2.6 Hz, 1H), 7.71 (dd, *J* = 9.0, 2.7 Hz, 1H), 7.27 (d, *J* = 8.8 Hz, 1H), 7.20 (s, 1H), 7.10 (d, *J* = 9.7 Hz, 1H), 6.69 (s, 1H), 6.52 (d, *J* = 4.8 Hz, 1H), 6.47 (d, *J* = 9.0 Hz, 1H), 4.38 (dd, *J* = 15.8, 6.7 Hz, 1H), 4.24 (dd, *J* = 15.8, 5.9 Hz, 1H), 3.75 (t, *J* = 6.7 Hz, 2H), 2.75 (d, *J* = 4.9 Hz, 3H), 2.63 – 2.52 (m, 1H), 2.17 – 2.04 (m, 1H). | 2.83 [393.2] |
| "A117" | 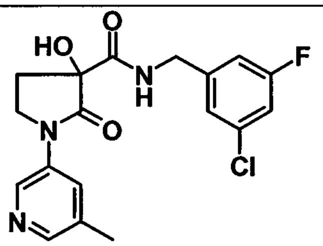<br>3-Hydroxy-1-(5-methyl-pyridin-3-yl)-2-oxo-pyrrolidin-3-carbonsäure-3-chlor-5-fluor-benzylamid | 8.77 (t, *J* = 6.3 Hz, 1H), 7.95 (s, 2H), 7.31 – 7.25 (m, 1H), 7.20 (s, 1H), 7.10 (d, *J* = 9.7 Hz, 1H), 6.83 (s, 1H), 4.38 (dd, *J* = 15.8, 6.7 Hz, 1H), 4.24 (dd, *J* = 15.8, 6.0 Hz, 1H), 3.87 (t, *J* = 6.1 Hz, 2H), 2.65 – 2.56 (m, 1H), 2.33 (s, 3H), 2.15 (dt, *J* = 13.1, 7.6 Hz, 1H). | 2.9 [378] |

| | | | |
|---|---|---|---|
| "A118" | 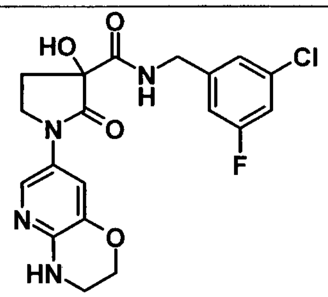 1-(3,4-Dihydro-2H-pyrido[3,2-b][1,4]oxazin-7-yl)-3-hydroxy-2-oxo-pyrrolidin-3-carbonsäure-3-chlor-5-fluor-benzylamid | 8.69 (t, *J* = 6.3 Hz, 1H), 7.79 (d, *J* = 2.1 Hz, 1H), 7.38 (d, *J* = 2.0 Hz, 1H), 7.27 (d, *J* = 8.7 Hz, 1H), 7.20 (s, 1H), 7.10 (d, *J* = 9.7 Hz, 1H), 6.74 (s, 1H), 6.70 (s, 1H), 4.38 (dd, *J* = 15.7, 6.9 Hz, 1H), 4.23 (dd, *J* = 15.7, 6.1 Hz, 1H), 4.17 – 4.04 (m, 2H), 3.75 (t, *J* = 6.6 Hz, 2H), 3.36 (m, 2H), 2.62 – 2.52 (m, 1H), 2.08 (dt, *J* = 14.9, 7.6 Hz, 1H). | 3.06 [421] |
| "A119" | 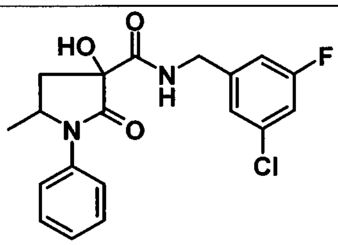 3-Hydroxy-5-methyl-2-oxo-1-phenyl-pyrrolidin-3-carbonsäure-3-chlor-5-fluor-benzylamid | 8.72 (dt, *J* = 24.7, 6.3 Hz, 1H), 7.50 – 7.31 (m, 4H), 7.31 – 7.17 (m, 3H), 7.11 (dd, *J* = 29.9, 9.3 Hz, 1H), 6.73 (s, 1H), 4.50 – 4.32 (m, 2H), 4.32 – 4.19 (m, 1H), 2.78 (dd, *J* = 13.2, 7.1 Hz, 1H), 2.36 (dd, *J* = 13.2, 7.4 Hz, 1H), 2.26 (dd, *J* = 13.5, 6.4 Hz, 1H), 1.75 (dd, *J* = 13.0, 6.9 Hz, 1H), 1.13 (dd, *J* = 6.2, 1.7 Hz, 3H). | 4.27 [377] |
| "A120" | 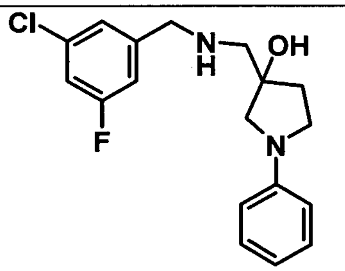 3-[(3-Chlor-5-fluor-benzylamino)-methyl]-1-phenyl-pyrrolidin-3-ol | 7.31 (1 H, s), 7.23 (2 H, dd, J 11.9, 9.8), 7.13 (2 H, t, J 7.8), 6.56 (1 H, t, J 7.2), 6.46 (2 H, d, J 8.1), 4.86 (2 H, bs, J 166.8), 3.81 (2 H, s), 3.30 (3 H, m), 3.11 (1 H, d, J 10.0), 2.00 (1 H, dt, J 12.4, 8.6), 1.90 (1 H, m). | 1.69 [335.0] |

| | | | |
|---|---|---|---|
| "A121" | 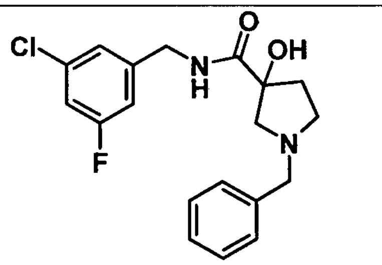<br>1-Benzyl-3-hydroxy-pyrrolidin-3-carbonsäure-3-chlor-5-fluor-benzylamid | * 7.58 (2 H, s), 7.47 (3 H, d, J 2.3), 7.20 (2 H, d, J 10.6), 7.10 (1 H, d, J 9.2), 4.48 (2 H, s), 4.37 (2 H, s), 3.68 (2 H, m), 3.42 (2 H, m), 2.29 (2 H, m). | 1.56 [363.0] |
| "A122" | 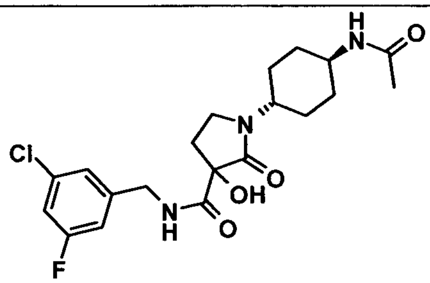<br>1-(4-Acetylamino-cyclohexyl)-3-hydroxy-2-oxo-pyrrolidin-3-carbonsäure-3-chlor-5-fluor-benzylamid | ** 8.52 (t, *J* = 6.4 Hz, 1H), 7.70 (d, *J* = 7.8 Hz, 1H), 7.25 (dt, *J* = 8.8, 2.1 Hz, 1H), 7.19 (s, 1H), 7.11 – 7.05 (m, 1H), 6.40 (s, 1H), 4.36 (dd, *J* = 15.8, 6.8 Hz, 1H), 4.21 (dd, *J* = 15.8, 6.0 Hz, 1H), 3.67 (m, 1H), 3.48 (m, 1H), 3.38 – 3.30 (m, 2H), 2.50 (s, 3H), 2.42 (m, 1H), 1.91 (m, 1H), 1.88 – 1.79 (m, 2H), 1.63 – 1.50 (m, 4H), 1.31 – 1.17 (m, 2H). | 1.74 [426.1] |
| "A123" | 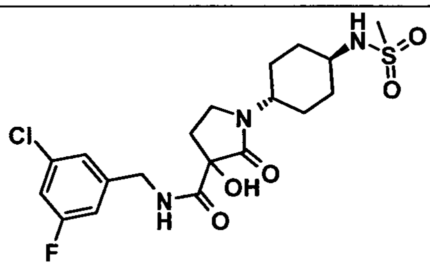<br>3-Hydroxy-1-(4-methansulfonylamino-cyclohexyl)-2-oxo-pyrrolidin-3-carbonsäure-3-chlor-5-fluor-benzylamid | 8.53 (t, *J* = 6.4 Hz, 1H), 7.27 (dt, *J* = 8.7, 2.1 Hz, 1H), 7.20 (s, 1H), 7.10 (d, *J* = 9.0 Hz, 1H), 7.01 (d, *J* = 7.4 Hz, 1H), 6.42 (s, 1H), 4.37 (dd, *J* = 15.8, 6.8 Hz, 1H), 4.23 (dd, *J* = 15.8, 5.9 Hz, 1H), 4.16 (s, 1H), 3.66 (m, 1H), 3.58 (s, 3H), 3.11 (m, 1H), 2.43 (m, 1H), 2.03 – 1.86 (m, 3H), 1.66 – 1.52 (m, 4H), 1.34 (m, 2H). | 1.81 [462.0] |

| | | | |
|---|---|---|---|
| "A124" | 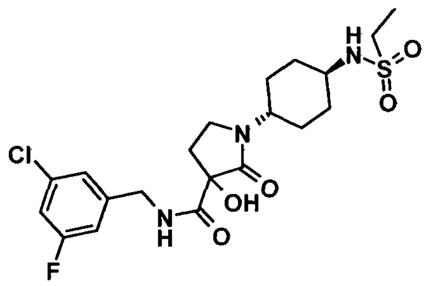 1-(4-Ethansulfonylamino-cyclohexyl)-3-hydroxy-2-oxo-pyrrolidin-3-carbonsäure-3-chlor-5-fluor-benzylamid | 8.51 (t, *J* = 6.3 Hz, 1H), 7.27 – 7.21 (m, 1H), 7.18 (s, 1H), 7.09 (d, *J* = 9.3 Hz, 1H), 7.01 (d, *J* = 7.7 Hz, 1H), 6.39 (s, 1H), 4.36 (dd, *J* = 15.8, 6.6 Hz, 1H), 4.21 (dd, *J* = 15.9, 6.1 Hz, 1H), 3.73 – 3.59 (m, 1H), 3.06 (m, 1H), 2.97 (q, *J* = 7.4 Hz, 2H), 2.46 – 2.35 (m, 1H), 1.92 (m, 3H), 1.65 – 1.48 (m, 4H), 1.36 (s, 3H), 1.19 (t, *J* = 7.3 Hz, 3H). | 1.87 [476.0] |
| "A125" | 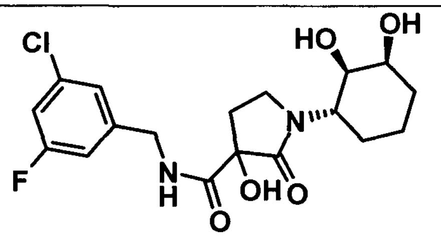 1-((1S,2R,3S)-2,3-Dihydroxy-cyclohexyl)-3-hydroxy-2-oxo-pyrrolidin-3-carbonsäure-3-chlor-5-fluor-benzylamid | ** 8.57 (t, *J* = 6.3 Hz, 1H), 8.50 (t, *J* = 6.4 Hz, 1H), 7.28 – 7.23 (m, 2H), 7.21 (t, *J* = 7.1 Hz, 2H), 7.10 (t, *J* = 9.8 Hz, 2H), 4.41 – 4.33 (m, 3H), 4.27 – 4.19 (m, 2H), 3.98 (m, 4.2 Hz, 2H), 3.90 (m, 2H), 3.49 – 3.43 (m, 3H), 2.47 – 2.37 (m, 2H), 2.02 – 1.88 (m, 2H), 1.74 – 1.56 (m, 4H), 1.56 – 1.47 (m, 2H), 1.46 – 1.39 (m, 2H), 1.39 – 1.28 (m, 5H), 1.26 – 1.14 (m, 1H). | 1.75 [401.0] |
| "A126" | 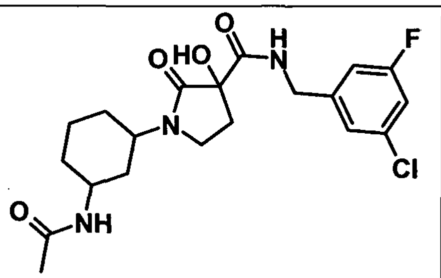 1-(3-Acetylamino-cyclohexyl)-3-hydroxy-2-oxo-pyrrolidin-3- | ** 8.55 (t, *J* = 6.4 Hz, 1H), 7.78 (d, *J* = 7.6 Hz, 1H), 7.27 (dt, *J* = 8.7, 2.1 Hz, 1H), 7.20 (s, 1H), 7.11 (d, *J* = 9.6 Hz, 1H), 4.44 – 4.31 (m, 1H), 4.24 (dd, *J* = 15.7, 6.0 Hz, 1H), 3.83 – 3.71 (m, 1H), 3.67 – 3.60 (m, 1H), 3.37 – 3.24 (m, 2H), 2.48 – 2.39 (m, 1H), 1.99 – 1.88 (m, | |

| | | | |
|---|---|---|---|
| | carbonsäure-3-chlor-5-fluor-benzylamide | 1H), 1.80 – 1.77 (s, 3H), 1.74 (m, 2H), 1.51 (m, 1H), 1.46 – 1.26 (m, 3H), 1.15 – 0.95 (m, 1H). | |
| "A127" | 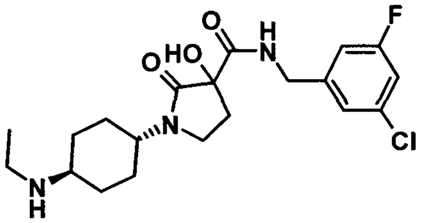 1-(4-Ethylamino-cyclohexyl)-3-hydroxy-2-oxo-pyrrolidin-3-carbonsäure-3-chlor-5-fluor-benzylamid | ** 8.55 (t, *J* = 6.4 Hz, 1H), 8.36 (s, 1H), 7.27 (dt, *J* = 8.8, 2.1 Hz, 1H), 7.20 (s, 1H), 7.10 (d, *J* = 9.7 Hz, 1H), 4.37 (dd, *J* = 15.8, 6.8 Hz, 1H), 4.23 (dd, *J* = 15.8, 6.0 Hz, 1H), 3.69 (m, 2H), 3.38 – 3.24 (m, 4H), 2.73 (m, 2H), 2.63 (m, 1H), 2.44 (m, 1H), 2.05 – 1.97 (m, 2H), 1.97 – 1.87 (m, 1H), 1.69 – 1.56 (m, 3H), 1.51 (m, 2H), 1.31 – 1.16 (m, 2H), 1.09 (t, *J* = 7.1 Hz, 3H). | |
| "A128" | 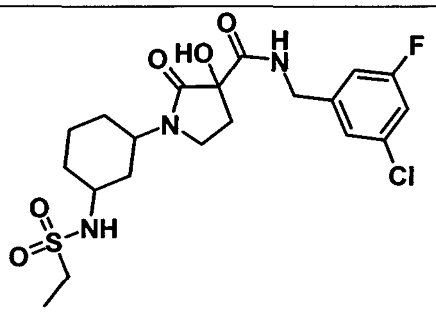 1-(3-Ethansulfonylamino-cyclohexyl)-3-hydroxy-2-oxo-pyrrolidin-3-carbonsäure-3-chlor-5-fluor-benzylamid | 8.53 (t, *J* = 6.4 Hz, 1H), 7.25 (dt, *J* = 8.8, 2.1 Hz, 1H), 7.19 (s, 1H), 7.09 (t, *J* = 7.6 Hz, 2H), 6.42 (s, 1H), 4.35 (dd, *J* = 15.7, 6.7 Hz, 1H), 4.24 (dd, *J* = 15.7, 6.0 Hz, 1H), 3.75 (m, 1H), 3.37 – 3.31 (m, 1H), 3.25 (m, 2H), 3.22 – 3.06 (m, 1H), 2.97 (q, *J* = 7.3 Hz, 2H), 2.42 (m, 1H), 1.92 (m, 1H), 1.80 (m, 3H), 1.52 (s, 1H), 1.46 – 1.27 (m, 3H), 1.23 – 1.14 (m, 3H), 1.11 (s, 1H). | |

| | | | |
|---|---|---|---|
| "A129" | 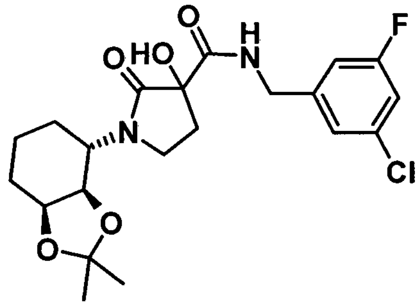<br>1-((3aR,4S,7aS)-2,2-Dimethyl-hexahydro-benzo[1,3]dioxol-4-yl)-3-hydroxy-2-oxo-pyrrolidin-3-carbonsäure-3-chlor-5-fluor-benzylamid | 8.49 (t, $J$ = 6.2 Hz, 1H), 7.23 (m, 2H), 7.09 (t, $J$ = 8.6 Hz, 1H), 6.41 (m, 1H), 4.36 (m, 1H), 4.28 – 4.17 (m, 2H), 4.17 – 4.05 (m, 1H), 3.73 (m, 1H), 3.48 – 3.36 (m, 1H), 3.36 – 3.21 (m, 2H), 2.51 (m, 1H), 2.04 – 1.87 (m, 2H), 1.64 (m, 1H), 1.59 – 1.47 (s, 3H), 1.42 (m, 5H), 1.25 (s, 3H). | |
| "A130" | 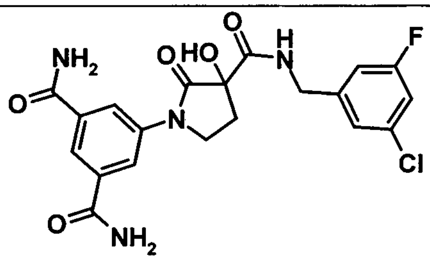<br>5-[3-(3-Chlor-5-fluor-benzylcarbamoyl)-3-hydroxy-2-oxo-pyrrolidin-1-yl]-isophthalamid | ** 8.73 (t, $J$ = 6.4 Hz, 1H), 8.26 (d, $J$ = 1.4 Hz, 2H), 8.16 (t, $J$ = 1.4 Hz, 1H), 8.04 (s, 2H), 7.50 (d, $J$ = 26.6 Hz, 2H), 7.29 – 7.23 (m, 1H), 7.21 (s, 1H), 7.11 (d, $J$ = 9.6 Hz, 1H), 6.80 (s, 1H), 4.39 (dd, $J$ = 15.7, 6.7 Hz, 1H), 4.28 (dd, $J$ = 15.7, 6.1 Hz, 1H), 3.95 (dd, $J$ = 14.8, 8.4 Hz, 2H), 2.69 – 2.58 (m, 1H), 2.17 (dt, $J$ = 13.0, 7.5 Hz, 1H). | |
| "A131" | 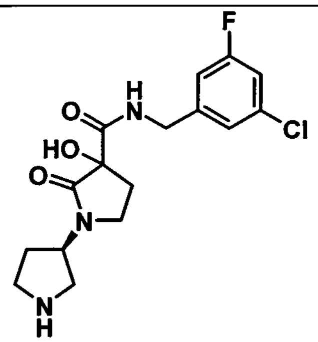<br>(R)-3-Hydroxy-2-oxo-[1,3']bipyrrolidinyl-3-carbonsäure-3-chlor-5-fluor-benzylamid | 8.58 (dd, $J$ = 12.2, 6.1 Hz, 1H), 7.25 (dt, $J$ = 8.7, 2.1 Hz, 1H), 7.19 (s, 1H), 7.09 (d, $J$ = 9.4 Hz, 1H), 4.51 (dd, $J$ = 14.4, 7.5 Hz, 1H), 4.36 (dd, $J$ = 15.7, 6.7 Hz, 1H), 4.23 (dd, $J$ = 15.6, 6.0 Hz, 1H), 3.50 – 3.33 (m, 5H), 3.16 – 2.99 (m, 3H), 2.47 – 2.38 (m, 1H), 2.13 – 1.82 (m, 3H). | 1.44 [356.1] |

| | | | |
|---|---|---|---|
| "A132" | 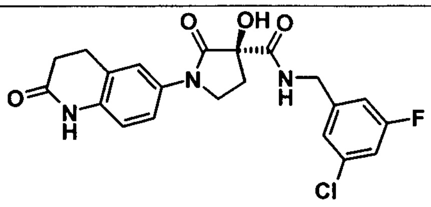 (R)-3-Hydroxy-2-oxo-1-(2-oxo-1,2,3,4-tetrahydro-chinolin-6-yl)-pyrrolidin-3-carbonsäure-3-chlor-5-fluor-benzylamid | | |
| "A133" | 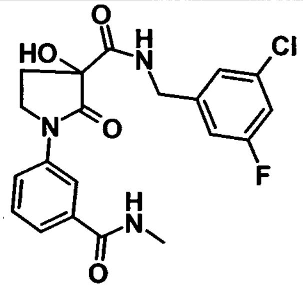 3-Hydroxy-1-(3-methyl-carbamoyl-phenyl)-2-oxo-pyrrolidin-3-carbonsäure-3-chlor-5-fluor-benzylamid | | 1.93 [420.1] |
| "A134" | 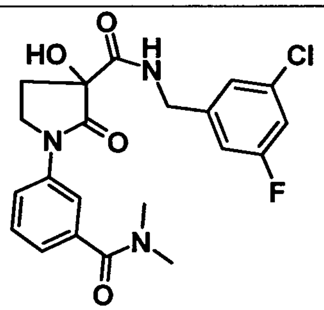 1-(3-Dimethylcarbamoyl-phenyl)-3-hydroxy-2-oxo-pyrrolidin-3-carbonsäure-3-chlor-5-fluor-benzylamid | | 1.99 [434.1] |
| "A135" | 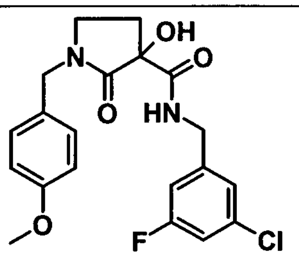 | | 2.21 [407.1] |

| | | | |
|---|---|---|---|
| | 3-Hydroxy-1-(4-methoxy-benzyl)-2-oxo-pyrrolidin-3-carbonsäure-3-chlor-5-fluor-benzylamid | | |
| "A136" | 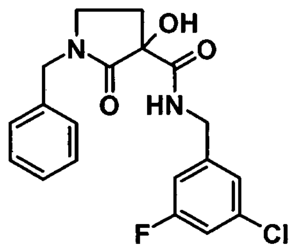 1-Benzyl-3-hydroxy-2-oxo-pyrrolidin-3-carbonsäure-3-chlor-5-fluor-benzylamid | | 2.21 [377.1] |
| "A137" | 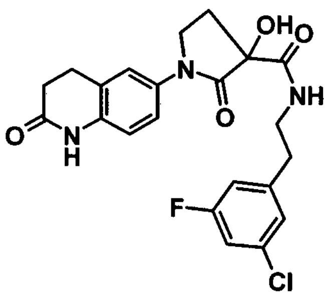 3-Hydroxy-2-oxo-1-(2-oxo-1,2,3,4-tetrahydro-chinolin-6-yl)-pyrrolidin-3-carbonsäure-[2-(3-chlor-5-fluor-phenyl)-ethyl]-amid | 10.05 (s, 1H), 7.99 (t, *J* = 6.0 Hz, 1H), 7.48 (d, *J* = 2.2 Hz, 1H), 7.42 (dd, *J* = 8.6, 2.4 Hz, 1H), 7.23 (dt, *J* = 8.9, 2.2 Hz, 1H), 7.16 (s, 1H), 7.11 – 7.04 (m, 1H), 6.86 (d, *J* = 8.6 Hz, 1H), 6.54 (s, 1H), 3.80 – 3.73 (m, 2H), 3.45 – 3.30 (m, 2H), 2.88 (t, *J* = 7.5 Hz, 2H), 2.79 (t, *J* = 7.0 Hz, 2H), 2.47 – 2.38 (m, 3H), 2.10 – 1.96 (m, 1H). | 2.03 [446.1] |
| "A138" | 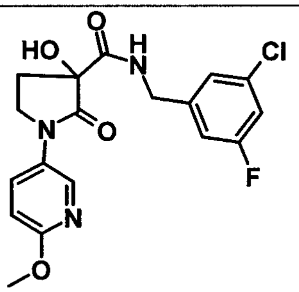 3-Hydroxy-1-(6-methoxy-pyridin-3-yl)-2-oxo-pyrrolidin-3-carbonsäure-3-chlor-5-fluor-benzylamid | | 2.06 [394.0] |

| | | | |
|---|---|---|---|
| "A139" | 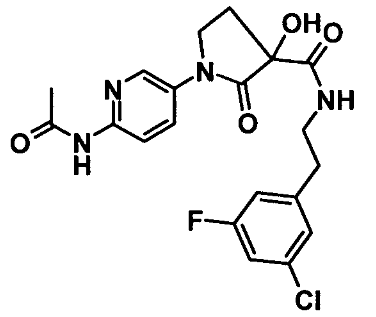 1-(6-Acetylamino-pyridin-3-yl)-3-hydroxy-2-oxo-pyrrolidin-3-carbonsäure-[2-(3-chlor-5-fluor-phenyl)-ethyl]-amid | ** 10.50 (s, 1H), 8.64 (dd, *J* = 2.5, 0.8 Hz, 1H), 8.15 – 8.02 (m, 3H), 7.23 (dt, *J* = 8.8, 2.1 Hz, 1H), 7.16 (s, 1H), 7.08 (dt, *J* = 12.8, 6.4 Hz, 1H), 6.65 (s, 1H), 3.91 – 3.76 (m, 2H), 3.44 – 3.36 (m, 2H), 2.80 (t, *J* = 6.9 Hz, 2H), 2.49 – 2.43 (m, 1H), 2.09 (s, 3H). | 1.99 [435.0] |
| "A140" | 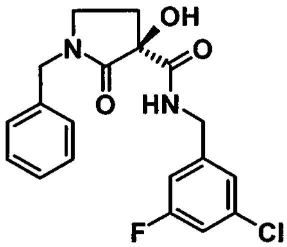 (S)-1-Benzyl-3-hydroxy-2-oxo-pyrrolidin-3-carbonsäure-3-chlor-5-fluor-benzylamid | | 2.25 [377.0] |
| "A141" | 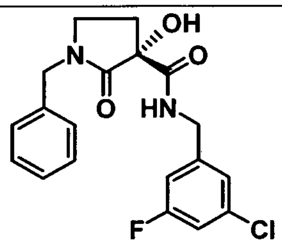 (R)-1-Benzyl-3-hydroxy-2-oxo-pyrrolidin-3-carbonsäure-3-chlor-5-fluor-benzylamid | | 2.23 [377.0] |

| | | | |
|---|---|---|---|
| "A142" | 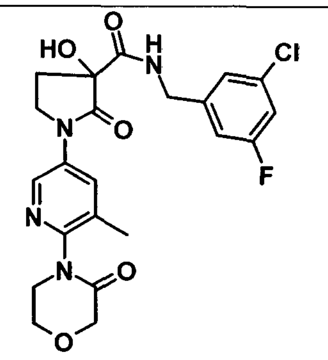<br>3-Hydroxy-1-[5-methyl-6-(3-oxo-morpholin-4-yl)-pyridin-3-yl]-2-oxo-pyrrolidin-3-carbonsäure-3-chlor-5-fluor-benzylamid | ** 8.75 (t, *J* = 6.4 Hz, 2H), 8.06 (s, 1H), 7.28 (dt, *J* = 8.8, 2.1 Hz, 1H), 7.22 (s, 1H), 7.11 (d, *J* = 9.5 Hz, 1H), 6.84 (s, 1H), 4.39 (dt, *J* = 18.6, 9.4 Hz, 1H), 4.31 – 4.17 (m, 3H), 4.01 (t, *J* = 5.0 Hz, 2H), 3.97 – 3.87 (m, 2H), 2.69 – 2.58 (m, 1H), 2.24 – 2.12 (m, 4H). | 2.02 [477.1] |
| "A143" | 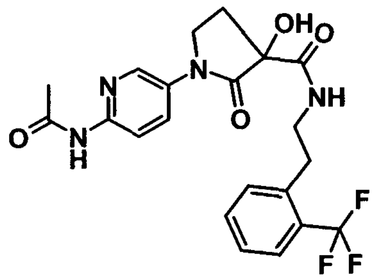<br>1-(6-Acetylamino-pyridin-3-yl)-3-hydroxy-2-oxo-pyrrolidin-3-carbonsäure-[2-(2-trifluormethyl-phenyl)-ethyl]-amid | 10.50 (s, 1H), 8.64 (d, *J* = 1.6 Hz, 1H), 8.17 (t, *J* = 6.0 Hz, 1H), 8.13 – 8.04 (m, 2H), 7.67 (d, *J* = 7.9 Hz, 1H), 7.60 (t, *J* = 7.4 Hz, 1H), 7.49 (d, *J* = 7.6 Hz, 1H), 7.42 (t, *J* = 7.6 Hz, 1H), 6.63 (s, 1H), 3.89 – 3.78 (m, 2H), 3.44 – 3.31 (m, 2H), 2.94 (t, *J* = 7.2 Hz, 2H), 2.56 – 2.52 (m, 1H), 2.17 – 2.09 (m, 1H), 2.09 (s, 3H). | 2.03 [451.1] |
| "A144" | 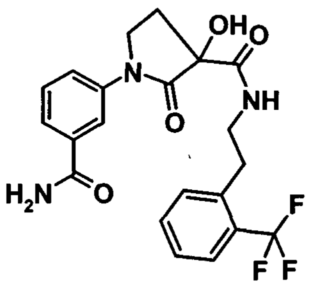<br>1-(3-Carbamoyl-phenyl)-3-hydroxy-2-oxo-pyrrolidin-3-carbonsäure-[2-(2-trifluormethyl-phenyl)-ethyl]-amid | 8.17 (t, *J* = 6.0 Hz, 1H), 8.06 – 8.03 (m, 1H), 8.00 (s, 1H), 7.96 – 7.90 (m, 1H), 7.72 – 7.65 (m, 2H), 7.60 (t, *J* = 7.6 Hz, 1H), 7.49 (d, *J* = 8.0 Hz, 2H), 7.46 – 7.35 (m, 2H), 6.62 (s, 1H), 3.88 (m, 2H), 3.38 (m, 2H), 2.94 (t, *J* = 7.2 Hz, 2H), 2.54 (m, 1H), 2.10 (dt, *J* = 12.9, 7.9 Hz, 1H). | 1.98 [436.1] |

| | | | |
|---|---|---|---|
| "A145" | 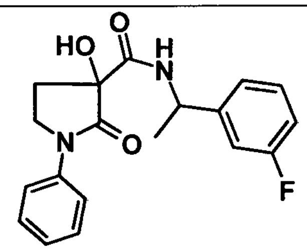 3-Hydroxy-2-oxo-1-phenyl-pyrrolidin-3-carbonsäure-[1-(3-fluor-phenyl)-ethyl]-amid | ** 8.32 – 8.24 (m, 1H), 7.73 – 7.65 (m, 2H), 7.44 – 7.37 (m, 2H), 7.37 – 7.29 (m, 1H), 7.25 – 7.15 (m, 3H), 7.07 – 6.99 (m, 1H), 6.69 (d, *J* = 19.3 Hz, 1H), 5.01 – 4.91 (m, 1H), 3.89 – 3.78 (m, 2H), 2.66 – 2.57 (m, 1H), 2.49 – 2.43 (m, 1H), 2.17 – 2.05 (m, 1H), 1.43 (d, *J* = 7.0 Hz, 3H). | 2.16 [343.1] |
| "A146" | 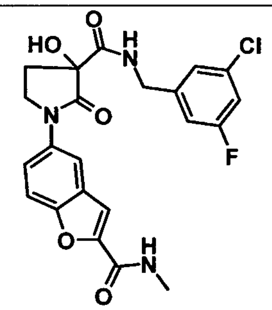 3-Hydroxy-1-(2-methylcarbamoyl-benzofuran-5-yl)-2-oxo-pyrrolidin-3-carbonsäure-3-chlor-5-fluor-benzylamid | | 2.11 [460.1] |
| "A147" | 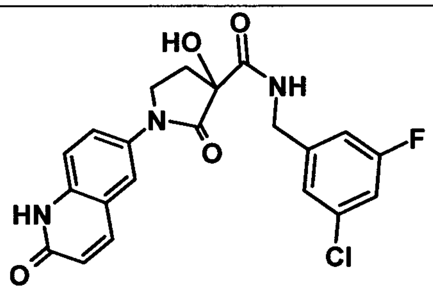 3-Hydroxy-2-oxo-1-(2-oxo-1,2-dihydro-chinolin-6-yl)-pyrrolidin-3-carbonsäure-3-chlor-5-fluor-benzylamid | ** 11.76 (s, 1H), 9.18 – 8.53 (m, 1H), 7.90 (dd, *J* = 6.3, 3.1 Hz, 3H), 7.36 – 7.29 (m, 1H), 7.26 (d, *J* = 8.7 Hz, 1H), 7.21 (s, 1H), 7.11 (d, *J* = 9.5 Hz, 1H), 6.52 (d, *J* = 9.6 Hz, 1H), 4.39 (dd, *J* = 15.6, 5.1 Hz, 1H), 4.26 (dd, *J* = 15.6, 4.2 Hz, 1H), 3.89 (t, *J* = 6.8 Hz, 2H), 2.66 – 2.57 (m, 1H), 2.16 (dt, *J* = 13.0, 7.6 Hz, 1H). | 2.07 [430.0] |

| | | | |
|---|---|---|---|
| "A148" | 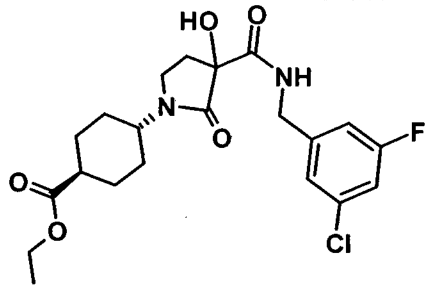 4-[3-(3-Chlor-5-fluor-benzylcarbamoyl)-3-hydroxy-2-oxo-pyrrolidin-1-yl]-cyclohexan-carbonsäure-ethylester | 8.52 (t, $J$ = 6.3 Hz, 1H), 7.25 (dt, $J$ = 8.8, 2.1 Hz, 1H), 7.19 (s, 1H), 7.09 (d, $J$ = 8.9 Hz, 1H), 6.40 (d, $J$ = 4.5 Hz, 1H), 4.36 (dd, $J$ = 15.8, 6.7 Hz, 1H), 4.22 (dd, $J$ = 15.8, 5.9 Hz, 1H), 4.05 (q, $J$ = 7.1 Hz, 2H), 3.76 – 3.62 (m, 1H), 2.42 (ddd, $J$ = 11.9, 7.1, 4.5 Hz, 1H), 2.25 (ddd, $J$ = 11.9, 8.5, 3.5 Hz, 1H), 2.02 – 1.85 (m, 3H), 1.71 – 1.33 (m, 6H), 1.17 (t, $J$ = 7.1 Hz, 3H). | 2.25 [441.1] |
| "A149" | 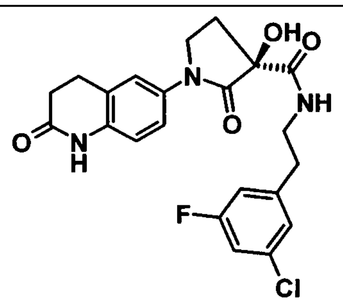 (S)-3-Hydroxy-2-oxo-1-(2-oxo-1,2,3,4-tetrahydro-chinolin-6-yl)-pyrrolidin-3-carbonsäure-[2-(3-chlor-5-fluor-phenyl)-ethyl]-amid | | 2.08 [446.1] |
| "A150" | 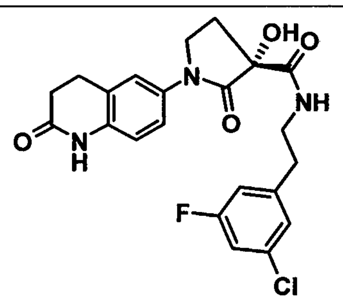 (R)-3-Hydroxy-2-oxo-1-(2-oxo-1,2,3,4-tetrahydro-chinolin-6-yl)-pyrrolidin-3-carbonsäure-[2-(3-chlor-5-fluor-phenyl)-ethyl]-amid | | 2.06 [446.1] |

| | | | |
|---|---|---|---|
| "A151" | 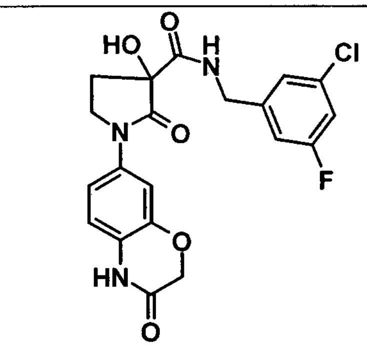<br>3-Hydroxy-2-oxo-1-(3-oxo-3,4-dihydro-2H-benzo[1,4]oxazin-7-yl)-pyrrolidin-3-carbonsäure-3-chlor-5-fluor-benzylamid | | 2.08 [434.0] |
| "A152" | 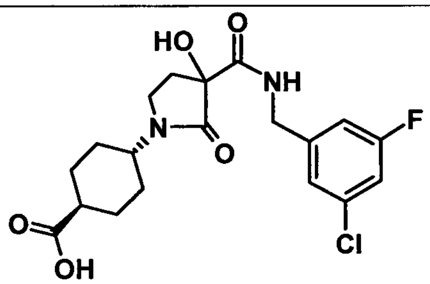<br>4-[3-(3-Chlor-5-fluor-benzylcarbamoyl)-3-hydroxy-2-oxo-pyrrolidin-1-yl]-cyclohexancarbonsäure | | 2.02 [413.1] |
| "A153" | 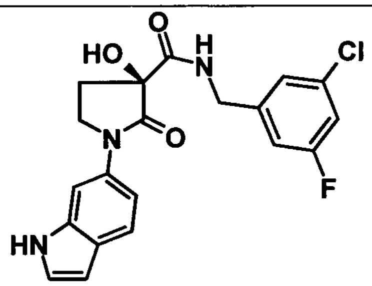<br>(S)-3-Hydroxy-1-(1H-indol-6-yl)-2-oxo-pyrrolidin-3-carbonsäure-3-chlor-5-fluor-benzylamid | 11.08 (d, *J* = 10.1 Hz, 1H), 8.68 (t, *J* = 6.3 Hz, 1H), 7.78 (dd, *J* = 3.4, 2.5 Hz, 1H), 7.54 (d, *J* = 8.6 Hz, 1H), 7.35 – 7.33 (m, 1H), 7.28 – 7.22 (m, 3H), 7.11 (t, *J* = 7.5 Hz, 1H), 6.70 (s, 1H), 6.46 – 6.38 (m, 1H), 4.41 (dd, *J* = 15.8, 6.7 Hz, 1H), 4.27 (dd, *J* = 15.8, 6.0 Hz, 1H), 3.90 (t, *J* = 6.8 Hz, 2H), 2.65 – 2.58 (m, 1H), 2.15 (dt, *J* = 13.0, 7.5 Hz, 1H). | 2.19 [402.0] |

| | | | |
|---|---|---|---|
| "A154" | 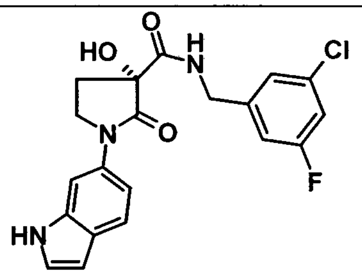 (R)-3-Hydroxy-1-(1H-indol-6-yl)-2-oxo-pyrrolidin-3-carbonsäure-3-chlor-5-fluor-benzylamid | | 2.18 [402.0] |
| "A155" | 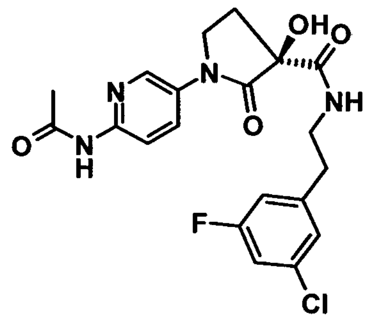 (S)-1-(6-Acetylamino-pyridin-3-yl)-3-hydroxy-2-oxo-pyrrolidin-3-carbonsäure-[2-(3-chlor-5-fluor-phenyl)-ethyl]-amid | | 2.04 [435.1] |
| "A156" | 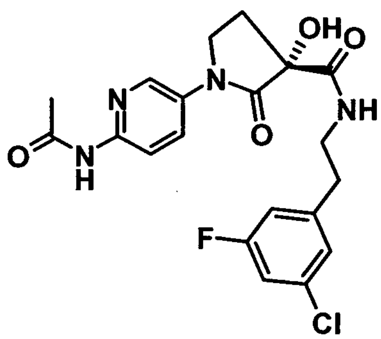 (R)-1-(6-Acetylamino-pyridin-3-yl)-3-hydroxy-2-oxo-pyrrolidin-3-carbonsäure-[2-(3-chlor-5-fluor-phenyl)-ethyl]-amid | | 2.06 [435.1] |

| | | | |
|---|---|---|---|
| "A157" | 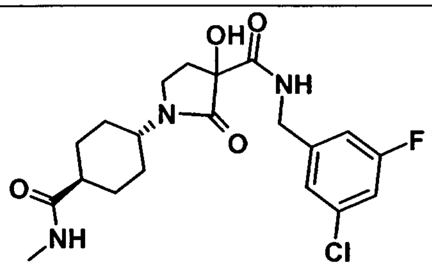<br>3-Hydroxy-1-(4-methylcarbamoyl-cyclohexyl)-2-oxo-pyrrolidin-3-carbonsäure-3-chlor-5-fluor-benzylamid | | 1.98 [426.1] |
| "A158" | 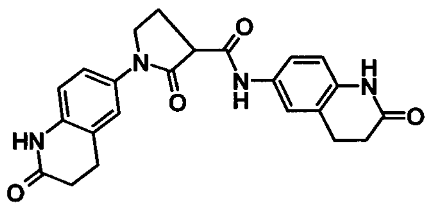<br>2-Oxo-1-(2-oxo-1,2,3,4-tetrahydro-chinolin-6-yl)-pyrrolidin-3-carbonsäure-(2-oxo-1,2,3,4-tetrahydro-chinolin-6-yl)-amid | ** 10.16 – 9.99 (m, 1H), 7.47 (d, $J$ = 7.4 Hz, 1H), 7.37 (ddd, $J$ = 23.0, 8.6, 1.9 Hz, 1H), 6.90 – 6.74 (m, 1H), 3.90 – 3.77 (m, 1H), 3.24 (d, $J$ = 7.1 Hz, 1H), 2.93 – 2.81 (m, 2H), 2.47 – 2.39 (m, 2H), 2.38 – 2.25 (m, 1H), 1.22 – 1.10 (m, 1H). | 1.77 [419.1] |
| "A159" | 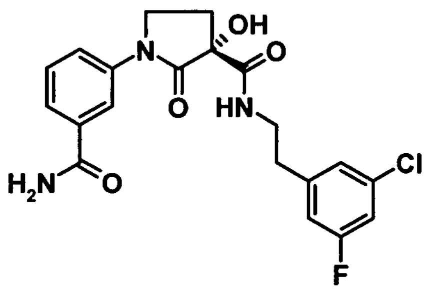<br>(R)-1-(3-Carbamoyl-phenyl)-3-hydroxy-2-oxo-pyrrolidin-3-carbonsäure-[2-(3-chlor-5-fluor-phenyl)-ethyl]-amid | | 2.13 [420.1] |

| | | | |
|---|---|---|---|
| "A160" | 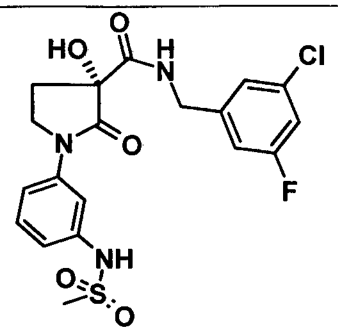 (R)-3-Hydroxy-1-(3-methansulfonylamino-phenyl)-2-oxo-pyrrolidin-3-carbonsäure-3-chlor-5-fluor-benzylamid | | |
| "A161" | 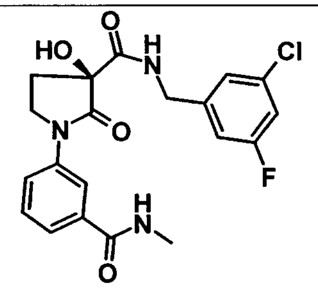 (S)-3-Hydroxy-1-(3-methyl-carbamoyl-phenyl)-2-oxo-pyrrolidin-3-carbonsäure-3-chlor-5-fluor-benzylamid | | 1.93 [420.1] |
| "A162" | 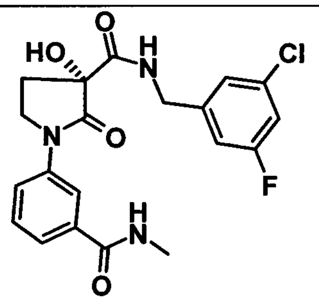 (R)-3-Hydroxy-1-(3-methyl-carbamoyl-phenyl)-2-oxo-pyrrolidin-3-carbonsäure-3-chlor-5-fluor-benzylamid | | 1.93 [420.1] |

| | | | |
|---|---|---|---|
| "A163" | 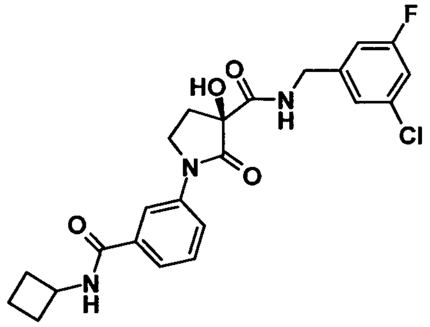<br>(S)-1-(3-Cyclobutylcarbamoyl-phenyl)-3-hydroxy-2-oxo-pyrrolidin-3-carbonsäure-3-chlor-5-fluor-benzylamid | | 2.22 [460.1] |
| "A164" | 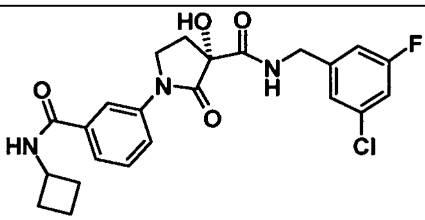<br>(R)-1-(3-Cyclobutylcarbamoyl-phenyl)-3-hydroxy-2-oxo-pyrrolidin-3-carbonsäure-3-chlor-5-fluor-benzylamid | | 2.22 [460.1] |
| "A165" | 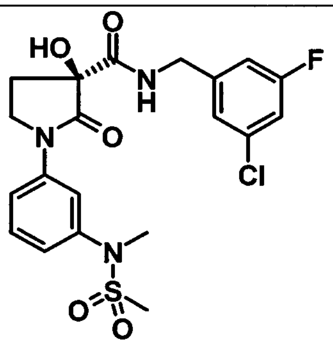<br>(S)-3-Hydroxy-1-[3-(methansulfonyl-methyl-amino)-phenyl]-2-oxo-pyrrolidin-3-carbonsäure-3-chlor-5-fluor-benzylamid | | 2.24 [470.1] |

| | | | |
|---|---|---|---|
| "A166" | 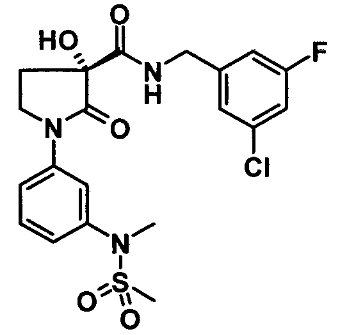<br>(R)-3-Hydroxy-1-[3-(methansulfonyl-methyl-amino)-phenyl]-2-oxo-pyrrolidin-3-carbonsäure-3-chlor-5-fluor-benzylamid | | 2.19 [470.0] |
| "A167" | 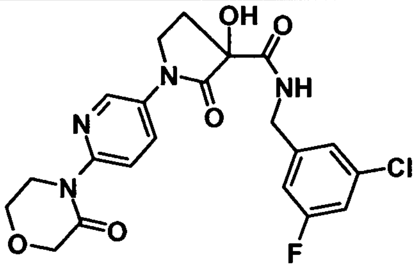<br>3-Hydroxy-2-oxo-1-[6-(3-oxo-morpholin-4-yl)-pyridin-3-yl]-pyrrolidin-3-carbonsäure-3-chlor-5-fluor-benzylamid | | 2.14 [463.1] |
| "A168" | 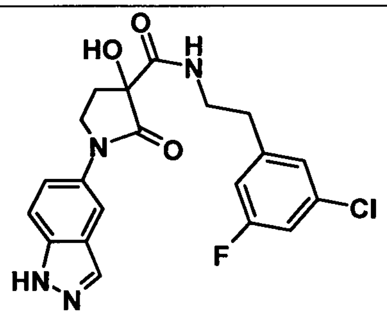<br>3-Hydroxy-1-(1H-indazol-5-yl)-2-oxo-pyrrolidin-3-carbonsäure-[2-(3-chlor-5-fluor-phenyl)-ethyl]-amid | 13.06 (s, 1H), 8.07 (s, 1H), 8.01 (t, $J$ = 5.9 Hz, 1H), 7.89 (s, 1H), 7.75 (dd, $J$ = 9.0, 1.9 Hz, 1H), 7.55 (d, $J$ = 9.0 Hz, 1H), 7.23 (dt, $J$ = 8.9, 2.1 Hz, 1H), 7.17 (s, 1H), 7.09 (d, $J$ = 9.4 Hz, 1H), 6.57 (s, 1H), 3.94 – 3.84 (m, 2H), 3.47 – 3.29 (m, 2H), 2.80 (t, $J$ = 7.0 Hz, 2H), 2.46 (t, $J$ = 6.4 Hz, 1H), 2.09 (dt, $J$ = 12.9, 7.7 Hz, 1H). | 2.15 [417.0] |

| | | | |
|---|---|---|---|
| "A169" | 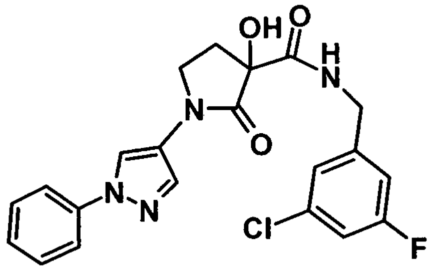<br>3-Hydroxy-2-oxo-1-(1-phenyl-1H-pyrazol-4-yl)-pyrrolidin-3-carbonsäure-3-chlor-5-fluor-benzylamid | ** 8.72 (t, $J$ = 6.3 Hz, 1H), 8.66 (s, 1H), 8.11 (s, 1H), 7.83 (d, $J$ = 7.8 Hz, 2H), 7.51 (t, $J$ = 7.9 Hz, 2H), 7.32 (t, $J$ = 7.4 Hz, 1H), 7.27 (d, $J$ = 8.7 Hz, 1H), 7.22 (s, 1H), 7.11 (d, $J$ = 9.5 Hz, 1H), 6.76 (s, 1H), 4.39 (dd, $J$ = 15.7, 6.7 Hz, 1H), 4.27 (dd, $J$ = 15.7, 6.1 Hz, 1H), 3.86 – 3.74 (m, 2H), 2.71 – 2.61 (m, 1H), 2.25 – 2.13 (m, 1H). | 2.28 [429.0] |
| "A170" | 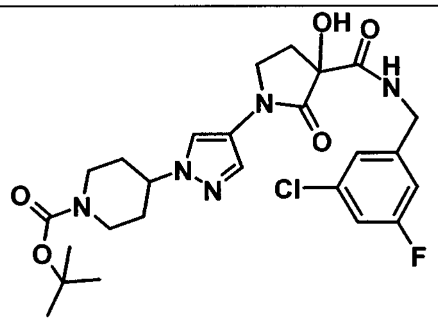<br>4-{4-[3-(3-Chlor-5-fluor-benzylcarbamoyl)-3-hydroxy-2-oxo-pyrrolidin-1-yl]-pyrazol-1-yl}-piperidin-1-carbonsäure-tert-butylester | ** 8.67 (t, $J$ = 6.4 Hz, 1H), 8.06 (s, 1H), 7.70 (s, 1H), 7.26 (dt, $J$ = 8.7, 2.1 Hz, 1H), 7.20 (s, 1H), 7.09 (d, $J$ = 9.0 Hz, 1H), 6.66 (s, 1H), 4.42 – 4.30 (m, 2H), 4.28 – 4.16 (m, 1H), 4.03 (m, 2H), 3.73 – 3.62 (m, 2H), 2.61 (ddd, $J$ = 12.1, 7.6, 4.2 Hz, 1H), 2.14 (ddd, $J$ = 13.0, 8.5, 6.8 Hz, 1H), 1.96 (m, 2H), 1.76 (m, 2H), 1.41 (s, 9H). | |
| "A171" | 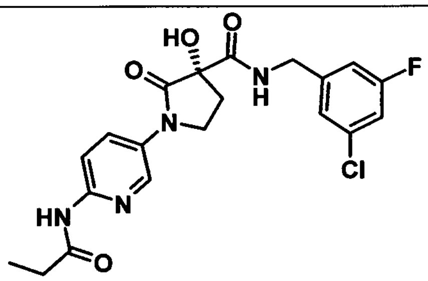<br>(S)-3-Hydroxy-2-oxo-1-(6-propionylamino-pyridin-3-yl)-pyrrolidin-3-carbonsäure-3-chlor-5-fluor-benzylamid | | 3.39 [435.2] |

| | | | |
|---|---|---|---|
| "A172" | 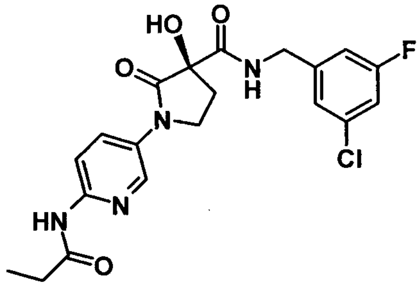 (R)-3-Hydroxy-2-oxo-1-(6-propionylamino-pyridin-3-yl)-pyrrolidin-3-carbonsäure-3-chlor-5-fluor-benzylamid | | |
| "A173" | 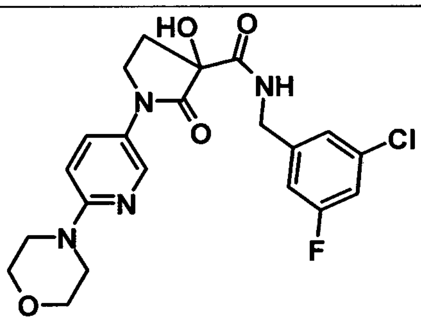 3-Hydroxy-1-(6-morpholin-4-yl-pyridin-3-yl)-2-oxo-pyrrolidin-3-carbonsäure-3-chlor-5-fluor-benzylamid | | 1.70 [449.1] |
| "A174" | 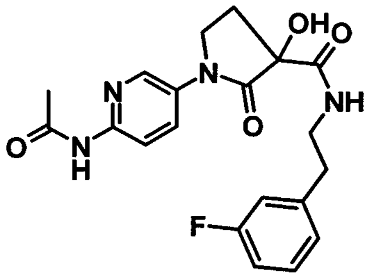 1-(6-Acetylamino-pyridin-3-yl)-3-hydroxy-2-oxo-pyrrolidin-3-carbonsäure-[2-(3-fluor-phenyl)-ethyl]-amid | 10.49 (s, 1H), 8.63 (s, 1H), 8.17 – 7.85 (m, 3H), 7.31 (dd, *J* = 14.3, 7.6 Hz, 1H), 7.13 – 6.81 (m, 3H), 6.61 (s, 1H), 3.83 (t, *J* = 6.7 Hz, 2H), 3.43 – 3.30 (m, 3H), 2.78 (t, *J* = 7.2 Hz, 2H), 2.08 (s, 3H). | 1.77 [401.1] |

| | | | |
|---|---|---|---|
| "A175" | 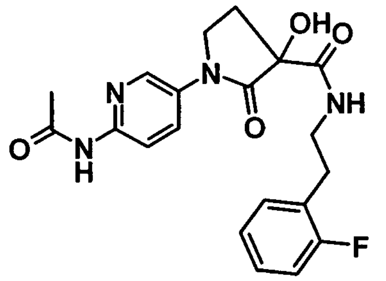<br>1-(6-Acetylamino-pyridin-3-yl)-3-hydroxy-2-oxo-pyrrolidin-3-carbonsäure-[2-(2-fluor-phenyl)-ethyl]-amid | 10.49 (s, 1H), 8.63 (s, 1H), 8.12 – 8.03 (m, 3H), 7.26 (dt, *J* = 7.7, 6.7 Hz, 2H), 7.17 – 7.08 (m, 2H), 6.60 (s, 1H), 3.83 (m, 2H), 3.39 (m, 1H), 2.86 – 2.73 (m, 2H), 2.08 (s, 3H). | 1.75 [401.1] |
| "A176" | 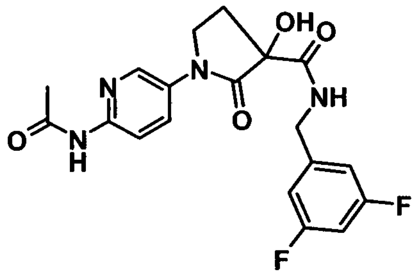<br>1-(6-Acetylamino-pyridin-3-yl)-3-hydroxy-2-oxo-pyrrolidin-3-carbonsäure-3,5-difluor-benzylamid | 10.50 (s, 1H), 8.70 (t, *J* = 6.2 Hz, 1H), 8.64 (s, 1H), 8.13 – 8.01 (m, 2H), 7.04 (dd, *J* = 19.0, 9.4 Hz, 1H), 6.98 (d, *J* = 7.5 Hz, 2H), 6.76 (s, 1H), 4.40 (dd, *J* = 15.7, 6.7 Hz, 1H), 4.26 (dd, *J* = 15.8, 5.9 Hz, 1H), 3.87 (t, *J* = 6.7 Hz, 2H), 2.68 – 2.56 (m, 1H), 2.21 – 2.11 (m, 1H), 2.08 (s, 3H). | 1.75 [405.1] |
| "A177" | 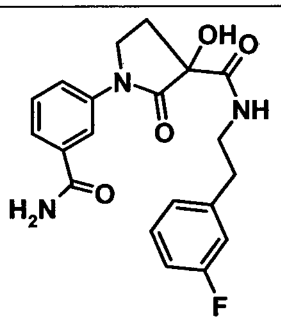<br>1-(3-Carbamoyl-phenyl)-3-hydroxy-2-oxo-pyrrolidin-3-carbonsäure-[2-(3-fluor-phenyl)-ethyl]-amid | ** 8.07 – 7.98 (m, 3H), 7.94 (d, *J* = 8.1 Hz, 1H), 7.67 (t, *J* = 11.7 Hz, 1H), 7.51 – 7.44 (m, 1H), 7.39 (s, 1H), 7.32 (dd, *J* = 14.4, 7.6 Hz, 1H), 7.12 – 6.95 (m, 3H), 3.88 (t, *J* = 6.7 Hz, 2H), 3.32 – 3.08 (m, 2H), 2.79 (t, *J* = 7.2 Hz, 2H), 2.47 (m, 1H), 2.09 (dt, *J* = 12.9, 7.9 Hz, 1H). | 1.80 [386.1] |

| | | | |
|---|---|---|---|
| "A178" | 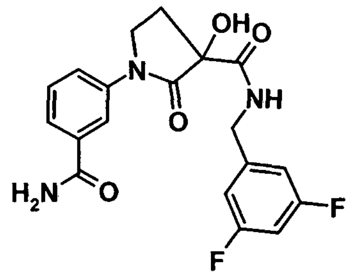<br>1-(3-Carbamoyl-phenyl)-3-hydroxy-2-oxo-pyrrolidin-3-carbonsäure-3,5-difluor-benzylamid | 8.70 (t, *J* = 6.3 Hz, 1H), 8.13 (s, 1H), 8.06 – 8.03 (m, 1H), 8.01 (s, 1H), 7.95 (dd, *J* = 8.2, 1.4 Hz, 1H), 7.68 (d, *J* = 7.8 Hz, 1H), 7.48 (t, *J* = 8.0 Hz, 1H), 7.38 (s, 1H), 7.09 – 7.01 (m, 1H), 6.99 (d, *J* = 6.6 Hz, 2H), 6.78 (s, 1H), 4.40 (dd, *J* = 15.8, 6.7 Hz, 1H), 4.27 (dd, *J* = 15.8, 6.0 Hz, 1H), 3.91 (t, *J* = 6.9 Hz, 2H), 2.66 – 2.57 (m, 1H), 2.16 (dt, *J* = 13.0, 7.5 Hz, 1H). | 1.77 [390.1] |
| "A179" | 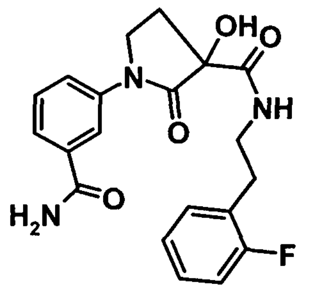<br>1-(3-Carbamoyl-phenyl)-3-hydroxy-2-oxo-pyrrolidin-3-carbonsäure-[2-(2-fluor-phenyl)-ethyl]-amid | * 8.15 (s, 1H), 7.96 (dd, *J* = 8.2, 1.4 Hz, 1H), 7.74 (d, *J* = 7.8 Hz, 1H), 7.50 (t, *J* = 8.0 Hz, 1H), 7.32 (t, *J* = 7.6 Hz, 1H), 7.26 (dd, *J* = 14.3, 6.6 Hz, 1H), 7.12 (dd, *J* = 14.9, 7.5 Hz, 2H), 4.00 – 3.86 (m, 2H), 3.48 (m, 1H), 3.43 – 3.32 (m, 1H), 2.93 – 2.80 (m, 2H), 2.54 (m, 1H), 2.17 (dt, *J* = 12.9, 8.1 Hz, 1H). | 1.77 [390.1] |
| "A180" | 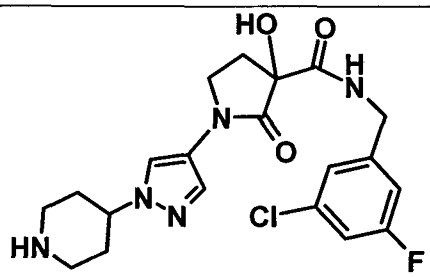<br>3-Hydroxy-2-oxo-1-(1-piperidin-4-yl-1H-pyrazol-4-yl)-pyrrolidin-3-carbonsäure-3-chlor-5-fluor-benzylamid | | 1.57 [436.1] |

| | | | |
|---|---|---|---|
| "A181" | 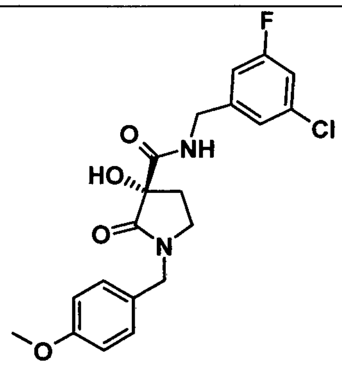 (S)-3-Hydroxy-1-(4-methoxy-benzyl)-2-oxo-pyrrolidin-3-carbonsäure-3-chlor-5-fluor-benzylamid | | 2.11 [407.1] |
| "A182" | 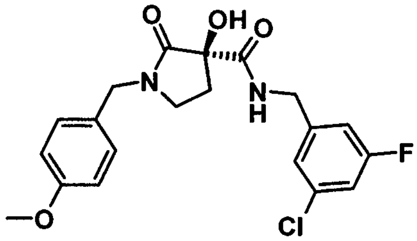 (R)-3-Hydroxy-1-(4-methoxy-benzyl)-2-oxo-pyrrolidin-3-carbonsäure-3-chlor-5-fluor-benzylamid | | 2.11 [407.1] |
| "A183" | 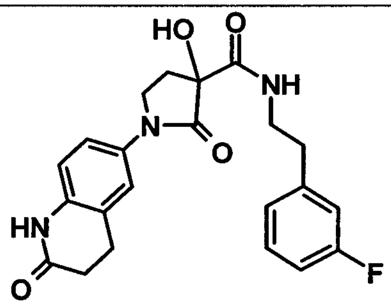 3-Hydroxy-2-oxo-1-(2-oxo-1,2,3,4-tetrahydro-chinolin-6-yl)-pyrrolidin-3-carbonsäure-[2-(3-fluor-phenyl)-ethyl]-amid | 10.06 (s, 1H), 7.96 (t, *J* = 5.9 Hz, 1H), 7.48 (s, 1H), 7.43 (dd, *J* = 8.6, 2.3 Hz, 1H), 7.32 (dd, *J* = 14.3, 7.7 Hz, 1H), 7.09 – 6.97 (m, 3H), 6.86 (d, *J* = 8.6 Hz, 1H), 6.55 (s, 1H), 3.81 – 3.73 (m, 2H), 3.40 (dd, *J* = 12.9, 6.7 Hz, 2H), 2.88 (t, *J* = 7.5 Hz, 2H), 2.78 (t, *J* = 7.3 Hz, 2H), 2.48 – 2.39 (m, 3H), 2.04 (dt, *J* = 12.9, 7.8 Hz, 1H). | 1,72 [412.2] |

| | | | |
|---|---|---|---|
| "A184" | 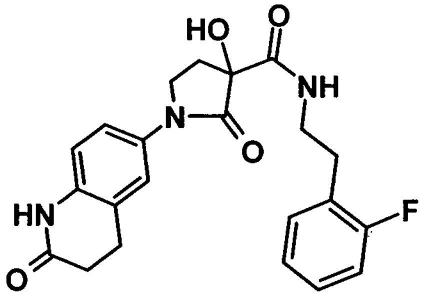 3-Hydroxy-2-oxo-1-(2-oxo-1,2,3,4-tetrahydro-chinolin-6-yl)-pyrrolidin-3-carbonsäure-[2-(2-fluor-phenyl)-ethyl]-amid | $^1$H NMR (400 MHz, DMSO) δ 10.06 (s, 1H), 8.02 (t, *J* = 5.8 Hz, 1H), 7.48 (s, 1H), 7.43 (dd, *J* = 8.6, 2.3 Hz, 1H), 7.27 (dt, *J* = 7.7, 6.7 Hz, 2H), 7.18 – 7.08 (m, 2H), 6.86 (d, *J* = 8.6 Hz, 1H), 3.77 (t, *J* = 6.8 Hz, 2H), 3.26 (m, 2H), 2.88 (t, *J* = 7.5 Hz, 2H), 2.84 – 2.72 (m, 2H), 2.44 (m, 3H), 2.10 – 1.97 (m, 1H). | 1.72 [412.2] |
| "A185" | 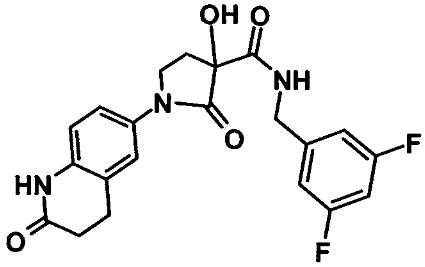 3-Hydroxy-2-oxo-1-(2-oxo-1,2,3,4-tetrahydro-chinolin-6-yl)-pyrrolidin-3-carbonsäure-3,5-difluor-benzylamid | 10.06 (d, *J* = 6.0 Hz, 1H), 8.65 (q, *J* = 6.3 Hz, 1H), 7.48 (d, *J* = 1.9 Hz, 1H), 7.43 (dd, *J* = 8.6, 2.3 Hz, 1H), 7.05 (ddd, *J* = 13.2, 6.6, 3.1 Hz, 1H), 6.99 (d, *J* = 6.8 Hz, 2H), 6.87 (d, *J* = 8.6 Hz, 1H), 6.69 (s, 1H), 4.40 (dd, *J* = 15.7, 6.6 Hz, 1H), 4.26 (dd, *J* = 15.8, 6.0 Hz, 1H), 3.81 (t, *J* = 6.8 Hz, 2H), 2.88 (t, *J* = 7.5 Hz, 2H), 2.62 – 2.54 (m, 1H), 2.47 – 2.39 (m, 2H), 2.11 (dt, *J* = 13.0, 7.6 Hz, 1H). | 1.70 [416.1] |
| "A186" | 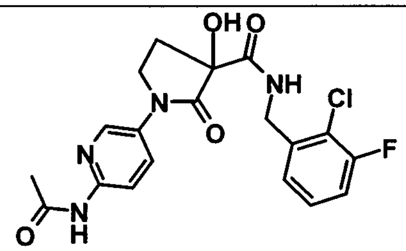 1-(6-Acetylamino-pyridin-3-yl)-3-hydroxy-2-oxo-pyrrolidin-3-carbonsäure-2-chlor-3-fluor-benzylamid | 10.50 (s, 1H), 8.65 (dd, *J* = 5.6, 4.1 Hz, 2H), 8.08 (d, *J* = 8.9 Hz, 2H), 7.42 – 7.25 (m, 2H), 7.21 (d, *J* = 7.5 Hz, 1H), 6.82 (s, 1H), 4.45 (dd, *J* = 16.2, 5.7 Hz, 1H), 4.36 (dd, *J* = 16.1, 5.3 Hz, 1H), 3.88 (m, 2H), 2.70 – 2.58 (m, 1H), 2.18 (dt, *J* = 13.0, 7.6 Hz, 1H), 2.08 (s, 3H). | 1.80 [421.0] |

| | | | |
|---|---|---|---|
| "A187" | 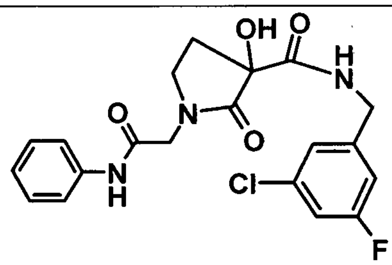 3-Hydroxy-2-oxo-1-phenylcarbamoylmethyl-pyrrolidin-3-carbonsäure-3-chlor-5-fluor-benzylamid | ** 9.89 (s, 1H), 8.75 (t, *J* = 6.3 Hz, 1H), 7.58 (d, *J* = 7.7 Hz, 2H), 7.28 (dd, *J* = 12.9, 4.6 Hz, 2H), 7.27 – 7.24 (m, 1H), 7.22 (s, 1H), 7.12 (d, *J* = 9.6 Hz, 1H), 7.06 (t, *J* = 7.4 Hz, 1H), 6.63 (s, 1H), 4.41 (dd, *J* = 15.6, 6.6 Hz, 1H), 4.26 (dd, *J* = 15.6, 6.0 Hz, 1H), 3.56 – 3.43 (m, 2H), 2.49 – 2.43 (m, 1H), 2.14 – 2.03 (m, 1H). | 2.18 [420.0] |
| "A188" | 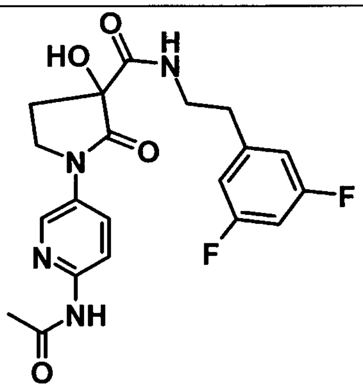 1-(6-Acetylamino-pyridin-3-yl)-3-hydroxy-2-oxo-pyrrolidin-3-carbonsäure-[2-(3,5-difluor-phenyl)-ethyl]-amid | 10.49 (s, 1H), 8.63 (dd, *J* = 2.4, 1.0 Hz, 1H), 8.13 – 8.05 (m, 2H), 8.02 (d, *J* = 6.1 Hz, 1H), 7.01 (ddt, *J* = 9.0, 6.7, 3.3 Hz, 1H), 6.94 (dt, *J* = 6.2, 3.1 Hz, 2H), 6.62 (s, 1H), 3.87 – 3.79 (m, 2H), 3.44 – 3.31 (m, 2H), 2.80 (t, *J* = 7.0 Hz, 2H), 2.46 (m, 1H), 2.08 (s, 3H). | 1.80 [419.1] |
| "A189" | 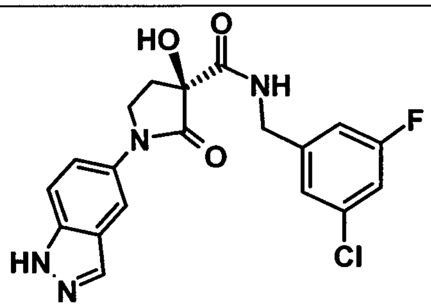 (S)-3-Hydroxy-1-(1H-indazol-5-yl)-2-oxo-pyrrolidin-3-carbonsäure-3-chlor-5-fluor-benzylamid | | 1.94 [403.0] |

| | | | |
|---|---|---|---|
| "A190" | 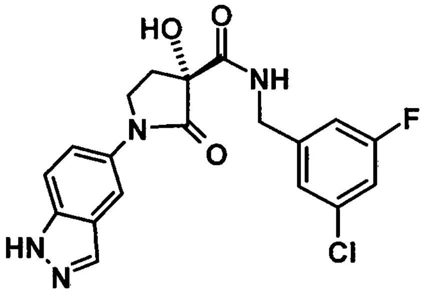 (R)-3-Hydroxy-1-(1H-indazol-5-yl)-2-oxo-pyrrolidin-3-carbonsäure-3-chlor-5-fluor-benzylamid | | 1.94 [403.0] |
| "A191" | 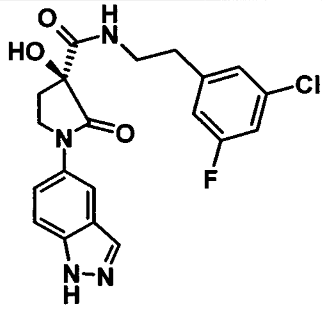 (S)-3-Hydroxy-1-(1H-indazol-5-yl)-2-oxo-pyrrolidin-3-carbonsäure-[2-(3-chlor-5-fluor-phenyl)-ethyl]-amid | | 2.01 [417.0] |
| "A192" | 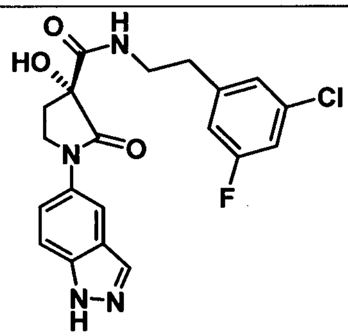 (R)-3-Hydroxy-1-(1H-indazol-5-yl)-2-oxo-pyrrolidin-3-carbonsäure-[2-(3-chlor-5-fluor-phenyl)-ethyl]-amid | | 2.01 [417.1] |

| | | | |
|---|---|---|---|
| "A193" | 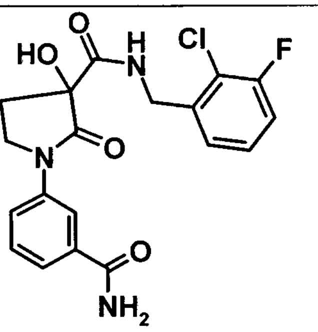 1-(3-Carbamoyl-phenyl)-3-hydroxy-2-oxo-pyrrolidin-3-carbonsäure-2-chlor-3-fluor-benzylamid | ** 8.66 (t, $J$ = 6.3 Hz, 1H), 8.05 (t, $J$ = 1.8 Hz, 1H), 8.00 (s, 1H), 7.97 – 7.91 (m, 1H), 7.68 (d, $J$ = 7.8 Hz, 1H), 7.48 (t, $J$ = 8.0 Hz, 1H), 7.43 – 7.33 (m, 2H), 7.32 – 7.25 (m, 1H), 7.22 (d, $J$ = 7.7 Hz, 1H), 6.80 (s, 1H), 4.45 (dd, $J$ = 16.2, 6.5 Hz, 1H), 4.36 (dd, $J$ = 16.2, 6.0 Hz, 1H), 2.64 (dt, $J$ = 11.8, 5.7 Hz, 1H), 2.38 (m, 2H), 2.17 (dt, $J$ = 13.0, 7.6 Hz, 1H). | 1.82 [406.0] |
| "A194" | 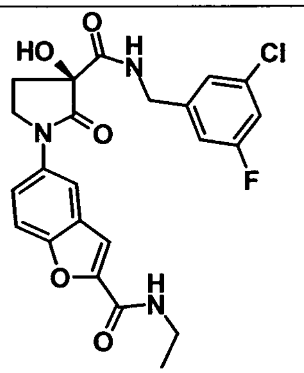 (S)-1-(2-Ethylcarbamoyl-benzofuran-5-yl)-3-hydroxy-2-oxo-pyrrolidin-3-carbonsäure-3-chlor-5-fluor-benzylamid | | 2.17 [474.1] |
| "A195" | 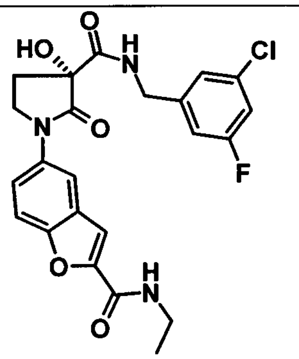 (R)-1-(2-Ethylcarbamoyl-benzofuran-5-yl)-3-hydroxy-2- | | 2.17 [474.1] |

| | | | |
|---|---|---|---|
| | oxo-pyrrolidin-3-carbonsäure-3-chlor-5-fluor-benzylamid | | |
| "A196" | 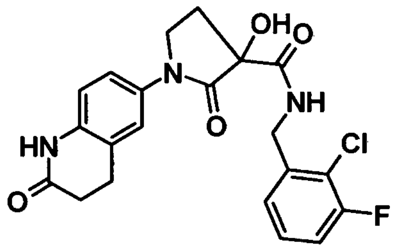<br>3-Hydroxy-2-oxo-1-(2-oxo-1,2,3,4-tetrahydro-chinolin-6-yl)-pyrrolidin-3-carbonsäure-2-chlor-3-fluor-benzylamid | ** 7.49 (d, $J$ = 2.2 Hz, 1H), 7.43 (dd, $J$ = 8.6, 2.4 Hz, 1H), 7.35 (tt, $J$ = 10.9, 5.5 Hz, 1H), 7.29 (dd, $J$ = 12.6, 4.7 Hz, 1H), 7.22 (d, $J$ = 7.6 Hz, 1H), 6.88 (d, $J$ = 8.6 Hz, 1H), 4.45 (d, $J$ = 16.2 Hz, 1H), 4.36 (d, $J$ = 16.2 Hz, 1H), 3.87 – 3.74 (m, 3H), 2.89 (dd, $J$ = 9.5, 5.6 Hz, 2H), 2.63 – 2.54 (m, 1H), 2.48 – 2.40 (m, 2H), 2.14 (dt, $J$ = 13.0, 7.6 Hz, 1H). | 1.87 [432.0] |
| "A197" | 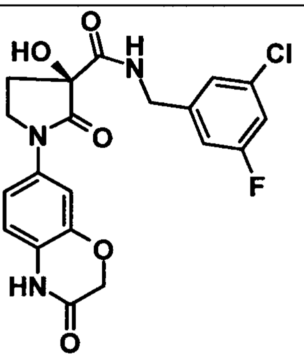<br>(S)-3-Hydroxy-2-oxo-1-(3-oxo-3,4-dihydro-2H-benzo[1,4]-oxazin-7-yl)-pyrrolidin-3-carbonsäure-3-chlor-5-fluor-benzylamid | | 2.08 [434.0] |

| | | | |
|---|---|---|---|
| "A198" | 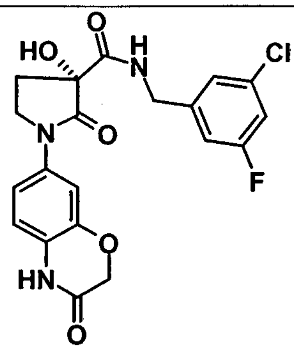<br>(R)-3-Hydroxy-2-oxo-1-(3-oxo-3,4-dihydro-2H-benzo[1,4]-oxazin-7-yl)-pyrrolidin-3-carbonsäure-3-chlor-5-fluor-benzylamid | | 2.08 [434.0] |
| "A199" | 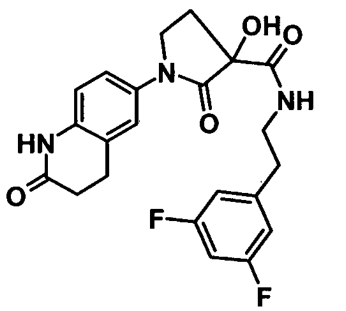<br>3-Hydroxy-2-oxo-1-(2-oxo-1,2,3,4-tetrahydro-chinolin-6-yl)-pyrrolidin-3-carbonsäure-[2-(3,5-difluor-phenyl)-ethyl]-amid | ** 10.06 (s, 1H), 7.99 (t, *J* = 6.0 Hz, 1H), 7.48 (d, *J* = 2.2 Hz, 1H), 7.42 (dd, *J* = 8.6, 2.4 Hz, 1H), 7.03 (tt, *J* = 9.5, 2.3 Hz, 1H), 6.99 – 6.93 (m, 2H), 6.86 (d, *J* = 8.6 Hz, 1H), 6.55 (s, 1H), 3.76 (dd, *J* = 14.4, 8.5 Hz, 2H), 3.44 – 3.36 (m, 2H), 2.88 (dd, *J* = 10.1, 4.9 Hz, 2H), 2.79 (t, *J* = 7.0 Hz, 2H), 2.48 – 2.39 (m, 3H), 2.04 (dt, *J* = 12.8, 7.8 Hz, 1H). | |
| "A200" | 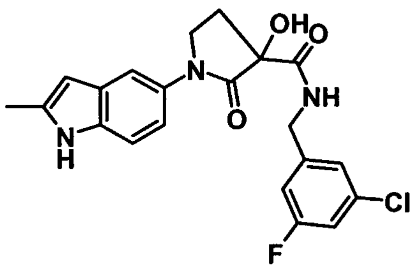<br>3-Hydroxy-1-(2-methyl-1H-indol-5-yl)-2-oxo-pyrrolidin-3-carbonsäure-3-chlor-5-fluor-benzylamid | ** 10.91 (s, 1H), 8.65 (t, *J* = 6.4 Hz, 1H), 7.57 (d, *J* = 1.8 Hz, 1H), 7.30 (dd, *J* = 8.7, 2.0 Hz, 1H), 7.27 – 7.20 (m, 3H), 7.11 (t, *J* = 11.6 Hz, 1H), 6.64 (s, 1H), 6.12 (s, 1H), 4.40 (dd, *J* = 15.7, 6.8 Hz, 1H), 4.26 (dd, *J* = 15.7, 6.0 Hz, 1H), 3.86 (dd, *J* = 14.7, 8.5 Hz, 2H), 2.62 – | |

| | | | |
|---|---|---|---|
| | | 2.54 (m, 1H), 2.37 (s, 3H), 2.12 (dt, $J$ = 12.9, 7.5 Hz, 1H). | |
| "A201" | 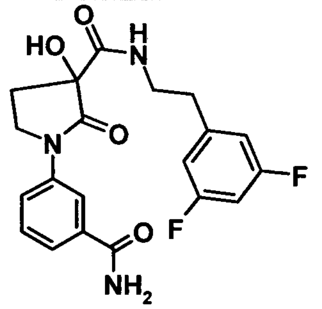 1-(3-Carbamoyl-phenyl)-3-hydroxy-2-oxo-pyrrolidin-3-carbonsäure-[2-(3,5-difluor-phenyl)-ethyl]-amid | ** 8.04 (dd, $J$ = 12.8, 7.1 Hz, 3H), 7.98 – 7.92 (m, 1H), 7.70 (t, $J$ = 10.4 Hz, 1H), 7.49 (t, $J$ = 7.9 Hz, 1H), 7.40 (s, 1H), 7.07 – 7.00 (m, 1H), 6.97 (d, $J$ = 6.8 Hz, 2H), 6.66 (s, 1H), 3.88 (dd, $J$ = 7.3, 6.1 Hz, 2H), 3.50 – 3.38 (m, 3H), 2.80 (dd, $J$ = 16.6, 9.7 Hz, 2H), 2.49 – 2.44 (m, 1H), 2.10 (dt, $J$ = 12.9, 7.8 Hz, 1H). | 1.83 [404.1] |
| "A202" | 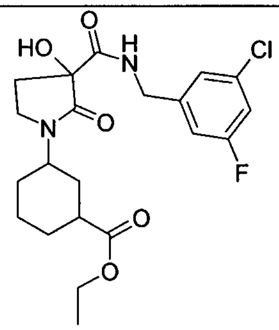 3-[3-(3-Chlor-5-fluor-benzylcarbamoyl)-3-hydroxy-2-oxo-pyrrolidin-1-yl]-cyclohexan-carbonsäure-ethylester | ** 7.20 (d, $J$ = 1.2 Hz, 1H), 7.09 (t, $J$ = 7.9 Hz, 2H), 4.47 (d, $J$ = 15.9 Hz, 1H), 4.33 (d, $J$ = 15.8 Hz, 1H), 4.12 – 4.04 (m, 2H), 3.89 (dt, $J$ = 11.7, 9.9 Hz, 1H), 3.43 (q, $J$ = 7.1 Hz, 2H), 2.56 – 2.41 (m, 2H), 2.07 (m, 1H), 2.00 – 1.82 (m, 3H), 1.69 (m, 1H), 1.65 – 1.53 (m, 1H), 1.54 – 1.38 (m, 2H), 1.32 – 1.23 (m, 1H), 1.21 (t, $J$ = 7.1 Hz, 3H). | 2.23 [441.1] |
| "A203" | 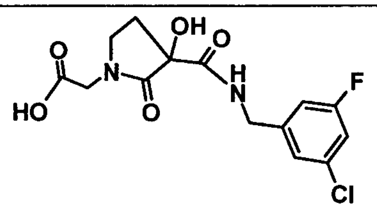 [3-(3-Chlor-5-fluor-benzylcarbamoyl)-3-hydroxy-2-oxo-pyrrolidin-1-yl]-essigsäure | | 1.70 [345.0] |

| | | | |
|---|---|---|---|
| "A204" | 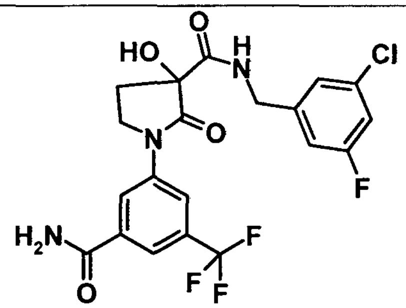 1-(3-Carbamoyl-5-trifluoromethyl-phenyl)-3-hydroxy-2-oxo-pyrrolidin-3-carbonsäure-3-chlor-5-fluor-benzylamid | | 2.14 [474.0] |
| "A205" | 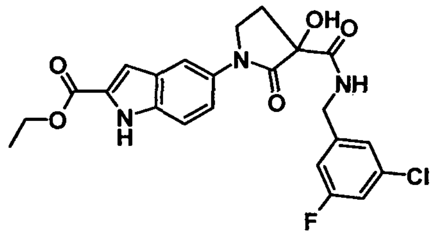 5-[3-(3-Chlor-5-fluor-benzylcarbamoyl)-3-hydroxy-2-oxo-pyrrolidin-1-yl]-1H-indol-2-carbonsäure-ethylester | ** 8.71 (s, 1H), 7.82 (d, *J* = 1.9 Hz, 1H), 7.64 (dd, *J* = 9.0, 2.1 Hz, 1H), 7.47 (d, *J* = 9.0 Hz, 1H), 7.26 (dt, *J* = 8.7, 2.1 Hz, 1H), 7.22 (s, 1H), 7.16 (d, *J* = 0.5 Hz, 1H), 7.12 (d, *J* = 9.6 Hz, 1H), 4.40 (d, *J* = 15.7 Hz, 1H), 4.35 (q, *J* = 7.1 Hz, 2H), 4.27 (d, *J* = 15.7 Hz, 1H), 3.90 (dd, *J* = 14.6, 8.4 Hz, 2H), 2.66 – 2.57 (m, 1H), 2.15 (dt, *J* = 12.9, 7.5 Hz, 1H), 1.35 (t, *J* = 7.1 Hz, 3H). | 2.28 [474.1] |
| "A206" | 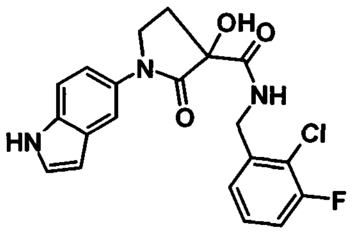 3-Hydroxy-1-(1H-indol-5-yl)-2-oxo-pyrrolidin-3-carbonsäure-2-chlor-3-fluor-benzylamid | ** 11.10 (s, 1H), 8.61 (t, *J* = 6.3 Hz, 1H), 7.71 (s, 1H), 7.43 – 7.37 (m, 2H), 7.35 (dt, *J* = 11.0, 4.2 Hz, 2H), 7.32 – 7.27 (m, 1H), 7.25 (d, *J* = 7.6 Hz, 1H), 6.70 (s, 1H), 6.46 – 6.41 (m, 1H), 4.46 (dd, *J* = 16.2, 6.5 Hz, 1H), 4.36 (dd, *J* = 16.2, 6.0 Hz, 1H), 3.94 – 3.83 (m, 2H), | 2.07 [402.0] |

| | | | |
|---|---|---|---|
| | | 2.64 (m, 1H), 2.21 – 2.12 (m, 1H). | |
| "A207" | 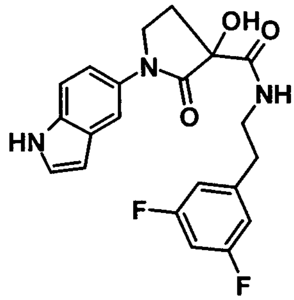 3-Hydroxy-1-(1H-indol-5-yl)-2-oxo-pyrrolidin-3-carbonsäure-[2-(3,5-difluor-phenyl)-ethyl]-amid | ** 11.12 (d, *J* = 37.6 Hz, 1H), 7.98 (t, *J* = 6.0 Hz, 1H), 7.72 – 7.67 (m, 1H), 7.43 – 7.37 (m, 2H), 7.37 – 7.33 (m, 1H), 7.03 (tt, *J* = 9.5, 2.3 Hz, 1H), 6.99 – 6.94 (m, 2H), 6.52 (s, 1H), 6.46 – 6.41 (m, 1H), 3.88 – 3.80 (m, 2H), 3.44 – 3.37 (m, 1H), 3.37 – 3.31 (m, 1H), 2.80 (t, *J* = 7.0 Hz, 2H), 2.48 – 2.44 (m, 1H), 2.07 (dt, *J* = 12.8, 7.7 Hz, 1H). | 2.08 [400.1] |
| "A208" | 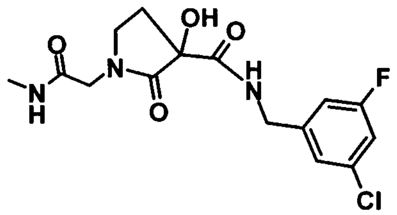 3-Hydroxy-1-methylcarbamoyl-methyl-2-oxo-pyrrolidin-3-carbonsäure-3-chlor-5-fluor-benzylamid | ** 8.69 (t, *J* = 6.3 Hz, 1H), 7.81 (d, *J* = 4.5 Hz, 1H), 7.26 (dt, *J* = 8.8, 2.0 Hz, 1H), 7.20 (s, 1H), 7.09 (d, *J* = 9.6 Hz, 1H), 6.55 (s, 1H), 4.38 (dd, *J* = 15.7, 6.7 Hz, 1H), 4.25 (dd, *J* = 15.7, 6.0 Hz, 1H), 3.47 – 3.35 (m, 2H), 2.59 (d, *J* = 4.6 Hz, 3H), 2.43 (ddd, *J* = 12.8, 7.5, 3.3 Hz, 1H), 2.11 – 1.97 (m, 1H). | 1.74 [358.0] |
| "A209" | 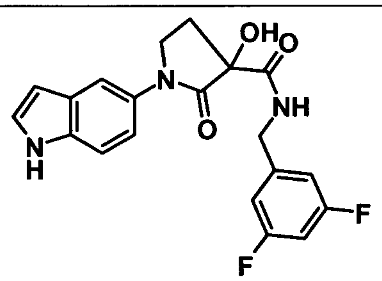 3-Hydroxy-1-(1H-indol-5-yl)-2-oxo-pyrrolidin-3-carbonsäure-3,5-difluor-benzylamid | ** 11.10 (s, 1H), 8.65 (t, *J* = 6.4 Hz, 1H), 7.72 – 7.68 (m, 1H), 7.44 – 7.37 (m, 2H), 7.36 (t, *J* = 2.7 Hz, 1H), 7.08 – 7.02 (m, 1H), 7.02 – 6.95 (m, 2H), 6.66 (s, 1H), 6.47 – 6.39 (m, 1H), 4.42 (dd, *J* = 15.8, 6.8 Hz, 1H), 4.27 (dd, *J* = 15.8, 6.0 Hz, | 2.00 [386.1] |

| | | | |
|---|---|---|---|
| | | 1H), 3.93 – 3.83 (m, 2H), 2.62 (ddd, *J* = 12.0, 6.9, 4.8 Hz, 1H), 2.14 (dt, *J* = 12.9, 7.5 Hz, 1H). | |
| "A210" | 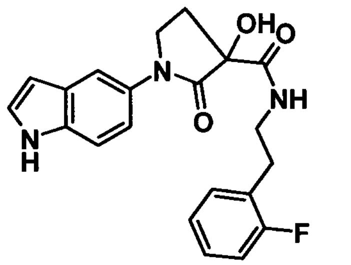<br>3-Hydroxy-1-(1H-indol-5-yl)-2-oxo-pyrrolidin-3-carbonsäure-[2-(2-fluor-phenyl)-ethyl]-amid | 11.08 (s, 1H), 7.98 (dd, *J* = 15.2, 9.2 Hz, 1H), 7.70 (s, 1H), 7.43 – 7.37 (m, 2H), 7.36 – 7.34 (m, 1H), 7.30 (dd, *J* = 8.4, 6.7 Hz, 1H), 7.28 – 7.22 (m, 1H), 7.15 (d, *J* = 7.1 Hz, 1H), 7.12 (ddd, *J* = 4.5, 3.8, 1.2 Hz, 1H), 6.67 – 6.22 (m, 2H), 3.90 – 3.77 (m, 2H), 3.40 (m, 1H), 3.34 – 3.29 (m, 1H), 2.86 – 2.76 (m, 2H), 2.49 – 2.42 (m, 1H), 2.07 (dt, *J* = 12.8, 7.9 Hz, 1H). | 2.01 [382.1] |
| "A211" | 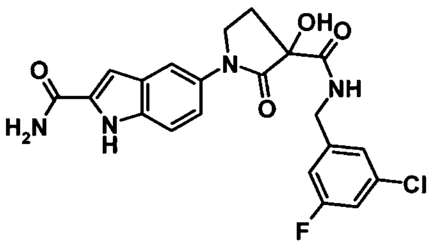<br>5-[3-(3-Chlor-5-fluor-benzylcarbamoyl)-3-hydroxy-2-oxo-pyrrolidin-1-yl]-1H-indol-2-carboxamid | ** 11.54 (s, 1H), 8.67 (t, *J* = 6.4 Hz, 1H), 7.94 (s, 1H), 7.77 (d, *J* = 2.0 Hz, 1H), 7.54 (dd, *J* = 8.9, 2.1 Hz, 1H), 7.42 (d, *J* = 8.9 Hz, 1H), 7.33 (s, 1H), 7.28 – 7.23 (m, 1H), 7.22 (s, 1H), 7.16 – 7.08 (m, 2H), 6.67 (s, 1H), 4.40 (dd, *J* = 15.8, 6.7 Hz, 1H), 4.27 (dd, *J* = 15.7, 6.0 Hz, 1H), 3.94 – 3.85 (m, 2H), 2.61 (dt, *J* = 11.9, 5.8 Hz, 1H), 2.14 (dt, *J* = 12.9, 7.5 Hz, 1H). | 1.91 [445.0] |

| | | | |
|---|---|---|---|
| "A212" | 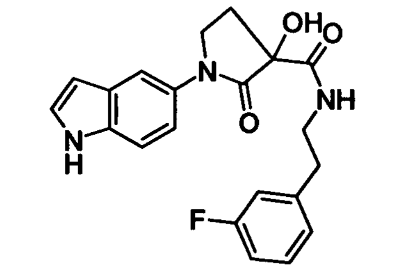 3-Hydroxy-1-(1H-indol-5-yl)-2-oxo-pyrrolidin-3-carbonsäure-[2-(3-fluor-phenyl)-ethyl]-amid | 11.08 (s, 1H), 7.94 (t, $J$ = 5.9 Hz, 1H), 7.70 (s, 1H), 7.44 – 7.36 (m, 2H), 7.33 (dt, $J$ = 14.2, 5.2 Hz, 2H), 7.11 – 6.95 (m, 3H), 6.50 (s, 1H), 6.43 (s, 1H), 3.85 (m, 2H), 3.46 – 3.29 (m, 2H), 2.79 (t, $J$ = 7.2 Hz, 2H), 2.49 – 2.37 (m, 1H), 2.07 (dt, $J$ = 12.8, 7.9 Hz, 1H). | 2.01 [382.1] |
| "A213" | 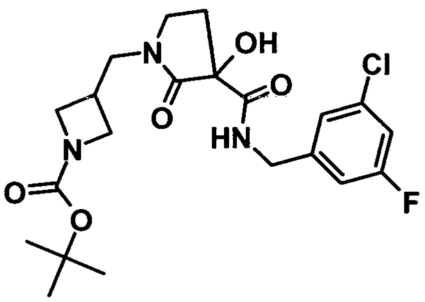 3-[3-(3-Chlor-5-fluor-benzylcarbamoyl)-3-hydroxy-2-oxo-pyrrolidin-1-ylmethyl]-azetidin-1-carbonsäure-tert-butylester | - | 2.18 [400.1] |
| "A214" | 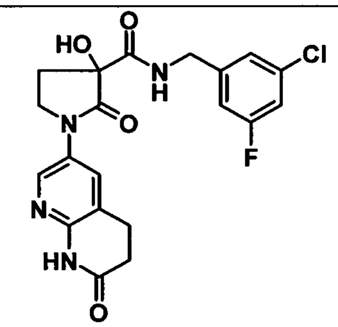 3-Hydroxy-2-oxo-1-(7-oxo-5,6,7,8-tetrahydro-[1,8]naphthyridin-3-yl)-pyrrolidin-3-carbonsäure-3-chlor-5-fluor-benzylamid | 10.51 (s, 1H), 8.74 (t, $J$ = 6.3 Hz, 1H), 8.40 (d, $J$ = 2.5 Hz, 1H), 7.94 (d, $J$ = 2.1 Hz, 1H), 7.27 (d, $J$ = 8.7 Hz, 1H), 7.20 (s, 1H), 7.10 (d, $J$ = 9.1 Hz, 1H), 6.79 (s, 1H), 4.38 (dd, $J$ = 15.6, 6.5 Hz, 1H), 4.24 (dd, $J$ = 15.7, 6.1 Hz, 1H), 3.84 (t, $J$ = 5.9 Hz, 2H), 2.90 (t, $J$ = 7.6 Hz, 2H), 2.63 – 2.55 (m, 1H), 2.18 – 2.09 (m, 1H). | 3.18 [433] |

| | | | |
|---|---|---|---|
| "A215" | 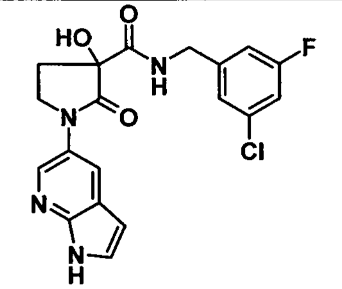 3-Hydroxy-2-oxo-1-(1H-pyrrolo[2,3-b]pyridin-5-yl)-pyrrolidin-3-carbonsäure-3-chlor-5-fluor-benzylamid | 11.70 (s, 1H), 8.74 (t, *J* = 6.4 Hz, 1H), 8.49 (d, *J* = 2.4 Hz, 1H), 8.16 (d, *J* = 2.4 Hz, 1H), 7.53 – 7.48 (m, 1H), 7.27 (dt, *J* = 8.8, 2.0 Hz, 1H), 7.22 (s, 1H), 7.12 (d, *J* = 9.6 Hz, 1H), 6.77 (s, 1H), 6.46 (dd, *J* = 3.3, 1.8 Hz, 1H), 4.40 (dd, *J* = 15.8, 6.7 Hz, 1H), 4.26 (dd, *J* = 15.8, 6.0 Hz, 1H), 3.96 – 3.85 (m, 2H), 2.68 – 2.57 (m, 1H), 2.16 (dt, *J* = 12.9, 7.5 Hz, 1H). | 3.2 [403] |
| "A216" | 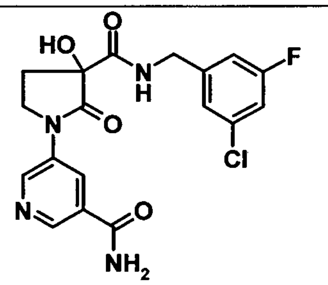 5-[3-(3-Chlor-5-fluor-benzylcarbamoyl)-3-hydroxy-2-oxo-pyrrolidin-1-yl]-nicotinamid | 9.10 (d, *J* = 2.0 Hz, 1H), 8.83 (s, 1H), 8.79 (t, *J* = 6.3 Hz, 1H), 8.44 (s, 1H), 8.23 (s, 1H), 7.67 (s, 1H), 7.28 (d, *J* = 8.7 Hz, 1H), 7.20 (s, 1H), 7.10 (d, *J* = 9.5 Hz, 1H), 6.88 (s, 1H), 4.38 (dd, *J* = 15.7, 6.6 Hz, 1H), 4.25 (dd, *J* = 15.6, 6.0 Hz, 1H), 3.94 (m, 2H), 2.68 – 2.58 (m, 1H), 2.23 – 2.13 (m, 1H). | 2.89 [407] |
| "A217" | 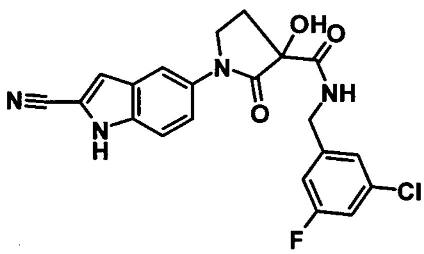 1-(2-Cyan-1H-indol-5-yl)-3-hydroxy-2-oxo-pyrrolidin-3-carbonsöure-3-chlor-5-fluor-benzylamid | ** 8.69 (t, *J* = 6.3 Hz, 1H), 7.86 (d, *J* = 1.8 Hz, 1H), 7.78 – 7.74 (m, 1H), 7.50 (d, *J* = 9.0 Hz, 1H), 7.38 (d, *J* = 0.7 Hz, 1H), 7.27 (dt, *J* = 8.7, 2.1 Hz, 1H), 7.22 (s, 1H), 7.12 (d, *J* = 9.6 Hz, 1H), 6.72 (s, 1H), 4.40 (dd, *J* = 15.7, 6.7 Hz, 1H), 4.26 (dd, *J* = 15.7, 6.0 Hz, 1H), 3.96 – 3.82 (m, 2H), 2.66 – 2.57 (m, 1H), 2.22 – 2.09 (m, 1H). | 2.17 [427.0] |

| | | | |
|---|---|---|---|
| "A218" | 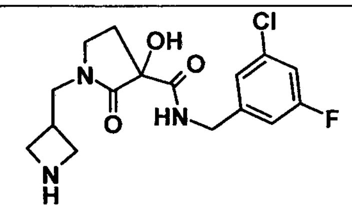<br>1-Azetidin-3-ylmethyl-3-hydroxy-2-oxo-pyrrolidin-3-carbonsäure-3-chlor-5-fluor-benzylamid | ** 8.60 (t, *J* = 6.4 Hz, 1H), 8.37 (s, 1H), 7.31 – 7.24 (m, 1H), 7.23 – 7.18 (m, 1H), 7.10 (d, *J* = 9.0 Hz, 1H), 4.38 (dd, *J* = 15.7, 6.7 Hz, 1H), 4.29 – 4.17 (m, 2H), 3.88 – 3.73 (m, 5H), 3.64 – 3.46 (m, 1), 3.06 – 2.91 (m, 3H), 2.48 – 2.40 (m, 1H), 2.05 – 1.90 (m, 1H). | 1.39 [356.1] |
| "A219" | 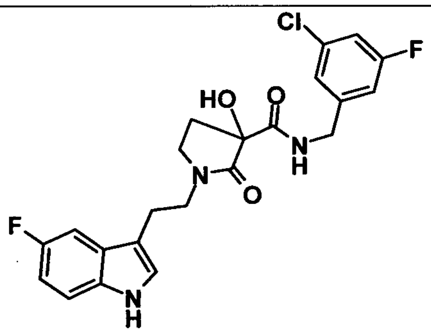<br>1-[2-(5-Fluor-1H-indol-3-yl)-ethyl]-3-hydroxy-2-oxo-pyrrolidin-3-carbonsäure-3-chlor-5-fluor-benzylamid | | 2.25 [448.1] |
| "A220" | 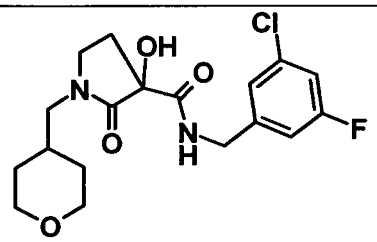<br>3-Hydroxy-2-oxo-1-(tetrahydro-pyran-4-ylmethyl)-pyrrolidin-3-carbonsäure-3-chlor-5-fluor-benzylamid | | 1.86 [385.1] |

| | | | |
|---|---|---|---|
| "A221" | 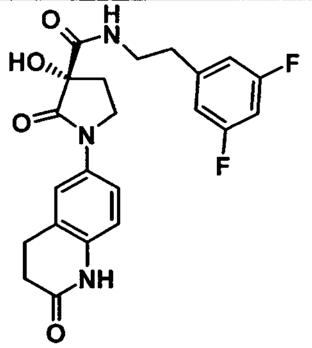<br>(S)-3-Hydroxy-2-oxo-1-(2-oxo-1,2,3,4-tetrahydro-chinolin-6-yl)-pyrrolidin-3-carbonsäure-[2-(3,5-difluor-phenyl)-ethyl]-amid | | 1.87 [430.1] |
| "A222" | 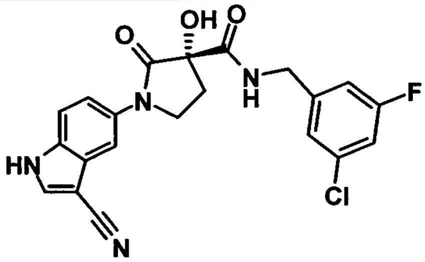<br>(S)-1-(3-Cyan-1H-indol-5-yl)-3-hydroxy-2-oxo-pyrrolidin-3-carbonsäure-3-chlor-5-fluor-benzylamid | | 2.07 [427.0] |
| "A223" | 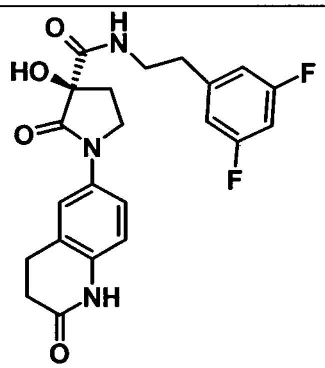<br>(R)-3-Hydroxy-2-oxo-1-(2-oxo-1,2,3,4-tetrahydro-chinolin-6-yl)-pyrrolidin-3-carbonsäure-[2-(3,5-difluor-phenyl)-ethyl]-amid | | 1.88 [430.1] |

| | | | |
|---|---|---|---|
| "A224" | 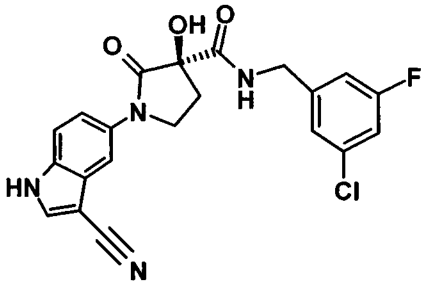 (R)-1-(3-Cyan-1H-indol-5-yl)-3-hydroxy-2-oxo-pyrrolidin-3-carbonsäure-3-chlor-5-fluor-benzylamid | | 2.07 [427.0] |
| "A225" | 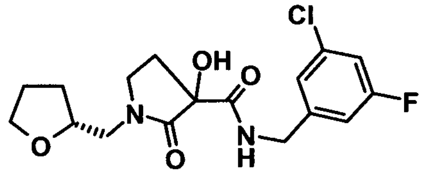 3-Hydroxy-2-oxo-1-[(R)-1-(tetrahydro-furan-2-yl)methyl]-pyrrolidin-3-carbonsäure-3-chlor-5-fluor-benzylamid | | 1.89 [371.1] |
| "A226" | 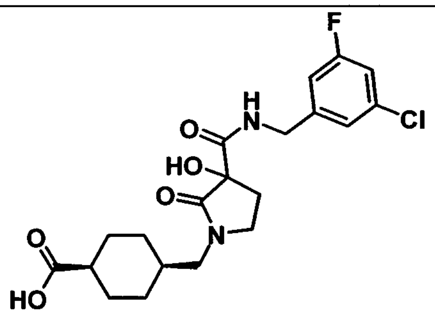 4-[3-(3-Chlor-5-fluor-benzylcarbamoyl)-3-hydroxy-2-oxo-pyrrolidin-1-ylmethyl]-cyclo-hexancarbonsäure | ** 12.01 (s, 1H), 8.53 (t, *J* = 6.4 Hz, 1H), 7.24 (dt, *J* = 8.7, 2.1 Hz, 1H), 7.18 (s, 1H), 7.10 – 7.06 (m, 1H), 6.40 (s, 1H), 4.41 – 4.32 (m, 1H), 4.23 (dd, *J* = 15.8, 6.0 Hz, 1H), 3.33 (ddd, *J* = 12.3, 8.6, 5.3 Hz, 2H), 3.19 (dd, *J* = 13.4, 8.4 Hz, 1H), 3.00 (dd, *J* = 13.4, 7.1 Hz, 1H), 2.48 – 2.38 (m, 2H), 1.95 (ddd, *J* = 12.9, 8.5, 6.7 Hz, 1H), 1.89 – 1.78 (m, 2H), 1.73 (dt, *J* = 16.1, 6.2 Hz, 1H), 1.54 | 1.98 [427.2] |

| | | | |
|---|---|---|---|
| | | – 1.38 (m, 4H), 1.22 – 1.04 (m, 2H). | |
| "A227" | 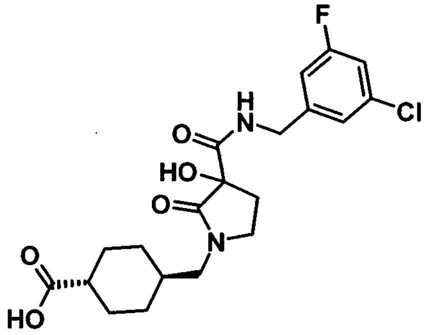 4-[3-(3-Chlor-5-fluor-benzylcarbamoyl)-3-hydroxy-2-oxo-pyrrolidin-1-ylmethyl]-cyclo-hexancarbonsäure | ** 11.94 (s, 1H), 8.54 (t, *J* = 6.4 Hz, 1H), 7.24 (dt, *J* = 8.7, 2.1 Hz, 1H), 7.18 (s, 1H), 7.11 – 7.05 (m, 1H), 6.41 (s, 1H), 4.37 (dd, *J* = 15.8, 6.8 Hz, 1H), 4.22 (dd, *J* = 15.8, 6.0 Hz, 1H), 3.42 – 3.30 (m, 2H), 3.12 (dd, *J* = 13.4, 7.9 Hz, 1H), 2.98 (dd, *J* = 13.4, 6.8 Hz, 1H), 2.45 (ddd, *J* = 11.6, 7.6, 3.8 Hz, 1H), 2.10 (tt, *J* = 12.0, 3.4 Hz, 1H), 2.01 – 1.91 (m, 1H), 1.91 – 1.78 (m, 2H), 1.68 (d, *J* = 12.5 Hz, 2H), 1.57 (dqd, *J* = 15.2, 7.6, 3.7 Hz, 1H), 1.34 – 1.16 (m, 2H), 1.00 – 0.79 (m, 2H). | 1.89 [427.1] |
| "A228" | 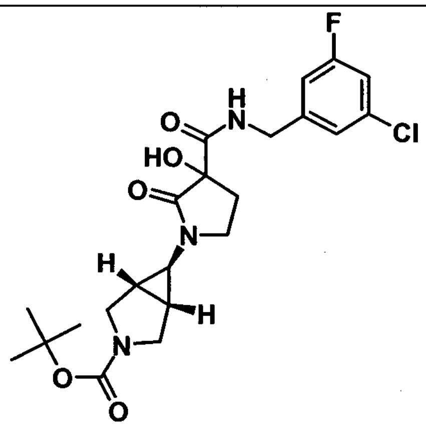 (1S,5R,6S)-6-[3-(3-Chlor-5-fluor-benzylcarbamoyl)-3-hydroxy-2-oxo-pyrrolidin-1-yl]-3-aza-bicyclo[3.1.0]hexan-3-carbonsäure-tert-butylester | ** 8.60 (dt, *J* = 12.7, 6.1 Hz, 1H), 7.27 (dt, *J* = 8.8, 2.1 Hz, 1H), 7.20 (s, 1H), 7.10 (d, *J* = 9.6 Hz, 1H), 6.47 (s, 1H), 4.37 (dd, *J* = 15.7, 6.7 Hz, 1H), 4.23 (dd, *J* = 15.7, 6.0 Hz, 1H), 3.58 (s, 1H), 3.55 – 3.48 (m, 2H), 3.40 – 3.32 (m, 2H), 3.27 (m, 1H), 2.42 (ddd, *J* = 12.3, 7.1, 4.9 Hz, 1H), 2.34 (m, 1H), 1.99 – 1.87 (m, 3H), 1.39 (s, 9H). | 2.23 [412.0] |

| | | | |
|---|---|---|---|
| "A229" | 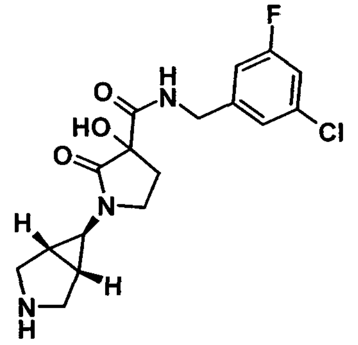 1-(1S,5R,6S)-3-Aza-bicyclo[3.1.0]hex-6-yl-3-hydroxy-2-oxo-pyrrolidin-3-carbonsäure-3-chlor-5-fluor-benzylamid | ** 8.56 (t, $J$ = 6.3 Hz, 1H), 8.24 (s, 1H), 7.27 (dt, $J$ = 8.8, 2.0 Hz, 1H), 7.20 (s, 1H), 7.10 (d, $J$ = 9.3 Hz, 1H), 4.37 (dd, $J$ = 15.7, 6.8 Hz, 1H), 4.23 (dd, $J$ = 15.7, 5.9 Hz, 1H), 3.30 – 3.23 (m, 2H), 3.07 (m, 2H), 2.90 (m, 2H), 2.66 – 2.61 (m, 1H), 2.45 – 2.39 (m, 1H), 1.98 – 1.89 (m, 1H), 1.89 – 1.78 (m, 2H). | 1.42 [368.0] |
| "A230" | 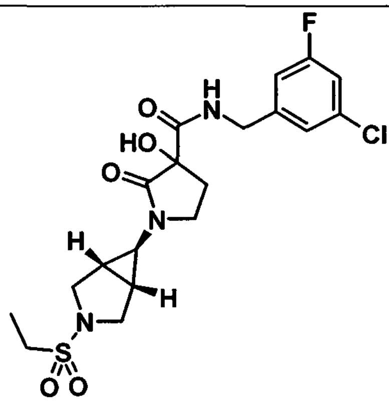 1-((1S,5R,6S)-3-Ethansulfonyl-3-aza-bicyclo[3.1.0]hex-6-yl)-3-hydroxy-2-oxo-pyrrolidin-3-carbonsäure-3-chlor-5-fluor-benzylamid | ** 8.56 (t, $J$ = 6.4 Hz, 1H), 7.26 (dt, $J$ = 8.8, 2.1 Hz, 1H), 7.18 (d, $J$ = 8.0 Hz, 1H), 7.08 (d, $J$ = 9.4 Hz, 1H), 6.47 (s, 1H), 4.35 (dd, $J$ = 15.7, 6.7 Hz, 1H), 4.22 (dd, $J$ = 15.7, 6.0 Hz, 1H), 3.49 – 3.39 (m, 4H), 3.09 (q, $J$ = 7.3 Hz, 2H), 2.56 (t, $J$ = 2.3 Hz, 1H), 2.42 (ddd, $J$ = 12.4, 7.1, 4.9 Hz, 1H), 2.05 – 2.01 (m, 1H), 2.01 – 1.96 (m, 1H), 1.92 (ddd, $J$ = 17.4, 10.0, 5.5 Hz, 1H), 1.18 (t, $J$ = 7.3 Hz, 4H). | 1.93 [460.0] |

| | | | |
|---|---|---|---|
| "A231" | 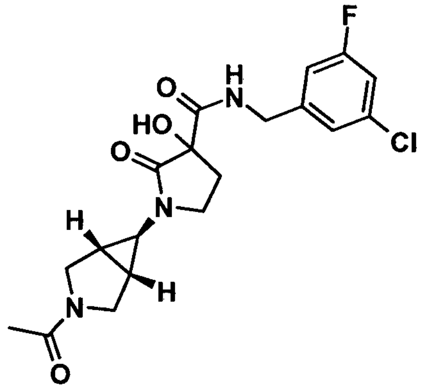 1-((1S,5R,6S)-3-Acetyl-3-aza-bicyclo[3.1.0]hex-6-yl)-3-hydroxy-2-oxo-pyrrolidin-3-carbonsäure-3-chlor-5-fluor-benzylamid | ** 8.57 (t, $J$ = 6.3 Hz, 1H), 7.96 (s, 1H), 7.31 – 7.23 (m, 2H), 7.19 (s, 1H), 7.09 (t, $J$ = 7.1 Hz, 1H), 4.36 (dd, $J$ = 15.7, 6.8 Hz, 1H), 4.28 – 4.19 (m, 2H), 3.67 – 3.57 (m, 4H), 3.34 – 3.26 (m, 3H), 2.46 – 2.34 (m, 2H), 2.08 – 2.05 (m, 1H), 2.05 – 2.00 (m, 1H), 2.00 – 1.92 (m, 2H), 1.90 (s, 3H). | 1.75 [410.1] |
| "A232" | 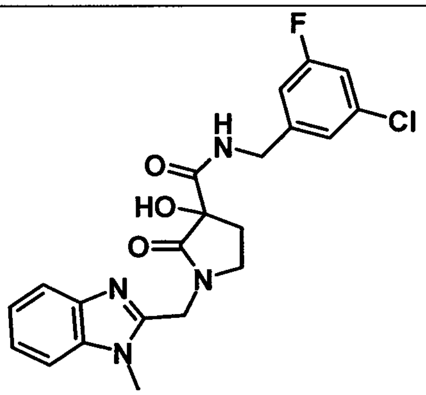 3-Hydroxy-1-(1-methyl-1H-benzimidazol-2-ylmethyl)-2-oxo-pyrrolidin-3-carbonsäure-3-chlor-5-fluor-benzylamid | ** 8.69 (t, $J$ = 6.3 Hz, 1H), 7.59 (d, $J$ = 7.8 Hz, 1H), 7.52 (d, $J$ = 7.9 Hz, 1H), 7.28 – 7.22 (m, 2H), 7.21 – 7.17 (m, 2H), 7.12 – 7.07 (m, 1H), 4.84 (d, $J$ = 15.3 Hz, 1H), 4.67 (d, $J$ = 15.3 Hz, 1H), 4.39 (dd, $J$ = 15.8, 6.7 Hz, 1H), 4.25 (dd, $J$ = 15.7, 6.1 Hz, 1H), 3.71 (s, 3H), 3.37 (m, 2H), 3.26 – 3.17 (m, 2H), 2.44 (ddd, $J$ = 12.0, 8.0, 3.8 Hz, 1H), 2.00 (ddd, $J$ = 13.1, 8.7, 6.4 Hz, 1H). | 1.78 [431.1] |
| "A233" | 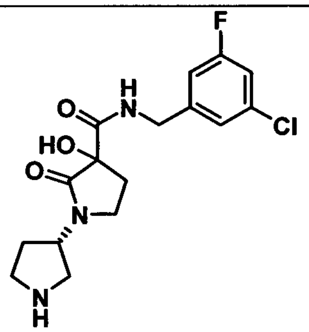 | 9.29 (s, 1H), 8.63 (q, $J$ = 6.1 Hz, 1H), 7.27 (dt, $J$ = 8.8, 2.1 Hz, 1H), 7.21 (s, 1H), 7.10 (d, $J$ = 9.7 Hz, 1H), 6.59 (s, 1H), 4.66 – 4.51 (m, 1H), 4.37 (dd, $J$ = 15.7, 6.7 Hz, 1H), 4.24 (dd, $J$ = 15.7, 6.0 Hz, 1H), 4.10 (br. | 1.40 [356.1] |

| | | | |
|---|---|---|---|
| | (S)-3-Hydroxy-2-oxo-[1,3']bipyrrolidinyl-3-carbonsäure-3-chlor-5-fluor-benzylamid | s, 1H), 3.53 – 3.37 (m, 3H), 3.25 – 3.11 (m, 4H), 2.49 – 2.41 (m, 1H), 2.12 (m, 1H), 2.07 – 1.95 (m, 2H). | |
| "A234" | 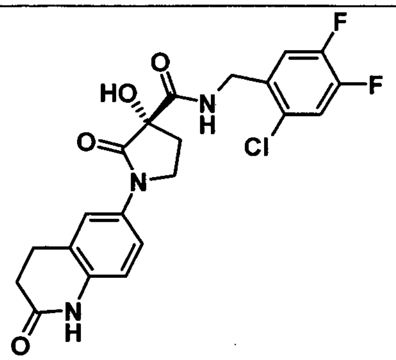 (S)-3-Hydroxy-2-oxo-1-(2-oxo-1,2,3,4-tetrahydro-chinolin-6-yl)-pyrrolidin-3-carbonsäure-2-chlor-4,5-difluor-benzylamid | ** 10.07 (s, 1H), 8.70 (t, *J* = 6.3 Hz, 1H), 7.70 (dd, *J* = 10.3, 7.3 Hz, 1H), 7.48 (d, *J* = 2.1 Hz, 1H), 7.44 (dd, *J* = 8.6, 2.4 Hz, 1H), 7.40 (dd, *J* = 11.7, 8.6 Hz, 1H), 6.87 (d, *J* = 8.6 Hz, 1H), 6.77 (s, 1H), 4.40 (dd, *J* = 16.2, 6.7 Hz, 1H), 4.22 (dd, *J* = 16.2, 5.8 Hz, 1H), 3.88 – 3.72 (m, 2H), 2.88 (t, *J* = 7.5 Hz, 2H), 2.62 (ddd, *J* = 12.4, 7.0, 5.0 Hz, 1H), 2.44 (dd, *J* = 8.3, 6.8 Hz, 2H), 2.12 (dt, *J* = 13.0, 7.4 Hz, 1H). | 1.93 [450.0] |
| "A235" | 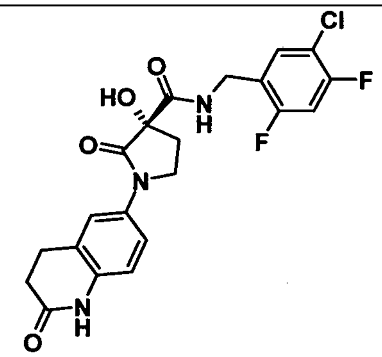 (S)-3-Hydroxy-2-oxo-1-(2-oxo-1,2,3,4-tetrahydro-chinolin-6-yl)-pyrrolidin-3-carbonsäure-5-chlor-2,4-difluor-benzylamid | ** 10.06 (s, 1H), 8.62 (t, *J* = 6.2 Hz, 1H), 7.54 (t, *J* = 8.0 Hz, 1H), 7.50 – 7.45 (m, 2H), 7.43 (dd, *J* = 8.6, 2.4 Hz, 1H), 6.86 (d, *J* = 8.6 Hz, 1H), 6.70 (s, 1H), 4.37 (dd, *J* = 15.6, 6.5 Hz, 1H), 4.25 (dd, *J* = 15.6, 5.8 Hz, 1H), 3.87 – 3.74 (m, 2H), 2.88 (t, *J* = 7.5 Hz, 2H), 2.57 (ddd, *J* = 11.8, 7.0, 4.7 Hz, 1H), 2.44 (dd, *J* = 8.3, 6.8 Hz, 2H), 2.10 (dt, *J* = 12.9, 7.6 Hz, 1H). | 1.92 [450.0] |

| | | | |
|---|---|---|---|
| "A236" | 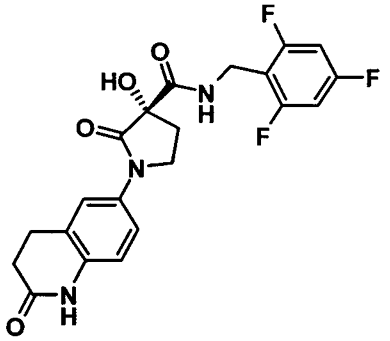<br>(S)-3-Hydroxy-2-oxo-1-(2-oxo-1,2,3,4-tetrahydro-chinolin-6-yl)-pyrrolidin-3-carbonsäure-2,4,6-trifluor-benzylamid | ** 10.06 (s, 1H), 8.20 (t, *J* = 5.7 Hz, 1H), 7.47 (d, *J* = 2.1 Hz, 1H), 7.41 (dd, *J* = 8.6, 2.4 Hz, 1H), 7.20 – 7.09 (m, 2H), 6.85 (d, *J* = 8.6 Hz, 1H), 6.55 (s, 1H), 4.35 (dd, *J* = 14.5, 5.8 Hz, 1H), 4.30 (dd, *J* = 14.5, 5.6 Hz, 1H), 3.88 – 3.67 (m, 2H), 2.88 (t, *J* = 7.5 Hz, 2H), 2.47 – 2.38 (m, 2H), 2.05 (dt, *J* = 12.8, 8.4 Hz, 1H). | 1.78 [434.1] |
| "A237" | 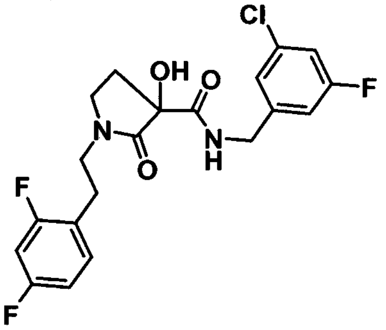<br>1-[2-(2,4-Difluor-phenyl)-ethyl]-3-hydroxy-2-oxo-pyrrolidin-3-carbonsäure-3-chlor-5-fluor-benzylamid | | 2.31 [427.0] |
| "A238" | 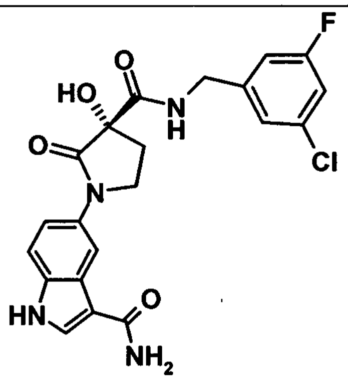<br>5-[(S)-3-(3-Chlor-5-fluor-benzylcarbamoyl)-3-hydroxy-2-oxo-pyrrolidin-1-yl]-1H-indol-3-carboxamid | | 1.90 [445.0] |

| | | | |
|---|---|---|---|
| "A239" | 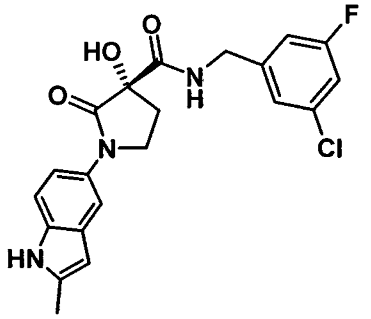 (S)-3-Hydroxy-1-(2-methyl-1H-indol-5-yl)-2-oxo-pyrrolidin-3-carbonsäure-3-chlor-5-fluor-benzylamid | | 2.19 [416.0] |
| "A240" | 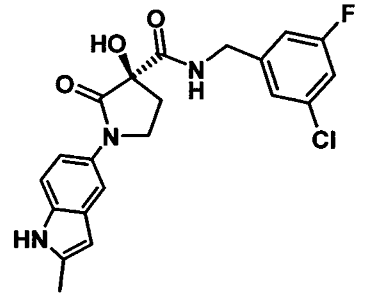 (R)-3-Hydroxy-1-(2-methyl-1H-indol-5-yl)-2-oxo-pyrrolidin-3-carbonsäure-3-chlor-5-fluor-benzylamid | | 2.19 [416.1] |
| "A241" | 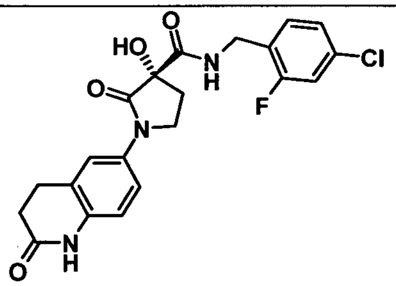 (S)-3-Hydroxy-2-oxo-1-(2-oxo-1,2,3,4-tetrahydro-chinolin-6-yl)-pyrrolidin-3-carbonsäure-4-chlor-2-fluor-benzylamid | ** 10.06 (s, 1H), 8.54 (t, *J* = 6.2 Hz, 1H), 7.48 (d, *J* = 2.2 Hz, 1H), 7.43 (dd, *J* = 8.6, 2.4 Hz, 1H), 7.39 – 7.34 (m, 2H), 7.25 (dd, *J* = 8.3, 1.9 Hz, 1H), 6.86 (d, *J* = 8.6 Hz, 1H), 6.66 (s, 1H), 4.35 (dd, *J* = 15.6, 6.4 Hz, 1H), 4.29 (dd, *J* = 15.6, 6.1 Hz, 1H), 3.88 – 3.75 (m, 2H), 2.88 (t, *J* = 7.5 Hz, 2H), 2.56 (ddd, *J* = 12.6, 6.8, 4.8 Hz, 1H), 2.44 (dd, *J* = 8.3, 6.8 Hz, | 1.91 [432.0] |

| | | | |
|---|---|---|---|
| | | 2H), 2.10 (dt, *J* = 12.9, 7.7 Hz, 1H). | |
| "A242" | 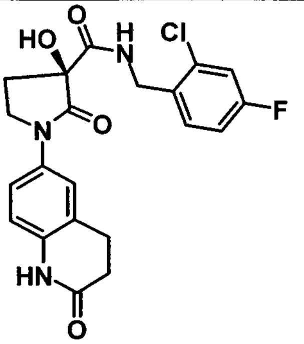 (S)-3-Hydroxy-2-oxo-1-(2-oxo-1,2,3,4-tetrahydro-chinolin-6-yl)-pyrrolidin-3-carbonsäure-2-chlor-4-fluor-benzylamid | ** 10.07 (s, 1H), 8.57 (t, *J* = 6.3 Hz, 1H), 7.48 (d, *J* = 2.1 Hz, 1H), 7.46 – 7.37 (m, 3H), 7.20 (td, *J* = 8.6, 2.6 Hz, 1H), 6.86 (d, *J* = 8.6 Hz, 1H), 6.72 (s, 1H), 4.38 (dd, *J* = 15.9, 6.5 Hz, 1H), 4.29 (dd, *J* = 15.9, 6.0 Hz, 1H), 3.90 – 3.63 (m, 2H), 2.88 (t, *J* = 7.5 Hz, 2H), 2.67 – 2.55 (m, 1H), 2.44 (dd, *J* = 8.3, 6.8 Hz, 2H), 2.19 – 2.03 (m, 1H). | 1.88 [432.1] |
| "A243" | 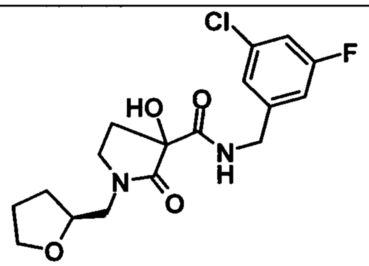 3-Hydroxy-2-oxo-1-[(S)-1-(tetrahydro-furan-2-yl)methyl]-pyrrolidin-3-carbonsäure-3-chlor-5-fluor-benzylamid | - | 1.89 [371.1] |
| "A244" | 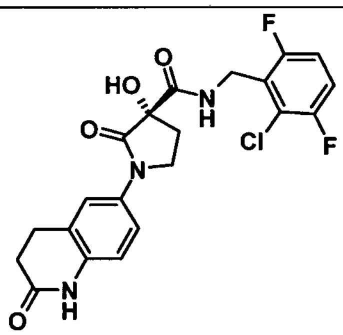 (S)-3-Hydroxy-2-oxo-1-(2-oxo-1,2,3,4-tetrahydro-chinolin-6-yl)- | ** 10.06 (s, 1H), 8.14 (t, *J* = 5.5 Hz, 1H), 7.47 (d, *J* = 2.0 Hz, 1H), 7.42 (ddd, *J* = 8.8, 6.8, 3.6 Hz, 2H), 7.28 (td, *J* = 9.2, 4.3 Hz, 1H), 6.85 (d, *J* = 8.6 Hz, 1H), 6.56 (s, 1H), 4.51 (dd, *J* = 14.3, 4.8 Hz, 1H), 4.44 (dd, *J* = 14.6, 5.1 Hz, 1H), 3.85 – 3.69 (m, 2H), 2.88 (t, *J* = 7.5 | 1.85 [450.0] |

| | | | |
|---|---|---|---|
| | pyrrolidin-3-carbonsäure-2-chlor-3,6-difluor-benzylamid | Hz, 2H), 2.48 – 2.37 (m, 2H), 2.06 (dt, *J* = 12.8, 8.4 Hz, 1H). | |
| "A245" | 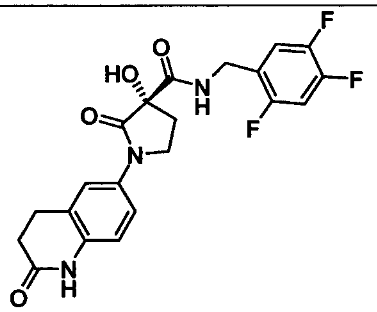 (S)-3-Hydroxy-2-oxo-1-(2-oxo-1,2,3,4-tetrahydro-chinolin-6-yl)-pyrrolidin-3-carbonsäure-2,4,5-trifluor-benzylamid | ** 10.08 (s, 1H), 8.64 (t, *J* = 6.3 Hz, 1H), 7.55 – 7.49 (m, 1H), 7.49 (s, 1H), 7.44 (dd, *J* = 8.6, 2.4 Hz, 1H), 7.39 (ddd, *J* = 11.2, 9.0, 7.2 Hz, 1H), 6.88 (d, *J* = 8.6 Hz, 1H), 6.72 (s, 1H), 4.38 (dd, *J* = 15.7, 6.5 Hz, 1H), 4.26 (dd, *J* = 15.7, 5.8 Hz, 1H), 3.89 – 3.70 (m, 2H), 2.89 (t, *J* = 7.5 Hz, 2H), 2.67 – 2.56 (m, 1H), 2.45 (dd, *J* = 8.3, 6.8 Hz, 2H), 2.12 (dt, *J* = 13.0, 7.5 Hz, 1H). | 1.83 [434.1] |
| "A246" | 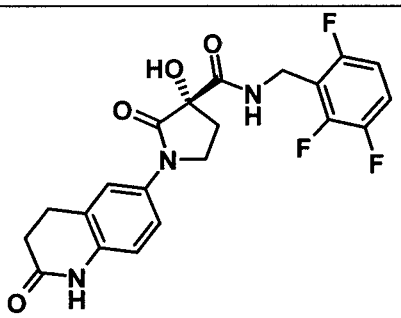 (S)-3-Hydroxy-2-oxo-1-(2-oxo-1,2,3,4-tetrahydro-chinolin-6-yl)-pyrrolidin-3-carbonsäure-2,3,6-trifluor-benzylamid | ** 10.07 (s, 1H), 8.32 (t, *J* = 5.7 Hz, 1H), 7.48 (d, *J* = 2.2 Hz, 1H), 7.47 – 7.40 (m, 2H), 7.11 (tdd, *J* = 9.2, 3.7, 2.1 Hz, 1H), 6.86 (d, *J* = 8.6 Hz, 1H), 6.57 (s, 1H), 4.44 (dd, *J* = 14.5, 5.8 Hz, 1H), 4.38 (dd, *J* = 14.5, 5.6 Hz, 1H), 3.88 – 3.68 (m, 2H), 2.89 (t, *J* = 7.5 Hz, 2H), 2.46 (td, *J* = 8.8, 5.6 Hz, 2H), 2.15 – 1.97 (m, 1H). | 1.77 [434.1] |
| "A247" | 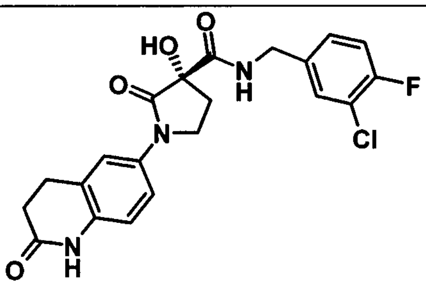 (S)-3-Hydroxy-2-oxo-1-(2-oxo-1,2,3,4-tetrahydro-chinolin-6-yl)- | ** 10.06 (s, 1H), 8.61 (t, *J* = 6.4 Hz, 1H), 7.48 (d, *J* = 2.4 Hz, 1H), 7.48 – 7.45 (m, 1H), 7.43 (dd, *J* = 8.6, 2.4 Hz, 1H), 7.36 – 7.31 (m, 1H), 7.29 – 7.25 (m, 1H), 6.86 (d, *J* = 8.6 Hz, 1H), 6.64 (s, 1H), 4.39 – 4.28 (m, | 1.89 [432.1] |

| | | | |
|---|---|---|---|
| | pyrrolidin-3-carbonsäure-3-chlor-4-fluor-benzylamid | 1H), 4.23 (dd, $J$ = 15.2, 6.1 Hz, 1H), 3.84 – 3.72 (m, 2H), 2.88 (t, $J$ = 7.5 Hz, 2H), 2.60 – 2.51 (m, 1H), 2.44 (dd, $J$ = 8.3, 6.8 Hz, 2H), 2.09 (dt, $J$ = 12.9, 7.7 Hz, 1H). | |
| "A248" | 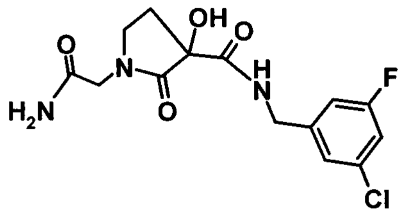 1-Carbamoylmethyl-3-hydroxy-2-oxo-pyrrolidin-3-carbonsäure-3-chlor-5-fluor-benzylamid | 8.71 (t, $J$ = 6.3 Hz, 1H), 7.33 (s, 1H), 7.26 (dt, $J$ = 8.8, 2.1 Hz, 1H), 7.20 (d, $J$ = 11.4 Hz, 2H), 7.08 (d, $J$ = 9.1 Hz, 1H), 6.57 (s, 1H), 4.37 (dd, $J$ = 15.7, 6.7 Hz, 1H), 4.25 (dd, $J$ = 15.7, 6.0 Hz, 1H), 3.89 (d, $J$ = 16.8 Hz, 1H), 3.74 (d, $J$ = 16.8 Hz, 1H), 3.42 (ddd, $J$ = 14.9, 10.9, 5.4 Hz, 3H), 2.41 (ddd, $J$ = 13.0, 7.2, 3.2 Hz, 1H), 2.05 (dt, $J$ = 13.0, 8.2 Hz, 1H). | 1.66 [344.0] |
| "A249" | 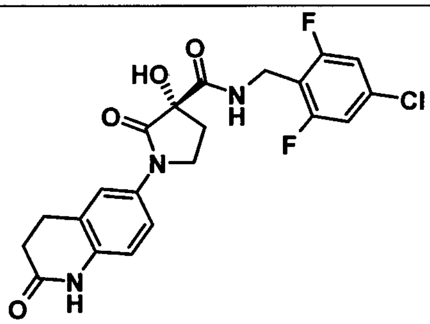 (S)-3-Hydroxy-2-oxo-1-(2-oxo-1,2,3,4-tetrahydro-chinolin-6-yl)-pyrrolidin-3-carbonsäure-4-chlor-2,6-difluor-benzylamid | ** 10.06 (s, 1H), 8.33 – 8.16 (m, 1H), 7.47 (d, $J$ = 2.1 Hz, 1H), 7.41 (dd, $J$ = 8.6, 2.4 Hz, 1H), 7.37 – 7.25 (m, 2H), 6.85 (d, $J$ = 8.6 Hz, 1H), 6.55 (s, 1H), 4.36 (dd, $J$ = 14.5, 5.8 Hz, 1H), 4.31 (dd, $J$ = 14.5, 5.5 Hz, 1H), 3.85 – 3.70 (m, 2H), 2.88 (t, $J$ = 7.5 Hz, 2H), 2.45 (ddd, $J$ = 15.1, 7.1, 4.4 Hz, 3H), 2.05 (dt, $J$ = 12.9, 8.4 Hz, 1H). | 1.92 [450.0] |

| | | | |
|---|---|---|---|
| "A250" | 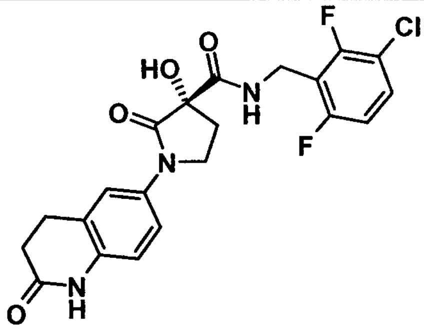 (S)-3-Hydroxy-2-oxo-1-(2-oxo-1,2,3,4-tetrahydro-chinolin-6-yl)-pyrrolidin-3-carbonsäure-3-chlor-2,6-difluor-benzylamid | ** 10.06 (s, 1H), 8.31 (t, *J* = 5.7 Hz, 1H), 7.57 (td, *J* = 8.7, 5.7 Hz, 1H), 7.47 (d, *J* = 2.2 Hz, 1H), 7.41 (dd, *J* = 8.6, 2.4 Hz, 1H), 7.14 (td, *J* = 9.0, 1.5 Hz, 1H), 6.85 (d, *J* = 8.6 Hz, 1H), 6.55 (s, 1H), 4.42 (dd, *J* = 14.5, 5.8 Hz, 1H), 4.37 (dd, *J* = 14.5, 5.6 Hz, 1H), 3.86 – 3.68 (m, 2H), 2.88 (t, *J* = 7.5 Hz, 2H), 2.45 (td, *J* = 8.9, 5.6 Hz, 2H), 2.16 – 1.97 (m, 1H). | 1.88 [450.1] |
| "A251" | 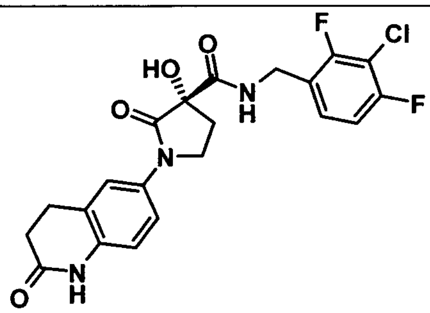 (S)-3-Hydroxy-2-oxo-1-(2-oxo-1,2,3,4-tetrahydro-chinolin-6-yl)-pyrrolidin-3-carbonsäure-3-chlor-2,4-difluor-benzylamid | ** 10.06 (s, 1H), 8.60 (t, *J* = 6.2 Hz, 1H), 7.48 (d, *J* = 2.2 Hz, 1H), 7.43 (dd, *J* = 8.6, 2.4 Hz, 1H), 7.40 – 7.34 (m, 1H), 7.29 (td, *J* = 8.8, 1.5 Hz, 1H), 6.86 (d, *J* = 8.6 Hz, 1H), 6.67 (s, 1H), 4.38 (dd, *J* = 15.4, 6.3 Hz, 1H), 4.31 (dd, *J* = 15.4, 6.0 Hz, 1H), 3.88 – 3.69 (m, 2H), 2.88 (t, *J* = 7.5 Hz, 2H), 2.56 (ddd, *J* = 12.6, 6.8, 4.8 Hz, 1H), 2.44 (dd, *J* = 8.3, 6.8 Hz, 2H), 2.10 (dt, *J* = 12.9, 7.6 Hz, 1H). | 1.95 [450.0] |
| "A252" | 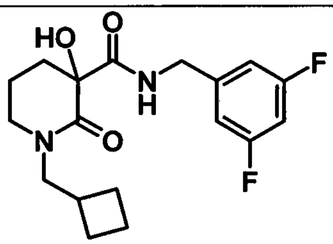 1-Cyclobutylmethyl-3-hydroxy-2-oxo-piperidin-3-carbonsäure-3,5-difluor-benzylamid | 8.49 (t, *J* = 6.3 Hz, 1H), 7.12 – 6.93 (m, 3H), 6.14 (s, 1H), 4.39 (dd, *J* = 16.1, 7.1 Hz, 1H), 4.20 (dd, *J* = 16.1, 5.7 Hz, 1H), 3.41 (dd, *J* = 13.1, 7.4 Hz, 1H), 3.25 (dd, *J* = 13.1, 7.3 Hz, 2H), 2.54 (m, 1H), 2.12 (m, 1H), 1.99 – 1.90 (m, 2H), 1.85 – | 3.93 [353.2] |

| | | | |
|---|---|---|---|
| | | 1.75 (m, 4H), 1.75 – 1.62 (m, 2H). | |
| "A253" | 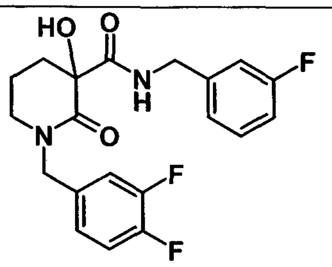 1-(3,4-Difluor-benzyl)-3-hydroxy-2-oxo-piperidin-3-carbonsäure-3-fluor-benzylamid | 8.52 (t, *J* = 6.3 Hz, 1H), 7.35 (m, 3H), 7.16 – 7.09 (m, 3H), 7.07 – 6.98 (m, 1H), 6.37 (s, 1H), 4.61 (d, *J* = 15.3 Hz, 1H), 4.41 (d, *J* = 15.3 Hz, 1H), 4.39 (dd, *J* = 15.6, 6.9 Hz,1H), 4.26 (dd, *J* = 15.6, 5.9 Hz, 1H), 3.25 (m, 2H), 2.24 – 2.12 (m, 1H), 1.86 (m, 3H). | 4.23 [393] |
| "A254" | 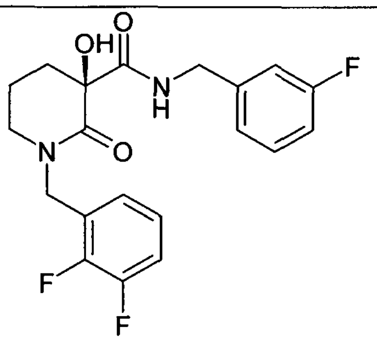 (S)-1-(2,3-Difluor-benzyl)-3-hydroxy-2-oxo-piperidin-3-carbonsäure-3-fluor-benzylamid | 8.51 (t, *J* = 6.3 Hz, 1H), 7.39 – 7.27 (m, 2H), 7.15 (m, 4H), 7.07 – 6.97 (m, 1H), 6.35 (s, 1H), 4.69 (d, *J* = 15.7 Hz, 1H), 4.52 (d, *J* = 15.7 Hz, 1H), 4.38 (dd, *J* = 15.6, 6.8 Hz, 1H), 4.26 (dd, *J* = 15.6, 5.9 Hz, 1H), 2.18 (m, 1H), 1.88 (m, 3H). | 4.16 [393] |
| "A255" | 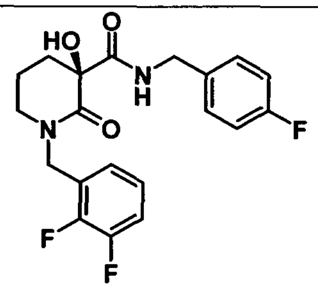 (S)-1-(2,3-Difluor-benzyl)-3-hydroxy-2-oxo-piperidin-3-carbonsäure-4-fluor-benzylamid | 8.44 (t, *J* = 6.3 Hz, 1H), 7.38 – 7.28 (m, 3H), 7.22 – 7.08 (m, 4H), 6.31 (s, 1H), 4.68 (d, *J* = 15.7 Hz, 1H), 4.51 (d, *J* = 15.7 Hz, 1H), 4.32 (dd, *J* = 15.2, 6.6 Hz, 1H), 4.24 (dd, *J* = 15.2, 6.0 Hz, 1H), 2.22 – 2.15 (m, 1H), 1.85 (m, 3H), 1.23 (m, 2H), 0.84 (m, 1H). | 4.13 [393] |

| | | | |
|---|---|---|---|
| "A256" | 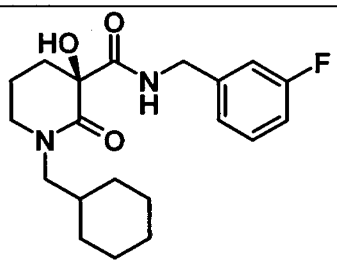 (S)-1-Cyclohexylmethyl-3-hydroxy-2-oxo-piperidin-3-carbonsäure-3-fluor-benzylamid | 8.40-8.39 (m, 1H), 7.31 (t, J = 7.48 Hz, 1H), 7.11 (m, 2H), 7.05-7.00 (m, 1H), 6.08 (s, 1H), 4.39-4.33 (m, 1H), 4.26-4.20 (m, 1H), 3.28-3.22 (m, 3H), 3.04-3.01 (m, 1H), 2.17 (m, 1H), 1.85-1.77 (m, 3H), 1.63-1.62 (m, 6H), 1.23-1.13 (m, 3H), 0.89-0.83 (m, 2H). | 4.44 [363.3] |
| "A257" | 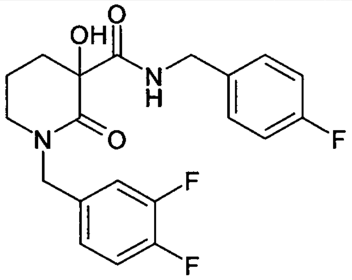 1-(3,4-Difluor-benzyl)-3-hydroxy-2-oxo-piperidin-3-carbonsäure-4-fluor-benzylamid | 8.45 (t, *J* = 6.4 Hz, 1H), 7.40 (dt, *J* = 10.8, 8.5 Hz, 1H), 7.31 (dt, *J* = 5.5, 4.5 Hz, 3H), 7.11 (dd, *J* = 12.3, 5.5 Hz, 3H), 6.32 (s, 1H), 4.61 (d, *J* = 15.3 Hz, 1H), 4.40 (d, *J* = 15.3 Hz, 1H), 4.33 (dd, *J* = 15.0, 6.7 Hz, 1H), 4.24 (dd, *J* = 15.2, 6.0 Hz, 1H), 3.25 (t, *J* = 8.7 Hz, 2H), 2.15 (d, *J* = 8.5 Hz, 1H), 1.84 (d, *J* = 3.2 Hz, 3H). | 4.19 [393.2] |
| "A258" | 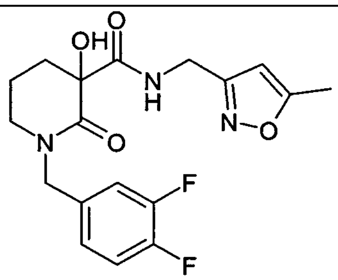 1-(3,4-Difluor-benzyl)-3-hydroxy-2-oxo-piperidin-3-carbonsäure-(5-methyl-isoxazol-3-ylmethyl)-amid | 8.49 (t, *J* = 6.1 Hz, 1H), 7.40 (dt, *J* = 10.7, 8.5 Hz, 1H), 7.35 – 7.28 (m, 1H), 7.13 (s, 1H), 6.32 (s, 1H), 6.12 (s, 1H), 4.61 (d, *J* = 15.3 Hz, 1H), 4.39 (d, *J* = 15.3 Hz, 1H), 4.28 (m, 2H), 3.24 (d, *J* = 5.2 Hz, 2H), 2.35 (s, 3H), 2.13 (m, 1H), 1.90 – 1.78 (m, 3H). | 3.45 [380] |

| | | | |
|---|---|---|---|
| "A259" | 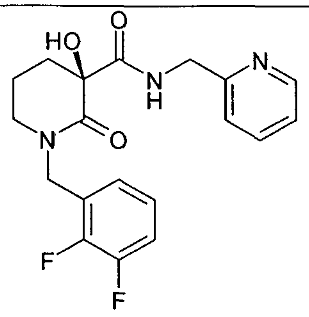 (S)-1-(2,3-Difluor-benzyl)-3-hydroxy-2-oxo-piperidin-3-carbonsäure-(pyridin-2-ylmethyl)-amid | 8.53 (t, *J* = 5.9 Hz, 1H), 8.49 (d, *J* = 4.1 Hz, 1H), 7.73 (td, *J* = 7.7, 1.8 Hz, 1H), 7.40 – 7.29 (m, 2H), 7.24 (dd, *J* = 7.0, 5.3 Hz, 1H), 7.21 – 7.11 (m, 2H), 6.40 (s, 1H), 4.69 (d, *J* = 15.7 Hz, 1H), 4.52 (d, *J* = 15.7 Hz, 1H), 4.44 (dd, *J* = 16.4, 6.2 Hz, 1H), 4.35 (dd, *J* = 16.4, 5.8 Hz, 1H), 3.30 (m, 1H), 2.52 (m, 1H), 2.21 (m, 1H), 1.89 (m, 3H). | 2.57 [376] |
| "A260" | 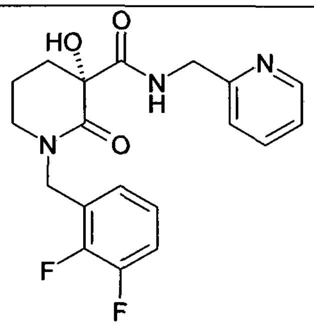 (R)-1-(2,3-Difluor-benzyl)-3-hydroxy-2-oxo-piperidin-3-carbonsäure-(pyridin-2-ylmethyl)-amid | 8.53 (t, *J* = 6.0 Hz, 1H), 8.49 (ddd, *J* = 4.8, 1.7, 0.9 Hz, 1H), 7.73 (td, *J* = 7.7, 1.8 Hz, 1H), 7.40 – 7.29 (m, 2H), 7.24 (dd, *J* = 7.0, 5.3 Hz, 1H), 7.22 – 7.10 (m, 2H), 6.40 (s, 1H), 4.69 (d, *J* = 15.6 Hz, 1H), 4.52 (d, *J* = 15.5 Hz, 1H), 4.44 (dd, *J* = 16.4, 6.2 Hz, 1H), 4.35 (dd, *J* = 16.4, 5.7 Hz, 1H), 3.30 (m, 1H), 2.52 (m, 1H), 2.29 – 2.16 (m, 1H), 1.90 (m, 3H). | 2.57 [376.2] |
| "A261" | 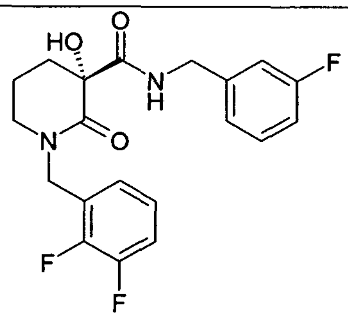 (R)-1-(2,3-Difluor-benzyl)-3-hydroxy-2-oxo-piperidin-3-carbonsäure-3-fluor-benzylamid | 8.51 (t, *J* = 6.2 Hz, 1H), 7.33 (dt, *J* = 8.1, 6.3 Hz, 2H), 7.15 (ddd, *J* = 24.5, 12.2, 8.4 Hz, 4H), 7.03 (t, *J* = 9.1 Hz, 1H), 6.35 (s, 1H), 4.69 (d, *J* = 15.6 Hz, 1H), 4.52 (d, *J* = 15.6 Hz, 1H), 4.38 (dd, *J* = 15.7, 6.8 Hz, 1H), 4.25 (dd, *J* = 15.7, 6.0 Hz, 1H), 3.38 – 3.33 (m, 1H), 2.52 | 4.16 [393] |

| | | | |
|---|---|---|---|
| | | (m, 1H), 2.19 (m, 1H), 1.88 (m, 3H). | |
| "A262" | 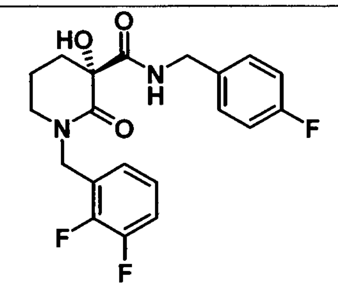 (R)-1-(2,3-Difluor-benzyl)-3-hydroxy-2-oxo-piperidin-3-carbonsäure-4-fluor-benzylamid | 8.45 (d, *J* = 6.3 Hz, 1H), 7.38 – 7.28 (m, 3H), 7.14 (dt, *J* = 17.8, 7.0 Hz, 4H), 6.31 (s, 1H), 4.68 (d, *J* = 15.7 Hz, 1H), 4.51 (d, *J* = 15.7 Hz, 1H), 4.32 (dd, *J* = 15.1, 6.6 Hz, 1H), 4.24 (dd, *J* = 15.1, 6.1 Hz, 1H), 2.52 (m, 2H), 2.23 – 2.11 (m, 1H), 1.87 (m, 3H). | 4.13 [393.2] |
| "A263" | 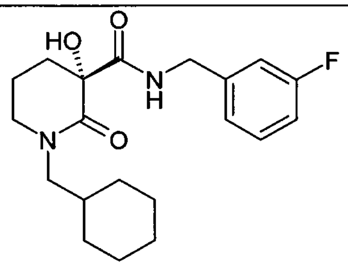 (R)-1-Cyclohexylmethyl-3-hydroxy-2-oxo-piperidin-3-carbonsäure-3-fluor-benzylamid | δ 8.40-8.39 (m, 1H), 7.31 (t, J = 7.48 Hz, 1H), 7.11-7.11 (m, 2H), 7.05-7.00 (m, 1H), 6.08 (s, 1H), 4.39-4.33 (m, 1H), 4.26-4.20 (m, 1H), 3.28-3.22 (m, 3H), 3.04-3.01 (m, 1H), 2.17-2.17 (m, 1H), 1.85-1.77 (m, 3H), 1.63-1.62 (m, 6H), 1.23-1.13 (m, 3H), 0.89-0.83 (m, 2H). | 4.44 [363.3] |
| "A264" | 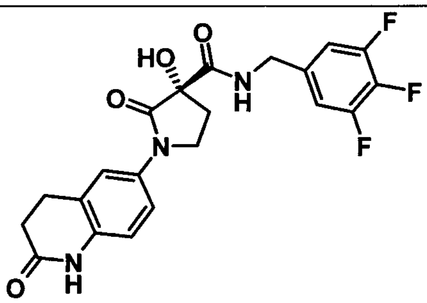 (S)-3-Hydroxy-2-oxo-1-(2-oxo-1,2,3,4-tetrahydro-chinolin-6-yl)-pyrrolidin-3-carbonsäure-3,4,5-trifluor-benzylamid | ** 10.07 (s, 1H), 8.69 (t, *J* = 6.4 Hz, 1H), 7.48 (d, *J* = 2.1 Hz, 1H), 7.43 (dd, *J* = 8.6, 2.4 Hz, 1H), 7.28 – 7.11 (m, 2H), 6.87 (d, *J* = 8.6 Hz, 1H), 6.70 (s, 1H), 4.37 (dd, *J* = 15.7, 6.7 Hz, 1H), 4.22 (dd, *J* = 15.7, 6.0 Hz, 1H), 3.92 – 3.69 (m, 2H), 2.88 (t, *J* = 7.5 Hz, 2H), 2.62 – 2.55 (m, 1H), 2.44 (dd, *J* = 8.3, 6.8 Hz, 2H), 2.10 (dt, *J* = 13.0, 7.5 Hz, 1H). | 1.87 [434.1] |

| | | | |
|---|---|---|---|
| "A265" | 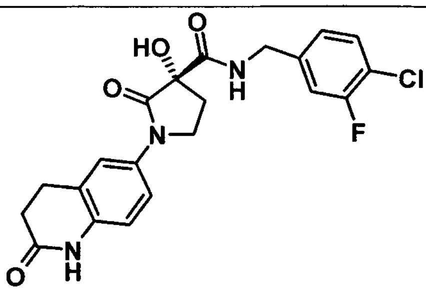 (S)-3-Hydroxy-2-oxo-1-(2-oxo-1,2,3,4-tetrahydro-chinolin-6-yl)-pyrrolidin-3-carbonsäure-4-chlor-3-fluor-benzylamid | ** 10.07 (s, 1H), 8.64 (t, *J* = 6.4 Hz, 1H), 7.51 (t, *J* = 8.0 Hz, 1H), 7.48 (d, *J* = 2.2 Hz, 1H), 7.43 (dd, *J* = 8.6, 2.4 Hz, 1H), 7.29 (dd, *J* = 10.5, 1.8 Hz, 1H), 7.14 (dd, *J* = 8.3, 1.3 Hz, 1H), 6.86 (d, *J* = 8.6 Hz, 1H), 6.67 (s, 1H), 4.36 (dd, *J* = 15.5, 6.7 Hz, 1H), 4.25 (dd, *J* = 15.6, 6.1 Hz, 1H), 3.85 – 3.69 (m, 2H), 2.88 (t, *J* = 7.5 Hz, 2H), 2.57 (dt, *J* = 11.8, 5.6 Hz, 1H), 2.44 (dd, *J* = 8.3, 6.8 Hz, 2H), 2.15 – 1.99 (m, 1H). | 1.90 [432.0] |
| "A266" | 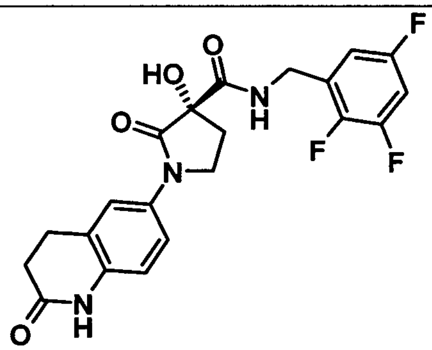 (S)-3-Hydroxy-2-oxo-1-(2-oxo-1,2,3,4-tetrahydro-chinolin-6-yl)-pyrrolidin-3-carbonsäure-2,3,5-trifluor-benzylamid | ** 10.07 (s, 1H), 8.68 (t, *J* = 6.2 Hz, 1H), 7.47 (t, *J* = 7.0 Hz, 1H), 7.43 (dd, *J* = 8.6, 2.3 Hz, 1H), 7.41 – 7.35 (m, 1H), 7.10 – 6.97 (m, 1H), 6.86 (d, *J* = 8.6 Hz, 1H), 6.72 (s, 1H), 4.45 (dd, *J* = 15.9, 6.6 Hz, 1H), 4.32 (dd, *J* = 15.9, 5.8 Hz, 1H), 3.92 – 3.67 (m, 2H), 2.88 (t, *J* = 7.5 Hz, 2H), 2.62 – 2.53 (m, 1H), 2.46 – 2.37 (m, 2H), 2.11 (dt, *J* = 13.0, 7.5 Hz, 1H). | 1.83 [434.1] |
| "A267" | 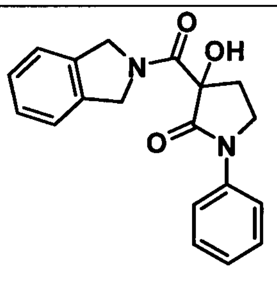 | $^1$H (300 MHz, DMSO-$d_6$/TFA-$d_1$) 7.75 (2 H, m), 7.30 (7 H, m), 4.81 (4 H, m), 3.88 (2 H, m), 2.81 (1 H, ddd, J 12.8, 6.5, 2.8), 2.25 (1 H, m). | 2.04 [323.0] |

| | | | |
|---|---|---|---|
| | 3-(1,3-Dihydro-isoindol-2-carbonyl)-3-hydroxy-1-phenyl-pyrrolidin-2-on | | |
| "A268" | 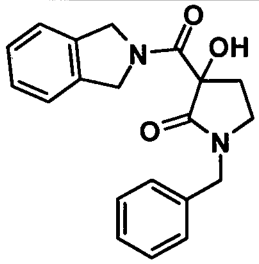 1-Benzyl-3-(1,3-dihydro-isoindol-2-carbonyl)-3-hydroxy-pyrrolidin-2-on | * 7.33 (9 H, m), 4.74 (6 H, m), 3.48 (2 H, m), 2.75 (1 H, m), 2.12 (1 H, m). | 2.04 [337.0] |
| "A269" | 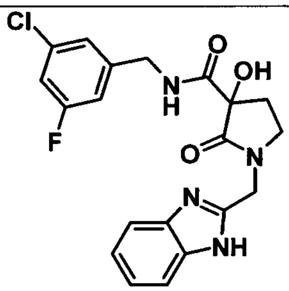 1-(1H-Benzimidazol-2-ylmethyl)-3-hydroxy-2-oxo-pyrrolidin-3-carbonsäure-3-chlor-5-fluor-benzylamid | * 7.83 (2 H, dd, J 6.2, 3.1), 7.58 (2 H, dd, J 6.2, 3.2), 7.23 (1 H, s), 7.13 (2 H, dt, J 16.1, 4.9), 5.06 (2 H, s), 4.48 (1 H, d, J 15.6), 4.33 (1 H, d, J 15.6), 3.65 (2 H, m), 2.63 (1 H, m), 2.25 (1 H, m). | 1.72 [417.0] |
| "A270" | 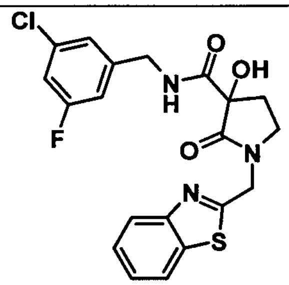 1-Benzothiazol-2-ylmethyl-3-hydroxy-2-oxo-pyrrolidin-3-carbonsäure-3-chlor-5-fluor-benzylamid | 8.65 (1 H, t, J 6.3), 8.08 (1 H, dd, J 8.0, 0.6), 7.99 (1 H, d, J 7.6), 7.52 (1 H, m), 7.45 (1 H, td, J 7.7, 1.2), 7.28 (1 H, ddd, J 12.3, 8.7, 4.9), 7.22 (1 H, s), 7.12 (1 H, dd, J 9.6, 0.7), 6.64 (1 H, s), 4.88 (2 H, s), 4.40 (1 H, dd, J 15.7, 6.7), 4.26 (1 H, dd, J 15.7, 6.0), 3.51 (2 H, m), 2.54 (1 H, m), 2.05 (1 H, dt, J 13.1, 7.0). | 2.16 [434.0] |

| | | | |
|---|---|---|---|
| "A271" | 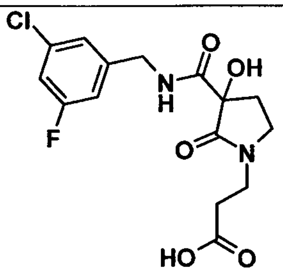 3-[3-(3-Chlor-5-fluor-benzylcarbamoyl)-3-hydroxy-2-oxo-pyrrolidin-1-yl]-propionsäure | $^1$H NMR (500 MHz, DMSO-$d_6$ / TFA-$d_1$) 7.20 (2 H, m), 7.11 (1 H, d, J 9.4), 4.41 (1 H, d, J 15.7), 4.28 (1 H, d, J 15.7), 3.48 (2 H, t, J 7.2), 3.42 (2 H, m), 2.48 (3 H, dt, J 7.3, 6.0), 2.01 (1 H, m). | 1.67 [359.0] |
| "A272" | 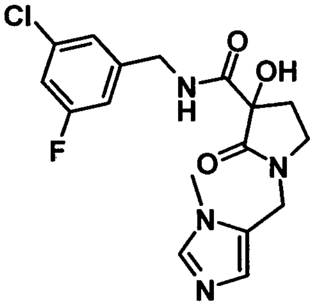 3-Hydroxy-1-(3-methyl-3H-imidazol-4-ylmethyl)-2-oxo-pyrrolidin-3-carbonsäure-3-chlor-5-fluor-benzylamid | * 9.10 (1 H, s), 7.67 (1 H, s), 7.20 (2 H, m), 7.10 (1 H, d, J 10.0), 4.83 (1 H, d, J 15.8), 4.52 (1 H, d, J 15.9), 4.44 (1 H, d, J 15.8), 4.28 (1 H, d, J 15.8), 3.78 (3 H, s), 3.33 (2 H, m), 2.49 (1 H, m), 2.10 (1 H, m). | 1.37 [381.0] |
| "A273" | 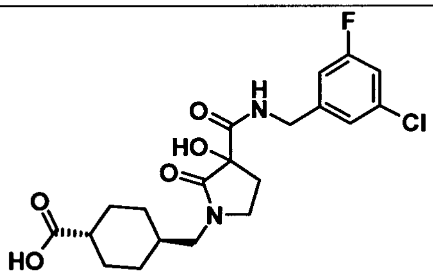 4-[3-(3-Chlor-5-fluor-benzylcarbamoyl)-3-hydroxy-2-oxo-pyrrolidin-1-ylmethyl]-cyclohexancarbonsäure | ** 11.94 (s, 1H), 8.54 (t, *J* = 6.4 Hz, 1H), 7.24 (dt, *J* = 8.7, 2.1 Hz, 1H), 7.18 (s, 1H), 7.11 – 7.06 (m, 1H), 6.41 (s, 1H), 4.37 (dd, *J* = 15.8, 6.8 Hz, 1H), 4.22 (dd, *J* = 15.8, 6.0 Hz, 1H), 3.41 – 3.30 (m, 2H), 3.12 (dd, *J* = 13.4, 7.9 Hz, 1H), 2.98 (dd, *J* = 13.4, 6.8 Hz, 1H), 2.45 (ddd, *J* = 11.6, 7.6, 3.8 Hz, 1H), 2.10 (tt, *J* = 12.0, 3.4 Hz, 1H), 1.95 (ddd, *J* = 12.9, 8.5, 6.7 Hz, 1H), 1.91 – 1.78 (m, 2H), 1.68 (d, *J* = 12.5 Hz, 2H), | 1.89 [427.1] |

| | | | |
|---|---|---|---|
| | | 1.57 (dqd, *J* = 15.2, 7.6, 3.7 Hz, 1H), 1.24 (dqd, *J* = 16.4, 13.0, 3.3 Hz, 2H), 0.90 (dh, *J* = 13.0, 3.3 Hz 2H). | |
| "A274" | 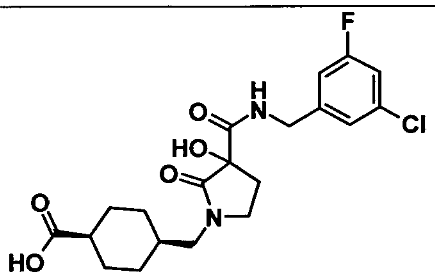 4-[3-(3-Chlor-5-fluor-benzylcarbamoyl)-3-hydroxy-2-oxo-pyrrolidin-1-ylmethyl]-cyclohexancarbonsäure | ** 12.01 (s, 1H), 8.53 (t, *J* = 6.4 Hz, 1H), 7.24 (dt, *J* = 8.7, 2.1 Hz, 1H), 7.18 (s, 1H), 7.09 – 7.04 (m, 1H), 6.40 (s, 1H), 4.36 (dd, *J* = 15.8, 6.6 Hz, 1H), 4.23 (dd, *J* = 15.8, 6.0 Hz, 1H), 3.39 – 3.29 (m, 3H), 3.19 (dd, *J* = 13.4, 8.4 Hz, 1H), 3.00 (dd, *J* = 13.4, 7.1 Hz, 1H), 2.47 – 2.37 (m, 2H), 2.00 – 1.90 (m, 1H), 1.88 – 1.76 (m, 2H), 1.73 (dt, *J* = 16.1, 6.2 Hz, 1H), 1.52 – 1.38 (m, 4H), 1.23 – 1.10 (m, 2H). | 1.98 [427.1] |
| "A275" | 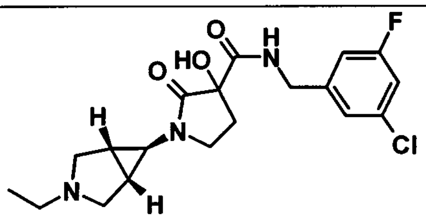 1-((1S,5R,6S)-3-Ethyl-3-aza-bicyclo[3.1.0]hex-6-yl)-3-hydroxy-2-oxo-pyrrolidin-3-carbonsäure-3-chlor-5-fluor-benzylamid | ** 8.54 (t, *J* = 6.4 Hz, 1H), 8.17 (s, 1H), 7.26 (dt, *J* = 8.8, 2.1 Hz, 1H), 7.19 (s, 1H), 7.09 (d, *J* = 9.1 Hz, 1H), 4.36 (dd, *J* = 15.8, 6.7 Hz, 1H), 4.22 (dd, *J* = 15.8, 6.0 Hz, 1H), 3.28 – 3.21 (m, 2H), 3.05 (dd, *J* = 8.9, 1.8 Hz, 2H), 2.87 (d, *J* = 14.1 Hz, 1H), 2.44 – 2.34 (m, 4H), 2.33 – 2.27 (m, 2H), 1.93 – 1.86 (m, 1H), 1.81 – 1.74 (m, 2H), 0.98 (t, *J* = 7.2 Hz, 3H). | 1.46 [396.1] |

| | | | |
|---|---|---|---|
| "A276" | 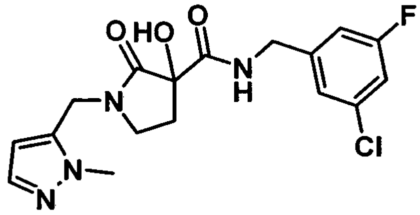 3-Hydroxy-1-(2-methyl-2H-pyrazol-3-ylmethyl)-2-oxo-pyrrolidin-3-carbonsäure-3-chlor-5-fluor-benzylamid | ** 8.63 (t, *J* = 6.4 Hz, 1H), 7.32 (d, *J* = 1.8 Hz, 1H), 7.26 (dt, *J* = 8.8, 2.1 Hz, 1H), 7.20 (s, 1H), 7.10 (dd, *J* = 9.7, 0.7 Hz, 1H), 6.56 (s, 1H), 6.22 (d, *J* = 1.8 Hz, 1H), 4.60 (d, *J* = 15.5 Hz, 1H), 4.44 (d, *J* = 15.5 Hz, 1H), 4.38 (dd, *J* = 15.7, 6.7 Hz, 1H), 4.24 (dd, *J* = 15.7, 6.0 Hz, 1H), 3.69 (s, 3H), 3.27 – 3.16 (m, 3H), 2.44 (ddd, *J* = 12.8, 7.6, 4.0 Hz, 1H), 1.97 (ddd, *J* = 13.0, 8.6, 6.7 Hz, 1H). | 1.84 [381.1] |
| "A277" | 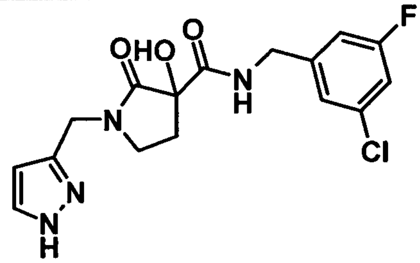 3-Hydroxy-2-oxo-1-(1H-pyrazol-3-ylmethyl)-pyrrolidin-3-carbonsäure-3-chlor-5-fluor-benzylamid | 12.66 (s, 1H), 8.60 (t, *J* = 6.0 Hz, 1H), 8.57 – 8.48 (m, 1H), 8.16 (s, 1H), 7.64 (s, 1H), 7.33 – 7.24 (m, 2H), 7.20 (d, *J* = 5.2 Hz, 1H), 7.11 (d, *J* = 9.7 Hz, 2H), 6.50 (s, 1H), 6.13 (d, *J* = 2.1 Hz, 1H), 4.40 (s, 2H), 4.34 (dd, *J* = 16.3, 6.4 Hz, 2H), 4.24 (dd, *J* = 15.8, 6.0 Hz, 1H), 3.57 (s, 2H), 2.43 (m, 1H), 2.02 – 1.90 (m, 1H). | 1.78 [367.0] |
| "A278" | 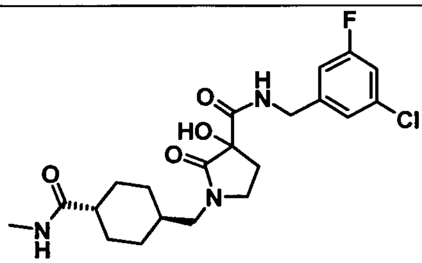 3-Hydroxy-1-(4-methyl-carbamoyl-cyclohexylmethyl)-2-oxo-pyrrolidin-3-carbonsäure-3-chlor-5-fluor-benzylamid | ** 8.53 (t, *J* = 6.4 Hz, 1H), 7.58 (q, *J* = 4.3 Hz, 1H), 7.24 (dt, *J* = 8.7, 2.1 Hz, 1H), 7.18 (s, 1H), 7.08 (d, *J* = 9.1 Hz, 1H), 6.40 (s, 1H), 4.37 (dd, *J* = 15.8, 6.8 Hz, 1H), 4.22 (dd, *J* = 15.8, 6.0 Hz, 1H), 3.39 – 3.29 (m, 2H), 3.11 (dd, *J* = 13.4, 7.9 Hz, 1H), 2.98 (dd, *J* = 13.3, 6.8 Hz, 1H), 2.53 (d, *J* = 4.6 Hz, | 1.80 [440.1] |

| | | | |
|---|---|---|---|
| | | 3H), 2.45 (ddd, J = 11.8, 7.6, 3.9 Hz, 1H), 2.05 – 1.91 (m, 2H), 1.69 (m, 4H), 1.62 – 1.49 (m, 1H), 1.29 (m, 4H), 0.96 – 0.77 (m, 2H). | |
| "A279" | 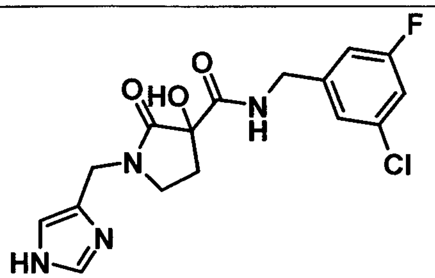 3-Hydroxy-1-(1H-imidazol-4-ylmethyl)-2-oxo-pyrrolidin-3-carbonsäure-3-chlor-5-fluor-benzylamid | 8.62 (t, J = 6.4 Hz, 1H), 8.15 (s, 1H), 7.58 (d, J = 1.1 Hz, 1H), 7.26 (dt, J = 8.8, 2.1 Hz, 1H), 7.21 (s, 1H), 7.11 (d, J = 9.7 Hz, 1H), 6.96 (s, 1H), 4.38 (dd, J = 15.8, 6.7 Hz, 1H), 4.32 (s, 2H), 4.25 (dd, J = 15.8, 6.0 Hz, 1H), 3.36 (dd, J = 14.4, 8.2 Hz, 4H), 2.43 (dt, J = 12.1, 5.7 Hz, 1H), 1.94 (dt, J = 13.0, 7.4 Hz, 1H). | 1.40 [367.0] |
| "A280" | 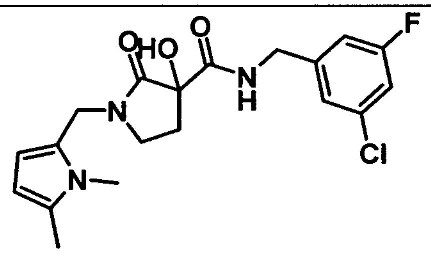 1-(1,5-Dimethyl-1H-pyrrol-2-ylmethyl)-3-hydroxy-2-oxo-pyrrolidin-3-carbonsäure-3-chlor-5-fluor-benzylamid | ** 8.59 (t, J = 6.4 Hz, 1H), 7.25 (dt, J = 8.7, 2.1 Hz, 1H), 7.20 (s, 1H), 7.12 – 7.07 (m, 1H), 6.48 (s, 1H), 5.89 (d, J = 3.4 Hz, 1H), 5.67 (dd, J = 3.3, 0.6 Hz, 1H), 4.45 (d, J = 15.1 Hz, 1H), 4.37 (dd, J = 15.8, 6.8 Hz, 1H), 4.25 (d, J = 15.1 Hz, 1H), 4.23 (dd, J = 15.8, 5.2 Hz, 1H), 3.57 (s, 1H), 3.28 (d, J = 4.5 Hz, 5H), 3.20 – 3.08 (m, 2H), 2.44 – 2.36 (m, 1H), 2.12 (s, 3H), 1.91 (ddd, J = 13.1, 8.6, 6.4 Hz, 1H). | 2.22 [394.1] |

| | | | |
|---|---|---|---|
| "A281" | 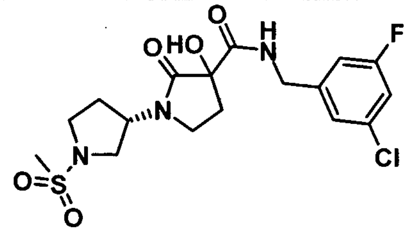 (S)-3-Hydroxy-1'-methansulfonyl-2-oxo-[1,3']bipyrrolidinyl-3-carbonsäure-3-chlor-5-fluor-benzylamid | ** 8.58 (t, *J* = 6.3 Hz, 1H), 7.25 (dt, *J* = 8.7, 2.1 Hz, 1H), 7.19 (s, 1H), 7.09 (d, *J* = 9.7 Hz, 1H), 6.50 (d, *J* = 1.1 Hz, 1H), 4.60 – 4.53 (m, 1H), 4.36 (dd, *J* = 15.7, 6.8 Hz, 1H), 4.26 – 4.20 (m, 1H), 3.48 – 3.35 (m, 4H), 3.26 (s, 2H), 3.18 (dd, *J* = 10.2, 6.0 Hz, 1H), 2.93 (d, *J* = 4.2 Hz, 3H), 2.47 – 2.40 (m, 1H), 2.15 – 1.83 (m, 6H). | 1.84 [434.0] |
| "A282" | 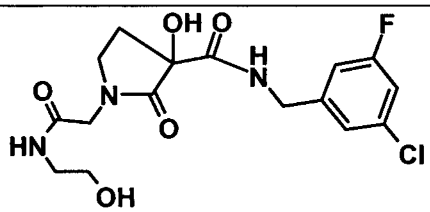 3-Hydroxy-1-[(2-hydroxy-ethylcarbamoyl)-methyl]-2-oxo-pyrrolidin-3-carbonsäure-3-chlor-5-fluor-benzylamid | 8.70 (t, *J* = 6.3 Hz, 1H), 7.93 (t, *J* = 5.5 Hz, 1H), 7.27 (dt, *J* = 8.7, 2.1 Hz, 1H), 7.20 (s, 1H), 7.09 (d, *J* = 9.6 Hz, 1H), 6.61 (br. s, 1H), 4.68 (br. s, 1H), 4.38 (dd, *J* = 15.7, 6.7 Hz, 1H), 4.24 (dd, *J* = 15.7, 6.0 Hz, 1H), 3.86 (s, 2H), 3.47 – 3.41 (m, 1H), 3.27 (br. m, 2H), 3.21 – 3.05 (m, 2H), 2.43 (ddd, *J* = 12.7, 7.5, 3.5 Hz, 1H), 2.08 – 1.97 (m, 1H). | 1.65 [388.0] |
| "A283" | 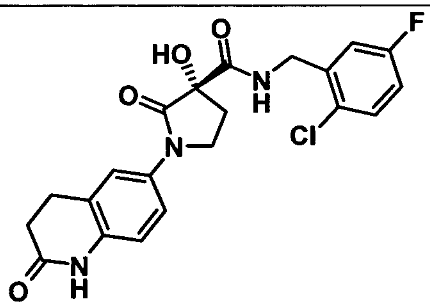 (S)-3-Hydroxy-2-oxo-1-(2-oxo-1,2,3,4-tetrahydro-chinolin-6-yl)- | ** 10.07 (s, 1H), 8.68 (t, *J* = 6.3 Hz, 1H), 7.49 – 7.45 (m, 2H), 7.44 (dd, *J* = 8.6, 2.4 Hz, 1H), 7.19 (dd, *J* = 9.8, 3.1 Hz, 1H), 7.14 (td, *J* = 8.4, 3.1 Hz, 1H), 6.87 (d, *J* = 8.6 Hz, 1H), 6.76 (s, 1H), 4.42 (dd, *J* = 16.5, 6.7 Hz, 1H), 4.26 (dd, *J* = 16.5, 5.8 Hz, 1H), 3.88 – 3.75 (m, 2H), 2.88 (t, *J* = 7.5 Hz, 2H), 2.61 (dt, *J* = 6.9, 5.8 Hz, | 1.87 [432.1] |

| | | | |
|---|---|---|---|
| | pyrrolidin-3-carbonsäure-2-chlor-5-fluor-benzylamid | 1H), 2.44 (dd, *J* = 8.3, 6.8 Hz, 2H), 2.13 (dt, *J* = 13.0, 7.5 Hz, 1H). | |
| "A284" | 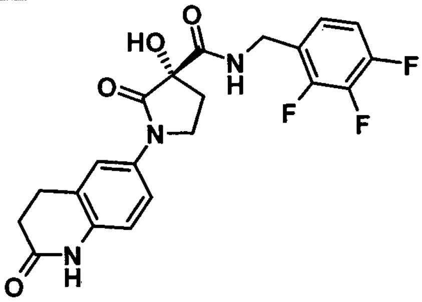 (S)-3-Hydroxy-2-oxo-1-(2-oxo-1,2,3,4-tetrahydro-chinolin-6-yl)-pyrrolidin-3-carbonsäure-2,3,4-trifluor-benzylamid | ** 10.06 (s, 1H), 8.59 (t, *J* = 6.2 Hz, 1H), 7.48 (d, *J* = 2.2 Hz, 1H), 7.43 (dd, *J* = 8.6, 2.4 Hz, 1H), 7.29 (qd, *J* = 7.5, 3.8 Hz, 1H), 7.19 (td, *J* = 8.3, 2.1 Hz, 1H), 6.86 (d, *J* = 8.6 Hz, 1H), 6.67 (s, 1H), 4.38 (dd, *J* = 15.5, 6.3 Hz, 1H), 4.31 (dd, *J* = 15.4, 6.0 Hz, 1H), 3.85 – 3.71 (m, 2H), 2.88 (t, *J* = 7.5 Hz, 2H), 2.60 – 2.52 (m, 1H), 2.44 (dd, *J* = 8.3, 6.8 Hz, 2H), 2.10 (dt, *J* = 12.9, 7.6 Hz, 1H). | 1.85 [434.1] |
| "A285" | 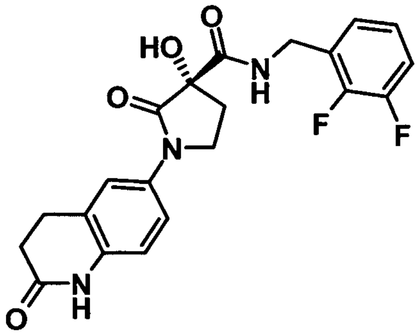 (S)-3-Hydroxy-2-oxo-1-(2-oxo-1,2,3,4-tetrahydro-chinolin-6-yl)-pyrrolidin-3-carbonsäure-2,3-difluor-benzylamid | ** 10.06 (s, 1H), 8.57 (t, *J* = 6.3 Hz, 1H), 7.48 (d, *J* = 2.2 Hz, 1H), 7.43 (dd, *J* = 8.6, 2.4 Hz, 1H), 7.36 – 7.23 (m, 1H), 7.21 – 7.09 (m, 2H), 6.86 (d, *J* = 8.6 Hz, 1H), 6.66 (s, 1H), 4.42 (dd, *J* = 15.5, 6.4 Hz, 1H), 4.35 (dd, *J* = 15.5, 6.0 Hz, 1H), 3.87 – 3.75 (m, 2H), 2.88 (t, *J* = 7.5 Hz, 2H), 2.57 (ddd, *J* = 12.6, 7.0, 4.5 Hz, 1H), 2.44 (dd, *J* = 8.3, 6.8 Hz, 2H), 2.10 (dt, *J* = 12.9, 7.7 Hz, 1H). | 1.78 [416.1] |

| | | | |
|---|---|---|---|
| "A286" | 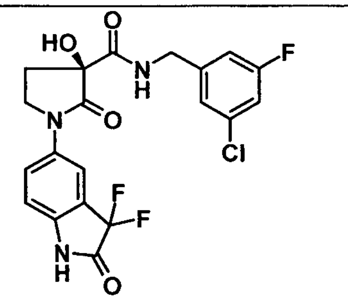 (S)-1-(3,3-Difluor-2-oxo-2,3-dihydro-1H-indol-5-yl)-3-hydroxy-2-oxo-pyrrolidin-3-carbonsäure-3-chlor-5-fluor-benzylamid | 11.24 (s, 1H), 8.75 (t, *J* = 6.4 Hz, 1H), 8.02 (d, *J* = 1.7 Hz, 1H), 7.79 (dd, *J* = 8.6, 2.0 Hz, 1H), 7.27 (dt, *J* = 8.7, 2.1 Hz, 1H), 7.20 (s, 1H), 7.10 (d, *J* = 9.6 Hz, 1H), 7.04 (d, *J* = 8.6 Hz, 1H), 6.79 (s, 1H), 4.38 (dd, *J* = 15.8, 6.8 Hz, 1H), 4.24 (dd, *J* = 15.8, 6.0 Hz, 1H), 3.86 (t, *J* = 7.1 Hz, 2H), 2.63 – 2.54 (m, 1H), 2.12 (dt, *J* = 12.9, 7.7 Hz, 1H). | 3.94 [452] |
| "A287" | 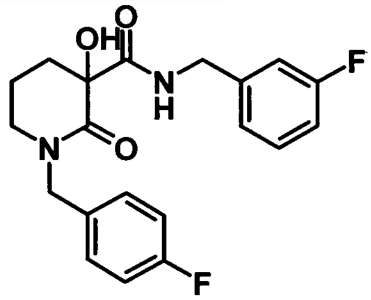 1-(4-Fluor-benzyl)-3-hydroxy-2-oxo-piperidin-3-carbonsäure-3-fluor-benzylamid | 8.50 (t, *J* = 6.3 Hz, 1H), 7.37 – 7.28 (m, 3H), 7.19 – 7.10 (m, 4H), 7.03 (td, *J* = 8.3, 2.2 Hz, 1H), 6.32 (s, 1H), 4.58 (d, *J* = 14.9 Hz, 1H), 4.44 (d, *J* = 14.9 Hz, 1H), 4.38 (dd, *J* = 15.6, 6.8 Hz,, 1H), 4.26 (dd, *J* = 15.6, 5.9 Hz, 1H), 3.23 (m, 2H), 2.16 (m, 1H), 1.84 (m, 3H). | 4.03 [375] |
| "A288" | 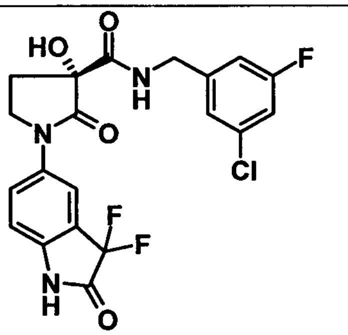 (R)-1-(3,3-Difluor-2-oxo-2,3-dihydro-1H-indol-5-yl)-3-hydroxy-2-oxo-pyrrolidin-3-carbonsäure-3-chlor-5-fluor-benzylamid | 11.24 (s, 1H), 8.75 (t, *J* = 6.3 Hz, 1H), 8.03 (s, 1H), 7.80 (dd, *J* = 8.6, 1.9 Hz, 1H), 7.27 (dt, *J* = 8.8, 2.1 Hz, 1H), 7.20 (s, 1H), 7.10 (d, *J* = 9.5 Hz, 1H), 7.04 (d, *J* = 8.6 Hz, 1H), 6.79 (s, 1H), 4.38 (dd, *J* = 15.7, 6.7 Hz, 1H), 4.24 (dd, *J* = 15.7, 6.1 Hz, 1H), 3.86 (m, 2H), 2.62 – 2.52 (m, 1H), 2.12 (dt, *J* = 13.1, 7.6 Hz, 1H). | 3.94 [452] |

| | | | |
|---|---|---|---|
| "A289" | 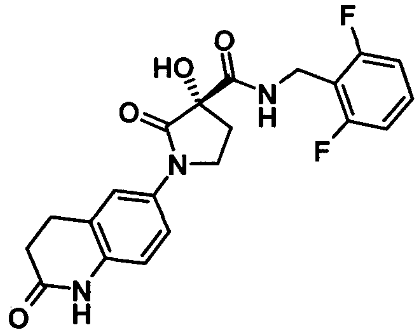 (S)-3-Hydroxy-2-oxo-1-(2-oxo-1,2,3,4-tetrahydro-chinolin-6-yl)-pyrrolidin-3-carbonsäure-2,6-difluor-benzylamid | ** 10.06 (s, 1H), 8.11 (t, *J* = 5.7 Hz, 1H), 7.47 (d, *J* = 2.2 Hz, 1H), 7.41 (dd, *J* = 8.6, 2.5 Hz, 1H), 7.38 (td, *J* = 8.4, 4.2 Hz, 1H), 7.12 – 7.00 (m, 2H), 6.85 (d, *J* = 8.6 Hz, 1H), 6.56 (s, 1H), 4.42 (dd, *J* = 14.4, 5.9 Hz, 1H), 4.36 (dd, *J* = 14.4, 5.6 Hz, 1H), 3.84 – 3.70 (m, 2H), 2.88 (t, *J* = 7.5 Hz, 2H), 2.44 (dd, *J* = 8.3, 6.8 Hz, 2H), 2.12 – 2.01 (m, 1H). | 1.72 [416.1] |
| "A290" | 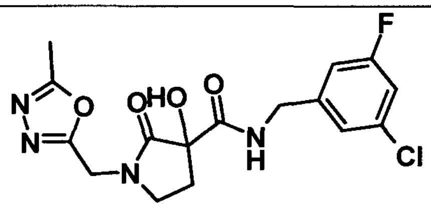 3-Hydroxy-1-(5-methyl-[1,3,4]oxadiazol-2-ylmethyl)-2-oxo-pyrrolidin-3-carbonsäure-3-chlor-5-fluor-benzylamid | ** 8.62 (t, *J* = 6.3 Hz, 1H), 7.26 (dt, *J* = 8.8, 2.1 Hz, 1H), 7.20 (s, 1H), 7.09 (t, *J* = 9.5 Hz, 1H), 6.60 (s, 1H), 4.72 (d, *J* = 16.0 Hz, 1H), 4.62 (d, *J* = 16.0 Hz, 1H), 4.37 (dd, *J* = 15.8, 6.7 Hz, 1H), 4.24 (dd, *J* = 15.8, 6.0 Hz, 1H), 3.57 (s, 1H), 3.49 – 3.36 (m, 2H), 2.47 (s, 3H), 2.01 (ddd, *J* = 13.3, 8.3, 6.2 Hz, 1H). | 1.77 [398.1] |
| "A291" | 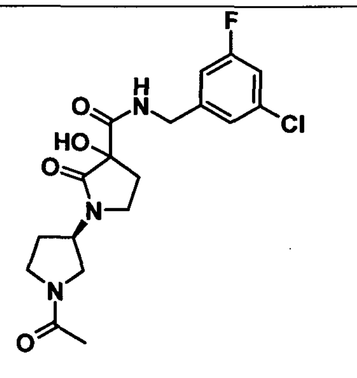 (R)-1'-Acetyl-3-hydroxy-2-oxo-[1,3']bipyrrolidinyl-3-carbonsäure-3-chlor-5-fluor-benzylamid | 8.59 (t, *J* = 5.1 Hz, 1H), 7.26 (d, *J* = 8.7 Hz, 1H), 7.20 (s, 1H), 7.10 (d, *J* = 9.7 Hz, 1H), 6.51 (dd, *J* = 5.3, 4.7 Hz, 1H), 4.60 – 4.43 (m, 1H), 4.36 (dd, *J* = 15.6, 6.7 Hz, 1H), 4.23 (dd, *J* = 15.8, 6.0 Hz, 1H), 3.66 – 3.53 (m, 1H), 3.53 – 3.42 (m, 2H), 3.42 – 3.33 (m, 2H), 3.26 (m, 1H), 2.44 (m, 1H), 2.16 – 2.05 (m, 1H), 2.05 – 1.96 (m, | 1.71 [398.1] |

| | | | |
|---|---|---|---|
| | | 2H), 1.94 (s, *J* = 2.9 Hz, 3H), 1.93 – 1.89 (m, 1H). | |
| "A292" | 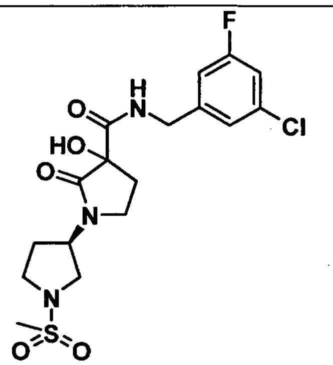<br>(R)-3-Hydroxy-1'-methansulfonyl-2-oxo-[1,3']bipyrrolidinyl-3-carbonsäure-3-chlor-5-fluor-benzylamid | ** 8.58 (t, *J* = 6.3 Hz, 1H), 7.25 (dt, *J* = 8.7, 2.1 Hz, 1H), 7.19 (s, 1H), 7.09 (d, *J* = 9.7 Hz, 1H), 6.50 (d, *J* = 1.0 Hz, 1H), 4.57 (p, *J* = 7.1 Hz, 1H), 4.36 (dd, *J* = 15.7, 6.8 Hz, 1H), 4.23 (dd, *J* = 15.7, 5.9 Hz, 1H), 3.57 (s, 3H), 3.48 – 3.35 (m, 4H), 3.30 – 3.29 (m, 1H), 3.21 (m, 1H), 2.93 (d, *J* = 4.2 Hz, 3H), 2.49 – 2.40 (m, 1H), 2.15 – 2.08 (m, 1H), 2.01 (m, 1H). | 1.85 [434.0] |
| "A293" | 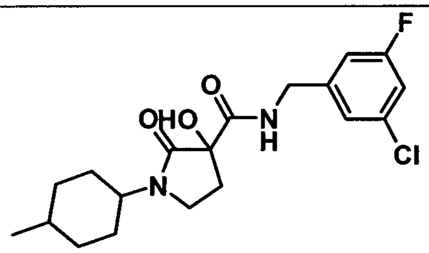<br>3-Hydroxy-1-(4-methyl-cyclohexyl)-2-oxo-pyrrolidin-3-carbonsäure-3-chlor-5-fluor-benzylamid | ** 8.51 (t, *J* = 6.3 Hz, 1H), 7.25 (dt, *J* = 8.7, 2.1 Hz, 1H), 7.19 (s, 1H), 7.09 (d, *J* = 9.6 Hz, 1H), 6.39 (s, 1H), 4.36 (dd, *J* = 15.7, 6.8 Hz, 1H), 4.22 (dd, *J* = 15.7, 6.0 Hz, 1H), 3.73 – 3.60 (m, 1H), 3.39 – 3.32 (m, 1H), 2.42 (dq, *J* = 7.4, 4.3 Hz, 1H), 1.92 (tt, *J* = 14.8, 6.4 Hz, 1H), 1.77 – 1.67 (m, 2H), 1.61 – 1.41 (m, 4H), 1.36 – 1.26 (m, 1H), 1.10 – 0.92 (m, 2H), 0.87 (d, *J* = 6.5 Hz, 3H). | |
| "A294" | 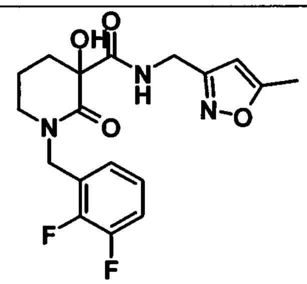 | 8.47 (t, *J* = 6.2 Hz, 1H), 7.34 (dd, *J* = 17.5, 9.2 Hz, 1H), 7.22 – 7.10 (m, 2H), 6.31 (s, 1H), 6.10 (s, 1H), 4.69 (d, *J* = 15.7 Hz, 1H), 4.50 (d, *J* = 15.7 Hz, 1H), 4.27 (d, *J* = 6.3 Hz, 2H), | 3.39 [380.2] |

| | | | |
|---|---|---|---|
| | 1-(2,3-Difluor-benzyl)-3-hydroxy-2-oxo-piperidin-3-carbonsäure-(5-methyl-isoxazol-3-ylmethyl)-amid | 3.27 (s, 2H), 2.34 (s, 3H), 2.22 – 2.09 (m, 1H), 1.94 – 1.80 (m, 3H). | |
| "A295" | 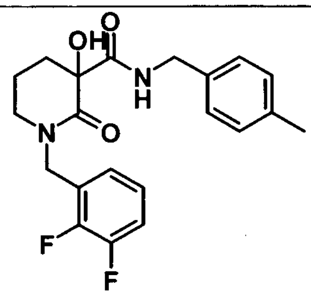 1-(2,3-Difluor-benzyl)-3-hydroxy-2-oxo-piperidin-3-carbonsäure-4-methyl-benzylamid | 8.30 (t, *J* = 6.1 Hz, 1H), 7.38 – 7.29 (m, 1H), 7.20 – 7.06 (m, 6H), 6.27 (s, 1H), 4.69 (d, *J* = 15.5 Hz, 1H), 4.50 (d, *J* = 15.6 Hz, 1H), 4.25 (t, *J* = 5.7 Hz, 2H), 3.36 (m, 1H), 3.27 (m, 1H), 2.26 (s, 3H), 2.16 (m, 1H), 1.85 (m, 3H). | 4.34 [389.2] |
| "A296" | 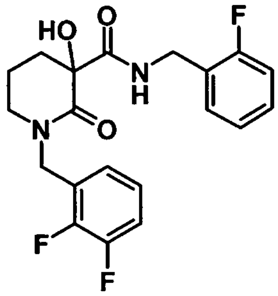 1-(2,3-Difluor-benzyl)-3-hydroxy-2-oxo-piperidin-3-carbonsäure-2-fluor-benzylamid | 8.41 (t, *J* = 6.2 Hz, 1H), 7.41 – 7.24 (m, 3H), 7.21 – 7.10 (m, 4H), 6.36 (s, 1H), 4.69 (d, *J* = 15.7 Hz, 1H), 4.52 (d, *J* = 15.5 Hz, 1H), 4.37 – 4.29 (m, 2H), 3.33 (s, 1H), 3.30 – 3.25 (m, 1H), 2.19 (d, *J* = 8.1 Hz, 1H), 1.92 – 1.82 (m, 3H). | 4.11 [393.2] |
| "A297" | 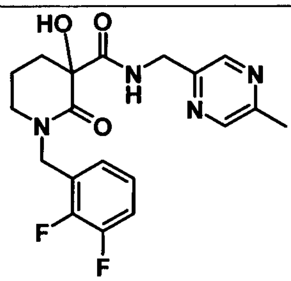 1-(2,3-Difluor-benzyl)-3-hydroxy-2-oxo-piperidin-3-carbonsäure-(5-methyl-pyrazin-2-ylmethyl)-amid | 8.61 (t, *J* = 6.1 Hz, 1H), 8.50 (s, 1H), 8.44 (s, 1H), 7.38 – 7.29 (m, 1H), 7.23 – 7.08 (m, 2H), 6.42 (s, 1H), 4.69 (d, *J* = 15.6 Hz, 1H), 4.51 (d, *J* = 15.6 Hz, 1H), 4.46 (dd, *J* = 16.1, 6.1 Hz, 1H), 4.35 (dd, *J* = 16.1, 5.6 Hz, 1H), 3.35 (m, 1H), 3.30 – 3.25 (m, 1H), 2.45 (s, 3H), 2.21 (m, 1H), 1.88 (m, 3H). | 2.97 [391] |

| | | | |
|---|---|---|---|
| "A298" | 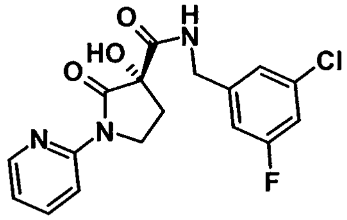 (S)-3-Hydroxy-2-oxo-1-pyridin-2-yl-pyrrolidin-3-carbonsäure-3-chlor-5-fluor-benzylamid | 8.77 (t, *J* = 6.3 Hz, 1H), 8.42 (dd, *J* = 4.9, 1.0 Hz, 1H), 8.29 (d, *J* = 8.4 Hz, 1H), 7.86 (ddd, *J* = 8.6, 7.4, 1.9 Hz, 1H), 7.27 (dt, *J* = 8.7, 2.1 Hz, 1H), 7.24 – 7.17 (m, 2H), 7.10 (d, *J* = 9.0 Hz, 1H), 6.85 (s, 1H), 4.38 (dd, *J* = 15.7, 6.7 Hz, 1H), 4.24 (dd, *J* = 15.7, 6.0 Hz, 1H), 4.06 (ddd, *J* = 10.6, 8.7, 3.9 Hz, 1H), 3.94 (dt, *J* = 10.6, 7.6 Hz, 1H), 2.61 – 2.52 (m, 2H), 2.12 (dt, *J* = 13.0, 8.6 Hz, 1H). | 3.49 [364] |
| "A299" | 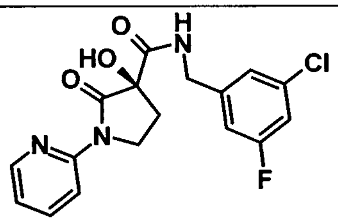 (R)-3-Hydroxy-2-oxo-1-pyridin-2-yl-pyrrolidin-3-carbonsäure-3-chlor-5-fluor-benzylamid | 8.77 (t, *J* = 6.4 Hz, 1H), 8.42 (dd, *J* = 4.9, 1.1 Hz, 1H), 8.29 (d, *J* = 8.5 Hz, 1H), 7.86 (ddd, *J* = 8.6, 7.4, 1.9 Hz, 1H), 7.27 (dt, *J* = 8.8, 2.1 Hz, 1H), 7.23 – 7.16 (m, 2H), 7.10 (d, *J* = 9.0 Hz, 1H), 6.85 (s, 1H), 4.38 (dd, *J* = 15.7, 6.8 Hz, 1H), 4.24 (dd, *J* = 15.7, 6.0 Hz, 1H), 4.06 (ddd, *J* = 10.6, 8.8, 3.9 Hz, 1H), 3.94 (dt, *J* = 10.6, 7.6 Hz, 1H), 2.56 (ddd, *J* = 17.2, 8.5, 4.6 Hz, 1H), 2.12 (dt, *J* = 13.0, 8.6 Hz, 1H). | 3.49 [364] |
| "A300" | 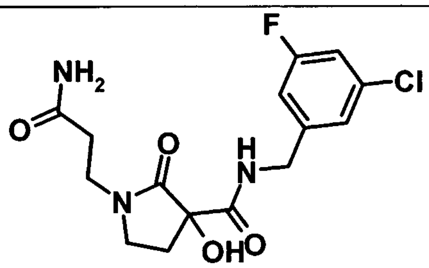 | * 7.21 (2 H, m), 7.11 (1 H, d, J 9.4), 4.40 (1 H, d, J 15.8), 4.27 (1 H, d, J 15.8), 3.44 (4 H, m), 2.47 (1 H, dt, J 6.8, 5.7), 2.34 (2 H, t, J 7.3), 2.00 (1 H, m). | 1.60 [358.0] |

| | | | |
|---|---|---|---|
| | 1-(2-Carbamoyl-ethyl)-3-hydroxy-2-oxo-pyrrolidin-3-carbonsäure-3-chlor-5-fluor-benzylamid | | |
| "A301" | 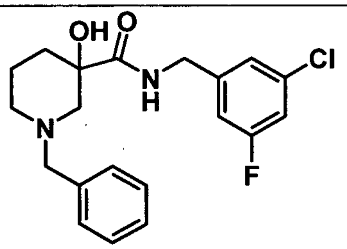 1-Benzyl-3-hydroxy-piperidin-3-carbonsäure-3-chlor-5-fluor-benzylamid | | 3.56 [378.0] |
| "A302" | 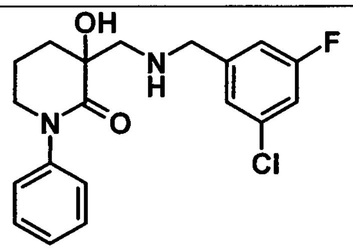 3-[(3-Chlor-5-fluor-benzylamino)-methyl]-3-hydroxy-1-phenyl-piperidin-2-on | | |
| "A303" | 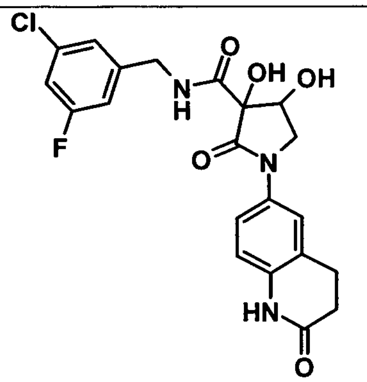 3,4-Dihydroxy-2-oxo-1-(2-oxo-1,2,3,4-tetrahydro-chinolin-6-yl)-pyrrolidin-3-carbonsäure-3-chlor-5-fluor-benzylamid | | |

| | | | |
|---|---|---|---|
| "A304" | 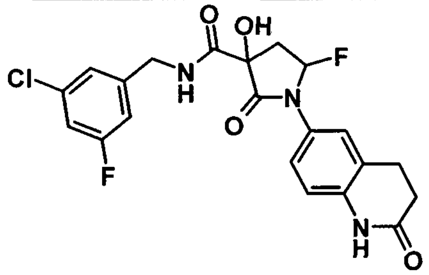 5-Fluor-3-hydroxy-2-oxo-1-(2-oxo-1,2,3,4-tetrahydro-chinolin-6-yl)-pyrrolidin-3-carbonsäure-3-chlor-5-fluor-benzylamid | | |
| | | | |
| "B1" | 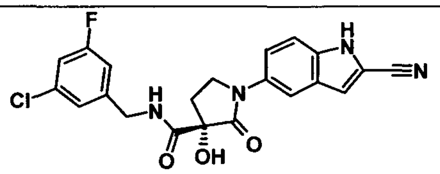 (S)-1-(2-Cyan-1H-indol-5-yl)-3-hydroxy-2-oxo-pyrrolidin-3-carbonsäure-3-chlor-5-fluor-benzylamid | ** 8.68 (t, $J$ = 6.3 Hz, 1H), 7.86 (d, $J$ = 1.7 Hz, 1H), 7.78 – 7.74 (m, 1H), 7.50 (d, $J$ = 9.0 Hz, 1H), 7.38 (d, $J$ = 0.7 Hz, 1H), 7.26 (dt, $J$ = 8.7, 2.1 Hz, 1H), 7.22 (s, 1H), 7.12 (d, $J$ = 9.6 Hz, 1H), 6.72 (s, 1H), 4.40 (dd, $J$ = 15.7, 6.7 Hz, 1H), 4.26 (dd, $J$ = 15.7, 6.0 Hz, 1H), 3.96 – 3.82 (m, 2H), 2.67 – 2.57 (m, 1H), 2.22 – 2.09 (m, 1H). | |
| "B2" | 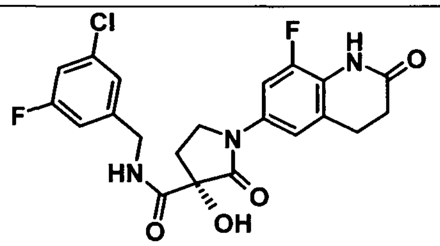 (S)-1-(8-Fluor-2-oxo-1,2,3,4-tetrahydro-chinolin-6-yl)-3-hydroxy-2-oxo-pyrrolidin-3-carbonsäure-3-chlor-5-fluor-benzylamid | 10.15 (s, 1H), 8.74 (s, 1H), 7.60 (dd, $J$ = 12.9, 1.9 Hz, 1H), 7.33 – 7.24 (m, 2H), 7.20 (s, 1H), 7.09 (d, $J$ = 9.4 Hz, 1H), 6.78 (s, 1H), 4.37 (d, $J$ = 15.6 Hz, 1H), 4.23 (d, $J$ = 15.6 Hz, 1H), 3.81 (m, 2H), 2.93 (t, $J$ = 7.4 Hz, 2H), 2.63 – 2.53 (m, 1H), 2.18 – 2.04 (m, 1H). | |

| | | | |
|---|---|---|---|
| "B3" | 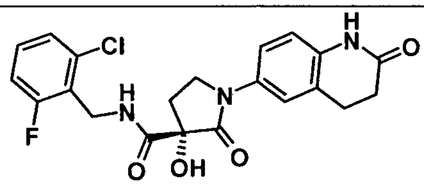 (S)-3-Hydroxy-2-oxo-1-(2-oxo-1,2,3,4-tetrahydro-chinolin-6-yl)-pyrrolidin-3-carbonsäure-2-chlor-6-fluor-benzylamid | ** 10.06 (s, 1H), 7.97 (t, *J* = 5.6 Hz, 1H), 7.48 (d, *J* = 2.2 Hz, 1H), 7.42 (dd, *J* = 8.6, 2.5 Hz, 1H), 7.38 (dt, *J* = 8.1, 4.0 Hz, 1H), 7.33 (d, *J* = 7.9 Hz, 1H), 7.25 – 7.19 (m, 1H), 6.85 (d, *J* = 8.6 Hz, 1H), 6.58 (s, 1H), 4.51 (ddd, *J* = 14.2, 5.8, 1.2 Hz, 1H), 4.44 (ddd, *J* = 14.4, 5.4, 1.0 Hz, 1H), 3.84 – 3.73 (m, 2H), 2.88 (t, *J* = 7.5 Hz, 2H), 2.44 (dd, *J* = 8.3, 6.8 Hz, 2H), 2.11 – 2.01 (m, 1H). | 1.82 [432.1] |
| "B4" | 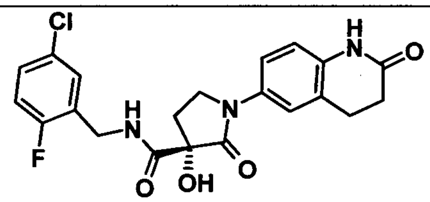 (S)-3-Hydroxy-2-oxo-1-(2-oxo-1,2,3,4-tetrahydro-chinolin-6-yl)-pyrrolidin-3-carbonsäure-5-chlor-2-fluor-benzylamid | ** 10.06 (s, 1H), 8.61 (t, *J* = 6.3 Hz, 1H), 7.49 (d, *J* = 2.2 Hz, 1H), 7.44 (dd, *J* = 8.6, 2.5 Hz, 1H), 7.38 (dd, *J* = 6.5, 2.7 Hz, 1H), 7.36 – 7.32 (m, 1H), 7.24 – 7.19 (m, 1H), 6.86 (d, *J* = 8.6 Hz, 1H), 6.70 (s, 1H), 4.39 (dd, *J* = 15.8, 6.6 Hz, 1H), 4.28 (dd, *J* = 15.8, 5.9 Hz, 1H), 3.86 – 3.76 (m, 2H), 2.91 – 2.85 (m, 2H), 2.57 (ddd, *J* = 11.7, 7.0, 4.6 Hz, 1H), 2.44 (dd, *J* = 8.3, 6.8 Hz, 2H), 2.11 (dt, *J* = 12.9, 7.6 Hz, 1H). | 1.87 [432.0] |
| "B5" | 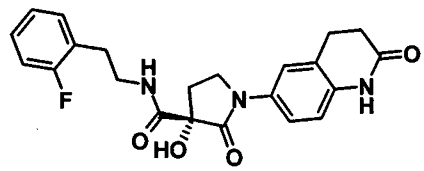 (S)-3-Hydroxy-2-oxo-1-(2-oxo-1,2,3,4-tetrahydro-chinolin-6-yl)- | | 1.81 [412.1] |

| | | | |
|---|---|---|---|
| | pyrrolidin-3-carbonsäure-[2-(2-fluor-phenyl)-ethyl]-amid | | |
| "B6" | 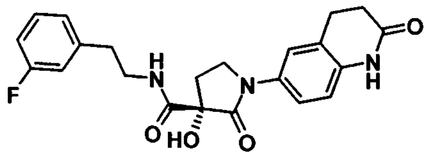 (S)-3-Hydroxy-2-oxo-1-(2-oxo-1,2,3,4-tetrahydro-chinolin-6-yl)-pyrrolidin-3-carbonsäure-[2-(3-fluor-phenyl)-ethyl]-amid | | 1.82 [412.1] |
| "B7" | 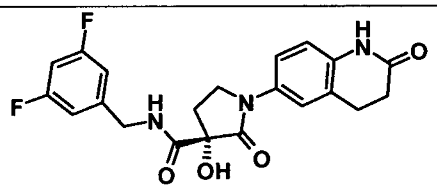 (S)-3-Hydroxy-2-oxo-1-(2-oxo-1,2,3,4-tetrahydro-chinolin-6-yl)-pyrrolidin-3-carbonsäure-3,5-difluor-benzylamid | | 1.81 [416.1] |
| "B8" | 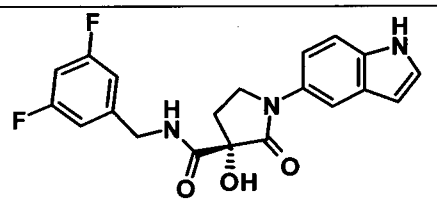 (S)-3-Hydroxy-1-(1H-indol-5-yl)-2-oxo-pyrrolidin-3-carbonsäure-3,5-difluor-benzylamid | | 2.00 [386.1] |
| "B9" | 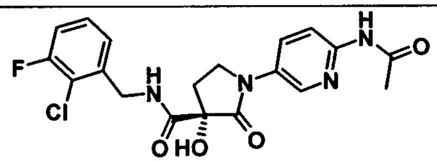 (S)-1-(6-Acetylamino-pyridin-3-yl)-3-hydroxy-2-oxo-pyrrolidin-3-carbonsäure-2-chlor-3-fluor-benzylamid | | 1.81 [421.1] |

| | | | |
|---|---|---|---|
| "B10" | 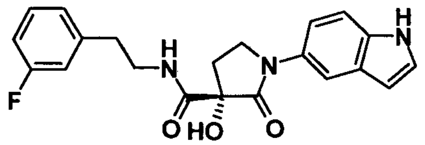<br>(S)-3-Hydroxy-1-(1H-indol-5-yl)-2-oxo-pyrrolidin-3-carbonsäure-[2-(3-fluor-phenyl)-ethyl]-amid | | 2.01<br>[382.1] |
| "B11" | 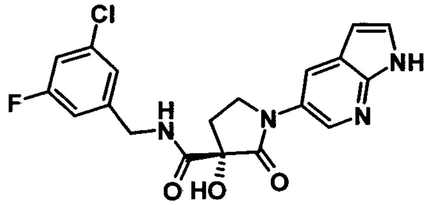<br>(S)-3-Hydroxy-2-oxo-1-(1H-pyrrolo[2,3-b]pyridin-5-yl)-pyrrolidin-3-carbonsäure-3-chlor-5-fluor-benzylamid | | |
| "B12" | 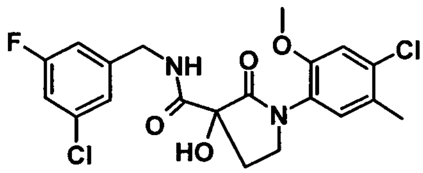<br>1-(4-Chlor-2-methoxy-5-methyl-phenyl)-3-hydroxy-2-oxo-pyrrolidin-3-carbonsäure-3-chlor-5-fluor-benzylamid | | 2.43<br>[441.0] |
| "B13" | 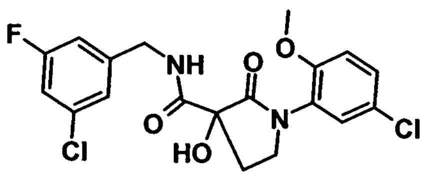<br>1-(5-Chloro-2-methoxy-phenyl)-3-hydroxy-2-oxo-pyrrolidine-3-carboxylic acid 3-chloro-5-fluoro-benzylamide | | 2.31<br>[427.0] |

| | | | |
|---|---|---|---|
| "B14" | 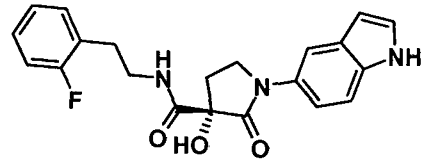 (S)-3-Hydroxy-1-(1H-indol-5-yl)-2-oxo-pyrrolidine-3-carboxylic acid [2-(2-fluoro-phenyl)-ethyl]-amide | | 2.00 [382.1] |
| "B15" | 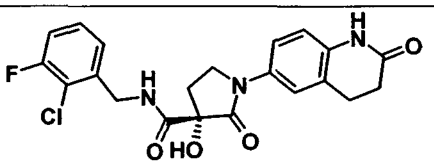 (S)-3-Hydroxy-2-oxo-1-(2-oxo-1,2,3,4-tetrahydro-chinolin-6-yl)-pyrrolidin-3-carbonsäure-2-chlor-3-fluor-benzylamid | | 1.88 [432.1] |
| "B16" | 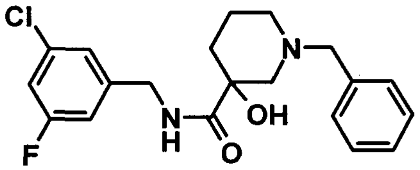 1-Benzyl-3-hydroxy-piperidin-3-carbonsäure-3-chlor-5-fluor-benzylamid | 8.55 (t, *J* = 6.2 Hz, 1H), 7.32 – 7.25 (m, 5H), 7.25 – 7.19 (m, 1H), 7.11 (s, 1H), 7.01 (d, *J* = 9.5 Hz, 1H), 5.19 (s, 1H), 4.25 (d, *J* = 6.3 Hz, 2H), 3.49 (d, *J* = 11.2 Hz, 2H), 2.58 (d, *J* = 10.7 Hz, 1H), 2.41 (dd, *J* = 23.0, 11.2 Hz, 2H), 2.08 (s, 1H), 1.82 – 1.68 (m, 2H), 1.44 (dd, *J* = 16.0, 8.0 Hz, 2H). | |
| "B17" | 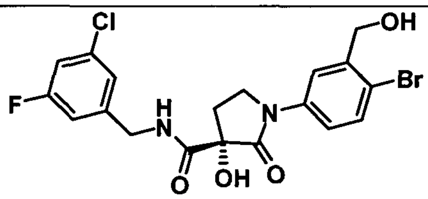 (S)-1-(4-Brom-3-hydroxymethyl-phenyl)-3-hydroxy-2-oxo-pyrrolidin-3-carbonsäure-3-chlor-5-fluor-benzylamid | 8.74 (t, *J* = 6.4 Hz, 1H), 7.84 (s, 1H), 7.60 – 7.53 (m, 2H), 7.27 (d, *J* = 8.7 Hz, 1H), 7.20 (s, 1H), 7.09 (d, *J* = 9.1 Hz, 1H), 6.80 (s, 1H), 5.51 (t, *J* = 5.6 Hz, 1H), 4.49 (d, *J* = 5.6 Hz, 2H), 4.37 (dd, *J* = 15.6, 6.8 Hz, 1H), 4.24 (dd, *J* = 15.7, 6.0 Hz, 1H), 3.85 (t, *J* = 6.8 Hz, | |

| | | | |
|---|---|---|---|
| | | 2H), 2.63 – 2.54 (m, 1H), 2.19 – 2.07 (m, 1H). | |
| "B18" | 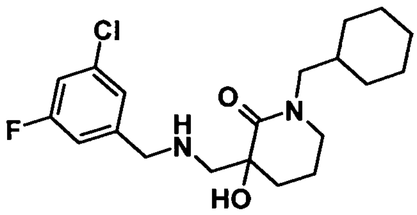 3-[(3-Chlor-5-fluor-benzylamino)-methyl]-1-cyclohexylmethyl-3-hydroxy-piperidin-2-on | 7.29 – 7.19 (m, 2H), 7.14 (d, *J* = 10.1 Hz, 1H), 4.94 (s, 1H), 3.77 – 3.61 (m, 2H), 3.26 (dd, *J* = 8.7, 4.3 Hz, 1H), 3.21 (dd, *J* = 9.1, 5.3 Hz, 2H), 2.93 (dd, *J* = 13.0, 6.7 Hz, 1H), 2.74 – 2.68 (m, 1H), 2.44 (d, *J* = 11.6 Hz, 1H), 2.13 (s, 1H), 2.00 (dd, *J* = 16.7, 6.2 Hz, 1H), 1.83 (d, *J* = 7.9 Hz, 1H), 1.69 – 1.49 (m, 8H), 1.10 (d, *J* = 8.6 Hz, 3H), 0.95 – 0.73 (m, 2H). | |
| "B19" | 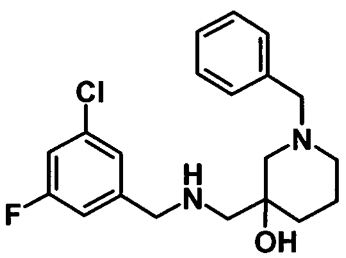 1-Benzyl-3-[(3-chlor-5-fluor-benzylamino)-methyl]-piperidin-3-ol | 7.32 – 7.17 (m, 7H), 7.15 (d, *J* = 9.7 Hz, 1H), 4.21 (s, 1H), 3.71 (s, 2H), 3.46 (d, *J* = 13.4 Hz, 1H), 3.37 (d, *J* = 13.4 Hz, 1H), 2.40 (d, *J* = 10.8 Hz, 2H), 2.09 (d, *J* = 9.7 Hz, 1H), 2.02 (d, *J* = 9.8 Hz, 2H), 1.66 – 1.49 (m, 2H), 1.44 – 1.30 (m, 1H), 1.25 (dd, *J* = 15.0, 6.6 Hz, 1H). | |
| "B20" | 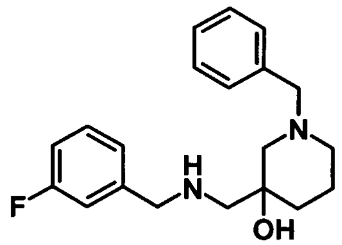 1-Benzyl-3-[(3-fluor-benzylamino)-methyl]-piperidin-3-ol | 7.36 – 7.30 (m, 1H), 7.29 – 7.24 (m, 4H), 7.23 – 7.18 (m, 1H), 7.16 – 7.09 (m, 2H), 7.06 – 6.98 (m, 1H), 4.20 (d, *J* = 12.6 Hz, 1H), 3.72 (s, 2H), 3.46 (d, *J* = 13.4 Hz, 1H), 3.38 (d, *J* = 13.4 Hz, 1H), 2.52 (d, *J* = 7.4 Hz, 1H), 2.35 (d, *J* = 28.1 Hz, 2H), 2.11 (s, 1H), 2.04 (d, *J* = 10.3 Hz, 1H), 1.58 (t, *J* = 12.7 Hz, 2H), 1.44 – 1.31 (m, | |

| | | | |
|---|---|---|---|
| | | 1H), 1.26 (dd, *J* = 15.1, 6.5 Hz, 1H). | |
| "B21" | 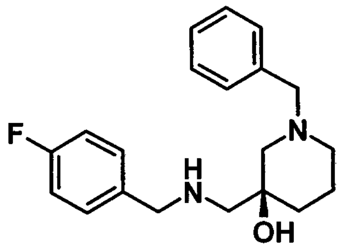 (S)-1-Benzyl-3-[(4-fluor-benzylamino)-methyl]-piperidin-3-ol | 7.34 – 7.28 (m, 3H), 7.26 (d, *J* = 3.9 Hz, 3H), 7.22 (dd, *J* = 13.6, 4.9 Hz, 2H), 7.10 (t, *J* = 8.9 Hz, 2H), 4.19 (s, 1H), 3.67 (s, 2H), 3.45 (d, *J* = 13.5 Hz, 1H), 3.41 – 3.34 (m, 1H), 2.59 – 2.51 (m, 2H), 2.38 (d, *J* = 10.7 Hz, 2H), 2.11 (s, 1H), 2.03 (d, *J* = 10.9 Hz, 1H), 1.58 (s, 2H), 1.45 – 1.31 (m, 1H), 1.29 – 1.18 (m, 1H). | |
| "B22" | 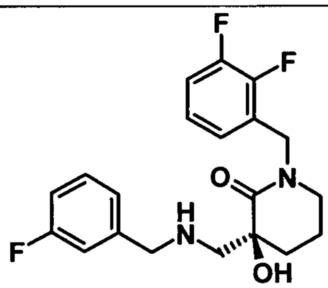 (S)-1-(2,3-Difluor-benzyl)-3-[(3-fluor-benzylamino)-methyl]-3-hydroxy-piperidin-2-on | | |
| "B23" | 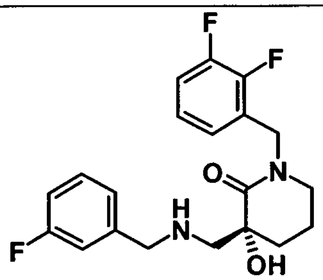 (R)-1-(2,3-Difluor-benzyl)-3-[(3-fluor-benzylamino)-methyl]-3-hydroxy-piperidin-2-on | | |

| | | | |
|---|---|---|---|
| "B24" | 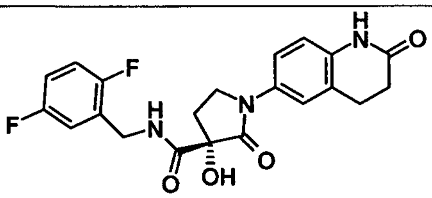 (S)-3-Hydroxy-2-oxo-1-(2-oxo-1,2,3,4-tetrahydro-chinolin-6-yl)-pyrrolidin-3-carbonsäure-2,5-difluor-benzylamid | ** 10.06 (s, 1H), 8.60 (t, J=6.3, 1H), 7.48 (d, J=2.4, 1H), 7.43 (dd, J=8.6, 2.5, 1H), 7.21 (td, J=9.2, 4.5, 1H), 7.18 - 7.06 (m, 2H), 6.86 (d, J=8.6, 1H), 6.70 (s, 1H), 4.40 (dd, J=15.9, 6.6, 1H), 4.27 (dd, J=15.9, 5.9, 1H), 3.81 (dd, J=7.6, 5.9, 2H), 2.88 (t, J=7.5, 2H), 2.59 (dt, J=12.9, 5.8, 1H), 2.48 - 2.41 (m, 2H), 2.11 (dt, J=12.9, 7.6, 1H). | 1.77 [416.1] |
| "B25" | 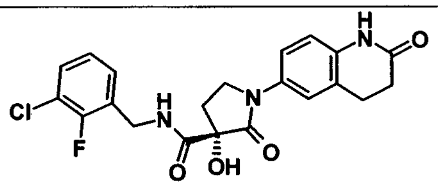 (S)-3-Hydroxy-2-oxo-1-(2-oxo-1,2,3,4-tetrahydro-chinolin-6-yl)-pyrrolidin-3-carbonsäure-3-chlor-2-fluor-benzylamid | ** 10.06 (s, 1H), 8.58 (t, J=6.3, 1H), 7.50 - 7.40 (m, 3H), 7.31 (t, J=6.4, 1H), 7.18 (t, J=8.1, 1H), 6.86 (d, J=8.6, 1H), 6.67 (s, 1H), 4.44 - 4.30 (m, 2H), 3.84 - 3.75 (m, 2H), 2.88 (t, J=7.5, 2H), 2.60 - 2.54 (m, 1H), 2.47 - 2.40 (m, 2H), 2.14 - 2.07 (m, 1H). | 1.90 [432.1] |
| "B26" | 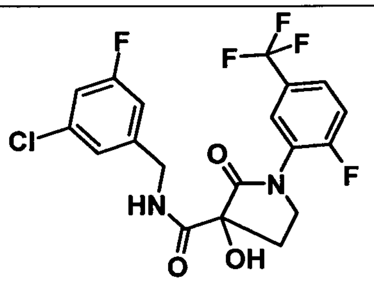 1-(2-Fluor-5-trifluormethyl-phenyl)-3-hydroxy-2-oxo-pyrrolidin-3-carbonsäure-3-chlor-5-fluor-benzylamid | ** 8.73 (t, J=6.4, 1H), 7.86 (dd, J=6.7, 2.3, 1H), 7.81 - 7.73 (m, 1H), 7.60 (t, J=10.4, 8.7, 1H), 7.26 (dt, J=8.7, 2.2, 1H), 7.21 (s, 1H), 7.13 - 7.08 (m, 1H), 6.81 (s, 1H), 4.41 (dd, J=15.7, 6.7, 1H), 4.27 (dd, J=15.7, 6.0, 1H), 3.92 - 3.82 (m, 2H), 2.65 (ddd, J=12.9, 7.0, 4.8, 1H), 2.26 - 2.17 (m, 1H). | 2.39 [449.0] |

| | | | |
|---|---|---|---|
| "B27" | 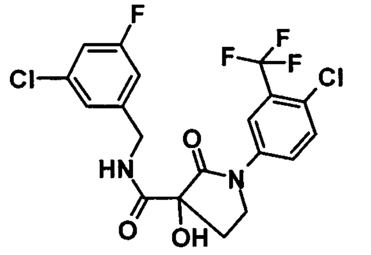<br>1-(4-Chlor-3-trifluormethyl-phenyl)-3-hydroxy-2-oxo-pyrrolidin-3-carbonsäure-3-chlor-5-fluor-benzylamid | ** 8.76 (t, J=6.4, 1H), 8.34 (d, J=2.7, 1H), 7.91 (dd, J=8.9, 2.7, 1H), 7.78 (d, J=8.9, 1H), 7.29 - 7.25 (m, 1H), 7.20 (s, 1H), 7.14 - 7.09 (m, 1H), 6.86 (s, 1H), 4.38 (dd, J=15.7, 6.7, 1H), 4.30 - 4.24 (m, 1H), 3.93 - 3.87 (m, 1H), 2.99 - 2.91 (m, 1H), 2.65 - 2.57 (m, 1H), 2.21 - 2.11 (m, 1H).<br>(mit DMF + EE verunreinigt) | |
| "B28" | 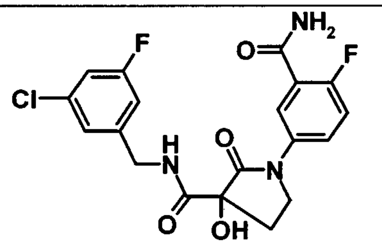<br>1-(3-Carbamoyl-4-fluor-phenyl)-3-hydroxy-2-oxo-pyrrolidin-3-carbonsäure-3-chlor-5-fluor-benzylamid | 8.76 (s, 1H), 7.91 (dd, *J* = 6.3, 2.9 Hz, 1H), 7.83 (ddd, *J* = 9.0, 4.3, 3.1 Hz, 1H), 7.76 (s, 1H), 7.70 (s, 1H), 7.37 – 7.30 (m, 1H), 7.27 (d, *J* = 8.7 Hz, 1H), 7.20 (s, 1H), 7.09 (d, *J* = 9.7 Hz, 1H), 6.83 (s, 1H), 4.37 (dd, *J* = 15.7, 6.2 Hz, 1H), 4.24 (dd, *J* = 15.6, 5.7 Hz, 1H), 3.86 (t, *J* = 6.7 Hz, 2H), 2.62 – 2.54 (m, 2H), 2.44 (s, 1H), 2.18 – 2.07 (m, 1H). | |
| "B29" | 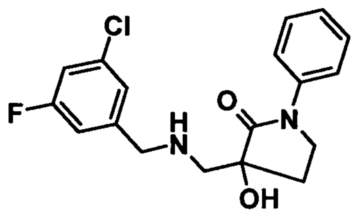<br>3-[(3-Chlor-5-fluor-benzylamino)-methyl]-3-hydroxy-1-phenyl-pyrrolidin-2-on | 7.73 – 7.67 (m, 2H), 7.38 (t, *J* = 8.0 Hz, 2H), 7.26 – 7.20 (m, 2H), 7.13 (dd, *J* = 10.5, 4.2 Hz, 2H), 5.51 (s, 1H), 3.84 – 3.75 (m, 1H), 3.70 (dd, *J* = 15.4, 7.7 Hz, 3H), 2.71 – 2.62 (m, 2H), 2.41 (ddd, *J* = 12.7, 8.0, 4.9 Hz, 2H), 2.02 – 1.92 (m, 1H). | |

| | | | |
|---|---|---|---|
| "B30" | 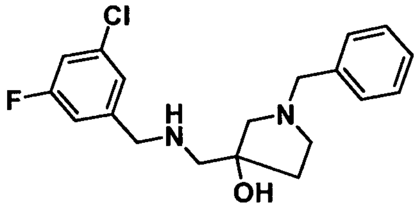 1-Benzyl-3-[(3-chlor-5-fluor-benzylamino)-methyl]-pyrrolidin-3-ol | 7.33 – 7.12 (m, 8H), 4.60 (s, 1H), 3.73 (s, 2H), 3.52 (t, *J* = 7.5 Hz, 2H), 2.58 (dd, *J* = 20.3, 8.8 Hz, 2H), 2.46 (s, 2H), 2.34 (d, *J* = 9.6 Hz, 1H), 1.81 (dt, *J* = 14.6, 7.5 Hz, 1H), 1.70 – 1.53 (m, 1H). | |
| "B31" | 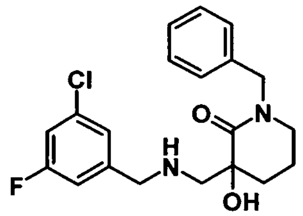 1-Benzyl-3-[(3-chlor-5-fluor-benzylamino)-methyl]-3-hydroxy-piperidin-2-on | 7.25 (dtd, *J* = 10.3, 8.1, 4.2 Hz, 7H), 7.17 (d, *J* = 9.5 Hz, 1H), 5.13 (s, 1H), 4.43 (d, *J* = 14.9 Hz, 1H), 3.71 (dd, *J* = 27.5, 11.9 Hz, 2H), 3.16 (dd, *J* = 7.0, 4.6 Hz, 2H), 2.80 (d, *J* = 11.4 Hz, 1H), 2.24 (s, 1H), 2.10 – 1.99 (m, 1H), 1.93 – 1.80 (m, 1H), 1.71 – 1.60 (m, 2H). | |
| "B32" | 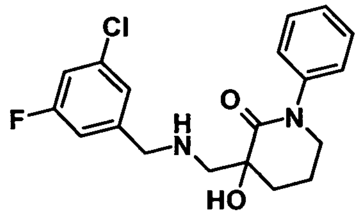 3-[(3-Chlor-5-fluor-benzylamino)-methyl]-3-hydroxy-1-phenyl-piperidin-2-on | 7.39 – 7.33 (m, 2H), 7.29 – 7.20 (m, 5H), 7.18 (d, *J* = 9.6 Hz, 1H), 5.19 (s, 1H), 3.77 (d, *J* = 14.9 Hz, 1H), 3.72 (d, *J* = 14.8 Hz, 1H), 3.66 – 3.53 (m, 2H), 2.81 (d, *J* = 11.8 Hz, 1H), 2.54 (d, *J* = 11.8 Hz, 1H), 2.18 (dt, *J* = 13.0, 4.7 Hz, 1H), 2.10 – 1.95 (m, 1H), 1.86 – 1.70 (m, 2H). | |
| "B33" | 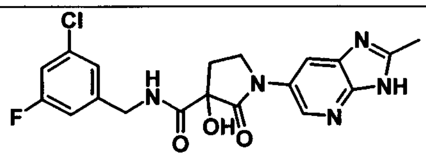 3-Hydroxy-1-(2-methyl-3H-imidazo[4,5-b]pyridin-6-yl)-2-oxo-pyrrolidin-3-carbonsäure-3-chlor-5-fluor-benzylamid | ** 8.73 (t, *J* = 6.4 Hz, 1H), 8.55 (d, *J* = 1.9 Hz, 1H), 8.25 (d, *J* = 2.1 Hz, 1H), 7.27 (dt, *J* = 8.8, 2.1 Hz, 1H), 7.22 (s, 1H), 7.12 (d, *J* = 9.6 Hz, 1H), 6.80 (s, 1H), 4.41 (dd, *J* = 15.7, 6.7 Hz, 1H), 4.27 (dd, *J* = 15.7, 6.0 Hz, | 1.64 [418.1] |

| | | | |
|---|---|---|---|
| | | 1H), 4.00 – 3.92 (m, 2H), 2.68 – 2.60 (m, 1H), 2.56 (s, 3H), 2.18 (dt, *J* = 13.0, 7.5 Hz, 1H). | |
| "B34" | 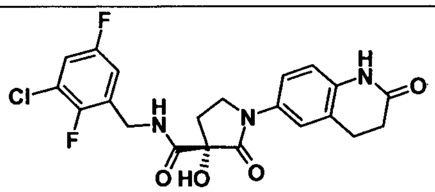 (S)-3-Hydroxy-2-oxo-1-(2-oxo-1,2,3,4-tetrahydro-chinolin-6-yl)-pyrrolidin-3-carbonsäure-3-chlor-2,5-difluor-benzylamid | ** 10.07 (s, 1H), 8.68 (t, J=6.3, 1H), 7.53 - 7.45 (m, 2H), 7.43 (dd, J=8.6, 2.5, 1H), 7.17 (ddd, J=8.7, 5.2, 3.2, 1H), 6.86 (d, J=8.6, 1H), 6.72 (s, 1H), 4.44 (dd, J=16.0, 6.6, 1H), 4.30 (dd, J=16.0, 5.9, 1H), 3.87 - 3.75 (m, 2H), 2.88 (t, J=7.5, 2H), 2.59 (dt, J=12.3, 5.9, 1H), 2.47 - 2.40 (m, 2H), 2.11 (dt, J=13.0, 7.6, 1H). | 1.96 [450.0] |
| "B35" | 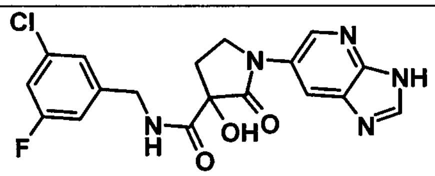 3-Hydroxy-1-(3H-imidazo[4,5-b]pyridin-6-yl)-2-oxo-pyrrolidin-3-carbonsäure-3-chlor-5-fluor-benzylamide | ** 8.77 – 8.69 (m, 1H), 8.68 (d, *J* = 2.3 Hz, 1H), 8.55 (s, 1H), 8.37 (d, *J* = 2.3 Hz, 1H), 7.26 (dt, *J* = 8.7, 2.1 Hz, 1H), 7.22 (s, 1H), 7.12 (d, *J* = 9.6 Hz, 1H), 4.41 (dd, *J* = 15.7, 6.7 Hz, 1H), 4.27 (dd, *J* = 15.7, 6.0 Hz, 1H), 3.97 (dd, *J* = 14.6, 8.3 Hz, 2H), 2.70 – 2.61 (m, 1H), 2.19 (dt, *J* = 13.0, 7.5 Hz, 1H). | 1.70 [404.0] |
| "B36" | 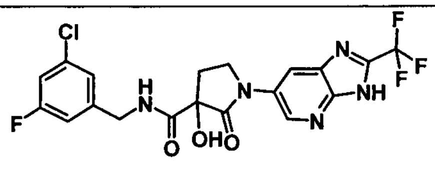 3-Hydroxy-2-oxo-1-(2-trifluormethyl-3H-imidazo[4,5-b]pyridin-6-yl)-pyrrolidin-3-carbonsäure-3-chlor-5-fluor-benzylamid | ** 8.89 (s, 1H), 8.75 (t, *J* = 6.3 Hz, 1H), 8.51 (d, *J* = 1.9 Hz, 1H), 7.27 (dt, *J* = 8.7, 2.1 Hz, 1H), 7.22 (s, 1H), 7.11 (t, *J* = 8.3 Hz, 1H), 6.84 (s, 1H), 4.40 (dd, *J* = 15.7, 6.7 Hz, 1H), 4.28 (dd, *J* = 15.7, 6.0 Hz, 1H), 4.01 | 1.98 [472.0] |

| | | | |
|---|---|---|---|
| | | (s, 2H), 2.70 – 2.62 (m, 1H), 2.25 – 2.16 (m, 1H). | |
| "B37" | 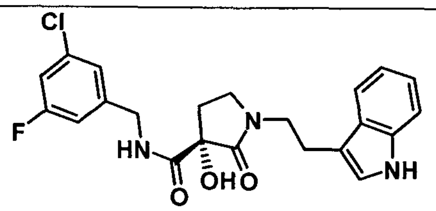 (S)-3-Hydroxy-1-[2-(1H-indol-3-yl)-ethyl]-2-oxo-pyrrolidin-3-carbonsäure-3-chlor-5-fluor-benzylamide | | |
| "B38" | 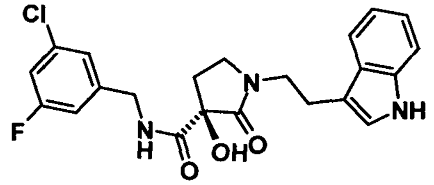 (R)-3-Hydroxy-1-[2-(1H-indol-3-yl)-ethyl]-2-oxo-pyrrolidin-3-carbonsäure-3-chlor-5-fluor-benzylamide | | |
| "B39" | 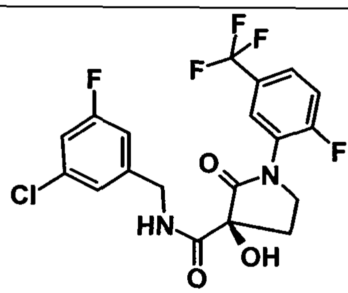 (S)-1-(2-Fluor-5-trifluormethyl-phenyl)-3-hydroxy-2-oxo-pyrrolidin-3-carbonsäure-3-chlor-5-fluor-benzylamid | ** 8.72 (t, J=6.4, 1H), 7.86 (dd, J=6.7, 2.3, 1H), 7.81 - 7.73 (m, 1H), 7.60 (t, J=10.4, 8.7, 1H), 7.26 (dt, J=8.7, 2.2, 1H), 7.21 (s, 1H), 7.13 - 7.08 (m, 1H), 6.81 (s, 1H), 4.41 (dd, J=15.7, 6.7, 1H), 4.27 (dd, J=15.7, 6.0, 1H), 3.92 - 3.82 (m, 2H), 2.65 (ddd, J=12.9, 7.0, 4.8, 1H), 2.25 - 2.17 (m, 1H). | 2.39 [449.0] |
| "B40" | 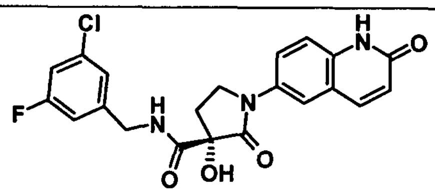 | ** 11.74 (s, 1H), 8.70 (t, J=6.4, 1H), 7.96 - 7.83 (m, 3H), 7.39 - 7.29 (m, 1H), 7.26 (dt, J=8.8, 2.2, 1H), 7.23 - | 2.07 [430.0] |

| | | | |
|---|---|---|---|
| | (S)-3-Hydroxy-2-oxo-1-(2-oxo-1,2-dihydro-chinolin-6-yl)-pyrrolidin-3-carbonsäure-3-chlor-5-fluor-benzylamid | 7.17 (m, 1H), 7.17 - 7.05 (m, 1H), 6.75 (s, 1H), 6.57 - 6.45 (m, 1H), 4.39 (dd, J=15.7, 6.7, 1H), 4.26 (dd, J=15.7, 6.0, 1H), 3.95 - 3.84 (m, 2H), 2.61 (dt, J=12.9, 5.6, 1H), 2.15 (dt, J=12.9, 7.6, 1H). | |
| "B41" | 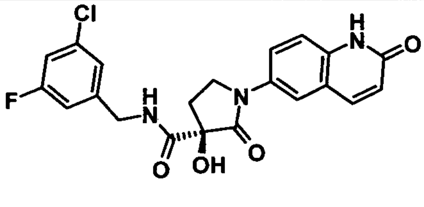 (R)-3-Hydroxy-2-oxo-1-(2-oxo-1,2-dihydro-chinolin-6-yl)-pyrrolidin-3-carbonsäure-3-chlor-5-fluor-benzylamid | ** 11.75 (s, 1H), 8.71 (t, J=6.4, 1H), 7.94 - 7.86 (m, 3H), 7.33 (d, J=9.6, 1H), 7.26 (dt, J=8.8, 2.2, 1H), 7.23 - 7.19 (m, 1H), 7.16 - 7.05 (m, 1H), 6.75 (s, 1H), 6.56 - 6.48 (m, 1H), 4.39 (dd, J=15.7, 6.7, 1H), 4.26 (dd, J=15.7, 6.0, 1H), 3.95 - 3.84 (m, 2H), 2.61 (dt, J=12.9, 5.7, 1H), 2.15 (dt, J=12.9, 7.6, 1H). | 2.07 [430.0] |
| "B42" | 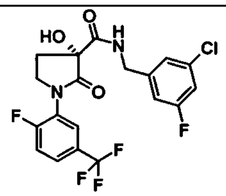 (R)-1-(2-Fluor-5-trifluoromethyl-phenyl)-3-hydroxy-2-oxo-pyrrolidin-3-carbonsäure-3-chlor-5-fluor-benzylamid | ** 8.72 (t, J=6.4, 1H), 7.86 (dd, J=6.8, 2.3, 1H), 7.81 - 7.73 (m, 1H), 7.61 (t, J=10.4, 8.7, 1H), 7.26 (dt, J=8.7, 2.2, 1H), 7.21 (s, 1H), 7.14 - 7.08 (m, 1H), 6.81 (s, 1H), 4.41 (dd, J=15.7, 6.7, 1H), 4.27 (dd, J=15.7, 6.0, 1H), 3.92 - 3.82 (m, 2H), 2.65 (ddd, J=12.9, 7.0, 4.8, 1H), 2.26 - 2.17 (m, 1H). | 2.39 [449.0] |
| "B43" | 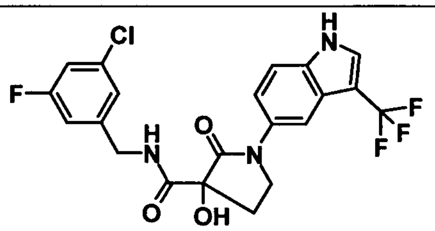 | 11.93 (s, 1H), 8.73 (t, *J* = 6.4 Hz, 1H), 7.99 (s, 1H), 7.86 (s, 1H), 7.54 (d, *J* = 8.6 Hz, 1H), 7.49 (dd, *J* = 8.9, 2.0 Hz, 1H), 7.27 (dt, *J* = 8.7, 2.1 Hz, 1H), | |

| | | | |
|---|---|---|---|
| | 3-Hydroxy-2-oxo-1-(3-trifluormethyl-1H-indol-5-yl)-pyrrolidin-3-carbonsäure-3-chlor-5-fluor-benzylamid | 7.21 (s, 1H), 7.11 (d, *J* = 9.6 Hz, 1H), 6.75 (s, 1H), 4.39 (dd, *J* = 15.7, 6.7 Hz, 1H), 4.25 (dd, *J* = 15.8, 5.9 Hz, 1H), 3.90 (t, *J* = 6.8 Hz, 2H), 2.64 – 2.56 (m, 1H), 2.19 – 2.08 (m, 1H). | |
| "B44" | 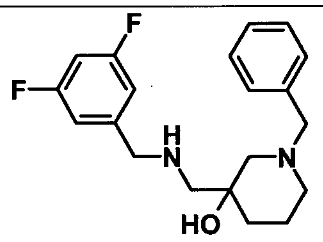 1-Benzyl-3-[(3,5-difluor-benzylamino)-methyl]-piperidin-3-ol | 7.30 – 7.17 (m, 3H), 7.03 (t, *J* = 8.0 Hz, 2H), 4.23 (s, 1H), 3.72 (s, 1H), 3.46 (d, *J* = 13.4 Hz, 1H), 3.38 (d, *J* = 13.4 Hz, 1H), 2.41 (d, *J* = 11.2 Hz, 1H), 2.08 (d, *J* = 22.0 Hz, 1H), 2.03 (d, *J* = 9.6 Hz, 1H), 1.59 (s, 1H), 1.37 (d, *J* = 9.1 Hz, 1H), 1.31 – 1.19 (m, 1H). | |
| "B45" | 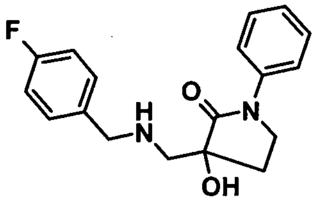 3-[(4-Fluor-benzylamino)-methyl]-3-hydroxy-1-phenyl-pyrrolidin-2-on | 7.70 (t, *J* = 1.6 Hz, 1H), 7.68 (d, *J* = 1.0 Hz, 1H), 7.41 – 7.35 (m, 2H), 7.35 – 7.29 (m, 2H), 7.17 – 7.05 (m, 3H), 5.50 (s, 1H), 3.81 – 3.73 (m, 1H), 3.73 – 3.61 (m, 3H), 2.64 (q, *J* = 11.9 Hz, 2H), 2.40 (ddd, *J* = 12.8, 8.0, 4.8 Hz, 1H), 1.95 (ddd, *J* = 13.0, 8.4, 6.1 Hz, 1H). | |
| "B46" | 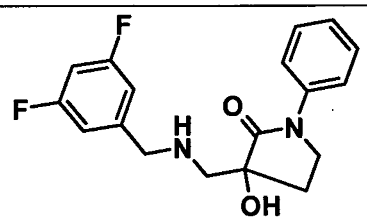 3-[(3,5-Difluor-benzylamino)-methyl]-3-hydroxy-1-phenyl-pyrrolidin-2-on | 7.71 (d, *J* = 1.0 Hz, 1H), 7.69 (s, 1H), 7.41 – 7.33 (m, 2H), 7.14 (t, *J* = 7.4 Hz, 1H), 7.08 – 6.96 (m, 3H), 5.52 (s, 1H), 3.84 – 3.75 (m, 1H), 3.72 (d, *J* = 7.5 Hz, 2H), 3.70 – 3.64 (m, 1H), 2.64 (q, *J* = 12.0 Hz, 2H), 2.41 (ddd, *J* = 12.9, 8.0, 4.8 Hz, 2H), 2.02 – 1.91 (m, 1H). | |

| | | | |
|---|---|---|---|
| "B47" | 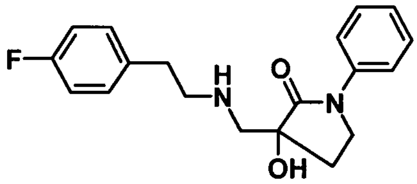 3-{[2-(4-Fluor-phenyl)-ethylamino]-methyl}-3-hydroxy-1-phenyl-pyrrolidin-2-on | 7.68 (d, *J* = 7.8 Hz, 2H), 7.38 (t, *J* = 8.0 Hz, 2H), 7.17 (dt, *J* = 15.1, 6.6 Hz, 3H), 7.01 (t, *J* = 8.9 Hz, 2H), 5.51 (s, 1H), 3.75 (td, *J* = 9.0, 4.6 Hz, 1H), 3.66 (dd, *J* = 15.2, 8.5 Hz, 1H), 2.82 – 2.69 (m, 4H), 2.68 – 2.61 (m, 2H), 2.35 (ddd, *J* = 12.8, 7.9, 4.6 Hz, 1H), 1.99 – 1.88 (m, 1H). | |
| "B48" | 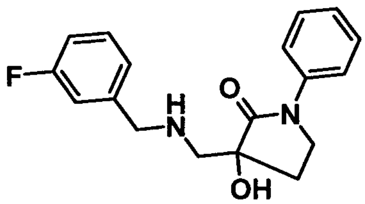 3-[(3-Fluor-benzylamino)-methyl]-3-hydroxy-1-phenyl-pyrrolidin-2-on | 7.73 – 7.65 (m, 2H), 7.38 (t, *J* = 8.0 Hz, 2H), 7.20 – 7.11 (m, 3H), 7.08 (d, *J* = 7.8 Hz, 2H), 5.48 (s, 1H), 3.81 – 3.73 (m, 1H), 3.73 – 3.66 (m, 1H), 3.64 (s, 2H), 2.64 (q, *J* = 11.9 Hz, 2H), 2.39 (ddd, *J* = 12.7, 7.9, 4.8 Hz, 1H), 1.99 – 1.90 (m, 1H). | |
| "B49" | 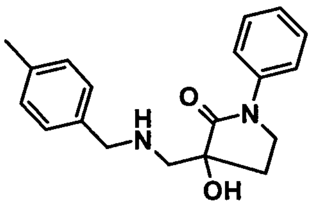 3-Hydroxy-3-[(4-methyl-benzylamino)-methyl]-1-phenyl-pyrrolidin-2-on | 7.73 – 7.64 (m, 2H), 7.38 (t, *J* = 8.0 Hz, 2H), 7.21 – 7.10 (m, 3H), 7.08 (d, *J* = 7.8 Hz, 2H), 5.48 (s, 1H), 3.81 – 3.73 (m, 1H), 3.73 – 3.66 (m, 1H), 3.64 (s, 2H), 2.67 (d, *J* = 11.9 Hz, 1H), 2.62 (d, *J* = 11.9 Hz, 1H), 2.39 (ddd, *J* = 12.7, 7.9, 4.8 Hz, 1H), 2.25 (s, 3H), 1.99 – 1.89 (m, 1H). | |
| "B50" | 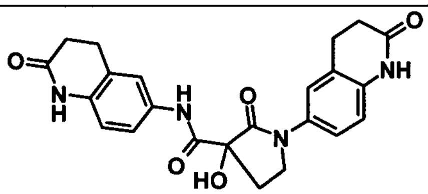 | | 1.45 [435.1] |

| | | | |
|---|---|---|---|
| | 3-Hydroxy-2-oxo-1-(2-oxo-1,2,3,4-tetrahydro-chinolin-6-yl)-pyrrolidin-3-carbonsäure-(2-oxo-1,2,3,4-tetrahydro-chinolin-6-yl)-amid | | |
| "B51" | 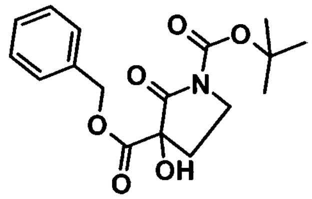 3-Hydroxy-2-oxo-pyrrolidin-1,3-dicarbonsäure-3-benzylester-1-tert-butylester | 7.49 – 7.29 (m, 5H), 6.73 (d, *J* = 5.5 Hz, 1H), 5.20 (d, *J* = 10.3 Hz, 2H), 3.72 (ddd, *J* = 10.4, 8.5, 4.5 Hz, 1H), 3.61 (dt, *J* = 10.4, 7.5 Hz, 1H), 2.47 – 2.37 (m, 1H), 2.05 (ddd, *J* = 13.4, 8.4, 7.0 Hz, 1H), 1.46 (s, 9H). | |
| "B52" | 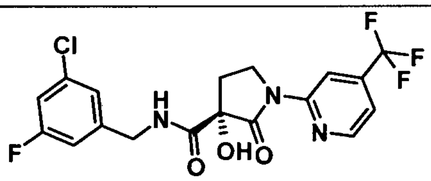 (S)-3-Hydroxy-2-oxo-1-(4-trifluormethyl-pyridin-2-yl)-pyrrolidin-3-carbonsäure-3-chlor-5-fluor-benzylamid | ** 8.80 (t, *J* = 6.4 Hz, 1H), 8.72 (d, *J* = 5.2 Hz, 1H), 8.62 (s, 1H), 7.59 (dd, *J* = 5.2, 1.0 Hz, 1H), 7.27 (dt, *J* = 8.8, 2.1 Hz, 1H), 7.20 (s, 1H), 7.13 – 7.07 (m, 1H), 6.94 (s, 1H), 4.39 (dd, *J* = 15.7, 6.7 Hz, 1H), 4.26 (dd, *J* = 15.7, 6.0 Hz, 1H), 4.15 – 4.09 (m, 1H), 3.98 (dt, *J* = 10.6, 7.6 Hz, 1H), 2.61 (ddd, *J* = 12.9, 7.9, 3.8 Hz, 1H), 2.18 (ddd, *J* = 13.1, 8.7, 7.3 Hz, 1H). | 2.42 [432.0] |
| "B53" | 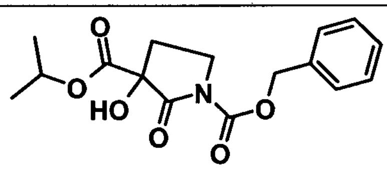 3-Hydroxy-2-oxo-pyrrolidin-1,3-dicarbonsäure-1-benzylester 3-isopropylester | | 2.10 [322.0] |

| | | | |
|---|---|---|---|
| "B54" | 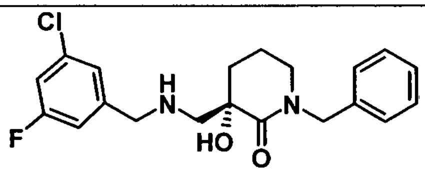 (S)-1-Benzyl-3-[(3-chlor-5-fluor-benzylamino)-methyl]-3-hydroxy-piperidin-2-on | | |
| "B55" | 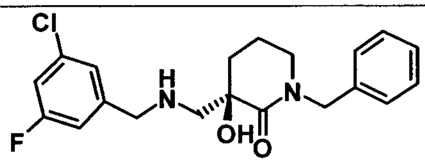 (R)-1-Benzyl-3-[(3-chlor-5-fluor-benzylamino)-methyl]-3-hydroxy-piperidin-2-on | | |
| "B56" | 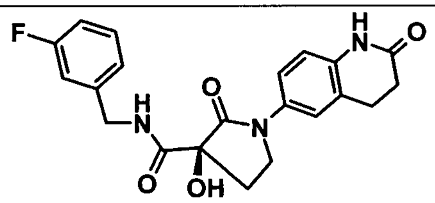 (S)-3-Hydroxy-2-oxo-1-(2-oxo-1,2,3,4-tetrahydro-chinolin-6-yl)-pyrrolidin-3-carbonsäure-3-fluor-benzylamid | | 1.74 [398.1] |
| "B57" | 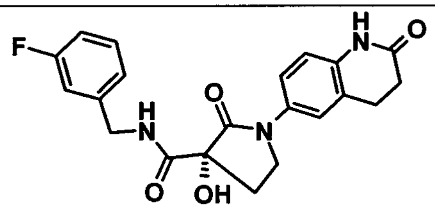 (R)-3-Hydroxy-2-oxo-1-(2-oxo-1,2,3,4-tetrahydro-chinolin-6-yl)-pyrrolidin-3-carbonsäure-3-fluor-benzylamid | | 1.74 [398.1] |

| | | | |
|---|---|---|---|
| "B58" | 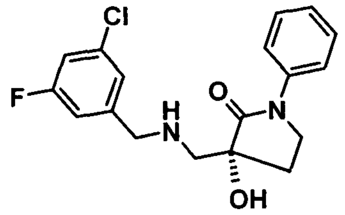<br>(R)-3-[(3-Chlor-5-fluor-benzylamino)-methyl]-3-hydroxy-1-phenyl-pyrrolidin-2-on | | |
| "B59" | 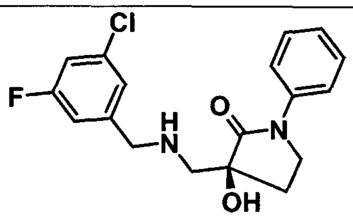<br>(S)-3-[(3-Chlor-5-fluor-benzylamino)-methyl]-3-hydroxy-1-phenyl-pyrrolidin-2-on | | |
| "B60" | 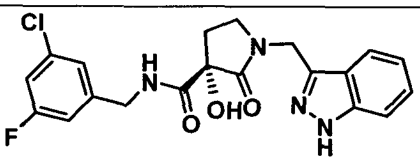<br>(S)-3-Hydroxy-1-(1H-indazol-3-ylmethyl)-2-oxo-pyrrolidin-3-carbonsäure-3-chlor-5-fluor-benzylamid | | |
| "B61" | 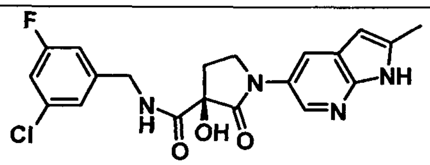<br>(R)-3-Hydroxy-1-(2-methyl-1H-pyrrolo[2,3-b]pyridin-5-yl)-2-oxo-pyrrolidin-3-carbonsäure-3-chlor-5-fluor-benzylamid | | 2.01 [417.0] |
| "B62" | 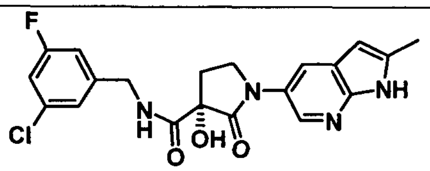 | | 2.01 [4.17.0] |

| | | | |
|---|---|---|---|
| | (S)-3-Hydroxy-1-(2-methyl-1H-pyrrolo[2,3-b]pyridin-5-yl)-2-oxo-pyrrolidin-3-carbonsäure-3-chlor-5-fluor-benzylamid | | |
| "B63" | 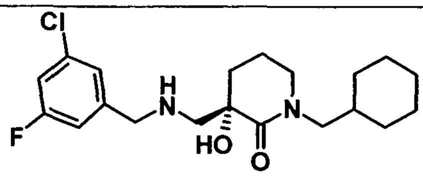 (S)-3-[(3-Chlor-5-fluor-benzylamino)-methyl]-1-cyclohexylmethyl-3-hydroxy-piperidin-2-on | | |
| "B64" | 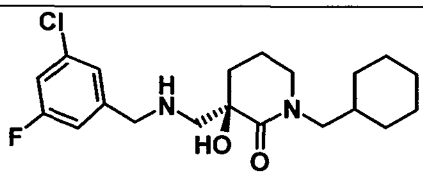 (R)-3-[(3-Chlor-5-fluor-benzylamino)-methyl]-1-cyclohexylmethyl-3-hydroxy-piperidin-2-on | | |
| "B65" | 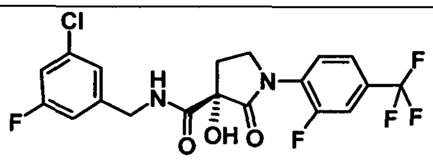 (S)-1-(2-Fluor-4-trifluormethyl-phenyl)-3-hydroxy-2-oxo-pyrrolidin-3-carbonsäure-3-chlor-5-fluor-benzylamid | | 2.43 [449.0] |
| "B66" | 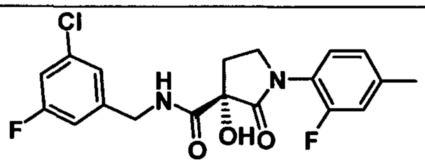 (S)-1-(2-Fluor-4-methyl-phenyl)-3-hydroxy-2-oxo-pyrrolidin-3- | 8.65 (t, *J* = 6.4 Hz, 1H), 7.30 – 7.23 (m, 2H), 7.21 (s, 1H), 7.14 (d, *J* = 11.8 Hz, 1H), 7.10 (d, *J* = 10.6 Hz, 1H), 7.06 (d, *J* = 8.1 Hz, 1H), 6.71 (s, 1H), 4.40 (dd, *J* = 15.9, 6.7 Hz, 1H), | 2.26 [395.1] |

| | | | |
|---|---|---|---|
| | carbonsäure-3-chlor-5-fluor-benzylamid | 4.27 (dd, J = 15.8, 6.0 Hz, 1H), 3.84 – 3.64 (m, 2H), 2.62 (ddd, J = 11.8, 9.3, 5.8 Hz, 1H), 2.33 (s, 3H), 2.25 – 2.07 (m, 1H). | |
| "B67" | 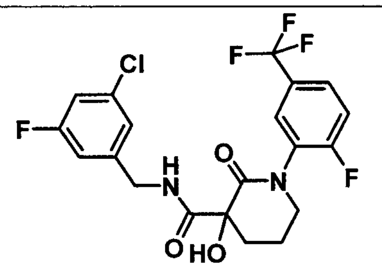 1-(2-Fluor-5-trifluormethyl-phenyl)-3-hydroxy-2-oxo-piperidin-3-carbonsäure-3-chlor-5-fluor-benzylamid | 8.64 (t, J = 6.1 Hz, 1H), 7.80 (t, J = 9.1 Hz, 2H), 7.56 (t, J = 9.1 Hz, 1H), 7.25 (d, J = 8.7 Hz, 1H), 7.21 (s, 1H), 7.11 (d, J = 9.4 Hz, 1H), 6.56 (s, 1H), 4.40 (dd, J = 16.0, 6.8 Hz, 1H), 4.23 (dd, J = 15.9, 5.7 Hz, 1H), 3.72 (d, J = 8.4 Hz, 1H), 3.61 (d, J = 11.8 Hz, 1H), 2.39 – 2.27 (m, 1H), 1.99 (m, 3H). | |
| "B68" | 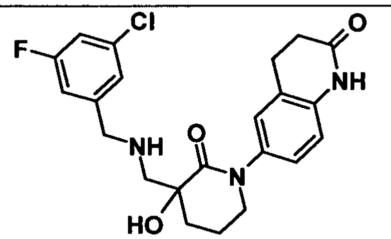 6-{3-[(3-Chlor-5-fluor-benzylamino)-methyl]-3-hydroxy-2-oxo-piperidin-1-yl}-3,4-dihydro-1H-chinolin-2-on | 10.10 (s, 1H), 7.25 (d, J = 10.4 Hz, 2H), 7.18 (d, J = 9.9 Hz, 1H), 7.03 (s, 1H), 6.99 (d, J = 8.4 Hz, 1H), 6.81 (d, J = 8.3 Hz, 1H), 5.15 (s, 1H), 3.74 (q, J = 15.2 Hz, 2H), 3.53 (s, 2H), 2.82 (dd, J = 19.6, 12.0 Hz, 3H), 2.14 (d, J = 10.7 Hz, 1H), 1.99 (s, 2H), 1.76 (s, 2H), 1.22 (s, 2H). | |
| "B69" | 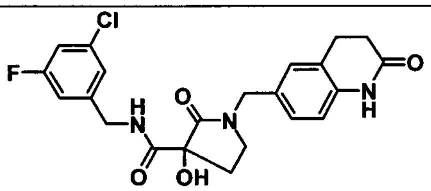 3-Hydroxy-2-oxo-1-(2-oxo-1,2,3,4-tetrahydro-chinolin-6-ylmethyl)-pyrrolidin-3-carbonsäure-3-chlor-5-fluor-benzylamid | 10.06 (s, 1H), 8.65 (t, J = 6.3 Hz, 1H), 7.27 (d, J = 8.7 Hz, 1H), 7.21 (s, 1H), 7.10 (d, J = 9.7 Hz, 1H), 7.03 (s, 1H), 7.00 (d, J = 8.1 Hz, 1H), 6.78 (d, J = 8.0 Hz, 1H), 6.56 (s, 1H), 4.38 (dd, J = 15.4, 6.2 Hz, 2H), 4.30 – 4.20 (m, 2H), 3.23 (dd, J = 12.5, 5.4 Hz, 2H), 2.80 (t, J = | |

| | | | |
|---|---|---|---|
| | | 7.5 Hz, 2H), 2.45 – 2.38 (m, 3H), 2.00 – 1.90 (m, 1H). | |
| "B70" | 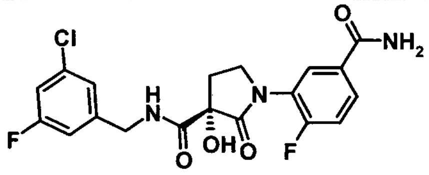 (S)-1-(5-Carbamoyl-2-fluor-phenyl)-3-hydroxy-2-oxo-pyrrolidin-3-carbonsäure-3-chlor-5-fluor-benzylamid | | 1.87 [424.0] |
| "B71" | 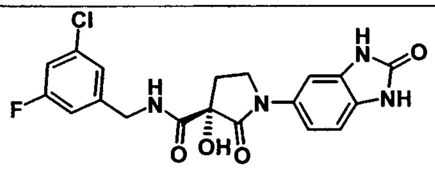 (S)-3-Hydroxy-2-oxo-1-(2-oxo-2,3-dihydro-1H-benzimidazol-5-yl)-pyrrolidin-3-carbonsäure-3-chlor-5-fluor-benzylamid | ** 10.60 (dd, *J* = 21.8, 6.3 Hz, 2H), 8.75 – 8.62 (m, 1H), 7.44 (t, *J* = 2.4 Hz, 1H), 7.26 (dt, *J* = 8.8, 2.1 Hz, 1H), 7.21 (d, *J* = 4.4 Hz, 1H), 7.14 – 7.05 (m, 2H), 6.91 (dd, *J* = 12.7, 9.9 Hz, 1H), 6.68 (d, *J* = 3.7 Hz, 1H), 4.39 (dd, *J* = 15.7, 6.7 Hz, 1H), 4.26 (dd, *J* = 15.7, 6.0 Hz, 1H), 3.87 – 3.77 (m, 2H), 2.62 – 2.54 (m, 1H), 2.11 (dt, *J* = 12.9, 7.5 Hz, 1H). | 1.82 [419.1] |
| "B72" | 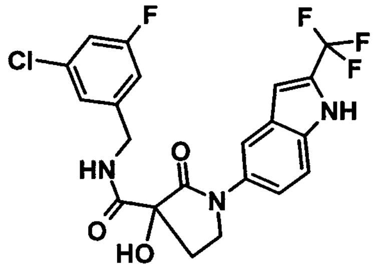 3-Hydroxy-2-oxo-1-(2-trifluormethyl-1H-indol-5-yl)-pyrrolidin-3-carbonsäure-3-chlor-5-fluor-benzylamid | 11.93 (s, 1H), 8.73 (t, *J* = 6.3 Hz, 1H), 7.99 (d, *J* = 1.2 Hz, 1H), 7.87 (s, 1H), 7.54 (d, *J* = 8.8 Hz, 1H), 7.50 (dd, *J* = 8.9, 1.9 Hz, 1H), 7.27 (dt, *J* = 8.8, 2.1 Hz, 1H), 7.22 (s, 1H), 7.11 (d, *J* = 9.6 Hz, 1H), 6.75 (s, 1H), 4.39 (dd, *J* = 15.8, 6.6 Hz, 1H), 4.25 (dd, *J* = 15.8, 5.9 Hz, 1H), 3.90 (t, *J* = 6.8 Hz, 2H), 2.65 – 2.55 (m, 1H), 2.14 (dt, *J* = 12.9, 7.5 Hz, 1H). | 2.41 [470.1] |

| | | | |
|---|---|---|---|
| "B73" | 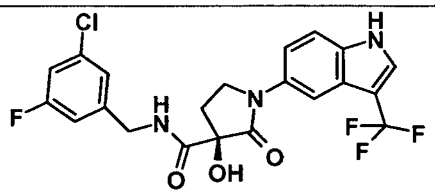<br>(R)-3-Hydroxy-2-oxo-1-(3-trifluormethyl-1H-indol-5-yl)-pyrrolidin-3-carbonsäure-3-chlor-5-fluor-benzylamid | | |
| "B74" | 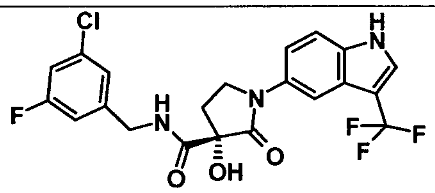<br>(S)-3-Hydroxy-2-oxo-1-(3-trifluormethyl-1H-indol-5-yl)-pyrrolidin-3-carbonsäure-3-chlor-5-fluor-benzylamid | 11.93 (s, 1H), 8.73 (t, *J* = 6.3 Hz, 1H), 7.99 (d, *J* = 1.3 Hz, 1H), 7.86 (s, 1H), 7.54 (d, *J* = 8.8 Hz, 1H), 7.50 (dd, *J* = 8.9, 1.9 Hz, 1H), 7.27 (dd, *J* = 8.7, 2.1 Hz, 1H), 7.22 (s, 1H), 7.11 (d, *J* = 9.5 Hz, 1H), 6.74 (s, 1H), 4.39 (dd, *J* = 15.8, 6.7 Hz, 1H), 4.25 (dd, *J* = 15.7, 6.0 Hz, 1H), 3.90 (t, *J* = 6.8 Hz, 2H), 2.65 – 2.56 (m, 1H), 2.14 (dt, *J* = 12.9, 7.5 Hz, 1H). | |
| "B75" | 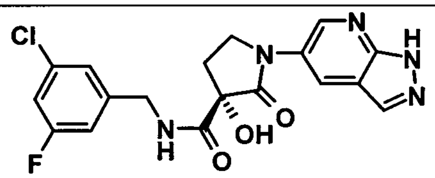<br>(S)-3-Hydroxy-2-oxo-1-(1H-pyrazolo[3,4-b]pyridin-5-yl)-pyrrolidin-3-carbonsäure-3-chlor-5-fluor-benzylamid | | |
| "B76" | 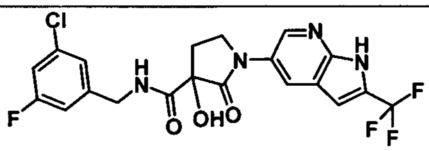<br>3-Hydroxy-2-oxo-1-(2-trifluormethyl-1H-pyrrolo[2,3-b]pyridin- | | |

| | | | |
|---|---|---|---|
| | 5-yl)-pyrrolidin-3-carbonsäure-3-chlor-5-fluor-benzylamid | | |
| "B77" | 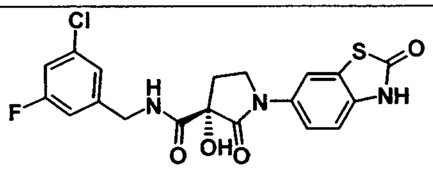 (S)-3-Hydroxy-2-oxo-1-(2-oxo-2,3-dihydro-benzothiazol-6-yl)-pyrrolidin-3-carbonsäure-3-chlor-5-fluor-benzylamid | | 1.99 [436.0] |
| "B78" | 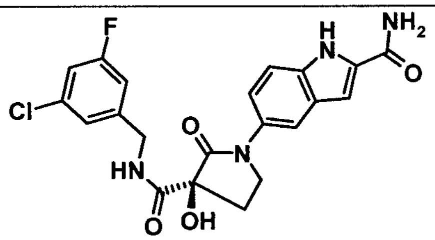 5-[(S)-3-(3-Chlor-5-fluor-benzylcarbamoyl)-3-hydroxy-2-oxo-pyrrolidin-1-yl]-1H-indol-2-carbonsäure-amid | | 1.91 [445.1] |
| "B79" | 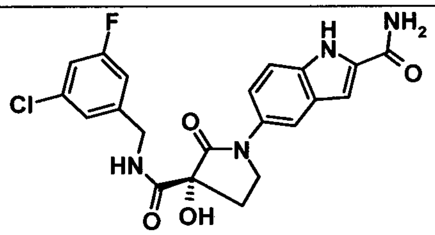 5-[(R)-3-(3-Chlor-5-fluor-benzylcarbamoyl)-3-hydroxy-2-oxo-pyrrolidin-1-yl]-1H-indol-2-carbonsäure-amid | | 1.91 [445.1] |
| "B80" | 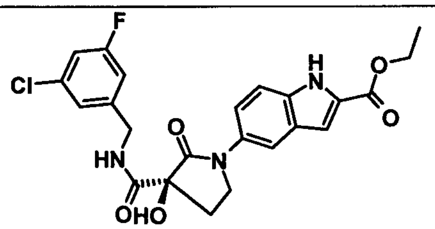 5-[(S)-3-(3-Chlor-5-fluor-benzylcarbamoyl)-3-hydroxy-2- | | 2.27 [474.0] |

| | | | |
|---|---|---|---|
| | oxo-pyrrolidin-1-yl]-1H-indol-2-carbonsäure-ethylester | | |
| "B81" | 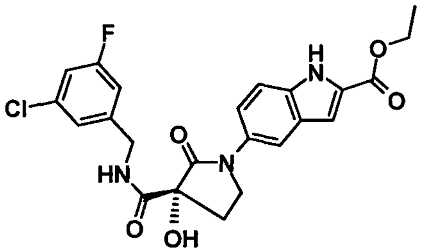 5-[(R)-3-(3-Chlor-5-fluor-benzylcarbamoyl)-3-hydroxy-2-oxo-pyrrolidin-1-yl]-1H-indol-2-carbonsäure-ethylester | | 2.27 [474.1] |
| "B82" | 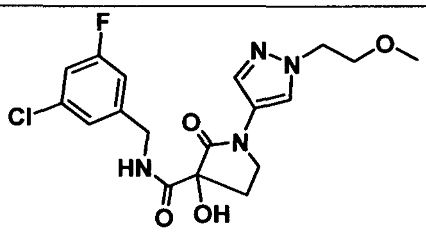 3-Hydroxy-1-[1-(2-methoxy-ethyl)-1H-pyrazol-4-yl]-2-oxo-pyrrolidin-3-carbonsäure-3-chlor-5-fluor-benzylamid | | 1.90 [411.1] |
| "B83" | 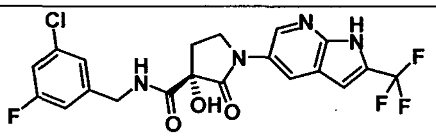 (S)-3-Hydroxy-2-oxo-1-(2-trifluormethyl-1H-pyrrolo[2,3-b]pyridin-5-yl)-pyrrolidin-3-carbonsäure-3-chlor-5-fluor-benzylamid | | 2.22 [471.0] |

| | | | |
|---|---|---|---|
| "B84" | 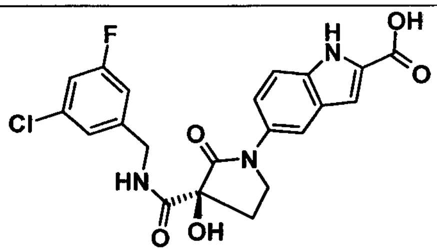 5-[(S)-3-(3-Chlor-5-fluor-benzylcarbamoyl)-3-hydroxy-2-oxo-pyrrolidin-1-yl]-1H-indol-2-carbonsäure | ** 12.94 (s, 1H), 11.78 (s, 1H), 8.68 (t, *J* = 6.4 Hz, 1H), 7.81 (d, *J* = 1.9 Hz, 1H), 7.62 (dd, *J* = 9.0, 2.1 Hz, 1H), 7.44 (d, *J* = 9.0 Hz, 1H), 7.26 (dt, *J* = 8.7, 2.1 Hz, 1H), 7.22 (s, 1H), 7.14 – 7.10 (m, 1H), 7.09 (d, *J* = 1.5 Hz, 1H), 6.69 (s, 1H), 4.40 (dd, *J* = 15.7, 6.7 Hz, 1H), 4.27 (dd, *J* = 15.7, 6.0 Hz, 1H), 3.89 (t, *J* = 6.8 Hz, 2H), 2.68 – 2.56 (m, 1H), 2.14 (dt, *J* = 12.9, 7.6 Hz, 1H). | 1.99 [446.0] |
| "B85" | 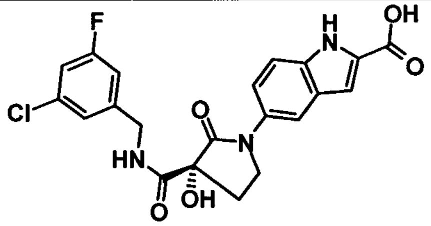 5-[(R)-3-(3-Chlor-5-fluor-benzylcarbamoyl)-3-hydroxy-2-oxo-pyrrolidin-1-yl]-1H-indol-2-carbonsäure | ** ppm = 12.95 (s, 1H), 11.78 (s, 1H), 8.68 (t, J=6.4, 1H), 7.80 (d, J=2.0, 1H), 7.62 (dd, J=9.0, 2.1, 1H), 7.44 (d, J=9.0, 1H), 7.26 (dt, J=8.8, 2.2, 1H), 7.22 (s, 1H), 7.14 - 7.10 (m, 1H), 7.09 (d, J=2.0, 1H), 6.69 (s, 1H), 4.40 (dd, J=15.7, 6.8, 1H), 4.27 (dd, J=15.7, 6.0, 1H), 3.92 - 3.85 (m, 2H), 2.65 - 2.57 (m, 1H), 2.14 (dt, J=12.8, 7.6, 1H). | 1.99 [446.0] |
| "B86" | 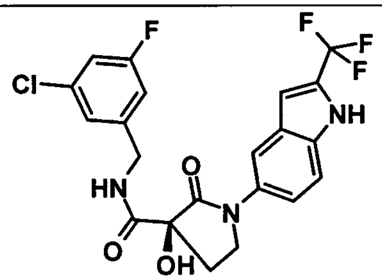 (S)-3-Hydroxy-2-oxo-1-(2-trifluormethyl-1H-indol-5-yl)- | | 2.36 [470.0] |

| | | | |
|---|---|---|---|
| | pyrrolidin-3-carbonsäure-3-chlor-5-fluor-benzylamid | | |
| "B87" | 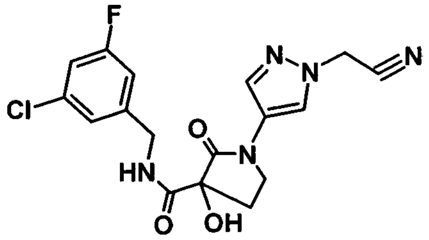 1-(1-Cyanmethyl-1H-pyrazol-4-yl)-3-hydroxy-2-oxo-pyrrolidin-3-carbonsäure-3-chlor-5-fluor-benzylamid | | 1.91 [392.0] |
| "B88" | 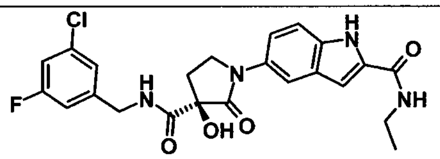 5-[(R)-3-(3-Chlor-5-fluor-benzylcarbamoyl)-3-hydroxy-2-oxo-pyrrolidin-1-yl]-1H-indol-2-carbonsäure-ethylamid | ** 11.59 - 11.55 (m, 1H), 8.71 - 8.64 (m, 1H), 8.46 (t, J=5.7, 1H), 7.80 - 7.75 (m, 1H), 7.52 (dd, J=8.9, 2.0, 1H), 7.42 (d, J=8.9, 1H), 7.28 - 7.24 (m, 1H), 7.22 (s, 1H), 7.14 - 7.10 (m, 1H), 7.10 - 7.07 (m, 1H), 6.68 (s, 1H), 4.41 (dd, J=15.7, 6.7, 1H), 4.27 (dd, J=15.8, 6.0, 1H), 3.93 - 3.86 (m, 2H), 3.35 - 3.30 (m, 2H), 2.65 - 2.58 (m, 1H), 2.14 (dt, J=12.9, 7.6, 1H), 1.15 (t, J=7.2, 3H). | 2.04 [473.1] |
| "B89" | 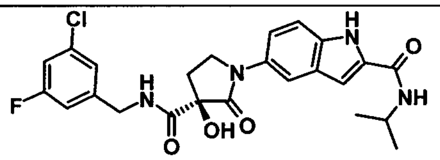 5-[(R)-3-(3-Chlor-5-fluor-benzylcarbamoyl)-3-hydroxy-2-oxo-pyrrolidin-1-yl]-1H-indol-2-carbonsäure-isopropylamid | ** 11.57 - 11.53 (m, 1H), 8.68 (t, J=6.4, 1H), 8.22 (d, J=7.8, 1H), 7.79 - 7.76 (m, 1H), 7.51 (dd, J=8.9, 2.0, 1H), 7.42 (d, J=8.9, 1H), 7.26 (dt, J=8.7, 2.2, 1H), 7.24 - 7.21 (m, 1H), 7.16 - 7.09 (m, 2H), 6.68 (s, 1H), 4.41 (dd, J=15.7, 6.8, 1H), 4.27 (dd, J=15.8, 6.0, 1H), 4.17 - 4.07 (m, J=6.7, | 2.13 [487.1] |

| | | | |
|---|---|---|---|
| | | 1H), 3.93 - 3.86 (m, 2H), 2.65 - 2.57 (m, 1H), 2.14 (dt, J=12.8, 7.5, 1H), 1.19 (d, J=6.6, 6H). | |
| "B90" | 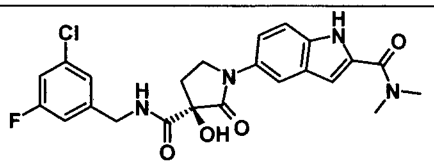 5-[(R)-3-(3-Chlor-5-fluor-benzylcarbamoyl)-3-hydroxy-2-oxo-pyrrolidin-1-yl]-1H-indol-2-carbonsäure-dimethylamid | ** 11.55 (s, 1H), 8.67 (t, J=6.4, 1H), 7.79 (d, J=2.0, 1H), 7.54 (dd, J=8.9, 2.1, 1H), 7.43 (d, J=8.8, 1H), 7.26 (dt, J=8.8, 2.2, 1H), 7.23 - 7.20 (m, 1H), 7.15 - 7.09 (m, 1H), 6.88 (d, J=2.0, 1H), 6.69 (s, 1H), 4.40 (dd, J=15.7, 6.8, 1H), 4.27 (dd, J=15.7, 6.0, 1H), 3.93 - 3.85 (m, 2H), 3.28 - 2.93 (m, 6H), 2.65 - 2.57 (m, 1H), 2.19 - 2.09 (m, 1H). | 2.04 [473.1] |
| "B91" | 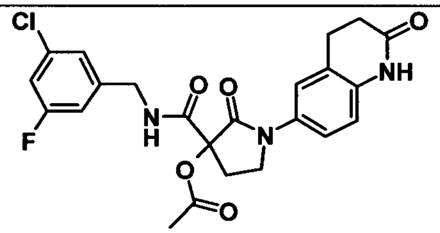 Essigsäure-3-(3-chlor-5-fluor-benzylcarbamoyl)-2-oxo-1-(2-oxo-1,2,3,4-tetrahydro-chinolin-6-yl)-pyrrolidin-3-yl-ester | ** 10.09 (s, 1H), 8.93 (t, J=6.2, 1H), 7.47 (d, J=2.5, 1H), 7.39 (dd, J=8.6, 2.5, 1H), 7.26 (dt, J=8.8, 2.2, 1H), 7.19 - 7.16 (m, 1H), 7.07 - 7.03 (m, 1H), 6.87 (d, J=8.6, 1H), 4.42 - 4.26 (m, 2H), 3.89 - 3.81 (m, 2H), 2.92 - 2.85 (m, 3H), 2.47 - 2.42 (m, 2H), 2.40 - 2.32 (m, 1H), 2.19 (s, 3H). | 2.11 [474.0] |
| "B92" | 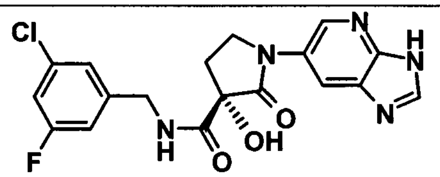 (S)-3-Hydroxy-1-(3H-imidazo[4,5-b]pyridin-6-yl)-2- | ** 8.73 (t, *J* = 6.4 Hz, 1H), 8.68 (d, *J* = 2.3 Hz, 1H), 8.55 (s, 1H), 8.37 (d, *J* = 2.3 Hz, 1H), 7.26 (dt, *J* = 8.7, 2.1 Hz, 1H), 7.22 (s, 1H), 7.12 (d, *J* = 9.6 Hz, 1H), 4.41 (dd, *J* = 15.7, 6.7 Hz, 1H), 4.27 (dd, *J* = 15.7, 6.0 Hz, 1H), 3.97 (dd, *J* = 14.6, 8.3 | 1.70 [404.0] |

| | | | |
|---|---|---|---|
| | oxo-pyrrolidin-3-carbonsäure-3-chlor-5-fluor-benzylamid | Hz, 2H), 2.70 – 2.60 (m, 1H), 2.19 (dt, *J* = 13.0, 7.5 Hz, 1H). | |
| "B93" | 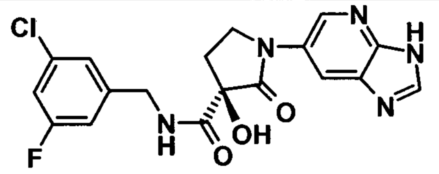 (R)-3-Hydroxy-1-(3H-imidazo[4,5-b]pyridin-6-yl)-2-oxo-pyrrolidin-3-carbonsäure-3-chlor-5-fluor-benzylamid | | |
| "B94" | 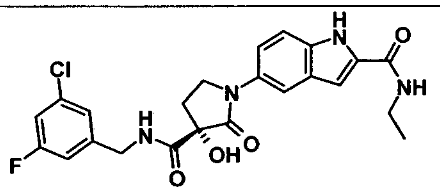 5-[(S)-3-(3-Chlor-5-fluor-benzylcarbamoyl)-3-hydroxy-2-oxo-pyrrolidin-1-yl]-1H-indol-2-carbonsäure-ethylamid | 11.57 (s, 1H), 8.68 (t, J=6.4, 1H), 8.47 (t, J=5.6, 1H), 7.77 (d, J=2.0, 1H), 7.52 (dd, J=8.9, 2.1, 1H), 7.42 (d, J=8.9, 1H), 7.26 (dt, J=8.7, 2.2, 1H), 7.23 (s, 1H), 7.14 - 7.10 (m, 1H), 7.09 (d, J=2.0, 1H), 6.68 (s, 1H), 4.40 (dd, J=15.8, 6.8, 1H), 4.26 (dd, J=15.8, 6.0, 1H), 3.93 - 3.85 (m, 2H), 3.35 - 3.31 (m, 2H), 2.61 (dt, J=12.2, 5.8, 1H), 2.14 (dt, J=12.8, 7.5, 1H), 1.15 (t, J=7.2, 3H). | 2.05 [473.1] |
| "B95" | 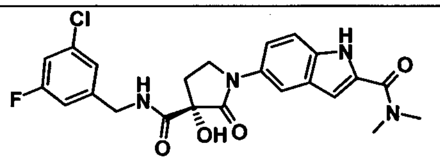 5-[(S)-3-(3-Chlor-5-fluor-benzylcarbamoyl)-3-hydroxy-2-oxo-pyrrolidin-1-yl]-1H-indol-2-carbonsäure-dimethylamid | 11.56 (s, 1H), 8.68 (t, J=6.4, 1H), 7.79 (d, J=2.0, 1H), 7.54 (dd, J=8.9, 2.1, 1H), 7.43 (d, J=8.9, 1H), 7.26 (dt, J=8.7, 2.2, 1H), 7.23 - 7.21 (m, 1H), 7.14 - 7.09 (m, 1H), 6.90 - 6.87 (m, 1H), 6.69 (s, 1H), 4.40 (dd, J=15.8, 6.7, 1H), 4.26 (dd, J=15.7, 6.0, 1H), | 2.04 [473.1] |

| | | | |
|---|---|---|---|
| | | 3.93 - 3.86 (m, 2H), 3.30 - 2.98 (m, 6H), 2.66 - 2.56 (m, 1H), 2.19 - 2.09 (m, 1H). | |
| "B96" | 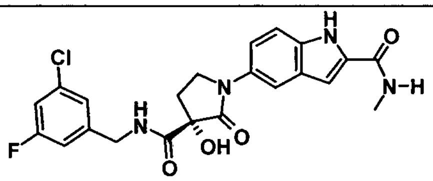 5-[(S)-3-(3-Chlor-5-fluor-benzylcarbamoyl)-3-hydroxy-2-oxo-pyrrolidin-1-yl]-1H-indol-2-carbonsäure-methylamid | ** 11.60 - 11.57 (m, 1H), 8.67 (t, J=6.4, 1H), 8.46 - 8.41 (m, 1H), 7.78 - 7.76 (m, 1H), 7.52 (dd, J=8.9, 2.1, 1H), 7.42 (d, J=8.9, 1H), 7.26 (dt, J=8.7, 2.2, 1H), 7.23 - 7.21 (m, 1H), 7.14 - 7.10 (m, 1H), 7.06 (d, J=2.0, 1H), 6.67 (s, 1H), 4.40 (dd, J=15.7, 6.8, 1H), 4.27 (dd, J=15.8, 6.0, 1H), 3.89 (t, J=6.8, 2H), 2.81 (d, J=4.5, 3H), 2.65 - 2.57 (m, 1H), 2.17 - 2.10 (m, 1H). | 1.97 [459.0] |
| "B97" | 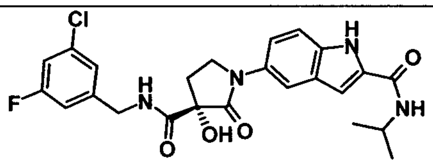 5-[(S)-3-(3-Chlor-5-fluor-benzylcarbamoyl)-3-hydroxy-2-oxo-pyrrolidin-1-yl]-1H-indol-2-carbonsäure-isopropylamid | ** 11.56 - 11.53 (m, 1H), 8.67 (t, J=6.4, 1H), 8.21 (d, J=7.8, 1H), 7.78 (d, J=2.0, 1H), 7.51 (dd, J=8.9, 2.1, 1H), 7.44 - 7.40 (m, 1H), 7.26 (dt, J=8.7, 2.2, 1H), 7.23 - 7.22 (m, 1H), 7.14 - 7.12 (m, 1H), 7.12 - 7.09 (m, 1H), 6.67 (s, 1H), 4.40 (dd, J=15.7, 6.7, 1H), 4.27 (dd, J=15.8, 6.0, 1H), 4.17 - 4.09 (m, 1H), 3.92 - 3.86 (m, 2H), 2.65 - 2.58 (m, 1H), 2.18 - 2.10 (m, 1H), 1.19 (d, J=6.6, 6H). | 2.14 [487.1] |
| "B98" | 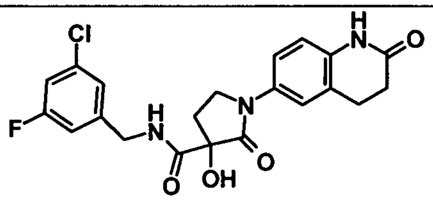 | ** 10.07 (s, 1H), 8.67 (t, J=6.4, 1H), 7.48 (d, J=2.4, 1H), 7.44 (dd, J=8.6, 2.5, 1H), 7.26 (dt, J=8.8, 2.2, 1H), 7.23 - 7.19 | |

| | | | |
|---|---|---|---|
| | 3-Hydroxy-2-oxo-1-(2-oxo-1,2,3,4-tetrahydro-chinolin-6-yl)-pyrrolidin-3-carbonsäure-3-chlor-5-fluor-benzylamid | (m, 1H), 7.13 - 7.07 (m, 1H), 6.86 (d, J=8.6, 1H), 6.69 (s, 1H), 4.39 (dd, J=15.7, 6.7, 1H), 4.25 (dd, J=15.7, 6.0, 1H), 3.84 - 3.75 (m, 2H), 2.88 (t, J=7.5, 2H), 2.61 - 2.54 (m, 1H), 2.47 - 2.41 (m, 2H), 2.14 - 2.06 (m, 1H). | |
| "B99" | 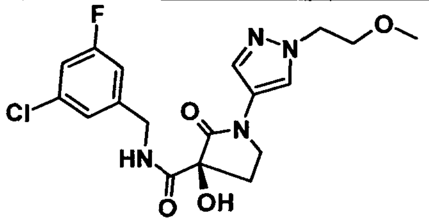 (S)-3-Hydroxy-1-[1-(2-methoxy-ethyl)-1H-pyrazol-4-yl]-2-oxo-pyrrolidin-3-carbonsäure-3-chlor-5-fluor-benzylamid | | 1.90 [411.1] |
| "B100" | 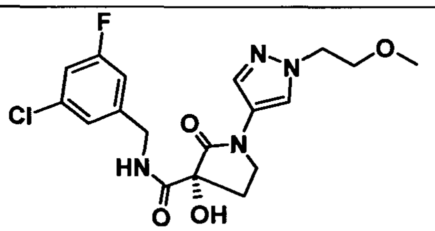 (R)-3-Hydroxy-1-[1-(2-methoxy-ethyl)-1H-pyrazol-4-yl]-2-oxo-pyrrolidin-3-carbonsäure-3-chlor-5-fluor-benzylamid | | 1.90 [411.1] |
| "B101" | 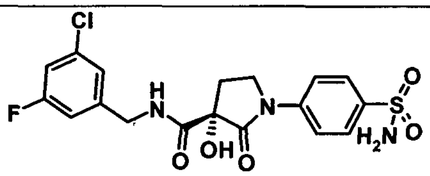 (S)-3-Hydroxy-2-oxo-1-(4-sulfamoyl-phenyl)-pyrrolidin-3- | | 1.96 [442.0] |

| | | | |
|---|---|---|---|
| | carbonsäure-3-chlor-5-fluor-benzylamid | | |
| "B102" | 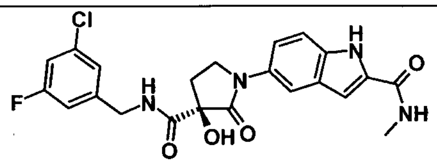 5-[(R)-3-(3-Chlor-5-fluor-benzylcarbamoyl)-3-hydroxy-2-oxo-pyrrolidin-1-yl]-1H-indol-2-carbonsäure-methylamid | ** 11.59 (s, 1H), 8.67 (t, J=6.4, 1H), 8.44 (q, J=4.5, 1H), 7.77 (d, J=2.0, 1H), 7.52 (dd, J=8.9, 2.1, 1H), 7.42 (d, J=8.9, 1H), 7.26 (dt, J=8.8, 2.2, 1H), 7.22 (s, 1H), 7.15 - 7.09 (m, 1H), 7.08 - 7.03 (m, 1H), 6.67 (s, 1H), 4.40 (dd, J=15.7, 6.8, 1H), 4.27 (dd, J=15.7, 6.0, 1H), 3.93 - 3.84 (m, 2H), 2.81 (d, J=4.5, 3H), 2.65 - 2.57 (m, 1H), 2.19 - 2.09 (m, 1H). | 1.97 [459.1] |
| "B103" | 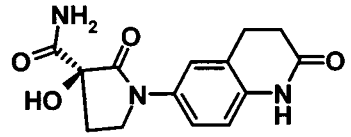 (S)-3-Hydroxy-2-oxo-1-(2-oxo-1,2,3,4-tetrahydro-chinolin-6-yl)-pyrrolidin-3-carbonsäure-amid | 10.07 (s, 1H), 7.48 (d, J=2.4, 1H), 7.46 - 7.38 (m, 2H), 7.31 - 7.27 (m, 1H), 6.86 (d, J=8.6, 1H), 6.43 (s, 1H), 3.83 - 3.72 (m, 2H), 2.88 (t, J=7.5, 2H), 2.56 - 2.51 (m, 1H), 2.47 - 2.40 (m, 2H), 2.10 - 2.00 (m, 1H). | 1.18 [290.1] |
| "B104" | 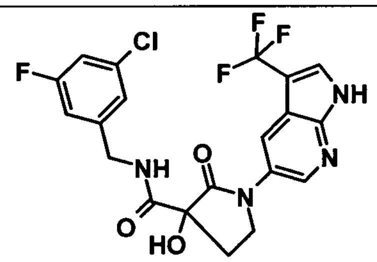 3-Hydroxy-2-oxo-1-(3-trifluormethyl-1H-pyrrolo[2,3-b]pyridin-5-yl)-pyrrolidin-3-carbonsäure-3-chlor-5-fluor-benzylamid | 12.61 (s, 1H), 8.78 (t, *J* = 6.4 Hz, 1H), 8.61 (d, *J* = 2.2 Hz, 1H), 8.34 (s, 1H), 8.21 (s, 1H), 7.27 (d, *J* = 8.8 Hz, 1H), 7.22 (s, 1H), 7.11 (d, *J* = 9.6 Hz, 1H), 6.82 (s, 1H), 4.39 (dd, *J* = 15.8, 6.7 Hz, 1H), 4.26 (dd, *J* = 15.8, 5.9 Hz, 1H), 3.97 (dd, *J* = 8.8, 5.4 Hz, 2H), 2.68 – 2.58 (m, 1H), 2.23 – 2.11 (m, 1H). | |

| | | | |
|---|---|---|---|
| "B105" | 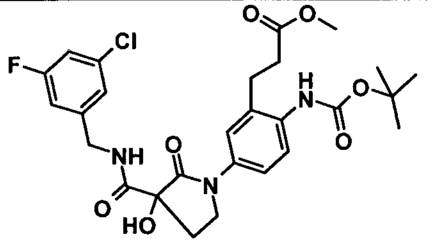 3-{2-tert-Butoxycarbonylamino-5-[3-(3-chlor-5-fluor-benzylcarbamoyl)-3-hydroxy-2-oxo-pyrrolidin-1-yl]-phenyl}-propionsäure-methylester | 8.73 (t, *J* = 6.4 Hz, 1H), 8.61 (s, 1H), 7.53 (dd, *J* = 8.7, 2.5 Hz, 1H), 7.49 (d, *J* = 2.5 Hz, 1H), 7.26 (dd, *J* = 8.6, 3.8 Hz, 2H), 7.20 (s, 1H), 7.09 (d, *J* = 9.6 Hz, 1H), 6.75 (s, 1H), 4.37 (dd, *J* = 15.8, 6.6 Hz, 1H), 4.24 (dd, *J* = 15.7, 5.9 Hz, 1H), 3.82 (t, *J* = 6.8 Hz, 2H), 3.59 (s, 3H), 2.83 (t, *J* = 7.8 Hz, 2H), 2.63 – 2.53 (m, 4H), 2.16 – 2.05 (m, 1H), 1.44 (s, 9H). | |
| "B106" | 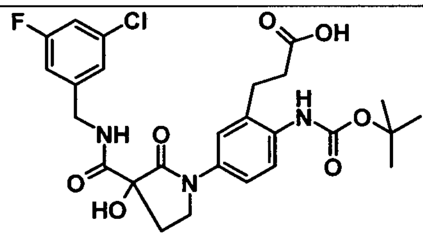 3-{2-tert-Butoxycarbonylamino-5-[3-(3-chlor-5-fluor-benzylcarbamoyl)-3-hydroxy-2-oxo-pyrrolidin-1-yl]-phenyl}-propionsäure | 8.73 (t, *J* = 6.5 Hz, 1H), 7.48 (d, *J* = 8.8 Hz, 1H), 7.45 (s, 1H), 7.34 (d, *J* = 8.7 Hz, 1H), 7.27 (d, *J* = 8.8 Hz, 1H), 7.20 (s, 1H), 7.10 (d, *J* = 9.5 Hz, 1H), 6.77 (s, 1H), 4.37 (dd, *J* = 15.8, 6.7 Hz, 1H), 4.24 (dd, *J* = 15.8, 6.0 Hz, 1H), 3.81 (t, *J* = 6.7 Hz, 2H), 2.73 (t, *J* = 7.3 Hz, 2H), 2.61 – 2.52 (m, 1H), 2.38 (t, *J* = 6.9 Hz, 2H), 2.14 – 2.04 (m, 1H), 1.44 (s, 9H). | |
| "B107" | 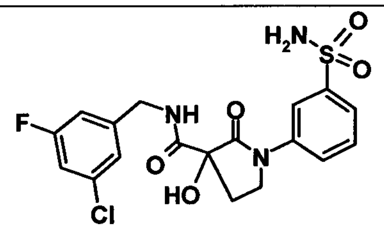 3-Hydroxy-2-oxo-1-(3-sulfamoyl-phenyl)-pyrrolidin-3-carbonsäure-3-chlor-5-fluor-benzylamid | ** 8.77 – 8.68 (m, 1H), 8.23 (t, *J* = 1.8 Hz, 1H), 7.90 – 7.83 (m, 1H), 7.67 – 7.57 (m, 2H), 7.39 (s, 2H), 7.26 (dt, *J* = 8.8, 2.1 Hz, 1H), 7.21 (s, 1H), 7.10 (d, *J* = 9.0 Hz, 1H), 6.82 (s, 1H), 4.38 (dd, *J* = 15.7, 6.7 Hz, 1H), 4.27 (dd, *J* = 15.7, 6.1 Hz, 1H), 3.89 (dd, *J* = 14.2, 8.1 Hz, 2H), 2.62 (dt, *J* = 11.8, 5.7 Hz, | 1.96 [442.0] |

| | | | |
|---|---|---|---|
| | | 1H), 2.17 (dt, *J* = 13.0, 7.6 Hz, 1H). | |
| "B108" | 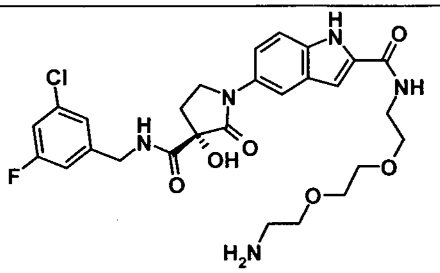 5-[(S)-3-(3-Chlor-5-fluor-benzylcarbamoyl)-3-hydroxy-2-oxo-pyrrolidin-1-yl]-1H-indol-2-carbonsäure-{2-[2-(2-amino-ethoxy)-ethoxy]-ethyl}-amid | ** 8.68 (t, J=6.4, 1H), 8.56 (t, J=5.7, 1H), 8.32 - 8.29 (m, 1H), 7.78 (d, J=2.1, 1H), 7.53 (dd, J=8.9, 2.1, 1H), 7.43 (d, J=8.9, 1H), 7.26 (dt, J=8.7, 2.2, 1H), 7.23 - 7.21 (m, 1H), 7.14 - 7.10 (m, 2H), 6.70 (s, 1H), 4.40 (dd, J=15.8, 6.8, 1H), 4.26 (dd, J=15.8, 6.0, 1H), 3.93 - 3.85 (m, 2H), 3.60 - 3.54 (m, 6H), 3.49 (t, J=5.5, 2H), 3.48 - 3.43 (m, 2H), 2.81 (t, J=5.4, 2H), 2.65 - 2.58 (m, 1H), 2.18 - 2.10 (m, 1H). | 1.69 [576.2] |
| "B109" | 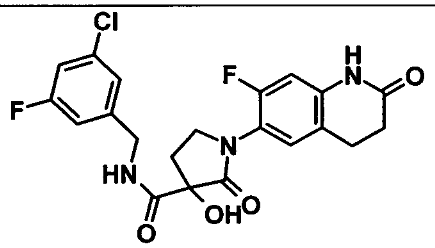 1-(7-Fluor-2-oxo-1,2,3,4-tetrahydro-chinolin-6-yl)-3-hydroxy-2-oxo-pyrrolidin-3-carbonsäure-3-chlor-5-fluor-benzylamid | 10.24 (s, 1H), 8.71 (t, *J* = 6.5 Hz, 1H), 7.27 (d, *J* = 8.8 Hz, 1H), 7.20 (s, 1H), 7.18 (d, *J* = 8.1 Hz, 1H), 7.10 (d, *J* = 9.5 Hz, 1H), 6.75 (s, 1H), 6.72 (d, *J* = 11.5 Hz, 1H), 4.39 (dd, *J* = 15.8, 6.8 Hz, 1H), 4.24 (dd, *J* = 15.7, 5.8 Hz, 1H), 3.70 (dd, *J* = 12.9, 5.5 Hz, 2H), 2.85 (t, *J* = 7.5 Hz, 2H), 2.59 (d, *J* = 4.6 Hz, 1H), 2.45 (d, *J* = 7.2 Hz, 2H), 2.15 (dd, *J* = 13.7, 6.5 Hz, 1H). | |
| "B110" | 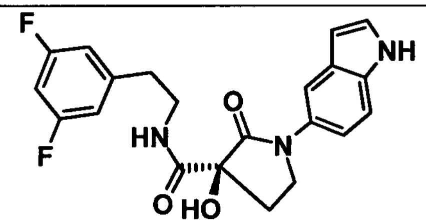 | | 2.07 [400.1] |

| | | | |
|---|---|---|---|
| | (S)-3-Hydroxy-1-(1H-indol-5-yl)-2-oxo-pyrrolidin-3-carbonsäure-[2-(3,5-difluor-phenyl)-ethyl]-amid | | |
| "B111" | 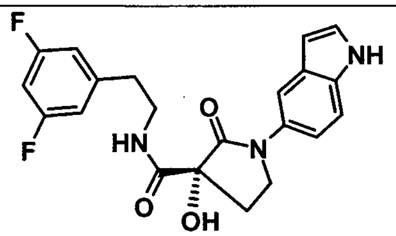 (R)-3-Hydroxy-1-(1H-indol-5-yl)-2-oxo-pyrrolidin-3-carbonsäure-[2-(3,5-difluor-phenyl)-ethyl]-amid | | 2.07 [400.1] |
| "B112" | 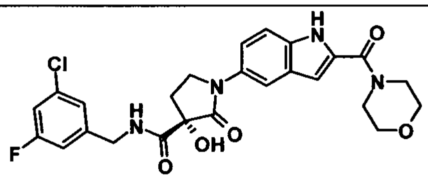 (S)-3-Hydroxy-1-[2-(morpholin-4-carbonyl)-1H-indol-5-yl]-2-oxo-pyrrolidin-3-carbonsäure-3-chlor-5-fluor-benzylamid | ** 11.65 - 11.59 (m, 1H), 8.68 (t, J=6.4, 1H), 7.79 (d, J=2.0, 1H), 7.55 (dd, J=8.9, 2.1, 1H), 7.43 (d, J=8.9, 1H), 7.27 (dt, J=8.8, 2.2, 1H), 7.24 - 7.21 (m, 1H), 7.14 - 7.09 (m, 1H), 6.84 - 6.81 (m, 1H), 6.69 (s, 1H), 4.40 (dd, J=15.7, 6.8, 1H), 4.27 (dd, J=15.8, 6.0, 1H), 3.91 - 3.85 (m, 2H), 3.76 (s, 4H), 3.69 - 3.63 (m, 4H), 2.65 - 2.58 (m, 1H), 2.18 - 2.10 (m, 1H). | 2.01 [515.1] |
| "B113" | 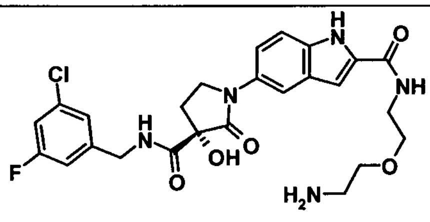 5-[(S)-3-(3-Chlor-5-fluor-benzylcarbamoyl)-3-hydroxy-2-oxo-pyrrolidin-1-yl]-1H-indol-2- | ** 12.17 - 11.24 (m, 1H), 8.69 (t, J=6.4, 1H), 8.62 (t, J=5.6, 1H), 8.36 (s, 1H), 7.79 (d, J=2.0, 1H), 7.53 (dd, J=8.9, 2.1, 1H), 7.46 - 7.42 (m, 1H), 7.26 (dt, J=8.7, 2.3, 1H), 7.24 - 7.21 (m, 1H), 7.14 - 7.10 (m, 2H), 7.01 - 6.42 (m, 1H), 4.41 | 1.63 [532.1] |

| | | | |
|---|---|---|---|
| | carbonsäure-[2-(2-amino-ethoxy)-ethyl]-amid | (dd, J=15.8, 6.8, 1H), 4.27 (dd, J=15.8, 6.0, 1H), 3.93 - 3.86 (m, 2H), 3.60 - 3.56 (m, 4H), 3.51 - 3.47 (m, 2H), 2.93 - 2.88 (m, 2H), 2.65 - 2.57 (m, 1H), 2.15 (dt, J=12.8, 7.6, 1H). | |
| "B114" | 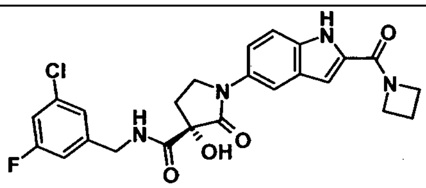 (S)-1-[2-(Azetidin-1-carbonyl)-1H-indol-5-yl]-3-hydroxy-2-oxo-pyrrolidin-3-carbonsäure-3-chlor-5-fluor-benzylamid | ** 11.61 (s, 1H), 8.68 (t, J=6.4, 1H), 7.80 (d, J=2.0, 1H), 7.56 (dd, J=8.9, 2.1, 1H), 7.44 (d, J=8.9, 1H), 7.27 (dt, J=8.8, 2.2, 1H), 7.24 - 7.21 (m, 1H), 7.14 - 7.10 (m, 1H), 6.81 (d, J=1.7, 1H), 6.69 (s, 1H), 4.58 - 4.46 (m, 2H), 4.40 (dd, J=15.7, 6.8, 1H), 4.27 (dd, J=15.8, 6.0, 1H), 4.15 - 4.03 (m, 2H), 3.91 - 3.86 (m, 2H), 2.65 - 2.58 (m, 1H), 2.39 - 2.31 (m, 2H), 2.18 - 2.10 (m, 1H). | 2.06 [485.1] |
| "B115" | 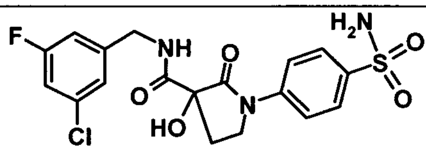 3-Hydroxy-2-oxo-1-(4-sulfamoyl-phenyl)-pyrrolidin-3-carbonsäure-3-chlor-5-fluor-benzylamid | 8.75 (t, *J* = 6.4 Hz, 1H), 7.93 – 7.87 (m, 2H), 7.86 – 7.81 (m, 2H), 7.30 (d, *J* = 11.8 Hz, 2H), 7.27 (dt, *J* = 8.8, 2.2 Hz, 1H), 7.21 (s, 1H), 7.10 (d, *J* = 9.7 Hz, 1H), 6.83 (s, 1H), 4.39 (dd, *J* = 15.8, 6.7 Hz, 1H), 4.26 (dd, *J* = 15.7, 6.0 Hz, 1H), 3.96 – 3.85 (m, 2H), 2.61 (ddd, *J* = 11.8, 6.9, 4.7 Hz, 1H), 2.16 (dt, *J* = 13.0, 7.6 Hz, 1H). | 1.95 [442.0] |

| | | | |
|---|---|---|---|
| "B116" | 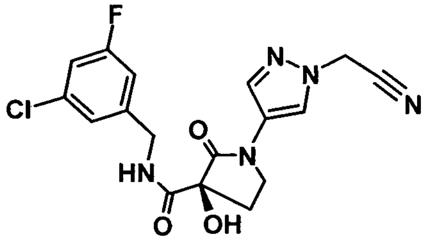 (S)-1-(1-Cyanmethyl-1H-pyrazol-4-yl)-3-hydroxy-2-oxo-pyrrolidin-3-carbonsäure-3-chlor-5-fluor-benzylamid | | 1.91 [392.0] |
| "B117" | 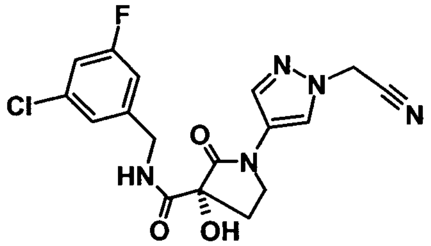 (R)-1-(1-Cyanmethyl-1H-pyrazol-4-yl)-3-hydroxy-2-oxo-pyrrolidin-3-carbonsäure-3-chlor-5-fluor-benzylamid | | 1.91 [392.0] |
| "B118" | 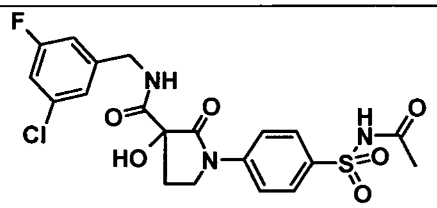 1-(4-Acetylsulfamoyl-phenyl)-3-hydroxy-2-oxo-pyrrolidin-3-carbonsäure-3-chlor-5-fluor-benzylamid | 8.76 (t, $J$ = 6.3 Hz, 1H), 7.89 (s, 4H), 7.27 (dt, $J$ = 8.8, 2.1 Hz, 1H), 7.20 (s, 1H), 7.10 (d, $J$ = 9.6 Hz, 1H), 6.90 (s, 1H), 4.38 (dd, $J$ = 15.6, 6.7 Hz, 1H), 4.26 (dd, $J$ = 15.7, 6.0 Hz, 1H), 3.97 – 3.84 (m, 2H), 2.60 (ddd, $J$ = 11.9, 7.3, 4.4 Hz, 1H), 2.18 (dt, $J$ = 12.9, 7.7 Hz, 1H), 1.86 (d, $J$ = 8.5 Hz, 3H). | 2.01 [484.0] |
| "B119" | 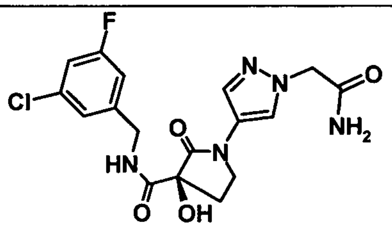 | ** 8.67 (t, $J$ = 6.4 Hz, 1H), 8.04 (s, 1H), 7.67 (s, 1H), 7.42 (s, 1H), 7.26 (dt, $J$ = 8.7, 2.0 Hz, 1H), 7.21 (s, 2H), 7.10 (d, | 1.72 [410.1] |

| | | | |
|---|---|---|---|
| | (S)-1-(1-Carbamoylmethyl-1H-pyrazol-4-yl)-3-hydroxy-2-oxo-pyrrolidin-3-carbonsäure-3-chlor-5-fluor-benzylamid | *J* = 9.5 Hz, 1H), 6.68 (s, 1H), 4.75 (s, 2H), 4.38 (dd, *J* = 15.7, 6.7 Hz, 1H), 4.25 (dd, *J* = 15.7, 6.0 Hz, 1H), 3.77 – 3.64 (m, 2H), 2.67 – 2.57 (m, 1H), 2.15 (ddd, *J* = 13.1, 8.5, 6.7 Hz, 1H). | |
| "B120" | 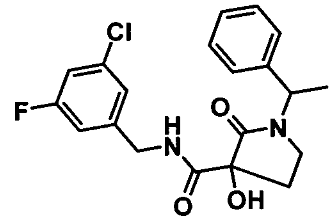 3-Hydroxy-2-oxo-1-(1-phenyl-ethyl)-pyrrolidin-3-carbonsäure-3-chlor-5-fluor-benzylamid | 8.62 (t, *J* = 6.2 Hz, 1H), 7.39 – 7.24 (m, 6H), 7.20 (s, 1H), 7.10 (d, *J* = 9.7 Hz, 1H), 6.54 (d, *J* = 15.9 Hz, 1H), 5.29 – 5.14 (m, 1H), 4.36 (dd, *J* = 15.7, 6.7 Hz, 1H), 4.23 (dd, *J* = 15.8, 5.9 Hz, 1H), 3.36 (dd, *J* = 12.8, 4.0 Hz, 1H), 2.99 (dd, *J* = 16.0, 7.7 Hz, 1H), 2.46 – 2.35 (m, 1H), 1.92 (ddd, *J* = 28.5, 14.1, 7.4 Hz, 1H), 1.47 (dd, *J* = 19.9, 7.1 Hz, 3H). | |
| "B121" | 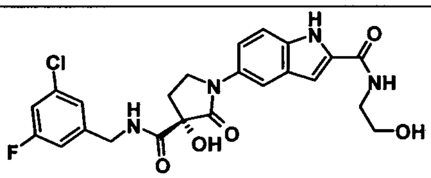 5-[(S)-3-(3-Chlor-5-fluor-benzylcarbamoyl)-3-hydroxy-2-oxo-pyrrolidin-1-yl]-1H-indol-2-carbonsäure-(2-hydroxy-ethyl)-amid | ** 11.59 (s, 1H), 8.67 (t, *J* = 6.4 Hz, 1H), 8.45 (t, *J* = 5.7 Hz, 1H), 7.78 (d, *J* = 1.9 Hz, 1H), 7.52 (dd, *J* = 8.9, 2.1 Hz, 1H), 7.42 (d, *J* = 8.9 Hz, 1H), 7.26 (dt, *J* = 8.7, 2.1 Hz, 1H), 7.22 (s, 1H), 7.12 (dd, *J* = 5.2, 3.6 Hz, 2H), 6.68 (s, 1H), 4.74 (s, 1H), 4.41 (dd, *J* = 15.7, 6.8 Hz, 1H), 4.27 (dd, *J* = 15.7, 6.0 Hz, 1H), 3.93 – 3.84 (m, 2H), 3.53 (d, *J* = 3.8 Hz, 2H), 3.36 (q, *J* = 6.1 Hz, 2H), 2.67 – 2.56 (m, 1H), 2.14 (dt, *J* = 12.9, 7.5 Hz, 1H). | 1.88 [489.1] |

| | | | |
|---|---|---|---|
| "B122" | 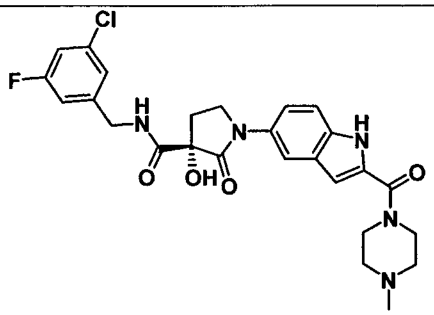 (S)-3-Hydroxy-1-[2-(4-methyl-piperazin-1-carbonyl)-1H-indol-5-yl]-2-oxo-pyrrolidin-3-carbonsäure-3-chlor-5-fluor-benzylamid | ** 11.59 (s, 1H), 8.67 (t, *J* = 6.4 Hz, 1H), 8.13 (s, 1H), 7.78 (d, *J* = 1.9 Hz, 1H), 7.54 (dd, *J* = 8.9, 2.1 Hz, 1H), 7.42 (d, *J* = 8.9 Hz, 1H), 7.29 – 7.23 (m, 1H), 7.22 (s, 1H), 7.12 (d, *J* = 9.6 Hz, 1H), 6.79 (d, *J* = 1.6 Hz, 1H), 4.40 (dd, *J* = 15.7, 6.8 Hz, 1H), 4.27 (dd, *J* = 15.7, 6.0 Hz, 1H), 3.88 (dt, *J* = 9.3, 5.7 Hz, 2H), 2.62 (dt, *J* = 12.0, 5.7 Hz, 1H), 2.44 – 2.35 (m, 4H), 2.23 (s, 3H), 2.19 – 2.08 (m, 1H). | 1.64 [528.2] |
| "B123" | 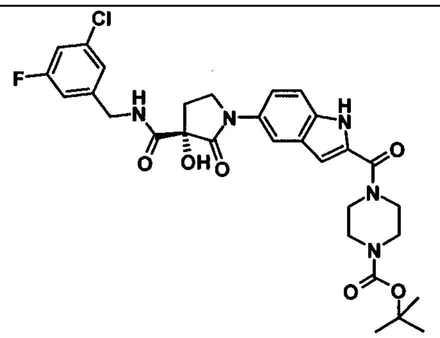 4-{5-[(S)-3-(3-Chlor-5-fluor-benzylcarbamoyl)-3-hydroxy-2-oxo-pyrrolidin-1-yl]-1H-indol-2-carbonyl}-piperazin-1-carbonsäure-tert-butylester | ** 11.63 - 11.59 (m, 1H), 8.67 (t, J=6.4, 1H), 7.80 (d, J=2.0, 1H), 7.55 (dd, J=8.9, 2.1, 1H), 7.43 (d, J=8.9, 1H), 7.26 (dt, J=8.7, 2.2, 1H), 7.23 - 7.21 (m, 1H), 7.14 - 7.10 (m, 1H), 6.85 - 6.82 (m, 1H), 6.68 (s, 1H), 4.40 (dd, J=15.7, 6.8, 1H), 4.27 (dd, J=15.8, 6.0, 1H), 3.92 - 3.86 (m, 2H), 3.74 (s, 4H), 3.47 - 3.40 (m, 4H), 2.65 - 2.58 (m, 1H), 2.18 - 2.11 (m, 1H), 1.43 (s, 9H). | 2.34 [558.0 + 514.9] |
| "B124" | 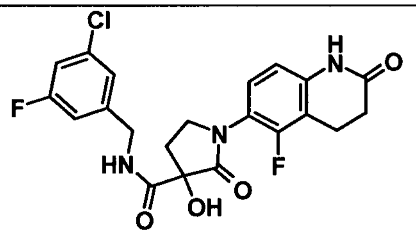 1-(5-Fluor-2-oxo-1,2,3,4-tetrahydro-chinolin-6-yl)-3- | 10.34 (s, 1H), 8.69 (t, *J* = 6.3 Hz, 1H), 7.27 (dt, *J* = 8.8, 2.2 Hz, 1H), 7.20 (s, 1H), 7.15 (t, *J* = 8.3 Hz, 1H), 7.10 (d, *J* = 9.7 Hz, 1H), 6.74 (s, 1H), 6.71 (d, *J* = 8.5 Hz, 1H), 4.39 (dd, *J* = 15.9, 6.6 Hz, 1H), 4.24 (dd, *J* = | |

| | | | |
|---|---|---|---|
| | hydroxy-2-oxo-pyrrolidin-3-carbonsäure-3-chlor-5-fluor-benzylamid | 15.7, 6.0 Hz, 1H), 3.69 (dd, *J* = 12.1, 5.5 Hz, 2H), 2.89 (t, *J* = 7.6 Hz, 2H), 2.60 (ddd, *J* = 11.7, 7.1, 4.4 Hz, 1H), 2.46 (s, 1H), 2.15 (dt, *J* = 12.9, 7.5 Hz, 1H). | |
| "B125" | 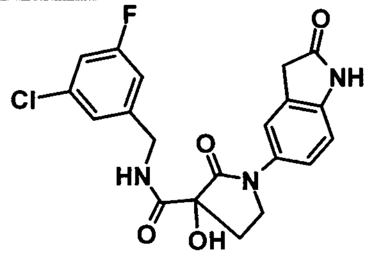 3-Hydroxy-2-oxo-1-(2-oxo-2,3-dihydro-1H-indol-5-yl)-pyrrolidin-3-carbonsäure-3-chlor-5-fluor-benzylamid | | 1.87 [418.0] |
| "B126" | 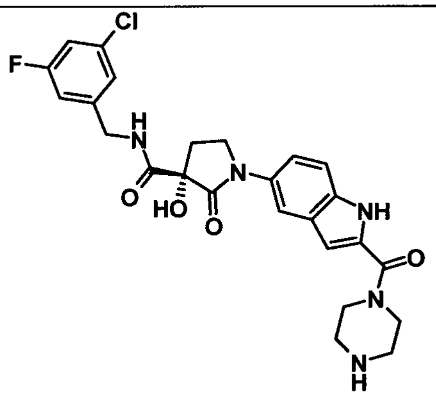 (S)-3-Hydroxy-2-oxo-1-[2-(piperazin-1-carbonyl)-1H-indol-5-yl]-pyrrolidin-3-carbonsäure-3-chlor-5-fluor-benzylamid | ** 11.61 (s, 1H), 8.69 (t, *J* = 6.4 Hz, 1H), 8.20 (s, 1H), 7.80 (d, *J* = 1.8 Hz, 1H), 7.55 (dd, *J* = 8.9, 2.0 Hz, 1H), 7.44 (d, *J* = 8.9 Hz, 1H), 7.28 (dt, *J* = 8.7, 2.0 Hz, 1H), 7.24 (s, 1H), 7.13 (d, *J* = 9.6 Hz, 1H), 6.81 (d, *J* = 1.5 Hz, 1H), 4.42 (dd, *J* = 15.7, 6.7 Hz, 1H), 4.28 (dd, *J* = 15.7, 6.0 Hz, 1H), 3.96 – 3.84 (m, 2H), 3.70 (m, 4H), 2.85 (m, 4H), 2.63 (dt, *J* = 12.3, 5.7 Hz, 1H), 2.16 (dt, *J* = 12.9, 7.6 Hz, 1H). | 1.64 [514.2] |
| "B127" | 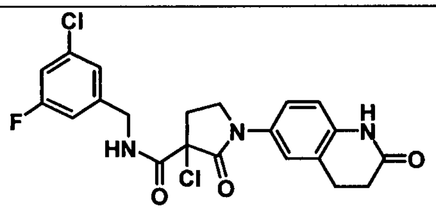 | | |

| | | | |
|---|---|---|---|
| | 3-Chlor-2-oxo-1-(2-oxo-1,2,3,4-tetrahydro-chinolin-6-yl)-pyrrolidin-3-carbonsäure-3-chlor-5-fluor-benzylamid | | |
| "B128" | 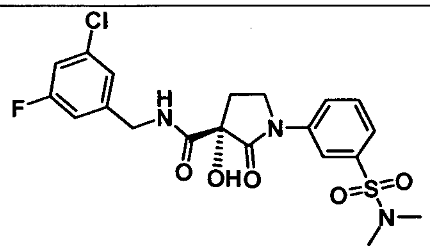 (S)-1-(3-Dimethylsulfamoyl-phenyl)-3-hydroxy-2-oxo-pyrrolidin-3-carbonsäure-3-chlor-5-fluor-benzylamid | | 2.20 [470.0] |
| "B129" | 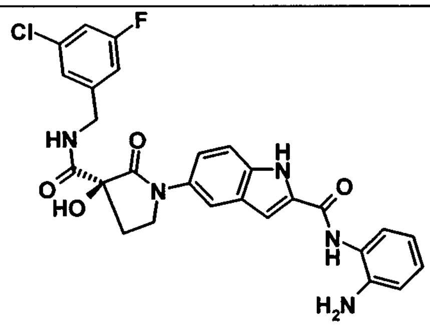 5-[(S)-3-(3-Chlor-5-fluor-benzylcarbamoyl)-3-hydroxy-2-oxo-pyrrolidin-1-yl]-1H-indol-2-carbonsäure-(2-amino-phenyl)-amid | ** 11.72 (s, 1H), 9.69 (s, 1H), 8.68 (t, J=6.4, 1H), 7.84 (d, J=2.0, 1H), 7.58 (dd, J=8.9, 2.1, 1H), 7.46 (d, J=8.9, 1H), 7.38 - 7.33 (m, 1H), 7.27 (dt, J=8.7, 2.2, 1H), 7.24 - 7.22 (m, 1H), 7.22 - 7.18 (m, 1H), 7.15 - 7.10 (m, 1H), 7.01 - 6.96 (m, 1H), 6.80 (dd, J=8.1, 1.4, 1H), 6.69 (s, 1H), 6.62 (td, J=7.5, 1.4, 1H), 4.93 (s, 2H), 4.41 (dd, J=15.7, 6.8, 1H), 4.27 (dd, J=15.8, 6.0, 1H), 3.94 - 3.89 (m, 2H), 2.62 (dt, J=12.8, 5.8, 1H), 2.15 (dt, J=12.8, 7.5, 1H). | 2.06 [536.1] |
| "B130" | 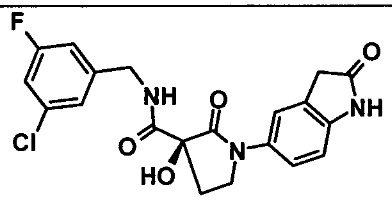 | | |

| | | | |
|---|---|---|---|
| | (S)-3-Hydroxy-2-oxo-1-(2-oxo-2,3-dihydro-1H-indol-5-yl)-pyrrolidin-3-carbonsäure-3-chlor-5-fluor-benzylamid | | |
| "B131" | 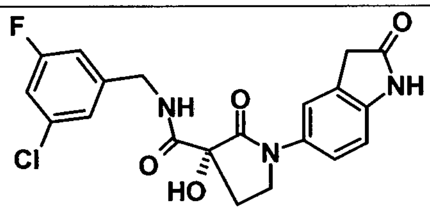 (R)-3-Hydroxy-2-oxo-1-(2-oxo-2,3-dihydro-1H-indol-5-yl)-pyrrolidin-3-carbonsäure-3-chlor-5-fluor-benzylamid | | |
| "B132" | 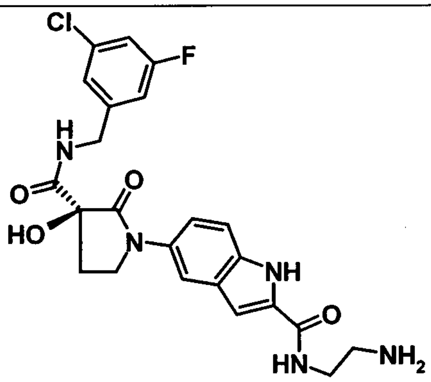 5-[(S)-3-(3-Chlor-5-fluor-benzylcarbamoyl)-3-hydroxy-2-oxo-pyrrolidin-1-yl]-1H-indol-2-carbonsäure-(2-amino-ethyl)-amid | 11.71 (s, 1H), 8.82 (t, J=5.6, 1H), 8.70 (t, J=6.4, 1H), 8.30 (s, 2H), 7.80 (d, J=2.0, 1H), 7.54 (dd, J=8.9, 2.1, 1H), 7.44 (d, J=8.9, 1H), 7.27 (dt, J=8.8, 2.2, 1H), 7.24 - 7.21 (m, 1H), 7.15 - 7.09 (m, 2H), 4.41 (dd, J=15.8, 6.8, 1H), 4.26 (dd, J=15.8, 6.0, 1H), 3.94 - 3.85 (m, 2H), 3.47 (q, J=6.1, 2H), 2.93 (t, J=6.3, 2H), 2.61 (dt, J=12.8, 5.7, 1H), 2.14 (dt, J=12.9, 7.5, 1H). | 1.59 [4.88.1] |
| "B133" | 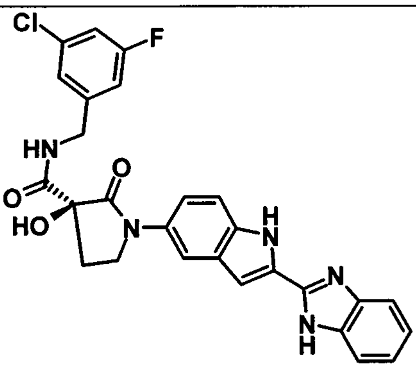 | ** 12.95 (s, 1H), 12.01 (s, 1H), 8.69 (t, J=6.4, 1H), 7.82 (d, J=2.0, 1H), 7.71 - 7.54 (m, 2H), 7.53 (dd, J=8.8, 2.1, 1H), 7.46 (d, J=8.8, 1H), 7.27 (dt, J=8.8, 2.2, 1H), 7.25 - 7.19 (m, 4H), 7.16 - 7.11 (m, 1H), | 1.97 [518.1] |

| | | | |
|---|---|---|---|
| | (S)-1-[2-(1H-Benzoimidazol-2-yl)-1H-indol-5-yl]-3-hydroxy-2-oxo-pyrrolidin-3-carbonsäure-3-chlor-5-fluor-benzylamid | 6.87 - 6.52 (m, 1H), 4.42 (dd, J=15.8, 6.8, 1H), 4.28 (dd, J=15.8, 6.0, 1H), 3.94 - 3.89 (m, 2H), 2.67 - 2.60 (m, 1H), 2.16 (dt, J=12.8, 7.6, 1H). | - |
| "B134" | 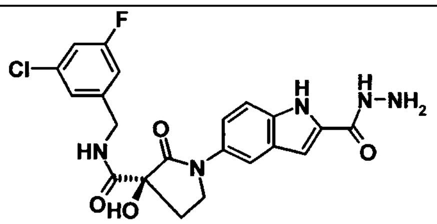 (S)-1-(2-Hydrazinocarbonyl-1H-indol-5-yl)-3-hydroxy-2-oxo-pyrrolidin-3-carbonsäure-3-chlor-5-fluor-benzylamid | ** 11.62 (s, 1H), 9.77 (s, 1H), 8.67 (t, J=6.4, 1H), 7.76 (d, J=2.0, 1H), 7.54 (dd, J=8.9, 2.1, 1H), 7.42 (d, J=8.9, 1H), 7.26 (dt, J=8.7, 2.2, 1H), 7.24 - 7.21 (m, 1H), 7.14 - 7.10 (m, 1H), 7.10 - 7.07 (m, 1H), 6.67 (s, 1H), 4.51 (s, 2H), 4.40 (dd, J=15.7, 6.7, 1H), 4.27 (dd, J=15.7, 6.0, 1H), 3.92 - 3.86 (m, 2H), 2.65 - 2.57 (m, 1H), 2.14 (dt, J=12.8, 7.6, 1H). | 1.82 [460.1] |
| "B135" | 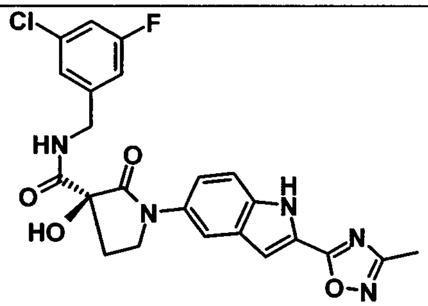 (S)-3-Hydroxy-1-[2-(3-methyl-[1,2,4]oxadiazol-5-yl)-1H-indol-5-yl]-2-oxo-pyrrolidin-3-carbonsäure-3-chlor-5-fluor-benzylamid | ** 12.34 (s, 1H), 8.69 (t, J=6.4, 1H), 7.88 (d, J=2.0, 1H), 7.70 (dd, J=9.0, 2.1, 1H), 7.50 (d, J=9.0, 1H), 7.38 (s, 1H), 7.26 (dt, J=8.8, 2.2, 1H), 7.24 - 7.21 (m, 1H), 7.14 - 7.10 (m, 1H), 6.72 (s, 1H), 4.40 (dd, J=15.7, 6.7, 1H), 4.27 (dd, J=15.7, 6.0, 1H), 3.94 - 3.88 (m, 2H), 2.62 (dt, J=12.8, 5.8, 1H), 2.44 (s, 3H), 2.16 (dt, J=12.7, 7.5, 1H). | 2.21 [484.1] |

| | | | |
|---|---|---|---|
| "B136" | 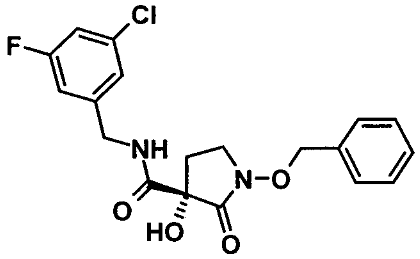<br>(S)-1-Benzyloxy-3-hydroxy-2-oxo-pyrrolidin-3-carbonsäure-3-chlor-5-fluor-benzylamid | | |
| "B137" | 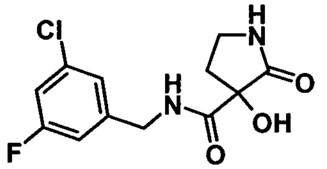<br>3-Hydroxy-2-oxo-pyrrolidin-3-carbonsäure-3-chlor-5-fluor-benzylamid | 8.56 (t, J=6.4, 1H), 8.00 (s, 1H), 7.26 (m, 2H), 7.20 (s, 1H), 7.09 (m, 1H), 6.39 (s, 1H), 4.36 (dd, J=15.7, 6.7, 1H), 4.21 (dd, J=15.7, 6.0, 1H), 3.21 (m, 2H), 2.47 (m, 1H), 1.98 (m, 1H). | 2.77 [287.0] |
| "B138" | 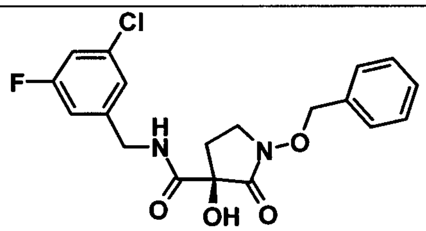<br>(R)-1-Benzyloxy-3-hydroxy-2-oxo-pyrrolidin-3-carbonsäure-3-chlor-5-fluor-benzylamid | | |
| "B139" | 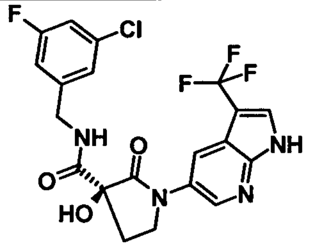<br>(S)-3-Hydroxy-2-oxo-1-(3-trifluormethyl-1H-pyrrolo[2,3-b]pyridin-5-yl)-pyrrolidin-3-carbonsäure-3-chlor-5-fluor-benzylamid | | |

| | | | |
|---|---|---|---|
| "B140" | 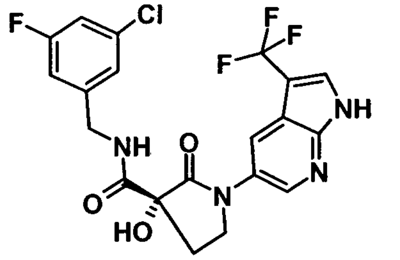<br>(R)-3-Hydroxy-2-oxo-1-(3-trifluormethyl-1H-pyrrolo[2,3-b]pyridin-5-yl)-pyrrolidin-3-carbonsäure-3-chlor-5-fluor-benzylamid | | |
| "B141" | 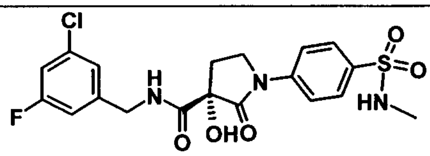<br>(S)-3-Hydroxy-1-(4-methyl-sulfamoyl-phenyl)-2-oxo-pyrrolidin-3-carbonsäure-3-chlor-5-fluor-benzylamid | | 2.06 [456.0] |
| "B142" | 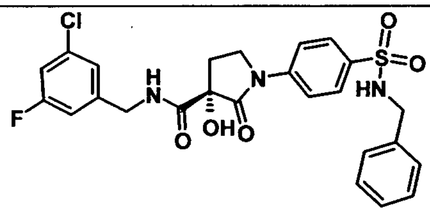<br>(S)-1-(4-Benzylsulfamoyl-phenyl)-3-hydroxy-2-oxo-pyrrolidin-3-carbonsäure-3-chlor-5-fluor-benzylamid | ** 8.76 (t, *J* = 6.4 Hz, 1H), 8.09 (t, *J* = 6.3 Hz, 1H), 7.97 – 7.89 (m, 2H), 7.89 – 7.80 (m, 2H), 7.32 – 7.21 (m, 7H), 7.15 – 7.08 (m, 1H), 6.85 (s, 1H), 4.40 (dd, *J* = 15.7, 6.7 Hz, 1H), 4.27 (dd, *J* = 15.7, 6.0 Hz, 1H), 3.97 (d, *J* = 6.3 Hz, 2H), 3.95 – 3.90 (m, 2H), 2.63 (ddd, *J* = 12.7, 6.9, 4.7 Hz, 1H), 2.18 (dt, *J* = 13.0, 7.7 Hz, 1H). | 2.86 [532.1] |
| "B143" | 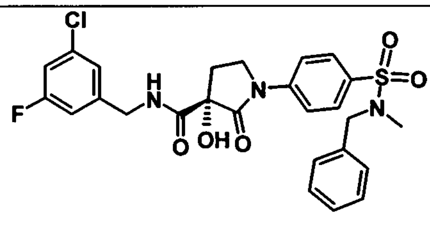 | ** 8.78 (t, *J* = 6.4 Hz, 1H), 8.04 – 7.99 (m, 2H), 7.92 – 7.88 (m, 2H), 7.42 – 7.35 (m, 2H), 7.32 (dd, *J* = 7.4, 3.9 Hz, 3H), 7.29 (ddd, *J* = 8.8, 3.6, | 2.63 [546.2] |

| | | | |
|---|---|---|---|
| | (S)-1-[4-(Benzyl-methyl-sulfamoyl)-phenyl]-3-hydroxy-2-oxo-pyrrolidin-3-carbonsäure-3-chlor-5-fluor-benzylamid | 2.0 Hz, 1H), 7.22 (s, 1H), 7.14 – 7.10 (m, 1H), 6.87 (s, 1H), 4.41 (dd, $J$ = 15.7, 6.7 Hz, 1H), 4.27 (dd, $J$ = 15.7, 6.0 Hz, 1H), 4.12 (s, 2H), 4.00 – 3.91 (m, 2H), 2.68 – 2.59 (m, 1H), 2.53 (d, $J$ = 5.8 Hz, 3H), 2.20 (dt, $J$ = 13.0, 7.8 Hz, 1H). | |
| "B144" | 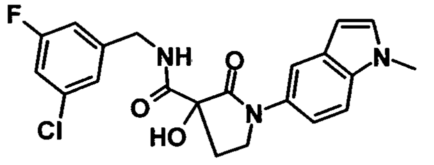 3-Hydroxy-1-(1-methyl-1H-indol-5-yl)-2-oxo-pyrrolidin-3-carbonsäure-3-chlor-5-fluor-benzylamid | | 2.24 [416.1] |
| "B145" | 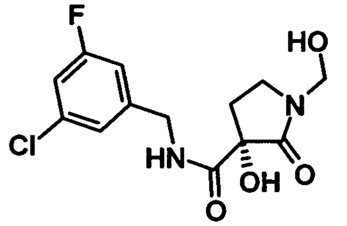 (S)-3-Hydroxy-1-hydroxymethyl-2-oxo-pyrrolidin-3-carbonsäure-3-chlor-5-fluor-benzylamid | 8.63 – 8.54 (m, 1H), 7.26 (dt, $J$ = 8.7, 2.2 Hz, 1H), 7.21 (s, 1H), 7.17 – 7.05 (m, 1H), 6.49 (s, 1H), 5.96 (t, $J$ = 7.1 Hz, 1H), 4.62 (qd, $J$ = 10.2, 7.0 Hz, 2H), 4.37 (dd, $J$ = 15.7, 6.7 Hz, 1H), 4.23 (dd, $J$ = 15.8, 5.9 Hz, 1H), 3.50 – 3.36 (m, 2H), 2.56 – 2.39 (m, 2H), 2.06 – 1.88 (m, 1H). | |
| "B146" | 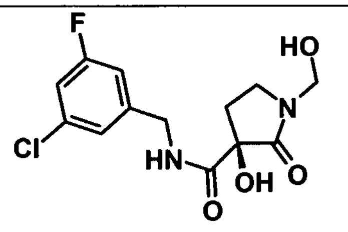 (R)-3-Hydroxy-1-hydroxymethyl-2-oxo-pyrrolidin-3-carbonsäure-3-chlor-5-fluor-benzylamid | 8.66 – 8.48 (m, 1H), 7.25 (dt, $J$ = 8.7, 2.0 Hz, 1H), 7.21 (s, 1H), 7.10 (d, $J$ = 9.2 Hz, 1H), 6.49 (s, 1H), 5.96 (t, $J$ = 7.0 Hz, 1H), 4.62 (qd, $J$ = 10.2, 7.1 Hz, 2H), 4.37 (dd, $J$ = 15.7, 6.7 Hz, 1H), 4.23 (dd, $J$ = 15.7, 6.0 Hz, 1H), 3.50 – 3.36 (m, 2H), | |

| | | | |
|---|---|---|---|
| | | 2.48 (ddd, *J* = 12.8, 5.8, 3.4 Hz, 2H), 2.06 – 1.86 (m, 1H). | |
| "B147" | 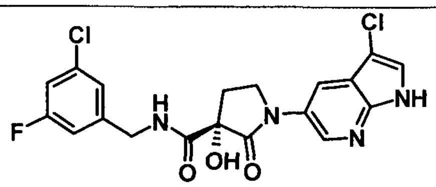 (S)-1-(3-Chlor-1H-pyrrolo[2,3-b]pyridin-5-yl)-3-hydroxy-2-oxo-pyrrolidin-3-carbonsäure-3-chlor-5-fluor-benzylamid | | 2.131 [437.0 + 439.0] |
| "B148" | 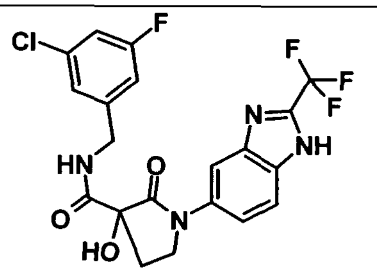 3-Hydroxy-2-oxo-1-(2-trifluormethyl-1H-benzimidazol-5-yl)-pyrrolidin-3-carbonsäure-3-chlor-5-fluor-benzylamid | | 2.093 [471.1] |
| "B149" | 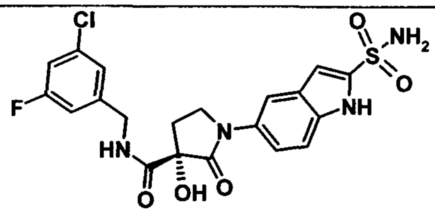 (S)-3-Hydroxy-2-oxo-1-(2-sulfamoyl-1H-indol-5-yl)-pyrrolidin-3-carbonsäure-3-chlor-5-fluor-benzylamid | | |

| | | | |
|---|---|---|---|
| "B150" | 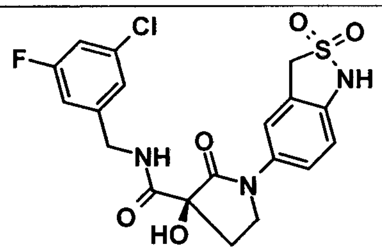 (3S)-N-[(3-chlor-5-fluor-phenyl)methyl]-1-(2,2-dioxo-1,3-dihydro-2,1-benzothiazol-5-yl)-3-hydroxy-2-oxo-pyrrolidin-3-carboxamid | | |
| "B151" | 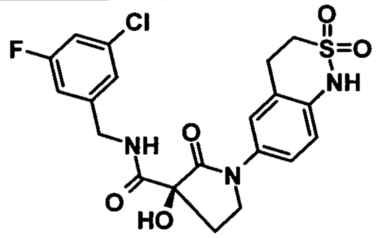 (S)-1-(2,2-Dioxo-1,2,3,4-tetrahydro-2lamda*6*-benzo[c]thiazin-6-yl)-3-hydroxy-2-oxo-1-(2-sulfamoyl-1H-indol-5-yl)-pyrrolidin-3-carbonsäure-3-chlor-5-fluor-benzylamid | | |
| "B152" | 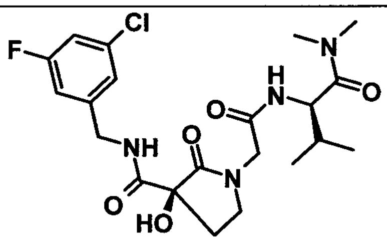 (S)-1-[((R)-1-Dimethylcarbamoyl-2-methyl-propylcarbamoyl)-methyl]-3-hydroxy-2-oxo-1-(2-sulfamoyl-1H-indol-5-yl)-pyrrolidin-3-carbonsäure-3-chlor-5-fluor-benzylamid | | |

| | | | |
|---|---|---|---|
| "B153" | 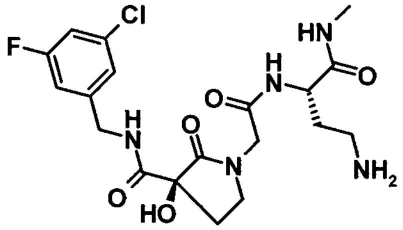 (S)-1-[((S)-3-Amino-1-methylcarbamoyl-propylcarbamoyl)-methyl]-3-hydroxy-2-oxo-1-(2-sulfamoyl-1H-indol-5-yl)-pyrrolidin-3-carbonsäure-3-chlor-5-fluor-benzylamid | | |
| "B154" | 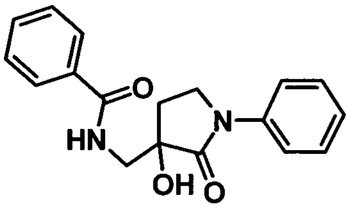 N-(3-Hydroxy-2-oxo-1-phenyl-pyrrolidin-3-yl)-benzamid | | |

Tabelle 1

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "A56" | C | | |
| "A57" | C | | |
| "A58" | C | | |
| "A59" | C | | |
| "A60" | C | | |
| "A61" | C | "A71" | C |
| "A62" | C | "A72" | A |
| "A63" | C | "A73" | A |
| "A64" | C | "A74" | C |
| "A65" | A | "A75" | A |
| "A66" | B | "A76" | A |
| "A67" | C | "A77" | A |
| "A68" | A | "A78" | A |

(fortgesetzt)

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "A69" | A | "A79" | A |
| "A70" | C | "A80" | A |
| | | | |
| "A81" | A | "A91" | C |
| "A82" | B | "A92" | B |
| "A83" | C | "A93" | B |
| "A84" | B | "A94" | A |
| "A85" | C | "A95" | A |
| "A86" | C | "A96" | A |
| "A87" | A | "A97" | A |
| "A88" | A | "A98" | C |
| "A89" | C | "A99" | A |
| "A90" | A | "A100" | A |
| | | | |
| "A101" | C | "A111" | C |
| "A102" | B | "A112" | A |
| "A103" | B | "A113" | C |
| "A104" | C | "A114" | A |
| "A105" | C | "A115" | A |
| "A106" | C | "A116" | A |
| "A107" | B | "A117" | A |
| "A108" | C | "A118" | A |
| "A109" | C | "A119" | B |
| "A110" | C | "A120" | C |
| | | | |
| "A121" | B | "A131" | C |
| "A122" | B | "A132" | B |
| "A123" | B | "A133" | A |
| "A124" | B | "A134" | A |
| "A125" | B | "A135" | A |
| "A126" | B | "A136" | A |
| "A127" | B | "A137" | A |
| "A128" | B | "A138" | A |
| "A129" | B | "A139" | A |
| "A130" | B | "A140" | A |
| | | | |

(fortgesetzt)

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "A141" | C | "A151" | A |
| "A142" | A | "A152" | B |
| "A143" | A | "A153" | A |
| "A144" | A | "A154" | C |
| "A145" | B | "A155" | A |
| "A146" | A | "A156" | C |
| "A147" | A | "A157" | B |
| "A148" | B | "A158" | C |
| "A149" | A | "A159" | B |
| "A150" | C | "A160" | C |
| | | | |
| "A161" | A | "A171" | A |
| "A162" | C | "A172" | B |
| "A163" | A | "A173" | A |
| "A164" | C | "A174" | A |
| "A165" | A | "A175" | A |
| "A166" | B | "A176" | A |
| "A167" | A | "A177" | A |
| "A168" | A | "A178" | A |
| "A169" | A | "A179" | A |
| "A170" | A | "A180" | A |
| | | | |
| "A181" | A | "A191" | A |
| "A182" | C | "A192" | B |
| "A183" | A | "A193" | A |
| "A184" | A | "A194" | A |
| "A185" | A | "A195" | C |
| "A186" | A | "A196" | A |
| "A187" | B | "A197" | A |
| "A188" | A | "A198" | C |
| "A189" | A | "A199" | A |
| "A190" | B | "A200" | A |
| | | | |
| "A201" | A | "A211" | A |
| "A202" | B | "A212" | A |
| "A203" | C | "A213" | B |

(fortgesetzt)

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "A204" | B | "A214" | A |
| "A205" | A | "A215" | A |
| "A206" | A | "A216" | A |
| "A207" | A | "A217" | A |
| "A208" | B | "A218" | C |
| "A209" | A | "A219" | A |
| "A210" | A | "A220" | B |
| | | | |
| "A221" | A | "A231" | B |
| "A222" | A | "A232" | B |
| "A223" | C | "A233" | C |
| "A224" | B | "A234" | A |
| "A225" | B | "A235" | A |
| "A226" | B | "A236" | A |
| "A227" | B | "A237" | B |
| "A228" | B | "A238" | A |
| "A229" | C | "A239" | A |
| "A230" | B | "A240" | B |
| | | | |
| "A241" | A | "A251" | A |
| "A242" | A | "A252" | B |
| "A243" | | "A253" | B |
| "A244" | A | "A254" | A |
| "A245" | A | "A255" | B |
| "A246" | A | "A256" | A |
| "A247" | A | "A257" | B |
| "A248" | B | "A258" | C |
| "A249" | A | "A259" | C |
| "A250" | A | "A260" | C |
| | | | |
| "A261" | C | "A271" | C |
| "A262" | C | "A272" | B |
| "A263" | C | "A273" | B |
| "A264" | A | "A274" | B |
| "A265" | B | "A275" | C |
| "A266" | A | "A276" | B |

(fortgesetzt)

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "A267" | B | "A277" | A |
| "A268" | C | "A278" | A |
| "A269" | B | "A279" | B |
| "A270" | A | "A280" | B |
| "A281" | B | "A291" | B |
| "A282" | B | "A292" | B |
| "A283" | A | "A293" | A |
| "A284" | A | "A294" | |
| "A285" | A | "A295" | |
| "A286" | A | "A296" | |
| "A287" | | "A297" | |
| "A288" | C | "A298" | |
| "A289" | A | "A299" | |
| "A290" | B | "A300" | B |
| "B1" | A | "B11" | A |
| "B2" | A | "B12" | B |
| "B3" | A | "B13" | B |
| "B4" | A | "B14" | A |
| "B5" | A | "B15" | A |
| "B6" | A | "B16" | B |
| "B7" | A | "B17" | A |
| "B8" | A | "B18" | A |
| "B9" | A | "B19" | C |
| "B10" | A | "B20" | C |
| "B21" | C | "B31" | A |
| "B22" | A | "B32" | A |
| "B23" | B | "B33" | A |
| "B24" | A | "B34" | A |
| "B25" | A | "B35" | A |
| "B26" | A | "B36" | B |
| "B27" | A | "B37" | A |
| "B28" | A | "B38" | B |
| "B29" | A | "B39" | A |
| "B30" | C | "B40" | A |
| "B41" | B | "B51" | C |
| "B42" | B | "B52" | A |

(fortgesetzt)

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "B43" | A | "B53" | C |
| "B44" | C | "B54" | A |
| "B45" | B | "B55" | B |
| "B46" | A | "B56" | A |
| "B47" | B | "B57" | C |
| "B48" | B | "B58" | B |
| "B49" | A | "B59" | A |
| "B50" | C | "B60" | A |
| "B61" | B | "B71" | A |
| "B62" | A | "B72" | A |
| "B63" | A | "B73" | C |
| "B64" | C | "B74" | A |
| "B65" | A | "B75" | A |
| "B66" | A | "B76" | A |
| "B67" | B | "B77" | A |
| "B68" | B | "B78" | A |
| "B69" | A | "B79" | B |
| "B70" | A | "B80" | A |
| "B81" | C | "B91" | B |
| "B82" | A | "B92" | A |
| "B83" | A | "B93" | B |
| "B84" | A | "B94" | A |
| "B85" | C | "B95" | A |
| "B86" | A | "B96" | A |
| "B87" | A | "B97" | A |
| "B88" | C | "B98" | A |
| "B89" | C | "B99" | A |
| "B90" | C | "B100" | C |
| "B101" | A | "B111" | B |
| "B102" | C | "B112" | A |
| "B103" | C | "B113" | A |
| "B104" | A | "B114" | A |
| "B105" | A | "B115" | A |
| "B106" | B | "B116" | A |
| "B107" | A | "B117" | C |
| "B108" | A | "B118" | A |

(fortgesetzt)

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "B109" | A | "B119" | A |
| "B110" | A | "B120" | A |
| "B121" | A | "B131" | C |
| "B122" | A | "B132" | A |
| "B123" | A | "B133" | A |
| "B124" | A | "B134" | A |
| "B125" | A | "B135" | A |
| "B126" | A | "B136" | A |
| "B127" | | "B137" | |
| "B128" | | "B138" | C |
| "B129" | A | "B 139" | A |
| "B130" | A | "B140" | C |
| "B141" | A | | |
| $IC_{50}$: 10nM- 1μM = A<br>1μM-10μM = B<br>>10μM = C | | | |

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4$. 2 $H_2O$, 28,48 g $Na_2HPO_4$ - 12 $H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

**1.** Verbindungen der Formel I

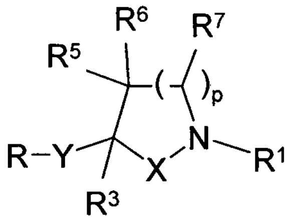

I

worin

R $NR^2R^4$, Alk, $C(=CH_2)[C(R^4)_{2]n}Ar^2$, $Het^2$, $O[C(R^4)_2]_nAr^2$ oder OA,
X CO oder $CH_2$,
Y CO oder $CH_2$,
$R^1$ H, $[C(R^4)_2]_nAr^1$, $(CH_2)_nHet$, $(CH_2)_nCyc$, $[C(R^4)_2]_nCOOH$, $[C(R_4)_2]_nCONHAr^1$, $[C(R^4)_2]_nCONH_2$, $[C(R^4)_2]_nN$-HA, $[C(R^4)_2]_nNA_2$, $O[C(R^4)_2]_nAr^1$, $[C(R^4)_2]_nOR^7$, $[C(R^4)_2]_nCOO(CH_2)_nAr^1$, $[C(R^4)_2]_nCOOA$, $[C(R^4)_2]_nCONH[C(R^4)_2]_pCON(R^4)_2$ oder $[C(R^4)_2]_nCONHCR^4[(CH_2)nN(R^4)2]CON(R^4)2$,
$R^2$ H, $[C(R^4)_2]_nAr^2$, $(CH_2)_nCOHet^1$, $(CH_2)_nCOAr^2$, $(CH_2)_mNA_2$ oder $(CH_2)_nHet$,
$R^3$ OH oder OCOA,
$R^4$ H oder Alkyl mit 1, 2, 3 oder 4 C-Atomen,
$R^2$ und $R^4$ zusammen auch Alkylen mit 2, 3, 4 oder 5 C-Atomen, wobei eine $CH_2$-Gruppe auch durch $N(CH_2)_mOH$ oder $SO_2$ ersetzt sein kann,
$R^5$, $R^6$ jeweils unabhängig voneinander H, F oder A,
$R^5$ und $R^6$ zusammen auch Alkylen mit 2, 3, 4 oder 5 C-Atomen, wobei eine $CH_2$-Gruppe auch durch NCOA oder O ersetzt sein kann,
$R^7$ H oder A,
$Ar^1$ unsubstituiertes oder ein-, zwei-, drei-, vier- oder fünffach durch Hal, OH, OA, $CONH_2$, CONHA, $CONA_2$, $NHSO_2A$, CONHCyc, $NHSO_2Cyc$, $CONHAr^2$, $COHet^1$ und/oder $NASO_2A$ substituiertes Phenyl,
$Ar^2$ unsubstituiertes oder ein-, zwei-, drei-, vier- oder fünffach durch Hal, A, $CONH_2$, und/oder $OAr^3$ substituiertes Phenyl,
$Ar^3$ unsubstituiertes oder einfach durch $NH_2$ substituiertes Phenyl,
Het einen unsubstituierten oder ein-, zwei- oder dreifach durch Hal, A, OA, CN, $NH_2$, NHA, $NA_2$, $NO_2$, CN, COOH, COOA, $(CH_2)nCONH2$, $(CH_2)_nCONHA$, $(CH_2)nCONA2$, NHCOA, COA, CHO, $Het^1$, $SO_2A$, $SO_2NH_2$, $SO_2NHA$, $SO_2NA_2$, $CONHNH_2$, $CONHAr^3$, =O und/oder $Ar^3$ substituierten ein- oder zweikernigen gesättigten, ungesättigten oder aromatischen Heterocyclus mit 1 bis 4 N-, und/oder O- und/oder S-Atomen,
$Het^1$ einen unsubstituierten oder ein-, zwei- oder dreifach durch =O und/oder COOA substituierten einkernigen gesättigten Heterocyclus mit 1 bis 4 N-, und/oder O- und/oder S-Atomen,
$Het^2$ Isoindolyl,

A unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können, und/oder worin eine oder zwei nicht-benachbarte CH- und/oder $CH_2$-Gruppen durch O ersetzt sein können, oder Cyc,
Alk Alkenyl mit 2, 3, 4, 5 oder 6 C-Atomen
Cyc unsubstituiertes oder ein-, zwei- oder dreifach durch NHCOA, $NHSO_2$, OH, OA, A, $NH_2$, NHA, $NA_2$, COOA, COOH und/oder CONHA substituiertes cyclisches Alkyl mit 3-7 C-Atomen,
Hal F, Cl, Br oder I,
m 1, 2, 3 oder 4,
n 0, 1, 2, 3 oder 4,
p 1, 2 oder 3,

bedeuten,
sowie ihre pharmazeutisch verwendbaren Salze, Tautomere und Stereoisomere, einschließlich deren Mischungen in allen Verhältnissen, wobei die folgenden Verbindungen ausgenommen sind: 3-Hydroxypiperidin-3-carbonsäuremethylester, 3-Hydroxypiperidin-1-carbonsäure-tert.-butylester-3-carbonsäuremethylester, 3-Allyl-3-hydroxypyrrolidin-1-carbonsäure-tert.-butylester, 3-Allyl-3-hydroxypiperidin-1-carbonsäure-tert.-butylester.

**2.** Verbindungen nach Anspruch 1, worin

Het unsubstituiertes oder ein-, zwei- oder dreifach durch Hal, A, OA, CN, $NH_2$, NHA, $NA_2$, $NO_2$, CN, COOH, COOA, $(CH_2)$nCONH2, $(CH_2)_n$CONHA, $(CH_2)$nCONA2, NHCOA, COA, CHO, $Het^1$, $SO_2A$, $SO_2NH_2$, $SO_2NHA$, $SO_2NA_2$, $CONHNH_2$, $CONHAr^3$, =O und/oder $Ar^3$ substituiertes Pyrazinyl, Pyrazolyl, Benzimidazolyl, Pyridyl, Indolyl, Dihydro-indolyl, Benzofuranyl, Tetrahydropyranyl, Dihydrochinolinyl, Dihydroisochinolinyl, Tetrahydrochinolinyl, Tetrahydroisochinolinyl, Indazolyl, Imidazolyl, Pyrrolyl, Oxazolyl, Oxadiazolyl, Isoxazolyl, Benzothiazolyl, Piperidin-1-yl, Pyrrolidin-1-yl, 3,4-Dihydro-2H-pyrido[3,2-b][1,4]oxazinyl, 3,4-Dihydro-2H-benzo[1,4]oxazinyl, Benzofuranyl, Azetidinyl, 3-Aza-bicylo[3.2.0]hexyl, Pyrrolo[2,3-b]pyridinyl, Tetrahydrofuranyl, Tetrahydro-[1,8]naphthyridinyl, 2,3-Dihydro-benzo-isothiazolyl, 1,2,3,4-Tetrahydro-benzo-thiazinyl oder Hexahydrobenzo[1,3]dioxolyl,

bedeutet,
sowie ihre pharmazeutisch verwendbaren Salze, Tautomere und Stereoisomere, einschließlich deren Mischungen in allen Verhältnissen.

**3.** Verbindungen nach Anspruch 1 oder 2, worin

$Het^1$ unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Irnidazolyl, Pyrrolyl, Oxazolyl, Oxadiazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl

bedeutet,
sowie ihre pharmazeutisch verwendbaren Salze, Tautomere und Stereoisomere, einschließlich deren Mischungen in allen Verhältnissen.

**4.** Verbindungen nach Anspruch 1, worin

R $NR^2R^4$, Alk, $C(=CH_2)[C(R^4)_2]_nAr^2$, $Het^2$, $O[C(R^4)_2]_nAr^2$ oder OA,
X CO oder $CH_2$,
Y CO oder $CH_2$,
$R^1$ H, $[C(R_4)_2]_nAr^1$, $(CH_2)_n$Het, $(CH_2)_n$Cyc, $[C(R^4)_2]_n$COOH, $[C(R^4)_2]_nCONHAr^1$, $[C(R^4)_2]_nCONH_2$, $[C(R^4)_2]_n$NHA, $[C(R^4)_{2]}$nNA2, $O[C(R_4)_2]_nAr^1$, $[C(R_4)_2]_nOR^7$, $[C(R4)_2]_nCOO(CH_2)_nAr^1$, $[C(R^4)_2]_n$COOA, $[C(R^4)_2]_nCONH[C(R^4)_2]_pCON(R^4)_2$ oder $[C(R^4)_2]_nCONHCR^4[(CH_2)_nN(R^4)_2]CON(R_4)_2$,
$R^2$ H, $[C(R^4)_2]_nAr^2$, $(CH_2)_nCOHet^1$, $(CH_2)_nCOAr^2$, $(CH_2)_mNA_2$ oder $(CH_2)_n$Het,
$R^3$ OH oder OCOA,
$R^4$ H oder Alkyl mit 1, 2, 3 oder 4 C-Atomen,
$R^2$ und $R^4$ zusammen auch Alkylen mit 2, 3, 4 oder 5 C-Atomen, wobei eine $CH_2$-Gruppe auch durch $N(CH_2)_mOH$ oder $SO_2$ ersetzt sein kann,
$R^5$, $R^6$ jeweils unabhängig voneinander H, F oder A,

$R^5$ und $R^6$ zusammen auch Alkylen mit 2, 3, 4 oder 5 C-Atomen, wobei eine $CH_2$-Gruppe auch durch NCOA oder O ersetzt sein kann,
$R^7$ H oder A,
$Ar^1$ unsubstituiertes oder ein-, zwei-, drei-, vier- oder fünffach durch Hal, OH, OA, $CONH_2$, CONHA, $CONA_2$, $NHSO_2A$, CONHCyc, $NHSO_2Cyc$, $CONHAr^2$, $COHet^1$ und/oder $NASO_2A$ substituiertes Phenyl,
$Ar^2$ unsubstituiertes oder ein-, zwei-, drei-, vier- oder füriffach durch Hal, A, $CONH_2$, und/oder $OAr^3$ substituiertes Phenyl,
$Ar^3$ unsubstituiertes oder einfach durch $NH_2$ substituiertes Phenyl,
Het unsubstituiertes oder ein-, zwei- oder dreifach durch Hal, A, OA, CN, $NH_2$, NHA, $NA_2$, $NO_2$, CN, COOH, COOA, $(CH_2)$nCONH2, $(CH_2)_nCONHA$, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O und/oder $Ar^3$ substituiertes Pyrazinyl, Pyrazolyl, Benzimidazolyl, Pyridyl, Indolyl, Dihydro-indolyl, Benzofuranyl, Tetrahydropyranyl, Dihydrochinolinyl, Dihydroisochinolinyl, Tetrahydrochinolinyl, Tetrahydroisochinolinyl, Indazolyl, Imidazolyl, Pyrrolyl, Oxazolyl, Oxadiazolyl, Isoxazolyl, Benzothiazolyl, Piperidin-1-yl, Pyrrolidin-1-yl, 3,4-Dihydro-2H-pyrido[3,2-b][1,4]oxazinyl, 3,4-Dihydro-2H-benzo[1,4]oxazinyl, Benzofuranyl, Azetidinyl, 3-Aza-bicylo[3.2.0]hexyl, Pyrrolo[2,3-b]pyridinyl, Tetrahydrofuranyl, Tetrahydro-[1,8]naphthyridinyl 2,3-Dihydro-benzo-isothiazolyl, 1,2,3,4-Tetrahydro-benzo-thiazinyl oder hexahydro-benzo[1,3]dioxolyl,
$Het^1$ unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl,
$Het^2$ Isoindolyl,
A unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können, und/oder worin eine oder zwei nicht-benachbarte CH- und/oder $CH_2$-Gruppen durch O ersetzt sein können, oder Cyc,
Alk Alkenyl mit 2, 3, 4, 5 oder 6 C-Atomen
Cyc unsubstituiertes oder ein-, zwei- oder dreifach durch NHCOA, $NHSO_2$, OH, OA, A, $NH_2$, NHA, $NA_2$, COOA, COOH und/oder CONHA substituiertes cyclisches Alkyl mit 3-7 C-Atomen,
Hal F, Cl, Br oder I,
m 1, 2, 3 oder 4,
n 0, 1, 2, 3 oder 4,
p 1, 2 oder 3,

bedeutet,
sowie ihre pharmazeutisch verwendbaren Salze, Tautomere und Stereoisomere, einschließlich deren Mischungen in allen Verhältnissen.

**5.** Verbindungen nach Anspruch 1, ausgewählt aus der Gruppe

| Verbindung Nr. | Name |
|---|---|
| "A56" | (S)-3-(1,1-Dioxo-1l6-thiomorpholin-4-carbonyl)-3-hydroxy-1-phenyl-pyrrolidin-2-on |
| "A57" | (S)-3-Hydroxy-3-[4-(2-hydroxy-ethyl)-piperazin-1-carbonyl]-1-phenyl-pyrrolidin-2-on |
| "A58" | (S)-3-Hydroxy-2-oxo-1-phenyl-pyrrolidin-3-carbonsäure-(3-imidazol-1-yl-propyl)-amid |
| "A59" | (S)-3-Hydroxy-2-oxo-1-phenyl-pyrrolidin-3-carbonsäure-(2-dimethylamino-ethyl)-amid |
| "A60" | (S)-3-Hydroxy-2-oxo-1-phenyl-pyrrolidin-3-carbonsäure-[2-(4-phenoxy-phenyl)-ethyl]-amid |
| "A61" | (S)-3-Hydroxy-2-oxo-1-phenyl-pyrrolidin-3-carbonsäure-(2-dimethylamino-ethyl)-methyl-amid |
| "A62" | (S)-3-Hydroxy-2-oxo-1-phenyl-pyrrolidin-3-carbonsäure-(2-morpholin-4-yl-2-oxo-ethyl)-amid |
| "A63" | (S)-3-Hydroxy-2-oxo-1-phenyl-pyrrolidin-3-carbonsäure-[2-(1-methyl-pyrrolidin-2-yl)-ethyl]-amid |
| "A64" | (S)-3-Hydroxy-2-oxo-1-phenyl-pyrrolidin-3-carbonsäure-(3-dimethylamino-propyl)-amid |
| "A65" | 3-Hydroxy-2-oxo-1-phenyl-piperidin-3-carbonsäure-3-chlor-5-fluor-benzylamid |
| "A66" | (S)-3-Hydroxy-2-oxo-1-phenyl-azepan-3-carbonsäure-3-chlor-5-fluor-benzylamid |

(fortgesetzt)

| Verbindung Nr. | Name |
|---|---|
| "A67" | (R)-3-Hydroxy-2-oxo-1-phenyl-azepan-3-carbonsäure-3-chlor-5-fluor-benzylamid |
| "A68" | 3-Hydroxy-1-(1H-indol-6-yl)-2-oxo-pyrrolidin-3-carbonsäure-3-chlor-5-fluor-benzylamid |
| "A69" | 1-[3-(3-Carbamoyl-phenylcarbamoyl)-phenyl]-3-hydroxy-2-oxo-pyrrolidin-3-carbonsäure-3-chlor-5-fluor-benzylamid |
| "A70" | (S)-3-((E)-But-2-enoyl)-3-hydroxy-1-phenyl-pyrrolidin-2-on |
| "A71" | (S)-8-Acetyl-4-hydroxy-3-oxo-2-phenyl-2,8-diaza-spiro[4.5]decan-4-carbonsäure-3-chlor-5-fluor-benzylamid |
| "A72" | (S)-3-Hydroxy-1-[3-(morpholin-4-carbonyl)-phenyl]-2-oxo-pyrrolidin-3-carbonsäure-3-chlor-5-fluor-benzylamid |
| "A73" | 3-Hydroxy-1-[3-(methansulfonyl-methyl-amino)-phenyl]-2-oxo-pyrrolidin-3-carbonsäure-3-chlor-5-fluor-benzylamid |
| "A74" | (R)-3-Hydroxy-1-[3-(morpholin-4-carbonyl)-phenyl]-2-oxo-pyrrolidin-3-carbonsäure-3-chlor-5-fluor-benzylamid |
| "A75" | (S)-1-(3-Cyclopropylcarbamoyl-phenyl)-3-hydroxy-2-oxo-pyrrolidin-3-carbonsäure-3-chlor-5-fluor-benzylamid |
| "A76" | 1-(3-Cyclobutylcarbamoyl-phenyl)-3-hydroxy-2-oxo-pyrrolidin-3-carbnsäure-3-chlor-5-fluor-benzylamid |
| "A77" | (S)-3-Hydroxy-2-oxo-1-[3-(pyrrolidin-1-carbonyl)-phenyl]-pyrrolidin-3-carbonsäure-3-chlor-5-fluor-benzylamid |
| "A78" | 3-Hydroxy-2-oxo-1-(6-propionylamino-pyridin-3-yl)-pyrrolidin-3-carbonsäure-3-chlor-5-fluor-benzylamid |
| "A79" | (S)-1-(3-Ethansulfonylamino-phenyl)-3-hydroxy-2-oxo-pyrrolidin-3-carbonsäure-3-chlor-5-fluor-benzylamid |
| "A80" | (S)-1-(3-Cyclopropansulfonylamino-phenyl)-3-hydroxy-2-oxo-pyrrolidin-3-carbonsäure-3-chlor-5-fluor-benzylamid |
| "A81" | (S)-1-Benzyl-3-hydroxy-2-oxo-piperidin-3-carbonsäure-3-chlor-5-fluor-benzylamid |
| "A82" | (R)-1-(3-Cyclopropylcarbamoyl-phenyl)-3-hydroxy-2-oxo-pyrrolidin-3-carbonsäure-3-chlor-5-fluor-benzylamid |
| "A83" | (R)-3-Hydroxy-2-oxo-1-[3-(pyrrolidin-1-carbonyl)-phenyl]-pyrrolidin-3-carbonsäure-3-chlor-5-fluor-benzylamid |
| "A84" | (R)-1-(3-Ethansulfonylamino-phenyl)-3-hydroxy-2-oxo-pyrrolidin-3-carbonsäure-3-chlor-5-fluor-benzylamid |
| "A85" | (R)-1-(3-Cyclopropansulfonylamino-phenyl)-3-hydroxy-2-oxo-pyrrolidin-3-carbonsäure-3-chlor-5-fluor-benzylamide |
| "A86" | (R)-1-Benzyl-3-hydroxy-2-oxo-piperidin-3-carbonsäure-3-chlor-5-fluor-benzylamid |
| "A87" | 1-(3-Cyan-1H-indol-5-yl)-3-hydroxy-2-oxo-pyrrolidin-3-carbonsäure-3-chlor-5-fluor-benzylamid |
| "A88" | (S)-3-Hydroxy-1-[3-(2-hydroxy-ethylcarbamoyl)-phenyl]-2-oxo-pyrrolidin-3-carbonsäure-3-chlor-5-fluor-benzylamid |
| "A89" | 3-(2-Benzyl-acryloyl)-3-hydroxy-1-phenyl-pyrrolidin-2-on |
| "A90" | (S)-3-Hydroxy-2-oxo-1-phenethyl-pyrrolidin-3-carbonsäure-3-chlor-5-fluor-benzylamid |
| "A91" | (R)-3-Hydroxy-1-[3-(2-hydroxy-ethylcarbamoyl)-phenyl]-2-oxo-pyrrolidin-3-carbonsäure-3-chlor-5-fluor-benzylamid |

(fortgesetzt)

| Verbindung Nr. | Name |
|---|---|
| "A92" | (R)-3-Hydroxy-2-oxo-1-phenethyl-pyrrolidin-3-carbonsäure-3-chlor-5-fluor-benzylamid |
| "A93" | (S)-3-Hydroxy-1-{3-[(2-hydroxy-ethyl)-methyl-carbamoyl]-phenyl}-2-oxo-pyrrolidin-3-carbonsäure-3-chlor-5-fluor-benzylamid |
| "A94" | (S)-3-Hydroxy-1-[3-(3-hydroxy-propylcarbamoyl)-phenyl]-2-oxo-pyrrolidin-3-carbonsäure-3-chlor-5-fluor-benzylamid |
| "A95" | (S)-3-Hydroxy-2-oxo-1-[3-(piperidin-1-carbonyl)-phenyl]-pyrrolidin-3-carbonsäure-3-chlor-5-fluor-benzylamid |
| "A96" | (S)-3-Hydroxy-2-oxo-1-(1-oxo-1,2,3,4-tetrahydro-isochinolin-6-yl)-pyrrolidin-3-carbonsäure-3-chlor-5-fluor-benzylamid |
| "A97" | (S)-1-(6-Cyan-pyridin-3-yl)-3-hydroxy-2-oxo-pyrrolidin-3-carbonsäure-3-chlor-5-fluor-benzylamid |
| "A98" | (R)-1-(5-Cyan-pyridin-3-yl)-3-hydroxy-2-oxo-pyrrolidin-3-carbonsäure-3-chlor-5-fluor-benzylamid |
| "A99" | (S)-3-Hydroxy-2-oxo-1-(6-trifluormethyl-pyridin-3-yl)-pyrrolidin-3-carbonsäure-3-chlor-5-fluor-benzylamid |
| "A100" | 5-[3-(3-Chlor-5-fluor-benzylcarbamoyl)-3-hydroxy-2-oxo-pyrrolidin-1-yl]-2-methyl-nicotinsäure-methylester |
| "A101" | (S)-3-Hydroxy-4,4-dimethyl-2-oxo-1-phenyl-pyrrolidin-3-carbonsäure-3-chlor-5-fluor-benzylamid |
| "A102" | (R)-3-Hydroxy-1-{3-[(2-hydroxy-ethyl)-methyl-carbamoyl]-phenyl}-2-oxo-pyrrolidin-3-carbonsäure-3-chlor-5-fluor-benzylamid |
| "A103" | (R)-3-Hydroxy-1-[3-(3-hydroxy-propylcarbamoyl)-phenyl]-2-oxo-pyrrolidin-3-carbonsäure-3-chlor-5-fluor-benzylamid |
| "A104" | (R)-3-Hydroxy-2-oxo-1-[3-(piperidin-1-carbonyl)-phenyl]-pyrrolidin-3-carbonsäure-3-chlor-5-fluor-benzylamid |
| "A105" | (R)-3-Hydroxy-2-oxo-1-(1-oxo-1,2,3,4-tetrahydro-isochiriolin-6-yl)-pyrrolidin-3-carbonsäure-3-chlor-5-fluor-benzylamid |
| "A106" | (R)-1-(6-Cyan-pyridin-3-yl)-3-hydroxy-2-oxo-pyrrolidin-3-carbonsäure-3-chlor-5-fluor-benzylamid |
| "A107" | (S)-1-(5-Cyan-pyridin-3-yl)-3-hydroxy-2-oxo-pyrrolidin-3-carbonsäure-3-chlor-5-fluor-benzylamid |
| "A108" | (R)-3-Hydroxy-2-oxo-1-(6-trifluoromethyl-pyridin-3-yl)-pyrrolidin-3-carbonsäure-3-chlor-5-fluor-benzylamid |
| "A109" | (R)-3-Hydroxy-4,4-dimethyl-2-oxo-1-phenyl-pyrrolidin-3-carbonsäure-3-chlor-5-fluor-benzylamid |
| "A110" | (S)-3-(2-Benzyl-acryloyl)-3-hydroxy-1-phenyl-pyrrolidin-2-on |
| "A111" | (R)-3-(2-Benzyl-acryloyl)-3-hydroxy-1-phenyl-pyrrolidin-2-on |
| "A112" | 3-Hydroxy-1-(1-hydroxymethyl-3-trifluormethyl-1H-indol-5-yl)-2-oxo-pyrrolidin-3-carbonsäure-3-chlor-5-fluor-benzylamid |
| "A113" | 8-Hydroxy-7-oxo-6-phenyl-2-oxa-6-aza-spiro[3.4]octan-8-carbonsäure-3-chlor-5-fluor-benzylamid |
| "A114" | 1-(8-Fluor-2-oxo-1,2,3,4-tetrahydro-chinolin-6-yl)-3-hydroxy-2-oxo-pyrrolidin-3-carbonsäure-3-chlor-5-fluor-benzylamid |

(fortgesetzt)

| Verbindung Nr. | Name |
|---|---|
| "A115" | 3-Hydroxy-2-oxo-1-(1-oxo-1,2,3,4-tetrahydro-isochinolin-7-yl)-pyrrolidin-3-carbonsäure-3-chlor-5-fluor-benzylamid |
| "A116" | 3-Hydroxy-1-(6-methylamino-pyridin-3-yl)-2-oxo-pyrrolidin-3-carbonsäure-3-chlor-5-fluor-benzylamid |
| "A117" | 3-Hydroxy-1-(5-methyl-pyridin-3-yl)-2-oxo-pyrrolidin-3-carbonsäure-3-chlor-5-fluor-benzylamid |
| "A118" | 1-(3,4-Dihydro-2H-pyrido[3,2-b][1,4]oxazin-7-yl)-3-hydroxy-2-oxo-pyrrolidin-3-carbonsäure-3-chlor-5-fluor-benzylamid |
| "A119" | 3-Hydroxy-5-methyl-2-oxo-1-phenyl-pyrrolidin-3-carbonsäure- 3-chlor-5-fluor-benzylamid |
| "A120" | 3-[(3-Chlor-5-fluor-benzylamino)-methyl]-1-phenyl-pyrrolidin-3-ol |
| "A121" | 1-Benzyl-3-hydroxy-pyrrolidin-3-carbonsäure-3-chlor-5-fluor-benzylamid |
| "A122" | 1-(4-Acetylamino-cyclohexyl)-3-hydroxy-2-oxo-pyrrolidin-3-carbonsäure-3-chlor-5-fluor-benzylamid |
| "A123" | 3-Hydroxy-1-(4-methansulfonylamino-cyclohexyl)-2-oxo-pyrrolidin-3-carbonsäure-3-chlor-5-fluor-benzylamid |
| "A124" | 1-(4-Ethansulfonylamino-cyclohexyl)-3-hydroxy-2-oxo-pyrrolidin-3-carbonsäure-3-chlor-5-fluor-benzylamid |
| "A125" | 1-((1S,2R,3S)-2,3-Dihydroxy-cyclohexyl)-3-hydroxy-2-oxo-pyrrolidin-3-carbonsäure-3-chlor-5-fluor-benzylamid |
| "A126" | 1-(3-Acetylamino-cyclohexyl)-3-hydroxy-2-oxo-pyrrolidin-3-carbonsäure-3-chlor-5-fluor-benzylamide |
| "A127" | 1-(4-Ethylamino-cyclohexyl)-3-hydroxy-2-oxo-pyrrolidin-3-carbonsäure-3-chlor-5-fluor-benzylamid |
| "A128" | 1-(3-Ethansulfonylamino-cyclohexyl)-3-hydroxy-2-oxo-pyrrolidin-3-carbonsäure-3-chlor-5-fluor-benzylamid |
| "A129" | 1-((3aR,4S,7aS)-2,2-Dimethyl-hexahydro-benzo[1,3]dioxol-4-yl)-3-hydroxy-2-oxo-pyrrolidin-3-carbonsäure-3-chlor-5-fluor-benzylamid |
| "A130" | 5-[3-(3-Chlor-5-fluor-benzylcarbamoyl)-3-hydroxy-2-oxo-pyrrolidin-1-yl]-isophthalamid |
| "A131" | (R)-3-Hydroxy-2-oxo-[1,3']bipyrrolidinyl-3-carbonsäure-3-chlor-5-fluor-benzylamid |
| "A132" | (R)-3-Hydroxy-2-oxo-1-(2-oxo-1,2,3,4-tetrahydro-chinolin-6-yl)-pyrrolidin-3-carbonsäure-3-chlor-5-fluor-benzylamid |
| "A133" | 3-Hydroxy-1-(3-methylcarbamoyl-phenyl)-2-oxo-pyrrolidin-3-carbonsäure-3-chlor-5-fluor-benzylamid |
| "A134" | 1-(3-Dimethylcarbamoyl-phenyl)-3-hydroxy-2-oxo-pyrrolidin-3-carbonsäure-3-chlor-5-fluor-benzylamid |
| "A135" | 3-Hydroxy-1-(4-methoxy-benzyl)-2-oxo-pyrrolidin-3-carbonsäure-3-chlor-5-fluor-benzylamid |
| "A136" | 1-Benzyl-3-hydroxy-2-oxo-pyrrolidin-3-carbonsäure-3-chlor-5-fluor-benzylamid |
| "A137" | 3-Hydroxy-2-oxo-1-(2-oxo-1,2,3,4-tetrahydro-chinolin-6-yl)-pyrrolidin-3-carbonsäure-[2-(3-chlor-5-fluor-phenyl)-ethyl]-amid |
| "A138" | 3-Hydroxy-1-(6-methoxy-pyridin-3-yl)-2-oxo-pyrrolidin-3-carbonsäure-3-chlor-5-fluor-benzylamid |
| "A139" | 1-(6-Acetylamino-pyridin-3-yl)-3-hydroxy-2-oxo-pyrrolidin-3-carbonsäure-[2-(3-chlor-5-fluor-phenyl)-ethyl]-amid |

(fortgesetzt)

| Verbindung Nr. | Name |
|---|---|
| "A140" | (S)-1-Benzyl-3-hydroxy-2-oxo-pyrrolidin-3-carbonsäure-3-chlor-5-fluor-benzylamid |
| "A141" | (R)-1-Benzyl-3-hydroxy-2-oxo-pyrrolidin-3-carbonsäure-3-chlor-5-fluor-benzylamid |
| "A142" | 3-Hydroxy-1-[5-methyl-6-(3-oxo-morpholin-4-yl)-pyridin-3-yl]-2-oxo-pyrrolidin-3-carbonsäure-3-chlor-5-fluor-benzylamid |
| "A143" | 1-(6-Acetylamino-pyridin-3-yl)-3-hydroxy-2-oxo-pyrrolidin-3-carbonsäure-[2-(2-trifluormethyl-phenyl)-ethyl]-amid |
| "A144" | 1-(3-Carbamoyl-phenyl)-3-hydroxy-2-oxo-pyrrolidin-3-carbonsäure-[2-(2-trifluormethyl-phenyl)-ethyl]-amid |
| "A145" | 3-Hydroxy-2-oxo-1-phenyl-pyrrolidin-3-carbonsäure-[1-(3-fluor-phenyl)-ethyl]-amid |
| "A146" | 3-Hydroxy-1-(2-methylcarbamoyl-benzofuran-5-yl)-2-oxo-pyrrolidin-3-carbonsäure-3-chlor-5-fluor-benzylamid |
| "A147" | 3-Hydroxy-2-oxo-1-(2-oxo-1,2-dihydro-chinolin-6-yl)-pyrrolidin-3-carbonsäure-3-chlor-5-fluor-benzylamid |
| "A148" | 4-[3-(3-Chlor-5-fluor-benzylcarbamoyl)-3-hydroxy-2-oxo-pyrrolidin-1-yl]-cyclohexancarbonsäure-ethylester |
| "A149" | (S)-3-Hydroxy-2-oxo-1-(2-oxo-1,2,3,4-tetrahydro-chinolin-6-yl)-pyrrolidin-3-carbonsäure-[2-(3-chlor-5-fluor-phenyl)-ethyl]-amid |
| "A150" | (R)-3-Hydroxy-2-oxo-1-(2-oxo-1,2,3,4-tetrahydro-chinolin-6-yl)-pyrrolidin-3-carbonsäure-[2-(3-chlor-5-fluor-phenyl)-ethyl]-amid |
| "A151" | 3-Hydroxy-2-oxo-1-(3-oxo-3,4-dihydro-2H-benzo[1,4]oxazin-7-yl)-pyrrolidin-3-carbonsäure-3-chlor-5-fluor-benzylamid |
| "A152" | 4-[3-(3-Chlor-5-fluor-benzylcarbamoyl)-3-hydroxy-2-oxo-pyrrolidin-1-yl]-cyclohexancarbonsäure |
| "A153" | (S)-3-Hydroxy-1-(1H-indol-6-yl)-2-oxo-pyrrolidin-3-carbonsäure-3-chlor-5-fluor-benzylamid |
| "A154" | (R)-3-Hydroxy-1-(1H-indol-6-yl)-2-oxo-pyrrolidin-3-carbonsäure-3-chlor-5-fluor-benzylamid |
| "A155" | (S)-1-(6-Acetylamino-pyridin-3-yl)-3-hydroxy-2-oxo-pyrrolidin-3-carbonsäure-[2-(3-chlor-5-fluor-phenyl)-ethyl]-amid |
| "A156" | (R)-1-(6-Acetylamino-pyridin-3-yl)-3-hydroxy-2-oxo-pyrrolidin-3-carbonsäure-[2-(3-chlor-5-fluor-phenyl)-ethyl]-amid |
| "A157" | 3-Hydroxy-1-(4-methylcarbamoyl-cyclohexyl)-2-oxo-pyrrolidin-3-carbonsäure-3-chlor-5-fluor-benzylamid |
| "A158" | 2-Oxo-1-(2-oxo-1,2,3,4-tetrahydro-chinolin-6-yl)-pyrrolidin-3-carbonsäure-(2-oxo-1,2,3,4-tetrahydro-chinolin-6-yl)-amid |
| "A159" | (R)-1-(3-Carbamoyl-phenyl)-3-hydroxy-2-oxo-pyrrolidin-3-carbonsäure-[2-(3-chlor-5-fluor-phenyl)-ethyl]-amid |
| "A160" | (R)-3-Hydroxy-1-(3-methansulfonylamino-phenyl)-2-oxo-pyrrolidin-3-carbonsäure-3-chlor-5-fluor-benzylamid |
| "A161" | (S)-3-Hydroxy-1-(3-methylcarbamoyl-phenyl)-2-oxo-pyrrolidin-3-carbonsäure-3-chlor-5-fluor-benzylamid |
| "A162" | (R)-3-Hydroxy-1-(3-methylcarbamoyl-phenyl)-2-oxo-pyrrolidin-3-carbonsäure-3-chlor-5-fluor-benzylamid |
| "A163" | (S)-1-(3-Cyclobutylcarbamoyl-phenyl)-3-hydroxy-2-oxo-pyrrolidin-3-carbonsäure-3-chlor-5-fluor-benzylamid |

(fortgesetzt)

| Verbindung Nr. | Name |
|---|---|
| "A164" | (R)-1-(3-Cyclobutylcarbamoyl-phenyl)-3-hydroxy-2-oxo-pyrrolidin-3-carbonsäure-3-chlor-5-fluor-benzylamid |
| "A165" | (S)-3-Hydroxy-1-[3-(methansulfonyl-methyl-amino)-phenyl]-2-oxo-pyrrolidin-3-carbonsäure-3-chlor-5-fluor-benzylamid |
| "A166" | (R)-3-Hydroxy-1-[3-(methansulfonyl-methyl-amino)-phenyl]-2-oxo-pyrrolidin-3-carbonsäure-3-chlor-5-fluor-benzylamid |
| "A167" | 3-Hydroxy-2-oxo-1-[6-(3-oxo-morpholin-4-yl)-pyridin-3-yl]-pyrrolidin-3-carbonsäure-3-chlor-5-fluor-benzylamid |
| "A168" | 3-Hydroxy-1-(1H-indazol-5-yl)-2-oxo-pyrrolidin-3-carbonsäure-[2-(3-chlor-5-fluor-phenyl)-ethyl]-amid |
| "A169" | 3-Hydroxy-2-oxo-1-(1-phenyl-1H-pyrazol-4-yl)-pyrrolidin-3-carbonsäure-3-chlor-5-fluor-benzylamid |
| "A170" | 4-{4-[3-(3-Chlor-5-fluor-benzylcarbamoyl)-3-hydroxy-2-oxo-pyrrolidin-1-yl}-pyrazol-1-yl}-piperidin-1-carbonsäure-tert-butylester |
| "A171" | (S)-3-Hydroxy-2-oxo-1-(6-propionylamino-pyridin-3-yl)-pyrrolidin-3-carbonsäure-3-chlor-5-fluor-benzylamid |
| "A172" | (R)-3-Hydroxy-2-oxo-1-(6-propionylam ino-pyridin-3-yl)-pyrrolidin-3-carbonsäure-3-chlor-5-fluor-benzylamid |
| "A173" | 3-Hydroxy-1-(6-morpholin-4-yl-pyridin-3-yl)-2-oxo-pyrrolidin-3-carbonsäure-3-chlor-5-fluor-benzylamid |
| "A174" | 1-(6-Acetylamino-pyridin-3-yl)-3-hydroxy-2-oxo-pyrrolidin-3-carbonsäure-[2-(3-fluor-phenyl)-ethyl]-amid |
| "A175" | 1-(6-Acetylamino-pyridin-3-yl)-3-hydroxy-2-oxo-pyrrolidin-3-carbonsäure-[2-(2-fluor-phenyl)-ethyl]-amid |
| "A176" | 1-(6-Acetylamino-pyridin-3-yl)-3-hydroxy-2-oxo-pyrrolidin-3-carbonsäure-3,5-difluor-benzylamid |
| "A177" | 1-(3-Carbamoyl-phenyl)-3-hydroxy-2-oxo-pyrrolidin-3-carbonsäure-[2-(3-fluor-phenyl)-ethyl]-amid |
| "A178" | 1-(3-Carbamoyl-phenyl)-3-hydroxy-2-oxo-pyrrolidin-3-carbonsäure-3,5-difluor-benzylamid |
| "A179" | 1-(3-Carbamoyl-phenyl)-3-hydroxy-2-oxo-pyrrolidin-3-carbonsäure-[2-(2-fluor-phenyl)-ethyl]-amid |
| "A180" | 3-Hydroxy-2-oxo-1-(1-piperidin-4-yl-1 H-pyrazol-4-yl)-pyrrolidin-3-carbonsäure-3-chlor-5-fluor-benzylamid |
| "A181" | (S)-3-Hydroxy-1-(4-methoxy-benzyl)-2-oxo-pyrrolidin-3-carbonsäure-3-chlor-5-fluor-benzylamid |
| "A182" | (R)-3-Hydroxy-1-(4-methoxy-benzyl)-2-oxo-pyrrolidin-3-carbonsäure-3-chlor-5-fluor-benzylamid |
| "A183" | 3-Hydroxy-2-oxo-1-(2-oxo-1,2,3,4-tetrahydro-chinolin-6-yl)-pyrrolidin-3-carbonsäure-[2-(3-fluor-phenyl)-ethyl]-amid |
| "A184" | 3-Hydroxy-2-oxo-1-(2-oxo-1,2,3,4-tetrahydro-chinolin-6-yl)-pyrrolidin-3-carbonsäure-[2-(2-fluor-phenyl)-ethyl]-amid |
| "A185" | 3-Hydroxy-2-oxo-1-(2-oxo-1,2,3,4-tetrahydro-chinolin-6-yl)-pyrrolidin-3-carbonsäure-3,5-difluor-benzylamid |

(fortgesetzt)

| Verbindung Nr. | Name |
|---|---|
| "A186" | 1-(6-Acetylamino-pyridin-3-yl)-3-hydroxy-2-oxo-pyrrolidin-3-carbonsäure-2-chlor-3-fluor-benzylamid |
| "A187" | 3-Hydroxy-2-oxo-1-phenylcarbamoylmethyl-pyrrolidin-3-carbonsäure-3-chlor-5-fluor-benzylamid |
| "A188" | 1-(6-Acetylamino-pyridin-3-yl)-3-hydroxy-2-oxo-pyrrolidin-3-carbonsäure-[2-(3,5-difluor-phenyl)-ethylj-amid |
| "A189" | (S)-3-Hydroxy-1-(1H-indazol-5-yl)-2-oxo-pyrrolidin-3-carbonsäure-3-chlor-5-fluor-benzylamid |
| "A190" | (R)-3-Hydroxy-1-(1H-indazol-5-yl)-2-oxo-pyrrolidin-3-carbonsäure-3-chlor-5-fluor-benzylamid |
| "A191" | (S)-3-Hydroxy-1-(1H-indazol-5-yl)-2-oxo-pyrrolidin-3-carbonsäure-[2-(3-chlor-5-fluor-phenyl)-ethyl]-amid |
| "A192" | (R)-3-Hydroxy-1-(1H-indazol-5-yl)-2-oxo-pyrrolidin-3-carbonsäure-[2-(3-chlor-5-fluor-phenyl)-ethyl]-amid |
| "A193" | 1-(3-Carbamoyl-phenyl)-3-hydroxy-2-oxo-pyrrolidin-3-carbonsäure-2-chlor-3-fluor-benzylamid |
| "A194" | (S)-1-(2-Ethylcarbamoyl-benzofuran-5-yl)-3-hydroxy-2-oxo-pyrrolidin-3-carbonsäure-3-chlor-5-fluor-benzylamid |
| "A195" | (R)-1-(2-Ethylcarbamoyl-benzofuran-5-yl)-3-hydroxy-2-oxo-pyrrolidin-3-carbonsäure-3-chlor-5-fluor-benzylamid |
| "A196" | 3-Hydroxy-2-oxo-1-(2-oxo-1,2,3,4-tetrahydro-chinolin-6-yl)-pyrrolidin-3-carbonsäure-2-chlor-3-fluor-benzylamid |
| "A197" | (S)-3-Hydroxy-2-oxo-1-(3-oxo-3,4-dihydro-2H-benzo[1,4]oxazin-7-yl)-pyrrolidin-3-carbonsäure-3-chlor-5-fluor-benzylamid |
| "A198" | (R)-3-Hydroxy-2-oxo-1-(3-oxo-3,4-dihydro-2H-benzo[1,4]oxazin-7-yl)-pyrrolidin-3-carbonsäure-3-chlor-5-fluor-benzylamid |
| "A199" | 3-Hydroxy-2-oxo-1-(2-oxo-1,2,3,4-tetrahydro-chinolin-6-yl)-pyrrolidin-3-carbonsäure-[2-(3,5-difluor-phenyl)-ethyl]-amid |
| "A200" | 3-Hydroxy-1-(2-methyl-1H-indol-5-yl)-2-oxo-pyrrolidin-3-carbonsäure-3-chlor-5-fluor-benzylamid |
| "A201" | 1-(3-Carbamoyl-phenyl)-3-hydroxy-2-oxo-pyrrolidin-3-carbonsäure-[2-(3,5-difluor-phenyl)-ethyl]-amid |
| "A202" | 3-[3-(3-Chlor-5-fluor-benzylcarbamoyl)-3-hydroxy-2-oxo-pyrrolidin-1-yl]-cyclohexancarbonsäure-ethylester |
| "A203" | [3-(3-Chlor-5-fluor-benzylcarbamoyl)-3-hydroxy-2-oxo-pyrrolidin-1-yl]-essigsäure |
| "A204" | 1-(3-Carbamoyl-5-trifluoromethyl-phenyl)-3-hydroxy-2-oxo-pyrrolidin-3-carbonsäure-3-chlor-5-fluor-benzylamid |
| "A205" | 5-[3-(3-Chlor-5-fluor-benzylcarbamoyl)-3-hydroxy-2-oxo-pyrrolidin-1-yl]-1H-indol-2-carbonsäure-ethylester |
| "A206" | 3-Hydroxy-1-(1H-indol-5-yl)-2-oxo-pyrrolidin-3-carbonsäure-2-chlor-3-fluor-benzylamid |
| "A207" | 3-Hydroxy-1-(1H-indol-5-yl)-2-oxo-pyrrolidin-3-carbonsäure-[2-(3,5-difluor-phenyl)-ethyl]-amid |
| "A208" | 3-Hydroxy-1-methylcarbamoylmethyl-2-oxo-pyrrolidin-3-carbonsäure-3-chlor-5-fluor-benzylamid |
| "A209" | 3-Hydroxy-1-(1H-indol-5-yl)-2-oxo-pyrrolidin-3-carbonsäure-3,5-difluor-benzylamid |

(fortgesetzt)

| Verbindung Nr. | Name |
|---|---|
| "A210" | 3-Hydroxy-1-(1H-indol-5-yl)-2-oxo-pyrrolidin-3-carbonsäure-[2-(2-fluor-phenyl)-ethyl]-amid |
| "A211" | 5-[3-(3-Chlor-5-fluor-benzylcarbamoyl)-3-hydroxy-2-oxo-pyrrolidin-1-yl]-1H-indol-2-carboxamid |
| "A212" | 3-Hydroxy-1-(1H-indol-5-yl)-2-oxo-pyrrolidin-3-carbonsäure-[2-(3-fluor-phenyl)-ethyl]-amid |
| "A213" | 3-[3-(3-Chlor-5-fluor-benzylcarbamoyl)-3-hydroxy-2-oxo-pyrrolidin-1-ylmethyl]-azetidin-1-carbonsäure-tert-butylester |
| "A214" | 3-Hydroxy-2-oxo-1-(7-oxo-5,6,7,8-tetrahydro-[1,8]naphthyridin-3-yl)-pyrrolidin-3-carbonsäure-3-chlor-5-fluor-benzylamid |
| "A215" | 3-Hydroxy-2-oxo-1-(1H-pyrrolo[2,3-b]pyridin-5-yl)-pyrrolidin-3-carbonsäure-3-chlor-5-fluor-benzylamid |
| "A216" | 5-[3-(3-Chlor-5-fluor-benzylcarbamoyl)-3-hydroxy-2-oxo-pyrrolidin-1-yl]-nicotinamid |
| "A217" | 1-(2-Cyan-1H-indol-5-yl)-3-hydroxy-2-oxo-pyrrolidin-3-carbonsöure-3-chlor-5-fluor-benzylamid |
| "A218" | 1-Azetidin-3-ylmethyl-3-hydroxy-2-oxo-pyrrolidin-3-carbonsäure-3-chlor-5-fluor-benzylamid |
| "A219" | 1-[2-(5-Fluor-1H-indol-3-yl)-ethyl]-3-hydroxy-2-oxo-pyrrolidin-3-carbonsäure-3-chlor-5-fluor-benzylamid |
| "A220" | 3-Hydroxy-2-oxo-1-(tetrahydro-pyran-4-ylmethyl)-pyrrolidin-3-carbonsäure-3-chlor-5-fluor-benzylamid |
| "A221" | (S)-3-Hydroxy-2-oxo-1-(2-oxo-1,2,3,4-tetrahydro-chinolin-6-yl)-pyrrolidin-3-carbonsäure-[2-(3,5-difluor-phenyl)-ethyl]-amid |
| "A222" | (S)-1-(3-Cyan-1H-indol-5-yl)-3-hydroxy-2-oxo-pyrrolidin-3-carbonsäure-3-chlor-5-fluor-benzylamid |
| "A223" | (R)-3-Hydroxy-2-oxo-1-(2-oxo-1,2,3,4-tetrahydro-chinolin-6-yl)-pyrrolidin-3-carbonsäure-[2-(3,5-difluor-phenyl)-ethyl]-amid |
| "A224" | (R)-1-(3-Cyan-1H-indol-5-yl)-3-hydroxy-2-oxo-pyrrolidin-3-carbonsäure-3-chlor-5-fluor-benzylamid |
| "A225" | 3-Hydroxy-2-oxo-1-[(R)-1-(tetrahydro-furan-2-yl)methyl]-pyrrolidin-3-carbonsäure-3-chlor-5-fluor-benzylamid |
| "A226" | 4-[3-(3-Chlor-5-fluor-benzylcarbamoyl)-3-hydroxy-2-oxo-pyrrolidin-1-ylmethyl]-cyclohexancarbonsäure |
| "A227" | 4-[3-(3-Chlor-5-fluor-benzylcarbamoyl)-3-hydroxy-2-oxo-pyrrolidin-1-ylmethyl]-cyclohexancarbonsäure |
| "A228" | (1S,5R,6S)-6-[3-(3-Chlor-5-fluor-benzylcarbamoyl)-3-hydroxy-2-oxo-pyrrolidin-1-yl]-3-aza-bicyclo[3.1.0]hexan-3-carbonsäure-tert-butylester |
| "A229" | 1-(1S,5R,6S)-3-Aza-bicyclo[3.1.0]hex-6-yl-3-hydroxy-2-oxo-pyrrolidin-3-carbonsäure-3-chlor-5-fluor-benzylamid |
| "A230" | 1-((1S,5R,6S)-3-Ethansulfonyl-3-aza-bicyclo[3.1.0]hex-6-yl)-3-hydroxy-2-oxo-pyrrolidin-3-carbonsäure-3-chlor-5-fluor-benzylamid |
| "A231" | 1-((1S,5R,6S)-3-Acetyl-3-aza-bicyclo[3.1.0]hex-6-yl)-3-hydroxy-2-oxo-pyrrolidin-3-carbonsäure-3-chlor-5-fluor-benzylamid |
| "A232" | 3-Hydroxy-1-(1-methyl-1H-benzimidazol-2-ylmethyl)-2-oxo-pyrrolidin-3-carbonsäure-3-chlor-5-fluor-benzylamid |
| "A233" | (S)-3-Hydroxy-2-oxo-[1,3']bipyrrolidinyl-3-carbonsäure-3-chlor-5-fluor-benzylamid |

(fortgesetzt)

| Verbindung Nr. | Name |
|---|---|
| "A234" | (S)-3-Hydroxy-2-oxo-1-(2-oxo-1,2,3,4-tetrahydro-chinolin-6-yl)-pyrrolidin-3-carbonsäure-2-chlor-4,5-difluor-benzylamid |
| "A235" | (S)-3-Hydroxy-2-oxo-1-(2-oxo-1,2,3,4-tetrahydro-chinolin-6-yl)-pyrrolidin-3-carbonsäure-5-chlor-2,4-difluor-benzylamid |
| "A236" | (S)-3-Hydroxy-2-oxo-1-(2-oxo-1,2,3,4-tetrahydro-chinolin-6-yl)-pyrrolidin-3-carbonsäure-2,4,6-trifluor-benzylamid |
| "A237" | 1-[2-(2,4-Difluor-phenyl)-ethyl]-3-hydroxy-2-oxo-pyrrolidin-3-carbonsäure-3-chlor-5-fluor-benzylamid |
| "A238" | 5-[(S)-3-(3-Chlor-5-fluor-benzylcarbamoyl)-3-hydroxy-2-oxo-pyrrolidin-1-yl]-1H-indol-3-carboxamid |
| "A239" | (S)-3-Hydroxy-1-(2-methyl-1H-indol-5-yl)-2-oxo-pyrrolidin-3-carbonsäure-3-chlor-5-fluor-benzylamid |
| "A240" | (R)-3-Hydroxy-1-(2-methyl-1H-indol-5-yl)-2-oxo-pyrrolidin-3-carbonsäure-3-chlor-5-fluor-benzylamid |
| "A241" | (S)-3-Hydroxy-2-oxo-1-(2-oxo-1,2,3,4-tetrahydro-chinolin-6-yl)-pyrrolidin-3-carbonsäure-4-chlor-2-fluor-benzylamid |
| "A242" | (S)-3-Hydroxy-2-oxo-1-(2-oxo-1,2,3,4-tetrahydro-chinolin-6-yl)-pyrrolidin-3-carbonsäure-2-chlor-4-fluor-benzylamid |
| "A243" | 3-Hydroxy-2-oxo-1-[(S)-1-(tetrahydro-furan-2-yl)methyl]-pyrrolidin-3-carbonsäure-3-chlor-5-fluor-benzylamid |
| "A244" | (S)-3-Hydroxy-2-oxo-1-(2-oxo-1,2,3,4-tetrahydro-chinolin-6-yl)-pyrrolidin-3-carbonsäure-2-chlor-3,6-difluor-benzylamid |
| "A245" | (S)-3-Hydroxy-2-oxo-1-(2-oxo-1,2,3,4-tetrahydro-chinolin-6-yl)-pyrrolidin-3-carbonsäure-2,4,5-trifluor-benzylamid |
| "A246" | (S)-3-Hydroxy-2-oxo-1-(2-oxo-1,2,3,4-tetrahydro-chinolin-6-yl)-pyrrolidin-3-carbonsäure-2,3,6-trifluor-benzylamid |
| "A247" | (S)-3-Hydroxy-2-oxo-1-(2-oxo-1,2,3,4-tetrahydro-chinolin-6-yl)-pyrrolidin-3-carbonsäure-3-chlor-4-fluor-benzylamid |
| "A248" | 1-Carbamoylmethyl-3-hydroxy-2-oxo-pyrrolidin-3-carbonsäure-3-chlor-5-fluor-benzylamid |
| "A249" | (S)-3-Hydroxy-2-oxo-1-(2-oxo-1,2,3,4-tetrahydro-chinolin-6-yl)-pyrrolidin-3-carbonsäure-4-chlor-2,6-difluor-benzylamid |
| "A250" | (S)-3-Hydroxy-2-oxo-1-(2-oxo-1,2,3,4-tetrahydro-chinolin-6-yl)-pyrrolidin-3-carbonsäure-3-chlor-2,6-difluor-benzylamid |
| "A251" | (S)-3-Hydroxy-2-oxo-1-(2-oxo-1,2,3,4-tetrahydro-chinolin-6-yl)-pyrrolidin-3-carbonsäure-3-chlor-2,4-difluor-benzylamid |
| "A252" | 1-Cyclobutylmethyl-3-hydroxy-2-oxo-piperidin-3-carbonsäure-3,5-difluor-benzylamid |
| "A253" | 1-(3,4-Difluor-benzyl)-3-hydroxy-2-oxo-piperidin-3-carbonsäure-3-fluor-benzylamid |
| "A254" | (S)-1-(2,3-Difluor-benzyl)-3-hydroxy-2-oxo-piperidin-3-carbonsäure-3-fluor-benzylamid |
| "A255" | (S)-1-(2,3-Difluor-benzyl)-3-hydroxy-2-oxo-piperidin-3-carbonsäure-4-fluor-benzylamid |
| "A256" | (S)-1-Cyclohexylmethyl-3-hydroxy-2-oxo-piperidin-3-carbonsäure-3-fluor-benzylamid |
| "A257" | 1-(3,4-Difluor-benzyl)-3-hydroxy-2-oxo-piperidin-3-carbonsäure-4-fluor-benzylamid |
| "A258" | 1-(3,4-Difluor-benzyl)-3-hydroxy-2-oxo-piperidin-3-carbonsäure-(5-methyl-isoxazol-3-ylmethyl)-amid |

(fortgesetzt)

| Verbindung Nr. | Name |
|---|---|
| "A259" | (S)-1-(2,3-Difluor-benzyl)-3-hydroxy-2-oxo-piperidin-3-carbonsäure-(pyridin-2-ylmethyl)-amid |
| "A260" | (R)-1-(2,3-Difluor-benzyl)-3-hydroxy-2-oxo-piperidin-3-carbonsäure-(pyridin-2-ylmethyl)-amid |
| "A261" | (R)-1-(2,3-Difluor-benzyl)-3-hydroxy-2-oxo-piperidin-3-carbonsäure-3-fluor-benzylamid |
| "A262" | (R)-1-(2,3-Difluor-benzyl)-3-hydroxy-2-oxo-piperidin-3-carbonsäure-4-fluor-benzylamid |
| "A263" | (R)-1-Cyclohexylmethyl-3-hydroxy-2-oxo-piperidin-3-carbonsäure-3-fluor-benzylamid |
| "A264" | (S)-3-Hydroxy-2-oxo-1-(2-oxo-1,2,3,4-tetrahydro-chinolin-6-yl)-pyrrolidin-3-carbonsäure-3,4,5-trifluor-benzylamid |
| "A265" | (S)-3-Hydroxy-2-oxo-1-(2-oxo-1,2,3,4-tetrahydro-chinolin-6-yl)-pyrrolidin-3-carbonsäure-4-chlor-3-fluor-benzylamid |
| "A266" | (S)-3-Hydroxy-2-oxo-1-(2-oxo-1,2,3,4-tetrahydro-chinolin-6-yl)-pyrrolidin-3-carbonsäure-2,3,5-trifluor-benzylamid |
| "A267" | 3-(1,3-Dihydro-isoindol-2-carbonyl)-3-hydroxy-1-phenyl-pyrrolidin-2-on |
| "A268" | 1-Benzyl-3-(1,3-dihydro-isoindol-2-carbonyl)-3-hydroxy-pyrrolidin-2-on |
| "A269" | 1-(1H-Benzimidazol-2-ylmethyl)-3-hydroxy-2-oxo-pyrrolidin-3-carbonsäure-3-chlor-5-fluor-benzylamid |
| "A270" | 1-Benzothiazol-2-ylmethyl-3-hydroxy-2-oxo-pyrrolidin-3-carbonsäure-3-chlor-5-fluor-benzylamid |
| "A271" | 3-[3-(3-Chlor-5-fluor-benzylcarbamoyl)-3-hydroxy-2-oxo-pyrrolidin-1-yl]-propionsäure |
| "A272" | 3-Hydroxy-1-(3-methyl-3H-imidazol-4-ylmethyl)-2-oxo-pyrrolidin-3-carbonsäure-3-chlor-5-fluor-benzylamid |
| "A273" | 4-[3-(3-Chlor-5-fluor-benzylcarbamoyl)-3-hydroxy-2-oxo-pyrrolidin-1-ylmethyl]-cyclohexancarbonsäure |
| "A274" | 4-[3-(3-Chlor-5-fluor-benzylcarbamoyl)-3-hydroxy-2-oxo-pyrrolidin-1-ylmethyl]-cyclohexancarbonsäure |
| "A275" | 1-((1S,5R,6S)-3-Ethyl-3-aza-bicyclo[3.1.0]hex-6-yl)-3-hydroxy-2-oxo-pyrrolidin-3-carbonsäure-3-chlor-5-fluor-benzylamid |
| "A276" | 3-Hydroxy-1-(2-methyl-2H-pyrazol-3-ylmethyl)-2-oxo-pyrrolidin-3-carbonsäure-3-chlor-5-fluor-benzylamid |
| "A277" | 3-Hydroxy-2-oxo-1-(1H-pyrazol-3-ylmethyl)-pyrrolidin-3-carbonsäure-3-chlor-5-fluor-benzylamid |
| "A278" | 3-Hydroxy-1-(4-methylcarbamoyl-cyclohexylmethyl)-2-oxo-pyrrolidin-3-carbonsäure-3-chlor-5-fluor-benzylamid |
| "A279" | 3-Hydroxy-1-(1H-imidazol-4-ylmethyl)-2-oxo-pyrrolidin-3-carbonsäure-3-chlor-5-fluor-benzylamid |
| "A280" | 1-(1,5-Dimethyl-1H-pyrrol-2-ylmethyl)-3-hydroxy-2-oxo-pyrrolidin-3-carbonsäure-3-chlor-5-fluor-benzylamid |
| "A281" | (S)-3-Hydroxy-1'-methansulfonyl-2-oxo-[1,3']bipyrrolidinyl-3-carbonsäure-3-chlor-5-fluor-benzylamid |
| "A282" | 3-Hydroxy-1-[(2-hydroxy-ethylcarbamoyl)-methyl]-2-oxo-pyrrolidin-3-carbonsäure-3-chlor-5-fluor-benzylamid |
| "A283" | (S)-3-Hydroxy-2-oxo-1-(2-oxo-1,2,3,4-tetrahydro-chinolin-6-yl)-pyrrolidin-3-carbonsäure-2-chlor-5-fluor-benzylamid |

(fortgesetzt)

| Verbindung Nr. | Name |
|---|---|
| "A284" | (S)-3-Hydroxy-2-oxo-1-(2-oxo-1,2,3,4-tetrahydro-chinolin-6-yl)-pyrrolidin-3-carbonsäure-2,3,4-trifluor-benzyiamid |
| "A285" | (S)-3-Hydroxy-2-oxo-1-(2-oxo-1,2,3,4-tetrahydro-chinolin-6-yl)-pyrrolidin-3-carbonsäure-2,3-difluor-benzylamid |
| "A286" | (S)-1-(3,3-Difluor-2-oxo-2,3-dihydro-1H-indol-5-yl)-3-hydroxy-2-oxo-pyrrolidin-3-carbonsäure-3-chlor-5-fluor-benzylamid |
| "A287" | 1-(4-Fluor-benzyl)-3-hydroxy-2-oxo-piperidin-3-carbonsäure-3-fluor-benzylamid |
| "A288" | (R)-1-(3,3-Difluor-2-oxo-2,3-dihydro-1H-indol-5-yl)-3-hydroxy-2-oxo-pyrrolidin-3-carbonsäure-3-chlor-5-fluor-benzylamid |
| "A289" | (S)-3-Hydroxy-2-oxo-1-(2-oxo-1,2,3,4-tetrahydro-chinolin-6-yl)-pyrrolidin-3-carbonsäure-2,6-difluor-benzylamid |
| "A290" | 3-Hydroxy-1-(5-methyl-[1,3,4]oxadiazol-2-ylmethyl)-2-oxo-pyrrolidin-3-carbonsäure-3-chlor-5-fluor-benzylamid |
| "A291" | (R)-1'-Acetyl-3-hydroxy-2-oxo-[1,3']bipyrrolidinyl-3-carbonsäure-3-chlor-5-fluor-benzylamid |
| "A292" | (R)-3-Hydroxy-1'-methansulfonyl-2-oxo-[1,3']bipyrrolidinyl-3-carbonsäure-3-chlor-5-fluor-benzylamid |
| "A293" | 3-Hydroxy-1-(4-methyl-cyclohexyl)-2-oxo-pyrrolidin-3-carbonsäure-3-chlor-5-fluor-benzylamid |
| "A294" | 1-(2,3-Difluor-benzyl)-3-hydroxy-2-oxo-piperidin-3-carbonsäure-(5-methyl-isoxazol-3-ylmethyl)-amid |
| "A295" | 1-(2,3-Difluor-benzyl)-3-hydroxy-2-oxo-piperidin-3-carbonsäure-4-methyl-benzylamid |
| "A296" | 1-(2,3-Difluor-benzyl)-3-hydroxy-2-oxo-piperidin-3-carbonsäure-2-fluor-benzylamid |
| "A297" | 1-(2,3-Difluor-benzyl)-3-hydroxy-2-oxo-piperidin-3-carbonsäure-(5-methyl-pyrazin-2-ylmethyl)-amid |
| "A298" | (S)-3-Hydroxy-2-oxo-1-pyridin-2-yl-pyrrolidin-3-carbonsäure-3-chlor-5-fluor-benzylamid |
| "A299" | (R)-3-Hydroxy-2-oxo-1-pyridin-2-yl-pyrrolidin-3-carbonsäure-3-chlor-5-fluor-benzylamid |
| "A300" | 1-(2-Carbamoyl-ethyl)-3-hydroxy-2-oxo-pyrrolidin-3-carbonsäure-3-chlor-5-fluor-benzylamid |
| "A301" | 1-Benzyl-3-hydroxy-piperidin-3-carbonsäure-3-chlor-5-fluor-benzylamid |
| "A302" | 3-[(3-Chlor-5-fluor-benzylamino)-methyl]-3-hydroxy-1-phenyl-piperidin-2-on |
| "A303" | 3,4-Dihydroxy-2-oxo-1-(2-oxo-1,2,3,4-tetrahydro-chinolin-6-yl)-pyrrolidin-3-carbonsäure-3-chlor-5-fluor-benzylamid |
| "A304" | 5-Fluor-3-hydroxy-2-oxo-1-(2-oxo-1,2,3,4-tetrahydro-chinolin-6-yl)-pyrrolidin-3-carbonsäure-3-chlor-5-fluor-benzylamid |
| "B1" | (S)-1-(2-Cyan-1H-indol-5-yl)-3-hydroxy-2-oxo-pyrrolidin-3-carbonsäure-3-chlor-5-fluor-benzylamid |
| "B2" | (S)-1-(8-Fluor-2-oxo-1,2,3,4-tetrahydro-chinolin-6-yl)-3-hydroxy-2-oxo-pyrrolidin-3-carbonsäure-3-chlor-5-fluor-benzylamid |
| "B3" | (S)-3-Hydroxy-2-oxo-1-(2-oxo-1,2,3,4-tetrahydro-chinolin-6-yl)-pyrrolidin-3-carbonsäure-2-chlor-6-fluor-benzylamid |
| "B4" | (S)-3-Hydroxy-2-oxo-1-(2-oxo-1,2,3,4-tetrahydro-chinolin-6-yl)-pyrrolidin-3-carbonsäure-5-chlor-2-fluor-benzylamid |
| "B5" | (S)-3-Hydroxy-2-oxo-1-(2-oxo-1,2,3,4-tetrahydro-chinolin-6-yl)-pyrrolidin-3-carbonsäure-[2-(2-fluor-phenyl)-ethyl]-amid |

(fortgesetzt)

| Verbindung Nr. | Name |
|---|---|
| "B6" | (S)-3-Hydroxy-2-oxo-1-(2-oxo-1,2,3,4-tetrahydro-chinolin-6-yl)-pyrrolidin-3-carbonsäure-[2-(3-fluor-phenyl)-ethyl]-amid |
| "B7" | (S)-3-Hydroxy-2-oxo-1-(2-oxo-1,2,3,4-tetrahydro-chinolin-6-yl)-pyrrolidin-3-carbonsäure-3,5-difluor-benzylamid |
| "B8" | (S)-3-Hydroxy-1-(1H-indol-5-yl)-2-oxo-pyrrolidin-3-carbonsäure-3,5-difluor-benzylamid |
| "B9" | (S)-1-(6-Acetylamino-pyridin-3-yl)-3-hydroxy-2-oxo-pyrrolidin-3-carbonsäure-2-chlor-3-fluor-benzylamid |
| "B10" | (S)-3-Hydroxy-1-(1H-indol-5-yl)-2-oxo-pyrrolidin-3-carbonsäure-[2-(3-fluor-phenyl)-ethyl]-amid |
| "B11" | (S)-3-Hydroxy-2-oxo-1-(1H-pyrrolo[2,3-b]pyridin-5-yl)-pyrrolidin-3-carbonsäure-3-chlor-5-fluor-benzylamid |
| "B12" | 1-(4-Chlor-2-methoxy-5-methyl-phenyl)-3-hydroxy-2-oxo-pyrrolidin-3-carbonsäure-3-chlor-5-fluor-benzylamid |
| "B13" | 1-(5-Chloro-2-methoxy-phenyl)-3-hydroxy-2-oxo-pyrrolidine-3-carboxylic acid 3-chloro-5-fluoro-benzylamide |
| "B14" | (S)-3-Hydroxy-1-(1H-indol-5-yl)-2-oxo-pyrrolidine-3-carboxylic acid [2-(2-fluoro-phenyl)-ethyl]-amide |
| "B15" | (S)-3-Hydroxy-2-oxo-1-(2-oxo-1,2,3,4-tetrahydro-chinolin-6-yl)-pyrrolidin-3-carbonsäure-2-chlor-3-fluor-benzylamid |
| "B16" | 1-Benzyl-3-hydroxy-piperidin-3-carbonsäure-3-chlor-5-fluor-benzylamid |
| "B17" | (S)-1-(4-Brom-3-hydroxymethyl-phenyl)-3-hydroxy-2-oxo-pyrrolidin-3-carbonsäure-3-chlor-5-fluor-benzylamid |
| "B18" | 3-[(3-Chlor-5-fluor-benzylamino)-methyl]-1-cyclohexylmethyl-3-hydroxy-piperidin-2-on |
| "B19" | 1-Benzyl-3-[(3-chlor-5-fluor-benzylamino)-methyl]-piperidin-3-ol |
| "B20" | 1-Benzyl-3-[(3-fluor-benzylamino)-methyl]-piperidin-3-ol |
| "B21" | (S)-1-Benzyl-3-[(4-fluor-benzylamino)-methyl]-piperidin-3-ol |
| "B22" | (S)-1-(2,3-Difluor-benzyl)-3-[(3-fluor-benzylamino)-methyl]-3-hydroxy-piperidin-2-on |
| "B23" | (R)-1-(2,3-Difluor-benzyl)-3-[(3-fluor-benzylamino)-methyl]-3-hydroxy-piperidin-2-on |
| "B24" | (S)-3-Hydroxy-2-oxo-1-(2-oxo-1,2,3,4-tetrahydro-chinolin-6-yl)-pyrrolidin-3-carbonsäure-2,5-difluor-benzylamid |
| "B25" | (S)-3-Hydroxy-2-oxo-1-(2-oxo-1,2,3,4-tetrahydro-chinolin-6-yl)-pyrrolidin-3-carbonsäure-3-chlor-2-fluor-benzylamid |
| "B26" | 1-(2-Fluor-5-trifluormethyl-phenyl)-3-hydroxy-2-oxo-pyrrolidin-3-carbonsäure-3-chlor-5-fluor-benzylamid |
| "B27" | 1-(4-Chlor-3-trifluormethyl-phenyl)-3-hydroxy-2-oxo-pyrrolidin-3-carbonsäure-3-chlor-5-fluor-benzylamid |
| "B28" | 1-(3-Carbamoyl-4-fluor-phenyl)-3-hydroxy-2-oxo-pyrrolidin-3-carbonsäure-3-chlor-5-fluor-benzylamid |
| "B29" | 3-[(3-Chlor-5-fluor-benzylamino)-methyl]-3-hydroxy-1-phenyl-pyrrolidin-2-on |
| "B30" | 1-Benzyl-3-[(3-chlor-5-fluor-benzylamino)-methyl]-pyrrolidin-3-ol |
| "B31" | 1-Benzyl-3-[(3-chlor-5-fluor-benzylamino)-methyl]-3-hydroxy-piperidin-2-on |
| "B32" | 3-[(3-Chlor-5-fluor-benzylamino)-methyl]-3-hydroxy-1-phenyl-piperidin-2-on |

(fortgesetzt)

| Verbindung Nr. | Name |
|---|---|
| "B33" | 3-Hydroxy-1-(2-methyl-3H-imdazo[4,5-b]pyridin-6-yl)-2-oxo-pyrrolidin-3-carbonsäure-3-chlor-5-fluor-benzylamid |
| "B34" | (S)-3-Hydroxy-2-oxo-1-(2-oxo-1,2,3,4-tetrahydro-chinolin-6-yl)-pyrrolidin-3-carbonsäure-3-chlor-2,5-difluor-benzylamid |
| "B35" | 3-Hydroxy-1-(3H-imidazo[4,5-b]pyridin-6-yl)-2-oxo-pyrrolidin-3-carbonsäure-3-chlor-5-fluor-benzylamide |
| "B36" | 3-Hydroxy-2-oxo-1-(2-trifluormethyl-3H-imidazo[4,5-b]pyridin-6-yl)-pyrrolidin-3-carbonsäure-3-chlor-5-fluor-benzylamid |
| "B37" | (S)-3-Hydroxy-1-[2-(1H-indol-3-yl)-ethyl]-2-oxo-pyrrolidin-3-carbonsäure-3-chlor-5-fluor-benzylamide |
| "B38" | (R)-3-Hydroxy-1-[2-(1H-indol-3-yl)-ethyl]-2-oxo-pyrrolidin-3-carbonsäure-3-chlor-5-fluor-benzylamide |
| "B39" | (S)-1-(2-Fluor-5-trifluormethyl-phenyl)-3-hydroxy-2-oxo-pyrrolidin-3-carbonsäure-3-chlor-5-fluor-benzylamid |
| "B40" | (S)-3-Hydroxy-2-oxo-1-(2-oxo-1,2-dihydro-chinolin-6-yl)-pyrrolidin-3-carbonsäure-3-chlor-5-fluor-benzylamid |
| "B41" | (R)-3-Hydroxy-2-oxo-1-(2-oxo-1,2-dihydro-chinolin-6-yl)-pyrrolidin-3-carbonsäure-3-chlor-5-fluor-benzylamid |
| "B42" | (R)-1-(2-Fluor-5-trifluoromethyl-phenyl)-3-hydroxy-2-oxo-pyrrolidin-3-carbonsäure-3-chlor-5-fluor-benzylamid |
| "B43" | 3-Hydroxy-2-oxo-1-(3-trifluomethyl-1H-indol-5-yl)-pyrrolidin-3-carbonsäure-3-chlor-5-fluor-benzylamid |
| "B44" | 1-Benzyl-3-[(3,5-difluor-benzylamino)-methyl]-piperidin-3-ol |
| "B45" | 3-[(4-Fluor-benzylamino)-methyl]-3-hydroxy-1-phenyl-pyrrolidin-2-on |
| "B46" | 3-[(3,5-Difluor-benzylamino)-methyl]-3-hydroxy-1-phenyl-pyrrolidin-2-on |
| "B47" | 3-{[2-(4-Fluor -phenyl)-ethylamino]-methyl}-3-hydroxy-1-phenyl-pyrrolidin-2-on |
| "B48" | 3-[(3-Fluor-benzylamino)-methyl]-3-hydroxy-1-phenyl-pyrrolidin-2-on |
| "B49" | 3-Hydroxy-3-[(4-methyl-benzylamino)-methyl]-1-phenyl-pyrrolidin-2-on |
| "B50" | 3-Hydroxy-2-oxo-1-(2-oxo-1,2,3,4-tetrahydro-chinolin-6-yl)-pyrrolidin-3-carbonsäure-(2-oxo-1,2,3,4-tetrahydro-chinolin-6-yl)-amid |
| "B51" | 3-Hydroxy-2-oxo-pyrrolidin-1,3-dicarbonsäure-3-benzylester-1-tert-butylester |
| "B52" | (S)-3-Hydroxy-2-oxo-1-(4-trifluormethyl-pyridin-2-yl)-pyrrolidin-3-carbonsäure-3-chlor-5-fluor-benzylamid |
| "B53" | 3-Hydroxy-2-oxo-pyrrolidin-1,3-dicarbonsäure-1-benzylester 3-isopropylester |
| "B54" | (S)-1-Benzyl-3-[(3-chlor-5-fluor-benzylamino)-methyl]-3-hydroxy-piperidin-2-on |
| "B55" | (R)-1-Benzyl-3-[(3-chlor-5-fluor-benzylamino)-methyl]-3-hydroxy-piperidin-2-on |
| "B56" | (S)-3-Hydroxy-2-oxo-1-(2-oxo-1,2,3,4-tetrahydro-chinolin-6-yl)-pyrrolidin-3-carbonsäure-3-fluor-benzylamid |
| "B57" | (R)-3-Hydroxy-2-oxo-1-(2-oxo-1,2,3,4-tetrahydro-chinolin-6-yl)-pyrrolidin-3-carbonsäure-3-fluor-benzylamid |
| "B58" | (R)-3-[(3-Chlor-5-fluor-benzylamino)-methyl]-3-hydroxy-1-phenyl-pyrrolidin-2-on |
| "B59" | (S)-3-[(3-Chlor-5-fluor-benzylamino)-methyl]-3-hydroxy-1-phenyl-pyrrolidin-2-on |

(fortgesetzt)

| Verbindung Nr. | Name |
|---|---|
| "B60" | (S)-3-Hydroxy-1-(1H-indazol-3-ylmethyl)-2-oxo-pyrrolidin-3-carbonsäure-3-chlor-5-fluor-benzylamid |
| "B61" | (R)-3-Hydroxy-1-(2-methyl-1H-pyrrolo[2,3-b]pyridin-5-yl)-2-oxo-pyrrolidin-3-carbonsäure-3-chlor-5-fluor-benzylamid |
| "B62" | (S)-3-Hydroxy-1-(2-methyl-1H-pyrrolo[2,3-b]pyridin-5-yl)-2-oxo-pyrrolidin-3-carbonsäure-3-chlor-5-fluor-benzylamid |
| "B63" | (S)-3-[(3-Chlor-5-fluor-benzylamino)-methyl]-1-cyclohexylmethyl-3-hydroxy-piperidin-2-on |
| "B64" | (R)-3-[(3-Chlor-5-fluor-benzylamino)-methyl]-1-cyclohexylmethyl-3-hydroxy-piperidin-2-on |
| "B65" | (S)-1-(2-Fluor-4-trifluormethyl-phenyl)-3-hydroxy-2-oxo-pyrrolidin-3-carbonsäure-3-chlor-5-fluor-benzylamid |
| "B66" | (S)-1-(2-Fluor-4-methyl-phenyl)-3-hydroxy-2-oxo-pyrrolidin-3-carbonsäure-3-chlor-5-fluor-benzylamid |
| "B67" | 1-(2-Fluor-5-trifluormethyl-phenyl)-3-hydroxy-2-oxo-piperidin-3-carbonsäure-3-chlor-5-fluor-benzylamid |
| "B68" | 6-{3-[(3-Chlor-5-fluor-benzylamino)-methyl]-3-hydroxy-2-oxo-piperidin-1-yl}-3,4-dihydro-1H-chinolin-2-on |
| "B69" | 3-Hydroxy-2-oxo-1-(2-oxo-1,2,3,4-tetrahydro-chinolin-6-ylmethyl)-pyrrolidin-3-carbonsäure-3-chlor-5-fluor-benzylamid |
| "B70" | (S)-1-(5-Carbamoyl-2-fluor-phenyl)-3-hydroxy-2-oxo-pyrrolidin-3-carbonsäure-3-chlor-5-fluor-benzylamid |
| "B71" | (S)-3-Hydroxy-2-oxo-1-(2-oxo-2,3-dihydro-1H-benzimidazol-5-yl)-pyrrolidin-3-carbonsäure-3-chlor-5-fluor-benzylamid |
| "B72" | 3-Hydroxy-2-oxo-1-(2-trifluormethyl-1H-indol-5-yl)-pyrrolidin-3-carbonsäure-3-chlor-5-fluor-benzylamid |
| "B73" | (R)-3-Hydroxy-2-oxo-1-(3-trifluormethyl-1H-indol-5-yl)-pyrrolidin-3-carbonsäure-3-chlor-5-fluor-benzylamid |
| "B74" | (S)-3-Hydroxy-2-oxo-1-(3-trifluormethyl-1 H-indol-5-yl)-pyrrolidin-3-carbonsäure-3-chlor-5-fluor-benzylamid |
| "B75" | (S)-3-Hydroxy-2-oxo-1-(1H-pyrazolo[3,4-b]pyridin-5-yl)-pyrrolidin-3-carbonsäure-3-chlor-5-fluor-benzylamid |
| "B76" | 3-Hydroxy-2-oxo-1-(2-trifluormethyl-1H-pyrrolo[2,3-b]pyridin-5-yl)-pyrrolidin-3-carbonsäure-3-chlor-5-fluor-benzylamid |
| "B77" | (S)-3-Hydroxy-2-oxo-1-(2-oxo-2,3-dihydro-benzothiazol-6-yl)-pyrrolidin-3-carbonsäure-3-chlor-5-fluor-benzylamid |
| "B78" | 5-[(S)-3-(3-Chlor-5-fluor-benzylcarbamoyl)-3-hydroxy-2-oxo-pyrrolidin-1-yl]-1H-indol-2-carbonsäure-amid |
| "B79" | 5-[(R)-3-(3-Chlor-5-fluor-benzylcarbamoyl)-3-hydroxy-2-oxo-pyrrolidin-1-yl]-1H-indol-2-carbonsäure-amid |
| "B80" | 5-[(S)-3-(3-Chlor-5-fluor-benzylcarbamoyl)-3-hydroxy-2-oxo-pyrrolidin-1-yl]-1H-indol-2-carbonsäure-ethylester |
| "B81" | 5-[(R)-3-(3-Chlor-5-fluor-benzylcarbamoyl)-3-hydroxy-2-oxo-pyrrolidin-1-yl]-1H-indol-2-carbonsäure-ethylester |
| "B82" | 3-Hydroxy-1-[1-(2-methoxy-ethyl)-1H-pyrazol-4-yl]-2-oxo-pyrrolidin-3-carbonsäure-3-chlor-5-fluor-benzylamid |

(fortgesetzt)

| Verbindung Nr. | Name |
|---|---|
| "B83" | (S)-3-Hydroxy-2-oxo-1-(2-trifluormethyl-1H-pyrrolo[2,3-b]pyridin-5-yl)-pyrrolidin-3-carbonsäure-3-chlor-5-fluor-benzylamid |
| "B84" | 5-[(S)-3-(3-Chlor-5-fluor-benzylcarbamoyl)-3-hydroxy-2-oxo-pyrrolidin-1-yl]-1 H-indol-2-carbonsäure |
| "B85" | 5-[(R)-3-(3-Chlor-5-fluor-benzylcarbamoyl)-3-hydroxy-2-oxo-pyrrolidin-1-yl]-1H-indol-2-carbonsäure |
| "B86" | (S)-3-Hydroxy-2-oxo-1-(2-trifluormethyl-1H-indol-5-yl)-pyrrolidin-3-carbonsäure-3-chlor-5-fluor-benzylamid |
| "B87" | 1-(1-Cyanmethyl-1H-pyrazol-4-yl)-3-hydroxy-2-oxo-pyrrolidin-3-carbonsäure-3-chlor-5-fluor-benzylamid |
| "B88" | 5-[(R)-3-(3-Chlor-5-fluor-benzylcarbamoyl)-3-hydroxy-2-oxo-pyrrolidin-1-yl]-1H-indol-2-carbonsäure-ethylamid |
| "B89" | 5-[(R)-3-(3-Chlor-5-fluor-benzylcarbamoyl)-3-hydroxy-2-oxo-pyrrolidin-1-yl]-1H-indol-2-carbonsäure-isopropylamid |
| "B90" | 5-[(R)-3-(3-Chlor-5-fluor-benzylcarbamoyl)-3-hydroxy-2-oxo-pyrrolidin-1-yl]-1 H-indol-2-carbonsäure-dimethylamid |
| "B91" | Essigsäure-3-(3-chlor-5-fluor-benzylcarbamoyl)-2-oxo-1-(2-oxo-1,2,3,4-tetrahydro-chinolin-6-yl)-pyrrolidin-3-yl-ester |
| "B92" | (S)-3-Hydroxy-1-(3H-imidazo[4,5-b]pyridin-6-yl)-2-oxo-pyrrolidin-3-carbonsäure-3-chlor-5-fluor-benzylamid |
| "B93" | (R)-3-Hydroxy-1-(3H-imidazo[4,5-b]pyridin-6-yl)-2-oxo-pyrrolidin-3-carbonsäure-3-chlor-5-fluor-benzylamid |
| "B94" | 5-[(S)-3-(3-Chlor-5-fluor-benzylcarbamoyl)-3-hydroxy-2-oxo-pyrrolidin-1-yl]-1H-indol-2-carbonsäure-ethylamid |
| "B95" | 5-[(S)-3-(3-Chlor-5-fluor-benzylcarbamoyl)-3-hydroxy-2-oxo-pyrrolidin-1-yl]-1H-indol-2-carbonsäure-dimethylamid |
| "B96" | 5-[(S)-3-(3-Chlor-5-fluor-benzylcarbamoyl)-3-hydroxy-2-oxo-pyrrolidin-1-yl]-1H-indol-2-carbonsäure-methylamid |
| "B97" | 5-[(S)-3-(3-Chlor-5-fluor-benzylcarbamoyl)-3-hydroxy-2-oxo-pyrrolidin-1-yl]-1H-indol-2-carbonsäure-isopropylamid |
| "B98" | 3-Hydroxy-2-oxo-1-(2-oxo-1,2,3,4-tetrahydro-chinolin-6-yl)-pyrrolidin-3-carbonsäure-3-chlor-5-ffuor-benzylamid |
| "B99" | (S)-3-Hydroxy-1-[1-(2-methoxy-ethyl)-1H-pyrazol-4-yl]-2-oxo-pyrrolidin-3-carbonsäure-3-chlor-5-fluor-benzylamid |
| "B100" | (R)-3-Hydroxy-1-[1-(2-methoxy-ethyl)-1H-pyrazol-4-yl]-2-oxo-pyrrolidin-3-carbonsäure-3-chlor-5-fluor-benzylamid |
| "B101" | (S)-3-Hydroxy-2-oxo-1-(4-sulfamoyl-phenyl)-pyrrolidin-3-carbonsäure-3-chlor-5-fluor-benzylamid |
| "B102" | 5-[(R)-3-(3-Chlor-5-fluor-benzylcarbamoyl)-3-hydroxy-2-oxo-pyrrolidin-1-yl]-1H-indol-2-carbonsäure-methylamid |
| "B103" | (S)-3-Hydroxy-2-oxo-1-(2-oxo-1,2,3,4-tetrahydro-chinolin-6-yl)-pyrrolidin-3-carbonsäure-amid |
| "B104" | 3-Hydroxy-2-oxo-1-(3-trifluormethyl-1H-pyrrolo[2,3-b]pyridin-5-yl)-pyrrolidin-3-carbonsäure-3-chlor-5-fluor-benzylamid |

(fortgesetzt)

| Verbindung Nr. | Name |
|---|---|
| "B105" | 3-{2-tert-Butoxycarbonylamino-5-[3-(3-chlor-5-fluor-benzylcarbamoyl)-3-hydroxy-2-oxo-pyrrolidin-1-yl]-phenyl}-propionsäure-methylester |
| "B106" | 3-{2-tert-Butoxycarbonylamino-5-[3-(3-chlor-5-fluor-benzylcarbamoyl)-3-hydroxy-2-oxo-pyrrolidin-1-yl]-phenyl}-propionsäure |
| "B107" | 3-Hydroxy-2-oxo-1-(3-sulfamoyl-phenyl)-pyrrolidin-3-carbonsäure-3-chlor-5-fluor-benzylamid |
| "B108" | 5-[(S)-3-(3-Chlor-5-fluor-benzylcarbamoyl)-3-hydroxy-2-oxo-pyrrolidin-1-yl]-1H-indol-2-carbonsäure-{2-[2-(2-amino-ethoxy)-ethoxy]-ethyl}-amid |
| "B109" | 1-(7-Fluor-2-oxo-1,2,3,4-tetrahydro-chinolin-6-yl)-3-hydroxy-2-oxo-pyrrolidin-3-carbonsäure-3-chlor-5-fluor-benzylamid |
| "B110" | (S)-3-Hydroxy-1-(1H-indol-5-yl)-2-oxo-pyrrolidin-3-carbonsäure-[2-(3,5-difluor-phenyl)-ethyl]-amid |
| "B111" | 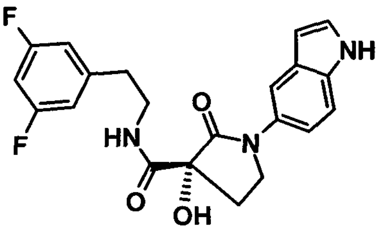 (R)-3-Hydroxy-1-(1H-indol-5-yl)-2-oxo-pyrrolidin-3-carbonsäure-[2-(3,5-difluor-phenyl)-ethyl]-amid |
| "B112" | (S)-3-Hydroxy-1-[2-(morpholin-4-carbonyl)-1H-indol-5-yl]-2-oxo-pyrrolidin-3-carbonsäure-3-chlor-5-fluor-benzylamid |
| "B113" | 5-[(S)-3-(3-Chlor-5-fluor-benzylcarbamoyl)-3-hydroxy-2-oxo-pyrrolidin-1-yl]-1H-indol-2-carbonsäure-[2-(2-amino-ethoxy)-ethyl]-amid |
| "B114" | (S)-1-[2-(Azetidin-1-carbonyl)-1H-indol-5-yl]-3-hydroxy-2-oxo-pyrrolidin-3-carbonsäure-3-chlor-5-fluor-benzylamid |
| "B115" | 3-Hydroxy-2-oxo-1-(4-sulfamoyl-phenyl)-pyrrolidin-3-carbonsäure-3-chlor-5-fluor-benzylamid |
| "B116" | (S)-1-(1-Cyanmethyl-1H-pyrazol-4-yl)-3-hydroxy-2-oxo-pyrrolidin-3-carbonsäure-3-chlor-5-fluor-benzylamid |
| "B117" | (R)-1-(1-Cyanmethyl-1H-pyrazol-4-yl)-3-hydroxy-2-oxo-pyrrolidin-3-carbonsäure-3-chlor-5-fluor-benzylamid |
| "B118" | 1-(4-Acetylsulfamoyl-phenyl)-3-hydroxy-2-oxo-pyrrolidin-3-carbonsäure-3-chlor-5-fluor-benzylamid |
| "B119" | (S)-1-(1-Carbamoylmethyl-1H-pyrazol-4-yl)-3-hydroxy-2-oxo-pyrrolidin-3-carbonsäure-3-chlor-5-fluor-benzylamid |
| "B120" | 3-Hydroxy-2-oxo-1-(1-phenyl-ethyl)-pyrrolidin-3-carbonsäure-3-chlor-5-fluor-benzylamid |
| "B121" | 5-[(S)-3-(3-Chlor-5-fluor-benzylcarbamoyl)-3-hydroxy-2-oxo-pyrrolidin-1-yl]-1H-indol-2-carbonsäure-(2-hydroxy-ethyl)-amid |
| "B122" | (S)-3-Hydroxy-1-[2-(4-methyl-piperazin-1-carbonyl)-1H-indol-5-yl]-2-oxo-pyrrolidin-3-carbonsäure-3-chlor-5-fluor-benzylamid |
| "B123" | 4-{5-[(S)-3-(3-Chlor-5-fluor-benzylcarbamoyl)-3-hydroxy-2-oxo-pyrrolidin-1-yl]-1H-indol-2-carbonyl}-piperazin-1-carbonsäure-tert-butylester |
| "B124" | 1-(5-Fluor-2-oxo-1,2,3,4-tetrahydro-chinolin-6-yl)-3-hydroxy-2-oxo-pyrrolidin-3-carbonsäure-3-chlor-5-fluor-benzylamid |

(fortgesetzt)

| Verbindung Nr. | Name |
|---|---|
| "B125" | 3-Hydroxy-2-oxo-1-(2-oxo-2,3-dihydro-1H-indol-5-yl)-pyrrolidin-3-carbonsäure-3-chlor-5-fluor-benzylamid |
| "B126" | (S)-3-Hydroxy-2-oxo-1-[2-(piperazin-1-carbonyl)-1H-indol-5-yl]-pyrrolidin-3-carbonsäure-3-chlor-5-fluor-benzylamid |
| "B127" | 3-Chlor-2-oxo-1-(2-oxo-1,2,3,4-tetrahydro-chinolin-6-yl)-pyrrolidin-3-carbonsäure-3-chlor-5-fluor-benzylamid |
| "B128" | (S)-1-(3-Dimethylsulfamoyl-phenyl)-3-hydroxy-2-oxo-pyrrolidin-3-carbonsäure-3-chlor-5-fluor-benzylamid |
| "B129" | 5-[(S)-3-(3-Chlor-5-fluor-benzylcarbamoyl)-3-hydroxy-2-oxo-pyrrolidin-1-yl]-1H-indol-2-carbonsäure-(2-amino-phenyl)-amid |
| "B130" | (S)-3-Hydroxy-2-oxo-1-(2-oxo-2,3-dihydro-1H-indol-5-yl)-pyrrolidin-3-carbonsäure-3-chlor-5-fluor-benzylamid |
| "B131" | (R)-3-Hydroxy-2-oxo-1-(2-oxo-2,3-dihydro-1H-indol-5-yl)-pyrrolidin-3-carbonsäure-3-chlor-5-fluor-benzylamid |
| "B132" | 5-[(S)-3-(3-Chlor-5-fluor-benzylcarbamoyl)-3-hydroxy-2-oxo-pyrrolidin-1-yl]-1H-indol-2-carbonsäure-(2-amino-ethyl)-amid |
| "B133" | (S)-1-[2-(1H-Benzoimidazol-2-yl)-1H-indol-5-yl]-3-hydroxy-2-oxo-pyrrolidin-3-carbonsäure-3-chlor-5-fluor-benzylamid |
| "B134" | (S)-1-(2-Hydrazinocarbonyl-1H-indol-5-yl)-3-hydroxy-2-oxo-pyrrolidin-3-carbonsäure-3-chlor-5-fluor-benzylamid |
| "B135" | (S)-3-Hydroxy-1-[2-(3-methyl-[1,2,4]oxadiazol-5-yl)-1H-indol-5-yl]-2-oxo-pyrrolidin-3-carbonsäure-3-chlor-5-fluor-benzylamid |
| "B136" | (S)-1-Benzyloxy-3-hydroxy-2-oxo-pyrrolidin-3-carbonsäure-3-chlor-5-fluor-benzylamid |
| "B137" | 3-Hydroxy-2-oxo-pyrrolidin-3-carbonsäure-3-chlor-5-fluor-benzylamid |
| "B138" | (R)-1-Benzyloxy-3-hydroxy-2-oxo-pyrrolidin-3-carbonsäure-3-chlor-5-fluor-benzylamid |
| "B139" | (S)-3-Hydroxy-2-oxo-1-(3-trifluormethyl-1H-pyrrolo[2,3-b]pyridin-5-yl)-pyrrolidin-3-carbonsäure-3-chlor-5-fluor-benzylamid |
| "B140" | (R)-3-Hydroxy-2-oxo-1-(3-trifluormethyl-1H-pyrrolo[2,3-b]pyridin-5-yl)-pyrrolidin-3-carbonsäure-3-chlor-5-fluor-benzylamid |
| "B141" | (S)-3-Hydroxy-1-(4-methylsulfamoyl-phenyl)-2-oxo-pyrrolidin-3-carbonsäure-3-chlor-5-fluor-benzylamid |
| "B142" | (S)-1-(4-Benzylsulfamoyl-phenyl)-3-hydroxy-2-oxo-pyrrolidin-3-carbonsäure-3-chlor-5-fluor-benzylamid |
| "B143" | (S)-1-[4-(Benzyl-methyl-sulfamoyl)-phenyl]-3-hydroxy-2-oxo-pyrrolidin-3-carbonsäure-3-chlor-5-fluor-benzylamid |
| "B144" | 3-Hydroxy-1-(1-methyl-1H-indol-5-yl)-2-oxo-pyrrolidin-3-carbonsäure-3-chlor-5-fluor-benzylamid |
| "B145" | (S)-3-Hydroxy-1-hydroxymethyl-2-oxo-pyrrolidin-3-carbonsäure-3-chlor-5-fluor-benzylamid |
| "B146" | (R)-3-Hydroxy-1-hydroxymethyl-2-oxo-pyrrolidin-3-carbonsäure-3-chlor-5-fluor-benzylamid |
| "B147" | (S)-1-(3-Chlor-1H-pyrrolo[2,3-b]pyridin-5-yl)-3-hydroxy-2-oxo-pyrrolidin-3-carbonsäure-3-chlor-5-fluor-benzylamid |
| "B148" | 3-Hydroxy-2-oxo-1-(2-trifluormethyl-1H-benzimidazol-5-yl)-pyrrolidin-3-carbonsäure-3-chlor-5-fluor-benzylamid |

(fortgesetzt)

| Verbindung Nr. | Name |
|---|---|
| "B149" | (S)-3-Hydroxy-2-oxo-1-(2-sulfamoyl-1H-indol-5-yl)-pyrrolidin-3-carbonsäure-3-chlor-5-fluor-benzylamid |
| "B150" | (3S)-N-[(3-chlor-5-fluor-phenyl)methyl]-1-(2,2-dioxo-1,3-dihydro-2,1-benzothiazol-5-yl)-3-hydroxy-2-oxo-pyrrolidin-3-carboxamid |
| "B151" | (S)-1-(2,2-Dioxo-1,2,3,4-tetrahydro-2lamda*6*-benzo[c]thiazin-6-yl)-3-hydroxy-2-oxo-1-(2-sulfamoyl-1H-indol-5-yl)-pyrrolidin-3-carbonsäure-3-chlor-5-fluor-benzylamid |
| "B152" | (S)-1-[((R)-1-Dimethylcarbamoyl-2-methyl-propylcarbamoyl)-methyl]-3-hydroxy-2-oxo-1-(2-sulfamoyl-1H-indol-5-yl)-pyrrolidin-3-carbonsäure-3-chlor-5-fluor-benzylamid |
| "B153" | (S)-1-[((S)-3-Amino-1-methylcarbamoyl-propylcarbamoyl)-methyl]-3-hydroxy-2-oxo-1-(2-sulfamoyl-1H-indol-5-yl)-pyrrolidin-3-carbonsäure-3-chlor-5-fluor-benzylamid |
| "B154" | N-(3-Hydroxy-2-oxo-1-phenyl-pyrrolidin-3-yl)-benzamid |

sowie ihre pharmazeutisch verwendbaren Salze, Tautomere und Stereoisomere, einschließlich deren Mischungen in allen Verhältnissen.

**6.** Verfahren zur Herstellung von Verbindungen der Formel I nach den Ansprüchen 1-5 sowie ihrer pharmazeutisch verwendbaren Salze, Tautomeren und Stereoisomeren, **dadurch gekennzeichnet, daß** man

a) zur Herstellung von Verbindungen der Formel I, worin Y CO und R $NR^2R^4$ bedeuten, eine Verbindung der Formel II

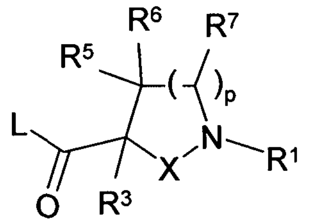

II

worin X, $R^1$, $R^3$, $R^5$, $R^6$, $R^7$ und p die in Anspruch 1 angegebenen Bedeutungen haben, und L Cl, Br, I oder eine freie oder reaktionsfähig funktionell abgewandelte OH-Gruppe bedeutet, mit einer Verbindung der Formel III

$R^2$-$NHR^4$ III

worin $R^2$ und $R^4$ die in Anspruch 1 angegebenen Bedeutungen haben, umsetzt,
oder
b) eine Verbindung der Formel IV

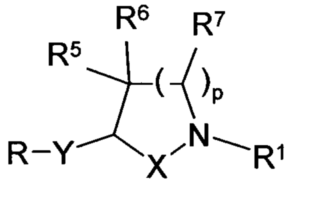

IV

worin $R^1$, $R^5$, $R^6$, $R^7$, R, X, Y und p die in Anspruch 1 angegebenen Bedeutungen haben, oxidiert,
oder
c) zur Herstellung von Verbindungen der Formel I, worin X und Y $CH_2$ bedeuten, eine Verbindung der Formel I, worin X und Y CO bedeuten, reduziert,

und/oder
eine Base oder Säure der Formel I in eines ihrer Salze umwandelt.

7. Arzneimittel, enthaltend mindestens eine Verbindung der Formel I nach Anspruch 1-5 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

8. Verbindungen der Formel I gemäß Anspruch 1-5,
sowie ihrer pharmazeutisch verwendbaren Salze, Tautomeren und Stereoisomeren, einschließlich deren Mischungen in allen Verhältnissen, zur Verwendung zur Behandlung von Tumoren, Tumormetastasen, proliferativer Erkrankungen der Mesangiumzellen, Hämangiom, proliferativer Retinopathie, rheumatoider Arthritis, atherosklerotischer Neovaskularisation, Psoriasis, okularer Neovaskularisation, Osteoporose, Diabetes und Fettleibigkeit, lymphoider Leukämie, Lymphom, Malaria und Prostatahypertrophie.

9. Verbindungen zur Verwendung zur Behandlung nach Anspruch 8, wobei die Tumorerkrankung ausgewählt ist aus der Gruppe des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom,

10. Verbindungen der Formel I gemäß Anspruch 1-5 und/oder ihrer physiologisch unbedenklichen Salze zur Verwendung zur Behandlung von Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

11. Verbindungen der Formel I gemäß Anspruch 1-5 und/oder ihrer physiologisch unbedenklichen Salze zur Verwendung zur Behandlung von Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

1. Compounds of the formula I

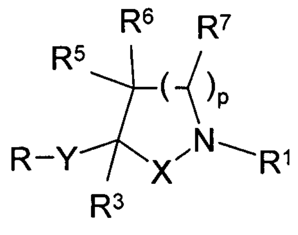

I

in which

R denotes $NR^2R^4$, Alk, $C(=CH_2)[C(R^4)_2]_nAr^2$, $Het^2$, $O[C(R^4)_2]_nAr^2$ or OA,
X denotes CO or $CH_2$,
Y denotes CO or $CH_2$,
$R^1$ denotes H, $[C(R^4)_2]_nAr^1$, $(CH_2)_nHet$, $(CH_2)_nCyc$, $[C(R^4)_2]_nCOOH$, $[C(R^4)_2]_nCONHAr^1$, $[C(R^4)_2]_nCONH_2$, $[C(R^4)_2]_nNHA$, $[C(R^4)_2]_nNA_2$, $O[C(R^4)_2]_nAr^1$, $[C(R^4)_2]_nOR^7$, $[C(R^4)_2]_nCOO(CH_2)_nAr^1$, $[C(R^4)_2]_nCOOA$, $[C(R^4)_2]_nCONH[C(R^4)_2]_pCON(R^4)_2$ or $[C(R^4)_n]_nCONHCR^4\text{-}[(CH_2)_nN(R^4)_2]CON(R^4)_2$,

$R^2$ denotes H, $[C(R^4)_2]_nAr^2$, $(CH_2)_nCOHet^1$, $(CH_2)_nCOAr^2$, $(CH_2)_mNA_2$ or $(CH_2)_nHet$,
$R^3$ denotes OH or OCOA,
$R^4$ denotes H or alkyl having 1, 2, 3 or 4 C atoms,
$R^2$ and $R^4$ together also denote alkylene having 2, 3, 4 or 5 C atoms, where a $CH_2$ group may also be replaced by $N(CH_2)_mOH$ or $SO_2$
$R^5$, $R^6$ each, independently of one another, denote H, F or A,
$R^5$ and $R^6$ together also denote alkylene having 2, 3, 4 or 5 C atoms, where a $CH_2$ group may also be replaced by NCOA or O,
$R^7$ denotes H or A,
$Ar^1$ denotes phenyl which is unsubstituted or mono-, di-, tri-, tetra- or pentasubstituted by Hal, OH, OA, $CONH_2$, CONHA, $CONA_2$, $NHSO_2A$, CONHCyc, $NHSO_2Cyc$, $CONHAr^2$, $COHet^1$ and/or $NASO_2A$,
$Ar^2$ denotes phenyl which is unsubstituted or mono-, di-, tri-, tetra- or pentasubstituted by Hal, A, $CONH_2$ and/or $OAr^3$,
$Ar^3$ denotes phenyl which is unsubstituted or monosubstituted by $NH_2$,
Het denotes a mono- or bicyclic saturated, unsaturated or aromatic heterocycle having 1 to 4 N and/or O and/or S atoms which is unsubstituted or mono-, di- or trisubstituted by Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O and/or $Ar^3$,
$Het^1$ denotes a monocyclic saturated heterocycle having 1 to 4 N and/or O and/or S atoms which is unsubstituted or mono-, di- or trisubstituted by =O and/or COOA,
$Het^2$ denotes isoindolyl,
A denotes unbranched or branched alkyl having 1-10 C atoms, in which 1-7 H atoms may be replaced by F, Cl, Br, OH, CHO, COA, COOA, CN, $CONA_2$, CONHA and/or $CONH_2$,
and/or in which one or two non-adjacent CH and/or $CH_2$ groups may be replaced by O, or Cyc,
Alk denotes alkenyl having 2, 3, 4, 5 or 6 C atoms,
Cyc denotes cyclic alkyl having 3-7 C atoms which is unsubstituted or mono-, di- or trisubstituted by NHCOA, $NHSO_2$ OH, OA, A, $NH_2$, NHA, $NA_2$, COOA, COOH and/or CONHA,
Hal denotes F, Cl, Br or I,
m denotes 1, 2, 3 or 4,
n denotes 0, 1, 2, 3 or 4,
p denotes 1, 2 or 3,

and pharmaceutically usable salts, tautomers and stereoisomers thereof, including mixtures thereof in all ratios, where the following compounds are excluded:

methyl 3-hydroxypiperidine-3-carboxylate,
1-tert-butyl 3-methyl 3-hydroxypiperidine-1,3-dicarboxylate,
tert-butyl 3-allyl-3-hydroxypyrrolidine-1-carboxylate,
tert-butyl 3-allyl-3-hydroxypiperidine-1-carboxylate.

**2.** Compounds according to Claim 1 in which

Het denotes pyrazinyl, pyrazolyl, benzimidazolyl, pyridyl, indolyl, dihydroindolyl, benzofuranyl, tetrahydropyranyl, dihydroquinolinyl, dihydroisoquinolinyl, tetrahydroquinolinyl, tetrahydroisoquinolinyl, indazolyl, imidazolyl, pyrrolyl, oxazolyl, oxadiazolyl, isoxazolyl, benzothiazolyl, piperidin-1-yl, pyrrolidin-1-yl, 3,4-dihydro-2H-pyrido[3,2-b]-1,4-oxazinyl, 3,4-dihydro-2H-benzo-1,4-oxazinyl, benzofuranyl, azetidinyl, 3-azabicyclo[3.2.0]hexyl, pyrrolo[2,3-b]pyridinyl, tetrahydrofuranyl, tetrahydro-1,8-naphthyridinyl, 2,3-dihydrobenzoisothiazolyl, 1,2,3,4-tetrahydrobenzothiazinyl or hexahydrobenzo-1,3-dioxolyl, each of which is unsubstituted or mono-, di- or trisubstituted by Hal, A, OA, CN, $NH_2$, NHA, $NA_2$, $NO_2$, CN, COOH, COOA, $(CH_2)_nCONH_2$, $(CH_2)_n$-CONHA, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O and/or $Ar^3$,

and pharmaceutically usable salts, tautomers and stereoisomers thereof, including mixtures thereof in all ratios.

**3.** Compounds according to Claim 1 or 2 in which

$Het^1$ denotes pyridazinyl, pyrazolyl, pyridyl, piperazinyl, morpholinyl, pyrimidinyl, furyl, thienyl, imidazolyl, pyrrolyl, oxazolyl, oxadiazolyl, isoxazolyl, thiazolyl, triazolyl, tetrazolyl, thiadiazole, piperidin-1-yl, pyrrolidin-1-yl,

tetrahydropyranyl, 1,2-oxazinan-2-yl, 1,2,5-oxadiazinan-2-yl, 1,3-oxazinan-3-yl or hexahydropyrimidinyl, each of which is unsubstituted or mono-, di- or trisubstituted by A and/or OA,

and pharmaceutically usable salts, tautomers and stereoisomers thereof, including mixtures thereof in all ratios.

**4.** Compounds according to Claim 1 in which

R denotes $NR^2R^4$, Alk, $C(=CH_2)[C(R^4)_2]_nAr^2$, $Het^2$, $O[C(R^4)_2]_n$-$Ar^2$ or OA,
X denotes CO or $CH_2$,
Y denotes CO or $CH_2$,
$R^1$ denotes H, $[C(R^4)_2]_nAr^1$, $(CH_2)_nHet$, $(CH_2)_nCyc$, $[C(R^4)_2]_n$-COOH, $[C(R^4)_2]_nCONHAr^1$, $[C(R^4)_2]_nCONH_2$, $[C(R^4)_2]_nNHA$, $[C(R^4)_2]_nNA_2$, $O[C(R^4)_2]_nAr^1$, $[C(R^4)_2]_nOR^7$, $[C(R^4)_2]_nCOO$-$(CH_2)_nAr^1$ $[C(R^4)_2]_nCOOA$, $[C(R^4)_2]_nCONH[C(R^4)_2]_p$-$CON(R^4)_2$ or $[C(R^4)_2]_nCONHCR^4[(CH_2)_nN(R^4)_2]CON(R^4)_2$,
$R^2$ denotes H, $[C(R^4)_2]_nAr^2$, $(CH_2)_nCOHet^1$, $(CH_2)_nCOAr^2$, $(CH_2)_mNA_2$ or $(CH_2)_nHet$,
$R^3$ denotes OH or OCOA,
$R^4$ denotes H or alkyl having 1, 2, 3 or 4 C atoms,
$R^2$ and $R^4$ together also denote alkylene having 2, 3, 4 or 5 C atoms, where a $CH_2$ group may also be replaced by $N(CH_2)_mOH$ or $SO_2$,
$R^5$, $R^6$ each, independently of one another, denote H, F or A,
$R^5$ and $R^6$ together also denote alkylene having 2, 3, 4 or 5 C atoms, where a $CH_2$ group may also be replaced by NCOA or O,
$R^7$ denotes H or A,
$Ar^1$ denotes phenyl which is unsubstituted or mono-, di-, tri-, tetra- or pentasubstituted by Hal, OH, OA, $CONH_2$, CONHA, $CONA_2$, $NHSO_2A$, CONHCyc, $NHSO_2Cyc$, $CONHAr^2$, $COHet^1$ and/or $NASO_2A$,
$Ar^2$ denotes phenyl which is unsubstituted or mono-, di-, tri-, tetra- or pentasubstituted by Hal, A, $CONH_2$ and/or $OAr^3$,
$Ar^3$ denotes phenyl which is unsubstituted or monosubstituted by $NH_2$,
Het denotes pyrazinyl, pyrazolyl, benzimidazolyl, pyridyl, indolyl, dihydroindolyl, benzofuranyl, tetrahydropyranyl, dihydroquinolinyl, dihydroisoquinolinyl, tetrahydroquinolinyl, tetrahydroisoquinolinyl, indazolyl, imidazolyl, pyrrolyl, oxazolyl, oxadiazolyl, isoxazolyl, benzothiazolyl, piperidin-1-yl, pyrrolidin-1-yl, 3,4-dihydro-2H-pyrido[3,2-b]-1,4-oxazinyl, 3,4-dihydro-2H-benzo-1,4-oxazinyl, benzofuranyl, azetidinyl, 3-azabicyclo[3.2.0]hexyl, pyrrolo[2,3-b]pyridinyl, tetrahydrofuranyl, tetrahydro-1,8-naphthyridinyl, 2,3-dihydro-benzoisothiazolyl, 1,2,3,4-tetrahydrobenzothiazinyl or hexahydrobenzo-1,3-dioxolyl, each of which is unsubstituted or mono-, di- or trisubstituted by Hal, A, OA, CN, $NH_2$, NHA, $NA_2$, $NO_2$, CN, COOH, COOA, $(CH_2)_nCONH_2$, $(CH_2)_n$-CONHA, $(CH_2)_nCONA_2$, NHCOA, COA, CHO, $Het^1$, $SO_2A$, $SO_2NH_2$, $SO_2NHA$, $SO_2NA_2$, $CONHNH_2$, $CONHAr^3$, =O and/or $Ar^3$,
$Het^1$ denotes pyridazinyl, pyrazolyl, pyridyl, piperazinyl, morpholinyl, pyrimidinyl, furyl, thienyl, imidazolyl, pyrrolyl, oxazolyl, oxadiazolyl, isoxazolyl, thiazolyl, triazolyl, tetrazolyl, thiadiazole, piperidin-1-yl, pyrrolidin-1-yl, tetrahydropyranyl, 1,2-oxazinan-2-yl, 1,2,5-oxadiazinan-2-yl, 1,3-oxazinan-3-yl or hexahydropyrimidinyl, each of which is unsubstituted or mono-, di- or trisubstituted by A and/or OA,
$Het^2$ denotes isoindolyl,
A denotes unbranched or branched alkyl having 1-10 C atoms, in which 1-7 H atoms may be replaced by F, Cl, Br, OH, CHO, COA, COOA, CN, $CONA_2$, CONHA and/or $CONH_2$,
and/or in which one or two non-adjacent CH and/or $CH_2$ groups may be replaced by O, or Cyc,
Alk denotes alkenyl having 2, 3, 4, 5 or 6 C atoms,
Cyc denotes cyclic alkyl having 3-7 C atoms which is unsubstituted or mono-, di- or trisubstituted by NHCOA, $NHSO_2$, OH, OA, A, $NH_2$, NHA, $NA_2$, COOA, COOH and/or CONHA,
Hal denotes F, Cl, Br or I,
m denotes 1, 2, 3 or 4,
n denotes 0, 1, 2, 3 or 4,
p denotes 1, 2 or 3,

and pharmaceutically usable salts, tautomers and stereoisomers thereof, including mixtures thereof in all ratios.

**5.** Compounds according to Claim 1, selected from the group

| Compound No. | Name |
|---|---|
| "A56" | (S)-3-(1,1-Dioxo-1,6-thiomorpholine-4-carbonyl)-3-hydroxy-1-phenylpyrrolidin-2-one |
| "A57" | (S)-3-Hydroxy-3-[4-(2-hydroxyethyl)piperazine-1-carbonyl)-1-phenylpyrrolidin-2-one |
| "A58" | N-(3-Imidazol-1-ylpropyl)-(S)-3-hydroxy-2-oxo-1-phenylpyrrolidine-3-carboxamide |
| "A59" | N-(2-Dimethylaminoethyl)-(S)-3-hydroxy-2-oxo-1-phenylpyrrolidine-3-carboxamide |
| "A60" | N-[2-(4-Phenoxyphenyl)ethyl]-(S)-3-hydroxy-2-oxo-1-phenylpyrrolidine-3-carboxamide |
| "A61" | N-[(2-Dimethylaminoethyl)methyl]-(S)-3-hydroxy-2-oxo-1-phenylpyrrolidine-3-carboxamide |
| "A62" | N-(2-Morpholin-4-yl-2-oxoethyl)-(S)-3-hydroxy-2-oxo-1-phenylpyrrolidine-3-carboxamide |
| "A63" | N-[2-(1-Methylpyrrolidin-2-yl)ethyl]-(S)-3-hydroxy-2-oxo-1-phenylpyrrolidine-3-carboxamide |
| "A64" | N-(3-Dimethylaminopropyl)-(S)-3-hydroxy-2-oxo-1-phenylpyrrolidine-3-carboxamide |
| "A65" | N-(3-Chloro-5-fluorobenzyl)-3-hydroxy-2-oxo-1-phenylpiperidine-3-carboxamide |
| "A66" | N-(3-Chloro-5-fluorobenzyl)-(S)-3-hydroxy-2-oxo-1-phenylazepane-3-carboxamide |
| "A67" | N-(3-Chloro-5-fluorobenzyl)-(R)-3-hydroxy-2-oxo-1-phenylazepane-3-carboxamide |
| "A68" | N-(3-Chloro-5-fluorobenzyl)-3-hydroxy-1-(1H-indol-6-yl)-2-oxopyrrolidine-3-carboxamide |
| "A69" | N-(3-Chloro-5-fluorobenzyl)-1-[3-(3-carbamoylphenylcarbamoyl)phenyl]-3-hydroxy-2-oxo-pyrrolidine-3-carboxamide |
| "A70" | (S)-3-((E)-But-2-enoyl)-3-hydroxy-1-phenylpyrrolidin-2-one |
| "A71" | N-(3-Chloro-5-fluorobenzyl)-(S)-8-acetyl-4-hydroxy-3-oxo-2-phenyl-2,8-diazaspiro[4.5]decane-4-carboxamide |
| "A72" | N-(3-Chloro-5-fluorobenzyl)-(S)-3-hydroxy-1-[3-(morpholine-4-carbonyl)phenyl]-2-oxopyrrolidine-3-carboxamide |
| "A73" | N-(3-Chloro-5-fluorobenzyl)-3-hydroxy-1-[3-(methane-sulfonylmethylamino)phenyl]-2-oxopyrrolidine-3-carboxamide |
| "A74" | N-(3-Chloro-5-fluorobenzyl)-(R)-3-hydroxy-1-[3-(morpholine-4-carbonyl)phenyl]-2-oxopyrrolidine-3-carboxamide |
| "A75" | N-(3-Chloro-5-fluorobenzyl)-(S)-1-(3-cyclopropyl-carbamoylphenyl)-3-hydroxy-2-oxopyrrolidine-3-carboxamide |
| "A76" | N-(3-Chloro-5-fluorobenzyl)-1-(3-cyclobutylcarbamoylphenyl)-3-hydroxy-2-oxopyrrolidine-3-carboxamide |
| "A77" | N-(3-Chloro-5-fluorobenzyl)-(S)-3-hydroxy-2-oxo-1-[3-(pyrrolidine-1-carbonyl)phenyl]pyrrolidine-3-carboxamide |
| "A78" | N-(3-Chloro-5-fluorobenzyl)-3-hydroxy-2-oxo-1-(6-propionylaminopyridin-3-yl)pyrrolidine-3-carboxamide |
| "A79" | N-(3-Chloro-5-fluorobenzyl)-(S)-1-(3-ethanesulfonylaminophenyl)-3-hydroxy-2-oxopyrrolidine-3-carboxamide |
| "A80" | N-(3-Chloro-5-fluorobenzyl)-(S)-1-(3-cyclopropane-sulfonylaminophenyl)-3-hydroxy-2-oxopyrrolidine-3-carboxamide |
| "A81" | N-(3-Chloro-5-fluorobenzyl)-(S)-1-benzyl-3-hydroxy-2-oxopiperidine-3-carboxamide |
| "A82" | N-(3-Chloro-5-fluorobenzyl)-(R)-1-(3-cyclopropylcarbamoylphenyl)-3-hydroxy-2-oxopyrrolidine-3-carboxamide |
| "A83" | N-(3-Chloro-5-fluorobenzyl)-(R)-3-hydroxy-2-oxo-1-[3-(pyrrolidine-1-carbonyl)phenyl]pyrrolidine-3-carboxamide |
| "A84" | N-(3-Chloro-5-fluorobenzyl)-(R)-1-(3-ethanesulfonylaminophenyl)-3-hydroxy-2-oxopyrrolidine-3-carboxamide |

(continued)

| Compound No. | Name |
|---|---|
| "A85" | N-(3-Chloro-5-fluorobenzyl)-(R)-1-(3-cyclopropanesulfonylaminophenyl)-3-hydroxy-2-oxopyrrolidine-3-carboxamide |
| "A86" | N-(3-Chloro-5-fluorobenzyl)-(R)-1-benzyl-3-hydroxy-2-oxopiperidine-3-carboxamide |
| "A87" | N-(3-Chloro-5-fluorobenzyl)-1-(3-cyano-1H-indol-5-yl)-3-hydroxy-2-oxopyrrolidine-3-carboxamide |
| "A88" | N-(3-Chloro-5-fluorobenzyl)-(S)-3-hydroxy-1-[3-(2-hydroxyethylcarbamoyl)phenyl]-2-oxopyrrolidine-3-carboxamide |
| "A89" | 3-(2-Benzylacryloyl)-3-hydroxy-1-phenylpyrrolidin-2-one |
| "A90" | N-(3-Chloro-5-fluorobenzyl)-(S)-3-hydroxy-2-oxo-1-phenethylpyrrolidine-3-carboxamide |
| "A91" | N-(3-Chloro-5-fluorobenzyl)-(R)-3-hydroxy-1-[3-(2-hydroxyethylcarbamoyl)phenyl]-2-oxopyrrolidine-3-carboxamide |
| "A92" | N-(3-Chloro-5-fluorobenzyl)-(R)-3-hydroxy-2-oxo-1-phenethylpyrrolidine-3-carboxamide |
| "A93" | N-(3-Chloro-5-fluorobenzyl)-(S)-3-hydroxy-1-{3-[(2-hydroxyethyl)methylcarbamoyl]phenyl}-2-oxopyrrolidine-3-carboxamide |
| "A94" | N-(3-Chloro-5-fluorobenzyl)-(S)-3-hydroxy-1-[3-(3-hydroxypropylcarbamoyl)phenyl]-2-oxopyrrolidine-3-carboxamide |
| "A95" | N-(3-Chloro-5-fluorobenzyl)-(S)-3-hydroxy-2-oxo-1-[3-(piperidine-1-carbonyl)phenyl]pyrrolidine-3-carboxamide |
| "A96" | N-(3-Chloro-5-fluorobenzyl)-(S)-3-hydroxy-2-oxo-1-(1-oxo-1,2,3,4-tetrahydroisoquinolin-6-yl)pyrrolidine-3-carboxamide |
| "A97" | N-(3-Chloro-5-fluorobenzyl)-(S)-1-(6-cyanopyridin-3-yl)-3-hydroxy-2-oxopyrrolidine-3-carboxamide |
| "A98" | N-(3-Chloro-5-fluorobenzyl)-(R)-1-(5-cyanopyridin-3-yl)-3-hydroxy-2-oxopyrrolidine-3-carboxamide |
| "A99" | N-(3-Chloro-5-fluorobenzyl)-(S)-3-hydroxy-2-oxo-1-(6-trifluoromethylpyridin-3-yl)pyrrolidine-3-carboxamide |
| "A100" | Methyl 5-[3-(3-chloro-5-fluorobenzylcarbamoyl)-3-hydroxy-2-oxopyrrolidin-1-yl]-2-methylnicotinate |
| "A101" | N-(3-Chloro-5-fluorobenzyl)-(S)-3-hydroxy-4,4-dimethyl-2-oxo-1-phenylpyrrolidine-3-carboxamide |
| "A102" | N-(3-Chloro-5-fluorobenzyl)-(R)-3-hydroxy-1-{3-[(2-hydroxyethyl)methylcarbamoyl]phenyl}-2-oxopyrrolidine-3-carboxamide |
| "A103" | N-(3-Chloro-5-fluorobenzyl)-(R)-3-hydroxy-1-[3-(3-hydroxypropylcarbamoyl)phenyl]-2-oxopyrrolidine-3-carboxamide |
| "A104" | N-(3-Chloro-5-fluorobenzyl)-(R)-3-hydroxy-2-oxo-1-[3-(piperidine-1-carbonyl)phenyl]pyrrolidine-3-carboxamide |
| "A105" | N-(3-Chloro-5-fluorobenzyl)-(R)-3-hydroxy-2-oxo-1-(1-oxo-1,2,3,4-tetrahydroisoquinolin-6-yl)pyrrolidine-3-carboxamide |
| "A106" | N-(3-Chloro-5-fluorobenzyl)-(R)-1-(6-cyanopyridin-3-yl)-3-hydroxy-2-oxopyrrolidine-3-carboxamide |
| "A107" | N-(3-Chloro-5-fluorobenzyl)-(S)-1-(5-cyanopyridin-3-yl)-3-hydroxy-2-oxopyrrolidine-3-carboxamide |
| "A108" | N-(3-Chloro-5-fluorobenzyl)-(R)-3-hydroxy-2-oxo-1-(6-trifluoromethylpyridin-3-yl)pyrrolidine-3-carboxamide |

(continued)

| Compound No. | Name |
|---|---|
| "A109" | N-(3-Chloro-5-fluorobenzyl)-(R)-3-hydroxy-4,4-dimethyl-2-oxo-1-phenylpyrrolidine-3-carboxamide |
| "A110" | (S)-3-(2-Benzylacryloyl)-3-hydroxy-1-phenylpyrrolidin-2-one |
| "A111" | (R)-3-(2-Benzylacryloyl)-3-hydroxy-1-phenylpyrrolidin-2-one |
| "A112" | N-(3-Chloro-5-fluorobenzyl)-3-hydroxy-1-(1-hydroxymethyl-3-trifluoromethyl-1H-indol-5-yl)-2-oxopyrrolidine-3-carboxamide |
| "A113" | N-(3-Chloro-5-fluorobenzyl)-8-hydroxy-7-oxo-6-phenyl-2-oxa-6-azaspiro[3.4]octane-8-carboxamide |
| "A114" | N-(3-Chloro-5-fluorobenzyl)-1-(8-fluoro-2-oxo-1,2,3,4-tetrahydroquinolin-6-yl)-3-hydroxy-2-oxopyrrolidine-3-carboxamide |
| "A115" | N-(3-Chloro-5-fluorobenzyl)-3-hydroxy-2-oxo-1-(1-oxo-1,2,3,4-tetrahydroisoquinolin-7-yl)pyrrolidine-3-carboxamide |
| "A116" | N-(3-Chloro-5-fluorobenzyl)-3-hydroxy-1-(6-methylamino-pyridin-3-yl)-2-oxopyrrolidine-3-carboxamide |
| "A117" | N-(3-Chloro-5-fluorobenzyl)-3-hydroxy-1-(5-methylpyridin-3-yl)-2-oxopyrrolidine-3-carboxamide |
| "A118" | N-(3-Chloro-5-fluorobenzyl)-1-(3,4-dihydro-2H-pyrido-[3,2-b]-1,4-oxazin-7-yl)-3-hydroxy-2-oxopyrrolidine-3-carboxamide |
| "A119" | N-(3-Chloro-5-fluorobenzyl)-3-hydroxy-5-methyl-2-oxo-1-phenylpyrrolidine-3-carboxamide |
| "A120" | 3-[(3-Chloro-5-fluorobenzylamino)methyl]-1-phenylpyrrolidin-3-ol |
| "A121" | N-(3-Chloro-5-fluorobenzyl)-1-benzyl-3-hydroxypyrrolidine-3-carboxamide |
| "A122" | N-(3-Chloro-5-fluorobenzyl)-1-(4-acetylaminocyclohexyl)-3-hydroxy-2-oxopyrrolidine-3-carboxamide |
| "A123" | N-(3-Chloro-5-fluorobenzyl)-3-hydroxy-1-(4-methane-sulfonylaminocyclohexyl)-2-oxopyrrolidine-3-carboxamide |
| "A124" | N-(3-Chloro-5-fluorobenzyl)-1-(4-ethanesulfonylaminocyclohexyl)-3-hydroxy-2-oxopyrrolidine-3-carboxamide |
| "A125" | N-(3-Chloro-5-fluorobenzyl)-1-((1S,2R,3S)-2,3-dihydroxycyclohexyl)-3-hydroxy-2-oxopyrrolidine-3-carboxamide |
| "A126" | N-(3-Chloro-5-fluorobenzyl)-1-(3-acetylaminocyclohexyl)-3-hydroxy-2-oxopyrrolidine-3-carboxamide |
| "A127" | N-(3-Chloro-5-fluorobenzyl)-1-(4-ethylaminocyclohexyl)-3-hydroxy-2-oxopyrrolidine-3-carboxamide |
| "A128" | N-(3-Chloro-5-fluorobenzyl)-1-(3-ethanesulfonylaminocyclohexyl)-3-hydroxy-2-oxopyrrolidine-3-carboxamide |
| "A129" | N-(3-Chloro-5-fluorobenzyl)-1-((3aR,4S,7aS)-2,2-dimethylhexahydrobenzo-1,3-dioxol-4-yl)-3-hydroxy-2-oxopyrrolidine-3-carboxamide |
| "A130" | 5-[3-(3-Chloro-5-fluorobenzylcarbamoyl)-3-hydroxy-2-oxopyrrolidin-1-yl]isophthalamide |
| "A131" | N-(3-Chloro-5-fluorobenzyl)-(R)-3-hydroxy-2-oxo-[1,3’]bipyrrolidinyl-3-carboxamide |
| "A132" | N-(3-Chloro-5-fluorobenzyl)-(R)-3-hydroxy-2-oxo-1-(2-oxo1,2,3,4-tetrahydroquinolin-6-yl)pyrrolidine-3-carboxamide |
| "A133" | N-(3-Chloro-5-fluorobenzyl)-3-hydroxy-1-(3-methylcarbamoylphenyl)-2-oxopyrrolidine-3-carboxamide |

(continued)

| Compound No. | Name |
|---|---|
| "A134" | N-(3-Chloro-5-fluorobenzyl)-1-(3-dimethylcarbamoylphenyl)-3-hydroxy-2-oxopyrrolidine-3-carboxamide |
| "A135" | N-(3-Chloro-5-fluorobenzyl)-3-hydroxy-1-(4-methoxybenzyl)-2-oxopyrrolidine-3-carboxamide |
| "A136" | N-(3-Chloro-5-fluorobenzyl)-1-benzyl-3-hydroxy-2-oxopyrrolidine-3-carboxamide |
| "A137" | N-[2-(3-Chloro-5-fluorophenyl)ethyl]-3-hydroxy-2-oxo-1-(2-oxo-1,2,3,4-tetrahydroquinolin-6-yl)pyrrolidine-3-carboxamide |
| "A138" | N-(3-Chloro-5-fluorobenzyl)-3-hydroxy-1-(6-methoxy-pyridin-3-yl)-2-oxopyrrolidine-3-carboxamide |
| "A139" | N-[2-(3-Chloro-5-fluorophenyl)ethyl]-1-(6-acetylaminopyridin-3-yl)-3-hydroxy-2-oxopyrrolidine-3-carboxamide |
| "A140" | N-(3-Chloro-5-fluorobenzyl)-(S)-1-benzyl-3-hydroxy-2-oxopyrrolidine-3-carboxamide |
| "A141" | N-(3-Chloro-5-fluorobenzyl)-(R)-1-benzyl-3-hydroxy-2-oxopyrrolidine-3-carboxamide |
| "A142" | N-(3-Chloro-5-fluorobenzyl)-3-hydroxy-1-[5-methyl-6-(3-oxomorpholin-4-yl)pyridin-3-yl)-2-oxopyrrolidine-3-carboxamide |
| "A143" | N-[2-(2-Trifluoromethylphenyl)ethyl]-1-(6-acetylaminopyridin-3-yl)-3-hydroxy-2-oxopyrrolidine-3-carboxamide |
| "A144" | N-[2-(2-Trifluoromethylphenyl)ethyl]-1-(3-carbamoylphenyl)-3-hydroxy-2-oxopyrrolidine-3-carboxamide |
| "A145" | N-[1-(3-Fluorophenyl)ethyl]-3-hydroxy-2-oxo-1-phenylpyrrolidine-3-carboxamide |
| "A146" | N-(3-Chloro-5-fluorobenzyl)-3-hydroxy-1-(2-methylcarbamoylbenzofuran-5-yl)-2-oxopyrrolidine-3-carboxamide |
| "A147" | N-(3-Ch loro-5-fluorobenzyl)-3-hyd roxy-2-oxo-1-(2-oxo-1,2-dihydroquinolin-6-yl)pyrrolidine-3-carboxamide |
| "A148" | Ethyl 4-[3-(3-chloro-5-fluorobenzylcarbamoyl)-3-hydroxy-2-oxopyrrolidin-1-yl]cyclohexanecarboxylate |
| "A149" | N-[2-(3-Ch loro-5-fluorophenyl)ethyl]-(S)-3-hyd roxy-2-oxo-1-(2-oxo-1,2,3,4-tetrahydroquinolin-6-yl)pyrrolidine-3-carboxamide |
| "A150" | N-[2-(3-Chloro-5-fluorophenyl)ethyl]-(R)-3-hydroxy-2-oxo-1-(2-oxo-1,2,3,4-tetrahydroquinolin-6-yl)pyrrolidine-3-carboxamide |
| "A151" | N-(3-Chloro-5-fluorobenzyl)-3-hydroxy-2-oxo-1-(3-oxo-3,4-dihydro-2H-benzo-1,4-oxazin-7-yl)pyrrolidine-3-carboxamide |
| "A152" | 4-[3-(3-Chloro-5-fluorobenzylcarbamoyl)-3-hydroxy-2-oxopyrrolidin-1-yl]cyclohexanecarboxylic acid |
| "A153" | N-(3-Chloro-5-fluorobenzyl)-(S)-3-hydroxy-1-(1H-indol-6-yl)-2-oxopyrrolidine-3-carboxamide |
| "A154" | N-(3-Chloro-5-fluorobenzyl)-(R)-3-hydroxy-1-(1H-indol-6-yl)-2-oxopyrrolidine-3-carboxamide |
| "A155" | N-[2-(3-Chloro-5-fluorophenyl)ethyl]-(S)-1-(6-acetylaminopyridin-3-yl)-3-hydroxy-2-oxopyrrolidine-3-carboxamide |
| "A156" | N-[2-(3-Chloro-5-fluorophenyl)ethyl]-(R)-1-(6-acetylaminopyridin-3-yl)-3-hydroxy-2-oxopyrrolidine-3-carboxamide |
| "A157" | N-(3-Chloro-5-fluorobenzyl)-3-hydroxy-1-(4-methylcarbamoylcyclohexyl)-2-oxopyrrolidine-3-carboxamide |
| "A158" | N-(2-Oxo-1,2,3,4-tetrahydroquinolin-6-yl)-2-oxo-1-(2-oxo-1,2,3,4-tetrahydroquinolin-6-yl)pyrrolidine-3-carboxamide |

(continued)

| Compound No. | Name |
|---|---|
| "A159" | N-[2-(3-Chloro-5-fluorophenyl)ethyl]-(R)-1-(3-carbamoylphenyl)-3-hydroxy-2-oxopyrrolidine-3-carboxamide |
| "A160" | N-(3-Chloro-5-fluorobenzyl)-(R)-3-hydroxy-1-(3-methane-sulfonylaminophenyl)-2-oxopyrrolidine-3-carboxamide |
| "A161" | N-(3-Chloro-5-fluorobenzyl)-(S)-3-hydroxy-1-(3-methylcarbamoylphenyl)-2-oxopyrrolidine-3-carboxamide |
| "A162" | N-(3-Chloro-5-fluorobenzyl)-(R)-3-hydroxy-1-(3-methylcarbamoylphenyl)-2-oxopyrrolidine-3-carboxamide |
| "A163" | N-(3-Chloro-5-fluorobenzyl)-(S)-1-(3-cyclobutylcarbamoylphenyl)-3-hydroxy-2-oxopyrrolidine-3-carboxamide |
| "A164" | N-(3-Chloro-5-fluorobenzyl)-(R)-1-(3-cyclobutylcarbamoylphenyl)-3-hydroxy-2-oxopyrrolidine-3-carboxamide |
| "A165" | N-(3-Chloro-5-fluorobenzyl)-(S)-3-hydroxy-1-[3-(methanesulfonylmethylamino)phenyl]-2-oxopyrrolidine-3-carboxamide |
| "A166" | N-(3-Chloro-5-fluorobenzyl)-(R)-3-hydroxy-1-[3-(methanesulfonylmethylamino)phenyl]-2-oxopyrrolidine-3-carboxamide |
| "A167" | N-(3-Chloro-5-fluorobenzyl)-3-hydroxy-2-oxo-1-[6-(3-oxomorpholin-4-yl)pyridin-3-yl]pyrrolidine-3-carboxamide |
| "A168" | N-[2-(3-Chloro-5-fluorophenyl)ethyl]-3-hydroxy-1-(1H-indazol-5-yl)-2-oxopyrrolidine-3-carboxamide |
| "A169" | N-(3-Chloro-5-fluorobenzyl)-3-hydroxy-2-oxo-1-(1-phenyl-1H-pyrazol-4-yl)pyrrolidine-3-carboxamide |
| "A170" | tert-Butyl 4-{4-[3-(3-chloro-5-fluorobenzylcarbamoyl)-3-hydroxy-2-oxopyrrolidin-1-yl]pyrazol-1-yl}piperidine-1-carboxylate |
| "A171" | N-(3-Chloro-5-fluorobenzyl)-(S)-3-hydroxy-2-oxo-1-(6-propionylaminopyridin-3-yl)pyrrolidine-3-carboxamide |
| "A172" | N-(3-Chloro-5-fluorobenzyl)-(R)-3-hydroxy-2-oxo-1-(6-propionylaminopyridin-3-yl)pyrrolidine-3-carboxamide |
| "A173" | N-(3-Chloro-5-fluorobenzyl)-3-hydroxy-1-(6-morpholin-4-ylpyridin-3-yl)-2-oxopyrrolidine-3-carboxamide |
| "A174" | N-[2-(3-Fluorophenyl)ethyl]-1-(6-acetylaminopyridin-3-yl)-3-hydroxy-2-oxopyrrolidine-3-carboxamide |
| "A175" | N-[2-(2-Fluorophenyl)ethyl]-1-(6-acetylaminopyridin-3-yl)-3-hydroxy-2-oxopyrrolidine-3-carboxamide |
| "A176" | N-(3,5-Difluorobenzyl)-1-(6-acetylaminopyridin-3-yl)-3-hydroxy-2-oxopyrrolidine-3-carboxamide |
| "A177" | N-[2-(3-Fluorophenyl)ethyl]-1-(3-carbamoylphenyl)-3-hydroxy-2-oxopyrrolidine-3-carboxamide |
| "A178" | N-(3,5-Difluorobenzyl)-1-(3-carbamoylphenyl)-3-hydroxy-2-oxopyrrolidine-3-carboxamide |
| "A179" | N-[2-(2-Fluorophenyl)ethyl]-1-(3-carbamoylphenyl)-3-hydroxy-2-oxopyrrolidine-3-carboxamide |
| "A180" | N-(3-Chloro-5-fluorobenzyl)-3-hydroxy-2-oxo-1-(1-piperidin-4-yl-1H-pyrazol-4-yl)pyrrolidine-3-carboxamide |
| "A181" | N-(3-Chloro-5-fluorobenzyl)-(S)-3-hydroxy-1-(4-methoxybenzyl)-2-oxopyrrolidine-3-carboxamide |

(continued)

| Compound No. | Name |
|---|---|
| "A182" | N-(3-Chloro-5-fluorobenzyl)-(R)-3-hydroxy-1-(4-methoxybenzyl)-2-oxopyrrolidine-3-carboxamide |
| "A183" | N-[2-(3-Fluorophenyl)ethyl]-3-hydroxy-2-oxo-1-(2-oxo-1,2,3,4-tetrahydroquinolin-6-yl)pyrrolidine-3-carboxamide |
| "A184" | N-[2-(2-Fluorophenyl)ethyl]-3-hydroxy-2-oxo-1-(2-oxo-1,2,3,4-tetrahydroquinolin-6-yl)pyrrolidine-3-carboxamide |
| "A185" | N-(3,5-Difluorobenzyl)-3-hydroxy-2-oxo-1-(2-oxo-1,2,3,4-tetrahydroquinolin-6-yl)pyrrolidine-3-carboxamide |
| "A186" | N-(2-Chloro-3-fluorobenzyl)-1-(6-acetylaminopyridin-3-yl)-3-hydroxy-2-oxopyrrolidine-3-carboxamide |
| "A187" | N-(3-Chloro-5-fluorobenzyl)-3-hyd roxy-2-oxo-1-phenylcarbamoylmethylpyrrolidine-3-carboxamide |
| "A188" | N-[2-(3,5-Difluorophenyl)ethyl]-1-(6-acetylaminopyridin-3-yl)-3-hydroxy-2-oxopyrrolidine-3-carboxamide |
| "A189" | N-(3-Chloro-5-fluorobenzyl)-(S)-3-hydroxy-1-(1H-indazol-5-yl)-2-oxopyrrolidine-3-carboxamide |
| "A190" | N-(3-Chloro-5-fluorobenzyl)-(R)-3-hydroxy-1-(1H-indazol-5-yl)-2-oxopyrrolidine-3-carboxamide |
| "A191" | N-[2-(3-Chloro-5-fluorophenyl)ethyl]-(S)-3-hydroxy-1-(1H-indazol-5-yl)-2-oxopyrrolidine-3-carboxamide |
| "A192" | N-[2-(3-Chloro-5-fluorophenyl)ethyl]-(R)-3-hydroxy-1-(1H-indazol-5-yl)-2-oxopyrrolidine-3-carboxamide |
| "A193" | N-(2-Chloro-3-fluorobenzyl)-1-(3-carbamoylphenyl)-3-hydroxy-2-oxopyrrolidine-3-carboxamide |
| "A194" | N-(3-Chloro-5-fluorobenzyl)-(S)-1-(2-ethylcarbamoylbenzofuran-5-yl)-3-hydroxy-2-oxopyrrolidine-3-carboxamide |
| "A195" | N-(3-Chloro-5-fluorobenzyl)-(R)-1-(2-ethylcarbamoylbenzofuran-5-yl)-3-hydroxy-2-oxopyrrolidine-3-carboxamide |
| "A196" | N-(2-Ch loro-3-fluorobenzyl)-3-hydroxy-2-oxo-1-(2-oxo-1,2,3,4-tetrahydroquinolin-6-yl)pyrrolidine-3-carboxamide |
| "A197" | N-(3-Chloro-5-fluorobenzyl)-(S)-3-hydroxy-2-oxo-1-(3-oxo-3,4-dihydro-2H-benzo-1,4-oxazin-7-yl)pyrrolidine-3-carboxamide |
| "A198" | N-(3-Chloro-5-fluorobenzyl)-(R)-3-hydroxy-2-oxo-1-(3-oxo-3,4-dihydro-2H-benzo-1,4-oxazin-7-yl)pyrrolidine-3-carboxamide |
| "A199" | N-[2-(3,5-Difluorophenyl)ethyl]-3-hydroxy-2-oxo-1-(2-oxo-1,2,3,4-tetrahydroquinolin-6-yl)pyrrolidine-3-carboxamide |
| "A200" | N-(3-Chloro-5-fluorobenzyl)-3-hydroxy-1-(2-methyl-1H-indol-5-yl)-2-oxopyrrolidine-3-carboxamide |
| "A201" | N-[2-(3,5-Difluorophenyl)ethyl]-1-(3-carbamoylphenyl)-3-hydroxy-2-oxopyrrolidine-3-carboxamide |
| "A202" | Ethyl 3-[3-(3-chloro-5-fluorobenzylcarbamoyl)-3-hydroxy-2-oxopyrrolidin-1-yl]cyclohexanecarboxylate |
| "A203" | [3-(3-Chloro-5-fluorobenzylcarbamoyl)-3-hydroxy-2-oxopyrrolidin-1-yl]acetic acid |

(continued)

| Compound No. | Name |
|---|---|
| "A204" | N-(3-Chloro-5-fluorobenzyl)-1-(3-carbamoyl-5-trifluoromethylphenyl)-3-hydroxy-2-oxopyrrolidine-3-carboxamide |
| "A205" | Ethyl 5-[3-(3-chloro-5-fluorobenzylcarbamoyl)-3-hydroxy-2-oxopyrrolidin-1-yl]-1H-indole-2-carboxylate |
| "A206" | N-(2-Chloro-3-fluorobenzyl)-3-hydroxy-1-(1H-indol-5-yl)-2-oxopyrrolidine-3-carboxamide |
| "A207" | N-[2-(3,5-Difluorophenyl)ethyl]-3-hydroxy-1-(1H-indol-5-yl)-2-oxopyrrolidine-3-carboxamide |
| "A208" | N-(3-Chloro-5-fluorobenzyl)-3-hydroxy-1-methylcarbamoylmethyl-2-oxopyrrolidine-3-carboxamide |
| "A209" | N-(3,5-Difluorobenzyl)-3-hydroxy-1-(1H-indol-5-yl)-2-oxopyrrolidine-3-carboxamide |
| "A210" | N-[2-(2-Fluorophenyl)ethyl]-3-hydroxy-1-(1H-indol-5-yl)-2-oxopyrrolidine-3-carboxamide |
| "A211" | 5-[3-(3-Chloro-5-fluorobenzylcarbamoyl)-3-hydroxy-2-oxopyrrolidin-1-yl]-1H-indole-2-carboxamide |
| "A212" | N-[2-(3-Fluorophenyl)ethyl]-3-hydroxy-1-(1H-indol-5-yl)-2-oxopyrrolidine-3-carboxamide |
| "A213" | tert-Butyl 3-[3-(3-chloro-5-fluorobenzylcarbamoyl)-3-hydroxy-2-oxopyrrolidin-1-ylmethyl]azetidine-1-carboxylate |
| "A214" | N-(3-Chloro-5-fluorobenzyl)-3-hydroxy-2-oxo-1-(7-oxo-5,6,7,8-tetrahydro-1,8-naphthyridin-3-yl)pyrrolidine-3-carboxamide |
| "A215" | N-(3-Chloro-5-fluorobenzyl)-3-hydroxy-2-oxo-1-(1H-pyrrolo[2,3-b]pyridin-5-yl)pyrrolidine-3-carboxamide |
| "A216" | 5-[3-(3-Chloro-5-fluorobenzylcarbamoyl)-3-hydroxy-2-oxopyrrolidin-1-yl]nicotinamide |
| "A217" | N-(3-Chloro-5-fluorobenzyl)-1-(2-cyano-1H-indol-5-yl)-3-hydroxy-2-oxopyrrolidine-3-carboxamide |
| "A218" | N-(3-Chloro-5-fluorobenzyl)-1-azetidin-3-ylmethyl-3-hydroxy-2-oxopyrrolidine-3-carboxamide |
| "A219" | N-(3-Chloro-5-fluorobenzyl)-1-[2-(5-fluoro-1H-indol-3-yl)-ethyl]-3-hydroxy-2-oxopyrrolidine-3-carboxamide |
| "A220" | N-(3-Chloro-5-fluorobenzyl)-3-hydroxy-2-oxo-1-(tetrahydropyran-4-ylmethyl)pyrrolidine-3-carboxamide |
| "A221" | N-[2-(3,5-Difluorophenyl)ethyl]-(S)-3-hydroxy-2-oxo-1-(2-oxo-1,2,3,4-tetrahydroquinolin-6-yl)pyrrolidine-3-carboxamide |
| "A222" | N-(3-Chloro-5-fluorobenzyl)-(S)-1-(3-cyano-1H-indol-5-yl)-3-hydroxy-2-oxopyrrolidine-3-carboxamide |
| "A223" | N-[2-(3,5-Difluorophenyl)ethyl]-(R)-3-hydroxy-2-oxo-1-(2-oxo-1,2,3,4-tetrahydroquinolin-6-yl)pyrrolidine-3-carboxamide |
| "A224" | N-(3-Chloro-5-fluorobenzyl)-(R)-1-(3-cyano-1H-indol-5-yl)-3-hydroxy-2-oxopyrrolidine-3-carboxamide |
| "A225" | N-(3-Chloro-5-fluorobenzyl)-3-hydroxy-2-oxo-1-[(R)-1-(tetrahydrofuran-2-yl)methyl]pyrrolidine-3-carboxamide |
| "A226" | 4-[3-(3-Chloro-5-fluorobenzylcarbamoyl)-3-hydroxy-2-oxopyrrolidin-1-ylmethyl]cyclohexanecarboxylic acid |
| "A227" | 4-[3-(3-Chloro-5-fluorobenzylcarbamoyl)-3-hydroxy-2-oxopyrrolidin-1-ylmethyl]cyclohexanecarboxylic acid |
| "A228" | tert-Butyl (1S,5R,6S)-6-[3-(3-chloro-5-fluorobenzylcarbamoyl)-3-hydroxy-2-oxopyrrolidin-1-yl]-3-azabicyclo-[3.1.0]hexane-3-carboxylate |

(continued)

| Compound No. | Name |
|---|---|
| "A229" | N-(3-Chloro-5-fluorobenzyl)-1-(1S,5R,6S)-3-azabicyclo-[3.1.0]hex-6-yl-3-hydroxy-2-oxopyrrolidine-3-carboxamide |
| "A230" | N-(3-Chloro-5-fluorobenzyl)-1-((1S,5R,6S)-3-ethanesulfonyl-3-azabicyclo[3.1.0]hex-6-yl)-3-hydroxy-2-oxopyrrolidine-3-carboxamide |
| "A231" | N-(3-Chloro-5-fluorobenzyl)-1-((1S,5R,6S)-3-acetyl-3-azabicyclo[3.1.0]hex-6-yl)-3-hydroxy-2-oxopyrrolidine-3-carboxamide |
| "A232" | N-(3-Chloro-5-fluorobenzyl)-3-hydroxy-1-(1-methyl-1H-benzimidazol-2-ylmethyl)-2-oxopyrrolidine-3-carboxamide |
| "A233" | N-(3-Chloro-5-fluorobenzyl)-(S)-3-hydroxy-2-oxo-[1,3']bipyrrolidinyl-3-carboxamide |
| "A234" | N-(2-Chloro-4,5-difluorobenzyl)-(S)-3-hydroxy-2-oxo-1-(2-oxo-1,2,3,4-tetrahydroquinolin-6-yl)pyrrolidine-3-carboxamide |
| "A235" | N-(5-Chloro-2,4-difluorobenzyl)-(S)-3-hydroxy-2-oxo-1-(2-oxo-1,2,3,4-tetrahydroquinolin-6-yl)pyrrolidine-3-carboxamide |
| "A236" | N-(2,4,6-Trifluorobenzyl)-(S)-3-hydroxy-2-oxo-1-(2-oxo-1,2,3,4-tetrahydroquinolin-6-yl)pyrrolidine-3-carboxamide |
| "A237" | N-(3-Chloro-5-fluorobenzyl)-1-[2-(2,4-difluorophenyl)ethyl]-3-hydroxy-2-oxopyrrolidine-3-carboxamide |
| "A238" | 5-[(S)-3-(3-Chloro-5-fluorobenzylcarbamoyl)-3-hydroxy-2-oxopyrrolidin-1-yl]-1H-indole-3-carboxamide |
| "A239" | N-(3-Chloro-5-fluorobenzyl)-(S)-3-hydroxy-1-(2-methyl-1H-indol-5-yl)-2-oxopyrrolidine-3-carboxamide |
| "A240" | N-(3-Chloro-5-fluorobenzyl)-(R)-3-hydroxy-1-(2-methyl-1H-indol-5-yl)-2-oxopyrrolidine-3-carboxamide |
| "A241" | N-(4-Chloro-2-fluorobenzyl)-(S)-3-hydroxy-2-oxo-1-(2-oxo-1,2,3,4-tetrahydroquinolin-6-yl)pyrrolidine-3-carboxamide |
| "A242" | N-(2-Chloro-4-fluorobenzyl)-(S)-3-hydroxy-2-oxo-1-(2-oxo-1,2,3,4-tetrahydroquinolin-6-yl)pyrrolidine-3-carboxamide |
| "A243" | N-(3-Chloro-5-fluorobenzyl)-3-hydroxy-2-oxo-1-[(S)-1-(tetrahydrofuran-2-yl)methyl]pyrrolidine-3-carboxamide |
| "A244" | N-(2-Chloro-3,6-difluorobenzyl)-(S)-3-hydroxy-2-oxo-1-(2-oxo-1,2,3,4-tetrahydroquinolin-6-yl)pyrrolidine-3-carboxamide |
| "A245" | N-(2,4,5-Trifluorobenzyl)-(S)-3-hydroxy-2-oxo-1-(2-oxo-1,2,3,4-tetrahydroquinolin-6-yl)pyrrolidine-3-carboxamide |
| "A246" | N-(2,3,6-Trifluorobenzyl)-(S)-3-hydroxy-2-oxo-1-(2-oxo-1,2,3,4-tetrahydroquinolin-6-yl)pyrrolidine-3-carboxamide |
| "A247" | N-(3-Chloro-4-fluorobenzyl)-(S)-3-hydroxy-2-oxo-1-(2-oxo-1,2,3,4-tetrahydroquinolin-6-yl)pyrrolidine-3-carboxamide |
| "A248" | N-(3-Chloro-5-fluorobenzyl)-1-carbamoylmethyl-3-hydroxy-2-oxopyrrolidine-3-carboxamide |
| "A249" | N-(4-Chloro-2,6-difluorobenzyl)-(S)-3-hydroxy-2-oxo-1-(2-oxo-1,2,3,4-tetrahydroquinolin-6-yl)pyrrolidine-3-carboxamide |
| "A250" | N-(3-Chloro-2,6-difluorobenzyl)-(S)-3-hydroxy-2-oxo-1-(2-oxo-1,2,3,4-tetrahydroquinolin-6-yl)pyrrolidine-3-carboxamide |
| "A251" | N-(3-Chloro-2,4-difluorobenzyl)-(S)-3-hydroxy-2-oxo-1-(2-oxo-1,2,3,4-tetrahydroquinolin-6-yl)pyrrolidine-3-carboxamide |

(continued)

| Compound No. | Name |
|---|---|
| "A252" | N-(3,5-Difluorobenzyl)-1-cyclobutylmethyl-3-hydroxy-2-oxopiperidine-3-carboxamide |
| "A253" | N-(3-Fluorobenzyl)-1-(3,4-difluorobenzyl)-3-hydroxy-2-oxopiperidine-3-carboxamide |
| "A254" | N-(3-Fluorobenzyl)-(S)-1-(2,3-difluorobenzyl)-3-hydroxy-2-oxopiperidine-3-carboxamide |
| "A255" | N-(4-Fluorobenzyl)-(S)-1-(2,3-difluorobenzyl)-3-hydroxy-2-oxopiperidine-3-carboxamide |
| "A256" | N-(3-Fluorobenzyl)-(S)-1-cyclohexylmethyl-3-hydroxy-2-oxopiperidine-3-carboxamide |
| "A257" | N-(4-Fluorobenzyl)-1-(3,4-difluorobenzyl)-3-hydroxy-2-oxopiperidine-3-carboxamide |
| "A258" | N-(5-Methylisoxazol-3-ylmethyl)-1-(3,4-difluorobenzyl)-3-hydroxy-2-oxopiperidine-3-carboxamide |
| "A259" | N-(Pyridin-2-ylmethyl)-(S)-1-(2,3-difluorobenzyl)-3-hydroxy-2-oxopiperidine-3-carboxamide |
| "A260" | N-(Pyridin-2-ylmethyl)-(R)-1-(2,3-difluorobenzyl)-3-hydroxy-2-oxopiperidine-3-carboxamide |
| "A261" | N-(3-Fluorobenzyl)-(R)-1-(2,3-difluorobenzyl)-3-hydroxy-2-oxopiperidine-3-carboxamide |
| "A262" | N-(4-Fluorobenzyl)-(R)-1-(2,3-difluorobenzyl)-3-hydroxy-2-oxopiperidine-3-carboxamide |
| "A263" | N-(3-Fluorobenzyl)-(R)-1-cyclohexylmethyl-3-hydroxy-2-oxopiperidine-3-carboxamide |
| "A264" | N-(3,4,5-Trifluorobenzyl)-(S)-3-hydroxy-2-oxo-1-(2-oxo-1,2,3,4-tetrahydroquinolin-6-yl)pyrrolidine-3-carboxamide |
| "A265" | N-(4-Chloro-3-fluorobenzyl)-(S)-3-hydroxy-2-oxo-1-(2-oxo-1,2,3,4-tetrahydroquinolin-6-yl)pyrrolidine-3-carboxamide |
| "A266" | N-(2,3,5-Trifluorobenzyl)-(S)-3-hydroxy-2-oxo-1-(2-oxo-1,2,3,4-tetrahydroquinolin-6-yl)pyrrolidine-3-carboxamide |
| "A267" | 3-(1,3-Dihydroisoindole-2-carbonyl)-3-hydroxy-1-phenylpyrrolidin-2-one |
| "A268" | 1-Benzyl-3-(1,3-dihydroisoindole-2-carbonyl)-3-hydroxypyrrolidin-2-one |
| "A269" | N-(3-Chloro-5-fluorobenzyl)-1-(1H-benzimidazol-2-ylmethyl)-3-hydroxy-2-oxopyrrolidine-3-carboxamide |
| "A270" | N-(3-Chloro-5-fluorobenzyl)-1-benzothiazol-2-ylmethyl-3-hydroxy-2-oxopyrrolidine-3-carboxamide |
| "A271" | 3-[3-(3-Chloro-5-fluorobenzylcarbamoyl)-3-hydroxy-2-oxopyrrolidin-1-yl]propionic acid |
| "A272" | N-(3-Chloro-5-fluorobenzyl)-3-hydroxy-1-(3-methyl-3H-imidazol-4-ylmethyl)-2-oxopyrrolidine-3-carboxamide |
| "A273" | 4-[3-(3-Chloro-5-fluorobenzylcarbamoyl)-3-hydroxy-2-oxopyrrolidin-1-ylmethyl]cyclohexanecarboxylic acid |
| "A274" | 4-[3-(3-Chloro-5-fluorobenzylcarbamoyl)-3-hydroxy-2-oxopyrrolidin-1-ylmethyl]cyclohexanecarboxylic acid |
| "A275" | N-(3-Chloro-5-fluorobenzyl)-1-((1S,5R,6S)-3-ethyl-3-azabicyclo[3.1.0]hex-6-yl)-3-hydroxy-2-oxopyrrolidine-3-carboxamide |
| "A276" | N-(3-Chloro-5-fluorobenzyl)-3-hydroxy-1-(2-methyl-2H-pyrazol-3-ylmethyl)-2-oxopyrrolidine-3-carboxamide |
| "A277" | N-(3-Chloro-5-fluorobenzyl)-3-hydroxy-2-oxo-1-(1H-pyrazol-3-ylmethyl)pyrrolidine-3-carboxamide |
| "A278" | N-(3-Chloro-5-fluorobenzyl)-3-hydroxy-1-(4-methylcarbamoylcyclohexylmethyl)-2-oxopyrrolidine-3-carboxamide |
| "A279" | N-(3-Chloro-5-fluorobenzyl)-3-hydroxy-1-(1H-imidazol-4-ylmethyl)-2-oxopyrrolidine-3-carboxamide |

(continued)

| Compound No. | Name |
|---|---|
| "A280" | N-(3-Chloro-5-fluorobenzyl)-1-(1,5-dimethyl-1H-pyrrol-2-ylmethyl)-3-hydroxy-2-oxopyrrolidine-3-carboxamide |
| "A281" | N-(3-Chloro-5-fluorobenzyl)-(S)-3-hydroxy-1-methanesulfonyl-2-oxo[1,3']bipyrrolidinyl-3-carboxamide |
| "A282" | N-(3-Chloro-5-fluorobenzyl)-3-hydroxy-1-[(2-hydroxyethylcarbamoyl)methyl]-2-oxopyrrolidine-3-carboxamide |
| "A283" | N-(2-Chloro-5-fluorobenzyl)-(S)-3-hydroxy-2-oxo-1-(2-oxo-1,2,3,4-tetrahydroquinolin-6-yl)pyrrolidine-3-carboxamide |
| "A284" | N-(2,3,4-Trifluorobenzyl)-(S)-3-hydroxy-2-oxo-1-(2-oxo-1,2,3,4-tetrahydroquinolin-6-yl)pyrrolidine-3-carboxamide |
| "A285" | N-(2,3-Difluorobenzyl)-(S)-3-hydroxy-2-oxo-1-(2-oxo-1,2,3,4-tetrahydroquinolin-6-yl)pyrrolidine-3-carboxamide |
| "A286" | N-(3-Chloro-5-fluorobenzyl)-(S)-1-(3,3-difluoro-2-oxo-2,3-dihydro-1H-indol-5-yl)-3-hydroxy-2-oxopyrrolidine-3-carboxamide |
| "A287" | N-(3-Fluorobenzyl)-1-(4-fluorobenzyl)-3-hydroxy-2-oxopiperidine-3-carboxamide |
| "A288" | N-(3-Chloro-5-fluorobenzyl)-(R)-1-(3,3-difluoro-2-oxo-2,3-dihydro-1H-indol-5-yl)-3-hydroxy-2-oxopyrrolidine-3-carboxamide |
| "A289" | N-(2,6-Difluorobenzyl)-(S)-3-hydroxy-2-oxo-1-(2-oxo-1,2,3,4-tetrahydroquinolin-6-yl)pyrrolidine-3-carboxamide |
| "A290" | N-(3-Chloro-5-fluorobenzyl)-3-hydroxy-1-(5-methyl-1,3,4-oxadiazol-2-ylmethyl)-2-oxopyrrolidine-3-carboxamide |
| "A291" | N-(3-Chloro-5-fluorobenzyl)-(R)-1'-acetyl-3-hydroxy-2-oxo-[1,3']bipyrrolidinyl-3-carboxamide |
| "A292" | N-(3-Chloro-5-fluorobenzyl)-(R)-3-hydroxy-1'-methanesulfonyl-2-oxo[1,3']bipyrrolidinyl-3-carboxamide |
| "A293" | N-(3-Chloro-5-fluorobenzyl)-3-hydroxy-1-(4-methylcyclohexyl)-2-oxopyrrolidine-3-carboxamide |
| "A294" | N-(5-Methylisoxazol-3-ylmethyl)-1-(2,3-difluorobenzyl)-3-hydroxy-2-oxopiperidine-3-carboxamide |
| "A295" | N-(4-Methylbenzyl)-1-(2,3-difluorobenzyl)-3-hydroxy-2-oxopiperidine-3-carboxamide |
| "A296" | N-(2-Fluorobenzyl)-1-(2,3-difluorobenzyl)-3-hydroxy-2-oxopiperidine-3-carboxamide |
| "A297" | N-(5-Methylpyrazin-2-ylmethyl)-1-(2,3-difluorobenzyl)-3-hydroxy-2-oxopiperidine-3-carboxamide |
| "A298" | N-(3-Chloro-5-fluorobenzyl)-(S)-3-hydroxy-2-oxo-1-pyridin-2-ylpyrrolidine-3-carboxamide |
| "A299" | N-(3-Chloro-5-fluorobenzyl)-(R)-3-hydroxy-2-oxo-1-pyridin-2-ylpyrrolidine-3-carboxamide |
| "A300" | N-(3-Chloro-5-fluorobenzyl)-1-(2-carbamoylethyl)-3-hydroxy-2-oxopyrrolidine-3-carboxamide |
| "A301" | N-(3-Chloro-5-fluorobenzyl)-1-benzyl-3-hydroxypiperidine-3-carboxamide |
| "A302" | 3-[(3-Chloro-5-fluorobenzylamino)methyl]-3-hydroxy-1-phenylpiperidin-2-one |
| "A303" | N-(3-Chloro-5-fluorobenzyl)-3,4-dihydroxy-2-oxo-1-(2-oxo-1,2,3,4-tetrahydroquinolin-6-yl)pyrrolidine-3-carboxamide |
| "A304" | N-(3-Chloro-5-fluorobenzyl)-5-fluoro-3-hydroxy-2-oxo-1-(2-oxo-1,2,3,4-tetrahydroquinolin-6-yl)pyrrolidine-3-carboxamide |
| "B1" | N-(3-Chloro-5-fluorobenzyl)-(S)-1-(2-cyano-1H-indol-5-yl)-3-hydroxy-2-oxopyrrolidine-3-carboxamide |

(continued)

| Compound No. | Name |
|---|---|
| "B2" | N-(3-Chloro-5-fluorobenzyl)-(S)-1-(8-fluoro-2-oxo-1,2,3,4-tetrahydroquinolin-6-yl)-3-hydroxy-2-oxopyrrolidine-3-carboxamide |
| "B3" | N-(2-Chloro-6-fluorobenzyl)-(S)-3-hydroxy-2-oxo-1-(2-oxo-1,2,3,4-tetrahydroquinolin-6-yl)pyrrolidine-3-carboxamide |
| "B4" | N-(5-Chloro-2-fluorobenzyl)-(S)-3-hydroxy-2-oxo-1-(2-oxo-1,2,3,4-tetrahydroquinolin-6-yl)pyrrolidine-3-carboxamide |
| "B5" | N-[2-(2-Fluorophenyl)ethyl]-(S)-3-hydroxy-2-oxo-1-(2-oxo-1,2,3,4-tetrahydroquinolin-6-yl)pyrrolidine-3-carboxamide |
| "B6" | N-[2-(3-Fluorophenyl)ethyl]-(S)-3-hydroxy-2-oxo-1-(2-oxo-1,2,3,4-tetrahydroquinolin-6-yl)pyrrolidine-3-carboxamide |
| "B7" | N-(3,5-Difluorobenzyl)-(S)-3-hydroxy-2-oxo-1-(2-oxo-1,2,3,4-tetrahydroquinolin-6-yl)pyrrolidine-3-carboxamide |
| "B8" | N-(3,5-Difluorobenzyl)-(S)-3-hydroxy-1-(1H-indol-5-yl)-2-oxopyrrolidine-3-carboxamide |
| "B9" | N-(2-Chloro-3-fluorobenzyl)-(S)-1-(6-acetylaminopyridin-3-yl)-3-hydroxy-2-oxopyrrolidine-3-carboxamide |
| "B10" | N-[2-(3-Fluorophenyl)ethyl]-(S)-3-hydroxy-1-(1H-indol-5-yl)-2-oxopyrrolidine-3-carboxamide |
| "B11" | N-(3-Chloro-5-fluorobenzyl)-(S)-3-hydroxy-2-oxo-1-(1H-pyrrolo[2,3-b]pyridin-5-yl)pyrrolidine-3-carboxamide |
| "B12" | N-(3-Chloro-5-fluorobenzyl)-1-(4-chloro-2-methoxy-5-methylphenyl)-3-hydroxy-2-oxopyrrolidine-3-carboxamide |
| "B13" | 1-(5-Chloro-2-methoxyphenyl)-3-hydroxy-2-oxopyrrolidine-3-carboxylic acid 3-chloro-5-fluorobenzylamide |
| "B14" | (S)-3-Hydroxy-1-(1H-indol-5-yl)-2-oxopyrrolidine-3-carboxylic acid [2-(2-fluorophenyl)ethyl]-amide |
| "B15" | N-(2-Chloro-3-fluorobenzyl)-(S)-3-hydroxy-2-oxo-1-(2-oxo-1,2,3,4-tetrahydroquinolin-6-yl)pyrrolidine-3-carboxamide |
| "B16" | N-(3-Chloro-5-fluorobenzyl)-1-benzyl-3-hydroxypiperidine-3-carboxamide |
| "B17" | N-(3-Chloro-5-fluorobenzyl)-(S)-1-(4-bromo-3-hydroxymethylphenyl)-3-hydroxy-2-oxopyrrolidine-3-carboxamide |
| "B18" | 3-[(3-Chloro-5-fluorobenzylamino)methyl]-1-cyclohexylmethyl-3-hydroxypiperidin-2-one |
| "B19" | 1-Benzyl-3-[(3-chloro-5-fluorobenzylamino)-methyl]piperidin-3-ol |
| "B20" | 1-Benzyl-3-[(3-fluorobenzylamino)methyl]piperidin-3-ol |
| "B21" | (S)-1-Benzyl-3-[(4-fluorobenzylamino)methyl]piperidin-3-ol |
| "B22" | (S)-1-(2,3-Difluorobenzyl)-3-[(3-fluorobenzylamino)methyl]-3-hydroxypiperidin-2-one |
| "B23" | (R)-1-(2,3-Difluorobenzyl)-3-[(3-fluorobenzylamino)methyl]-3-hydroxypiperidin-2-one |
| "B24" | N-(2,5-Difluorobenzyl)-(S)-3-hydroxy-2-oxo-1-(2-oxo-1,2,3,4-tetrahydroquinolin-6-yl)pyrrolidine-3-carboxamide |
| "B25" | N-(3-Chloro-2-fluorobenzyl)-(S)-3-hydroxy-2-oxo-1-(2-oxo-1,2,3,4-tetrahydroquinolin-6-yl)pyrrolidine-3-carboxamide |
| "B26" | N-(3-Chloro-5-fluorobenzyl)-1-(2-fluoro-5-trifluoromethylphenyl)-3-hydroxy-2-oxopyrrolidine-3-carboxamide |
| "B27" | N-(3-Chloro-5-fluorobenzyl)-1-(4-chloro-3-trifluoromethylphenyl)-3-hydroxy-2-oxopyrrolidine-3-carboxamide |

(continued)

| Compound No. | Name |
|---|---|
| "B28" | N-(3-Chloro-5-fluorobenzyl)-1-(3-carbamoyl-4-fluorophenyl)-3-hydroxy-2-oxopyrrolidine-3-carboxamide |
| "B29" | 3-[(3-Chloro-5-fluorobenzylamino)methyl]-3-hydroxy-1-phenylpyrrolidin-2-one |
| "B30" | 1-Benzyl-3-[(3-chloro-5-fluorobenzylamino)methyl]-pyrrolidin-3-ol |
| "B31" | 1-Benzyl-3-[(3-chloro-5-fluorobenzylamino)methyl]-3-hydroxypiperidin-2-one |
| "B32" | 3-[(3-Chloro-5-fluorobenzylamino)methyl]-3-hydroxy-1-phenylpiperidin-2-one |
| "B33" | N-(3-Chloro-5-fluorobenzyl)-3-hydroxy-1-(2-methyl-3H-imidazo[4,5-b]pyridin-6-yl)-2-oxopyrrolidine-3-carboxamide |
| "B34" | N-(3-Chloro-2,5-difluorobenzyl)-(S)-3-hydroxy-2-oxo-1-(2-oxo-1,2,3,4-tetrahydroquinolin-6-yl)pyrrolidine-3-carboxamide |
| "B35" | N-(3-Chloro-5-fluorobenzyl)-3-hydroxy-1-(3H-imidazo[4,5-b]pyridin-6-yl)-2-oxopyrrolidine-3-carboxamide |
| "B36" | N-(3-Chloro-5-fluorobenzyl)-3-hydroxy-2-oxo-1-(2-trifluoromethyl-3H-imidazo[4,5-b]pyridin-6-yl)pyrrolidine-3-carboxamide |
| "B37" | N-(3-Chloro-5-fluorobenzyl)-(S)-3-hydroxy-1-[2-(1H-indol-3-yl)ethyl]-2-oxopyrrolidine-3-carboxamide |
| "B38" | N-(3-Chloro-5-fluorobenzyl)-(R)-3-hydroxy-1-[2-(1H-indol-3-yl)ethyl]-2-oxopyrrolidine-3-carboxamide |
| "B39" | N-(3-Chloro-5-fluorobenzyl)-(S)-1-(2-fluoro-5-trifluoromethylphenyl)-3-hydroxy-2-oxopyrrolidine-3-carboxamide |
| "B40" | N-(3-Chloro-5-fluorobenzyl)-(S)-3-hydroxy-2-oxo-1-(2-oxo-1,2-dihydroquinolin-6-yl)pyrrolidine-3-carboxamide |
| "B41" | N-(3-Chloro-5-fluorobenzyl)-(R)-3-hydroxy-2-oxo-1-(2-oxo-1,2-dihydroquinolin-6-yl)pyrrolidine-3-carboxamide |
| "B42" | N-(3-Chloro-5-fluorobenzyl)-(R)-1-(2-fluoro-5-trifluoromethylphenyl)-3-hydroxy-2-oxopyrrolidine-3-carboxamide |
| "B43" | N-(3-Chloro-5-fluorobenzyl)-3-hydroxy-2-oxo-1-(3-trifluoromethyl-1H-indol-5-yl)pyrrolidine-3-carboxamide |
| "B44" | 1-Benzyl-3-[(3,5-difluorobenzylamino)methyl]piperidin-3-ol |
| "B45" | 3-[(4-Fluorobenzylamino)methyl]-3-hydroxy-1-phenylpyrrolidin-2-one |
| "B46" | 3-[(3,5-Difluorobenzylamino)methyl]-3-hydroxy-1-phenylpyrrolidin-2-one |
| "B47" | 3-{[2-(4-Fluorophenyl)ethylamino]methyl}-3-hydroxy-1-phenylpyrrolidin-2-one |
| "B48" | 3-[(3-Fluorobenzylamino)methyl]-3-hydroxy-1-phenylpyrrolidin-2-one |
| "B49" | 3-Hydroxy-3-[(4-methylbenzylamino)methyl]-1-phenylpyrrolidin-2-one |
| "B50" | N-(2-Oxo-1,2,3,4-tetrahydroquinolin-6-yl)-3-hydroxy-2-oxo-1-(2-oxo-1,2,3,4-tetrahydroquinolin-6-yl)pyrrolidine-3-carboxamide |
| "B51" | 3-Benzyl 1-tert-butyl 3-hydroxy-2-oxopyrrolidine-1,3-dicarboxylate |
| "B52" | N-(3-Chloro-5-fluorobenzyl)-(S)-3-hydroxy-2-oxo-1-(4-trifluoromethylpyridin-2-yl)pyrrolidine-3-carboxamide |
| "B53" | 1-Benzyl 3-isopropyl 3-hydroxy-2-oxopyrrolidine-1,3-dicarboxylate |
| "B54" | (S)-1-Benzyl-3-[(3-chloro-5-fluorobenzylamino)methyl]-3-hydroxypiperidin-2-one |
| "B55" | (R)-1-Benzyl-3-[(3-chloro-5-fluorobenzylamino)methyl]-3-hydroxypiperidin-2-one |

(continued)

| Compound No. | Name |
|---|---|
| "B56" | N-(3-Fluorobenzyl)-(S)-3-hydroxy-2-oxo-1-(2-oxo-1,2,3,4-tetrahydroquinolin-6-yl)pyrrolidine-3-carboxamide |
| "B57" | N-(3-Fluorobenzyl)-(R)-3-hydroxy-2-oxo-1-(2-oxo-1,2,3,4-tetrahydroquinolin-6-yl)pyrrolidine-3-carboxamide |
| "B58" | (R)-3-[(3-Chloro-5-fluorobenzylamino)methyl]-3-hydroxy-1-phenylpyrrolidin-2-one |
| "B59" | (S)-3-[(3-Chloro-5-fluorobenzylamino)methyl]-3-hydroxy-1-phenylpyrrolidin-2-one |
| "B60" | N-(3-Chloro-5-fluorobenzyl)-(S)-3-hydroxy-1-(1H-indazol-3-ylmethyl)-2-oxopyrrolidine-3-carboxamide |
| "B61" | N-(3-Chloro-5-fluorobenzyl)-(R)-3-hydroxy-1-(2-methyl-1H-pyrrolo[2,3-b]pyridin-5-yl)-2-oxopyrrolidine-3-carboxamide |
| "B62" | N-(3-Chloro-5-fluorobenzyl)-(S)-3-hydroxy-1-(2-methyl-1H-pyrrolo[2,3-b]pyridin-5-yl)-2-oxopyrrolidine-3-carboxamide |
| "B63" | (S)-3-[(3-Chloro-5-fluorobenzylamino)methyl]-1-cyclohexylmethyl-3-hydroxypiperidin-2-one |
| "B64" | (R)-3-[(3-Chloro-5-fluorobenzylamino)methyl]-1-cyclohexylmethyl-3-hydroxypiperidin-2-one |
| "B65" | N-(3-Chloro-5-fluorobenzyl)-(S)-1-(2-fluoro-4-trifluoromethylphenyl)-3-hydroxy-2-oxopyrrolidine-3-carboxamide |
| "B66" | N-(3-Chloro-5-fluorobenzyl)-(S)-1-(2-fluoro-4-methyl-phenyl)-3-hydroxy-2-oxopyrrolidine-3-carboxamide |
| "B67" | N-(3-Chloro-5-fluorobenzyl)-1-(2-fluoro-5-trifluoromethyl-phenyl)-3-hydroxy-2-oxopiperidine-3-carboxamide |
| "B68" | 6-{3-[(3-Chloro-5-fluorobenzylamino)methyl]-3-hydroxy-2-oxopiperidin-1-yl}-3,4-dihydro-1H-quinolin-2-one |
| "B69" | N-(3-Chloro-5-fluorobenzyl)-3-hydroxy-2-oxo-1-(2-oxo-1,2,3,4-tetrahydroquinolin-6-ylmethyl)pyrrolidine-3-carboxamide |
| "B70" | N-(3-Chloro-5-fluorobenzyl)-(S)-1-(5-carbamoyl-2-fluoro-phenyl)-3-hydroxy-2-oxopyrrolidine-3-carboxamide |
| "B71" | N-(3-Chloro-5-fluorobenzyl)-(S)-3-hydroxy-2-oxo-1-(2-oxo-2,3-dihydro-1H-benzimidazol-5-yl)pyrrolidine-3-carboxamide |
| "B72" | N-(3-Chloro-5-fluorobenzyl)-3-hydroxy-2-oxo-1-(2-trifluoromethyl-1H-indol-5-yl)pyrrolidine-3-carboxamide |
| "B73" | N-(3-Chloro-5-fluorobenzyl)-(R)-3-hydroxy-2-oxo-1-(3-trifluoromethyl-1H-indol-5-yl)pyrrolidine-3-carboxamide |
| "B74" | N-(3-Chloro-5-fluorobenzyl)-(S)-3-hydroxy-2-oxo-1-(3-trifluoromethyl-1H-indol-5-yl)pyrrolidine-3-carboxamide |
| "B75" | N-(3-Chloro-5-fluorobenzyl)-(S)-3-hydroxy-2-oxo-1-(1H-pyrazolo[3,4-b]pyridin-5-yl)pyrrolidine-3-carboxamide |
| "B76" | N-(3-Chloro-5-fluorobenzyl)-3-hydroxy-2-oxo-1-(2-trifluoromethyl-1H-pyrrolo[2,3-b]pyridin-5-yl)pyrrolidine-3-carboxamide |
| "B77" | N-(3-Chloro-5-fluorobenzyl)-(S)-3-hydroxy-2-oxo-1-(2-oxo-2,3-dihydrobenzothiazol-6-yl)pyrrolidine-3-carboxamide |
| "B78" | 5-[(S)-3-(3-Chloro-5-fluorobenzylcarbamoyl)-3-hydroxy-2-oxopyrrolidin-1-yl]-1H-indole-2-carboxamide |
| "B79" | 5-[(R)-3-(3-Chloro-5-fluorobenzylcarbamoyl)-3-hydroxy-2-oxopyrrolidin-1-yl]-1H-indole-2-carboxamide |

(continued)

| Compound No. | Name |
|---|---|
| "B80" | Ethyl 5-[(S)-3-(3-chloro-5-fluorobenzylcarbamoyl)-3-hydroxy-2-oxopyrrolidin-1-yl]-1H-indole-2-carboxylate |
| "B81" | Ethyl 5-[(R)-3-(3-chloro-5-fluorobenzylcarbamoyl)-3-hydroxy-2-oxopyrrolidin-1-yl]-1H-indole-2-carboxylate |
| "B82" | N-(3-Chloro-5-fluorobenzyl)-3-hydroxy-1-[1-(2-methoxy-ethyl)-1H-pyrazol-4-yl]-2-oxopyrrolidine-3-carboxamide |
| "B83" | N-(3-Ch loro-5-fluorobenzyl)-(S)-3-hydroxy-2-oxo-1-(2-trifluoromethyl-1H-pyrrolo[2,3-b]pyridin-5-yl)pyrrolidine-3-carboxamide |
| "B84" | 5-[(S)-3-(3-Chloro-5-fluorobenzylcarbamoyl)-3-hydroxy-2-oxopyrrolidin-1-yl]-1H-indole-2-carboxylic acid |
| "B85" | 5-[(R)-3-(3-Chloro-5-fluorobenzylcarbamoyl)-3-hydroxy-2-oxopyrrolidin-1-yl]-1H-indole-2-carboxylic acid |
| "B86" | N-(3-Chloro-5-fluorobenzyl)-(S)-3-hydroxy-2-oxo-1-(2-trifluoromethyl-1H-indol-5-yl)pyrrolidine-3-carboxamide |
| "B87" | N-(3-Chloro-5-fluorobenzyl)-1-(1-cyanomethyl-1H-pyrazol-4-yl)-3-hydroxy-2-oxopyrrolidine-3-carboxamide |
| "B88" | N-Ethyl-5-[(R)-3-(3-chloro-5-fluorobenzylcarbamoyl)-3-hydroxy-2-oxopyrrolidin-1-yl]-1H-indole-2-carboxamide |
| "B89" | N-Isopropyl-5-[(R)-3-(3-chloro-5-fluorobenzylcarbamoyl)-3-hydroxy-2-oxopyrrolidin-1-yl]-1H-indole-2-carboxamide |
| "B90" | N,N-Dimethyl-5-[(R)-3-(3-chloro-5-fluorobenzylcarbamoyl)-3-hydroxy-2-oxopyrrolidin-1-yl]-1H-indole-2-carboxamide |
| "B91" | 3-(3-Chloro-5-fluorobenzylcarbamoyl)-2-oxo-1-(2-oxo-1,2,3,4-tetrahydroquinolin-6-yl)pyrrolidin-3-yl acetate |
| "B92" | N-(3-Chloro-5-fluorobenzyl)-(S)-3-hydroxy-1-(3H-imidazo-[4,5-b]pyridin-6-yl)-2-oxopyrrolidine-3-carboxamide |
| "B93" | N-(3-Chloro-5-fluorobenzyl)-(R)-3-hydroxy-1-(3H-imidazo-[4,5-b]pyridin-6-yl)-2-oxopyrrolidine-3-carboxamide |
| "B94" | N-Ethyl-5-[(S)-3-(3-chloro-5-fluorobenzylcarbamoyl)-3-hydroxy-2-oxopyrrolidin-1-yl]-1H-indole-2-carboxamide |
| "B95" | N,N-Dimethyl-5-[(S)-3-(3-chloro-5-fluorobenzylcarbamoyl)-3-hydroxy-2-oxopyrrolidin-1-yl]-1H-indole-2-carboxamide |
| "B96" | N-Methyl-5-[(S)-3-(3-chloro-5-fluorobenzylcarbamoyl)-3-hydroxy-2-oxopyrrolidin-1-yl]-1H-indole-2-carboxamide |
| "B97" | N-Isopropyl-5-[(S)-3-(3-chloro-5-fluorobenzylcarbamoyl)-3-hydroxy-2-oxopyrrolidin-1-yl]-1H-indole-2-carboxamide |
| "B98" | N-(3-Chloro-5-fluorobenzyl)-3-hydroxy-2-oxo-1-(2-oxo-1,2,3,4-tetrahydroquinolin-6-yl)pyrrolidine-3-carboxamide |
| "B99" | N-(3-Chloro-5-fluorobenzyl)-(S)-3-hydroxy-1-[1-(2-methoxyethyl)-1H-pyrazol-4-yl]-2-oxopyrrolidine-3-carboxamide |
| "B100" | N-(3-Chloro-5-fluorobenzyl)-(R)-3-hydroxy-1-[1-(2-methoxyethyl)-1H-pyrazol-4-yl]-2-oxopyrrolidine-3-carboxamide |
| "B101" | N-(3-Chloro-5-fluorobenzyl)-(S)-3-hydroxy-2-oxo-1-(4-sulfamoylphenyl)pyrrolidine-3-carboxamide |

(continued)

| Compound No. | Name |
|---|---|
| "B102" | N-Methyl-5-[(R)-3-(3-chloro-5-fluorobenzylcarbamoyl)-3-hydroxy-2-oxopyrrolidin-1-yl]-1H-indole-2-carboxamide |
| "B103" | (S)-3-Hydroxy-2-oxo-1-(2-oxo-1,2,3,4-tetrahydroquinolin-6-yl)pyrrolidine-3-carboxamide |
| "B104" | N-(3-Chloro-5-fluorobenzyl)-3-hydroxy-2-oxo-1-(3-trifluoromethyl-1H-pyrrolo[2,3-b]pyridin-5-yl)pyrrolidine-3-carboxamide |
| "B105" | Methyl 3-{2-tert-butoxycarbonylamino-5-[3-(3-chloro-5-fluorobenzylcarbamoyl)-3-hydroxy-2-oxopyrrolidin-1-yl]-phenyl}propionate |
| "B106" | 3-{2-tert-Butoxycarbonylamino-5-[3-(3-chloro-5-fluorobenzylcarbamoyl)-3-hydroxy-2-oxopyrrolidin-1-yl]-phenyl}propionic acid |
| "B107" | N-(3-Chloro-5-fluorobenzyl)-3-hydroxy-2-oxo-1-(3-sulfamoylphenyl)pyrrolidine-3-carboxamide |
| "B 108" | N-{2-[2-(2-Aminoethoxy)ethoxy]ethyl)-5-[(S)-3-(3-chloro-5-fluorobenzylcarbamoyl)-3-hydroxy-2-oxopyrrolidin-1-yl]-1H-indole-2-carboxamide |
| "B109" | N-(3-Chloro-5-fluorobenzyl)-1-(7-fluoro-2-oxo-1,2,3,4-tetrahydroquinolin-6-yl)-3-hydroxy-2-oxopyrrolidine-3-carboxamide |
| "B110" | N-[2-(3,5-Difluorophenyl)ethyl]-(S)-3-hydroxy-1-(1H-indol-5-yl)-2-oxopyrrolidine-3-carboxamide |
| "B111" | 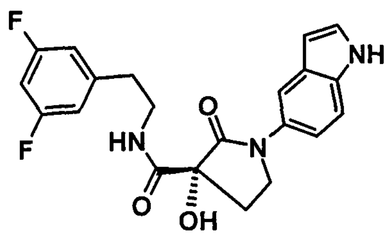 N-[2-(3,5-Difluorophenyl)ethyl]-(R)-3-hydroxy-1-(1H-indol-5-yl)-2-oxopyrrolidine-3-carboxamide |
| "B112" | N-(3-Chloro-5-fluorobenzyl)-(S)-3-hydroxy-1-[2-(morpholine-4-carbonyl)-1H-indol-5-yl]-2-oxopyrrolidine-3-carboxamide |
| "B113" | N-[2-(2-Aminoethoxy)ethyl]-5-[(S)-3-(3-chloro-5-fluorobenzylcarbamoyl)-3-hydroxy-2-oxopyrrolidin-1-yl]-1H-indole-2-carboxamide |
| "B114" | N-(3-Chloro-5-fluorobenzyl)-(S)-1-[2-(azetidine-1-carbonyl)-1H-indol-5-yl]-3-hydroxy-2-oxopyrrolidine-3-carboxamide |
| "B115" | N-(3-Chloro-5-fluorobenzyl)-3-hydroxy-2-oxo-1-(4-sulfamoylphenyl)pyrrolidine-3-carboxamide |
| "B116" | N-(3-Chloro-5-fluorobenzyl)-(S)-1-(1-cyanomethy(-1H-pyrazol-4-yl)-3-hydroxy-2-oxopyrrolidine-3-carboxamide |
| "B117" | N-(3-Chloro-5-fluorobenzyl)-(R)-1-(1-cyanomethyl-1H-pyrazol-4-yl)-3-hydroxy-2-oxopyrrolidine-3-carboxamide |
| "B118" | N-(3-Chloro-5-fluorobenzyl)-1-(4-acetylsulfamoylphenyl)-3-hydroxy-2-oxopyrrolidine-3-carboxamide |
| "B119" | N-(3-Chloro-5-fluorobenzyl)-(S)-1-(1-carbamoylmethyl-1H-pyrazol-4-yl)-3-hydroxy-2-oxopyrrolidine-3-carboxamide |
| "B120" | N-(3-Chloro-5-fluorobenzyl)-3-hydroxy-2-oxo-1-(1-phenyl-ethyl)pyrrolidine-3-carboxamide |
| "B121" | N-(2-Hydroxyethyl)-5-[(S)-3-(3-chloro-5-fluorobenzyl-carbamoyl)-3-hydroxy-2-oxopyrrolidin-1-yl]-1H-indole-2-carboxamide |

(continued)

| Compound No. | Name |
|---|---|
| "B122" | N-(3-Chloro-5-fluorobenzyl)-(S)-3-hyd roxy-1-[2-(4-methyl-piperazine-1-carbonyl)-1H-indol-5-yl]-2-oxopyrrolidine-3-carboxamide |
| "B123" | tert-Butyl 4-{5-[(S)-3-(3-chloro-5-fluorobenzylcarbamoyl)-3-hydroxy-2-oxopyrrolidin-1-yl]-1H-indole-2-carbonyl}piperazine-1-carboxylate |
| "B124" | N-(3-Chloro-5-fluorobenzyl)-1-(5-fluoro-2-oxo-1,2,3,4-tetrahydroquinolin-6-yl)-3-hydroxy-2-oxopyrrolidine-3-carboxamide |
| "B125" | N-(3-Chloro-5-fluorobenzyl)-3-hydroxy-2-oxo-1-(2-oxo-2,3-dihydro-1H-indol-5-yl)pyrrolidine-3-carboxamide |
| "B126" | N-(3-Chloro-5-fluorobenzyl)-(S)-3-hydroxy-2-oxo-1-[2-(piperazine-1-carbonyl)-1H-indol-5-yl]pyrrolidine-3-carboxamide |
| "B127" | N-(3-Chloro-5-fluorobenzyl)-3-chloro-2-oxo-1-(2-oxo-1,2,3,4-tetrahydroquinolin-6-yl)pyrrolidine-3-carboxamide |
| "B128" | N-(3-Chloro-5-fluorobenzyl)-(S)-1-(3-dimethylsulfamoyl-phenyl)-3-hydroxy-2-oxopyrrolidine-3-carboxamide |
| "B129" | N-(2-Aminophenyl)-5-[(S)-3-(3-chloro-5-fluorobenzylcarbamoyl)-3-hydroxy-2-oxopyrrolidin-1-yl]-1H-indole-2-carboxamide |
| "B130" | N-(3-Chloro-5-fluorobenzyl)-(S)-3-hydroxy-2-oxo-1-(2-oxo-2,3-dihydro-1H-indol-5-yl)pyrrolidine-3-carboxamide |
| "B131" | N-(3-Chloro-5-fluorobenzyl)-(R)-3-hydroxy-2-oxo-1-(2-oxo-2,3-dihydro-1H-indol-5-yl)pyrrolidine-3-carboxamide |
| "B132" | N-(2-Aminoethyl)-5-[(S)-3-(3-chloro-5-fluorobenzylcarbamoyl)-3-hydroxy-2-oxopyrrolidin-1-yl]-1H-indole-2-carboxamide |
| "B133" | N-(3-Chloro-5-fluorobenzyl)-(S)-1-[2-(1H-benzoimidazol-2-yl)-1H-indol-5-yl]-3-hydroxy-2-oxopyrrolidine-3-carboxamide |
| "B134" | N-(3-Chloro-5-fluorobenzyl)-(S)-1-(2-hydrazinocarbonyl-1H-indol-5-yl)-3-hydroxy-2-oxopyrrolidine-3-carboxamide |
| "B135" | N-(3-Chloro-5-fluorobenzyl)-(S)-3-hydroxy-1-[2-(3-methyl-1,2,4-oxadiazol-5-yl)-1H-indol-5-yl]-2-oxopyrrolidine-3-carboxamide |
| "B136" | N-(3-Chloro-5-fluorobenzyl)-(S)-1-benzyloxy-3-hydroxy-2-oxopyrrolidine-3-carboxamide |
| "B137" | N-(3-Chloro-5-fluorobenzyl)-3-hydroxy-2-oxopyrrolidine-3-carboxamide |
| "B138" | N-(3-Chloro-5-fluorobenzyl)-(R)-1-benzyloxy-3-hydroxy-2-oxopyrrolidine-3-carboxamide |
| "B139" | N-(3-Chloro-5-fluorobenzyl)-(S)-3-hydroxy-2-oxo-1-(3-trifluoromethyl-1H-pyrrolo[2,3-b]pyridin-5-yl)pyrrolidine-3-carboxamide |
| "B140" | N-(3-Chloro-5-fluorobenzyl)-(R)-3-hydroxy-2-oxo-1-(3-trifluoromethyl-1H-pyrrolo[2,3-b]pyridin-5-yl)pyrrolidine-3-carboxamide |
| "B141" | N-(3-Chloro-5-fluorobenzyl)-(S)-3-hydroxy-1-(4-methylsulfamoylphenyl)-2-oxopyrrolidine-3-carboxamide |
| "B142" | N-(3-Chloro-5-fluorobenzyl)-(S)-1-(4-benzylsulfamoyl-phenyl)-3-hydroxy-2-oxopyrrolidine-3-carboxamide |
| "B143" | N-(3-Chloro-5-fluorobenzyl)-(S)-1-[4-(benzylmethylsulfamoyl)phenyl]-3-hydroxy-2-oxopyrrolidine-3-carboxamide |
| "B144" | N-(3-Chloro-5-fluorobenzyl)-3-hydroxy-1-(1-methyl-1H-indol-5-yl)-2-oxopyrrolidine-3-carboxamide |
| "B145" | N-(3-Chloro-5-fluorobenzyl)-(S)-3-hydroxy-1-hydroxymethyl-2-oxopyrrolidine-3-carboxamide |

(continued)

| Compound No. | Name |
|---|---|
| "B146" | N-(3-Chloro-5-fluorobenzyl)-(R)-3-hydroxy-1-hydroxymethyl-2-oxopyrrolidine-3-carboxamide |
| "B147" | N-(3-Chloro-5-fluorobenzyl)-(S)-1-(3-chloro-1H-pyrrolo-[2,3-b]pyridin-5-yl)-3-hydroxy-2-oxopyrrolidine-3-carboxamide |
| "B148" | N-(3-Chloro-5-fluorobenzyl)-3-hydroxy-2-oxo-1-(2-trifluoromethyl-1H-benzimidazol-5-yl)pyrrolidine-3-carboxamide |
| "B149" | N-(3-Chloro-5-fluorobenzyl)-(S)-3-hydroxy-2-oxo-1-(2-sulfamoyl-1H-indol-5-yl)pyrrolidine-3-carboxamide |
| "B150" | (3S)-N-[(3-Chloro-5-fluorophenyl)methyl]-1-(2,2-dioxo-1,3-dihydro-2,1-benzothiazol-5-yl)-3-hydroxy-2-oxopyrrolidine-3-carboxamide |
| "B151" | N-(3-Chloro-5-fluorobenzyl)-(S)-1-(2,2-dioxo-1,2,3,4-tetrahydro-2lamda*6*-benzo[c]thiazin-6-yl)-3-hydroxy-2-oxo-1-(2-sulfamoyl-1H-indol-5-yl)pyrrolidine-3-carboxamide |
| "B152" | N-(3-Chloro-5-fluorobenzyl)-(S)-1-[((R)-1-dimethylcarbamoyl-2-methylpropylcarbamoyl)methyl]-3-hydroxy-2-oxo-1-(2-sulfamoyl-1H-indol-5-yl)pyrrolidine-3-carboxamide |
| "B153" | N-(3-Chloro-5-fluorobenzyl)-(S)-1-[((S)-3-amino-1-methylcarbamoylpropylcarbamoyl)methyl]-3-hydroxy-2-oxo-1-(2-sulfamoyl-1H-indol-5-yl)pyrrolidine-3-carboxamide |
| "B154" | N-(3-Hydroxy-2-oxo-1-phenylpyrrolidin-3-yl)benzamide |

and pharmaceutically usable salts, tautomers and stereoisomers thereof, including mixtures thereof in all ratios.

**6.** Process for the preparation of compounds of the formula I according to Claims 1-5 and pharmaceutically usable salts, tautomers and stereoisomers thereof, **characterised in that**

a) for the preparation of compounds of the formula I in which Y denotes CO and R denotes $NR^2R^4$, a compound of the formula II

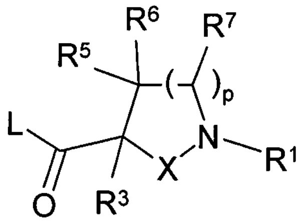

II

in which X, $R^1$, $R^3$, $R^5$, $R^6$, $R^7$ and p have the meanings indicated in Claim 1, and L denotes Cl, Br, I or a free or reactively functionally modified OH group, is reacted with a compound of the formula III

$R^2$-$NHR^4$ III

in which $R^2$ and $R^4$ have the meanings indicated in Claim 1,
or
b) a compound of the formula IV

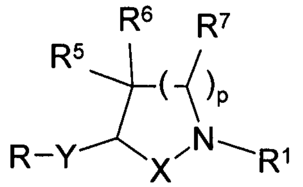

IV

in which $R^1$, $R^5$, $R^6$, $R^7$, R, X, Y and p have the meanings indicated in Claim 1, is oxidised,

or

c) for the preparation of compounds of the formula I in which X and Y denote $CH_2$, a compound of the formula I in which X and Y denote CO is reduced,

and/or

a base or acid of the formula I is converted into one of its salts.

**7.** Medicaments comprising at least one compound of the formula I according to Claims 1-5 and/or pharmaceutically usable salts, tautomers and stereoisomers thereof, including mixtures thereof in all ratios, and optionally excipients and/or adjuvants.

**8.** Compounds of the formula I according to Claims 1-5,
and pharmaceutically usable salts, tautomers and stereoisomers thereof, including mixtures thereof in all ratios, for use for the treatment of tumours, tumour metastases, proliferative diseases of the mesangial cells, haemangioma, proliferative retinopathy, rheumatoid arthritis, atherosclerotic neovascularisation, psoriasis, ocular neovascularisation, osteoporosis, diabetes and obesity, lymphoid leukaemia, lymphoma, malaria and prostate hypertrophy.

**9.** Compounds for use for the treatment according to Claim 8, where the tumour disease is selected from the group of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

**10.** Compounds of the formula I according to Claims 1-5 and/or physiologically acceptable salts thereof for use for the treatment of tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

**11.** Compounds of the formula I according to Claims 1-5 and/or physiologically acceptable salts thereof for use for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

**1.** Composés de formule I

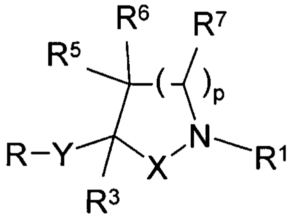

I

dans lesquels

R désigne $NR^2R^4$, Alk, $C(=CH_2)[C(R^4)_2]_nAr^2$, $Hét^2$, $O[C(R^4)_2]_nAr^2$ ou OA,
X désigne CO ou $CH_2$,
Y désigne CO ou $CH_2$,
$R^1$ désigne H, $[C(R^4)_2]nAr^1$, $(CH_2)_nHét$, $(CH_2)_nCyc$, $[C(R^4)_2]nCOOH$, $[C(R^4)_2]_nCONHAr^1$, $[C(R^4)_2]_nCONH_2$, $[C(R^4)_2]_nNHA$, $[C(R^4)_2]_nNA_2$, $O[C(R^4)_2]_nAr^1$, $[C(R^4)^2]_nOR^7$, $[C(R^4)_2]_nCOO(CH_2)_nAr^1$, $[C(R^4)_2]_nCOOA$, $[C(R^4)_2]_nCONH[C(R^4)_2]_pCON(R^4)_2$ ou $[C(R^4)_2]_nCONHCR^4[(CH_2)_nN(R^4)_2]CON(R^4)_2$,
$R^2$ désigne H, $[C(R^4)_2]_nAr^2$, $(CH_2)_nCOHét^1$, $(CH_2)_nCOAr^2$, $(CH_2)_mNA_2$ ou $(CH_2)_nHét$,
$R^3$ désigne OH ou OCOA,
$R^4$ désigne H ou alkyle ayant 1, 2, 3 ou 4 atomes de C,
$R^2$ et $R^4$ désignent également ensemble alkylène ayant 2, 3, 4 ou 5 atomes de C, où un groupement $CH_2$ peut également être remplacé par $N(CH_2)_mOH$ ou $SO_2$,
$R^5$, $R^6$ désignent chacun, indépendamment l'un de l'autre, H, F ou A,
$R^5$ et $R^6$ désignent également ensemble alkylène ayant 2, 3, 4 ou 5 atomes de C, où un groupement $CH_2$ peut également être remplacé par NCOA ou O,
$R^7$ désigne H ou A,
$Ar^1$ désigne phényle qui est non substitué ou mono-, di-, tri-, tétra- ou pentasubstitué par Hal, OH, OA, $CONH_2$, CONHA, $CONA_2$, $NHSO_2A$, CONHCyc, $NHSO_2Cyc$, $CONHAr^2$, $COHét^1$ et/ou $NASO_2A$,
$Ar^2$ désigne phényle qui est non substitué ou mono-, di-, tri-, tétra- ou pentasubstitué par Hal, A, $CONH_2$ et/ou $OAr^3$,
$Ar^3$ désigne phényle qui est non substitué ou monosubstitué par $NH_2$,
Hét désigne un hétérocycle mono- ou bicyclique saturé, insaturé ou aromatique ayant de 1 à 4 atomes de N et/ou O et/ou S qui est non substitué ou mono-, di- ou trisubstitué par Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Hét^1$, $SO_2A$, $SO_2NH_2$, $SO_2NHA$, $SO_2NA_2$, $CONHNH_2$, $CONHAr^3$, =O et/ou $Ar^3$,
$Hét^1$ désigne un hétérocycle monocyclique saturé ayant de 1 à 4 atomes de N et/ou O et/ou S qui est non substitué ou mono-, di- ou trisubstitué par =O et/ou COOA,
$Hét^2$ désigne isoindolyle,
A désigne alkyle non ramifié ou ramifié ayant 1-10 atomes de C, où 1-7 atomes de H peuvent être remplacés par F, Cl, Br, OH, CHO, COA, COOA, CN, $CONA_2$, CONHA et/ou $CONH_2$,
et/ou où un ou deux groupements CH et/ou $CH_2$ non adjacents peuvent être remplacés par O, ou Cyc,
Alk désigne alcényle ayant 2, 3, 4, 5 ou 6 atomes de C,
Cyc désigne alkyle cyclique ayant 3-7 atomes de C qui est non substitué ou mono-, di- ou trisubstitué par NHCOA, $NHSO_2$, OH, OA, A, $NH_2$, NHA, $NA_2$, COOA, COOH et/ou CONHA,
Hal désigne F, Cl, Br ou I,
m désigne 1, 2, 3 ou 4,
n désigne 0, 1, 2, 3 ou 4,
p désigne 1, 2 ou 3,

ainsi que les sels, tautomères et stéréoisomères pharmaceutiquement utilisables de ceux-ci, y compris des mélanges de ceux-ci en toutes proportions, où les composés suivants sont exclus:

le 3-hydroxypipéridine-3-carboxylate de méthyle,
l'ester 1-tertio-butylique de l'acide 3-hydroxypipéridine-1-carboxylique ester méthylique de l'acide 3-carboxylique,
le 3-allyl-3-hydroxypyrrolidine-1-carboxylate de tertio-butyle,
le 3-allyl-3-hydroxypipéridine-1-carboxylate de tertio-butyle.

**2.** Composés selon la revendication 1, dans lesquels

Hét désigne pyrazinyle, pyrazolyle, benzimidazolyle, pyridyle, indolyle, dihydroindolyle, benzofuranyle, tétrahydropyranyle, dihydroquinoléinyle, dihydroisoquinoléinyle, tétrahydroquinoléinyle, tétrahydroisoquinoléinyle, indazolyle, imidazolyle, pyrrolyle, oxazolyle, oxadiazolyle, isoxazolyle, benzothiazolyle, pipéridin-1-yle, pyrrolidin-1-yle, 3,4-dihydro-2H-pyrido[3,2-b]-1,4-oxazinyle, 3,4-dihydro-2H-benzo-1,4-oxazinyle, benzofuranyle, azétidinyle, 3-aza-bicyclo[3.2.0]hexyle, pyrrolo[2,3-b]pyridinyle, tétrahydrofuranyle, tétrahydro-1,8-naphtyridinyle, 2,3-dihydrobenzo-isothiazolyle, 1,2,3,4-tétrahydrobenzothiazinyle ou hexahydrobenzo-1,3-dioxolyle, chacun d'entre eux étant non substitué ou mono-, di- ou trisubstitué par Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Hét^1$, $SO_2A$, $SO_2NH_2$, $SO_2NHA$, $SO_2NA_2$, $CONHNH_2$, $CONHAr^3$, =O et/ou $Ar^3$,

ainsi que les sels, tautomères et stéréoisomères pharmaceutiquement utilisables de ceux-ci, y compris des mélanges de ceux-ci en toutes proportions.

**3.** Composés selon la revendication 1 ou 2, dans lesquels

$Hét^1$ désigne pyridazinyle, pyrazolyle, pyridyle, pipérazinyle, morpholinyle, pyrimidinyle, furyle, thiényle, imidazolyle, pyrrolyle, oxazolyle, oxadiazolyle, isoxazolyle, thiazolyle, triazolyle, tétrazolyle, thiadiazole, pipéridin-1-yle, pyrrolidin-1-yle, tétrahydropyranyle, 1,2-oxazinan-2-yle, 1,2,5-oxa-diazinan-2-yle, 1,3-oxazinan-3-yle ou hexahydropyrimidinyle, chacun d'entre eux étant non substitué ou mono-, di- ou trisubstitué par A et/ou OA,

ainsi que les sels, tautomères et stéréoisomères pharmaceutiquement utilisables de ceux-ci, y compris des mélanges de ceux-ci en toutes proportions.

**4.** Composés selon la revendication 1, dans lesquels

R désigne $NR^2R^4$, Alk, $C(=CH_2)[C(R^4)_2]_nAr^2$, $Hét^2$, $O[C(R^4)_2]_nAr^2$ ou OA,
X désigne CO ou $CH_2$,
Y désigne CO ou $CH_2$,
$R^1$ désigne H, $[C(R^4)_2]_nAr^1$, $(CH_2)_nHét$, $(CH_2)_nCyc$, $[C(R^4)_2]_nCOOH$, $[C(R^4)_2]_nCONHAr^1$, $[C(R^4)_2]_nCONH_2$, $[C(R^4)_2]_nNHA$, $[C(R^4)_2]_nNA_2$, $O[C(R^4)_2]_nAr^1$, $[C(R^4)_2]_nOR^7$, $[C(R^4)_2]_nCOO(CH_2)_nAr^1$, $[C(R^4)_2]_nCOOA$, $[C(R^4)_2]_nCONH[C(R^4)_2]_pCON(R^4)_2$ ou $[C(R^4)_2]_nCONHCR^4[(CH_2)_nN(R^4)_2]CON(R^4)_2$,
$R^2$ désigne H, $(C(R^4)_2]_nAr^2$, $(CH_2)_nCOHét^1$, $(CH_2)_nCOAr^2$, $(CH_2)_mNA_2$ ou $(CH_2)_nHét$,
$R^3$ désigne OH ou OCOA,
$R^4$ désigne H ou alkyle ayant 1, 2, 3 ou 4 atomes de C,
$R^2$ et $R^4$ désignent également ensemble alkylène ayant 2, 3, 4 ou 5 atomes de C, où un groupement $CH_2$ peut également être remplacé par $N(CH_2)_mOH$ ou $SO_2$,
$R^5$, $R^6$ désignent chacun, indépendamment l'un de l'autre, H, F ou A,
$R^5$ et $R^6$ désignent également ensemble alkylène ayant 2, 3, 4 ou 5 atomes de C, où un groupement $CH_2$ peut également être remplacé par NCOA ou O,
$R^7$ désigne H ou A,
$Ar^1$ désigne phényle qui est non substitué ou mono-, di-, tri-, tétra- ou pentasubstitué par Hal, OH, OA, $CONH_2$, CONHA, $CONA_2$, $NHSO_2A$, CONHCyc, $NHSO_2Cyc$, $CONHAr^2$, $COHét^1$ et/ou $NASO_2A$,
$Ar^2$ désigne phényle qui est non substitué ou mono-, di-, tri-, tétra- ou pentasubstitué par Hal, A, $CONH_2$ et/ou $OAr^3$,
$Ar^3$ désigne phényle qui est non substitué ou monosubstitué par $NH_2$,
Hét désigne pyrazinyle, pyrazolyle, benzimidazolyle, pyridyle, indolyle, dihydroindolyle, benzofuranyle, tétrahydropyranyle, dihydroquinoléinyle, dihydroisoquinoléinyle, tétrahydroquinoléinyle, tétrahydroisoquinoléinyle, indazolyle, imidazolyle, pyrrolyle, oxazolyle, oxadiazolyle, isoxazolyle, benzothiazolyle, pipéridin-1-yle, pyrrolidin-1-yle, 3,4-dihydro-2H-pyrido[3,2-b]-1,4-oxazinyle, 3,4-dihydro-2H-benzo-1,4-oxazinyle, benzofuranyle, azétidinyle, 3-aza-bicyclo[3.2.0]hexyle, pyrrolo[2,3-b]pyridinyle, tétrahydrofuranyle, tétrahydro-1,8-naphtyridinyle, 2,3-dihydrobenzo-isothiazolyle, 1,2,3,4-tétrahydrobenzothiazinyle ou hexahydrobenzo-1,3-dioxolyle, chacun d'entre eux étant non substitué ou mono-, di- ou trisubstitué par Hal, A, OA, CN, $NH_2$, NHA, $NA_2$, $NO_2$, CN, COOH, COOA, $(CH_2)_nCONH_2$, $(CH_2)_nCONHA$, $(CH_2)_nCONA_2$, NHCOA, COA, CHO, $Hét^1$, $SO_2A$, $SO_2NH_2$, $SO_2NHA$, $SO_2NA_2$, $CONHNH_2$, $CONHAr^3$, =O et/ou $Ar^3$,
$Hét^1$ désigne pyridazinyle, pyrazolyle, pyridyle, pipérazinyle, morpholinyle, pyrimidinyle, furyle, thiényle, imidazolyle, pyrrolyle, oxazolyle, oxadiazolyle, isoxazolyle, thiazolyle, triazolyle, tétrazolyle, thiadiazole, pipéridin-1-yle, pyrrolidin1-yle, tétrahydropyranyle, 1,2-oxazinan-2-yle, 1,2,5-oxa-diazinan-2-yle, 1,3-oxazinan-3-yle ou hexahydropyrimidinyle, chacun d'entre eux étant non substitué ou mono-, di- ou trisubstitué par A et/ou OA,
Hét$^2$ désigne isoindolyle,
A désigne alkyle non ramifié ou ramifié ayant 1-10 atomes de C, où 1-7 atomes de H peuvent être remplacés par F, Cl, Br, OH, CHO, COA, COOA, CN, $CONA_2$, CONHA et/ou $CONH_2$,
et/ou où un ou deux groupements CH et/ou $CH_2$ non adjacents peuvent être remplacés par O, ou Cyc,
Alk désigne alcényle ayant 2, 3, 4, 5 ou 6 atomes de C,
Cyc désigne alkyle cyclique ayant 3-7 atomes de C qui est non substitué ou mono-, di- ou trisubstitué par NHCOA, $NHSO_2$, OH, OA, A, $NH_2$, NHA, $NA_2$, COOA, COOH et/ou CONHA,
Hal désigne F, Cl, Br ou I,
m désigne 1, 2, 3 ou 4,
n désigne 0, 1, 2, 3 ou 4,
p désigne 1, 2 ou 3,

ainsi que les sels, tautomères et stéréoisomères pharmaceutiquement utilisables de ceux-ci, y compris des mélanges de ceux-ci en toutes proportions.

**5.** Composés selon la revendication 1, choisis dans le groupe constitué par

| Composé n° | Nom |
|---|---|
| « A56 » | (S)-3-(1,1-Dioxo-1,6-thiomorpholine-4-carbonyl)-3-hydroxy-1-phénylpyrrolidin-2-one |
| « A57 » | (S)-3-Hydroxy-3-[4-(2-hydroxyéthyl)pipérazine-1-carbonyl]-1-phénylpyrrolidin-2-one |
| « A58 » | N-(3-Imidazol-1-ylpropyl)-(S)-3-hydroxy-2-oxo-1-phénylpyrrolidine-3-carboxamide |
| « A59 » | N-(2-Diméthylaminoéthyl)-(S)-3-hydroxy-2-oxo-1-phénylpyrrolidine-3-carboxamide |
| « A60 » | N-[2-(4-Phénoxyphényl)éthyl]-(S)-3-hydroxy-2-oxo-1-phénylpyrrolidine-3-carboxamide |
| « A61 » | N-[(2-Diméthylaminoéthyl)méthyl]-(S)-3-hydroxy-2-oxo-1-phénylpyrrolidine-3-carboxamide |
| « A62 » | N-(2-Morpholin-4-yl-2-oxoéthyl)-(S)-3-hydroxy-2-oxo-1-phénylpyrrolidine-3-carboxamide |
| « A63 » | N-[2-(1-Méthylpyrrolidin-2-yl)éthyl]-(S)-3-hydroxy-2-oxo-1-phénylpyrrolidine-3-carboxamide |
| « A64 » | N-(3-Diméthylaminopropyl)-(S)-3-hydroxy-2-oxo-1-phénylpyrrolidine-3-carboxamide |
| « A65 » | N-(3-Chloro-5-fluorobenzyl)-3-hydroxy-2-oxo-1-phénylpipéridine-3-carboxamide |
| « A66 » | N-(3-Chloro-5-fluorobenzyl)-(S)-3-hydroxy-2-oxo-1-phénylazépane-3-carboxamide |
| « A67 » | N-(3-Chloro-5-fluorobenzyl)-(R)-3-hydroxy-2-oxo-1-phénylazépane-3-carboxamide |
| « A68 » | N-(3-Chloro-5-fluorobenzyl)-3-hydroxy-1-(1H-indol-6-yl)-2-oxopyrrolidine-3-carboxamide |
| « A69 » | N-(3-Chloro-5-fluorobenzyl)-1-[3-(3-carbamoyl-phénylcarbamoyl)phényl]-3-hydroxy-2-oxopyrrolidine-3-carboxamide |
| « A70 » | (S)-3-((E)-But-2-énoyl)-3-hydroxy-1-phénylpyrrolidin-2-one |
| «A71 » | N-(3-Chloro-5-fluorobenzyl)-(S)-8-acétyl-4-hydroxy-3-oxo-2-phényl-2,8-diazaspiro[4.5]décane-4-carboxamide |
| « A72 » | N-(3-Chloro-5-fluorobenzyl)-(S)-3-hydroxy-1-[3-(morpholine-4-carbonyl)phényl]-2-oxopyrrolidine-3-carboxamide |
| « A73 » | N-(3-Chloro-5-fluorobenzyl)-3-hydroxy-1-[3-(méthanesulfonylméthylamino)phényl]-2-oxopyrrolidine-3-carboxamide |
| « A74 » | N-(3-Chloro-5-fluorobenzyl)-(R)-3-hydroxy-1-[3-(morpholine-4-carbonyl)phényl]-2-oxopyrrolidine-3-carboxamide |
| « A75 » | N-(3-Chloro-5-fluorobenzyl)-(S)-1-(3-cyclopropyl-carbamoylphényl)-3-hydroxy-2-oxopyrrolidine-3-carboxamide |
| « A76 » | N-(3-Chloro-5-fluorobenzyl)-1-(3-cyclobutyl-carbamoylphényl)-3-hydroxy-2-oxopyrrolidine-3-carboxamide |

(suite)

| Composé n° | Nom |
|---|---|
| « A77 » | N-(3-Chloro-5-fluorobenzyl)-(S)-3-hydroxy-2-oxo-1-[3-(pyrrolidine-1-carbonyl)phényl]pyrrolidine-3-carboxamide |
| « A78 » | N-(3-Chloro-5-fluorobenzyl)-3-hydroxy-2-oxo-1-(6-propionylaminopyridin-3-yl)pyrrolidine-3-carboxamide |
| « A79 » | N-(3-Chloro-5-fluorobenzyl)-(S)-1-(3-éthanesulfonyl-aminophényl)-3-hydroxy-2-oxopyrrolidine-3-carboxamide |
| « A80 » | N-(3-Chloro-5-fluorobenzyl)-(S)-1-(3-cyclopropane-sulfonylaminophényl)-3-hydroxy-2-oxopyrrolidine-3-carboxamide |
| « A81 » | N-(3-Chloro-5-fluorobenzyl)-(S)-1-benzyl-3-hydroxy-2-oxopipéridine-3-carboxamide |
| « A82 » | N-(3-Chloro-5-fluorobenzyl)-(R)-1-(3-cyclopropyl-carbamoylphényl)-3-hydroxy-2-oxopyrrolidine-3-carboxamide |
| « A83 » | N-(3-Chloro-5-fluorobenzyl)-(R)-3-hydroxy-2-oxo-1-[3-(pyrrolidine-1-carbonyl)phényl]pyrrolidine-3-carboxamide |
| « A84 » | N-(3-Chloro-5-fluorobenzyl)-(R)-1-(3-éthanesulfonyl-aminophényl)-3-hydroxy-2-oxopyrrolidine-3-carboxamide |
| « A85 » | N-(3-Chloro-5-fluorobenzyl)-(R)-1-(3-cyclopropane-sulfonylaminophényl)-3-hydroxy-2-oxopyrrolidine-3-carboxamide |
| « A86 » | N-(3-Chloro-5-fluorobenzyl)-(R)-1-benzyl-3-hydroxy-2-oxopipéridine-3-carboxamide |
| « A87 » | N-(3-Chloro-5-fluorobenzyl)-1-(3-cyano-1H-indol-5-yl)-3-hydroxy-2-oxopyrrolidine-3-carboxamide |
| « A88 » | N-(3-Chloro-5-fluorobenzyl)-(S)-3-hydroxy-1-[3-(2-hydroxyéthylcarbamoyl)phényl]-2-oxopyrrolidine-3-carboxamide |
| « A89 » | 3-(2-Benzylacryloyl)-3-hydroxy-1-phénylpyrrolidin-2-one |
| « A90 » | N-(3-Chloro-5-fluorobenzyl)-(S)-3-hydroxy-2-oxo-1-phénéthylpyrrolidine-3-carboxamide |
| « A91 » | N-(3-Chloro-5-fluorobenzyl)-(R)-3-hydroxy-1-[3-(2-hydroxyéthylcarbamoyl)phényl]-2-oxopyrrolidine-3-carboxamide |
| « A92 » | N-(3-Chloro-5-fluorobenzyl)-(R)-3-hydroxy-2-oxo-1-phénéthylpyrrolidine-3-carboxamide |
| « A93 » | N-(3-Chloro-5-fluorobenzyl)-(S)-3-hydroxy-1-{3-[(2-hydroxyéthyl)méthylcarbamoyl]phényl}-2-oxopyrrolidine-3-carboxamide |
| « A94 » | N-(3-Chloro-5-fluorobenzyl)-(S)-3-hydroxy-1-[3-(3-hydroxypropylcarbamoyl)phényl]-2-oxopyrrolidine-3-carboxamide |
| « A95 » | N-(3-Chloro-5-fluorobenzyl)-(S)-3-hydroxy-2-oxo-1-[3-(pipéridine-1-carbonyl)phényl]pyrrolidine-3-carboxamide |
| « A96 » | N-(3-Chloro-5-fluorobenzyl)-(S)-3-hydroxy-2-oxo-1-(1-oxo-1,2,3,4-tétrahydroisoquinoléin-6-yl)pyrrolidine-3-carboxamide |
| « A97 » | N-(3-Chloro-5-fluorobenzyl)-(S)-1-(6-cyanopyridin-3-yl)-3-hydroxy-2-oxopyrrolidine-3-carboxamide |
| « A98 » | N-(3-Chloro-5-fluorobenzyl)-(R)-1-(5-cyanopyridin-3-yl)-3-hydroxy-2-oxopyrrolidine-3-carboxamide |
| « A99 » | N-(3-Chloro-5-fluorobenzyl)-(S)-3-hydroxy-2-oxo-1-(6-trifluorométhylpyridin-3-yl)pyrrolidine-3-carboxamide |
| « A100 » | 5-[3-(3-Chloro-5-fluorobenzylcarbamoyl)-3-hydroxy-2-oxopyrrolidin-1-yl]-2-méthylnicotinate de méthyle |

(suite)

| Composé n° | Nom |
|---|---|
| « A101 » | N-(3-Chloro-5-fluorobenzyl)-(S)-3-hydroxy-4,4-diméthyl-2-oxo-1-phénylpyrrolidine-3-carboxamide |
| « A102 » | N-(3-Chloro-5-fluorobenzyl)-(R)-3-hydroxy-1-{3-[(2-hydroxyéthyl)méthylcarbamoyl]phényl}-2-oxopyrrolidine-3-carboxamide |
| « A103 » | N-(3-Chloro-5-fluorobenzyl)-(R)-3-hydroxy-1-[3-(3-hydroxypropylcarbamoyl)phényl]-2-oxopyrrolidine-3-carboxamide |
| « A104 » | N-(3-Ch loro-5-fluorobenzyl)-(R)-3-hydroxy-2-oxo-1-[3-(pipéridine-1-carbonyl)phényl]pyrrolidine-3-carboxamide |
| « A105 » | N-(3-Chloro-5-fluorobenzyl)-(R)-3-hydroxy-2-oxo-1-(1-oxo-1,2,3,4-tétrahydroisoquinoléin-6-yl)pyrrolidine-3-carboxamide |
| « A106 » | N-(3-Chloro-5-fluorobenzyl)-(R)-1-(6-cyanopyridin-3-yl)-3-hydroxy-2-oxopyrrolidine-3-carboxamide |
| « A107 » | N-(3-Chloro-5-fluorobenzyl)-(S)-1-(5-cyanopyridin-3-yl)-3-hydroxy-2-oxopyrrolidine-3-carboxamide |
| « A108 » | N-(3-Chloro-5-fluorobenzyl)-(R)-3-hydroxy-2-oxo-1-(6-trifluorométhylpyridin-3-yl)pyrrolidine-3-carboxamide |
| « A109 » | N-(3-Chloro-5-fluorobenzyl)-(R)-3-hydroxy-4,4-diméthyl-2-oxo-1-phénylpyrrolidine-3-carboxamide |
| « A110 » | (S)-3-(2-Benzylacryloyl)-3-hydroxy-1-phénylpyrrolidin-2-one |
| « A111 » | (R)-3-(2-Benzylacryloyl)-3-hydroxy-1-phénylpyrrolidin-2-one |
| « A112 » | N-(3-Chloro-5-fluorobenzyl)-3-hydroxy-1-(1-hydroxyméthyl-3-trifluorométhyl-1H-indol-5-yl)-2-oxopyrrolidine-3-carboxamide |
| « A113 » | N-(3-Chloro-5-fluorobenzyl)-8-hydroxy-7-oxo-6-phényl-2-oxa-6-azaspiro[3.4]octane-8-carboxamide |
| « A114 » | N-(3-Chloro-5-fluorobenzyl)-1-(8-fluoro-2-oxo-1,2,3,4-tétrahydroquinoléin-6-yl)-3-hydroxy-2-oxopyrrolidine-3-carboxamide |
| « A115 » | N-(3-Chloro-5-fluorobenzyl)-3-hydroxy-2-oxo-1-(1-oxo-1,2,3,4-tétrahydroisoquinoléin-7-yl)pyrrolidine-3-carboxamide |
| « A116 » | N-(3-Chloro-5-fluorobenzyl)-3-hydroxy-1-(6-méthylaminopyridin-3-yl)-2-oxopyrrolidine-3-carboxamide |
| « A117 » | N-(3-Chloro-5-fluorobenzyl)-3-hydroxy-1-(5-méthylpyridin-3-yl)-2-oxopyrrolidine-3-carboxamide |
| « A118 » | N-(3-Chloro-5-fluorobenzyl)-1-(3,4-dihydro-2H-pyrido[3,2-b]-1,4-oxazin-7-yl)-3-hydroxy-2-oxopyrrolidine-3-carboxamide |
| « A119 » | N-(3-Chloro-5-fluorobenzyl)-3-hydroxy-5-méthyl-2-oxo-1-phénylpyrrolidine-3-carboxamide |
| « A120 » | 3-[(3-Chloro-5-fluorobenzylamino)méthyl]-1-phénylpyrrolidin-3-ol |
| « A121 » | N-(3-Chloro-5-fluorobenzyl)-1-benzyl-3-hydroxypyrrolidine-3-carboxamide |
| « A122 » | N-(3-Chloro-5-fluorobenzyl)-1-(4-acétylaminocyclohexyl)-3-hydroxy-2-oxopyrrolidine-3-carboxamide |
| « A123 » | N-(3-Chloro-5-fluorobenzyl)-3-hydroxy-1-(4-méthane-sulfonylaminocyclohexyl)-2-oxopyrrolidine-3-carboxamide |
| « A124 » | N-(3-Chloro-5-fluorobenzyl)-1-(4-éthanesulfonylamino-cyclohexyl)-3-hydroxy-2-oxopyrrolidine-3-carboxamide |

(suite)

| Composé n° | Nom |
|---|---|
| « A125 » | N-(3-Chloro-5-fluorobenzyl)-1-((1S,2R,3S)-2,3-dihydroxycyclohexyl)-3-hydroxy-2-oxopyrrolidine-3-carboxamide |
| « A126 » | N-(3-Chloro-5-fluorobenzyl)-1-(3-acétylaminocyclohexyl)-3-hydroxy-2-oxopyrrolidine-3-carboxamide |
| « A127 » | N-(3-Chloro-5-fluorobenzyl)-1-(4-éthylaminocyclohexyl)-3-hydroxy-2-oxopyrrolidine-3-carboxamide |
| « A128 » | N-(3-Chloro-5-fluorobenzyl)-1-(3-éthanesulfonyl-aminocyclohexyl)-3-hydroxy-2-oxopyrrolidine-3-carboxamide |
| « A129 » | N-(3-Chloro-5-fluorobenzyl)-1-((3aR,4S,7aS)-2,2-diméthylhexahydrobenzo-1,3-dioxol-4-yl)-3-hydroxy-2-oxopyrrolidine-3-carboxamide |
| « A130 » | 5-[3-(3-Chloro-5-fluorobenzylcarbamoyl)-3-hydroxy-2-oxopyrrolidin-1-yl]isophtalamide |
| « A131 » | N-(3-Chloro-5-fluorobenzyl)-(R)-3-hydroxy-2-oxo-[1,3']bipyrrolidinyl-3-carboxamide |
| « A132 » | N-(3-Chloro-5-fluorobenzyl)-(R)-3-hydroxy-2-oxo-1-(2-oxo-1,2,3,4-tétrahydroquinoléin-6-yl)pyrrolidine-3-carboxamide |
| « A133 » | N-(3-Chloro-5-fluorobenzyl)-3-hydroxy-1-(3-méthyl-carbamoylphényl)-2-oxopyrrolidine-3-carboxamide |
| « A134 » | N-(3-Chloro-5-fluorobenzyl)-1-(3-diméthylcarbamoyl-phényl)-3-hydroxy-2-oxopyrrolidine-3-carboxamide |
| « A135 » | N-(3-Chloro-5-fluorobenzyl)-3-hydroxy-1-(4-méthoxybenzyl)-2-oxopyrrolidine-3-carboxamide |
| « A136 » | N-(3-Chloro-5-fluorobenzyl)-1-benzyl-3-hydroxy-2-oxopyrrolidine-3-carboxamide |
| « A137 » | N-[2-(3-Chloro-5-fluorophényl)éthyl]-3-hydroxy-2-oxo-1-(2-oxo-1,2,3,4-tétrahydroquinoléin-6-yl)pyrrolidine-3-carboxamide |
| « A138 » | N-(3-Chloro-5-fluorobenzyl)-3-hydroxy-1-(6-méthoxy-pyridin-3-yl)-2-oxopyrrolidine-3-carboxamide |
| « A139 » | N-[2-(3-Chloro-5-fluorophényl)éthyl]-1-(6-acétylamino-pyridin-3-yl)-3-hydroxy-2-oxopyrrolidine-3-carboxamide |
| « A140 » | N-(3-Chloro-5-fluorobenzyl)-(S)-1-benzyl-3-hydroxy-2-oxopyrrolidine-3-carboxamide |
| « A141 » | N-(3-Chloro-5-fluorobenzyl)-(R)-1-benzyl-3-hydroxy-2-oxopyrrolidine-3-carboxamide |
| « A142 » | N-(3-Chloro-5-fluorobenzyl)-3-hydroxy-1-[5-méthyl-6-(3-oxomorpholin-4-yl)pyridin-3-yl]-2-oxopyrrolidine-3-carboxamide |
| « A143 » | N-[2-(2-Trifluorométhylphényl)éthyl]-1-(6-acétylaminopyridin-3-yl)-3-hydroxy-2-oxopyrrolidine-3-carboxamide |
| « A144 » | N-[2-(2-Trifluorométhylphényl)éthyl]-1-(3-carbamoyl-phényl)-3-hydroxy-2-oxopyrrolidine-3-carboxamide |
| « A145 » | N-[1-(3-Fluorophényl)éthyl]-3-hydroxy-2-oxo-1-phénylpyrrolidine-3-carboxamide |
| « A146 » | N-(3-Chloro-5-fluorobenzyl)-3-hydroxy-1-(2-méthyl-carbamoylbenzofuran-5-yl)-2-oxopyrrolidine-3-carboxamide |
| « A147 » | N-(3-Chloro-5-fluorobenzyl)-3-hydroxy-2-oxo-1-(2-oxo-1,2-dihydroquinoléin-6-yl)pyrrolidine-3-carboxamide |
| « A148 » | 4-[3-(3-Chloro-5-fluorobenzylcarbamoyl)-3-hydroxy-2-oxopyrrolidin-1-yl]cyclohexanecarboxylate d'éthyle |
| « A149 » | N-[2-(3-Chloro-5-fluorophényl)éthyl]-(S)-3-hydroxy-2-oxo-1-(2-oxo-1,2,3,4-tétrahydroquinoléin-6-yl)pyrrolidine-3-carboxamide |

(suite)

| Composé n° | Nom |
|---|---|
| « A150 » | N-[2-(3-Chloro-5-fluorophényl)éthyl]-(R)-3-hydroxy-2-oxo-1-(2-oxo-1,2,3,4-tétrahydroquinoléin-6-yl)pyrrolidine-3-carboxamide |
| « A151 » | N-(3-Chloro-5-fluorobenzyl)-3-hydroxy-2-oxo-1-(3-oxo-3,4-dihydro-2H-benzo-1,4-oxazin-7-yl)pyrrolidine-3-carboxamide |
| « A152 » | Acide 4-[3-(3-chloro-5-fluorobenzylcarbamoyl)-3-hydroxy-2-oxopyrrolidin-1-yl]cyclohexanecarboxylique |
| « A153 » | N-(3-Chloro-5-fluorobenzyl)-(S)-3-hydroxy-1-(1H-indol-6-yl)-2-oxopyrrolidine-3-carboxamide |
| « A154 » | N-(3-Chloro-5-fluorobenzyl)-(R)-3-hydroxy-1-(1H-indol-6-yl)-2-oxopyrrolidine-3-carboxamide |
| « A155 » | N-[2-(3-Chloro-5-fluorophényl)éthyl]-(S)-1-(6-acétylamino-pyridin-3-yl)-3-hydroxy-2-oxopyrrolidine-3-carboxamide |
| « A156 » | N-[2-(3-Chloro-5-fluorophényl)éthyl]-(R)-1-(6-acétylamino-pyridin-3-yl)-3-hydroxy-2-oxopyrrolidine-3-carboxamide |
| « A157 » | N-(3-Chloro-5-fluorobenzyl)-3-hydroxy-1-(4-méthyl-carbamoylcyclohexyl)-2-oxopyrrolidine-3-carboxamide |
| « A158 » | N-(2-Oxo-1,2,3,4-tétrahydroquinoléin-6-yl)-2-oxo-1-(2-oxo-1,2,3,4-tétrahydroquinoléin-6-yl)pyrrolidine-3-carboxamide |
| « A159 » | N-[2-(3-Chloro-5-fluorophényl)éthyl]-(R)-1-(3-carbamoyl-phényl)-3-hydroxy-2-oxopyrrolidine-3-carboxamide |
| « A160 » | N-(3-Chloro-5-fluorobenzyl)-(R)-3-hydroxy-1-(3-méthanesulfonylaminophényl)-2-oxopyrrolidine-3-carboxamide |
| « A161 » | N-(3-Chloro-5-fluorobenzyl)-(S)-3-hydroxy-1-(3-méthyl-carbamoylphényl)-2-oxopyrrolidine-3-carboxamide |
| « A162 » | N-(3-Chloro-5-fluorobenzyl)-(R)-3-hydroxy-1-(3-méthyl-carbamoylphényl)-2-oxopyrrolidine-3-carboxamide |
| « A163 » | N-(3-Chloro-5-fluorobenzyl)-(S)-1-(3-cyclobutylcarbamoylphényl)-3-hydroxy-2-oxopyrrolidine-3-carboxamide |
| « A164 » | N-(3-Chloro-5-fluorobenzyl)-(R)-1-(3-cyclobutylcarbamoylphényl)-3-hydroxy-2-oxopyrrolidine-3-carboxamide |
| « A165 » | N-(3-Chloro-5-fluorobenzyl)-(S)-3-hydroxy-1-[3-(méthanesulfonylméthylamino)phényl]-2-oxopyrrolidine-3-carboxamide |
| « A166 » | N-(3-Chloro-5-fluorobenzyl)-(R)-3-hydroxy-1-[3-(méthanesulfonylméthylamino)phényl]-2-oxopyrrolidine-3-carboxamide |
| « A167 » | N-(3-Chloro-5-fluorobenzyl)-3-hydroxy-2-oxo-1-[6-(3-oxomorpholin-4-yl)pyridin-3-yl]pyrrolidine-3-carboxamide |
| « A168 » | N-[2-(3-Chloro-5-fluorophényl)éthyl]-3-hydroxy-1-(1H-indazol-5-yl)-2-oxopyrrolidine-3-carboxamide |
| « A169 » | N-(3-Chloro-5-fluorobenzyl)-3-hydroxy-2-oxo-1-(1-phényl-1H-pyrazol-4-yl)pyrrolidine-3-carboxamide |
| « A170 » | 4-{4-[3-(3-Chloro-5-fluorobenzylcarbamoyl)-3-hydroxy-2-oxopyrrolidin-1-yl]pyrazol-1-yl}pipéridine-1-carboxylate de tertio-butyle |
| « A171 » | N-(3-Chloro-5-fluorobenzyl)-(S)-3-hydroxy-2-oxo-1-(6-propionylaminopyridin-3-yl)pyrrolidine-3-carboxamide |
| « A172 » | N-(3-Chloro-5-fluorobenzyl)-(R)-3-hydroxy-2-oxo-1-(6-propionylaminopyridin-3-yl)pyrrolidine-3-carboxamide |

(suite)

| Composé n° | Nom |
|---|---|
| « A173 » | N-(3-Chloro-5-fluorobenzyl)-3-hydroxy-1-(6-morpholin-4-ylpyridin-3-yl)-2-oxopyrrolidine-3-carboxamide |
| « A174 » | N-[2-(3-Fluorophényl)éthyl]-1-(6-acétylaminopyridin-3-yl)-3-hydroxy-2-oxopyrrolidine-3-carboxamide |
| « A175 » | N-[2-(2-Fluorophényl)éthyl]-1-(6-acétylaminopyridin-3-yl)-3-hydroxy-2-oxopyrrolidine-3-carboxamide |
| « A176 » | N-(3,5-Difluorobenzyl)-1-(6-acétylaminopyridin-3-yl)-3-hydroxy-2-oxopyrrolidine-3-carboxamide |
| « A177 » | N-[2-(3-Fluorophényl)éthyl]-1-(3-carbamoylphényl)-3-hydroxy-2-oxopyrrolidine-3-carboxamide |
| « A178 » | N-(3,5-Difluorobenzyl)-1-(3-carbamoylphényl)-3-hydroxy-2-oxopyrrolidine-3-carboxamide |
| « A179 » | N-[2-(2-Fluorophényl)éthyl]-1-(3-carbamoylphényl)-3-hydroxy-2-oxopyrrolidine-3-carboxamide |
| « A180 » | N-(3-Chloro-5-fluorobenzyl)-3-hydroxy-2-oxo-1-(1-pipéridin-4-yl-1H-pyrazol-4-yl)pyrrolidine-3-carboxamide |
| « A181 » | N-(3-Chloro-5-fluorobenzyl)-(S)-3-hydroxy-1-(4-méthoxybenzyl)-2-oxopyrrolidine-3-carboxamide |
| « A182 » | N-(3-Chloro-5-fluorobenzyl)-(R)-3-hydroxy-1-(4-méthoxybenzyl)-2-oxopyrrolidine-3-carboxamide |
| « A183 » | N-[2-(3-Fluorophényl)éthyl]-3-hydroxy-2-oxo-1-(2-oxo-1,2,3,4-tétrahydroquinoléin-6-yl)pyrrolidine-3-carboxamide |
| « A184 » | N-[2-(2-Fluorophényl)éthyl]-3-hydroxy-2-oxo-1-(2-oxo-1,2,3,4-tétrahydroquinoléin-6-yl)pyrrolidine-3-carboxamide |
| « A185 » | N-(3,5-Difluorobenzyl)-3-hydroxy-2-oxo-1-(2-oxo-1,2,3,4-tétrahydroquinoléin-6-yl)pyrrolidine-3-carboxamide |
| « A186 » | N-(2-Chloro-3-fluorobenzyl)-1-(6-acétylaminopyridin-3-yl)-3-hydroxy-2-oxopyrrolidine-3-carboxamide |
| « A187 » | N-(3-Chloro-5-fluorobenzyl)-3-hydroxy-2-oxo-1-phényl-carbamoylméthylpyrrolidine-3-carboxamide |
| « A188 » | N-[2-(3,5-Difluorophényl)éthyl]-1-(6-acétylaminopyridin-3-yl)-3-hydroxy-2-oxopyrrolidine-3-carboxamide |
| « A189 » | N-(3-Chloro-5-fluorobenzyl)-(S)-3-hydroxy-1-(1H-indazol-5-yl)-2-oxopyrrolidine-3-carboxamide |
| « A190 » | N-(3-Chloro-5-fluorobenzyl)-(R)-3-hydroxy-1-(1H-indazol-5-yl)-2-oxopyrrolidine-3-carboxamide |
| « A191 » | N-[2-(3-Chloro-5-fluorophényl)éthyl]-(S)-3-hydroxy-1-(1H-indazol-5-yl)-2-oxopyrrolidine-3-carboxamide |
| « A192 » | N-[2-(3-Chloro-5-fluorophényl)éthyl]-(R)-3-hydroxy-1-(1H-indazol-5-yl)-2-oxopyrrolidine-3-carboxamide |
| « A193 » | N-(2-Chloro-3-fluorobenzyl)-1-(3-carbamoylphényl)-3-hydroxy-2-oxopyrrolidine-3-carboxamide |
| « A194 » | N-(3-Chloro-5-fluorobenzyl)-(S)-1-(2-éthylcarbamoyl-benzofuran-5-yl)-3-hydroxy-2-oxopyrrolidine-3-carboxamide |
| « A195 » | N-(3-Chloro-5-fluorobenzyl)-(R)-1-(2-éthylcarbamoyl-benzofuran-5-yl)-3-hydroxy-2-oxopyrrolidine-3-carboxamide |
| « A196 » | N-(2-Chloro-3-fluorobenzyl)-3-hydroxy-2-oxo-1-(2-oxo-1,2,3,4-tétrahydroquinoléin-6-yl)pyrrolidine-3-carboxamide |
| « A197 » | N-(3-Chloro-5-fluorobenzyl)-(S)-3-hydroxy-2-oxo-1-(3-oxo-3,4-dihydro-2H-benzo-1,4-oxazin-7-yl)pyrrolidine-3-carboxamide |
| « A198 » | N-(3-Chloro-5-fluorobenzyl)-(R)-3-hydroxy-2-oxo-1-(3-oxo-3,4-dihydro-2H-benzo-1,4-oxazin-7-yl)pyrrolidine-3-carboxamide |

(suite)

| Composé n° | Nom |
|---|---|
| « A199 » | N-[2-(3,5-Difluorophényl)éthyl]-3-hydroxy-2-oxo-1-(2-oxo-1,2,3,4-tétrahydroquinoléin-6-yl)pyrrolidine-3-carboxamide |
| « A200 » | N-(3-Chloro-5-fluorobenzyl)-3-hydroxy-1-(2-méthyl-1H-indol-5-yl)-2-oxopyrrolidine-3-carboxamide |
| « A201 » | N-[2-(3,5-Difluorophényl)éthyl]-1-(3-carbamoylphényl)-3-hydroxy-2-oxopyrrolidine-3-carboxamide |
| « A202 » | 3-[3-(3-Chloro-5-fluorobenzylcarbamoyl)-3-hydroxy-2-oxopyrrolidin-1-yl]cyclohexanecarboxylate d'éthyle |
| « A203 » | Acide [3-(3-chloro-5-fluorobenzylcarbamoyl)-3-hydroxy-2-oxopyrrolidin-1-yl]acétique |
| « A204 » | N-(3-Chloro-5-fluorobenzyl)-1-(3-carbamoyl-5-trifluorométhylphényl)-3-hydroxy-2-oxopyrrolidine-3-carboxamide |
| « A205 » | 5-[3-(3-Chloro-5-fluorobenzylcarbamoyl)-3-hydroxy-2-oxopyrrolidin-1-yl]-1H-indole-2-carboxylate d'éthyle |
| « A206 » | N-(2-Chloro-3-fluorobenzyl)-3-hydroxy-1-(1H-indol-5-yl)-2-oxopyrrolidine-3-carboxamide |
| « A207 » | N-[2-(3,5-Difluorophényl)éthyl]-3-hydroxy-1-(1H-indol-5-yl)-2-oxopyrrolidine-3-carboxamide |
| « A208 » | N-(3-Chloro-5-fluorobenzyl)-3-hydroxy-1-méthylcarbamoyl-méthyl-2-oxopyrrolidine-3-carboxamide |
| « A209 » | N-(3,5-Difluorobenzyl)-3-hydroxy-1-(1H-indol-5-yl)-2-oxopyrrolidine-3-carboxamide |
| « A210 » | N-[2-(2-Fluorophényl)éthyl]-3-hydroxy-1-(1H-indol-5-yl)-2-oxopyrrolidine-3-carboxamide |
| « A211 » | 5-[3-(3-Chloro-5-fluorobenzylcarbamoyl)-3-hydroxy-2-oxopyrrolidin-1-yl]-1 H-indole-2-carboxamide |
| « A212 » | N-[2-(3-Fluorophényl)éthyl]-3-hydroxy-1-(1H-indol-5-yl)-2-oxopyrrolidine-3-carboxamide |
| « A213 » | 3-[3-(3-Chloro-5-fluorobenzylcarbamoyl)-3-hydroxy-2-oxopyrrolidin-1-ylméthyl]azétidine-1-carboxylate de tertio-butyle |
| « A214 » | N-(3-Chloro-5-fluorobenzyl)-3-hydroxy-2-oxo-1-(7-oxo-5,6,7,8-tétrahydro-1,8-naphtyridin-3-yl)pyrrolidine-3-carboxamide |
| « A215 » | N-(3-Chloro-5-fluorobenzyl)-3-hydroxy-2-oxo-1-(1H-pyrrolo[2,3-b]pyridin-5-yl)pyrrolidine-3-carboxamide |
| « A216 » | 5-[3-(3-Chloro-5-fluorobenzylcarbamoyl)-3-hydroxy-2-oxopyrrolidin-1-yl]nicotinamide |
| « A217 » | N-(3-Chloro-5-fluorobenzyl)-1-(2-cyano-1H-indol-5-yl)-3-hydroxy-2-oxopyrrolidine-3-carboxamide |
| « A218 » | N-(3-Chloro-5-fluorobenzyl)-1-azétidin-3-ylméthyl-3-hydroxy-2-oxopyrrolidine-3-carboxamide |
| « A219 » | N-(3-Chloro-5-fluorobenzyl)-1-[2-(5-fluoro-1H-indol-3-yl)-éthyl]-3-hydroxy-2-oxopyrrolidine-3-carboxamide |
| « A220 » | N-(3-Chloro-5-fluorobenzyl)-3-hydroxy-2-oxo-1-(tétrahydropyran-4-ylméthyl)pyrrolidine-3-carboxamide |
| « A221 » | N-[2-(3,5-Difluorophényl)éthyl]-(S)-3-hydroxy-2-oxo-1-(2-oxo-1,2,3,4-tétrahydroquinoléin-6-yl)pyrrolidine-3-carboxamide |
| « A222 » | N-(3-Chloro-5-fluorobenzyl)-(S)-1-(3-cyano-1H-indol-5-yl)-3-hydroxy-2-oxopyrrolidine-3-carboxamide |
| « A223 » | N-[2-(3,5-Difluorophényl)éthyl]-(R)-3-hydroxy-2-oxo-1-(2-oxo-1,2,3,4-tétrahydroquinoléin-6-yl)pyrrolidine-3-carboxamide |

(suite)

| Composé n° | Nom |
|---|---|
| « A224 » | N-(3-Chloro-5-fluorobenzyl)-(R)-1-(3-cyano-1H-indol-5-yl)-3-hydroxy-2-oxopyrrolidine-3-carboxamide |
| « A225 » | N-(3-Chloro-5-fluorobenzyl)-3-hydroxy-2-oxo-1-[(R)-1-(tétrahydrofuran-2-yl)méthyl]pyrrolidine-3-carboxamide |
| « A226 » | Acide 4-[3-(3-chloro-5-fluorobenzylcarbamoyl)-3-hydroxy-2-oxopyrrolidin-1-ylméthyl]cyclohexanecarboxylique |
| « A227 » | Acide 4-[3-(3-chloro-5-fluorobenzylcarbamoyl)-3-hydroxy-2-oxopyrrolidin-1-ylméthyl]cyclohexanecarboxylique |
| « A228 » | (1S,5R,6S)-6-[3-(3-Chloro-5-fluorobenzylcarbamoyl)-3-hydroxy-2-oxopyrrolidin-1-yl]-3-azabicyclo[3.1.0]hexane-3-carboxylate de tertio-butyle |
| « A229 » | N-(3-Chloro-5-fluorobenzyl)-1-(1S,5R,6S)-3-azabicyclo-[3.1.0]hex-6-yl-3-hydroxy-2-oxopyrrolidine-3-carboxamide |
| « A230 » | N-(3-Chloro-5-fluorobenzyl)-1-((1S,5R,6S)-3-éthanesulfonyl-3-azabicyclo[3.1.0]hex-6-yl)-3-hydroxy-2-oxopyrrolidine-3-carboxamide |
| « A231 » | N-(3-Chloro-5-fluorobenzyl)-1-((1S,5R,6S)-3-acétyl-3-azabicyclo[3.1.0]hex-6-yl)-3-hydroxy-2-oxopyrrolidine-3-carboxamide |
| « A232 » | N-(3-Chloro-5-fluorobenzyl)-3-hydroxy-1-(1-méthyl-1H-benzimidazol-2-ylméthyl)-2-oxopyrrolidine-3-carboxamide |
| « A233 » | N-(3-Chloro-5-fluorobenzyl)-(S)-3-hydroxy-2-oxo-[1,3']bipyrrolidinyl-3-carboxamide |
| « A234 » | N-(2-Chloro-4,5-difluorobenzyl)-(S)-3-hydroxy-2-oxo-1-(2-oxo-1,2,3,4-tétrahydroquinoléin-6-yl)pyrrolidine-3-carboxamide |
| « A235 » | N-(5-Chloro-2,4-difluorobenzyl)-(S)-3-hydroxy-2-oxo-1-(2-oxo-1,2,3,4-tétrahydroquinoléin-6-yl)pyrrolidine-3-carboxamide |
| « A236 » | N-(2,4,6-Trifluorobenzyl)-(S)-3-hydroxy-2-oxo-1-(2-oxo-1,2,3,4-tétrahydroquinoléin-6-yl)pyrrolidine-3-carboxamide |
| « A237 » | N-(3-Chloro-5-fluorobenzyl)-1-[2-(2,4-difluorophényl)éthyl]-3-hydroxy-2-oxopyrrolidine-3-carboxamide |
| « A238 » | 5-[(S)-3-(3-Chloro-5-fluorobenzylcarbamoyl)-3-hydroxy-2-oxopyrrolidin-1-yl]-1H-indole-3-carboxamide |
| « A239 » | N-(3-Chloro-5-fluorobenzyl)-(S)-3-hydroxy-1-(2-méthyl-1H-indol-5-yl)-2-oxopyrrolidine-3-carboxamide |
| « A240 » | N-(3-Chloro-5-fluorobenzyl)-(R)-3-hydroxy-1-(2-méthyl-1H-indol-5-yl)-2-oxopyrrolidine-3-carboxamide |
| « A241 » | N-(4-Chloro-2-fluorobenzyl)-(S)-3-hydroxy-2-oxo-1-(2-oxo-1,2,3,4-tétrahydroquinoléin-6-yl)pyrrolidine-3-carboxamide |
| « A242 » | N-(2-Chloro-4-fluorobenzyl)-(S)-3-hydroxy-2-oxo-1-(2-oxo-1,2,3,4-tétrahydroquinoléin-6-yl)pyrrolidine-3-carboxamide |
| « A243 » | N-(3-Chloro-5-fluorobenzyl)-3-hydroxy-2-oxo-1-[(S)-1-(tétrahydrofuran-2-yl)méthyl]pyrrolidine-3-carboxamide |
| « A244 » | N-(2-Chloro-3,6-difluorobenzyl)-(S)-3-hydroxy-2-oxo-1-(2-oxo-1,2,3,4-tétrahydroquinoléin-6-yl)pyrrolidine-3-carboxamide |
| « A245 » | N-(2,4,5-Trifluorobenzyl)-(S)-3-hydroxy-2-oxo-1-(2-oxo-1,2,3,4-tétrahydroquinoléin-6-yl)pyrrolidine-3-carboxamide |

(suite)

| Composé n° | Nom |
|---|---|
| « A246 » | N-(2,3,6-Trifluorobenzyl)-(S)-3-hydroxy-2-oxo-1-(2-oxo-1,2,3,4-tétrahydroquinoléin-6-yl)pyrrolidine-3-carboxamide |
| « A247 » | N-(3-Chloro-4-fluorobenzyl)-(S)-3-hydroxy-2-oxo-1-(2-oxo-1,2,3,4-tétrahydroquinoléin-6-yl)pyrrolidine-3-carboxamide |
| « A248 » | N-(3-Chloro-5-fluorobenzyl)-1-carbamoylméthyl-3-hydroxy-2-oxopyrrolidine-3-carboxamide |
| « A249 » | N-(4-Chloro-2,6-difluorobenzyl)-(S)-3-hydroxy-2-oxo-1-(2-oxo-1,2,3,4-tétrahydroquinoléin-6-yl)pyrrolidine-3-carboxamide |
| « A250 » | N-(3-Chloro-2,6-difluorobenzyl)-(S)-3-hydroxy-2-oxo-1-(2-oxo-1,2,3,4-tétrahydroquinoléin-6-yl)pyrrolidine-3-carboxamide |
| « A251 » | N-(3-Chloro-2,4-difluorobenzyl)-(S)-3-hydroxy-2-oxo-1-(2-oxo-1,2,3,4-tétrahydroquinoléin-6-yl)pyrrolidine-3-carboxamide |
| « A252 » | N-(3,5-Difluorobenzyl)-1-cyclobutylméthyl-3-hydroxy-2-oxopipéridine-3-carboxamide |
| « A253 » | N-(3-Fluorobenzyl)-1-(3,4-difluorobenzyl)-3-hydroxy-2-oxopipéridine-3-carboxamide |
| « A254 » | N-(3-Fluorobenzyl)-(S)-1-(2,3-difluorobenzyl)-3-hydroxy-2-oxopipéridine-3-carboxamide |
| « A255 » | N-(4-Fluorobenzyl)-(S)-1-(2,3-difluorobenzyl)-3-hydroxy-2-oxopipéridine-3-carboxamide |
| « A256 » | N-(3-Fluorobenzyl)-(S)-1-cyclohexylméthyl-3-hydroxy-2-oxopipéridine-3-carboxamide |
| « A257 » | N-(4-Fluorobenzyl)-1-(3,4-difiuorobenzyl)-3-hydroxy-2-oxopipéridine-3-carboxamide |
| « A258 » | N-(5-Méthylisoxazol-3-ylméthyl)-1-(3,4-difluorobenzyl)-3-hydroxy-2-oxopipéridine-3-carboxamide |
| « A259 » | N-(Pyridin-2-ylméthyl)-(S)-1-(2,3-difluorobenzyl)-3-hydroxy-2-oxopipéridine-3-carboxamide |
| « A260 » | N-(Pyridin-2-ylméthyl)-(R)-1-(2,3-difluorobenzyl)-3-hydroxy-2-oxopipéridine-3-carboxamide |
| « A261 » | N-(3-Fluorobenzyl)-(R)-1-(2,3-difluorobenzyl)-3-hydroxy-2-oxopipéridine-3-carboxamide |
| « A262 » | N-(4-Fluorobenzyl)-(R)-1-(2,3-difluorobenzyl)-3-hydroxy-2-oxopipéridine-3-carboxamide |
| « A263 » | N-(3-Fluorobenzyl)-(R)-1-cyclohexylméthyl-3-hydroxy-2-oxopipéridine-3-carboxamide |
| « A264 » | N-(3,4,5-Trifluorobenzyl)-(S)-3-hydroxy-2-oxo-1-(2-oxo-1,2,3,4-tétrahydroquinoléin-6-yl)pyrrolidine-3-carboxamide |
| « A265 » | N-(4-Chloro-3-fluorobenzyl)-(S)-3-hydroxy-2-oxo-1-(2-oxo-1,2,3,4-tétrahydroquinoléin-6-yl)pyrrolidine-3-carboxamide |
| « A266 » | N-(2,3,5-Trifluorobenzyl)-(S)-3-hydroxy-2-oxo-1-(2-oxo-1,2,3,4-tétrahydroquinoléin-6-yl)pyrrolidine-3-carboxamide |
| « A267 » | 3-(1,3-Dihydroisoindole-2-carbonyl)-3-hydroxy-1-phénylpyrrolidin-2-one |
| « A268 » | 1-Benzyl-3-(1,3-dihydroisoindole-2-carbonyl)-3-hydroxypyrrolidin-2-one |
| « A269 » | N-(3-Chloro-5-fluorobenzyl)-1-(1H-benzimidazol-2-ylméthyl)-3-hydroxy-2-oxopyrrolidine-3-carboxamide |
| « A270 » | N-(3-Chloro-5-fluorobenzyl)-1-benzothiazol-2-ylméthyl-3-hydroxy-2-oxopyrrolidine-3-carboxamide |
| « A271 » | Acide 3-[3-(3-chloro-5-fluorobenzylcarbamoyl)-3-hydroxy-2-oxopyrrolidin-1-yl]propionique |
| « A272 » | N-(3-Chloro-5-fluorobenzyl)-3-hydroxy-1-(3-méthyl-3H-imidazol-4-ylméthyl)-2-oxopyrrolidine-3-carboxamide |
| « A273 » | Acide 4-[3-(3-chloro-5-fluorobenzylcarbamoyl)-3-hydroxy-2-oxopyrrolidin-1-ylméthyl]cyclohexanecarboxylique |

(suite)

| Composé n° | Nom |
|---|---|
| « A274 » | Acide 4-[3-(3-chloro-5-fluorobenzylcarbamoyl)-3-hydroxy-2-oxopyrrolidin-1-ylméthyl]cyclohexanecarboxylique |
| « A275 » | N-(3-Chloro-5-fluorobenzyl)-1-((1S,5R,6S)-3-éthyl-3-aza-bicyclo[3.1.0]hex-6-yl)-3-hydroxy-2-oxopyrrolidine-3-carboxamide |
| « A276 » | N-(3-Chloro-5-fluorobenzyl)-3-hydroxy-1-(2-méthyl-2H-pyrazol-3-ylméthyl)-2-oxopyrrolidine-3-carboxamide |
| « A277 » | N-(3-Chloro-5-fluorobenzyl)-3-hydroxy-2-oxo-1-(1H-pyrazol-3-ylméthyl)pyrrolidine-3-carboxamide |
| « A278 » | N-(3-Chloro-5-fluorobenzyl)-3-hydroxy-1-(4-méthyl-carbamoylcyclohexylméthyl)-2-oxopyrrolidine-3-carboxamide |
| « A279 » | N-(3-Chloro-5-fluorobenzyl)-3-hydroxy-1-(1H-imidazol-4-ylméthyl)-2-oxopyrrolidine-3-carboxamide |
| « A280 » | N-(3-Chloro-5-fluorobenzyl)-1-(1,5-diméthyl-1H-pyrrol-2-ylméthyl)-3-hydroxy-2-oxopyrrolidine-3-carboxamide |
| « A281 » | N-(3-Chloro-5-fluorobenzyl)-(S)-3-hydroxy-1'-méthanesulfonyl-2-oxo[1,3']bipyrrolidinyl-3-carboxamide |
| « A282 » | N-(3-Chloro-5-fluorobenzyl)-3-hydroxy-1-[(2-hydroxyéthyl-carbamoyl)méthyl]-2-oxopyrrolidine-3-carboxamide |
| « A283 » | N-(2-Chloro-5-fluorobenzyl)-(S)-3-hydroxy-2-oxo-1-(2-oxo-1,2,3,4-tétrahydroquinoléin-6-yl)pyrrolidine-3-carboxamide |
| « A284 » | N-(2,3,4-Trifluorobenzyl)-(S)-3-hydroxy-2-oxo-1-(2-oxo-1,2,3,4-tétrahydroquinoléin-6-yl)pyrrolidine-3-carboxamide |
| « A285 » | N-(2,3-Difluorobenzyl)-(S)-3-hydroxy-2-oxo-1-(2-oxo-1,2,3,4-tétrahydroquinoléin-6-yl)pyrrolidine-3-carboxamide |
| « A286 » | N-(3-Chloro-5-fluorobenzyl)-(S)-1-(3,3-difluoro-2-oxo-2,3-dihydro-1H-indol-5-yl)-3-hydroxy-2-oxopyrrolidine-3-carboxamide |
| « A287 » | N-(3-Fluorobenzyl)-1-(4-fluorobenzyl)-3-hydroxy-2-oxopipéridine-3-carboxamide |
| « A288 » | N-(3-Chloro-5-fluorobenzyl)-(R)-1-(3,3-difluoro-2-oxo-2,3-dihydro-1H-indol-5-yl)-3-hydroxy-2-oxopyrrolidine-3-carboxamide |
| « A289 » | N-(2,6-Difluorobenzyl)-(S)-3-hydroxy-2-oxo-1-(2-oxo-1,2,3,4-tétrahydroquinoléin-6-yl)pyrrolidine-3-carboxamide |
| « A290 » | N-(3-Chloro-5-fluorobenzyl)-3-hydroxy-1-(5-méthyl-1,3,4-oxadiazol-2-ylméthyl)-2-oxopyrrolidine-3-carboxamide |
| « A291 » | N-(3-Chloro-5-fluorobenzyl)-(R)-1'-acétyl-3-hydroxy-2-oxo[1,3']bipyrrolidinyl-3-carboxamide |
| « A292 » | N-(3-Chloro-5-fluorobenzyl)-(R)-3-hydroxy-1'-méthanesulfonyl-2-oxo[1,3']bipyrrolidinyl-3-carboxamide |
| « A293 » | N-(3-Chloro-5-fluorobenzyl)-3-hydroxy-1-(4-méthylcyclohexyl)-2-oxopyrrolidine-3-carboxamide |
| « A294 » | N-(5-Méthylisoxazol-3-ylméthyl)-1-(2,3-difluorobenzyl)-3-hydroxy-2-oxopipéridine-3-carboxamide |
| « A295 » | N-(4-Méthylbenzyl)-1-(2,3-difluorobenzyl)-3-hydroxy-2-oxopipéridine-3-carboxamide |
| « A296 » | N-(2-Fluorobenzyl)-1-(2,3-difluorobenzyl)-3-hydroxy-2-oxopipéridine-3-carboxamide |
| « A297 » | N-(5-Méthylpyrazin-2-ylméthyl)-1-(2,3-difluorobenzyl)-3-hydroxy-2-oxopipéridine-3-carboxamide |
| « A298 » | N-(3-Chloro-5-fluorobenzyl)-(S)-3-hydroxy-2-oxo-1-pyridin-2-ylpyrrolidine-3-carboxamide |

(suite)

| Composé n° | Nom |
|---|---|
| « A299 » | N-(3-Chloro-5-fluorobenzyl)-(R)-3-hydroxy-2-oxo-1-pyridin-2-ylpyrrolidine-3-carboxamide |
| « A300 » | N-(3-Chloro-5-fluorobenzyl)-1-(2-carbamoyléthyl)-3-hydroxy-2-oxopyrrolidine-3-carboxamide |
| « A301 » | N-(3-Chloro-5-fluorobenzyl)-1-benzyl-3-hydroxypipéridine-3-carboxamide |
| « A302 » | 3-[(3-Chloro-5-fluorobenzylamino)méthyl]-3-hydroxy-1-phénylpipéridin-2-one |
| « A303 » | N-(3-Chloro-5-fluorobenzyl)-3,4-dihydroxy-2-oxo-1-(2-oxo-1,2,3,4-tétrahydroquinoléin-6-yl)pyrrolidine-3-carboxamide |
| « A304 » | N-(3-Chloro-5-fluorobenzyl)-5-fluoro-3-hydroxy-2-oxo-1-(2-oxo-1,2,3,4-tétrahydroquinoléin-6-yl)pyrrolidine-3-carboxamide |
| « B1 » | N-(3-Chloro-5-fluorobenzyl)-(S)-1-(2-cyano-1H-indol-5-yl)-3-hydroxy-2-oxopyrrolidine-3-carboxamide |
| « B2 » | N-(3-Chloro-5-fluorobenzyl)-(S)-1-(8-fluoro-2-oxo-1,2,3,4-tétrahydroquinoléin-6-yl)-3-hydroxy-2-oxopyrrolidine-3-carboxamide |
| « B3 » | N-(2-Chloro-6-fluorobenzyl)-(S)-3-hydroxy-2-oxo-1-(2-oxo-1,2,3,4-tétrahydroquinoléin-6-yl)pyrrolidine-3-carboxamide |
| « B4 » | N-(5-Chloro-2-fluorobenzyl)-(S)-3-hydroxy-2-oxo-1-(2-oxo-1,2,3,4-tétrahydroquinoléin-6-yl)pyrrolidine-3-carboxamide |
| « B5 » | N-[2-(2-Fluorophényl)éthyl]-(S)-3-hydroxy-2-oxo-1-(2-oxo-1,2,3,4-tétrahydroquinoléin-6-yl)pyrrolidine-3-carboxamide |
| « B6 » | N-[2-(3-Fluorophényl)éthyl]-(S)-3-hydroxy-2-oxo-1-(2-oxo-1,2,3,4-tétrahydroquinoléin-6-yl)pyrrolidine-3-carboxamide |
| « B7 » | N-(3,5-Difluorobenzyl)-(S)-3-hydroxy-2-oxo-1-(2-oxo-1,2,3,4-tétrahydroquinoléin-6-yl)pyrrolidine-3-carboxamide |
| « B8 » | N-(3,5-Difluorobenzyl)-(S)-3-hydroxy-1-(1H-indol-5-yl)-2-oxopyrrolidine-3-carboxamide |
| « B9 » | N-(2-Chloro-3-fluorobenzyl)-(S)-1-(6-acétylaminopyridin-3-yl)-3-hydroxy-2-oxopyrrolidine-3-carboxamide |
| « B10 » | N-[2-(3-Fluorophényl)éthyl]-(S)-3-hydroxy-1-(1H-indol-5-yl)-2-oxopyrrolidine-3-carboxamide |
| « B11 » | N-(3-Chloro-5-fluorobenzyl)-(S)-3-hydroxy-2-oxo-1-(1H-pyrrolo[2,3-b]pyridin-5-yl)pyrrolidine-3-carboxamide |
| « B12 » | N-(3-Chloro-5-fluorobenzyl)-1-(4-chloro-2-méthoxy-5-méthylphényl)-3-hydroxy-2-oxopyrrolidine-3-carboxamide |
| « B13 » | Acide 1-(5-chloro-2-méthoxyphényl)-3-hydroxy-2-oxopyrrolidine-3-carboxylique 3-chloro-5-fluorobenzylamide |
| « B14 » | Acide (S)-3-hydroxy-1-(1H-indol-5-yl)-2-oxopyrrolidine-3-carboxylique [2-(2-fluorophényl)éthyl]-amide |
| « B15 » | N-(2-Chloro-3-fluorobenzyl)-(S)-3-hydroxy-2-oxo-1-(2-oxo-1,2,3,4-tétrahydropuinoléin-6-yl)pyrrolidine-3-carboxamide |
| « B16 » | N-(3-Chloro-5-fluorobenzyl)-1-benzyl-3-hydroxypipéridine-3-carboxamide |
| « B17 » | N-(3-Chloro-5-fluorobenzyl)-(S)-1-(4-bromo-3-hydroxyméthylphényl)-3-hydroxy-2-oxopyrrolidine-3-carboxamide |
| « B18 » | 3-[(3-Chloro-5-fluorobenzylamino)méthyl]-1-cyclohexylméthyl-3-hydroxypipéridin-2-one |
| « B19 » | 1-Benzyl-3-[(3-chloro-5-fluorobenzylamino)-méthyl]pipéridin-3-ol |
| « B20 » | 1-Benzyl-3-[(3-fluorobenzylamino)méthyl]pipéridin-3-ol |
| « B21 » | (S)-1-Benzyl-3-[(4-fluorobenzylamino)méthyl]pipéridin-3-ol |

(suite)

| Composé n° | Nom |
|---|---|
| « B22 » | (S)-1-(2,3-Difluorobenzyl)-3-[(3-fluorobenzylamino)méthyl]-3-hydroxypipéridin-2-one |
| « B23 » | (R)-1-(2,3-Difluorobenzyl)-3-[(3-fluorobenzylamino)méthyl]-3-hydroxypipéridin-2-one |
| « B24 » | N-(2,5-Difluorobenzyl)-(S)-3-hydroxy-2-oxo-1-(2-oxo-1,2,3,4-tétrahydroquinoléin-6-yl)pyrrolidine-3-carboxamide |
| « B25 » | N-(3-Chloro-2-fluorobenzyl)-(S)-3-hydroxy-2-oxo-1-(2-oxo-1,2,3,4-tétrahydroquinoléin-6-yl)pyrrolidine-3-carboxamide |
| « B26 » | N-(3-Chloro-5-fluorobenzyl)-1-(2-fluoro-5-trifluorométhyl-phényl)-3-hydroxy-2-oxopyrrolidine-3-carboxamide |
| « B27 » | N-(3-Chloro-5-fluorobenzyl)-1-(4-chloro-3-trifluorométhylphényl)-3-hydroxy-2-oxopyrrolidine-3-carboxamide |
| « B28 » | N-(3-Chloro-5-fluorobenzyl)-1-(3-carbamoyl-4-fluorophényl)-3-hydroxy-2-oxopyrrolidine-3-carboxamide |
| « B29 » | 3-[(3-Chloro-5-fluorobenzylamino)méthyl]-3-hydroxy-1-phénylpyrrolidin-2-one |
| « B30 » | 1-Benzyl-3-[(3-chloro-5-fluorobenzylamino)méthyl]-pyrrolidin-3-ol |
| « B31 » | 1-Benzyl-3-[(3-chloro-5-fluorobenzylamino)méthyl]-3-hydroxypipéridin-2-one |
| « B32 » | 3-[(3-Chloro-5-fluorobenzylamino)méthyl]-3-hydroxy-1-phénylpipéridin-2-one |
| « B33 » | N-(3-Chloro-5-fluorobenzyl)-3-hydroxy-1-(2-méthyl-3H-imidazo[4,5-b]pyridin-6-yl)-2-oxopyrrolidine-3-carboxamide |
| « B34 » | N-(3-Chloro-2,5-difluorobenzyl)-(S)-3-hydroxy-2-oxo-1-(2-oxo-1,2,3,4-tétrahydroquinoléin-6-yl)pyrrolidine-3-carboxamide |
| « B35 » | N-(3-Chloro-5-fluorobenzyl)-3-hydroxy-1-(3H-imidazo[4,5-b]pyridin-6-yl)-2-oxopyrrolidine-3-carboxamide |
| « B36 » | N-(3-Chloro-5-fluorobenzyl)-3-hydroxy-2-oxo-1-(2-trifluorométhyl-3H-imidazo[4,5-b]pyridin-6-yl)pyrrolidine-3-carboxamide |
| « B37 » | N-(3-Chloro-5-fluorobenzyl)-(S)-3-hydroxy-1-[2-(1H-indol-3-yl)éthyl]-2-oxopyrrolidine-3-carboxamide |
| « B38 » | N-(3-Chloro-5-fluorobenzyl)-(R)-3-hydroxy-1-[2-(1H-indol-3-yl)éthyl]-2-oxopyrrolidine-3-carboxamide |
| « B39 » | N-(3-Chloro-5-fluorobenzyl)-(S)-1-(2-fluoro-5-trifluorométhylphényl)-3-hydroxy-2-oxopyrrolidine-3-carboxamide |
| « B40 » | N-(3-Chloro-5-fluorobenzyl)-(S)-3-hydroxy-2-oxo-1-(2-oxo-1,2-dihydroquinoléin-6-yl)pyrrolidine-3-carboxamide |
| « B41 » | N-(3-Chloro-5-fluorobenzyl)-(R)-3-hydroxy-2-oxo-1-(2-oxo-1,2-dihydroquinoléin-6-yl)pyrrolidine-3-carboxamide |
| « B42 » | N-(3-Chloro-5-fluorobenzyl)-(R)-1-(2-fluoro-5-trifluorométhylphényl)-3-hydroxy-2-oxopyrrolidine-3-carboxamide |
| « B43 » | N-(3-Chloro-5-fluorobenzyl)-3-hydroxy-2-oxo-1-(3-trifluorométhyl-1H-indol-5-yl)pyrrolidine-3-carboxamide |
| « B44 » | 1-Benzyl-3-[(3,5-difluorobenzylamino)méthyl]pipéridin-3-ol |
| « B45 » | 3-[(4-Fluorobenzylamino)méthyl]-3-hydroxy-1-phénylpyrrolidin-2-one |
| « B46 » | 3-[(3,5-Difluorobenzylamino)méthyl]-3-hydroxy-1-phénylpyrrolidin-2-one |
| « B47 » | 3-{[2-(4-Fluorophényl)éthylamino]méthyl}-3-hydroxy-1-phénylpyrrolidin-2-one |
| « B48 » | 3-[(3-Fluorobenzylamino)méthyl]-3-hydroxy-1-phénylpyrrolidin-2-one |

(suite)

| Composé n° | Nom |
|---|---|
| « B49 » | 3-Hydroxy-3-[(4-méthylbenzylamino)méthyl]-1-phénylpyrrolidin-2-one |
| « B50 » | N-(2-Oxo-1,2,3,4-tétrahydroquinoléin-6-yl)-3-hydroxy-2-oxo-1-(2-oxo-1,2,3,4-tétrahydroquinoléin-6-yl)pyrrolidine-3-carboxamide |
| « B51 » | 3-Hydroxy-2-oxopyrrolidine-1,3-dicarboxylate de 3-benzyle et de 1-tertio-butyle |
| « B52 » | N-(3-Chloro-5-fluorobenzyl)-(S)-3-hydroxy-2-oxo-1-(4-trifluorométhylpyridin-2-yl)pyrrolidine-3-carboxamide |
| « B53 » | 3-Hydroxy-2-oxopyrrolidine-1,3-dicarboxylate de 1-benzyle et de 3-isopropyle |
| « B54 » | (S)-1-Benzyl-3-[(3-chloro-5-fluorobenzylamino)méthyl]-3-hydroxypipéridin-2-one |
| « B55 » | (R)-1-Benzyl-3-[(3-chloro-5-fluorobenzylamino)méthyl]-3-hydroxypipéridin-2-one |
| « B56 » | N-(3-Fluorobenzyl)-(S)-3-hyd roxy-2-oxo-1-(2-oxo-1,2,3,4-tétrahydroquinoléin-6-yl)pyrrolidine-3-carboxamide |
| « B57 » | N-(3-Fluorobenzyl)-(R)-3-hydroxy-2-oxo-1-(2-oxo-1,2,3,4-tétrahydroquinoléin-6-yl)pyrrolidine-3-carboxamide |
| « B58 » | (R)-3-[(3-Chloro-5-fluorobenzylamino)méthyl]-3-hydroxy-1-phénylpyrrolidin-2-one |
| « B59 » | (S)-3-[(3-Chloro-5-fluorobenzylamino)méthyl]-3-hydroxy-1-phénylpyrrolidin-2-one |
| « B60 » | N-(3-Chloro-5-fluorobenzyl)-(S)-3-hydroxy-1-(1H-indazol-3-ylméthyl)-2-oxopyrrolidine-3-carboxamide |
| « B61 » | N-(3-Chloro-5-fluorobenzyl)-(R)-3-hydroxy-1-(2-méthyl-1H-pyrrolo[2,3-b]pyridin-5-yl)-2-oxopyrrolidine-3-carboxamide |
| « B62 » | N-(3-Chloro-5-fluorobenzyl)-(S)-3-hydroxy-1-(2-méthyl-1H-pyrrolo[2,3-b]pyridin-5-yl)-2-oxopyrrolidine-3-carboxamide |
| « B63 » | (S)-3-[(3-Chloro-5-fluorobenzylamino)méthyl]-1-cyclohexylméthyl-3-hydroxypipéridin-2-one |
| « B64 » | (R)-3-[(3-Chloro-5-fluorobenzylamino)méthyl]-1-cyclohexylméthyl-3-hydroxypipéridin-2-one |
| « B65 » | N-(3-Chloro-5-fluorobenzyl)-(S)-1-(2-fluoro-4-trifluorométhylphényl)-3-hydroxy-2-oxopyrrolidine-3-carboxamide |
| « B66 » | N-(3-Chloro-5-fluorobenzyl)-(S)-1-(2-fluoro-4-méthylphényl)-3-hydroxy-2-oxopyrrolidine-3-carboxamide |
| « B67 » | N-(3-Chloro-5-fluorobenzyl)-1-(2-fluoro-5-trifluorométhyl-phényl)-3-hydroxy-2-oxopipéridine-3-carboxamide |
| « B68 » | 6-{3-[(3-Chloro-5-fluorobenzylamino)méthyl]-3-hydroxy-2-oxopipéridin-1-yl}-3,4-dihydro-1H-quinoléin-2-one |
| « B69 » | N-(3-Chloro-5-fluorobenzyl)-3-hydroxy-2-oxo-1-(2-oxo-1,2,3,4-tétrahydroquinoléin-6-ylméthyl)pyrrolidine-3-carboxamide |
| « B70 » | N-(3-Chloro-5-fluorobenzyl)-(S)-1-(5-carbamoyl-2-fluorophényl)-3-hydroxy-2-oxopyrrolidine-3-carboxamide |
| « B71 » | N-(3-Chloro-5-fluorobenzyl)-(S)-3-hydroxy-2-oxo-1-(2-oxo-2,3-dihydro-1H-benzimidazol-5-yl)pyrrolidine-3-carboxamide |
| « B72 » | N-(3-Chloro-5-fluorobenzyl)-3-hydroxy-2-oxo-1-(2-trifluorométhyl-1H-indol-5-yl)pyrrolidine-3-carboxamide |
| « B73 » | N-(3-Chloro-5-fluorobenzyl)-(R)-3-hydroxy-2-oxo-1-(3-trifluorométhyl-1H-indol-5-yl)pyrrolidine-3-carboxamide |
| « B74 » | N-(3-Chloro-5-fluorobenzyl)-(S)-3-hydroxy-2-oxo-1-(3-trifluorométhyl-1H-indol-5-yl)pyrrolidine-3-carboxamide |

(suite)

| Composé n° | Nom |
|---|---|
| « B75 » | N-(3-Chloro-5-fluorobenzyl)-(S)-3-hydroxy-2-oxo-1-(1H-pyrazolo[3,4-b]pyridin-5-yl)pyrrolidine-3-carboxamide |
| « B76 » | N-(3-Chloro-5-fluorobenzyl)-3-hydroxy-2-oxo-1-(2-trifluorométhyl-1H-pyrrolo[2,3-b]pyridin-5-yl)pyrrolidine-3-carboxamide |
| « B77 » | N-(3-Chloro-5-fluorobenzyl)-(S)-3-hydroxy-2-oxo-1-(2-oxo-2,3-dihydrobenzothiazol-6-yl)pyrrolidine-3-carboxamide |
| « B78 » | 5-[(S)-3-(3-Chloro-5-fluorobenzylcarbamoyl)-3-hydroxy-2-oxopyrrolidin-1-yl]-1H-indole-2-carboxamide |
| « B79 » | 5-[(R)-3-(3-Chloro-5-fluorobenzylcarbamoyl)-3-hydroxy-2-oxopyrrolidin-1-yl]-1H-indole-2-carboxamide |
| « B80 » | 5-[(S)-3-(3-Chloro-5-fluorobenzylcarbamoyl)-3-hydroxy-2-oxopyrrolidin-1-yl]-1H-indole-2-carboxylate d'éthyle |
| « B81 » | 5-[(R)-3-(3-Chloro-5-fluorobenzylcarbamoyl)-3-hydroxy-2-oxopyrrolidin-1-yl]-1H-indole-2-carboxylate d'éthyle |
| « B82 » | N-(3-Chloro-5-fluorobenzyl)-3-hydroxy-1-[1-(2-méthoxyéthyl)-1H-pyrazol-4-yl]-2-oxopyrrolidine-3-carboxamide |
| « B83 » | N-(3-Chloro-5-fluorobenzyl)-(S)-3-hydroxy-2-oxo-1-(2-trifluorométhyl-1H-pyrrolo[2,3-b]pyridin-5-yl)pyrrolidine-3-carboxamide |
| « B84 » | Acide 5-[(S)-3-(3-chloro-5-fluorobenzylcarbamoyl)-3-hydroxy-2-oxopyrrolidin-1-yl]-1H-indole-2-carboxylique |
| « B85 » | Acide 5-[(R)-3-(3-chloro-5-fluorobenzylcarbamoyl)-3-hydroxy-2-oxopyrrolidin-1-yl]-1H-indole-2-carboxylique |
| « B86 » | N-(3-Chloro-5-fluorobenzyl)-(S)-3-hydroxy-2-oxo-1-(2-trifluorométhyl-1H-indol-5-yl)pyrrolidine-3-carboxamide |
| « B87 » | N-(3-Chloro-5-fluorobenzyl)-1-(1-cyanométhyl-1H-pyrazol-4-yl)-3-hydroxy-2-oxopyrrolidine-3-carboxamide |
| « B88 » | N-Éthyl-5-[(R)-3-(3-chloro-5-fluorobenzylcarbamoyl)-3-hydroxy-2-oxopyrrolidin-1-yl]-1H-indole-2-carboxamide |
| « B89 » | N-Isopropyl-5-[(R)-3-(3-chloro-5-fluorobenzylcarbamoyl)-3-hydroxy-2-oxopyrrolidin-1-yl]-1H-indole-2-carboxamide |
| « B90 » | N,N-Diméthyl-5-[(R)-3-(3-chloro-5-fluorobenzylcarbamoyl)-3-hydroxy-2-oxopyrrolidin-1-yl]-1H-indole-2-carboxamide |
| « B91 » | Acétate de 3-(3-chloro-5-fluorobenzylcarbamoyl)-2-oxo-1-(2-oxo-1,2,3,4-tétrahydroquinoléin-6-yl)pyrrolidin-3-yle |
| « B92 » | N-(3-Chloro-5-fluorobenzyl)-(S)-3-hydroxy-1-(3H-imidazo[4,5-b]pyridin-6-yl)-2-oxopyrrolidine-3-carboxamide |
| « B93 » | N-(3-Chloro-5-fluorobenzyl)-(R)-3-hydroxy-1-(3H-imidazo[4,5-b]pyridin-6-yl)-2-oxopyrrolidine-3-carboxamide |
| « B94 » | N-Éthyl-5-[(S)-3-(3-chloro-5-fluorobenzylcarbamoyl)-3-hydroxy-2-oxopyrrolidin-1-yl]-1H-indole-2-carboxamide |
| « B95 » | N,N-Diméthyl-5-[(S)-3-(3-chloro-5-fluorobenzylcarbamoyl)-3-hydroxy-2-oxopyrrolidin-1-yl]-1H-indole-2-carboxamide |
| « B96 » | N-Méthyl-5-[(S)-3-(3-chloro-5-fluorobenzylcarbamoyl)-3-hydroxy-2-oxopyrrolidin-1-yl]-1H-indole-2-carboxamide |

(suite)

| Composé n° | Nom |
|---|---|
| « B97 » | N-Isopropyl-5-[(S)-3-(3-chloro-5-fluorobenzylcarbamoyl)-3-hydroxy-2-oxopyrrolidin-1-yl]-1H-indole-2-carboxamide |
| « B98 » | N-(3-Chloro-5-fluorobenzyl)-3-hydroxy-2-oxo-1-(2-oxo-1,2,3,4-tétrahydroquinoléin-6-yl)pyrrolidine-3-carboxamide |
| « B99 » | N-(3-Chloro-5-fluorobenzyl)-(S)-3-hydroxy-1-[1-(2-méthoxyéthyl)-1H-pyrazol-4-yl]-2-oxopyrrolidine-3-carboxamide |
| « B100 » | N-(3-Chloro-5-fluorobenzyl)-(R)-3-hydroxy-1-[1-(2-méthoxyéthyl)-1H-pyrazol-4-yl]-2-oxopyrrolidine-3-carboxamide |
| « B101 » | N-(3-Chloro-5-fluorobenzyl)-(S)-3-hydroxy-2-oxo-1-(4-sulfamoylphényl)pyrrolidine-3-carboxamide |
| « B102 » | N-Méthyl-5-[(R)-3-(3-chloro-5-fluorobenzylcarbamoyl)-3-hydroxy-2-oxopyrrolidin-1-yl]-1H-indole-2-carboxamide |
| « B103 » | (S)-3-Hydroxy-2-oxo-1-(2-oxo-1,2,3,4-tétrahydroquinoléin-6-yl)pyrrolidine-3-carboxamide |
| « B104 » | N-(3-Chloro-5-fluorobenzyl)-3-hydroxy-2-oxo-1-(3-trifluorométhyl-1H-pyrrolo[2,3-b]pyridin-5-yl)pyrrolidine-3-carboxamide |
| « B105 » | 3-{2-tertio-Butoxycarbonylamino-5-[3-(3-chloro-5-fluorobenzylcarbamoyl)-3-hydroxy-2-oxopyrrolidin-1-yl]-phényl}propionate de méthyle |
| « B106 » | Acide 3-{2-tertio-butoxycarbonylamino-5-[3-(3-chloro-5-fluorobenzylcarbamoyl)-3-hydroxy-2-oxopyrrolidin-1-yl]-phényl}propionique |
| « B107 » | N-(3-Chloro-5-fluorobenzyl)-3-hydroxy-2-oxo-1-(3-sulfamoylphényl)pyrrolidine-3-carboxamide |
| « B108 » | N-{2-[2-(2-Aminoéthoxy)éthoxy]éthyl}-5-[(S)-3-(3-chloro-5-fluorobenzylcarbamoyl)-3-hydroxy-2-oxopyrrolidin-1-yl]-1H-indole-2-carboxamide |
| « B109 » | N-(3-Chloro-5-fluorobenzyl)-1-(7-fluoro-2-oxo-1,2,3,4-tétrahydroquinoléin-6-yl)-3-hydroxy-2-oxopyrrolidine-3-carboxamide |
| « B110 » | N-[2-(3,5-Difluorophényl)éthyl]-(S)-3-hydroxy-1-(1H-indol-5-yl)-2-oxopyrrolidine-3-carboxamide |
| « B111 » | 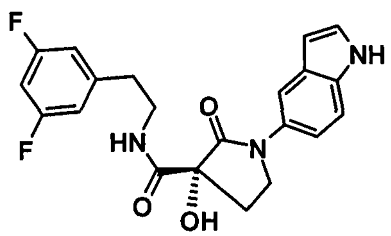 N-[2-(3,5-Difluorophényl)éthyl]-(R)-3-hydroxy-1-(1H-indol-5-yl)-2-oxopyrrolidine-3-carboxamide |
| « B112 » | N-(3-Chloro-5-fluorobenzyl)-(S)-3-hydroxy-1-[2-(morpholine-4-carbonyl)-1H-indol-5-yl]-2-oxopyrrolidine-3-carboxamide |
| « B113 » | N-[2-(2-Aminoéthoxy)éthyl]-5-[(S)-3-(3-chloro-5-fluorobenzylcarbamoyl)-3-hydroxy-2-oxopyrrolidin-1-yl]-1H-indole-2-carboxamide |
| « B114 » | N-(3-Chloro-5-fluorobenzyl)-(S)-1-[2-(azétidine-1-carbonyl)-1H-indol-5-yl]-3-hydroxy-2-oxopyrrolidine-3-carboxamide |
| « B115 » | N-(3-Chloro-5-fluorobenzyl)-3-hydroxy-2-oxo-1-(4-sulfamoylphényl)pyrrolidine-3-carboxamide |
| « B116 » | N-(3-Chloro-5-fluorobenzyl)-(S)-1-(1-cyanométhyl-1H-pyrazol-4-yl)-3-hydroxy-2-oxopyrrolidine-3-carboxamide |
| « B117 » | N-(3-Chloro-5-fluorobenzyl)-(R)-1-(1-cyanométhyl-1H-pyrazol-4-yl)-3-hydroxy-2-oxopyrrolidine-3-carboxamide |

(suite)

| Composé n° | Nom |
|---|---|
| « B118 » | N-(3-Chloro-5-fluorobenzyl)-1-(4-acétylsulfamoylphényl)-3-hydroxy-2-oxopyrrolidine-3-carboxamide |
| « B119 » | N-(3-Chloro-5-fluorobenzyl)-(S)-1-(1-carbamoylméthyl-1H-pyrazol-4-yl)-3-hydroxy-2-oxopyrrolidine-3-carboxamide |
| « B120 » | N-(3-Chloro-5-fluorobenzyl)-3-hydroxy-2-oxo-1-(1-phényléthyl)pyrrolidine-3-carboxamide |
| « B121 » | N-(2-Hydroxyéthyl)-5-[(S)-3-(3-chloro-5-fluorobenzyl-carbamoyl)-3-hydroxy-2-oxopyrrolidin-1-yl]-1H-indole-2-carboxamide |
| « B122 » | N-(3-Chloro-5-fluorobenzyl)-(S)-3-hydroxy-1-[2-(4-méthyl-pipérazine-1-carbonyl)-1H-indol-5-yl]-2-oxopyrrolidine-3-carboxamide |
| « B123 » | 4-{5-[(S)-3-(3-Chloro-5-fluorobenzylcarbamoyl)-3-hydroxy-2-oxopyrrolidin-1-yl]-1H-indole-2-carbonyl}pipérazine-1-carboxylate de tertio-butyle |
| « B124 » | N-(3-Chloro-5-fluorobenzyl)-1-(5-fluoro-2-oxo-1,2,3,4-tétrahydroquinoléin-6-yl)-3-hydroxy-2-oxopyrrolidine-3-carboxamide |
| « B125 » | N-(3-Chloro-5-fluorobenzyl)-3-hydroxy-2-oxo-1-(2-oxo-2,3-dihydro-1H-indol-5-yl)pyrrolidine-3-carboxamide |
| « B126 » | N-(3-Chloro-5-fluorobenzyl)-(S)-3-hydroxy-2-oxo-1-[2-(pipérazine-1-carbonyl)-1H-indol-5-yl]pyrrolidine-3-carboxamide |
| « B127 » | N-(3-Chloro-5-fluorobenzyl)-3-chloro-2-oxo-1-(2-oxo-1,2,3,4-tétrahydroquinoléin-6-yl)pyrrolidine-3-carboxamide |
| « B128 » | N-(3-Chloro-5-fluorobenzyl)-(S)-1-(3-diméthylsulfamoyl-phényl)-3-hydroxy-2-oxopyrrolidine-3-carboxamide |
| « B129 » | N-(2-Aminophényl)-5-[(S)-3-(3-chloro-5-fluorobenzyl-carbamoyl)-3-hydroxy-2-oxopyrrolidin-1-yl]-1H-indole-2-carboxamide |
| « B130 » | N-(3-Chloro-5-fluorobenzyl)-(S)-3-hydroxy-2-oxo-1-(2-oxo-2,3-dihydro-1H-indol-5-yl)pyrrolidine-3-carboxamide |
| « B131 » | N-(3-Chloro-5-fluorobenzyl)-(R)-3-hydroxy-2-oxo-1-(2-oxo-2,3-dihydro-1H-indol-5-yl)pyrrolidine-3-carboxamide |
| « B132 » | N-(2-Aminoéthyl)-5-[(S)-3-(3-chloro-5-fluorobenzyl-carbamoyl)-3-hydroxy-2-oxopyrrolidin-1-yl]-1H-indole-2-carboxamide |
| « B133 » | N-(3-Chloro-5-fluorobenzyl)-(S)-1-[2-(1H-benzoimidazol-2-yl)-1H-indol-5-yl]-3-hydroxy-2-oxopyrrolidine-3-carboxamide |
| « B134 » | N-(3-Chloro-5-fluorobenzyl)-(S)-1-(2-hydrazinocarbonyl-1H-indol-5-yl)-3-hydroxy-2-oxopyrrolidine-3-carboxamide |
| « B135 » | N-(3-Chloro-5-fluorobenzyl)-(S)-3-hydroxy-1-[2-(3-méthyl-1,2,4-oxadiazol-5-yl)-1H-indol-5-yl]-2-oxopyrrolidine-3-carboxamide |
| « B136 » | N-(3-Chloro-5-fluorobenzyl)-(S)-1-benzyloxy-3-hydroxy-2-oxopyrrolidine-3-carboxamide |
| « B137 » | N-(3-Chloro-5-fluorobenzyl)-3-hydroxy-2-oxopyrrolidine-3-carboxamide |
| « B138 » | N-(3-Chloro-5-fluorobenzyl)-(R)-1-benzyloxy-3-hydroxy-2-oxopyrrolidine-3-carboxamide |
| « B139 » | N-(3-Chloro-5-fluorobenzyl)-(S)-3-hydroxy-2-oxo-1-(3-trifluorométhyl-1H-pyrrolo[2,3-b]pyridin-5-yl)pyrrolidine-3-carboxamide |
| « B140 » | N-(3-Chloro-5-fluorobenzyl)-(R)-3-hydroxy-2-oxo-1-(3-trifluorométhyl-1H-pyrrolo[2,3-b]pyridin-5-yl)pyrrolidine-3-carboxamide |
| « B141 » | N-(3-Chloro-5-fluorobenzyl)-(S)-3-hydroxy-1-(4-méthylsulfamoylphényl)-2-oxopyrrolidine-3-carboxamide |

(suite)

| Composé n° | Nom |
|---|---|
| « B142 » | N-(3-Chloro-5-fluorobenzyl)-(S)-1-(4-benzylsulfamoyl-phényl)-3-hydroxy-2-oxopyrrolidine-3-carboxamide |
| « B143 » | N-(3-Chloro-5-fluorobenzyl)-(S)-1-[4-(benzylméthyl-sulfamoyl)phényl]-3-hydroxy-2-oxopyrrolidine-3-carboxamide |
| « B144 » | N-(3-Chloro-5-fluorobenzyl)-3-hydroxy-1-(1-méthyl-1H-indol-5-yl)-2-oxopyrrolidine-3-carboxamide |
| « B145 » | N-(3-Chloro-5-fluorobenzyl)-(S)-3-hydroxy-1-hydroxyméthyl-2-oxopyrrolidine-3-carboxamide |
| « B146 » | N-(3-Chloro-5-fluorobenzyl)-(R)-3-hydroxy-1-hydroxyméthyl-2-oxopyrrolidine-3-carboxamide |
| « B147 » | N-(3-Chloro-5-fluorobenzyl)-(S)-1-(3-chloro-1H-pyrrolo-[2,3-b]pyridin-5-yl)-3-hydroxy-2-oxopyrrolidine-3-carboxamide |
| « B148 » | N-(3-Chloro-5-fluorobenzyl)-3-hydroxy-2-oxo-1-(2-trifluorométhyl-1H-benzimidazol-5-yl)pyrrolidine-3-carboxamide |
| « B149 » | N-(3-Chtoro-5-fluorobenzyl)-(S)-3-hydroxy-2-oxo-1-(2-sulfamoyl-1H-indol-5-yl)pyrrolidine-3-carboxamide |
| « B150 » | (3S)-N-[(3-Chloro-5-fluorophényl)méthyl]-1-(2,2-dioxo-1,3-dihydro-2,1-benzothiazol-5-yl)-3-hydroxy-2-oxopyrrolidine-3-carboxamide |
| « B151 » | N-(3-Chloro-5-fluorobenzyl)-(S)-1-(2,2-dioxo-1,2,3,4-tétrahydro-2lamda*6*-benzo[c]thiazin-6-yl)-3-hydroxy-2-oxo-1-(2-sulfamoyl-1H-indol-5-yl)pyrrolidine-3-carboxamide |
| « B152 » | N-(3-Chloro-5-fluorobenzyl)-(S)-1-[((R)-1-diméthylcarbamoyl-2-méthylpropylcarbamoyl)méthyl]-3-hydroxy-2-oxo-1-(2-sulfamoyl-1H-indol-5-yl)pyrrolidine-3-carboxamide |
| « B153 » | N-(3-Chloro-5-fluorobenzyl)-(S)-1-[((S)-3-amino-1-méthylcarbamoylpropylcarbamoyl)méthyl]-3-hydroxy-2-oxo-1-(2-sulfamoyl-1H-indol-5-yl)pyrrolidine-3-carboxamide |
| « B154 » | N-(3-Hydroxy-2-oxo-1-phénylpyrrolidin-3-yl)benzamide |

ainsi que les sels, tautomères et stéréoisomères pharmaceutiquement utilisables de ceux-ci, y compris des mélanges de ceux-ci en toutes proportions.

**6.** Procédé de préparation de composés de formule I selon les revendications 1-5 et de sels, tautomères et stéréoisomères pharmaceutiquement utilisables de ceux-ci, **caractérisé en ce que**

a) pour la préparation de composés de formule I dans lesquels Y désigne CO et R désigne $NR^2R^4$, un composé de formule II

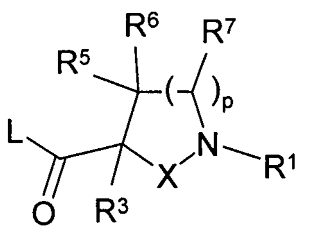

II

dans laquelle X, $R^1$, $R^3$, $R^5$, $R^6$, $R^7$ et p revêtent les significations indiquées selon la revendication 1, et L désigne Cl, Br, I ou un groupement OH libre ou modifié de manière réactive et fonctionnelle, est réagi avec un composé de formule III

$R^2$-$NHR^4$ III

dans laquelle $R^2$ et $R^4$ revêtent les significations indiquées selon la revendication 1,

ou
b) un composé de formule IV

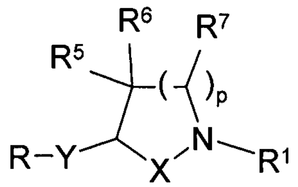

IV

dans laquelle $R^1$, $R^5$, $R^6$, $R^7$, R, X, Y et p revêtent les significations indiquées selon la revendication 1, est oxydé,
ou
c) pour la préparation de composés de formule I, dans lesquels X et Y désignent $CH_2$,
un composé de formule I dans laquelle X et Y désignent CO est réduit,
et/ou
une base ou un acide de formule I est converti(e) en l'un de ses sels.

**7.** Médicaments comprenant au moins un composé de formule I selon les revendications 1-5 et/ou des sels, tautomères et stéréoisomères pharmaceutiquement utilisables de celui-ci, y compris des mélanges de ceux-ci en toutes proportions, et éventuellement des excipients et/ou des adjuvants.

**8.** Composés de formule I selon les revendications 1-5,
ainsi que les sels, tautomères et stéréoisomères pharmaceutiquement utilisables de ceux-ci, y compris des mélanges de ceux-ci en toutes proportions, pour une utilisation dans le traitement de tumeurs, de métastases tumorales, de maladies prolifératives des cellules mésangiales, de l'hémangiome, de la rétinopathie proliférative, de la polyarthrite rhumatoïde, de la néovascularisation athérosclérotique, du psoriasis, de la néovascularisation oculaire, de l'ostéoporose, du diabète et de l'obésité, de la leucémie lymphoïde, du lymphome, du paludisme et de l'hypertrophie de la prostate.

**9.** Composés pour une utilisation destinée à un traitement selon la revendication 8, où la maladie tumorale est choisie dans le groupe de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, du poumon, de la peau, la leucémie monocytique, l'adénocarcinome du poumon, le carcinome du poumon à petites cellules, le cancer du pancréas, le glioblastome, le carcinome du sein, la leucémie myéloïde aiguë, la leucémie myéloïde chronique, la leucémie lymphoïde aiguë, la leucémie lymphoïde chronique, le lymphome hodgkinien, le lymphome non hodgkinien.

**10.** Composés de formule I selon les revendications 1-5 et/ou des sels physiologiquement acceptables de ceux-ci, pour une utilisation dans le traitement de tumeurs, où une quantité thérapeutiquement efficace d'un composé de formule I est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**11.** Composés de formule I selon les revendications 1-5 et/ou des sels physiologiquement acceptables de ceux-ci, pour une utilisation dans le traitement de tumeurs, où une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010003475 A2
- WO 2012033956 A1
- WO 0179157 A
- WO 02081415 A
- WO 2008011114 A
- WO 2011085201 A1
- WO 2011085198 A1
- WO 0050032 A
- US 6069134 A


### In der Beschreibung aufgeführte Nicht-Patentliteratur


- *Chemical Sciences,* 1994, vol. 49 (11), 1586-96
- *Analytica Chimica Acta,* 1987, vol. 202, 167-74
- *Journal of Electroanalytical Chemistry and Interfacial Electrochemistry,* 1988, vol. 239 (1-2), 161-73
- *Anorg. Chem. Org. Chem,* 1978, vol. 33B (12), 1540-6
- *J. Chem. Soc.,* Oktober 1965, 5556-62
- *J. Chem. Soc.,* Oktober 1965, 5551-6
- **A.L. MOSKALENKO et al.** *Russian Journal of Organic Chemistry,* 2011, vol. 47 (7), 1091-1096
- **S.-Q. YIN et al.** *Current Medicinal Chemistry,* 2012, vol. 19, 1021-1035
- **F. SPINELLA et al.** *J. Cardiovasc. Pharmacol.,* 2004, vol. 44, S140
- **A. MORABITO et al.** *Crit. Rev. Oncol./Hematol.,* 2004, vol. 49, 91
- **NICHOLSON et al.** *Cancer Metastas. Rev.,* 2001, vol. 20, 297
- **E. NG et al.** *Can. J. Ophthalmol.,* 2005, vol. 23, 3706
- **HENRI R. LIJNEN et al.** *Obesity,* 2010, vol. 18 (12), 2241-2246
- **X. CHEM et al.** *Chemistry & Biology,* 2009, vol. 16, 193-202
- *Angew. Chem.,* 1980, vol. 92, 129
- *Pharmaceutical Research,* 1986, vol. 3 (6), 318
- **HANZLIK et al.** *J. Org. Chem.,* 1990, vol. 55, 3992-3997
- **REIDER et al.** *J. Org. Chem.,* 1987, vol. 52, 3326-3334
- **FOSTER.** *Adv. Drug Res.,* 1985, vol. 14, 1-40
- **GILLETTE et al.** *Biochemistry,* 1994, vol. 33 (10), 2927-2937
- **JARMAN et al.** *Carcinogenesis,* 1993, vol. 16 (4), 683-688